# EUROPEAN PATENT APPLICATION

(11) **EP 2 249 159 A1**
(43) Date of publication of application: **10.11.2010**
(21) Application number: 09005931.2
(22) Date of filing: 29.04.2009
(51) Int. Cl.: G01N 33/574

(54) **Identification of tumor-associated markers for diagnosis and therapy**

(71) Applicant: Ganymed Pharmaceuticals AG, 55131 Mainz (DE); Johannes- Gutenberg-Universität Mainz, 55122 Mainz (DE)
(72) Inventor: Sahin, Ugur, 55116 Mainz (DE); Türeci, Özlem, 55116 Mainz (DE); Koslowski, Michael, 65933 Frankfurt (DE)
(74) Representative: Schnappauf, Georg

(57) **Abstract**

The invention relates to genetic products the expression of which is associated with cancer diseases. The invention also relates to the therapy and diagnosis of diseases in which the genetic products are expressed or aberrantly expressed, in particular cancer diseases.

SEQUENCE LISTING

<110> Ganymed Pharmaceuticals AG et al.

<120> Identification of tumor-associated markers for diagnosis and therapy

<130> 342-51

<160> 688

<170> PatentIn version 3.3

<210> 1
<211> 398
<212> DNA
<213> Homo sapiens

<220>
<221> misc_feature
<222> (272)..(272)
<223> n is a, c, g, t or u

<220>
<221> misc_feature
<222> (274)..(274)
<223> n is a, c, g, t or u

<220>
<221> misc_feature
<222> (284)..(284)
<223> n is a, c, g, t or u

<400> 1
aacataggtg gaccgctgct gagtccaggc ttacttgcag agatctatgc tggccaggcc 60

ctgtgctagg cagcagagga catggaataa aatcaaataa ggtcactgtg tgcaggcacc 120

tcacggtgtg gtaaaggagc agccccatcc acaggttcta ttaattccag cctgtgagaa 180

ttggaaccac agggtgaatt ttggaggaca ggcacttaca ctaatctgga agcataatat 240

ataaagagta cctacaaatc aataaaaaaa ananaaaaaa aaanagcaaa gtatatgaac 300

agaaaattca atgaaaagga aatagaaatg gctcttaaat gaatgaaaac atactctcac 360

tcagagaaat gaaaatttaa cccatgtcaa gatacttg 398

<210> 2
<211> 99
<212> DNA
<213> Homo sapiens

<400> 2
atgcacagag catcacgtac aatggctcca tggacagccc agtgcccttg taccctaccg 60

attgcccccc ttcttatgag gcagtcatgg gactacgag 99

<210> 3
<211> 266
<212> DNA
<213> Homo sapiens

<400> 3
aggagaaaac ctctacttgt cctgcttcgc ggaatctaac ccaccggcag agtattttg 60

gacaattaat gggaagtttc agcaatcagg acaaaagctc tctatccccc aaattactac    120

aaagcataga gggctctata cttgctctgt tcgtaactca gccactggca aggaaagctc    180

caaatccatg acagtcgaag tctctgctcc ttcaggaata ggacgtcttc ctctccttaa    240

tccaatatag cagccgtgaa gtcatt                                          266

<210>  4
<211>  492
<212>  DNA
<213>  Homo sapiens

<220>
<221>  misc_feature
<222>  (369)..(369)
<223>  n is a, c, g, t or u

<220>
<221>  misc_feature
<222>  (372)..(372)
<223>  n is a, c, g, t or u

<220>
<221>  misc_feature
<222>  (418)..(419)
<223>  n is a, c, g, t or u

<220>
<221>  misc_feature
<222>  (424)..(424)
<223>  n is a, c, g, t or u

<220>
<221>  misc_feature
<222>  (443)..(443)
<223>  n is a, c, g, t or u

<400>  4
aaaggagtca tcagcgtctc tttcctggat tatatctggg ttacctaaag ctctgtagct     60

ctgtggatca aatcaaagtt cttgttaccc aaaagttgcc caacattctc tgccacgtga    120

agatccgtga aaacaataat atttctagag aggaatggga atggatccaa aagctttctg    180

gctctgaatc tatggaaagt gtggatcata cttctgactg ccccatgcaa ttgttcttct    240

acgagctcca gatggcagtg aaagctctcc ttcagcagat caatatacct ctacaccagg    300

caaggaactt ccgcctctac acacaggagg tgttggaaat gggtcacaat gtgtcctttc    360

ttctcctgnt cnctgcctca gacgacgtct gtacagcccc aggacagaat aatccttnna    420

cccnacactc agggtttctt aanctccctc ttcagatgtt tgaacttggt atagtagctt    480

gtttcaccta ga                                                         492

<210>  5
<211>  133
<212>  DNA
<213>  Homo sapiens

<400>  5
ttcaaaaact gctggtgagc ctatggaaga ggagccagcc ttgtgaagtg ccaagtcccc     60

ctctgatatt tcctgtgtgt gacatcattg tgtatccccc caccccagta ccctcagaca 120

tgtcttgtct gct 133

<210> 6
<211> 371
<212> DNA
<213> Homo sapiens

<220>
<221> misc_feature
<222> (178)..(178)
<223> n is a, c, g, t or u

<400> 6
ggtttcggcc tctgcgaaag tgaaatgccc aagcctccgg ccaaagccca gaccagtaca 60

gtatgaattg tcctatgaga ctgaggggtt cggcttcatt cctacctgcc cgcaaagctc 120

gcccccagcc tcgaaaacaa agcgactggt ctgacgtggg gtccctgcgc ccctcctnta 180

gcgcgacagg acccccccag ggaagagcca gtacccgtgg gatgtcaccc cgtccccatc 240

taccggggtg gggggcctga aaggagaacg atttaaaata atcttcagaa agaaaaggga 300

ggagggagcg ggtgacacat cgttcacata aacccaattt ctggtttcga gtgaagtcaa 360

gatctccgcc c 371

<210> 7
<211> 215
<212> DNA
<213> Homo sapiens

<220>
<221> misc_feature
<222> (153)..(153)
<223> n is a, c, g, t or u

<400> 7
ccatttaaca tgtatagtag gtcaacattg gtgcatccag aaaatgaagc atttaggaaa 60

tctgtttcag tgtcttttca atgtgtgtaa cttttacttg caaaccaatg gaaccaagaa 120

agtcatcatt tgcctaaaat gcagtcatca ccncaaatga ttcatttata ctatgtgagt 180

taattgcctt catctcatta atggccaagg aggga 215

<210> 8
<211> 322
<212> DNA
<213> Homo sapiens

<400> 8
actgttcagt actgcaaccc caggatacac aatggcatct ggctctgttt attcaccacc 60

tactcggcca ctacctagaa acaccctatc aagaagtgct tttaaattca agaagtcttc 120

aaagtactgt agctggaaat gcactgcact gtgtgccgta ggggtctcgg tgctcctggc 180

aatactcctg tcttatttta tagcaatgca tctctttggc ctcaactggc agctacagca 240

gactgaaaat gacacatttg agaatggaaa agtgaattct gataccatgc caacaaacac 300

tgtgtcatta ccttctggag ac 322

<210> 9
<211> 547
<212> DNA
<213> Homo sapiens

<400> 9
ttacccttca ttcacctatt acggttcagg agaaaacctc gacttgtcct gcttcacgga 60

atctaaccca ccggcagagt atttttggac aattaatggg aagtttcagc aatcaggaca 120

aaagctcttt atcccccaaa ttactagaaa tcatagcggg ctctatgttt gctctgttca 180

taactcagcc actggcaagg aaatctccaa atccatgaca gtcaaagtct ctggtccctg 240

ccatggagac ctgacagagt ttcagtcatg actgcaacaa ctgagacact gagaaaaaga 300

acaggctgat accttcatga aattcaagac aaagaagaaa aaaactcaat gttattggac 360

taaataatca aaaggataat gttttcataa ttttttattg gaaaatgtgc tgattctttg 420

aatgttttat tctccagatt tatgaacttt ttttcttcag caattggtaa agtatacttt 480

tgtaaacaaa aattgaaata tttgcttttg ctgtctatct gaatgcccca gaattgtgaa 540

actactc 547

<210> 10
<211> 396
<212> DNA
<213> Homo sapiens

<400> 10
gcaggggacg caccaaggat ggagatgttc caggggctgc tgctgttgct gctgctgagc 60

atgggcggga catgggcatc caaggagccg cttcggccac ggtgccgccc catcaatgcc 120

accctggctg tggagaagga gggctgcccc gtgtgcatca ccgtcaacac caccatctgt 180

gccggctact gccccaccat gacccgcgtg ctgcaggggg tcctgccggc cctgcctcag 240

gtggtgtgca actaccgcga tgtgcgcttc gagtccatcc ggctccctgg ctgcccgcgc 300

ggcgtgaacc ccgtggtctc ctacgccgtg gctctcagct gtcaatgtgc actctgccgc 360

cgcagcacca ctgactgcgg gggtcccaag gaccac 396

<210> 11
<211> 311
<212> DNA
<213> Homo sapiens

<400> 11
aaatggtggt gtttgactgg tatatgacct tcctctggag gtgatcaacc agtaagggaa 60

aatcgctcca agtgagcatg cacacaacct cagtaaacac actgtgcatg tggcttctcc 120

caagtactag caggccactg cacatgtcac aactgagcaa cagcccaccc caatggaggg 180

atcaagggag gagaagaaaa accccggaac caaaagccag tttataaaaa tcctgagcca 240

aaggctgagg ggggcacttg atctctcaag ttccctactt ggccctcttc caagtgtgat 300

ttgcttcttt t                                                          311

<210>  12
<211>  246
<212>  DNA
<213>  Homo sapiens

<220>
<221>  misc_feature
<222>  (184)..(184)
<223>  n is a, c, g, t or u

<400>  12
gccttagccc ccgggattta gagcatcctc gcgaccaccc ggaggcttct gggggccact      60

ctgcggatga ggaagctgac gcctgggtgc agaaccccgg acccccggat tcagagccca     120

ggtccagccg cgcttccgca caaacttgcg ctcggagcaa gtcccctcct tcccagcact     180

catntgagac cagaggtgtc cccaccgtcc ccgctagcag cgctggttat attgtgggcc     240

aacctt                                                                246

<210>  13
<211>  516
<212>  DNA
<213>  Homo sapiens

<220>
<221>  misc_feature
<222>  (149)..(150)
<223>  n is a, c, g, t or u

<220>
<221>  misc_feature
<222>  (153)..(159)
<223>  n is a, c, g, t or u

<220>
<221>  misc_feature
<222>  (161)..(163)
<223>  n is a, c, g, t or u

<220>
<221>  misc_feature
<222>  (165)..(165)
<223>  n is a, c, g, t or u

<220>
<221>  misc_feature
<222>  (168)..(184)
<223>  n is a, c, g, t or u

<400>  13
ccttttgctt gagcagggtt cccaggaggg agaaagagaa gacaagagcc tgatgcccaa      60

ctttgtgtgt gtggggacgg gggagtcagg gccccccaag tcccacaata gccccaatgt     120

ttgcctatcc acctccccca agccccttnn ccnnnnnnnc nnntnacnnn nnnnnnnnnn     180

nnnnctgctg ctgctgctgc tgctgcttaa aggctcatgc ttggagtggg gactggtcgg     240

tgcccagaaa gtctcttctg ccactgacgc ccccatcagg gattgggcct tctttccccc     300

ttcctttctg tgtctcctgc ctcatcggcc tgccatgacc tgcagccaag cccagccccg 360

tggggaaggg gagaaagtgg gggatggcta agaaagctgg gagataggga acagaagagg 420

gtagtgggtg ggctaggggg gctgccttat ttaaagtggt tgtttatgat tcttatacta 480

atttatacaa agatattaag gccctgttca ttaaga 516

<210> 14
<211> 162
<212> DNA
<213> Homo sapiens

<220>
<221> misc_feature
<222> (92)..(92)
<223> n is a, c, g, t or u

<400> 14
gagcctagag agtaaggaac gttatatagt tttccccaaa ggttcacttg aaagaacttt 60

tcattggttg tcatggtagt aatgtcctga tnttgaaatc tcccagaacc tagtagctct 120

taaacatgct ttcatcttgg ttcctttggt ctgacggaaa ct 162

<210> 15
<211> 523
<212> DNA
<213> Homo sapiens

<220>
<221> misc_feature
<222> (49)..(49)
<223> n is a, c, g, t or u

<400> 15
tttgcaaaag gtttccggga cactggaaat ggccgaagag aaaaaagana acagctcacc 60

ctgcagtcca tgagggtgtt tgatgaaaga cacaaaaagg agaatgggac ctctgatgag 120

tcctccagtg aacaagcagc tttcaactgc ttcgcccagg cttcttctcc agccgcctcc 180

actgtaggga catcgaacct caaagattta tgtcccagcg agggtgagag cgacgccgag 240

gccgagagca aagaggagca tggccccgag gcctgcgacg cggccaagat ctccaccacc 300

acgtcggagg agccctgccg tgacaagggc agccccgcgg tcaaggctca ccttttcgct 360

gctgagcggc cccgggacag cgggcggctg gacaaagcgt cgcccgactc acgccatagc 420

cccgccacca tctcgtccag cactcgcggc ctgggcgcgg aggagcgcag gagcccggtt 480

cgcgagggca cagcgccggc caaggtggaa gaggcgcgcg cgc 523

<210> 16
<211> 424
<212> DNA
<213> Homo sapiens

<400> 16
actcggtcac actcagtaag tccttgcaga gtccatgggt ttcttcgaca agtggcttca 60

aggaagggaa ttcccaccct tgtcttccag caaggccaca cacatgaaac cagcagaaaa 120

gagtcttatt tgctggaaag acccccagca agggcatagt gagcccttac agtggttcca 180

gtcagaaaag gcaccacttg ggtgggcaca gccccatggg tgtccaactt ggtaagcaga 240

gcaaggctgg acttgagtcc ccgtcctcca caaaacacag agccacaagc cccagccctg 300

cagcagccct ccggaagcag cggggcactg gtttccttgt cccctgccat ctaccgagtg 360

gctcactctc aggtgggagt gctggtgatg gttaattagg actgcagaaa catgagcctc 420

ctta 424

<210> 17
<211> 524
<212> DNA
<213> Homo sapiens

<400> 17
ctatctttct ggtcacattg tcggtgtttc tgcatgttct ccattccgct cctgatgtgc 60

aggattgccc agaatgcacg ctacaggaaa acccattctt ctcccagccg ggtgccccaa 120

tacttcagtg catgggctgc tgcttctcta gagcatatcc cactccacta aggtccaaga 180

agacgatgtt ggtccaaaag aacgtcacct cagagtccac ttgctgtgta gctaaatcat 240

ataacagggt cacagtaatg gggggtttca aagtggagaa ccacacggcg tgccactgca 300

gtacttgtta ttatcacaaa tcttaaatgt tttaccaagt gctgtcttga tgactgctga 360

ttttctggaa tggaaaatta agttgtttag tgtttatggc tttgtgagat aaaactctcc 420

ttttccttac cataccactt tgacacgctt caaggatata ctgcagcttt actgccttcc 480

tccttatcct acagtacaat cagcagtcta gttcttttca tttg 524

<210> 18
<211> 538
<212> DNA
<213> Homo sapiens

<400> 18
gtagggcgaa ctctgctata cagtttatga tgtcagagtg aatactttct ttgagttgca 60

gtcagaaact gtagattttt aaaaatttaa aattcattat tctctgtcag tattccaaag 120

tgtatacaga aagctattgc actgttcagg agatggcgct taacattttg gaaattcaag 180

gtgatgaatg tccagataag actatctctc ctggtacaaa gtttgacaat gctgaacatt 240

tttaaaggtt ctttttgata tacaaagtgc accaatgagt gctttttaat tcttacaata 300

attctgggtg aggtaggtat ttttccaatt cccattttat gcttcggtag ccctttgtat 360

ttatacttca aaacacttgg ctctcttgta attatttaag aaattagttg tgattatttg 420

tttaatgtgc aggagttaca aaaggcaagc tttagaacaa gacagacctg gttatgattc 480

ctggctctga aagctgtaca ccctgtgacc ctagacaggt gttttaatgc ctcgctgc 538

<210> 19
<211> 324
<212> DNA

<213>  Homo sapiens

<220>
<221>  misc_feature
<222>  (294)..(295)
<223>  n is a, c, g, t or u

<400>  19
tcttggtctt ctgctcatgg atgacgcacc agtcgtgcat gttcttggtg aagtcggcca      60

gcctgagcaa gcggaggacc tacgccggcc tggcattcca cgcctacggg aaggcaggca     120

agatgctggt ggagaccagc atgatcgggc tgatgctggg cacctgcatc gccttctacg     180

tcgtgatcgg cgacttgggg tccaacttct ttgcccggct gttcgggttt caggtgggcg     240

gcaccttccg catgttcctg ctgttcgccg tgtcgctgtg catcgtgctc ccgnncagcc     300

tgcagcggaa catgatggcc tcca                                            324

<210>  20
<211>  414
<212>  DNA
<213>  Homo sapiens

<400>  20
agaaagcaga gcagcctcct ggaagaaggc cttgtcagct ttgtctgtgc ctcgcaaatc      60

agaggcaagg gagaggttgt taccagggga cactgagaat gtacatttga tctgccccag     120

ccacggaagt cagagtagga tgcacagtac aaaggagggg ggagtggagg cctgagaggg     180

aagtttctgg agttcagata ctctctgttg ggaacaggac atctcaacag tctcaggttc     240

gatcagtggg tcttttggca ctttgaacct tgaccacagg gaccaagaag tggcaatgag     300

gacacctgca ggaggggcta gcctgactcc cagaacttta agactttctc cccactgcct     360

tctgctgcag cccaagcagg gagtgtcccc ctcccagaag catatcccag atga           414

<210>  21
<211>  531
<212>  DNA
<213>  Homo sapiens

<400>  21
caaagtcatc tgaacttccg tttccccagg gcctccagct gccctcagac actgatgtct      60

gtccccaggt gctctctgcc cctcatgccc ctctcaccgg cccagtgccc cgactctcca     120

ggctttatca aggtgctaag gcccgggtgg gcagctcctc gtctcagagc cctcctccgg     180

cctggtgctg cctttacaaa cacctgcagg agaagggcca cggaagcccc aggctttaga     240

gccctcagca ggtctgggga gctagagcaa aggagggacc tcaggccttc cgtttcttct     300

tccagggtgg ggtggcctgg tgttccccta gccttccaaa cccaggtggc ctgcccttct     360

ccccagaggg aggcggcctc cgcccattgg tgctcatgca gactctgggg ctgaggtgcc     420

ccggggggtg atctctggtg ctcacagccg agggagccgt ggctccatgg ccagatgacg     480

gaaacagggt ctgaccaagt gccaggaaga cctgtgctat aaaccaccct g              531

<210> 22
<211> 522
<212> DNA
<213> Homo sapiens

<400> 22
atgtcctgcc atttggtcat aagacagttg catttactct gctaccattg cttcagttga 60

tatgaagaga gaaagctgtg ttgtgattta cactggatat ggaaatagag aggaacaaat 120

ctgtctgatc tactttcttc aacctctgta gtagctaata atataggaca gaatgctcca 180

aagaatgaaa atgaaagtca agattcaatg gatgaaagtg agaactcctc caggtcctgg 240

aaacaaacca tttagcatca ggtcagaagc tactccatgg aattctgaga ccacgaaagc 300

caggtcaggt ctcaaattca gtagcccacc acccacacca ccacccacac caccctgctt 360

cccctcatgc ttgctgcctc catttccttc tggaccacca ataattcccc caccacctcc 420

cacaggtcta gattttcttg atgatgttaa tgttttatga agtatgctaa tctcttggta 480

cattaagtgg ctatcatact ggctattata cagggctcaa gc 522

<210> 23
<211> 520
<212> DNA
<213> Homo sapiens

<220>
<221> misc_feature
<222> (98)..(98)
<223> n is a, c, g, t or u

<220>
<221> misc_feature
<222> (142)..(142)
<223> n is a, c, g, t or u

<400> 23
gaaaggcctg caattgtgtc ttcacgatgc ttttccaaga cagccaaggc aggtataatt 60

ttcctcagca agaaagagga acctcggagg tgtgcacngc ctggctggcc acccaggtat 120

tggcaaaagt gactgtcggg cntgctggcc cggcccccgc ccgccgtccc tggagcactc 180

acgatgcggt ccggcggcgg cgtgctccgg atgaagcact tgatctggcc cttctcgccg 240

tggagggcgt gctgggtctg ggtgctggag atgatggggg gtcctgttga gaaacagcgt 300

cccattaggc acccgggaag ggcacgtccc tgctggcgcc ctcttgggtg ggttcagaag 360

tgtattcatt aatccaagca ttcagcaaac atttgccgaa ggcctgtatg tgcaaggtaa 420

agtgcaaggt agaggactca gagataaatt aggcattcag tcataaacct ctcaagggat 480

catgagcgaa tgcttctaag tcagaacccc cagaagatac 520

<210> 24
<211> 488
<212> DNA
<213> Homo sapiens

<400> 24
agcagaacct cctagggttt ctccttccac tccttgccat gatcttcttc tactcccgta 60

ttggttgtgt cttggtgagg ctgaggcccg caggccaggg ccgggcttta aaaatagctg 120

cagccttggt ggtggccttc ttcgtgctat ggttcccata caatctcacc ttgtttctgc 180

atacgctgtt ggacctgcaa gtattcggga actgtgaggt cagccagcat ctagactacg 240

cactccaggt aacagagagc atcgccttcc ttcactgctg cttttccccc atcctgtatg 300

ccttctccag tcaccgcttc cgccagtacc tgaaggcttt cctggctgcc gtgcttggat 360

ggcacctggc acctggcact gcccaggcct cattatccag ctgttctgag agcagcatac 420

ttactgccct tgaggaaatg actggcatga atgaccttgg agagaggcag tctgagaact 480

accctaac 488

<210> 25
<211> 552
<212> DNA
<213> Homo sapiens

<400> 25
gaggagagac ctgcgtggga taatcaacag gggtctggag gacggggaga gctgggaata 60

tcagatctga ctgcgtgttc tcacttcgct tcctggaact tgctctcatt ttcctgggtg 120

catcaaacaa aacaaaaacc aaacacccag aggtctcatc tcccaggccc caggggagaa 180

agaggagtag catgaacgcc aaggaatgta cgttgagaat cactgctcca ggcctgcatt 240

actccttcag ctctggggca gaggaagccc agcccaagca cggggctggc agggcgtgag 300

gaactctcct gtggcctgct catcaccctt ccgacaggag cactgcatgt cagagcactt 360

taaaaacagg ccagcctgct tgggcgctcg gtctccaccc cagggtcata agtggggaga 420

gagcccttcc cagggcaccc aggcaggtgc agggaagtgc agagcttgtg gaaagcgtgt 480

gagtgaggga gacaggaacg gctctggggg tgggaagtgg ggctaggtct tgccaactcc 540

atcttcaata aa 552

<210> 26
<211> 511
<212> DNA
<213> Homo sapiens

<400> 26
aagcctcaag gcacttctag gacctggctc ttctcaccaa gatgaactca ctggtttctt 60

ggcagctact gcttttcctc tgtgccaccc actttgggga gccattagaa aaggtggcct 120

ctgtggggaa ttctagaccc acaggccagc agctagaatc cctgggcctc ctggcccccg 180

gggagcagag cctgccgtgc accgagagga agccagctgc tactgccagg ctgagccgtc 240

gggggacctc gctgtccccg ccccccgaga gctccgggag ccgccagcag ccgggcctgt 300

ccgcccccca cagccgccag atccccgcac cccagggcgc ggtgctggtg cagcgggaga 360

aggacctgcc gaactacaac tggaactcct tcggcctgcg cttcggcaag cgggaggcgg 420

caccagggaa ccacggcaga agcgctgggc ggggctgggg cgcaggtgcg gggcagtgaa 480

cttcagaccc caaaggagtc agagcatgcg g 511

<210>  27
<211>  131
<212>  DNA
<213>  Homo sapiens

<400>  27
ctcctccagc aagacagatg cctagcccgt cctcaggaat ctgccgccag ggagaatggc      60

aaccctggcc agatagctgg aagcacaggg ttgctcttca acctgcctcc cggctcagtt     120

cactataaga a                                                          131


<210>  28
<211>  304
<212>  DNA
<213>  Homo sapiens


<220>
<221>  misc_feature
<222>  (41)..(41)
<223>  n is a, c, g, t or u

<220>
<221>  misc_feature
<222>  (84)..(84)
<223>  n is a, c, g, t or u

<400>  28
tttcctctga gagaacagcg gtcttctgtc tgctgtggca nagcaagtca cttcttcttg      60

tagtgagaac tgaaaccaga accnatcatg tgccacttcc tggacacctc ctattaaata     120

ttaaagtcct ctcaccacag aagccggagt ttagtggtta ggggcacagg ttcttagata     180

tgaacatcag ttgcaaccta ccaactgcat gctcttggac aatttacatt tctgtgtatc     240

agctttcctt tttctttaga atgagatatt aatagtagca acccagaatt gtcatgaagc     300

ctaa                                                                  304


<210>  29
<211>  226
<212>  DNA
<213>  Homo sapiens

<400>  29
catggcaaag gcttgcccca aatctcaact tctcagacgt tccatacccc cacatgccaa      60

tttcagcacc caactgagat ccgaggagct cctgggaagc cctgggtgca ggacactggt     120

cgagagccaa aggtccctcc ccagacatct ggacactggg catagatttc tcaagaagga     180

agactcccct gcctccccag ggcctctgct ctcctgggag acaaag                    226


<210>  30
<211>  567
<212>  DNA
<213>  Homo sapiens


<220>
<221>  misc_feature

<222>   (195)..(195)
<223>   n is a, c, g, t or u

<220>
<221>   misc_feature
<222>   (216)..(216)
<223>   n is a, c, g, t or u

<220>
<221>   misc_feature
<222>   (226)..(226)
<223>   n is a, c, g, t or u

<220>
<221>   misc_feature
<222>   (255)..(256)
<223>   n is a, c, g, t or u

<220>
<221>   misc_feature
<222>   (261)..(261)
<223>   n is a, c, g, t or u

<220>
<221>   misc_feature
<222>   (284)..(284)
<223>   n is a, c, g, t or u

<220>
<221>   misc_feature
<222>   (313)..(313)
<223>   n is a, c, g, t or u

<220>
<221>   misc_feature
<222>   (328)..(328)
<223>   n is a, c, g, t or u

<220>
<221>   misc_feature
<222>   (333)..(334)
<223>   n is a, c, g, t or u

<220>
<221>   misc_feature
<222>   (356)..(356)
<223>   n is a, c, g, t or u

<220>
<221>   misc_feature
<222>   (411)..(411)
<223>   n is a, c, g, t or u

<220>
<221>   misc_feature
<222>   (471)..(471)
<223>   n is a, c, g, t or u

<220>
<221>   misc_feature
<222>   (473)..(473)
<223>   n is a, c, g, t or u

<220>
<221>   misc_feature
<222>   (492)..(492)
<223>   n is a, c, g, t or u

<220>
<221> misc_feature
<222> (497)..(500)
<223> n is a, c, g, t or u

<220>
<221> misc_feature
<222> (503)..(503)
<223> n is a, c, g, t or u

<220>
<221> misc_feature
<222> (510)..(511)
<223> n is a, c, g, t or u

<220>
<221> misc_feature
<222> (513)..(513)
<223> n is a, c, g, t or u

<400> 30
ggccaccaga ggattctcag ggctcctttg tcctggactg tggaactggg ggcagctggt 60

ccctggggtc tctgaagtca gtgtctccct cactgctcac tgccatggtg tctctgcctc 120

tgcttctctg tgtccctcat cttcctccca cttcattctg actggcaagc cctgtcctgc 180

acagcttctt ccccnacccc taggccttcc ccaganactc cctctnacta ggctggctgt 240

tctgttccct tcccnnctaa nactgtggcc tggcccacct cccnaggaaa taggaaaggt 300

gcagaaatca ccntggagtt gccactcntg ccnnggcttc atctcgagcc aatgtnccca 360

ggtcactaag agaatgagct tccactgtat tcccatccag ggctctttcc ntttgtgagg 420

ctgacctgtg gacaagacaa tgggacaggg ataggcagtt cctccatcca ntntcataat 480

tgccaggcaa gntcttnnnn ccncctgcan nancctcccc agtggatcag gggttagaga 540

tattcaaggg tagtttcagg agcacag 567

<210> 31
<211> 448
<212> DNA
<213> Homo sapiens

<220>
<221> misc_feature
<222> (82)..(82)
<223> n is a, c, g, t or u

<220>
<221> misc_feature
<222> (96)..(97)
<223> n is a, c, g, t or u

<400> 31
taatgcggac gtaccgactg ccagatcttt acactcaccc ctccacctgc cccgaggagt 60

ccggtcacaa gggcccagcc antcacaaag acaccnnggt gtcccttcca tttttttcca 120

cgaaggccca gaatccattt taggtttcca aacagacctt tcgtcccttc aaggtgtaac 180

caccgttttc cattccagcc attttattgg ccacaccgtt accttactta taggtatttc 240

cccagaagaa gactccagag aggaagctca tctgaggaaa gctgagaggg aagagaaacc 300

caaacatact gaagcaaaaa aaagcctatc cttcagaaaa aagcaacaaa aagatttctg 360

ttttatcttt cgaaactaaa actattggat ttgaagatta agtatcctaa acatcactga 420

ctagaaactg ttctctttgt cagcagtg 448

<210> 32
<211> 396
<212> DNA
<213> Homo sapiens

<220>
<221> misc_feature
<222> (141)..(141)
<223> n is a, c, g, t or u

<400> 32
agtgtgcatg ttcactgggc atcttccctt cgaccccttt gcccacgtgg tgaccgctgg 60

ggagctgtga gagtgtgagg ggcacgttcc agccgtctgg actctttctc tcctactgag 120

acgcagccta taggtccgca ngccagtcct cccaggaact gaaatagtga aatatgagtt 180

ggcgaggaag atcaacatat aggcctaggc caagaagaag tttacagcct cctgagctga 240

ttggggctat gcttgaaccc actgatgaag agcctaaaga agagaaacca cccactaaaa 300

gtcggaatcc tacacctgat cagaagagag aagatgatca gggtgcagct gagattcaag 360

tgcctgacct ggaagccgat ctccaggagc tatgtc 396

<210> 33
<211> 484
<212> DNA
<213> Homo sapiens

<400> 33
cggtggcttg caacatgctc atgccagagc ccgctcaacg ctggctggtg ggcttcgtgt 60

tgtacacatt tctcatgggc ttcctgctgc ccgtgggggc tatctgcctg tgctacgtgc 120

tcatcattgc taagatgcgc atggtggccc tcaaggccgg ctggcagcag cgcaagcgct 180

cggagcgcaa gatcacctta atggtgatga tggtggtgat ggtgtttgtc atctgctgga 240

tgcctttcta cgtggtgcag ctggttaacg tgtttgctga gcaggacgac gccacggtga 300

gtcagctgtc ggtcatcctc ggctatgcca acagctgcgc caaccccatc ctctatggct 360

ttctctcaga caacttcaag cgctctttcc aacgcatcct atgcctcagc tggatggaca 420

acgccgcgga ggagccggtt gactattacg ccaccgcgct caagagccgt gcctacagtg 480

tgga 484

<210> 34
<211> 393
<212> DNA
<213> Homo sapiens

<400> 34
tctgacttcc agaacctacg ataatagact ccatgaaatc tgtaatcagt ggccacagga 60

aactcatgca acagcccttc caaggggctc cccagcaaag cctccgtggt gtctgccccc 120

aacccctgtg cctcctggga cacaagacag gcccagcaag ggtgggggtg ccacggaaag 180

cttggtggct gggcaggtcc ccagagggcc gccatcagtc ctcaaagaca tgctcagatg 240

cagtggctca ggcctggcac cagctggtcc caaggtgggg tggtgagggt acatctgctg 300

tgcacacgtg gctggacgcg ctgggggcag gtccaggtca gcttcaagga ctctgcccag 360

gctaacccta gaggcctcta gtgccagcag tta 393

<210> 35
<211> 493
<212> DNA
<213> Homo sapiens

<400> 35
aggcccatgt gctgtttttg acttcagtac ttcagattgc tgtgggaaca caggaggcag 60

cagccagatg agaaattgag tctgactctg gagtattata aagtccttat agttactggc 120

attaggtata gggtctgtat tattaaagag aaattattca ccaaacactt gttaaaaatg 180

gcaagacagt ttatttaaga gcattgcaat aggtaagtgc tatggtctca atgtttgtgt 240

ctccctcaaa ttcataagtt gaaacttcac ttccaagatg aaggaattag gaggtgggca 300

ctttaaggga tgattatgtc ataggccaga gccctcatga acgagatcag tgcccttcta 360

aaagaggcat tgggagagac ccctcacctt ttccatcata tgaggacaca gccaggaagc 420

atcatccacg aaccagaaaa ttggccctta ccagacactg aatctgctga tgtcctgacc 480

atggacttct gag 493

<210> 36
<211> 336
<212> DNA
<213> Homo sapiens

<400> 36
acgctgcagc gtcacattaa tctttgtgcc gccagtgcct atgccatgct tagtatgcat 60

caaatatttg agcagtacac aagtgagtac tctgagagct ccccccacca aaaatatgat 120

gattaaatac agttatgatc agatccccag agtgtggctc taaactgtat gggggccaag 180

tttgaatact gttgtgtctt acactgttat tacctatcca gtatctattt ccccatattc 240

cttataaata aaacctagat tttgattggg acagtaaggt gtcccactga aaactcattt 300

ctctaaccaa tgtgatgcca gtgcttgccc aaaaag 336

<210> 37
<211> 507
<212> DNA
<213> Homo sapiens

<400> 37
gtgagtgaac gtgaaggcct gcagcattac tgtacactac catagatttt tatcaacact 60

gtacacttag ggacactaaa cttatttaaa cattttttct tcaaaaataa attaacctca 120

gctcactgta actttataag ctttatattt aaaaaaactt tttgactctt ttgtagtaac 180

acttagctta aaacacaaac acattgtaca gttacacaaa atattttctt aaaaaatatt    240

ttattatatc ctattctata agcttttcct tgtttttcac ttttttttaa cttttaaact    300

ttttataaaa actaagacac aaacacacac attagtgcag gcctgcatag catcaggatc    360

atcagtatca ctgtctccca cctccgcatc ttgtcccact gaaaggtctt cagcgggaat    420

atcatgcatg gagctgtcat ctcctgtgat aacaatgcct tcttctggat acctcctgaa    480

ggacctggtt gagcctgttt tacagtt                                        507

<210>  38
<211>  423
<212>  DNA
<213>  Homo sapiens

<400>  38
gaatccctta agcagaacaa ccgtgatgcc atggaactca agcccaacgg cggtgctgac     60

caaaaatgtc tcaaagtcaa cagcccaata agaatgaaga atggaaatgg aaaagggtgg    120

ctgcgactca agaataatat gggagcccat gaggagaaaa aggaagactg gaataatgtc    180

actaaagctg agtcaatggg gctattgtct gaggacccca agagcagtga ttcagagaac    240

agtgtgacca aaaacccact aaggaaaaca gattcttgtg acagtggaat tacaaaaagt    300

gaccttcgtt tggataaggc tggggaggcc cgaagtccgc tagagcacag tcccatccag    360

gctgatgcca agcacccctt ttatcccatc cccgagcagg ccttacagac cacactgcag    420

gaa                                                                  423

<210>  39
<211>  365
<212>  DNA
<213>  Homo sapiens


<220>
<221>  misc_feature
<222>  (244)..(244)
<223>  n is a, c, g, t or u

<220>
<221>  misc_feature
<222>  (253)..(253)
<223>  n is a, c, g, t or u

<400>  39
ttactctgtg attaacttcc ttctccctca ccccaaaata cagaagagtg aaatctctga     60

ccagaaagtt cctggcacct accttgggtt ctgtgaaaaa ataatggccc tggttttcaa    120

tgctgccaaa gttaagaaaa gttttcaccc cttcatttta aagcagccat aaagtgccat    180

gtgtttaacc gcaggaaaaa aagggtcttt ttaactattg agaagtagct tttcatatcc    240

ccancagggg aangaaagag cgggaaccag gagactcgtg aggactgcaa agatggtcct    300

ccctgggtac ttctgctgct ctcttctctc cagagctact ttgtgattgg cctgatggtc    360

agacc                                                                365

<210> 40
<211> 389
<212> DNA
<213> Homo sapiens

<400> 40
gatggagcat catgggttgg attattactg agttcaataa tctggtgggt tttgccagct 60

agaaacataa taaaatacat gataaaggaa tagaaaggaa atatatttat ttgaaattaa 120

attactgctt ataaattcat gtctctgatt ttacaaagtg taatgggtaa aattaccata 180

ttctttttct tatttcaatc catacaatga gagtcatgtt cagtttttca ctgacttcat 240

gctgggtaat gttcactctg cattagcggt tgccatgttc accgttttct tacaatgtct 300

atccagtgct tgttactgtc tcactgacag acagaagtct agctgttttc atccacataa 360

tggcaggcag ggctagtgtt gctgctgct 389

<210> 41
<211> 537
<212> DNA
<213> Homo sapiens

<400> 41
ttatgtccct gctggtattt ttgctttttc ataaaaatta tcatttattt ttctcaactg 60

catattgcca tcttcatttt ttaattttct cacatattca tagaattgtt ctctgtaata 120

gatactgtac agtaattatt tgttgatcga ttaacctttt caatgctact tccgacacct 180

acttcccatc ctccgtgtaa cagatactgt cagttaccta tccttaacag cctttccact 240

cccaacttct gtgaatggac aagagatgca attgtgatca ctgaacatga ggcaacatct 300

tctaggaaga catttccata gtcttcagac aaaagggaga gatatctttt cagacaatct 360

ttgaacaatc ctatatgaag cttacctgaa gttgctgtag ccgtttggca agtctgggga 420

gactaacaga cacactgagg atagcagaaa ataaagatag aaacagccca ggtttttggt 480

gaaattcatg agcttctgaa taacgaaccc cataccaccc tacctctata aaagaat 537

<210> 42
<211> 351
<212> DNA
<213> Homo sapiens

<400> 42
tggatcccag catcgttggc aatagggttt taggtggagt ctatctggca ttcagagaag 60

agtcaggaaa acaattgtat tcccagcctg tgtccctagg gcacaagcaa atcccaaatt 120

ctcctcctga accctccaaa tttgtctaag aacttcgaaa actttaacaa acaggctgat 180

atcttcataa tattcccagc ctagaccaag caggaagaac attgatttca ttgaaataat 240

tgataataat gaagataatg tttttatgat ttttatttga aaatttgcta attctttaaa 300

tggtttgttt tctacattga tggaattttt ctcttttaat ctatctacag c 351

<210> 43
<211> 528

<212>  DNA
<213>  Homo sapiens

<400>  43
tctgtttatc ccccaaatta ctacaaagca tagcgggctc tatgtttgct ctgttcgtaa      60

ctcagccact gggcaggaaa gctccacatc gttgacagtc aaagtctctg cttctacaag     120

aataggactt cttcctctcc ttaatccaac atagcagctg tgatgtcatt tctgtatttc     180

aggaagactg gcaggagatt tatggaaagg tctcttacaa ggactcttga atacaagctc     240

ctgataactt caagatcata ccactggact aagaactttc aaaattttaa tgaacaggct     300

gataccttca tgaaattcaa gacaaagaag aaaaatactc aatgttattg gactaaataa     360

tcaaaaggat aatgatttca taattttcta tttgaaaatg tgctgattct tggaatgttt     420

cattctccag atttatgaac atttttcttt gagcaattgg taaagtatac ttttgtaaac     480

aaaaattgaa acatttcctt ttgctctcta tctgagtgcc ccagaatt                  528


<210>  44
<211>  545
<212>  DNA
<213>  Homo sapiens

<400>  44
gggacacacc agcacagtct ggtaggctac agcagcaagt ctctaaagaa aggctgagaa      60

cacccagaac aggagagttc aggtccagga tggccagcct gttccggtcc tatctgccag     120

caatctggct gctgctgagc caactcctta gagaaagcct agcagcagag ctgaggggat     180

gtggtccccg atttggaaaa cacttgctgt catattgccc catgcctgag aagacattca     240

ccaccacccc aggagggtgg ctgctggaat ctggacgtcc caaagaaatg gtgtcaacct     300

ccaacaacaa agatggacaa gccttaggta cgacatcaga attcattcct aatttgtcac     360

cagagctgaa gaaaccactg tctgaagggc agccatcatt gaagaaaata atactttccc     420

gcaaaaagag aagtggacgt cacagatttg atccattctg ttgtgaagta atttgtgacg     480

atggaacttc agttaaatta tgtacatagt agagtaatca tggactggac atctcatcca     540

ttctc                                                                 545


<210>  45
<211>  166
<212>  DNA
<213>  Homo sapiens


<220>
<221>  misc_feature
<222>  (35)..(36)
<223>  n is a, c, g, t or u

<220>
<221>  misc_feature
<222>  (38)..(41)
<223>  n is a, c, g, t or u

<220>
<221>  misc_feature

<222>  (43)..(45)
<223>  n is a, c, g, t or u

<220>
<221>  misc_feature
<222>  (57)..(57)
<223>  n is a, c, g, t or u

<400>  45
tgctgtttgt gtgaaacctc cactgtgcca agcannannn nannngactg tgaatanttt      60

aacatttatt cacagatagc atgaaaagcc acagtccatt tgccatttag cttatttgat     120

tgagagaaaa ctgaggcaca ggaaggcaca gtgactgagc aagagt                    166


<210>  46
<211>  205
<212>  DNA
<213>  Homo sapiens


<220>
<221>  misc_feature
<222>  (28)..(28)
<223>  n is a, c, g, t or u

<400>  46
ggatcagtct taagaggagc tttttttngg agcgagaaat catataaaat aaaatgaaat      60

aaaacaagga ggaaggcaac cagctgttag gggaaaaata aggcagataa aggagcgggg     120

agagaaatta attgccaacc aggaggagtt gggctgtatt tttcaaaggt ggggagagtg     180

gagcacacac cttgaggagg aaagc                                           205


<210>  47
<211>  294
<212>  DNA
<213>  Homo sapiens


<220>
<221>  misc_feature
<222>  (68)..(68)
<223>  n is a, c, g, t or u

<220>
<221>  misc_feature
<222>  (132)..(132)
<223>  n is a, c, g, t or u

<220>
<221>  misc_feature
<222>  (193)..(193)
<223>  n is a, c, g, t or u

<220>
<221>  misc_feature
<222>  (207)..(207)
<223>  n is a, c, g, t or u

<220>
<221>  misc_feature
<222>  (213)..(213)
<223>  n is a, c, g, t or u

<220>
<221>  misc_feature
<222>  (219)..(220)
<223>  n is a, c, g, t or u

<220>
<221>  misc_feature
<222>  (242)..(242)
<223>  n is a, c, g, t or u

<220>
<221>  misc_feature
<222>  (260)..(260)
<223>  n is a, c, g, t or u

<220>
<221>  misc_feature
<222>  (267)..(267)
<223>  n is a, c, g, t or u

<400>  47
gaaccatttg agattcaatg cctgtgtcca gctcccagga gtccaaccgt gaaatccaca      60

agtgcagncc ccaccctgtc ctgcagttct ctttccctta tgataatgtg gttgagtcct     120

ttgtcactcc cntcctcctg ctggctgcag aaatgacctc agcccaggcc agagacccca     180

gctctggcaa ggncctcttg tggtcgncca ggncccagnn tgaaagccaa gcagaatcag     240

gncaggatct ctagcgggan gggaaancct gataggacct ttgtcagact tttg           294


<210>  48
<211>  432
<212>  DNA
<213>  Homo sapiens

<400>  48
acatttaccg tattacctag cactttcatt ccttgttgtc tactccaaag gaaaaaaacc      60

tatgtccaca caacacatga atgtgaatat tagtagcagc tttatccata atagtccata     120

aagtagaaac acatcaaata tctatcagct gatgaaagaa taaacaaatg ggagtgatcc     180

atacaattta atagaatcta gcacctaaaa aaataaaata ttgatacgtg ctacaacaca     240

ggtgaaccac aaaagcacat taatctaagt gaaagaagac agatacaaaa aaccacatgt     300

tgtatgactc tatttttatg atatccagaa aagacaaatc tgtagtgtca gtaagtcaat     360

taggggttgt ctggagctgg ggagtgggaa taaggggtgg tattgatgag catgagggat     420

ttcttaggaa tt                                                         432


<210>  49
<211>  541
<212>  DNA
<213>  Homo sapiens


<220>
<221>  misc_feature
<222>  (54)..(54)
<223>  n is a, c, g, t or u

<220>
<221>  misc_feature

<222>  (67)..(67)
<223>  n is a, c, g, t or u

<220>
<221>  misc_feature
<222>  (79)..(79)
<223>  n is a, c, g, t or u

<220>
<221>  misc_feature
<222>  (103)..(103)
<223>  n is a, c, g, t or u

<220>
<221>  misc_feature
<222>  (127)..(127)
<223>  n is a, c, g, t or u

<220>
<221>  misc_feature
<222>  (168)..(168)
<223>  n is a, c, g, t or u

<220>
<221>  misc_feature
<222>  (498)..(499)
<223>  n is a, c, g, t or u

<400>  49
gtgaatccta gagtagtttg ctatcaactt ctgatctttg cacattctgg attnggcata      60

taatgtnaca gcagtgccna ttgtaatgtt gcacaaagta gtntagcaat ttcttggttc     120

accaggntta gagataacat tgtagaaatg atccagcatc tttaacantc tgtggtttaa     180

ggtggggcac ttaggggtag aatcaataac aatgttagaa atcaaattag acaagataac     240

tgaaacagca tgatccatgt gtgactccaa gttataaagg aggacatgga ttaatggtat     300

acttctaggc tataggggta gtacaagtgg aaggacacca tcttagcatc agatcacttt     360

ctgagcaact ttggcaaatc ttttaaattc tctaatgtgt agttttttaa tatatgacac     420

aggtgtaaag aaaataaagc aagtgaatgt atgtgaaagc caatgctgac tgggcacggg     480

ggctcacgcc tgaaattnnt agcactttgg gaggcagagc cggggatatc acttgagccc     540

a                                                                     541


<210>  50
<211>  393
<212>  DNA
<213>  Homo sapiens


<220>
<221>  misc_feature
<222>  (63)..(63)
<223>  n is a, c, g, t or u

<220>
<221>  misc_feature
<222>  (65)..(65)
<223>  n is a, c, g, t or u

<220>
<221>  misc_feature

<222>  (67)..(69)
<223>  n is a, c, g, t or u

<220>
<221>  misc_feature
<222>  (86)..(86)
<223>  n is a, c, g, t or u

<220>
<221>  misc_feature
<222>  (88)..(88)
<223>  n is a, c, g, t or u

<220>
<221>  misc_feature
<222>  (191)..(191)
<223>  n is a, c, g, t or u

<220>
<221>  misc_feature
<222>  (285)..(285)
<223>  n is a, c, g, t or u

<400>  50
tcatctacac gctggccagc aaggagatgc ggcgggcctt cttccgtctg gtctgcaact      60

gcntngnnng gggacggggg gcccgngncc tcacccatcc agcctgcgct cgacccaagc     120

agaagtaaat caagcagcag caacaatagc agccactctc cgaaggtcaa ggaagacctg     180

ccccacacag ncccctcatc ctgcatcatg gacaagaacg cagcacttca gaatgggatc     240

ttctgcaact gatcgtctcc atgcgccctg ctctgcggct gtgtncttat ttattgcatg     300

cgtcgcttcc acaggggccc ctcaagagct gtgactcggg agagctacct tactttgacc     360

aacagcctgc ccagtgtgga tgtctcttac aga                                  393


<210>  51
<211>  543
<212>  DNA
<213>  Homo sapiens

<400>  51
cctccttagc cttagaagcc agtggtgccc tgaccagagg ggaccctgtg ttcacaggca      60

tcctcaagga ttatcttcga gagttgccca ccccactcat cacccagccc ctgtataagg     120

tggtactgga ggccatggcc cgggaccccc caaacagagt tccccccacc actgagggca     180

cccgagggct cctcagctgc ctgccagatg tggaaagggc cacgctgacg cttctcctgg     240

accacctgcg cctcgtctcc tccttccatg cctacaaccg catgacccca cagaacttgg     300

ccgtgtgctt cgggcctgtg ctgctgccgg cacgccaggc gcccacaagg cctcgtgccc     360

gcagctccgg cccaggcctt gccagtgcag tggacttcaa gcaccacatc gaggtgctgc     420

actacctgct gcagtcttgg ccaggtgagt tcatgcccag ggcctgcacc accaatctga     480

gccaggctgc tacaatcccc gcctgccccg acaatctcca gatgtcgcgc cttacttgcg     540

acc                                                                   543


<210>  52
<211>  367

<212>   DNA
<213>   Homo sapiens

<400>   52
tcgcctgtac cagctggcat atgacaccta tcaggagttt aacccccaga cctccctctg      60

cttctcagag tctattccaa caccttccaa cagggtgaaa acgcagcaga aatctaacct     120

agagctgctc cgcatctccc tgctgctcat ccagtcatgg ctggagcccg tgcagctcct     180

caggagcgtc ttcgccaaca gcctggtgta tggcgcctcg gacagcaacg tctatcgcca     240

cctgaaggac ctagaggaag gcatccaaac gctgatgtgg aggctggaag atggcagccc     300

ccggactggg cagatcttca atcagtccta cagcaagttt gacacaaaat cgcacaacga     360

tgacgca                                                               367

<210>   53
<211>   470
<212>   DNA
<213>   Homo sapiens

<400>   53
cccccgagga caacctggag atcgttctgc acagatggga gaacaacagc tgtgttgaga      60

agaaggtcct tggagagaag actgggaatc caaagaagtt caagatcaac tatacggtgg     120

cgaacgaggc cacgctgctc gatactgact acgacaattt cctgtttctc tgcctacagg     180

acaccaccac ccccatccag agcatgatgt gccagtacct ggccagagtc ctggtggagg     240

acgatgagat catgcaggga ttcatcaggg ctttcaggcc cctgcccagg cacctatggt     300

acttgctgga cttgaaacag atggaagagc cgtgccgttt ctagctcacc tccgcctcca     360

ggaagaccag actcccaccc ttccacacct ccagagcagt gggacttcct cctgcccttt     420

caaagaataa ccacagctca gaagacgatg acgtggtcat ctgtgtcgcc                470

<210>   54
<211>   504
<212>   DNA
<213>   Homo sapiens

<400>   54
gtgtgtggat tcaacagtcg accccagctg tcgcagagcg cgaggaagct gcgcagtaaa      60

ccccttacat atcaacctct gaggaccggt ttttctgcac ctggtggtcc ttctagacgt     120

ctaggaggat cgtgttctca ggagagggtt cttcagcatc tgtgctgaag aacactgccc     180

cagcgggtca catgcaagat tccaccttcg agcaacatag ctgacactct gcagcccagt     240

tgtcacttgt aacaaacccc agtgggtcac atagtgaggg gaggcaaggc agcgtaaggc     300

agtggctgaa ctatcccaga aaacaaggat cacaggcccc cagtgacacc aatgttgcag     360

aaacacctgc agtggcaagt cagatgtcct ccaggaccag gcagataaca aggagtaggg     420

gtctgcagag gcctcgggag ggtctgcacc atccaaagaa atcaattgtt ctgcacagtg     480

gtaaggatcc agtgttccca gcac                                            504

<210>   55

<211>  382
<212>  DNA
<213>  Homo sapiens


<220>
<221>  misc_feature
<222>  (27)..(27)
<223>  n is a, c, g, t or u

<220>
<221>  misc_feature
<222>  (65)..(66)
<223>  n is a, c, g, t or u

<220>
<221>  misc_feature
<222>  (70)..(70)
<223>  n is a, c, g, t or u

<220>
<221>  misc_feature
<222>  (72)..(72)
<223>  n is a, c, g, t or u

<220>
<221>  misc_feature
<222>  (345)..(345)
<223>  n is a, c, g, t or u

<400>  55
gaacaccatt gtcttcaata acctgtnggg catatccagg aggcacatag ataggaggca      60

caganncatn tngggacatc attggaacct gagcaggacc tgtaatgcac tgaaactgtc     120

catcttctct tcttattgta aatgcttctc ctgggttaac ttgtaccaga ataacctgtt     180

gtgttccatc tgcacttaca ataggggcag acaaaagaga aatatcacta cttaagatct     240

gagttgtatc cagtagtggt ggatgttctg ccattatcaa taagacatta atatactgaa     300

taacgctcca attctccgag tcacgccgtt ctgaggcaga aggcngctcc tctggcgcct     360

cttcttaggg ttcctgatcg tt                                              382


<210>  56
<211>  440
<212>  DNA
<213>  Homo sapiens


<220>
<221>  misc_feature
<222>  (83)..(83)
<223>  n is a, c, g, t or u

<220>
<221>  misc_feature
<222>  (141)..(141)
<223>  n is a, c, g, t or u

<220>
<221>  misc_feature
<222>  (262)..(262)
<223>  n is a, c, g, t or u

<220>

<221>  misc_feature
<222>  (373)..(373)
<223>  n is a, c, g, t or u

<400>  56
gaagtgggag cggctcagca taggatgggc acgccatcag ccgtcaccag gcgcccggtg      60

gtggggtcgt aggtgcccgc cangtagtag aggtcctgcc ggtcctggca cttgcggctc     120

cgggccatct gctcatactg ntcgcgcgcc acgacctggc acttgtggga gatggtctgc     180

acgtcgttct catcctcgtg gcaggactgg tacagcgcat tcttgccgtc gcactgcctc     240

ttgcccagct tggtctcctc angggtggta gaaccacttg accttgacca ccatgttgct     300

gccccacgac tcccacatgc tctcgatgcg gccgatgtag gggaggttgg gccgcccagc     360

tgacaggaag acngcacagt ccccgacacg cagggtctcc tcgccccgca cgatggcctt     420

gtagaacagc ttccgggcct                                                 440

<210>  57
<211>  265
<212>  DNA
<213>  Homo sapiens

<400>  57
catcgcccac caaggcctgg gtgggtgaga acagtgccca caaggagacc ctgagtaaca      60

gagactcaca gcccatccag gtctctgggc aggaaattga aggaatcatc acattttaca     120

gaggaggaga ctgcagctca gagtggggga agtgtgtgca ccaggccaca ggcaagtctg     180

tccagagcac tggtaggaat gagggaaact aggaatgacc actttaaaaa gttagatgag     240

aagaatttca aggccgggcg cggtg                                           265

<210>  58
<211>  355
<212>  DNA
<213>  Homo sapiens


<220>
<221>  misc_feature
<222>  (229)..(229)
<223>  n is a, c, g, t or u

<400>  58
gctttatgca gtttgtcctt tcagttttca ggaatgagac ctcttgaccc ctcccctcca      60

atgcagcccc tactaagggg gagtttaagg agccatacat agttctataa ttcaaatcaa     120

gtaaacatgc ttcttgtccc aggttaactt gtgctgcctc agtcgctgtt taaacatttt     180

tatacgcact gttaacctgc ctgcccatta ccctattact tttaatggnt aaactactgt     240

tccctgggca gttgtctctt ttaacgtccc accctaaact tgccaaccct catatgaagg     300

cctcaggctt gttattggca aaggtcagaa gtcttaagct agtgaccttg caggc          355

<210>  59
<211>  443
<212>  DNA
<213>  Homo sapiens

<400>  59
ccctgacggc agaagagccc agcttcctgc agcccctgag gcgacaggct ttcctgagga      60

gtgtgagtat gccagccgag acagcccaca tctcttcacc ccaccatgag ctccggcggc     120

cggtgctgca acgccagacg tccatcacac agaccatccg cagggggacc gccgactggt     180

ttggagtgag caaggacagt gacagcaccc agaaatggca gcgcaagagc atccgtcact     240

gcagccagcg ctacgggaag ctgaagcccc aggtcctccg ggagctggac ctgcccagcc     300

aggacaacgt gtcgctgacc agcaccgaga cgccaccccc actctacgtg gggccatgcc      360

agctgggcat gcagaagatc atagaccccc tggcccgtgg ccgtgccttc cgtgtggcag     420

atgacactgc ggaaggcctg agt                                             443

<210>  60
<211>  552
<212>  DNA
<213>  Homo sapiens

<400>  60
gtctcgaggc agggctgaca catggtgcca tagccagcgg agggcgctca gtgagtgccc      60

cgggccttct agacaacagg caggaaggat gaacctcagg gcacccccag gtggtgcgga     120

aagccaggca gttgggacag aggtgcccac gagggcagag gccggtgcta aggggatggg     180

gaagaaggga caagattccc agagaggaga ggaggctgtt ggtaggaaag tggcagggct     240

gggggagacc cagccccaag ggtccggggc ggaggatgct ttgttctttt ctggttttgg     300

ttcctctttc gcggggggtg ggggaggtca acagggactg agtggggcag aggcccagaa      360

gtgccagcct ggggagccgt ttgggggcag ccccttctgc ccaccccatc cttcttcctc      420

tccagagatg ccaggggggc gtgtatgctc tgccccttcc ctcagacagg ggctgggtgg     480

ggaggctctt taggctcagg agaagcattt taaagaaacc cccaccctgc cgcccgcatt     540

ataaacacag ga                                                         552

<210>  61
<211>  361
<212>  DNA
<213>  Homo sapiens

<400>  61
ctctttatcc ctcagattac tccaaagcat aatgggctct atgcttgctc tgctcgtaac      60

tcagccactg gcgaggaaag ctccacatcc ttgacaatca gagtcattgc tcctccagga     120

ttaggaactt tttgctttca ataatccaag tagcagccct gatgtcattt ttgtatttca     180

ggaagactgg caggagattt atggaaaaga ctatgaaaag gactcttgaa tacaagttcc     240

tgataacttc aagatcatac cactggacta agaactttca aaattttgat gaacaggctg     300

ataccttcat gaaattcaag acaaagaaga aaagaactcc atttcattgg actaaataac     360

a                                                                     361

<210>  62

<211>  238
<212>  DNA
<213>  Homo sapiens


<220>
<221>  misc_feature
<222>  (29)..(29)
<223>  n is a, c, g, t or u

<220>
<221>  misc_feature
<222>  (33)..(33)
<223>  n is a, c, g, t or u

<220>
<221>  misc_feature
<222>  (38)..(38)
<223>  n is a, c, g, t or u

<220>
<221>  misc_feature
<222>  (69)..(69)
<223>  n is a, c, g, t or u

<220>
<221>  misc_feature
<222>  (77)..(77)
<223>  n is a, c, g, t or u

<220>
<221>  misc_feature
<222>  (91)..(91)
<223>  n is a, c, g, t or u

<400>  62
caaagtggga ggattacaag tgttatccna ccnatgcntg gacaggaata tttttaaata      60

atgaaaccna agttccnttt cgctttgtaa ngttaatgca tgtattgatg gtgagtagag     120

aacaatgaca caatctctag agagacatag gtgttcggcc tggctcaatc actagcctta     180

tagtctcaca ggaaaatatg aacttcatca aaatagctaa ttattaccac atcatgga       238


<210>  63
<211>  355
<212>  DNA
<213>  Homo sapiens

<400>  63
atgcagatga cgttgtggcc accgcactgg ccgtggagcc catgaagttt gtctacagag      60

gcaggatcgc tgtgttctct gtgaccgtgc tgcacgacga ccggattgtc ctggtggctg     120

agcagcggcc ggatgcctcg gaggaggaca gcttccagtg gatgagccgt gtgctgcagg     180

tgggcgcccc ggcacggcct atggttcggt gaatctccca agctggcacc cccactccac     240

tccaagtgcc aagtggttgg cttgtcccgc ccggtcctcc ctggctccag ctttgtttat     300

ctgtattttt cattgcaaat tgacaaatta cagctgtatg tatttacggg ataca          355


<210>  64
<211>  230
<212>  DNA
<213>  Homo sapiens

<400>  64
cctccctcaa agctactaaa catgaaaaca ttgtgcctat atgataaaaa tgtcaatatt      60

gctggtgata ctgatgctga tggaaatgac gatattagct gccattaacg tagtatctaa     120

tgtgtgccaa acaatattaa aaattgctgt atatacatgt ttgccattta ttatttataa     180

ccttaacaag atgtctcact cataagacta ctttccgcac tatgatacag                230


<210>  65
<211>  552
<212>  DNA
<213>  Homo sapiens

<400>  65
agtggcctta gacataactg ctgcccaagg agccacctgt gcccttttag gaacacaatg      60

ttgtacctta tccctgacaa tcagcagaac ataacagcag ccctgcaaag gggtcttcca     120

ggagattaag gtgactgaga gcctcactgt caaccccctg cagagatggt gagcatccct     180

aggttctggc gtacattggg ccctaatagt cataagtatc atagctgaga tcctagtagt     240

gagctgttgc tctctgtatt gttgttgtgg gttatggact cagggctccg ccatataggc     300

atgtgtccct gcctggagga cgccctcagc ctagggggtg tagtgtaagg gaaatggctg     360

tgctttagtc aggagtaggc tgaggcagcc ttctggtgca gcatgactca gtgggtttgg     420

agtgcaagca cacaaccttg ctcgttatgt aaccacacca catgaggccc attaggtaac     480

aactcacatg agctcgtgtt tggctcagag ccactattgt ctgtaaaagg tataccttgc     540

tgatgctgca ca                                                         552


<210>  66
<211>  508
<212>  DNA
<213>  Homo sapiens


<220>
<221>  misc_feature
<222>  (48)..(48)
<223>  n is a, c, g, t or u

<220>
<221>  misc_feature
<222>  (55)..(55)
<223>  n is a, c, g, t or u

<220>
<221>  misc_feature
<222>  (125)..(125)
<223>  n is a, c, g, t or u

<220>
<221>  misc_feature
<222>  (127)..(129)
<223>  n is a, c, g, t or u

<220>
<221>  misc_feature
<222>  (132)..(137)
<223>  n is a, c, g, t or u

<220>
<221> misc_feature
<222> (139)..(139)
<223> n is a, c, g, t or u

<220>
<221> misc_feature
<222> (378)..(378)
<223> n is a, c, g, t or u

<220>
<221> misc_feature
<222> (421)..(421)
<223> n is a, c, g, t or u

<220>
<221> misc_feature
<222> (474)..(474)
<223> n is a, c, g, t or u

<400> 66
gggtgactgg tctaagtgct caattacctg tggcaaagga atgcagtncc gtgtnatcca 60

atgcatgcat aagatcacag gaagacatgg aaatgaatgt ttttcctcag aaaaacctgc 120

agcanannng cnnnnnnanc ttcaaccctg caatgagaaa attaatgtaa ataccataac 180

atcacccaga ctggctgctc tgactttcaa gtgcctggga gatcagtggc cagtgtactg 240

ccgagtgata cgtgaaaaga acctatgtca ggacatgcgg tggtatcagc gctgctgtga 300

aacatgcagg gacttctatg cccaaaagct gcagcagaag agttgacctc tagcaggctg 360

gctggatcac agctcttngc aattacatta tttataaaca cacacactag catgtttttc 420

nagaccaaat attatcagat tacatataat ttaatcaaat taatttattt tttntgcctg 480

ccaaacatcc aatgtggtgc ttgttttg 508

<210> 67
<211> 410
<212> DNA
<213> Homo sapiens

<400> 67
gcatgtgtaa aaagtccttc agccacaaaa ccaacctgcg gtctcatgag agaatccaca 60

caggagaaaa gccttataca tgtccctttt gtaagacaag ctaccgccag tcatccacat 120

accaccgcca tatgaggact catgagaaaa ttaccctgcc aagtgttccc tccacaccag 180

aagcttccta agctgctggt ctgataatgt gtataaatat gtatgcaagt atgtatattc 240

ctatagtatt tatctactta ggatataaga tataatctcc tgattatgct ttcaatttat 300

tgtcttgctt cattaaaatg taaggctaag gagagcatgg aatttgtcag ttttgttcac 360

taaagtattc caagtggttg ggaaagtgga acatttccaa gaaccaataa 410

<210> 68
<211> 291
<212> DNA
<213> Homo sapiens

<400> 68

cacaggatgt ggtctctacc gtgattcctg agcatgcatg caccccttct cctgccaata     60

gaggggagga agtcggaggg gtgtctttat gcctataaac ttgccttgga atccagcctc    120

actccctttc ctcctggagt tgagaagccc ccacagagac tggctatggg ggagtgactg    180

tctataggtt ccttggatgt cctgcctatc tgcaaaatga gaatgagatc gataccttca    240

tgaggctgta agatggcaga tataaaagtg ctgtgttatc tcaaaagggt g             291

<210>  69
<211>  326
<212>  DNA
<213>  Homo sapiens

<220>
<221>  misc_feature
<222>  (47)..(47)
<223>  n is a, c, g, t or u

<220>
<221>  misc_feature
<222>  (58)..(58)
<223>  n is a, c, g, t or u

<220>
<221>  misc_feature
<222>  (60)..(60)
<223>  n is a, c, g, t or u

<220>
<221>  misc_feature
<222>  (65)..(65)
<223>  n is a, c, g, t or u

<220>
<221>  misc_feature
<222>  (218)..(219)
<223>  n is a, c, g, t or u

<400>  69
actgtgtgca gcatattgca ggctttcact catttaatat ctacaangtc ctcaatangn     60

atatnaatta cttatgattt ccctgttttt tcttcctata aggaagctga ggcacaagtt    120

aatcaaagtc tcttggccta gggtgacaca gctaagattt gtacctagag atttctgagt    180

gttgacttct ctcctgcccc cacctatctc cccccccnna aaaaaaaaca caacaacaac    240

aacaacagaa cataccaggg attcatggct tgcccaatgt tggaggggga gaagagagga    300

gagggatgag ataagctcct cccacc                                         326

<210>  70
<211>  352
<212>  DNA
<213>  Homo sapiens

<220>
<221>  misc_feature
<222>  (61)..(61)
<223>  n is a, c, g, t or u

<220>

<221>  misc_feature
<222>  (120)..(120)
<223>  n is a, c, g, t or u

<220>
<221>  misc_feature
<222>  (152)..(152)
<223>  n is a, c, g, t or u

<400>  70
ttctgttttc ttcttaaagt catttatatt atgtattact cttaaagaat gttttagtct      60

ncattttagt agtctgtgca taaggtagta atacatgtac acaaagaaaa attcacaagn     120

cccattcagg tgtcttttag aacattattt anccactaaa tatttataca gttgacataa     180

tgcttattat gcccttgaat aatagaattt gttttgtttt tacttcttat ccataagcat     240

tggccttaca ttgcctcaag aggaacagaa tttattatta aacaggattc ttaaatccat     300

aactcatatt gtgacttcat acattttgta accctagtag tgaatatacc ct             352


<210>  71
<211>  414
<212>  DNA
<213>  Homo sapiens

<400>  71
gcccaaatcg cgcaggtctg ggacctgatt gcgggccacg aggcgcaatt cggggcggag      60

ctgctgctca ggctcttcac ggtgtacccc agcaccaagg tctacttccc gcacctgagc     120

gcctgccagg acgcgacgca gctgctgagc cacgggcagc gcatgctggc ggctgtgggc     180

gcggcggtgc agcacgtgga caacctgcgc gccgcgctga gcccgctggc ggacctgcac     240

gcgctcgtgc tgcgcgtgga cccagccaac tttccgctgc taatccagtg tttccacgtc     300

gtgctggcct cccacctgca ggacgagttc accgtgcaaa tgcaagcggc gtgggacaag     360

ttcctgactg gtgtggccgt ggtgctgacc gaaaaatacc gctgagccct gtgc           414


<210>  72
<211>  533
<212>  DNA
<213>  Homo sapiens


<220>
<221>  misc_feature
<222>  (51)..(51)
<223>  n is a, c, g, t or u

<220>
<221>  misc_feature
<222>  (68)..(68)
<223>  n is a, c, g, t or u

<220>
<221>  misc_feature
<222>  (124)..(124)
<223>  n is a, c, g, t or u

<220>
<221>  misc_feature
<222>  (138)..(138)

<223>  n is a, c, g, t or u

<220>
<221>  misc_feature
<222>  (208)..(208)
<223>  n is a, c, g, t or u

<220>
<221>  misc_feature
<222>  (213)..(213)
<223>  n is a, c, g, t or u

<220>
<221>  misc_feature
<222>  (220)..(220)
<223>  n is a, c, g, t or u

<220>
<221>  misc_feature
<222>  (242)..(242)
<223>  n is a, c, g, t or u

<220>
<221>  misc_feature
<222>  (251)..(251)
<223>  n is a, c, g, t or u

<220>
<221>  misc_feature
<222>  (258)..(258)
<223>  n is a, c, g, t or u

<220>
<221>  misc_feature
<222>  (281)..(281)
<223>  n is a, c, g, t or u

<220>
<221>  misc_feature
<222>  (286)..(286)
<223>  n is a, c, g, t or u

<220>
<221>  misc_feature
<222>  (357)..(357)
<223>  n is a, c, g, t or u

<400>  72
tgccagctac aggtgctcac ctgaaaagca agccagacca tattaaccct nggcattgct      60

ggtacctngg aagactttct gattcaatgc tttccacctc ctcctacccc tcaccacccc     120

cgtnggcatg aaatcctngg gggctgcttt agaaattgtt ttctttggct gctggtgggg     180

gtgctgctgg tgggggtttg cacagctngg canactgcan ccagtctggt gggggtttgc     240

anagctggca nactgcancc agtctcctgc ctgctgccaa naaggnccat ttcccaagca     300

ctggctttgg agaagttggg gctctgaagt gggaacacaa ggctgccttt tgcaggncca     360

ggtgtaaatt ctccccctgc cactttcagc ctagcgtgaa acagatggag tgtgcattcc     420

cacttccctt tatggtaccc tggaatgatg gagctgccca gggcatcgcc acgttactct     480

ctagacagtc tctttgtctt cctgcaatgg cagcgccgag gttgtatatt tct            533

<210>  73

<211>  492
<212>  DNA
<213>  Homo sapiens


<220>
<221>  misc_feature
<222>  (226)..(226)
<223>  n is a, c, g, t or u

<220>
<221>  misc_feature
<222>  (234)..(235)
<223>  n is a, c, g, t or u

<220>
<221>  misc_feature
<222>  (253)..(253)
<223>  n is a, c, g, t or u

<220>
<221>  misc_feature
<222>  (272)..(272)
<223>  n is a, c, g, t or u

<400>  73
gaagggctgc cttattttag agcacagatt ttctgaatat ctattttgac aggttcgatc      60

ctctcccctt cctgccttcc ttctgtcgat tttcaatgtc ttgatggtgt cccacctgag     120

tggcctttag agatgtgagt tgtgaggcac tggggaggca ggcacacgtc ctccagccca     180

agactgccta atttaacagg gatttctgca ttctggaaca agcctnccat tttnncccca     240

agcaggatta ctnccagagg gcaaaacaca gncccaatag tatcacattt cctttctgct     300

ttagcaaaaa taaccactgt ctcattcatg ggaaaaggcc gccaaacaaa tttgttactg     360

gaaccatttg taacaacttc tagtttgcac tgccttggag caagcacact ttgtagagga     420

gggatttgca gttacttggg caacaaggta accactgatc attacaggaa gcttcagaaa     480

ccgtgggacc ag                                                         492


<210>  74
<211>  354
<212>  DNA
<213>  Homo sapiens


<220>
<221>  misc_feature
<222>  (90)..(90)
<223>  n is a, c, g, t or u

<220>
<221>  misc_feature
<222>  (108)..(108)
<223>  n is a, c, g, t or u

<220>
<221>  misc_feature
<222>  (261)..(261)
<223>  n is a, c, g, t or u

<400>  74
ctgttgctgc tgctgagcat gggcggggca tgggcatcca gggagccgct tcggccatgg      60

tgccacccca tcaatgccat cctggctgtn gagaaggagg gctgcccngt gtgcatcacc 120

gtcaacacca ccatctgtgc cggctactgc cccaccatga tgcgcgtgct gcaggcggtc 180

ctgccgcccc tgcctcaggt ggtgtgcacc taccgtgatg tgcgcttcga gtccatccgg 240

ctccctggct gcccgcgtgg ngtggacccc gtggtctcct tccctgtggc tctcagctgt 300

cgctgtggac cctgccgccg cagcacctct gactgtgggg gtcccaaaga ccac 354

<210> 75
<211> 275
<212> DNA
<213> Homo sapiens

<400> 75
agttccagaa atccagtgac gaatgtggta tacaaaaaaa tatataaatt ctttcaactt 60

agaataatta agtcataaaa tacatagggt acaaatacca cattccgttc taaaatgata 120

tcttaggatc atcaaaagaa aaagaggatt tggattatgc aaaaaatgat tcctatatat 180

ataatcaatt atctaactga catttttgca aatctaccac aacttcgcct tttattgcat 240

atgctaaaca agcagatgct aagtctgtaa actgt 275

<210> 76
<211> 62
<212> DNA
<213> Homo sapiens

<400> 76
ttgcttaatc atgcgctttg ttttttatgc attcacttcc tgtctttatc tctattttct 60

tt 62

<210> 77
<211> 471
<212> DNA
<213> Homo sapiens

<400> 77
ttaacctaag tatcagccct ggcatgctta tactggtcca agcaagcatt acgtcacagc 60

ctgttcctct tctttatcta aaagtgcttt ttcctttctc agcattccac aagttacttc 120

ctccttcctt tgttctcctc tgcctttgcc tcttttaaat agttccaagg tgctggccaa 180

tcgggacaaa tacagaatgt gaggtcccat tccagccctg gaaactggac acagcagtag 240

ggcggacgca tcaagtgata aatgaccctg tcccctttgt tcgctgtact ctcctggcaa 300

aactgctgga gagtgtaccc tttctgcaga aagtaaaaaa aaatggcctt gctgaggaaa 360

ttaatgttca agtgctattt ctttatggca ctggggaaca agcatttcaa acagacctga 420

ggtttacccg atttctgctg gaaaagaaac ctcaggtctg ctgccttaga a 471

<210> 78
<211> 373
<212> DNA
<213> Homo sapiens

<400>  78
tctgtaggag atcttccaaa ttactgctta tatacatgta tattctatta caaaaattac      60

accactcaat gtagtctaaa ttattgagag taaattgtag ccattctttt acatgttttc     120

tgaacttagt tgccaataat cataatcatt agcttttcaa ggtttgctct gaaacttaca     180

aaccatgcaa aagtgaaaac ttaggcttaa catatttggc aatttaaatc aactaaattg     240

aatcaatcta aatactgctt tgcaaagtaa aaaaggaatc aaaatgacac ataagacaat     300

cactaatccc tatattttta gggtctattt caagaaattt actactactt cttaccagcc     360

taaggactgt gta                                                        373


<210>  79
<211>  505
<212>  DNA
<213>  Homo sapiens


<220>
<221>  misc_feature
<222>  (334)..(334)
<223>  n is a, c, g, t or u

<220>
<221>  misc_feature
<222>  (471)..(471)
<223>  n is a, c, g, t or u

<400>  79
aggccaggtg ctatgctcag agttcacacc tgcctgatac tgtgaggatt gggctacaga      60

ttctaaacca cactctccat agaggacatg gcaggtgagc ggctggcttc tgtgggtctg     120

ggcctggtgg gttagtgtgg gctgcatggc cccaaggctg ggagctgtgt tgggatctgg     180

tggcaggggg tttatctgac aacctcacta ttccatgtct cctctctgtg tggaggaatg     240

ggatgcagcg aggaggccag gctggagttc tgtagagtgt aaaatcctgg atgtcctctc     300

agcctgtctc cttgagagga cctgctgcct gccnttctgg agcacgtcat tctcttcttg     360

gatgaccaaa taaatcattc aagaatgaaa tgaaaactcc ttatctcctt ataggatctg     420

agctcagtga tgagaagtgg aaggacaata attgaccaat cacacattta natgaataaa     480

ttaggccgtt ggtgttcagc agcaa                                           505


<210>  80
<211>  366
<212>  DNA
<213>  Homo sapiens

<400>  80
tgtttccttt ccacttgcta gaagttattt tgccaatcac atatgattat tttatcattt      60

tttaattacc atcagtgcat gaaattatct ttattattca cttgttttta ttataatctt     120

ataatttcaa ataaaatgta aatctactgt cccttgcttt acctccgtgt cttcagtgcc     180

tagaacagga ctgtcataca cagtgactca atacacattt acttatgggt gattccctgc     240

ctgactgtta caggaagaag gaccaggaat atcagaatct gaagtgtcct ctaaagtcat     300

aaagactaga aggcattgaa taatgtttct taactatgca aggacttcag aattagatct 360

cacata 366

<210> 81
<211> 455
<212> DNA
<213> Homo sapiens

<400> 81
agatctcatt ttctggaggt gcatgtctcc cgtgaccccc tctttggatt gcccgcagag 60

cccgtgaaga tggtgttatc actcctgtga ttactttact gatcaggtga ctttgagtca 120

atcaaaaggt agattatcca ggtgtgcctg atttgatcag gtggtccctt aaggaggctt 180

aaaatgaccc tttctgaagt agagtaattg gaaaagtaag agggtctatg ggtggggtca 240

cctggcaagg aactgaactc agcctccatg agctctggcc accagctgac ctttagcaag 300

aaagcaaatc tttctttggt cagtctccac aacaggacga agctggctga gcccttgcct 360

ttggccctgt gagatgctga cccgagtatc cagcgaacac gtgccagagt cctgacccat 420

ggaaactgag atgatgagtc tgtgttgctt taagc 455

<210> 82
<211> 119
<212> DNA
<213> Homo sapiens

<400> 82
ccgattttct gtttgaagca gttcccttct atgttgcagt ctccttgaag gcaaaggttg 60

tgcactgtca tgttttgaag cccagtatcg ctgagaacaa tgacagacac atgcagtgg 119

<210> 83
<211> 137
<212> DNA
<213> Homo sapiens

<400> 83
tggctctcag agaaaccgta tttgatcaga gagctaaagg aagtgaggtt gtgagccaca 60

gggttatctt gaagaagagc attccaagga caggggaaac ttcctcaaag accagtaagc 120

cagagtgttc ttggtgc 137

<210> 84
<211> 345
<212> DNA
<213> Homo sapiens

<400> 84
agcttacaca gcattcttag agaggacaca gaatttggag tttgagtctt gccaagttat 60

agggccttga gaaacattta gggctttcca tggatccacc ctaacgaagc ataaaattaa 120

gcctaggatt ttagggtcat cagccaaaaa tggaactgcc ttctagaaca aaaaatgaca 180

tccttttgag gaagacagtc atccagagtc tttacaatct tttacccaca ttgcctagta 240

cataattaaa catttctaga tatgaatagg aacaggaaaa tgtgacccat aatcaagaca 300

acaagcaata aatggaaacc tacccttaag tagctaaact gttgc 345

<210> 85
<211> 459
<212> DNA
<213> Homo sapiens

<400> 85
tatgtttatt cagggctctg gaacataaaa aggctttgcc acactcttca catctataag 60

gtttctctcc agtatgaatt ttcttatgtt tactcaggta tgcagaccat ccaaaggctt 120

tgccacactc ttcacatttg taaggtttct ctccagtatg aattatctta tgtttattca 180

ggtctgtgga ccatccaaag gctttgccac actcttcaca tttgtggggc ctctctccag 240

tatgaattct cttatgttca ttaagggttg tgaaccgact aaaggctttt ccacattctt 300

cacatgtgta gggtttctct ccagtatgaa tactcttatg tttattaagg gttgcggatt 360

gtctaaaggc tttgccacat tgttcacatt tgtagggctt ctctccagta tgaattctct 420

tatgttcatt cagaactgag gacctactaa aggctttgc 459

<210> 86
<211> 229
<212> DNA
<213> Homo sapiens

<220>
<221> misc_feature
<222> (78)..(78)
<223> n is a, c, g, t or u

<220>
<221> misc_feature
<222> (81)..(81)
<223> n is a, c, g, t or u

<400> 86
gggagtggac ctgcattagc aagcagagaa tgtccagagc ctagagacag ccagcccatg 60

cagagggtag ggcataancc naggcagtgg agagggtgag gagtggtgta tagaagagag 120

catggagttt aaggggttat tatggctgag atccagacca tgagcagaga aaagttcagt 180

ttatctcacg gaaaacttta atgttaggct taatcctctg ttccttcct 229

<210> 87
<211> 351
<212> DNA
<213> Homo sapiens

<220>
<221> misc_feature
<222> (80)..(80)
<223> n is a, c, g, t or u

<400> 87
ggttgatggt aatttatgta ccctgacagg ggtttggttt acatagctgt attcatttgt 60

caaaacttat attaaatggn tgcaattaat attgatgcat ttcattgtat gtaaatttta 120

ccctaaaata attttagaca aattgtaaaa cctagttaaa gacatatatg ctgatatttt 180

cagggttacc tctcttgatg tctgcaactt actttgaaat gcttcaaaag gaaaatagga 240

taatggatgg aaatagggag agagaaatgg atcgatgtgt aaataaaaca aatctatcta 300

aatgttaaag cttaattgta gatgatgaat gtaggagtgt tgaatgttaa a 351

<210> 88
<211> 482
<212> DNA
<213> Homo sapiens

<400> 88
aagagtctat gaagaaccca acggaagttt gtggcacatc cctaccctca aattcacagt 60

gagggtggaa tgacagtaac caaatctgtg aaaatattca catgagacag gaaagaagtc 120

agaatatcca gtgtacaatg agagtgaaag aggatgtcta aaaggggaca gcccattcac 180

aacccacaca caacccacgc acaaatattt ttgggggggc ctcccatggg catttataat 240

cttctaagtg ctccgaagaa catgtgtcac aaaagatgaa gagaatattt tccagaacat 300

agcccaacaa agaacttctt tgacattttt tagtgtaaag gtaactgacg gtatctacca 360

aattagcaat ttgtaaaact ggaatttcta aaagcaaata cttggagctg agattacctc 420

ccacttccca aattcgagtt atatgatctc aagtataata ccctttggta tagacctagc 480

ca 482

<210> 89
<211> 37
<212> DNA
<213> Homo sapiens

<400> 89
tctgagtggg cctgctctct gtagactgaa ttcagca 37

<210> 90
<211> 394
<212> DNA
<213> Homo sapiens

<220>
<221> misc_feature
<222> (130)..(130)
<223> n is a, c, g, t or u

<220>
<221> misc_feature
<222> (135)..(135)
<223> n is a, c, g, t or u

<400> 90
tgcactcaat ggcttctgtt cgaagtccct attaaatgtt tctttcttaa atactgtatt 60

tgtcagcttc ttccttcagc atcccaactt cctcagactt tggggtactt ttgcacagac 120

ctagccaccn caaancactg tcatagatgc agcaatccac tttcacaaaa ccccatggac 180

aatgcagagg gggagaacag ggactgatta aagaaaggga cagaaatggc atcactatcc 240

aagactgaaa aacaggctga atggattatc actctgaccc aactgcacat ttctaatgtc 300

ttcatgtttt caattactcc atgaattccc ttatctgatg ctgattatgc acaggactgt 360

gtaagagtta aacaacacct gacactggtg actc 394

<210> 91
<211> 300
<212> DNA
<213> Homo sapiens

<220>
<221> misc_feature
<222> (175)..(175)
<223> n is a, c, g, t or u

<400> 91
cctcatcact atgtcaccaa agtgttttgg aacttggtat tccagagact tctggaacgc 60

cgtgcaaggc ctgcccccag caagccacaa ccaggaaggt gcaggcacgc ccccactagc 120

tcctccccta tttattgcct cctggaaaac ccaggaccct cttccccatc tccancccct 180

acccctgggg gcagcccagg gagagccagg cacaatgagg gctcccaaca gctgcaagga 240

tttatctgaa cctttgagaa agaggaggag ccatctaagt ttctggaaac ctgagcccca 300

<210> 92
<211> 490
<212> DNA
<213> Homo sapiens

<220>
<221> misc_feature
<222> (49)..(49)
<223> n is a, c, g, t or u

<220>
<221> misc_feature
<222> (69)..(69)
<223> n is a, c, g, t or u

<400> 92
cccattgcga tctggctctg ggggaccctg ggatgatatc cctaccccna gggacaggac 60

ccaaccccng gggacctgga gagactctgt gccctgcagg accgatgggg gactcctccc 120

tgtatgtacg tgtgcgtggc cctgccttgt tcttgccccg gacctggcct ggtgaaggag 180

gcacgaggaa gattgcagtc agggacgctc agcctgggag ctgaccctca ggtgaggccc 240

taaggaagtt cccagacctc cctgaacctc agtatgctca tctgtccagc agcaaccctg 300

ggccttaagt gagaacatct atgcggaaga ggcaggtgcc aatcaagccc tctgtaaagt 360

tacctcccct tttcccttct tctcctctca cagagctgaa gaatattttg caaagttcat 420

tgtaaacatt aaaataatct tgggtgttta tcattcgtta aacctgttgg gctgacttta 480

ggtctaccgc 490

<210> 93
<211> 317

<212>  DNA
<213>  Homo sapiens

<400>  93
gagaaaagta gactccccaa tgcctcgcag taaatgagga cgcctggcgc ctgcggcgag      60

gtcaactgag gtcagacgag cttatctctc ctgtcccggg aattaagggc atcctgggga     120

cagctgcaga gcaggaggct ccccgtgccc tcctcttcct aagcaagtca ggatcccaag     180

aggcgcgtgc ggggaggccc ctccgaaggg ctgctggctt gtgtcttcca ccagcgcaaa     240

gggaagctat cggttgcttc tgcagtgagg caagctcagc cggacgccca gaagagagac     300

gaggtgtcgc tgtcggg                                                    317

<210>  94
<211>  208
<212>  DNA
<213>  Homo sapiens

<220>
<221>  misc_feature
<222>  (37)..(37)
<223>  n is a, c, g, t or u

<400>  94
atagatattt tttagtccac ttggctggat aataaantct taataacagg gggaaaaaaa      60

gaaagaaaaa ggaggaaaag atttaggaaa gaaaacaaca actttagtat ggaatgtgaa     120

gaactggcag gatattcacg ttgagctgtg cagtaagtag cttactggac atgtgaggct     180

gaagatacag ttgttcatat ggaagcaa                                        208

<210>  95
<211>  361
<212>  DNA
<213>  Homo sapiens

<400>  95
tccctcgtgt atcttatctt tattattgaa ttttccctca caatccactg ttaaaagaag      60

aaagtatcac acacgtgggt tcttttggct atggaagtgt ccttgagatc actttttgca     120

cgtgactcag ctgaagtgtt caaagcacat ggaaatcact tgccagtgac aggtggacgt     180

tgtatgtgtt ttctctctcc taaggatgcc taaactttct tttcttcaca ggtaaagtca     240

gtgataaatc ttttgtttgc tgcatatact ggagatgtgt ctgcacttcg aaggtatgtt     300

tacaggatgg attagcatgc actttacaga tatttatgaa gttgcttctg ggcgagcagc     360

c                                                                     361

<210>  96
<211>  377
<212>  DNA
<213>  Homo sapiens

<220>
<221>  misc_feature
<222>  (32)..(32)

<223>  n is a, c, g, t or u

<220>
<221>  misc_feature
<222>  (49)..(49)
<223>  n is a, c, g, t or u

<220>
<221>  misc_feature
<222>  (170)..(170)
<223>  n is a, c, g, t or u

<220>
<221>  misc_feature
<222>  (270)..(270)
<223>  n is a, c, g, t or u

<400>  96
gaaacgccat ggaatgtatt gtatttctct antctatccc ttaaaatgnc cattgataat      60

tattggcaat ggttattgat agtctcaacg taatttcagt agaatttgtt ttgagatttt     120

ttttatgcac ataaaagatt tctttaggga ttattgtaca gagttctagn aaaatatata     180

attttttttt ctgggcttat aactttcttt tctaaaaatt tatttggcag cctgattaga     240

aatgtggtaa aatctgaaca ataaaatagn aaatagacta gttgcataga atgtttcaaa     300

aacaggcatt agattggcgg ctactcggga ggctgaggcg ggagaatcgc ttgagcctga     360

gaggtggagg ttgcggt                                                    377

<210>  97
<211>  525
<212>  DNA
<213>  Homo sapiens


<220>
<221>  misc_feature
<222>  (72)..(72)
<223>  n is a, c, g, t or u

<220>
<221>  misc_feature
<222>  (203)..(203)
<223>  n is a, c, g, t or u

<220>
<221>  misc_feature
<222>  (207)..(207)
<223>  n is a, c, g, t or u

<400>  97
cacctttctg ttctgtgacg ggctgtccct gcttgtcctg ctttaggagg taggtaccca      60

gtggctcccc gncccctcag cggctcattc ctctcgctct ccccacgttg gtctgtgtga     120

gctccgctgt gtggctgcca ttcatccgat ccatctgtgg acttgctggg gctgcgccgt     180

gcacggtgtg gtgaatgcta canccanccc caggggcggg gctgagagtg gctgggacct     240

ggagcacatg gggatgctgt gtgggaacca acttgccccc caccctgtgt ctctaggggt     300

ccgcagcagt agagaagcag acagccagcc ctgtccctgc ggcgtcaccc tccaccccat     360

actaacccag cagcgcatgg agagatttcg ggagtgctct aaaggccttt ggagcaattt     420

agggcaatta cgggcagttt tagaaatgct gaggggttgt tttgcctgcg gggcggggat 480

ggttgcctta tgcccacagt gaagcgggcg agatgcggta gctgg 525

<210> 98
<211> 434
<212> DNA
<213> Homo sapiens

<220>
<221> misc_feature
<222> (30)..(30)
<223> n is a, c, g, t or u

<400> 98
gaagttcaac tcaggaaggt gcaatataan caaatgtgct atattataat gaggaatggt 60

actaccgttc cagattttct gtaattgctt ctgcaaagta ataggcttct tgtccctttt 120

ttttctggca tgttatggaa tgatcattgt aaatcaggac catttatcaa gcagtacacc 180

aactcataag atcaaatttc attgaatggt ttgaggttgt agctctataa atagtagttt 240

ttaacatgcc tgtagtattg ctaactgcaa aaacatactc tttgtacaag aagtgcttct 300

aagaatttca ttgacattaa tgacactgta tacaataaat gtgtagtttc ttaatcgcac 360

tacctatgca acactgtgta ttaggtttat catcctcatg tatttttatg tgacctgtat 420

gtatattcta atct 434

<210> 99
<211> 412
<212> DNA
<213> Homo sapiens

<220>
<221> misc_feature
<222> (47)..(47)
<223> n is a, c, g, t or u

<220>
<221> misc_feature
<222> (202)..(202)
<223> n is a, c, g, t or u

<220>
<221> misc_feature
<222> (386)..(386)
<223> n is a, c, g, t or u

<400> 99
gggagacaga tcacaatcag atccataagg aaaagtgtgt ctgtgtntat cttcctctct 60

agggaaaaat acagcagggt gaggggattg agtgggagtg caatcaggga agacttcctg 120

aaggcagtga ctggtgactg gaatgaagca tgagaatgag ccatgcaggt tgcccagaga 180

gagcatccag gcagagggag cngaaagttc catcctcacc cagctctgcc ggcccaggta 240

ctttctcctc tgccttctac tcccagtctc actccagtgc aacacacttc agttttctgg 300

gaactcctga tggaaagtgg ctgtatttgt tcatccctat agccttgggg cacagccagc 360

agcccctgga ggaagccccg caggtnggta aagagacaca gggctcccag cc 412

<210> 100
<211> 493
<212> DNA
<213> Homo sapiens

<400> 100
actgttttca gacctaacct tggcaaggtc agtcctactt tgatgttctt gtttcatcac 60

acttcttggc atttgtagat ttggaagaat tgggcctttg gtacctctga tctcttcgtt 120

tagcaactta ctgtgcaccc atatgcttag cttttgctgt tttagctttt tttttttttt 180

tttttaacct gccacctagt ggccgaaatg ttgctatact attgataagg tactcctaat 240

tttggcaaaa tagtaagagg caaagcacca aagattatgt tctctccctt ctccaaatct 300

ctcttggtga gaatgatctt taaaacatac cactcagatt attagcaatc ttggtatgga 360

acgtttttaa aaataataat aatgtacttt atgtggtgat ttatgttatt atttaggccc 420

aaagttttga tttaattgtt tccttttagc ttatttttga gatatgcagt ctgttaggaa 480

gctgtctctg tct 493

<210> 101
<211> 415
<212> DNA
<213> Homo sapiens

<400> 101
gccctcttgt gtagttttca ttgtgtctag tgcaatgccg taaaccttaa caccatgaga 60

cccatatgaa gtgccaacag tgatgatgga agcgctttca aagaaagaag tcatagacat 120

tataagaata aagcgacttg cttgatatgt acagtagata ggtacagctg tagctgctgg 180

ccatttcaga cagatgcttc atcttgtaaa cagcaacata aatgtatggt accaataaat 240

acagtacagt actgtaaatg tgttttctct tccttatgat tttcttggta catgttcttt 300

tctctagttt actttattgt taagaatata ctatataata cacatacaaa atatgtgtta 360

ttgcctgttt atgttgtggg tagggcttct ggtcaacagt gggctacatt atcga 415

<210> 102
<211> 530
<212> DNA
<213> Homo sapiens

<400> 102
ggactagcag tcttcttctt cagacgccat gggaccccca ggcgactgct ctactgccag 60

cgttccctgc tggacaaggt ctgacgccca ccgccggccc gcccactcct accacaagga 120

ctttgcctct gaagaccagt gtcagcaagg tggtggtggg tgggctgctc ccatccgtcc 180

ggagccccct ccccgcagcc tccttgcttc tctcagtccc ctggctggcc tccttcaccc 240

tcaccgcctg tagcttgtgt ctgtccagcc ccatctgaat gtgttggggg ctctgcactt 300

gaaggcagga ccctcagacc tcgctggtaa aggtcaaatg gggtcatctg ctccttttcc 360

atcccctgac ataccttaac ctctgaactc tgacctcagg aggctctggg cactccagcc     420

ctgaaagccc caagtgtacc cagttggcag cctcccgtca ctctgactaa aaagaatctt     480

cagagtgcat atttggaggt ggaaagattg ttcagttacc ctaaagactt                530


<210>  103
<211>  509
<212>  DNA
<213>  Homo sapiens


<220>
<221>  misc_feature
<222>  (47)..(48)
<223>  n is a, c, g, t or u

<220>
<221>  misc_feature
<222>  (50)..(53)
<223>  n is a, c, g, t or u

<220>
<221>  misc_feature
<222>  (56)..(59)
<223>  n is a, c, g, t or u

<220>
<221>  misc_feature
<222>  (85)..(85)
<223>  n is a, c, g, t or u

<220>
<221>  misc_feature
<222>  (112)..(112)
<223>  n is a, c, g, t or u

<220>
<221>  misc_feature
<222>  (117)..(117)
<223>  n is a, c, g, t or u

<220>
<221>  misc_feature
<222>  (121)..(122)
<223>  n is a, c, g, t or u

<220>
<221>  misc_feature
<222>  (125)..(125)
<223>  n is a, c, g, t or u

<220>
<221>  misc_feature
<222>  (179)..(179)
<223>  n is a, c, g, t or u

<220>
<221>  misc_feature
<222>  (266)..(266)
<223>  n is a, c, g, t or u

<220>
<221>  misc_feature
<222>  (272)..(272)
<223>  n is a, c, g, t or u

<220>
<221>  misc_feature
<222>  (277)..(277)
<223>  n is a, c, g, t or u

<220>
<221>  misc_feature
<222>  (285)..(285)
<223>  n is a, c, g, t or u

<220>
<221>  misc_feature
<222>  (359)..(359)
<223>  n is a, c, g, t or u

<220>
<221>  misc_feature
<222>  (467)..(467)
<223>  n is a, c, g, t or u

<400>  103
taattttagc tccaatccat ctttctcttc tccaaaaccc tacctcnntn nnntcnnnnc      60

caccccttaa gtacttagtc atgcntagcc ttatattctt gtttgaattc tnatgtnctg     120

nnccncccaa acagattata catttcttgg gtcccatact ttgcatttac catagcagnt     180

ttcatagccc atacaaacat taggccttca aaatatttgt caagtatttc ttcaataaaa     240

atgaaaacat cccaaatctt gatccnccta anatgtnaaa tgggnactta gttaagcaaa     300

ctaacatcat gatatactgg aaacaggtat ctctttcctt tacccttgtg cctgctgang     360

atcttattct cagccttgct gttttaaact caggggtgtg tgtacaacat atttaagcaa     420

attctggaat accaaagcca agcagtcttc caggggcttc atcctgncac acagcagctt     480

acctggtggg tgttgggtag cacacagta                                       509

<210>  104
<211>  338
<212>  DNA
<213>  Homo sapiens

<400>  104
catgatcagt gtattttagg gggactaata tggcaactaa agctactttg gaagagaaag      60

agtggagata catagattgc tattatagtt caggccaata gagaggaatt gggtttaaga     120

gatacattat ggaggcagaa gtgttcattc aacaagcgtt tgttaaatat ctactatgta     180

atcatgatta tacaactaga gagaatatga aaaaaatgaa ttacgtatgt tagcttatag     240

atggatgctc tcagtaccca tccctattaa tcgtcatttc cctttgttta gtgaaccttc     300

tgatatattg gatatcaaat atcctttcca agtattgt                             338

<210>  105
<211>  279
<212>  DNA
<213>  Homo sapiens

<220>
<221>  misc_feature
<222>  (26)..(26)

<223>  n is a, c, g, t or u

<220>
<221>  misc_feature
<222>  (34)..(35)
<223>  n is a, c, g, t or u

<220>
<221>  misc_feature
<222>  (41)..(41)
<223>  n is a, c, g, t or u

<220>
<221>  misc_feature
<222>  (238)..(238)
<223>  n is a, c, g, t or u

<220>
<221>  misc_feature
<222>  (241)..(241)
<223>  n is a, c, g, t or u

<400>  105
gttccaggtc ccggatagcg agggcngccg cgcnngctcc nagggccatg aagcccccag      60

gaggagaatc gagcaatctt tttggaagtc cagaagaagc tactccttcc agcaggccta     120

ataggatggc atctaatatt tttggaccaa cagaagaacc tcagaacata cccaagagga     180

caaatccccc aggatcatgt tttcttatgt gaaggagaag aaccaaaatc ggatcttnaa     240

ngcttgcaag gagcatcccg gctgggagca gagccaggg                            279


<210>  106
<211>  395
<212>  DNA
<213>  Homo sapiens

<400>  106
ccaggctact gctaagactc gtacttccca gtttggtgtg ggcagctttc agactccatc      60

ctccttcagc tccatgtccc tccctggtgc cccaactgca tcgcctggtg ctgctgccta     120

ccctagtctc accaatcgtg gatctaactt tgctcctgag actggacaga ctgcaggaca     180

attccagaca cggacagcag agggtgtggg tgtctggcca cagtggcagg gccagcagcc     240

tcatcatcgt tcaagttcta gtgagcaaca tgttcaacaa ccgccagcac agcaacctgg     300

ccagcctgag gtcttccagg agatgctgtc catgctggga gatcagagca acagctacaa     360

caatgaagaa ttccctgatc taactatgtt tcccc                                395


<210>  107
<211>  412
<212>  DNA
<213>  Homo sapiens

<400>  107
acatagagag gtgactcatt ctttttaaag gttacattaa gtttgtagta tgtcagaatg      60

gcaatactat aattgtttta accagtgacg tttaagttgt ttccagattt tttgatctaa     120

caaataatgt gtcatgagta tagaattttt atgttcatgt actagtatag ttataggatg     180

actcatattt gaagcaaagt acaaaacgca tgctttctgt agctactcat aaattctggt     240

atgagcaaaa tgtcaagatg cttgcttatc accgaccaag tgatgattaa gctcttgcta 300

aactgtatca aaggagaaaa agggaaatac aggcttatcc taacaatttc acagtgaaca 360

gtaatctctg gcattcagtt aaagctagac ttgttctaat tactttgatt tt 412

<210> 108
<211> 531
<212> DNA
<213> Homo sapiens

<220>
<221> misc_feature
<222> (121)..(121)
<223> n is a, c, g, t or u

<220>
<221> misc_feature
<222> (248)..(248)
<223> n is a, c, g, t or u

<400> 108
gtaagtggta ccagccacaa ctgaatatcc atctgggata aaataaaatt gcactcgtct 60

tagagatcca aatcaacttc agatggatta aaactttgaa tgtaaaaaac ataaatgact 120

nacagtcctg caaaatatct tggagacaac ctgtgccatc tggagagtgg gaagagcaca 180

tgcaaaggcc aaggggtgga gcagcccagc atgttctgga aaaggtaggg ctccccaagg 240

ctgggatnat ggtggagacc tgggtgtgtg ggagcacagg ggtgggggcc cgtgggccag 300

gaatgcacag agaggggctg gtgctctgcc gcaggcccaa gcccccaaag cccggtcatt 360

cccagcacca tcttcacggg tttctgccca ggtctttctg ctgcatctct tcctcccccg 420

attccttaat catttttttt aaaatcagtt catgtctttg taaaccaaat tatttctaaa 480

aggcaaattt atattactgc cgaaatcaag ggtcagtgag ctagttgtgt a 531

<210> 109
<211> 541
<212> DNA
<213> Homo sapiens

<220>
<221> misc_feature
<222> (53)..(53)
<223> n is a, c, g, t or u

<220>
<221> misc_feature
<222> (98)..(98)
<223> n is a, c, g, t or u

<400> 109
gacttgggat tccggagcag tcgcccctat cgctgctcct gcagttgcgg acnccaccga 60

ccccgccgcc ggaggactgg gcactgaaag gcctctangc ctaggcgcgg cccgcggagc 120

cagacgtgtt gctgccgtga gtaaaacgag cgccctctcc gcactcgttt acaaattaaa 180

atggaggaaa tttcgttggc caacctggat actaacaagc tagaggccat cgctcaggag 240

atttacgtag acctgataga ggattcttgt ttgggattct gctttgaggt gcaccgggca 300

gtcaagtgtg gctacttcta cctggagttc gcagagactg gtagcgtgaa ggattttggc 360

attcagccag tggaagacaa aggagcgtgc cgcctcccgc tttgctccct tcccggagaa 420

cctgggaatg ggcctgatca gcagctccag cgctcacctc cggaattcca gtagctgcaa 480

aatgagagtc tgaaagtggc caggacaata acatagactg gtcctgtggc ttcgaggagt 540

a 541

<210> 110
<211> 359
<212> DNA
<213> Homo sapiens

<400> 110
ctccctgcaa atgcacatgt caatcaatga ttaatgcacc caggttatgt acaaggcact 60

gggcttagca ccacagggaa cttccttcca gaggctcgct ttctagttgt gtagacaaga 120

atacatgcat gagaagatac aagacaattc acccatgcca aatgattcat acaggctgtt 180

taagtactgc agaaaataaa agaaggaaag gctaccagac ttttcaataa ggtctacagc 240

ttcccaagag catgtctttg ttaaatcagg aaatataaaa attatgtgtg tatgtgtatg 300

tatatatata taccacccta ttaactattt taaaatcgta ttctattttg ggggttgtg 359

<210> 111
<211> 491
<212> DNA
<213> Homo sapiens

<220>
<221> misc_feature
<222> (56)..(56)
<223> n is a, c, g, t or u

<220>
<221> misc_feature
<222> (151)..(151)
<223> n is a, c, g, t or u

<400> 111
cagagtggac tgttccctga ggtgggagat gtggaaaagc caagaggctg cagccnaggc 60

cactggcccc tgagatctct gcaggaaatg gctgtggagt gtggcagttt ggcaaactct 120

ccaccacacg taatgaaact tggatttgct ncagtgtctg gctgcagagc agtgggcctg 180

gccagcaggt ccccagcttt ggctatgagg gccttgagtc cccccaaaca ccgggttcca 240

gcaccacact cagccctcat tggctcttga actgagcttg gaagcttctg gtgaccttcc 300

aagagcctga gagtgaggtg gaattatttt aaaagataaa tattatatta tatatatata 360

tatttccctg aaggaaccaa agcgaatttt aaaagatgca atgtagaggg gaaaagagat 420

gatgaaaata tttaaaggcc ctatctgttt acagtgttcc gtggttaaac tcgctcactg 480

ctaagaatat t 491

<210> 112
<211> 287
<212> DNA
<213> Homo sapiens

<400> 112
gtgatcatga gaatgctgcc tttaaagatg tggccctggt cctgactgtt ctgctagagg 60

aggaaacatt agaagcaagt gtaggcccaa gggaaacgga agaaaaagtg agagacttac 120

tctgggccaa gtttaccaac tctgacactc ccacctcctt caaccacatg gactcagaca 180

aattgagtgg gctgtggagc cgaatttcac acctggtact gccagtccag ccaatcttag 240

atgctagcgt tacatccaca aaaccagtgt tgccttgtat aactatt 287

<210> 113
<211> 389
<212> DNA
<213> Homo sapiens

<400> 113
tagccgatcg ttacctcaag ggagtgggaa ttgggcccag ctccggcccc tcctggtcac 60

ctttggccat gatggccggg gccatgcctt gacccgacgc cggagggcca agcgtagccc 120

taagcatcac tcacagcggg ccaggaagaa gaataagaac tgccggcgcc actcgctcta 180

tgtggacttc agcgatgtgg gctggaatga ctggattgtg gccccaccag gctaccaggc 240

cttctactgc catggggact gcccctttcc actggctgac cacctcaact caaccaacca 300

tgccattgtg cagaccctgg tcaattctgt caattccagt atccccaaag cctgttgtgt 360

gcccactgaa ctgagtgcca tctccatgc 389

<210> 114
<211> 499
<212> DNA
<213> Homo sapiens

<400> 114
gtacctcgct ggacctggag ttagacctgc aggcgacaag aacctggcac agccaactga 60

cccaggagat ctcggtgctg aaggagctca aggagcagct ggaacaagcc aagagccacg 120

gggagaagga gctgccacag tggttgcgtg aggacgagcg tttccgcctg ctgctgagga 180

tgctggagaa gcggatggac cgagcggagc acaagggtga gcttcagaca gacaagatga 240

tgagggcagc tgccaaggat gtgcacaggc tccgaggcca gagctgtaag gaacccccag 300

aagttcagtc tttcagggag aagatggcat ttttcacccg gcctcggatg aatatcccag 360

ctctctctgc agatgacgtc taatcgccag aaaagtattt cctttgttcc actgaccagg 420

ctgtgaacat tgactgtggc taaagttatt tatgtggtgt tatatgaagg tactgagtca 480

caagtcctct agtgctctt 499

<210> 115
<211> 504
<212> DNA
<213> Homo sapiens

<400>  115
gagtttcagg accaggcagc ttgattacag catcaagggc ccctgtgttc tctgttttct      60

gcagccatag tattggcttc ttcccaagac ttatttttcc catcagtgtc acctgtgcta     120

caagctcctt cagtcacatc tatttttgat atttgtgggt acctaggagg tgcatatatt     180

tgtgggatac atgagatact ctgacacaga tgtgcagtgt gcacggatca cagggaaatg     240

gggcagccat ccatcccttc aagcattcat gatttctttg tgttgtgaac attcccgttg     300

tgctctctta gttattctga atgtacaaga aattattgct gactatagtc accctgtcgt     360

gctatcaaat actagacctc attcgtggta tctaactata ttttgtaccc attaaccatc     420

cccatctccc accccctacc tttcccacta tccatcccag cctctggtaa ccatccttcg     480

tctatctcca cgagttcaat tgaa                                            504

<210>  116
<211>  476
<212>  DNA
<213>  Homo sapiens

<220>
<221>  misc_feature
<222>  (423)..(423)
<223>  n is a, c, g, t or u

<400>  116
agcacagtct ggctggatga gacagggtcg tgcccagatg atggagaaat cgacccagaa      60

gcctgaggag gtgtcctggg tttggctggc tggctcctgc tccagcggcc cggcttcagg     120

tgtccggggg cgtggctgcc tggagcaggt gtgctgaata ccctggatgg gaactgagcg     180

aacccgggcc tccgctcaga gagacgtggc aggaccagcg aggaatccag cctgtccact     240

tccagaacag tgtttcccag gccccgctga gtggaccgga cctctgacac ctccaggttc     300

ttgctgactc cggcctggtg aaagggagcg ccatggtcct ggctgttggg gtcccaggga     360

gaggctctct tctggacaaa cacaccctcc cagcccccag ggctgtgcaa acacatgccc     420

ctnccataag caccaacaag aacttcttgc aggtggagtg gctgtttttt ataagt         476

<210>  117
<211>  494
<212>  DNA
<213>  Homo sapiens

<400>  117
atccttgtac ctgatgtctg agccactcag aactcaccaa aatgttcaac accataacaa      60

cagctgctca aactgtaaac aaggaaaaca agttgatgac ttcacactgt ggacagtttt     120

tcccaagatg tcagaataag actccccatc atgatgaggc tctcacccct cttagctgtc     180

cttgcttgtg cctgcctctt tcacttggca ggataatgca gtcattagaa tttcacatgt     240

agtataggag cttctgaggg taacaacaga gtgtcagata tgtcatctca acctcaaact     300

tttacataac atctcaggag gaaatgtggc tctctccatc ttgcatacag ggctcccaat     360

agaaatgaac acagagatat tgcctgtgtg tttgcagaga agatggtttc tataaagagt 420

aggaaagctg aaattatagt agagtcccct ttaaatgcac attgtgtgga tggctctcac 480

catttcctaa gaga 494

<210> 118
<211> 553
<212> DNA
<213> Homo sapiens

<220>
<221> misc_feature
<222> (191)..(191)
<223> n is a, c, g, t or u

<220>
<221> misc_feature
<222> (235)..(235)
<223> n is a, c, g, t or u

<400> 118
gataacccca atctacgaag actagctatg gaacttccta cactgagaca actccagtgg 60

aactctgata attatcctaa aataaggagg cttcttcagt agccctcgaa atatgttcaa 120

atacatgatt acatttatgt ccttaatatt gctattagtt tctgatgtta atgtaaaagt 180

tggggaaaaa ngtggaaaag ttaaagcagt gcaggttaat tcaatgccag agtancttct 240

cagagggtgt atattcagtg tgaacaattt tcaacagaga aatgtcaact tctggccaca 300

acggcaacca gtaaaatgac tatttttact gtcttatcta ttaatgaaga ggagattgca 360

taatatagat gaaggagcat agtatttgca ggtggaacgc ctagcagggc ttgagtctca 420

actctgctgc ttttactcta attgaccgag acaagtcatt taaactaata gagcttcaat 480

tttctcatat ctaatgtaac ataacaattc acagcctttt actttgtagt tatcgtgaag 540

atctaatcgc agt 553

<210> 119
<211> 462
<212> DNA
<213> Homo sapiens

<400> 119
ctcctgttca tcctgttcac agagtggctc ggctgatggt agctcgacca atggctgcaa 60

ccatgagagg gctcccctga aacttctctg tgacaatatg aagtaccaga tcctctccag 120

agccttctat ggatggctgg cctactgcag acacctgtcc accgtgagaa cccacctatc 180

agccctggtc aatcacatga tcgtgtctcc agacttgccc tgcgatgctg gacagggact 240

gacagccagg atctgggagc agtaccttca cgacagcaca agttacgagg agcaggagct 300

gctgcgcctc atctactacg gggcatccca gcctgagatc cgcaaggccg tgtggccctt 360

cctcctgggc cactaccagt tcgggatgac ggaaacagaa aggaaagagg tggacgagca 420

gattcatgcc tgctatgcac agaccatggc tgagtggctg gg 462

<210>  120
<211>  524
<212>  DNA
<213>  Homo sapiens


<220>
<221>  misc_feature
<222>  (28)..(28)
<223>  n is a, c, g, t or u

<220>
<221>  misc_feature
<222>  (47)..(47)
<223>  n is a, c, g, t or u

<220>
<221>  misc_feature
<222>  (56)..(56)
<223>  n is a, c, g, t or u

<400>  120
tctgctgctg aaggcctgtg attttgtngg ggaagggcct gttctangca actggnaaag      60

gcactgccac ctgccgttgg atgccaggac tcaagagctg gccccagtca ctgtgcgcag     120

agctgtctga gaatgtgtga gtggactggg tccttcggca ctgcctgcat tggctcaggg     180

cagtcaaccg tcgcagagga tgaggggcac actcaggcag cctccccggc cctggaggca     240

gaaaggccca ggcagaacca ctgactggga ggaaacagaa aaagcagagg agagccaggc     300

tgcaggcgtg tggatgggac cagctcaggc agacgctgtc tcatacccac tctcccctct     360

cttgccaggg cctggcctgg tgtctctcag gagcctgggc atgagacaaa agcagagatt     420

gttctcttgt ggtaccacag gctgtaacca gtccacccag tgttgtttta gaaatttaaa     480

tcggttgccc atctttttaa attggcaaca tcgtttacca catt                      524


<210>  121
<211>  326
<212>  DNA
<213>  Homo sapiens

<400>  121
ccccaagttg gcgggcctga ttgggcggca cgggccccag aacaagcagc ccttcatggt      60

ggctttcttc aaggccacgg aggtccactt ccgcagcatc cggtccacgg ggagcaaaca     120

gcgcagccag aaccgctcca agacgcccaa gaaccaggaa gccctgcgga tggccaacgt     180

ggcagagaac agcagcagcg accagaggca ggcctgtaag aagcacgagc tgtatgtcag     240

cttccgagac ctgggctggc aggactggat catcgcgcct gaaggctacg ccgcctacta     300

ctgtgagggg gagtgtgcct tccctc                                          326


<210>  122
<211>  372
<212>  DNA
<213>  Homo sapiens

<400>  122
atgcggagtg agaaaagcct gttgcagaag actacataca acaggatttg acacttgtaa      60

ggctccaaaa caaagaaaat taaatgatat tgtttaggtt ttcatacata ggtgataaaa 120

gtgtgtttct ttgtttttaa tgagaaaatt agtcacagaa tttaagatct tagttacttc 180

tatagggaag gcaggggaat gggacaagga ggaagcccac agcattggtc atgctctcat 240

gttgaagttg ggttcaaagg tgttcattat taaaatgctt cataatgatg accatacatt 300

tggtatttct aggacaatct tggtttacat ctattgtctc aacataatta ttcagtgcaa 360

gcctttcctt tc 372

<210> 123
<211> 197
<212> DNA
<213> Homo sapiens

<400> 123
ctaccttgcc tgctgagaca tagggctcca cgggtctctc tcctggggcc gggctgactg 60

tggcctgcga ggggcagtca tcgtgttggg ttttcctgcc agaggcagaa accacaaaat 120

tacctggaac atacacgccc caagtgacag attcaattca attccacaaa tattgacctc 180

gcgtctaatc cactcgt 197

<210> 124
<211> 379
<212> DNA
<213> Homo sapiens

<400> 124
ctctgagcct tgcttggttg tcagaggcca tgagaggtgc cagttatagg tggatgtgcc 60

aagatgctgg tgaacttggt cttcagctat acccaggctc agaaagggca agagccatgc 120

tgcagcgtag gtgactttgg aggtgcactt ggggcccagg gctttgagtg ttgcgggtgt 180

gcctgtccct ccagatagtg ctctgtttct ctctgttgtc cccctgcctg gtcctctggg 240

gccactgtgc tttctgctgt gtgcatttat aaatgatgtg tattttatat agacctgctt 300

gcattggctg atgctcctct aattccctga gtttgattca accacccttg ggttgttttg 360

ctatggcctt agcctttga 379

<210> 125
<211> 495
<212> DNA
<213> Homo sapiens

<400> 125
gaaccagaat ccttggaagc tctaggtcct acctcagaaa atctcatcgt catggttctg 60

cttcaagaac agaatttggg aattaggtat aagttcaatg ttcccatcac tcgaactggc 120

agtggagata atgaagttgg ctttacatgg aatcatcagc cttggtcaga atgctcagct 180

acttgtgctg gaggtaagat gcccactagg cagcccaccc agagggcaag atggagaaca 240

aaacacattc tgagctatgc tttgtgtttg ttaaaaaagc taattggaaa catttcttgc 300

aggtttgctt caagctgtaa tttagcaaaa gaaactttgc tttaattata ttatattcca 360

tttgttttca acctcatgta atttgtgcag atttgttggt aaaatacatc ttggcacaat 420

gagtgtctct gctggtgctt ctcccaagac tatcttgaag gtgggctgtt tgcctttcgt 480

gaacacattc ttggt 495

<210> 126
<211> 491
<212> DNA
<213> Homo sapiens

<400> 126
atacctcatg cagccttcag gttcagttct gacaccaggg atggaccatc ccatttctct 60

ccagcctgcc tccatgatgg gaccccttac ccagcaactg ggccatctct ccctcagcag 120

cacaggcacg tatatgccga cggctgcagc tatgcaagga gcttacatct cccagtacac 180

ccctgtgcct tcttccagtg tttcagtcga ggagagcagc ggccaacaga accaagtggc 240

agtggacgca ccctcagagc atggggtcta ttctttccag ttcaacaagt aacagtggga 300

ttcccctccc catctttact gaatagaaat gaattcttgg agatactcat gctcccagat 360

tccagagggt taaccaggaa tggagaccat ccgtcggccc tgctaaggac taacacttag 420

ccatcgtttt tcacaggcct gggcctggaa aaagaaatct ctacgttcct gccctttact 480

attgctgatg g 491

<210> 127
<211> 391
<212> DNA
<213> Homo sapiens

<400> 127
ggtgctgccc tgtgtacata taaatgaatc tggtgttggg gaaaccttca tctgaaaccc 60

acagatgtct ctggggcaga tccccactgt cctaccagtt gccctagccc agactctgag 120

ctgctcaccg gagtcattgg gaaggaaaag tggagaaatg gcaagtctag agtctcagaa 180

actcccctgg gggtttcacc tgggccctgg aggaattcag ctcagcttct tcctaggtcc 240

aagcccccca caccttttcc ccaaccacag agaacaagag tttgttctgt tctgggggac 300

agagaaggcg cttcccaact tcatactggc aggagggtga ggaggttcac tgagctcccc 360

agatctccca ctgcggggag acagaagcct g 391

<210> 128
<211> 458
<212> DNA
<213> Homo sapiens

<400> 128
tgtatggtcg ctggccagtg attctccttc tgagccgtgt ttcccctctc cctccctctc 60

cacgtgggca gggcaggccc catcgctttc ctctgataac cacatggaca catcctgaag 120

tcagcccagg cgccctgagc atcttggggc acctggaccc catcacaata ctccttcttc 180

cttcaggtcc ctgggtgaag gctttgctga aaccgacccc ccttttcacg tcccttctgc 240

ctctgccccg ttggatgccc tgactggggg caggggaaga gacagggcac agctggccac 300

agggctcagc cactgagcag gctgttccgg gcctttggct ttgcatcctg gacggggagt 360

gtcctgtcag ggaccagatg tgtcctgcct catccctagc tccaatccct tccccacgtg 420

accggggatt ctggttgcaa taaaacatgc tgctgctg 458

<210> 129
<211> 496
<212> DNA
<213> Homo sapiens

<400> 129
gcagtctcgt ccaatttcta tagcaccgtg ggcaggaacg gcgtcctgcc acaggctttc 60

gaccagtttt tcgagacagc ctacggcacc ccggaaaacc tcgcctcctc cgactacccc 120

ggggacaaga gcgccgagaa ggggcccccg gcggccacgg cgacctccgc ggcggcggcg 180

gcggctgcaa cgggcgcgcc ggcaacttca agttcggaca gcggcggcgg cggcggctgc 240

cgggagacgg cggcggcagc agaggagaaa gagcggcggc ggcgccccga gagcagcagc 300

agccccgagt cgtcttccgg ccacactgag gacaaggccg gcggctccag tggccaacgc 360

acccgcaaaa agcgctgccc ctataccaag taccagatcc gagagctgga acgggagttc 420

ttcttcagcg tctacattaa caaagagaag cgcctgcaac tgtcccgcat gctcaacctc 480

actgatcgtc aagtca 496

<210> 130
<211> 538
<212> DNA
<213> Homo sapiens

<220>
<221> misc_feature
<222> (475)..(475)
<223> n is a, c, g, t or u

<400> 130
aggtcaccca gctgtgaatg aacgtggtca gaacacagaa tctgagttgg tcacacttcc 60

cactgatcca tggggccttt aagccctctg gaagcttcca ttaaagatga ttatttgagg 120

ataattgtat tgggatgcct atgatcttat ctagggtttt cctacccatc cccaacattc 180

agctcagctg cctctttctt gaggacaccc tcactgatca ccccagccca gccagagtgg 240

ttgctcctgc tcctgcccct gaacctatga catacccaag tcccaatact ttcgagccat 300

ctgccactgc cttttgacat ctctgccttg gctagattca aatggtgttt cataataaaa 360

gtctgagttt aagcagcttt accgaaaacg caagggaagt ttcattccat ttatacttct 420

ccagaccccc tgccatcctc tgctgctacc cacacaggca gaataaaagg cttanatgtg 480

taagtcccat gaaggcaaag attggtctct tgtgttcact gctgtctgta gtacttag 538

<210> 131
<211> 414
<212> DNA
<213> Homo sapiens

<400> 131
gtggcaaaag gggattcggc agctgtgatt aagaaccttg tgatggggag ggattcctgg 60

attacccagg tgagtttaat gtaaccacaa agatcctttc aagagggagg caggaaggtc 120

tgaggcagac gaaagagctg tgccaaggga agcaggcggc agtgggatgc aggtggcctc 180

tagaagctgg aaaaggcaag tccatgggtt ctttcctgga gccttcagaa ggagcacggc 240

cttgctgacc catcttagaa cggcaggata atcaatgtgt gttgtttgag gccactaagt 300

ttgtggcaat ttgttacagc agcaatagga aactactaca ctgtgtctga ttagatcagg 360

ccaatgaatg gagaaagtat tggatttcag ttgagtgcta aaacctggtc tgtt 414

<210> 132
<211> 408
<212> DNA
<213> Homo sapiens

<400> 132
ccagcttcac atggtctctc aagtgcttat gcttttcttc tctctgccac ccacattccc 60

acatcccgcc caccccccaa ctttcctccc ttcaccttcc catggagact ttttgcctgg 120

gctaaatctg atcctcagcc cactctcaga atcgataaat gcccctaggt gattgtaagc 180

tcacctaaga tatacttttt ctcctctaga attttagttt attagatttt tctagttgtc 240

tttgcaaaag cgttaacagg ctctgacttc tgacattcaa ctagatgtgg aatatccaac 300

ccctagcatt tcatggaatg tactgaccaa gataaaatgt gttcttatta aacaatgcca 360

tttcttgacc acttctgttt ttaggaattg tggtatctga gtcatggt 408

<210> 133
<211> 483
<212> DNA
<213> Homo sapiens

<220>
<221> misc_feature
<222> (94)..(94)
<223> n is a, c, g, t or u

<220>
<221> misc_feature
<222> (96)..(96)
<223> n is a, c, g, t or u

<220>
<221> misc_feature
<222> (425)..(425)
<223> n is a, c, g, t or u

<400> 133
gcgacaaggt tgtgatccac gtggcaggtg ttcagaaggc tgggggcggg cagcgctggg 60

gagagccctg ggtacttcga ggagaccccg aagngnggct gctcccacac ctgcgccagt 120

ttccaccctc tctgtgagca gggctgcggt cacctcccac atctgaagag aaccaacctg 180

aggatttcac gctggctgcg tgccagacca gtccctgaca ggttgtgcga ggcccttcgc 240

tggacagccc attgctggcc actggacgga gaggcagagg gggctgaaat tcgggcccat 300

gcctctgtga gcgatgacgg agcaacagct ctccagcacg tgaagctctc cagacagctg 360

ttcgtgagaa gccagacaga ggcctggggt ctcagtccag atttctgggg agtggggtgt 420

ccaancgtgg gccacgctgc tgggagccac ctagggaagc aggtcgcctg tttctatagt 480

gac 483

<210> 134
<211> 496
<212> DNA
<213> Homo sapiens

<400> 134
gggaaaaccc ttgtacctga agcatgagcc actcagaact caccaaaata ttcgacacca 60

taacaacaga tgctcaaact gtaaaccagg acaacaagtg gatgacttca cactgtggac 120

agtttttccc aagatgtcag aacaagactc cccatcatga tgaggctctc ccccctctta 180

actgtccttg ctcatgcctg cctctttcac ttggcaggat aatgcagtca ttagaatttc 240

acatgtagta gcttctgaga gtaacaacag agtgtcagat atgtcatctc aacctcaaac 300

ttttacataa catctcaggg ggaaatgtgg ctctctccac cttgcataca gggctcccaa 360

tagaaatgaa cacagagata ttgcctgtgt gtttgcagag aagatggttt gtatgaagac 420

gtaggaaagc tgaaattata atagagtccc ctttaaatcc acattgtgtg gatggctctt 480

gccgtttcct aagaga 496

<210> 135
<211> 479
<212> DNA
<213> Homo sapiens

<220>
<221> misc_feature
<222> (305)..(305)
<223> n is a, c, g, t or u

<400> 135
gagtccgagg atttcagggg cagctgggcg caggagctgg tgggctgttg ggagtgcccc 60

tttactgggc aggcttcctt cctcctggtg atggggggtt cctcagcaca aaagtgaagg 120

ggtggagggg ctggaggagc aggaatctct cttgttgata ggtatgaggc cttgaagtcc 180

ttttctttgt cccaggattc atggacgctt cggggctgat ctttgagttt tcaagcatgg 240

ggtgcagaga cgtttaggta aactcttacc gtcctctctc ttcgtcaggg cttcccagga 300

atcancaatg cccaagaagg aagggattgt agaaatagct taaccctttc atttaccaac 360

gtggaaattg aagcccaggg aagggaaggg accggtcgtg gaagggagag ccatcagcag 420

aaagagaccc tgagatcttc gcctgggatt cccaggaagt ccagcccgag ctgattcac 479

<210> 136
<211> 393
<212> DNA
<213> Homo sapiens

<220>
<221>  misc_feature
<222>  (101)..(101)
<223>  n is a, c, g, t or u

<400>  136
tcccaagccc ttagggaccg cagaggactt ggggaccagc aagcaacccc cagggcacga      60

gaagagctct tgctgtctgc cctgcctcac cctgccccac nccaggcccg gtggccccca     120

gctgcatcaa gtggaggcgg aggaggaggc ggaggagggt ggcaccatgg gcccgggcgg     180

tgccctccat gcccggggga tgaagacact gctgccatgg acagcccgtg ccagccgcag     240

cccctaagtc aggctctccc tcagttacca gggtcttcgt cagagccctt ggagcctgag     300

cctggccggg ccaggatggg agtggagagt tacctgccct gtcccctgct cccctcctac     360

cactgtccag gagtgcctag tgaggcctcg gca                                  393

<210>  137
<211>  377
<212>  DNA
<213>  Homo sapiens

<400>  137
aacctatcgc tgacttagca accaaagcct ccatcgttag gcaaggaata aaataaaacc      60

agcacgcttt ttccactgtg atttttaaaa gtcattaaaa aatatctttt cccttatgta     120

cagaaaaatt ggaacagaaa aatatctaac ttgctgagca tttgatggga aaaagtaaaa     180

gataacttcc atttggtaca caacttattg tacatagagc tatgatttga ggaggcatct     240

aatttctgaa caaattcacc aagaaatacc atcacttaaa gtcattatcg caatcatgct     300

gcagtgaaca ctctatacaa aatggccagg tcattaaaca tcaaagatgg aaaacaagcc     360

agcaatctct tctgttc                                                    377

<210>  138
<211>  483
<212>  DNA
<213>  Homo sapiens

<400>  138
tgggcctcac ctatgatggg atgctgagtg atgtccagag catgcccaag actggcattc      60

tcatacttat cctaagcata atcttcatag agggctactg cacccctgag gaggtcatct     120

gggaagcact gaatatgatg gggctgtatg atgggatgga gcacctcatt tatggggagc     180

ccaggaagct gctcacccaa gattgggtgc aggaaaacta cctggagtac cggcaggtgc     240

ctggcagtga tcctgcacgg tatgagtttc tgtggggtcc aagggctcat gctgaaatta     300

ggaagatgag tctcctgaaa tttttggcca aggtaaatgg gagtgatcca agatccttcc     360

cactgtggta tgaggaggct ttgaaagatg aggaagagag agcccaggac agaattgcca     420

ccacagatga tactactgcc atggccagtg caagttctag cgctacaggt agcttctcct     480

acc                                                                   483

<210>  139
<211>  200
<212>  DNA
<213>  Homo sapiens

<400>  139
ttttgcttgt cttattggcc cagcaaccag cttgacactg gggactatca ggctccaaat      60

aataaccaat gtctcactcc aaacagacag gatactacgg agccagggtc agcaaacatt     120

ttctgtaaag ggccagatag taaatatttt gggctttgtg ggccctatgg tctctgtcac     180

aacgattcaa ctctgctgtt                                                 200

<210>  140
<211>  243
<212>  DNA
<213>  Homo sapiens

<400>  140
gagcgcctcc agtctagaag gcataagcca ataggataat atattcaggg tgcagggtgg      60

gtaggttgct ctggggatgg gtttatttaa gggagattgc aaggaagcta tttaacatgg     120

tgctgagcta gccaggactg atggagcccc tgggggtgtg ggatggagga gggtctgcag     180

ccagttcatt cccagggccc catcttgatg ggccaagggc taaacatgca tgtgtcagtg     240

gct                                                                   243

<210>  141
<211>  554
<212>  DNA
<213>  Homo sapiens


<220>
<221>  misc_feature
<222>  (63)..(63)
<223>  n is a, c, g, t or u

<220>
<221>  misc_feature
<222>  (185)..(185)
<223>  n is a, c, g, t or u

<220>
<221>  misc_feature
<222>  (187)..(187)
<223>  n is a, c, g, t or u

<220>
<221>  misc_feature
<222>  (189)..(189)
<223>  n is a, c, g, t or u

<220>
<221>  misc_feature
<222>  (195)..(195)
<223>  n is a, c, g, t or u

<220>
<221>  misc_feature
<222>  (199)..(199)
<223>  n is a, c, g, t or u

<220>
<221>  misc_feature
<222>  (204)..(204)
<223>  n is a, c, g, t or u

<220>
<221>  misc_feature
<222>  (206)..(206)
<223>  n is a, c, g, t or u

<220>
<221>  misc_feature
<222>  (211)..(211)
<223>  n is a, c, g, t or u

<220>
<221>  misc_feature
<222>  (219)..(219)
<223>  n is a, c, g, t or u

<220>
<221>  misc_feature
<222>  (223)..(223)
<223>  n is a, c, g, t or u

<220>
<221>  misc_feature
<222>  (237)..(237)
<223>  n is a, c, g, t or u

<220>
<221>  misc_feature
<222>  (246)..(246)
<223>  n is a, c, g, t or u

<220>
<221>  misc_feature
<222>  (265)..(265)
<223>  n is a, c, g, t or u

<220>
<221>  misc_feature
<222>  (269)..(269)
<223>  n is a, c, g, t or u

<220>
<221>  misc_feature
<222>  (275)..(275)
<223>  n is a, c, g, t or u

<220>
<221>  misc_feature
<222>  (278)..(279)
<223>  n is a, c, g, t or u

<220>
<221>  misc_feature
<222>  (407)..(407)
<223>  n is a, c, g, t or u

<400>  141
tgagtgggct ttgagagagg gggaagagtg agtctgagca cgagttgcag ccagggccag      60

tgnggagggg gtttgggcca gtgcaccttc cggggcccca tcccttagtt tccactgcct     120

cctgtgacgt gaggcccatt cttcactctt tgaagcgagc agtcagcatt cttagtagtg     180

ggttncngnt ctgtnggang actntngaga ntattcttng ttncctgttg gagttgntca 240

aatgtncctt ttaacggatg gttgnatgng cgtcngcnnc caggtttatg aatgacagta 300

gtcacacata gtgctgttta tatagtttag gagtaagagt cttgtttttt attcagattg 360

ggaaatccat tccattttgt gaattgtgac ataataatag cagtggnaaa agtatttgct 420

taaaattgtg agcgaattag caataacata catgagataa ctcaagaaat caaaagatag 480

ttgattcttg ccttgtacct caatctattc tgtaaaatta aacaaatatg caaaccagga 540

tttccttgac ttct 554

<210> 142
<211> 479
<212> DNA
<213> Homo sapiens

<400> 142
ggacatggtt atctacagca ctgagataca ctactcttct aagggcacgc catctaagtt 60

tgtgatccca gtgtcatgtg ctgcccccca aaagtcccca tggctcacca agccctgctc 120

catgagagta gccagcaaga gcagggccac agcccagaag gatgagaaat gctacgaggt 180

gttcagcttg tcacagtcca gtcaaaggcc caactgcgat tgtccacctt gtgtcttcag 240

tgaagaagag catacccagg tcccttgtca ccaagcaggg gctcaggagg ctcaacctct 300

gcagccatct cactttcttg atatttctga ggattggtct cttcacacag atgatatgat 360

tgggtccatg tgatcctcag gtttggggtc tcctgaagat gctatttcta gaattagtat 420

atagtgtaca aatgtctgac aaataagtgc tcttgtgacc ctcatgtgag cacttttga 479

<210> 143
<211> 514
<212> DNA
<213> Homo sapiens

<400> 143
cagttgctgc cctacatgga gaacaggagg ggtgctgtca tcctggtctc ttccattgca 60

gcttataatc cagtagtggc gctgggtgtc tacaatgtca gcaagacagc gctgctgggt 120

ctcactagaa cactggcatt ggagctggcc cccaaggaca tccgggtaaa ctgcgtggtt 180

ccaggaatta taaaaactga cttcagcaaa gtgtttcatg ggaatgagtc tctctggaag 240

aacttcaagg aacatcatca gctgcagagg attggggagt cagaggactg tgcaggaatc 300

gtgtccttcc tgtgctctcc agatgccagc tacgtcaacg gggagaacat tgcggtggca 360

ggctactcca ctcggctctg agaggagtgg gggcggctgc gtagctgtgg tcccagccca 420

ggagcctgag ggggtgtcta ggtgatcatt tggatctgga gcagagtctg ccattctgcc 480

agactagcaa tttgggggct tactcatgct aggc 514

<210> 144
<211> 265
<212> DNA
<213> Homo sapiens

<220>
<221>  misc_feature
<222>  (74)..(75)
<223>  n is a, c, g, t or u

<220>
<221>  misc_feature
<222>  (79)..(79)
<223>  n is a, c, g, t or u

<220>
<221>  misc_feature
<222>  (126)..(126)
<223>  n is a, c, g, t or u

<400>  144
gtgtggtgtt tgtgtcttaa ctatgcactg ggcccttgtc tgcgtcggct tgcatacaga      60

gggcccctgg ggtnngccnt ccggcctggc ctcagccagt gggatggaca gggccaggca     120

ggcctntgaa cttccacctc ctggggcctc ccagacctcc tgtgccccca cctgtgtggg     180

caggtgggcc agtcttcggg tgatgggacc aaaccccttc agttcagtag agaaaggcta     240

ggtcctctac aaagagctgc aagac                                           265

<210>  145
<211>  419
<212>  DNA
<213>  Homo sapiens

<220>
<221>  misc_feature
<222>  (53)..(53)
<223>  n is a, c, g, t or u

<220>
<221>  misc_feature
<222>  (57)..(57)
<223>  n is a, c, g, t or u

<220>
<221>  misc_feature
<222>  (61)..(61)
<223>  n is a, c, g, t or u

<220>
<221>  misc_feature
<222>  (73)..(73)
<223>  n is a, c, g, t or u

<220>
<221>  misc_feature
<222>  (78)..(78)
<223>  n is a, c, g, t or u

<220>
<221>  misc_feature
<222>  (82)..(82)
<223>  n is a, c, g, t or u

<220>
<221>  misc_feature
<222>  (114)..(115)
<223>  n is a, c, g, t or u

<220>
<221>  misc_feature
<222>  (130)..(130)
<223>  n is a, c, g, t or u

<220>
<221>  misc_feature
<222>  (144)..(144)
<223>  n is a, c, g, t or u

<220>
<221>  misc_feature
<222>  (165)..(165)
<223>  n is a, c, g, t or u

<220>
<221>  misc_feature
<222>  (177)..(177)
<223>  n is a, c, g, t or u

<220>
<221>  misc_feature
<222>  (189)..(190)
<223>  n is a, c, g, t or u

<220>
<221>  misc_feature
<222>  (192)..(192)
<223>  n is a, c, g, t or u

<220>
<221>  misc_feature
<222>  (218)..(218)
<223>  n is a, c, g, t or u

<220>
<221>  misc_feature
<222>  (225)..(225)
<223>  n is a, c, g, t or u

<400>  145
ggaggcgcag aagattgatc gcatgatgga ggctttcgct tctcgctact gcntgtncaa      60

ncccggggtc ttncagtnca cnaggtcagt gcagagccca cagcctggcc cctnnccagg     120

cacagcctcn agctctggag gggncggccc ctgtgggcac agccnagcgt gtgttcntgg     180

ggacctgcnn tnccctgagc gaggacgacc tgtgggcngg gcacntcttg caggcgggcc     240

cccagcacgc ggggtcccac tgtccactgg aggttctggc tgagcccagc accccggact     300

cgttgcagac acgtgctacg tgctgtcatt cgccatcatc atgctcaaca ccagcctcca     360

caaccacaac gtgcgtgaca agcccacggc agaacggttc atcgccatga accgcggca      419


<210>  146
<211>  492
<212>  DNA
<213>  Homo sapiens


<220>
<221>  misc_feature
<222>  (411)..(411)
<223>  n is a, c, g, t or u

<400>  146
tatgagaaac ctctgcgacc attcccagat gatgtctgcg ttgtccctga gaaatttgaa      60

ggagacatca agcaggaagg ggtcggtgca tttcgagagg ggccgcccta ccagcgccgg     120

ggtgccctgc agctgtggca atttctggtg gccttgctgg atgacccaac aaatgcccat     180

ttcattgcct ggacgggccg gggaatggag ttcaagctca ttgagcctga ggaggtcgcc     240

aggctctggg gcatccagaa gaaccggcca gccatgaatt acgacaagct gagccgctcg     300

ctccgatact attatgagaa aggcatcatg cagaaggtgg ctggtgagcg ttacgtgtac     360

aagtttgtgt gtgagcccga ggccctcttc tctttggcct tcccggacaa ntcagcgtcc     420

agctctcaag gctgagtttg accggcctgt cagtgaggag gacacagtcc ctttgtccca     480

cttggatgag ag                                                         492

<210>  147
<211>  527
<212>  DNA
<213>  Homo sapiens

<400>  147
   aatattgtct cataagcatc tttctatatt tgttcacatc gtacataatc atgtttttgc      60
   acagatacat taatattatc atagtttgtt taactacttg gctttttcta acagtttttt     120
   tttttgagat ggtcttgctc tgttgcccag gctggagtgc agtgacgtga tctcggctca     180
   ctgcagcctt gacttcctgg gctcaagtga tcatcccacc tcagcctcct gagtagctgg     240
   gactacaggt atgcaccacg accagctaat tttttgtatt ttttttttgt agagagggta     300
   ttttgccatg ttgcccaggc tagtcttgaa ctcctgggct caagcgatct gcctgcttca     360
   gcctcccaga gtgctaggat tacaggcatg agccactgca cccagcctct taacaaattt     420
   tgaatataac tcctgtctta aaatctgcag aatattgaat ttttccagct attttttact      480
   tttgcttagc ttatagatgc taaaggatac tgtcatttgc attttta                   527

<210>  148
<211>  476
<212>  DNA
<213>  Homo sapiens


<220>
<221>  misc_feature
<222>  (50)..(50)
<223>  n is a, c, g, t or u

<220>
<221>  misc_feature
<222>  (92)..(92)
<223>  n is a, c, g, t or u

<220>
<221>  misc_feature
<222>  (105)..(105)
<223>  n is a, c, g, t or u

<400>  148
ctctctcact ttctatagct ttgttggacc agatggtgag gaaaggaatn ggcctcttcc      60

cttctagagg gggctggctg gagtgagacc tnggggcttg gcctnggaac ccaccacaca 120

gccccaaagt caggaagcct ggggaaacca gagctgagac ctcttcaaca gggtttcttt 180

gagatcctac acctccattg ggcccttttt cagtcttcaa tgggggccca gttggctcta 240

gaaggagaag aggtgaagca ggatcctttg ccctggggga gtctgagggc gcggtccttg 300

gactcattca ggccgtcttt gtagttgggg gagttccact gggcgatccc agcccctccc 360

cacccaccct ctaatggacc tcctcataga agccccattt cacttttgtt ttatctacct 420

cttagcaaaa caatagataa attaggtagt ggcagctcca cttgcttagg ttaggg 476

<210> 149
<211> 177
<212> DNA
<213> Homo sapiens

<400> 149
gggagtttga ccagagatgc aaggggtgaa ggagcgcttc ctaccgttag ggaactctgg 60

ggacagagcg ccccggccgc ctgatggccg aggcagggtg cgacccagga cccaggacgg 120

cgtcgggaac cataccatgg cccggatccc caagacccta aagttcgtcg tcgtcat 177

<210> 150
<211> 497
<212> DNA
<213> Homo sapiens

<220>
<221> misc_feature
<222> (109)..(109)
<223> n is a, c, g, t or u

<220>
<221> misc_feature
<222> (113)..(113)
<223> n is a, c, g, t or u

<220>
<221> misc_feature
<222> (146)..(147)
<223> n is a, c, g, t or u

<400> 150
ctaaccactg aggctctcta atcttcctct ggagttttag tgaaaggatt tattgagcag 60

cttctggaat ataatgtgca tgtccaaaat gaactcagcg cttcaaaang acnaagtctg 120

tagcctggag gggcttgagt ggatgnnagc tgatgctgtg attttgagct gtggttacat 180

gcagtcagta aacctgtgag actgctggag gaaatgtagc agacagcatg gaggctggga 240

cccagcagct actttgggtc atgtctttac tgtcctgcct ccaacccttt agtctcgtag 300

acttttgttc ttgtggaaat ttcttctgta ttccagttgt gtaaatatgt atggaaaact 360

gatattacta ggttttacgt tgcatctcca gtattgatct ttggaaactg atgttacatt 420

aggttccaat tcgcaatagt agcagagact gacatgcttt tattgagctg ctaagccccg 480

tggatgatgg agcgaga 497

<210>  151
<211>  529
<212>  DNA
<213>  Homo sapiens


<220>
<221>  misc_feature
<222>  (195)..(195)
<223>  n is a, c, g, t or u

<220>
<221>  misc_feature
<222>  (393)..(393)
<223>  n is a, c, g, t or u

<220>
<221>  misc_feature
<222>  (403)..(403)
<223>  n is a, c, g, t or u

<220>
<221>  misc_feature
<222>  (417)..(417)
<223>  n is a, c, g, t or u

<220>
<221>  misc_feature
<222>  (431)..(433)
<223>  n is a, c, g, t or u

<220>
<221>  misc_feature
<222>  (435)..(435)
<223>  n is a, c, g, t or u

<220>
<221>  misc_feature
<222>  (440)..(440)
<223>  n is a, c, g, t or u

<220>
<221>  misc_feature
<222>  (442)..(443)
<223>  n is a, c, g, t or u

<400>  151
gccgacagct cctttaattt catggcgttt ttcttcatct tcggagccca gtttgtcctg      60

accgtcatcc aggcgattgg cttctccggc tggggcgcgt gcggctggct gtcggcaatt     120

ggattcttcc agtacagccc gggcgctgcc gtggtcatgc tgcttccagc catcatgttc     180

tccgtgtcgg ctgcnatgat ggccatcgcg atcatgaagg tgcacaggat ctaccgaggg     240

gctggcggaa gcttccagaa ggcacagacg gagtggaaca cgggcacttg gcggaaccca     300

ccgtcgaggg aggcccagta caacaacttc tcaggcaaca gcctgcccga gtaccccact     360

gtgcccagct acccgggcag tggccagtgg ccnttagagg gangcctgcc ctgcccncac     420

cgcccaccac nnncnccccn tnnttcctgc tgctacccct gtgtcccgag ggctgggagt     480

acctggggcc ccatcccccc agctgtgatg gtggaagccg gtggtggcc                 529

<210> 152
<211> 437
<212> DNA
<213> Homo sapiens

<220>
<221> misc_feature
<222> (145)..(146)
<223> n is a, c, g, t or u

<400> 152
agatgaagcc cttcaagcgc tacgtgaaga agaaagccaa gcccaagaaa tgtgcccggc 60

gtttcaccga ctactgtgac ctgaacaaag acaaggtcat ttcactgcct gagctgaagg 120

gctgcctggg tgttagcaaa gaagnngacg cctcgtctaa ggagcagaaa acccaagggc 180

aggtggagag tccagggagg caggatggat caccagacac ctaaccttca gcgttgccca 240

tggccctgcc acatcccgtg taacataagt ggtgcccacc atgtttgcac ttttaataac 300

tcttacttgc gtgttttgtt tttggtttca ttttaaaaca ccaatatcta ataccacagt 360

gggaaaagga aagggaagaa agactttatt ctctctctta ttgtaagttt ttggatctgc 420

tactgacaac ttttaga 437

<210> 153
<211> 87
<212> DNA
<213> Homo sapiens

<400> 153
ttctttcaca ccctgtcggg agaatgtgtg ccctgcgact gtaatggcaa ttccaacgag 60

tgtttggacg gctcaggata ctgtgtg 87

<210> 154
<211> 417
<212> DNA
<213> Homo sapiens

<400> 154
cccgctggtg cagtggaaga gcccggcggc cgccgccgca gccttctcgg cccgcgcccc 60

cgccgcctgc acccccatct gctcttcccc gcgggggccg cgcggcgcgg gctgggggcc 120

cgggcagccg cgctcgggca gcgggggcgc ggggctgccg cctgcgctcg cagctggtgc 180

cggtgcgcgc gctcggcctg ggccaccgct ccgacgagct ggtgcgtttc cgcttctgca 240

gcggctcctg ccgccgcgcg cgctctccac acgacctcag cctggccagc ctactgggcg 300

ccggggccct gcgaccgccc ccgggctccc ggcccgtcag ccagccctgc tgccgaccca 360

cgcgctacga agcggtctcc ttcatggacg tcaacagcac ctggagaacc gtggacc 417

<210> 155
<211> 407
<212> DNA
<213> Homo sapiens

<400> 155
taagagactg agccgctagc agcgcctggg gaccagacag acgcatgtgg caaagctcac 60

catcttcact acaaacacgc ctgagagtgg cactggggaa acataactcc atctacacct 120

tggatttgga ctgattctcc attttatcac ctgaaggctt gggccagagc tcaacagcta 180

ctcaactgga ggggtgaggg ggataaggtc tgtagtatac agacaggaag atggtaggtt 240

tatgccttct gtggccagag tcttggactc atggaaatag aatgaataga ggggcattca 300

caaggcacac cagtgcaagc agatgacaaa aaggtgcaga aggcaatctt aaaacagaaa 360

ggtgcaggag gtaccttaac tcacccctca gcaaatacct atgtcaa 407

<210> 156
<211> 399
<212> DNA
<213> Homo sapiens

<400> 156
gagaccagtt cacggggcaa gagatgaacg tggcccagtt cctcatgcac atgggcttcg 60

acatgcagac ggtggcccag ccgcagggac tggagcccag tgagctgctg gggatgctga 120

gcaacggaag ctaggcagac tgtctggagg aggagccggc actgaggggc ccagacaccc 180

gctgccccag tgccacctca ccccccacca gcaggccctc ccgtctcttc gggacagggc 240

cccagccgtc ccccctgtct gggtctgccc actgccctcc tgccccggct ttccctgccc 300

ctctcccaca gcccagccag agacaaggga cctgctgtca tccccatctg tggcctgggg 360

gtccttcctg acaacgaggg ggtagccaga agagaagca 399

<210> 157
<211> 422
<212> DNA
<213> Homo sapiens

<400> 157
gtgaccagta ccgcaagggg atcatctcgg gctccgtctg ccaggacctg tgtgagctgc 60

atatggtgga gtggaggacc tgcctctcgg tggccccggg ccagcaggtg tacagcgggc 120

tctggcggga caaggatgta accatcaagt gtggcattga ggagaccctc gactccaagg 180

cccggtcgga tgcggccccc cggcgggagc tggtactgtt tgacaagccc acccggggca 240

cctccatcaa ggaattccgg gagatgaccc tcggcttcct caaggcgaac ctgggagacc 300

tgccttccct gccggcgctg gttggccagg tcctgctcat ggctgacttc aacaaggaca 360

accgggtgtc cctggcggaa gccaagtccg tgtgggccct gctgcagcgt aacgagttcc 420

tg 422

<210> 158
<211> 414
<212> DNA
<213> Homo sapiens

<220>
<221> misc_feature
<222> (364)..(364)
<223> n is a, c, g, t or u

<220>
<221>  misc_feature
<222>  (373)..(373)
<223>  n is a, c, g, t or u

<220>
<221>  misc_feature
<222>  (375)..(376)
<223>  n is a, c, g, t or u

<220>
<221>  misc_feature
<222>  (378)..(380)
<223>  n is a, c, g, t or u

<220>
<221>  misc_feature
<222>  (383)..(384)
<223>  n is a, c, g, t or u

<400>  158
acgcagcccg cgacaacaaa aagacccgca tcatcccgcg ccacttgcag ctggccatcc      60

gcaacgacga ggagctcaac aagctgcttg gtaaagttac catcgctcag ggcggtgttc     120

tgcctaacat ccaggccgta ctgctcccca agaagactga gagccaccac aaagctaagg     180

gcaagtaagg gctgaacttt aaaaatgtaa acttacaaga caaaaggctc ttttcagagc     240

cacccaccat ttctacggaa gaactgagca ctctgttctc caaacctatc agaaatttgt     300

ggccgagttc aagcactgag gccattactt tcctattggg taaaataaaa gtattgaatc     360

aggnctagta aanannannn aanngctacc ttataacatg aaggaacctc ctta           414


<210>  159
<211>  470
<212>  DNA
<213>  Homo sapiens

<400>  159
tatcaagatt gcccctgcgg aaggcccaga cgtcagcgaa aggatggtca tcatcaccgg      60

gccaccggaa gcccagttca aggcccaggg acggatcttt gggaaactga aagaggaaaa     120

cttctttaac cccaaagaag aagtgaagct ggaagcgcat atcagagtgc cctcttccac     180

agctggccgg gtgattggca aaggtggcaa gaccgtgaac gaactgcaga acttaaccag     240

tgcagaagtc atcgtgcctc gtgaccaaac gccagatgaa aatgaggaag tgatcgtcag     300

aattatcggg cacttctttg ctagccagac tgcacagcgc aagatcaggg aaattgtaca     360

acaggtgaag cagcaggagc agaaataccc tcagggagtc gcctcacagc gcagcaagtg     420

aggctcccac aggcaccagc aaaacaacgg atgaatgtag cccttccaac                470


<210>  160
<211>  383
<212>  DNA
<213>  Homo sapiens

<400>  160
agagagactc agagacccgg gagggccttc ctctgaaagg ccaagccaag ccatgcttgg      60

cagggtgagg ggccagttga gttctgggag ctgggcacta ctctgccagt ccagagttgt 120

acagcagaag cctctctcct agactgaaaa tgaatgtgaa actaggaaat aaaatgtgcc 180

cctcccagtc tgggaggagg atgttgcaga gccctctccc atagtttatt atgttgcatc 240

gtttattatt attattgata atattattat tactattttt ttgtgtcatg tgagtcctct 300

ctccttttct ctttctgaca ttccaaaacc aggccccttc ctacctctgg ggctgcttga 360

gtctagaacc cttcgtatgt gtg 383

<210> 161
<211> 474
<212> DNA
<213> Homo sapiens

<400> 161
aggatgcccc tttgagaaat gctgttccac agaaccctgc ctttcaggcc ttggagacgt 60

gggcagggga gaagcagcgt ccctcagagc caggcctggc agtggtgcta gcaggggcca 120

aggccaggga gcagggtctc ctgtcggagg gacctgggca agcccctcca cgcgccagcg 180

ggtttctcag caggggaggt ccacaccaca ccgcttggga acctgggtgc ctaaacgcaa 240

caggagccaa ggcacaaatt taaccaaaca ccaaggttgc gtgaggcccc atttcatgag 300

ccgggctcca aggacgtgtc cttaggcggc tctggaaggc ccagcgccag cccccgtcct 360

ctgttaaagg gagccagccc cggcgtccgc ccaggcatgg tagcctgagc gcgcccccag 420

ggtagtaggg ggcacctgag gagcagggtc tgccctggca tgagcagagc ccag 474

<210> 162
<211> 371
<212> DNA
<213> Homo sapiens

<220>
<221> misc_feature
<222> (134)..(134)
<223> n is a, c, g, t or u

<400> 162
gatacttgga tgcttttcct ctgactgatg aagatcctga ataccaaaga gggccgctga 60

caggtctagg agtacacttc tagcacctag cagagagagg cttcactaca tcatgcttcc 120

tgacatctct cccnttgaag agcagtcaga ctcctgcttt gctcttcaga cttaatttgg 180

gggtttaaca ggtgaggttg ctgggggaac tcttttacaa catctctctg aaagaatccg 240

ggctgccagt ttcatttggt ttgggtgtca gtagcatgat ggaaagacaa aaaaacacaa 300

cttgacatct gcagaaatgg gttcaaattt tacctgcaac tcaccaattc tgtggccttg 360

gttcagcaat t 371

<210> 163
<211> 445
<212> DNA
<213> Homo sapiens

<400> 163
caacaagacg gacctggctg ataagaggca gataaccatc gaggaggggg agcagcgcgc 60

caaagaactg agcgtcatgt tcattgagac cagtgcgaag actggctaca acgtgaagca 120

gctttttcga cgtgtggcgt cggctctacc cggaatggag aatgtccagg agaaaagcaa 180

agaagggatg attgacatca agctggacaa accccaggag cccccggcca gcgagggcgg 240

ctgctcctgc taatgcagag ccgacctgtg gcttcccatg acactccttg cttgttgtgt 300

tgcttcctat tggctagctt cctaaggggg gagggaaccg agttatcaag atgggaggat 360

ttttcttttc tctctgtctt taggagtagg gtgggatggg gagggaggct gggcatcagg 420

gatcacatca ctcttaacgg ctgtt 445

<210> 164
<211> 313
<212> DNA
<213> Homo sapiens

<400> 164
ggtggcctct ggatcctccg tggaccgaac cgtcccccca ggaacacacc ttcaggtaga 60

ccccgaagcc tcaaggccgg ggctggagcg gagaccccag ggcctctcag gagacagtga 120

ggctgcccct cctaccacct acctcattct gcctactcac cccaggggcc acagccacag 180

cctgctggac tcaggactgt cctgtcaact ccagacaact gaataaacag gccgggtaca 240

gtggctcgca cctgtaatcc tagcactttg ggaggccgaa gcgggtggac cacttgacgt 300

ccgtagttcg aga 313

<210> 165
<211> 344
<212> DNA
<213> Homo sapiens

<400> 165
aatgtcatgt ttattcaggc tgggaactgt attcacagta gaagtttcag tggtcaacat 60

atctatgact ctttaggctg ctgtagtttt acagtcaatt atttaaaagt gagtagttac 120

atttataaga gcctgagaat acttagactc agtcatttgt tagtattttt accaaaatct 180

cttagtttca gacatgtcag aagcagctat atagcatatc ttattctatg atatacatca 240

ggctatctca agttcctgtc tcacagttaa ttcaaagaag gattaggatt tctgtatttt 300

ttctcatttg aatctttatg tgcatttggt ttgtgtacat gctt 344

<210> 166
<211> 448
<212> DNA
<213> Homo sapiens

<400> 166
tcttacccca ctgaaaccaa cagggatcgg gccaggctcc cagattcttg aggacaggga 60

cttcggcatt tactaatggg ggactactgt ggggtaaggg ggcgcctgct tgcctgatac 120

aggatggggt caagggacag tgggcaggtc ctcactcagg agtggggggt gtaggctggc 180

cagcccccag ggcttgtcca ccagtcttct ccccgcaagg ccctcagagc agcgcctgtg 240

ggtgtcagta ttacctgagc ctaggccaaa gctagcccaa ggctggggaa ggggaggaga 300

ctccaggtca gaatgtgagg tctcagtctg tgatttaagg tgttgcatgt ggactcttaa 360

ctgtacgtgt agtttctagt ggagaaatca aggctctgat cattttgttt ttagtatgaa 420

aatgtgattt cctttctgtt tgtaactc 448

<210> 167
<211> 334
<212> DNA
<213> Homo sapiens

<400> 167
agatgccagt aatcaatatt gaggacctga cagaaaagga caaattgaag atggaagttg 60

accagctcaa gaaagaagtg acactggaaa gaatgctagt ttccaaatgt tgtgaagaag 120

taagagatta cgttgaagaa cgatctggcg aggatccact ggtaaagggc atcccagagg 180

acaaaaatcc cttcaaggag ctcaaaggag gctgtgtgat ttcataatac aaacaaaaag 240

aaaaaaaatt aaacaaattc ttggaaatat ctcaaatgtt aataacaata tgaatttttc 300

tcatgcatac tattactact aagcatgtac gtga 334

<210> 168
<211> 561
<212> DNA
<213> Homo sapiens

<400> 168
gcccccgact gaggcggaga cgaaggtgct gcaggcgcga cgggagcggc aagatcgcat 60

ctcccggctc atgggcgact atctgctgcg cggttaccgc atgctgggcg agacgtgtgc 120

ggactgcggg acgatcctcc tccaagacaa acagcggaaa atctactgcg tggcttgtca 180

ggaactcgac tcagacgtgg ataaagataa tcccgctctg aatgcccagg ctgccctctc 240

ccaagctcgg gagcaccagc tggcctcagc ctcagagctc cccctgggct ctcgacctgc 300

gccccagccc ccagtacctc gtccggagca ctgtgaggga gctgcagcag gactcaaggc 360

agcccagggg ccacctgctc ctgctgtgcc tccaaataca gatgtcatgg cctgcacaca 420

gacagccctc ttgcagaagc tgacctgggc ctctgctgaa ctgggctcca gcacctccct 480

ggagactagc atccagctgt gtggccttat ccgcgcatgt gcggaggccc tgcgcagcct 540

gcagcagcta cagcactaag a 561

<210> 169
<211> 244
<212> DNA
<213> Homo sapiens


<220>
<221> misc_feature
<222> (94)..(94)
<223> n is a, c, g, t or u

<220>
<221> misc_feature
<222> (128)..(128)
<223> n is a, c, g, t or u

<400> 169
   aatgtgtatg tctgggtaag tgtatagatt ttacaactat tttgaaggcg acctttttaa 60
   ctttaaacag accactctgg aggagacgcc tganccagag cgctttacct aaagttcggt 120
   gcctaaantg cacccttcct ctggctggtg tctcccttct gccaagctat gcctcctgca 180
   gaggtaggct ccgtggtgtc tcccactccg ccccaactgg agaacggtgt aaagaactgt 240
   cagc 244

<210> 170
<211> 408
<212> DNA
<213> Homo sapiens

<220>
<221> misc_feature
<222> (262)..(262)
<223> n is a, c, g, t or u

<220>
<221> misc_feature
<222> (268)..(268)
<223> n is a, c, g, t or u

<400> 170
caggatggca ttagctctgt gtctgcaggt gctgtgcagc ctgtgtggct ggctctcgct 60

ctatatttct ttctgccacc tgaataagca ccgaagctat gagtggagct gccgcctggt 120

caccttcacc catggagtcc tctctatagg cctctccgct tatattggct tcattgatgg 180

cccatggcct tttacccacc caggctcacc caatacacct ctccaagttc atgtcctgtg 240

tctcaccttg ggctacttca tnttcganct tgggctgcat ctggcgcttt gcatggagga 300

agagcatcaa gaagtaccat gcttggagaa gcaggcggag tgaggaacgg cagctgaaac 360

acaacggaca tctcaaaata cactagccaa ggcttgctcc agattatg 408

<210> 171
<211> 359
<212> DNA
<213> Homo sapiens

<400> 171
aggacatcga ggctgcggtg aaccatgatt gtaccactgt attccagcct ggacgactga 60

gtgagaccct gtctcaaaca aaacaaaaca aaacaaaaaa aagtacaaga ggaaaaaaat 120

tgatttctga ttgcctcact caagataagg tcaacattga aggtggaggt ggaagatgca 180

gtttatgtag gggtctgaag attttaccat tctggggact gtctttaaga aagagaatcc 240

aaaattaggt agaaaagtga acgtctgacc gggcgcggtg gctcatccct gtaatcccag 300

cacttaagga gtacgagacg ggaggatcac gaggtcaaga gatcgacagc atgctggcc 359

<210>  172
<211>  386
<212>  DNA
<213>  Homo sapiens


<220>
<221>  misc_feature
<222>  (182)..(182)
<223>  n is a, c, g, t or u

<400>  172
gtttctgcct ttgaacgtgg ctgtgggaag acatgatgct tagtgttgct gcagctatct      60

catgaccttg ggcaaaacat cccaacacac aggagggcca aacaagcagt cagaagaagc     120

ctgagtcttg tgggtgttgt tgagcagctg aacaaaccct aggatggctt ccttccagac     180

tncttaggat tgcgaacaat gaagctctat tgtttaagca aggtatcgat ggctattttc     240

acttgccact gaaagcacca ggacagagaa tcgtctttct aggaatacag ccacaaaagc     300

cttcattatg gtatatgcac ataaagaata taaaagtttc ctttatgttt ctctttaaaa     360

tatagctgaa gtctgcctca ggcaaa                                          386


<210>  173
<211>  408
<212>  DNA
<213>  Homo sapiens

<400>  173
ggttccaggc tttgcatctg gagcctttac cggttgactg ttgccttcca cacaaacagc      60

ctctgaaaag cactttctcc atacataatt ctggagaaga tgagggatct tgccctccag     120

gagccttcct tcctccccca atgaggaaat cagtcactgc actggtgcaa aggcaagcag     180

attggaattt ctgctcttca ccgattttct cagggaaaga ccccttcccc ttgccagcag     240

aggaacctgt agttttttcc atttctttct tcagaaccaa agtatgtatc actcctcatg     300

ctcacaggga ttgacaggag agaattcacc aggatcttag ctcaaaagac acagcctcag     360

aatggccaga tggattgcac gaaacctgac ttggattcac catcttcc                  408


<210>  174
<211>  331
<212>  DNA
<213>  Homo sapiens


<220>
<221>  misc_feature
<222>  (227)..(227)
<223>  n is a, c, g, t or u

<220>
<221>  misc_feature
<222>  (229)..(229)
<223>  n is a, c, g, t or u

<220>
<221>  misc_feature
<222>  (264)..(264)
<223>  n is a, c, g, t or u

<400>  174
gtggacgagt gactgtccct ggtttgggct ggtgccattt agagggcaac cagagtgcag      60

ggaagggagg agcttgggca agagggacat tgctgtcgct ggttgatggt gagatggcac     120

ttaatgagaa cctggtcatt gggaaagccc caagcctgcg tcttgctgtg atgccttccc     180

cattatgaag ggtccattgg catgggagtg gggagacctg gactcanana agctacaagg     240

gcaagggtgg aaaggcatag cttntgcaag ttgatgctga aaaagatcca agactcatat     300

tcagcagaca gcccataacc aagagccaag g                                    331

<210>  175
<211>  260
<212>  DNA
<213>  Homo sapiens

<400>  175
aggtcttcaa agaattggcc agtcttacag ctcaccttgg ggtgtagatg actctccact      60

gtggtgctag gcaattttat tgaacaggtg gccactggtg gtgatggctg aaccactcat     120

taaacaaatt gctctaaatg gcctcagtat caaggtgtgc tttctgtacc cttaatctga     180

ctttaatcct gcagaacctc agtcttacca tgtttaacag cattgccatg tacgatatgc     240

ctttatccta cactgtatat                                                 260

<210>  176
<211>  528
<212>  DNA
<213>  Homo sapiens

<400>  176
gctggctatg tacatggtcc cattccctac ctgcacttct ttatgcctgt cttcaccctg      60

ctgaccatcc acagcagcca gcactaccag gccctcatag tgcctgagct cacccagcag     120

atggttgatg ccaagaacat gatggttccc tgagacccct gccatggcca ctacctaaag     180

gtggccacag tgttcacgga ctacatgtcc atgaaggagt tggatgagca aatgcttaat     240

gtccaaaaca agaacagcag ctactttgtt gagtgaatcc ccaactatgt gaaaacagct     300

gtctgtgaca tcccactctt ggggctataa atgtctgcca ccttcaacat caacagcgtg     360

gccatccagg agctgttcaa gcacatctct gagtggtcat gtttcggtgc aaagcctttc     420

tgcactggca catgggcaag agcatggact agatggagtt caccaaggct gagagcaaca     480

tgaacaacct ggtgtcccgg taccagtaat accaggacac ctcagcca                  528

<210>  177
<211>  540
<212>  DNA
<213>  Homo sapiens

<220>
<221>  misc_feature
<222>  (31)..(31)
<223>  n is a, c, g, t or u

<220>
<221>  misc_feature
<222>  (34)..(34)
<223>  n is a, c, g, t or u

<220>
<221>  misc_feature
<222>  (48)..(49)
<223>  n is a, c, g, t or u

<220>
<221>  misc_feature
<222>  (52)..(52)
<223>  n is a, c, g, t or u

<220>
<221>  misc_feature
<222>  (54)..(54)
<223>  n is a, c, g, t or u

<220>
<221>  misc_feature
<222>  (64)..(64)
<223>  n is a, c, g, t or u

<220>
<221>  misc_feature
<222>  (66)..(66)
<223>  n is a, c, g, t or u

<220>
<221>  misc_feature
<222>  (76)..(76)
<223>  n is a, c, g, t or u

<220>
<221>  misc_feature
<222>  (78)..(78)
<223>  n is a, c, g, t or u

<220>
<221>  misc_feature
<222>  (122)..(122)
<223>  n is a, c, g, t or u

<400>  177
acttatctgt gctgtaacta ttgaaatgaa nccncttcaa atatgtannc cncntttctt      60

tttnanattt ctaganangg tttcaatata gactttctga cttttatggt atacatatag     120

gncaatattc tattcttctt tccttttaaa tacttactgt ttcaatttca aataaaaaat     180

cagcattcta gtttgtacat tttagcacag aaatgtttac aaccttcagc acaattgctt     240

ttgtaattta ctgacttggc attttgaggc gtttttaaca aattatgaga aataacacct     300

tcagaaagca tgtgactact ttgatgcaac tatttacaat gtattcataa gaagtcatta     360

acctgtagag ttcttagaca tgtggaacct ttaacaatta tactaaagag tacatacaaa     420

atacagagct atgtaataat aactaatttt aaatcctgac aaattagaag ttaagcctac     480

tatctgtaaa aatatgtcct gattcatttt tttaagtata tacctgagcc tttaaaaagt     540

<210>  178
<211>  560
<212>  DNA

<213>  Homo sapiens


<220>
<221>  misc_feature
<222>  (460)..(460)
<223>  n is a, c, g, t or u

<220>
<221>  misc_feature
<222>  (462)..(466)
<223>  n is a, c, g, t or u

<220>
<221>  misc_feature
<222>  (468)..(469)
<223>  n is a, c, g, t or u

<220>
<221>  misc_feature
<222>  (471)..(472)
<223>  n is a, c, g, t or u

<220>
<221>  misc_feature
<222>  (475)..(481)
<223>  n is a, c, g, t or u

<220>
<221>  misc_feature
<222>  (483)..(487)
<223>  n is a, c, g, t or u

<220>
<221>  misc_feature
<222>  (489)..(493)
<223>  n is a, c, g, t or u

<220>
<221>  misc_feature
<222>  (496)..(503)
<223>  n is a, c, g, t or u

<220>
<221>  misc_feature
<222>  (505)..(510)
<223>  n is a, c, g, t or u

<220>
<221>  misc_feature
<222>  (512)..(535)
<223>  n is a, c, g, t or u

<400>  178
gccattttga gtgccagatc tagttatttt gctgcaatgc tgagtggctg ttgggctgaa      60

agctcccaag agtacgttac tcttcaaggt ataagccatg tagaactgaa tgttatgatg     120

cattttatat atggaggaac tctggacatt ccagacaaaa ctaatgttgg tcagatactc     180

aatatggctg atatgtatgg actagaagga ttaaaagaag tagcaatcta tattttaaga     240

agagattact gtaatttctt tcagaagcct gttcccagaa cattgacgtc tatactagaa     300

tgcctgatta ttgctcattc agttggagtg gaaagtcttt ttgctgactg catgaagtgg     360

attgtaaagc attttgcaag gttttggtct gagagaagct ttgcaaatat acctcctgag     420

attcagaaaa gttgtcttaa tatgttgatt cagtccttan tnnnnntnnc nngannnnnn 480

ntnnnnntnn nnnccnnnnn nnngnnnnnn cnnnnnnnnn nnnnnnnnnn nnnnncaggg 540

tgcactcaca gcacagaaca 560

<210> 179
<211> 385
<212> DNA
<213> Homo sapiens

<400> 179
gggttcacgt cattttcctg tctcagcctc cccagtagct gggactccag gcacccacca 60

ccactcccgg ctaatttttt gtatttttag tacagacagg gtttcactgt gttggccagg 120

atggtcttga tctcctgacc ttgtgatcca cccacctcgg cctcccaaag tgctgggatt 180

gcaggcatga atgaccgcgc ccagccgcag gcgcaacttt tttgagtttt cctggccagg 240

cgcggtggct caggcctgta gtcccagcat tttgggaggc cgaggtgggc ggatcacttg 300

aggtcaggag ttagaaacca gcctggccaa cgtggtgaaa ccccgtctcc agtaaacata 360

caaagccatt acagggcatg gtggg 385

<210> 180
<211> 173
<212> DNA
<213> Homo sapiens

<400> 180
gacaacctta gttcacttgg gtattcccat aatccttgtc tttcagggtt gacctgttac 60

agctgcttaa acacatcact gtatgctagg tattgcctac cttcacttac ttttctaacc 120

ttgccgatgt gctgccttca taaactgggt atatctccgc cacacttcta cgt 173

<210> 181
<211> 340
<212> DNA
<213> Homo sapiens

<220>
<221> misc_feature
<222> (167)..(167)
<223> n is a, c, g, t or u

<400> 181
ggtaactttg gccaagactt ttcagtagga aatgcttcaa aatacaaagc aagagctatt 60

ttcaagaaag accttctaaa tttatattag gacatagtga gaagaaagcc atctgaaaac 120

caggaagaga gccctcacca gaatctgacc atgctggtgc cctgatnctt ggactttcag 180

cctccagaac tgcaaaattc tggtgtggtg tgaatgctgt ggctcagtcc gaacatgttt 240

ttttctgtaa ttttatcatt attacacgat tgcaatatca gttttgtttt ttaattggaa 300

agcaacattt tctactgttg aaagacgttt tttgacaaat 340

<210> 182
<211> 416

<212>  DNA
<213>  Homo sapiens

<400>  182
acagcttgtc tgtcacagtg cctgttctga ttgcaggctt tggtgttctc ctggtgttaa      60

tcctgacttt tttcctagtg atccaccctc tgggaaactt ctggctaatt cttagcgtca     120

cctcaattga gctgggcgtt ctgggcttaa tgacattatg gaacgtcgac atggattgca     180

tttctatctt gtgccttatc tacaccttga atttcgccat tgaccactgt gcaccactgc     240

ttttcacatt tgtattagca actgagcaca cccgaacaca atgtataaaa agctccttgc     300

aagaccatgg gacagccatt ttgcaaaatg ttacttcttt tcttattggg ttagtccccc     360

ttctatttgt gccttcgaac ctgaccttca cactgttcaa atgcttgctg ctcact         416


<210>  183
<211>  503
<212>  DNA
<213>  Homo sapiens


<220>
<221>  misc_feature
<222>  (78)..(78)
<223>  n is a, c, g, t or u

<220>
<221>  misc_feature
<222>  (84)..(84)
<223>  n is a, c, g, t or u

<220>
<221>  misc_feature
<222>  (210)..(210)
<223>  n is a, c, g, t or u

<220>
<221>  misc_feature
<222>  (247)..(247)
<223>  n is a, c, g, t or u

<220>
<221>  misc_feature
<222>  (371)..(371)
<223>  n is a, c, g, t or u

<400>  183
aggccgggct cagaggcgga gaagcctgcc tggtgcccac agccgtctgg ctcagggact      60

ccaccctggc cccgagtngc cgtntgctgg gcctttcctt cctggctctg caccccatgc     120

tggctgcccg gtctggcttc ccttcttgtc tctgtcttgg gcgaggcagc tgtgagcatt     180

gcacagaggc aaagaccctc ctgcagcctn tgcgctgggc cgtagaaaca agagcctttg     240

taatacngaa cctcattcaa ggattaggag tggtggttag gtcagggcca cccccagtgc     300

tgcaggaacg gcctccaccc agctctgttg gtcagagcct gggtcatgca cctggagttg     360

ggagatcaag ntgggtctca gggcagtgag gtggccatat ccaccacatc gcatttcgtg     420

ggggaagagg tgacctcttt gttttaaact taaggtgtct gcttatccag ccagaaataa     480

aaatctgcca gtggtgttcc caa                                             503

<210>  184
<211>  377
<212>  DNA
<213>  Homo sapiens


<220>
<221>  misc_feature
<222>  (26)..(26)
<223>  n is a, c, g, t or u

<220>
<221>  misc_feature
<222>  (294)..(294)
<223>  n is a, c, g, t or u

<400>  184
gagtcccgtc tcagtgtgga ggaacnggct gcacatggga cctgaaggtg ccctctgtgt      60

ttatgttggg ggtggggggg cagtgctggc tgcctctgtc ctgtgtgtga ccctaccctc     120

gaagggtcct gtcctgtcag tcccgaggga gccacaacca aagctgcgga gagaaggtgg     180

ggaagggtgc ggaatggccg tggggcacag cgtggcagac tgttcagtct ctgctgggtc     240

tttcctaggg acctggaagg ccagtgttgc ttccccctca ctccctttca ctgnaggcag     300

cctctctgct tccccaatgc cttatgcctg ggcacactgc cacagaatat gcaatatgtg     360

tgggtgacca tgccctc                                                    377


<210>  185
<211>  390
<212>  DNA
<213>  Homo sapiens

<400>  185
gtcatcctgt gctcagttag cagctcatcc agctgggtca ggaaagcctt ttggaagcgt      60

aggaccttgc cagccagcgc tgggatatgc aggaggacgg ggacagcatt cagcacctcg     120

cgcagaaagc ccgactcctc cttcagtccc tcctgagcta ggtccagcag cctgaggaag     180

cgagggtcgt cgtactcgaa gcggcgcccg caggtgaggg aggcgatcac gttgctcacg     240

gctttgtcca agagaccgtt ggggcgaaag gggcgtcgga gtggttggcg aaggcggcac     300

aaaggcaggc ggcctcctcg gtcacccact gctccagcga cttcttgccc aggcccaagt     360

tgcgcaaggt ggagacggag aagcgcctct                                      390


<210>  186
<211>  188
<212>  DNA
<213>  Homo sapiens

<400>  186
ggctggcaac ccagaaagat tggatttcag tgccatggtg ctggctgcgg agagcttcac      60

ctcagggagg cactactggg aggtggacgt ggaaaaggca accaggtggc aagtgggcat     120

ataccacggc tctgcagacg cgaagggcag cacggccaga gcttccggag agaaagtctt     180

gctcacgg                                                              188

<210>  187
<211>  549
<212>  DNA
<213>  Homo sapiens


<220>
<221>  misc_feature
<222>  (213)..(213)
<223>  n is a, c, g, t or u

<400>  187
taggaatgga gcccgagcag tctcgctctc agggccctgt gtggagtcac tgtgctgtcc      60

cagctctgga gacgcagaat tccacatgag gaatgtggaa ttcagcatgg ggatgacgct     120

gcttcaccca gacttggagg agcgtggtga attgcccgtg cccatgctct gatgtgcctc     180

tctggccgct gcgttcctcc tttctccctg ccntgggtca gtgcctgtaa acactgccct     240

aaatcagcag ggcccccgtc acttctgctt tatgcacctt tttcctcaga cacattaata     300

caggggagtt ttgtttccaa gggaccacat ccagatggag gggctgtttt tggtgatctg     360

cactgccaaa tgcccgagtg tccctgacag tcggagctga tgaggccaag gctgtgtgtg     420

gttcctctgg atggccagaa gaggaaccaa aacactgaat tctgggcctt cttaagagtg     480

gtgatcagca cattgtgata gaagcatatc tgggaatgaa cttggcctca agcttttggc     540

cttttaatt                                                             549


<210>  188
<211>  459
<212>  DNA
<213>  Homo sapiens


<220>
<221>  misc_feature
<222>  (120)..(120)
<223>  n is a, c, g, t or u

<400>  188
ctactctctg tctccataaa ctggtctatt ttggacattt cacataagcc tccctggatc      60

ccagtttaag catcctgggg tttgtctgcc tgccagagcc atggtgccac tggggctacn     120

tgtcctgtgg gatgacaagg caggtccaaa cctttgcctg ctctcccatc cattcctttt     180

gtgttagtcc atgtgtctcc cgactgttct ctccaacaac aacacagact gacaaaacct     240

actgacttgg agtcaggaac agactttgct attttctggc tgtgtgatcc tgatgagtcc     300

cttgaacctc ctggacttgt tcctcagcct aaaaaccaag actaataaat caagtctatc     360

tcacagcctt acgtggggat caaaaaacat ggagcatgtg aacacacatt gtacatcacg     420

aagctgtgtg caaataaata tcgtgtaact ccagcccttt                           459


<210>  189
<211>  430
<212>  DNA
<213>  Homo sapiens

<220>
<221>  misc_feature
<222>  (112)..(112)
<223>  n is a, c, g, t or u

<400>  189
gcccgaggcc tgctgagaag catgggggcc ttgggatagt tccagaatga ggatgtgcgt      60

ttctagctgc tttgcgccct cctcccccaa aaatctgcta ccacaattcc ancccggcgg     120

cacgccccca agactccttt gtcgccccag gggcgggacc tgagctgtcg gtttcaggag     180

cccttcgtga cttcaaaagt cctgggcact gttgctcatg agtgctgcac aactgtcgcc     240

ctctaaagcc acctccatcc ctcactgggc tggcctcctg agccttcggt gaggaaacgg     300

ggttccgagt tgcccgcctg agagcttaac agtctgacta gaaaagggct aattcgcttt     360

ctgtgcaaat ctcttgagct aattatttaa tctgaaacat ggacaggtaa aggaccattg     420

gcgggcgtgg                                                            430

<210>  190
<211>  406
<212>  DNA
<213>  Homo sapiens

<400>  190
acatcaagca gctttctcgc tttgctggag cttcaagtaa gattgctcca gtggaagcac      60

cagatgctaa ggtgaggatg gtgatgatcg ctggatcacc agaggctcgg ttcaaggctc     120

agggaagaat tatggaaaaa tgaaagaaga aaacttcgtt agtcctaaag aagaggtgaa     180

acttgaagct catatcagag tgccatcctt tgctgctggc agttactgga aaaggaggca     240

aaacggtgaa tgaacttcag aatttgtcaa gtgcagaagt tgttgtccct tgtgaccaga     300

cacctgatga gaatgaccaa gtggttgtca aaataactgg tcacttctat gcttgccagg     360

ttgcccagag aaaaattcag gaaattctga ctcaggtaaa gcagca                    406

<210>  191
<211>  555
<212>  DNA
<213>  Homo sapiens

<400>  191
aatgctgtca gcccttaggc aagactaaat tggaaagaaa ggtgtctgcc aaagaaaaca      60

ggcaggcccc tgtcctcctt caaacataca gggaatcctg gaatggagaa aacatagaat     120

cagtgaaaca aagccgtagt ccagtttctg tgttttcctg ggacaatgaa aagaatgaca     180

aggactcctg gagtcaactt ttcactgaag attctcaagg ccagcgggtc attgcccaca     240

acactagagc tccttttcaa gatgtaacca ataactggaa ttgggactta gggccgtttc     300

ctaacagtcc ttgggctcag tgccaggagg atgggccaac tcaaaatctg aagcctgatt     360

tgctctttac ccaggactct gaaggtaatc aagttatcag acaccaattc taaatgtttg     420

aagctttgtt tctaaaagta ccttgaaatg atagagatgt aggaaaatat agttgtgggt     480

ggagagagga gtgagtttgt ttaggtggga aggtggcatg ggatgaagtt gtcattactg 540

agcatcttct ctgtg 555

<210> 192
<211> 554
<212> DNA
<213> Homo sapiens

<400> 192
gccctgctca gaggtcagag ggtctgggca gaggagggac cacattcccc tgccttgccc 60

ctgagcactt ctggagactg cgtcctgtcc tatctgctca ccatcaccct tcctgcccga 120

cggagctgct tctgctccct ggggcatatg gactgaccca cctcctgctg agaaccttcc 180

cctaggccct gtgcagaagg gctactgccc cttaggcctc agctggggga aaggcagttc 240

tggtgctgta gaggccctgg tgcagaaagt gggacgtctt ttttcctaag gtgtttaagc 300

acaggcttga taagtttggt ttttaaaaaa taatctagga aatgaataat tctaaatcta 360

gtaatgagga aactgagcat ttcttttgcc ctccagggtg ccaagaccct acatatgaca 420

gaacccttgg cccttctcca tgcctgtggg atctgtttct ttaaagcact ttgtactgtt 480

attcaggagg ttgataatct ccttgaccca tgtctttcta ccctaatccc cacttccctg 540

cagaatcaat ctga 554

<210> 193
<211> 319
<212> DNA
<213> Homo sapiens

<400> 193
   acgcgtccaa catctcaaac ttgatctcca tctttggctc cggcttctcg gggctggtga 60
   gccgacagcc ggactcctcg gagcagccgc cgccgctcaa cgggcagctg tgcgccaagc 120
   aggcgctcgc cagcctcggc gcctggactc gagccattgt cgccttctag ggacccccga 180
   gggcacaggg acccggggcc ccgcggggct ggggccagac aaagactcgg caaaggggcg 240
   agaggaggga acgagcgggc gccgggccac tcggggctga gctgggggcg agcgggggca 300
   ggcggctgat gttttataa 319

<210> 194
<211> 218
<212> DNA
<213> Homo sapiens

<400> 194
gaagactttc taaataatga taatcagagc tgtactctct ctggaggcaa acatcatggt 60

cctgttgaag ccctgaaaca aatgttattt aaccttcaag cagtacaaga acgttttaat 120

caaaataaga ccacagatcc aaaagaagag attaaacaag tttcagaaga tgatttctct 180

aaattacagt tgaaggaaag tatgattcct attactag 218

<210> 195
<211> 246

<212>  DNA
<213>  Homo sapiens

<400>  195
ccccacccaa atacaagtcc cagtggaaag gaaaggtagt acctattctt ctccatgggg      60

ttcctaacac cctccattac tctttcagtc tccaagcact ttgaatccat ttttaaacat      120

tcaggttgcc agacctgtca cacagtgggc tctgataggg ttacggaggg ggcctggctc      180

tcagtctcta ctctcctatg tcccatcagt tggttggagg ccaccttcca gggggtatgg      240

gagaca                                                                 246

<210>  196
<211>  283
<212>  DNA
<213>  Homo sapiens

<400>  196
caccttgacg gttccagtgt ctgtatttat gttgaaagtc caggtgaatg acatcatcag      60

tcgtcagtac ctgagccaag cagttgtaga agtgtttgta aactacacga agacaaattc      120

cacagtaact aaaagcaatg gagcagtgct gataaaagta ccctacaaat taggacttag      180

tttaactatt attgcttaca aagatggcta cgtgttgacc cctctgcctt ggaaaaccag      240

aagaatgcca atatattcat cagttacact ttcactgttc ccg                        283

<210>  197
<211>  391
<212>  DNA
<213>  Homo sapiens

<400>  197
cgtccgagtg tgagtcagtc agcgacaagg ctcccagccc tgccaccctg ccagccacct      60

cctcctccct gcccagccca gccaccccat cccatggctc tcccagttcc catgggcctc      120

cagccaccca ccctacctcc cccactcccc cttcgacagc cagtggggcc accacagctg      180

ccaacggggg tagcttgaac tgcctgcaga caccatcctc caccagcagg gggcgcaaga      240

tgactgtcaa cggcgctccc gtgcccccct taacttgagg ccagggaccc tctcccttct      300

tccagccaag cctctccact ccttccactt tttctgggcc cttttttcca cctcttctac      360

tttccccagc tcttcccacc ttgggggtgg g                                     391

<210>  198
<211>  563
<212>  DNA
<213>  Homo sapiens


<220>
<221>  misc_feature
<222>  (116)..(116)
<223>  n is a, c, g, t or u

<220>
<221>  misc_feature
<222>  (165)..(165)
<223>  n is a, c, g, t or u

<220>
<221>  misc_feature
<222>  (168)..(168)
<223>  n is a, c, g, t or u

<220>
<221>  misc_feature
<222>  (175)..(175)
<223>  n is a, c, g, t or u

<220>
<221>  misc_feature
<222>  (225)..(225)
<223>  n is a, c, g, t or u

<400>  198
agaggcaggc atagaggctt ctccgccagc ctcctctgga cggcaggctc actgccaggc      60

cagcctccga gagggagaga gagagagaga ggacagcttg agccgggccc ctgggnttgg     120

cctgctgtga ttccactaca cctggctgag gttcctctgc ctgcnccngc ccccnagtcc     180

ccacccctgc ccccagcccc ggggtgagtc cattctccca ggtanccagc tgcgcttgct     240

tttctgtatt ttatttagac aagagatggg aatgaggtgg gaggtggaag aagggagaag     300

aaaggtgagt ttgagctgcc ttccctagct ttagaccctg ggtgggctct gtgcagtcac     360

tggaggttga agccaagtgg ggtgctggga ggagggagag ggaggtcact ggaaagggga     420

gagcctgctg gcacccaccg tggaggagga aggcaagagg gggtggaggg gtgtggcagt     480

ggttttggca aacgctaaag agcccttgcc tccccatttc ccatctgcac cccttctctc     540

ctccccaaat caatacacta gtt                                              563


<210>  199
<211>  591
<212>  DNA
<213>  Homo sapiens


<220>
<221>  misc_feature
<222>  (60)..(84)
<223>  n is a, c, g, t or u

<220>
<221>  misc_feature
<222>  (122)..(146)
<223>  n is a, c, g, t or u

<220>
<221>  misc_feature
<222>  (182)..(200)
<223>  n is a, c, g, t or u

<220>
<221>  misc_feature
<222>  (453)..(478)
<223>  n is a, c, g, t or u

<220>
<221>  misc_feature
<222>  (495)..(536)
<223>  n is a, c, g, t or u

<220>
<221>  misc_feature
<222>  (540)..(554)
<223>  n is a, c, g, t or u

<400>  199
   ctggagagcc agtgcccatg gcccgctgcg tctccacagg gggtcgcccg ccagcccaan      60
   nnnnnnnnnn nnnnnnnnnn nnnnggatgc ccaatacgag ccaggtgcca gggttcctgt     120
   cnnnnnnnnn nnnnnnnnnn nnnnnntgga tattggtgcc ctcaagccag gtggacggca     180
   annnnnnnnn nnnnnnnnnn gagcacgaga gctttgagaa gcctcagctg ctgactgtga     240
   acctcaccgt gtactacccc ccagaggtat ccatctctgg ctatgataac aactggtacc     300
   ttggccagaa tgaggccacc ctgacctgcg atgctcgcag caacccagag cccacaggct     360
   ataattggag cacgaccatg ggtcccctgc caccctttgc tgtggcccag ggcgcccagc     420
   tcctgatccg tcctgtggac aaaccaatca acnnnnnnnn nnnnnnnnnn nnnnnnnntg     480
   ccctaggagc tcgcnnnnnn nnnnnnnnnn nnnnnnnnnn nnnnnnnnnn nnnnnnagcn     540
   nnnnnnnnnn nnnncatgtc tcctattcag ctgtgagcag agagaacagc t              591

<210>  200
<211>  485
<212>  DNA
<213>  Homo sapiens

<400>  200
catcagattt ctgcagatct gctttaaagc tgtacatttt tgttacagtc taagatgtgt      60

tcttaaatca ccattccttc ctggtcctca ccctccaggg tggtctcaca ctgtaattag     120

agctattgag gagtctttac agcaaattaa gattcagatg ccttgctaag tctagagttc     180

tagagttatg tttcagaaag tctaagaaac ccacctcttg agaggtcagt aaagaggact     240

taatatttca tatctacaaa atgaccacag gattggatac agaacgagag ttatcctgga     300

taactcagag ctgagtactg ctccagggtg gtgtgcaatc ttatattgat gcttgtgaat     360

ctgccatttg atttgtagga taaataaata tgtttaatat taacaacttc catcaaaact     420

ataataataa tattatatct actgttgacc tctaacaaca atcaggtgct gtattcagag     480

tcata                                                                 485

<210>  201
<211>  432
<212>  DNA
<213>  Homo sapiens

<400>  201
gccctgactg actgtattct ctggccacat tcaagtcccc cattggtggg ggcagagaag      60

taggaccagg ccatccttgg ctccagagct cgaagacccc aagacagccc tctgctctca     120

gcggcgccac agagagcctg ggctcagcct tctgcatcag gacatggcct cgtccactga     180

gggcacgatt taaacatttg acatcagaag ctttatttgt aaacctcaca cagataagga     240

ccaagggctg gcggtgtggc cagaggacag gggaagctga aggccccgtg cttgagctcg     300

gcagtcctgc tccttgcagt gaagccacca tgggtgaccg tccagcctca cccggtggcc 360

tgcacagtga gggaagggct tcagggccat ctgctcccag ggcaggggac aggccaccaa 420

ggacctttgg ca 432

<210> 202
<211> 499
<212> DNA
<213> Homo sapiens

<220>
<221> misc_feature
<222> (425)..(425)
<223> n is a, c, g, t or u

<400> 202
ggtggagaag ctggcgcgtg agaacagcag catgcggctg gagctggacg ccctgcgctc 60

caagtacgag gcgctgcaga ccttcgcgcg caccgtggcc cggggacctg tggcgccctc 120

caaggtggcc accaccagcg tcatcaccat cgtcaagtcc accgagctct cctccacctc 180

cgtgcccttc tcggctgcat cctagtgccg gccgggggcg gggggtggcg ggcggcgggc 240

ggcgggcagg cgggtggggg cacacccctc gtacctgtca ctgggatgca gactctcgac 300

atccgagtcc aagcgcaggc ccctcgggcg caggcagctc acaccaggaa gagactgtat 360

tgcagggtga agagtgggct cccgtgggcc cagagctgca cgccggtcca cagacacact 420

cacgnccgcc acctgctccc cgcagatgtg tctgtgtgtg ggaattggta tcttgcaccc 480

gtgggagtcg ggacatata 499

<210> 203
<211> 569
<212> DNA
<213> Homo sapiens

<220>
<221> misc_feature
<222> (107)..(107)
<223> n is a, c, g, t or u

<400> 203
ttccagcacc attcttttcc tattaaatta cactggcaaa tttgattaaa aaaaacaact 60

gactatatat gcttgtaaac atttccagat tatgttattc ttttaancta aatatgtgtc 120

cttatgccaa taccccactc catctattac tgcagtgtat gataagtctt gaaatctagt 180

agtgtaagtt cttcaacgtt gcccttaatt tttaaaatca ctcttgctat ttaaaattgt 240

ttgtattaca tggaaatttt ataatcagct tgccaatttc tacaaaagtc ctgctgagat 300

tttaattggt attttgcttg ttctgcagct taatgcaaga aaattatctt aacaatattg 360

aatttttcaa tctattaaca tgttatatat tactgtttac ttaggatttt ttcacttttc 420

ctgccttgtt ttgaactgat attgtggttt taagtaattt tttttatttc tactattggc 480

ttagtaacta tgccccactt tttgattttg tagcacagtt gaccattgaa caacacaagt 540

ttgaattgtg catgtccaat tgtctatgg 569

<210> 204
<211> 266
<212> DNA
<213> Homo sapiens

<400> 204
ggagcagaga cagagcgacc catacctggc ccaggccccg gccccgcagg cagctgaatt 60

cctgagccca gtgacaaccc cttccccctg cactctgtcg tccgcccaag cctcaggccc 120

tgaggctgca gatgagactt gtccccagct ggctgtccat cctcctggtg tcagcaagct 180

gggtttgcag tgtcttccaa gcgacggtgt tcagaatgtg aaccagtgac tctcgggcgc 240

ccctgtggta actttgcagg cggccc 266

<210> 205
<211> 506
<212> DNA
<213> Homo sapiens

<220>
<221> misc_feature
<222> (41)..(41)
<223> n is a, c, g, t or u

<220>
<221> misc_feature
<222> (99)..(99)
<223> n is a, c, g, t or u

<220>
<221> misc_feature
<222> (103)..(103)
<223> n is a, c, g, t or u

<400> 205
gcaagagctt tatccagagc tcccacctga tccgccaccg nccgcatcca cacgggcaac 60

aagccgcaca agtgtgcggg ctgcggcaaa ggcttccgnt atnaaaacgc acctcgcgca 120

gcaccagaag ctgcacctgt gttaggggct gggtccgcgg gaggctgccg tctggggagc 180

ctgtgggggg tagatatcct gggactgacc caggggaagg aagtggggaa ggggcgggag 240

ggacaatctg agagtgactg gggagccttt ggtgtttggg gtttcctgaa gtgggaggag 300

tgttgagtaa gttggtcttt cccggtgcta tacttgcctc ctctccacgg aagaattgtt 360

caggagatgc gcttggggtg atgacttcct taaatacacg ctgtaggggg tgaagagctt 420

ggaggaccag gcactttgag gaagggcagt tcgtgggctg gggtgggaac aggatggcgg 480

gcaatagact agggtaggcc gcgatg 506

<210> 206
<211> 439
<212> DNA
<213> Homo sapiens

<220>
<221>  misc_feature
<222>  (53)..(53)
<223>  n is a, c, g, t or u

<220>
<221>  misc_feature
<222>  (56)..(57)
<223>  n is a, c, g, t or u

<220>
<221>  misc_feature
<222>  (60)..(62)
<223>  n is a, c, g, t or u

<220>
<221>  misc_feature
<222>  (64)..(64)
<223>  n is a, c, g, t or u

<400>  206
tcatttagcc ggtgtccact aactcagtgt tgtgggccat ttgtaaaccc ttntgnngtn      60

nncnccaggc agacgtaggg aaagaaagag aggatctgta tagacaagaa agctggccat     120

gtgggaagtc cagagctcaa accatgtgcc ccagaggact ggtgctggca ttaagcctgt     180

aaatcaaagg cttctttggc aggaccctgg gctgttagaa tcaccctagg gagcagagcc     240

aggggacatt ttggcccctg actagcaagg cacaacccta taatggcaga agcccttctt     300

tcccctcccc gtttcccacc agacccactt ccttgatggg cctctagcac ccttccaagc     360

tgatggggtc gggaatgtga gctggtaaaa tgggcagtgg aaggggctgt actgtttctt     420

tacatctcac ggggactag                                                  439

<210>  207
<211>  375
<212>  DNA
<213>  Homo sapiens

<400>  207
aagaaatgct acctcggtgc catgttctgg actccgcaga acaaggactt tttggagaac      60

tccagcctat accctctatt gccatgacca gtacttcagc cactctggtg tcatctcagg     120

ctgatctccc tgaattccac ccttcagatt caatgcaaat caggcactgt tgcagaggtt     180

ataaacatga gataccagcc acgaccttgc cagtaccttc cttaggcaac caccatactt     240

attgtaacct gcctctgacg ctactcaacg gacagctacc ccttaataac accctgaaag     300

atacccagga atttcacagg aacagttctt tgctgccttt atcctccaaa gagcttagct     360

ttaccagtga tattt                                                      375

<210>  208
<211>  502
<212>  DNA
<213>  Homo sapiens

<400>  208
gttcttgagt acatagccaa tgccaatggg agggatccca cttcttaccc atccctgtat      60

gaagatgctt tgagagagga gggagaggga gtctgagcat gagatgcaac cagggccagc     120

gggcagggaa atgggccaat gcatgcttca gggccacacc cagcagtttc cctgtcctgt    180

gtgaaatcag gcccattctt ccctctgtgt ttgatgagag aagtcagtgt tctcagtagt    240

agaaggcaca gtgaatggaa gggaacacat tgtatactgc ctttaggttt ctcttccatc    300

gggtgacttg gagatttctt tttgtttccc tttggtaatt ttcaaatatt gttcctgtaa    360

taaaagtttt agttagcttc aacatctaag tgtatggatg atactgacca cacatgttgt    420

tttgcttatc catttcaagt gcaagtgttt gccattttgt aaaacatttt gggaaatctt    480

ccatcttgct gtgatttgca at                                              502

<210>  209
<211>  250
<212>  DNA
<213>  Homo sapiens

<220>
<221>  misc_feature
<222>  (110)..(110)
<223>  n is a, c, g, t or u

<220>
<221>  misc_feature
<222>  (112)..(112)
<223>  n is a, c, g, t or u

<220>
<221>  misc_feature
<222>  (114)..(116)
<223>  n is a, c, g, t or u

<220>
<221>  misc_feature
<222>  (118)..(119)
<223>  n is a, c, g, t or u

<220>
<221>  misc_feature
<222>  (121)..(122)
<223>  n is a, c, g, t or u

<220>
<221>  misc_feature
<222>  (124)..(124)
<223>  n is a, c, g, t or u

<220>
<221>  misc_feature
<222>  (127)..(127)
<223>  n is a, c, g, t or u

<220>
<221>  misc_feature
<222>  (129)..(129)
<223>  n is a, c, g, t or u

<220>
<221>  misc_feature
<222>  (131)..(132)
<223>  n is a, c, g, t or u

<220>

<221>  misc_feature
<222>  (135)..(135)
<223>  n is a, c, g, t or u

<220>
<221>  misc_feature
<222>  (145)..(145)
<223>  n is a, c, g, t or u

<220>
<221>  misc_feature
<222>  (152)..(152)
<223>  n is a, c, g, t or u

<220>
<221>  misc_feature
<222>  (154)..(154)
<223>  n is a, c, g, t or u

<400>  209
tcccctagct tggggtccag acagcccagt ggacccaggc gcctgagcag gagggtaacc      60

caggccaccc ggccccttcg gccctctcgg ccccaccccc tgcagccggn gncnnncnnc     120

nncnacnana nngcngcgag aagangacag angngactga gcaaaggggg gtgggctcca     180

ggcgacccct agcccaattc tgcccctcca tcccaagggg cagagaaatt gtctttcttt     240

gctgactcct                                                            250


<210>  210
<211>  440
<212>  DNA
<213>  Homo sapiens


<220>
<221>  misc_feature
<222>  (142)..(144)
<223>  n is a, c, g, t or u

<400>  210
tttggacatg tccattttgg aagaaacttt tgtgttaaaa taaactaata tattatgggc      60

tagaacataa aattcaccaa gaatttcaag ataaaaatac taatgttttg cttgtttggg     120

ttatttcaaa caataacttt gnnntctata attttttcac caccgaccct ctacctcctt     180

gcatgctcat tctcctgtgt ggctagatgc atttcgggtg ttttgaatat tatttcagag     240

caagtatcat tccagaaaat aagtttaaag tttgaaatgt ttattttttg taacccatga     300

atcttcagct taagtatctt ctgacataaa agcattttca taattataaa agtgctgata     360

ttactctcca cagtattata tctgatcctg caaagtagtt cagataccag agaatactct     420

taaacatttt gactcacgca                                                 440


<210>  211
<211>  573
<212>  DNA
<213>  Homo sapiens

<400>  211
ggactcaggg agtacacact taccagtgcc cttaaagata gccgttttcc cccaatgaca      60

agggatgagc tgccacggct tttctgctca gtgtctctgc tcactaactt tgaagatgtc 120

tgtgattatt tggactggga ggtgggtgta catggcatta gaatagaatt catcaatgaa 180

aaaggatcaa aacgcaccgc cacctaccta ccggaggttg caaaggagca aggatgggac 240

catatacaga ccatagactc cttattgagg aaaggaggat acaaagctcc gattactaat 300

gaattcagga aaaccataaa actgaccagg tatcgtagtg aaaagatgac cctgagctat 360

gctgaatacc ttgctcatcg ccagcatcat catttccaaa atggcattgg gcatcccctt 420

ccgccataca accattattc ctgacactga gccgcacaac cagtcactgg gcctctctgc 480

agacctcttc ccaggagacc ctacaccttc ttggtctagc tatctctttt actgtaccat 540

tttatgatga tagtttccgt tgccatggtg aag 573

<210> 212
<211> 514
<212> DNA
<213> Homo sapiens

<400> 212
cgtccttgtc atatcctttt aactaggcat ctcagagaag cagagacagg gcagccttcg 60

tcctggggga aaagggaccc tcaggatggc atgagaggtc ctcaatccca agtgtggaac 120

tgtccccctc aacttgttaa aatgcagatt tctgggtctt gccaatgggg cctgggactc 180

catgtgacaa ctggcccagg agcttctgat gtcacacaga attctgcagt cccaagctcc 240

agccccgacc tgctctgctg ttcctaggtg actgccctca cactgctgac cacagtggat 300

ttctccccct gctgctcggg ctcagctggg gtcagccctg cttataaggt caactgtgca 360

aaaccttata ctggccaaga acaaactagt gctgggggag gagggctggg tgccccggcc 420

actggtggag tccccaggaa atcctcagag ctgttgcgag gatgagacac atttgtggac 480

acgtccacct gtcctcctga ccgtctggag agaa 514

<210> 213
<211> 504
<212> DNA
<213> Homo sapiens

<400> 213
ccggctatgg gctcgagccg agttccttca acatgcactg cgcgcccttt gagcagaacc 60

tctccggggt gtgtcccggc gactccgcca aggcggcggg cgccaaggag cagagggact 120

cggacttggc ggccgagagt aacttccgga tctacccctg gatgcgaagc tcaggaactg 180

accgcaaacg aggccgccag acctacaccc gctaccagac cctggagctg gagaaagaat 240

ttcactacaa tcgctacctg acgcggcggc ggcgcatcga gatcgcgcac acgctctgcc 300

tcacggaaag acagatcaag atttggtttc agaaccggcg catgaagtgg aaaaaggaga 360

acaagaccgc gggcccgggg accaccggcc aagacagggc tgaagcagag gaggaagagg 420

aagagtgagg gatggagaaa gggcagagga agagacatga gaaagggaga ggaagagaag 480

cccagctctg ggaactgaat cagg 504

<210> 214
<211> 529
<212> DNA
<213> Homo sapiens

<400> 214
gaaattattc actccgtata ctgaaacaga aataaacgag gaagaactta caaagccaag 60

actcttgtgg gctctttatt ttaatatgag agattcctcg ggaatcagca gaagctcgta 120

taatggcttg ccttccaatg tttatgtctg ctctgggcct gactgtggcc tgggaaatga 180

gcatgctgtc aagcaagctg aaacactttt ccaggagatc tttccaactg aagaattctg 240

ccctccacct ccaaatccag aagacattat ctttgatggt gatgataagc agccagaggc 300

tcctggaacc aataatgtag taatggccaa actagaatcc tctgaggaaa gcaaaaacct 360

agaaagccca gagaagcacc ttcaaaatta gaaaagagca atctcgaaat gctgttttgg 420

acctccttca tggcatcaga attttctcat ttaaaggaca gtttcccata tgagtaatta 480

gaagtggtta tatatgatga atgctatgca gatgttgtct ttaactctc 529

<210> 215
<211> 480
<212> DNA
<213> Homo sapiens

<400> 215
tctttgctct agtattccac ggtgcctctg acatgagaac aggatggaga ctggcttctg 60

atttgacatg cattttgtag gtatgatcca aaatagcttg gaaactatcc cagtcttcaa 120

ccatcccatt ttttagaggt gaaatggcct ccatattctc cctcggaaca cgcagagcat 180

tagtatctat gtagtaggtg ggaccgcctt gtttgccttt atcgccatct atttccatta 240

atgtgcttcc gtcatctctt tctaccacca taccaatagc tgtaggaaaa tccaccttgg 300

ggcagtcctc accagcataa ccagctctca cagtatagga tccaatgtca aaaacaaggg 360

ctccaacttc atctcccccg taccacgccg ccgctcatgg ctgctgccgg cgcgactcct 420

accctaaggg ctaactggcg aagtgactgc agtggccgcg actgcgagtc tcgaggagcg 480

<210> 216
<211> 282
<212> DNA
<213> Homo sapiens

<400> 216
tggaagcatt tgttgcctcg atcttccact ttagaaaaat gaagtttctc cttttctttg 60

ggagaggata tatctgaata cttgccttct tggcatttat acattcaaag ctcagtgcta 120

gattagagct attatttgca tagtcttttg gtattgccca cttttggcat taccatatta 180

tttgacaatt agaaggaata gggaaggaat attacatgac tgtaaaagag ttggttatat 240

tttatgttga cttcaagggt tccatttgaa ctattatggg ca 282

<210> 217
<211> 563

<212>  DNA
<213>  Homo sapiens

<400>  217
gcaggaccac cttgaattct gccctgacac actggattgg agagcagcag aacccagggc      60

ctggcccacc aagctggagt gggaaaggca caagattcgg gccaggcaga acagggccta     120

cctggagagg gactgccctg cacagctgca gcagttgctg gagctgggga gaggtgtttt     180

ggaccaacaa gtgaccactc tacggtgtcg ggccttgaac tactaccccc agaacatcac     240

catgaagtgg ctgaaggata agcagccaat ggatgccaag gagttcgaac ctaaagacgt     300

attgcccaat ggggatggga cctaccaggg ctggataacc ttggctgtac cccctgggga     360

agagcagaga tatacgtgcc aggtggagca cccaggcctg gatcagcccc tcattgtgat     420

ctgggagccc tcaccgtctg gcaccctagt cattggagtc atcagtggaa ttgctgtttt     480

tgtcgtcatc ttgttcattg gaattttgtt cataatatta aggaagaggc agggttcaag     540

aggagccatg gggcactacg tct                                             563


<210>  218
<211>  391
<212>  DNA
<213>  Homo sapiens


<220>
<221>  misc_feature
<222>  (100)..(100)
<223>  n is a, c, g, t or u

<220>
<221>  misc_feature
<222>  (112)..(112)
<223>  n is a, c, g, t or u

<400>  218
gccagacaac ctgagtgtga atccagcttc accacttcat tcattcactc acccattcat      60

tcaacaacat atttgaagca catactttgt accagggacn tttccaggca cnggactaca     120

gctatgaaca agacaaacag tccctagcct cccaagagcc gtcacttcag aagggcagac     180

atgacacgca aacaaaatga tgccaggtgg taccaagtgc cttggggaaa cagtgccacc     240

tttctgagac cgtttctcca tccgtccatg gagctgataa caccagtccc tcagggtgga     300

ggtgaagact aagaggttgc tttgagaggg ggaacttggt ggcttttttt caccacctag     360

aacctggcac atactaagct ctcaataaaa g                                    391


<210>  219
<211>  474
<212>  DNA
<213>  Homo sapiens


<220>
<221>  misc_feature
<222>  (417)..(417)
<223>  n is a, c, g, t or u

<400>  219
aactacgcct ggtacaagct ggcagaggag gtttctgggc gcacagaagt cactgtgaaa      60

cagccagaca gccgcctgag gctcagccaa gcccagggga acctgtcggt tctggagacc     120

cggcaggtac agctggagtg tgtggttctc aaccgcacca gcataacctc ccagctcatg     180

gtggaatggt ttgtatggaa gcccaaccac cctgagcggg agactgtggc ccgcttgagc     240

cgtgacgcca ccttccacta tggagagcag gcagccaaga acaatctgaa ggggcggctg     300

catttggaga gtccttcccc cggcgtgtac cgtctcttca tccagaacgt ggctgtgcag     360

gacagcggga cctacagctg ccatgtggag gagtggctgc ccagccccag tggcatntgg     420

tataagcggg cagaggacac cgctgggcag acagctctga cagtcatgcg acca           474


<210>  220
<211>  471
<212>  DNA
<213>  Homo sapiens


<220>
<221>  misc_feature
<222>  (125)..(125)
<223>  n is a, c, g, t or u

<220>
<221>  misc_feature
<222>  (133)..(133)
<223>  n is a, c, g, t or u

<400>  220
gggaccttgt aacttccttg caagttaagt gagctatcct gtcacggttt tatgttgagt      60

gagtgggaag ctgggactct gttttacagc catctgtact ggagcctgga caaaccactg     120

gtctntatgg gangccccag cctcacattt ccctggcaag gagagagagg tttagccatg     180

tcctgggtct aggattacag cccagagatg ggcacttaag aagacctggt cattggtcca     240

gacttgggcc aaggctctcc tctgtgaggg atgggtttta ctggtgaatt acctgtgtgg     300

agaagctatc agggccatgt ttagcacact gaagggacca gtctccacca agcactttaa     360

catccctcca gccagcatag attgatctcg tgttacagag agggcaaggt ttttggcccc      420

tgtttgcaga ctccatgtct taatcagaga ccacagtttt ctctttgttc c              471


<210>  221
<211>  527
<212>  DNA
<213>  Homo sapiens


<220>
<221>  misc_feature
<222>  (408)..(408)
<223>  n is a, c, g, t or u

<220>
<221>  misc_feature
<222>  (439)..(439)
<223>  n is a, c, g, t or u

<400>  221
taaataatgt cctctacgtg ccggtgtgga agtagcccgg atgcaattga atgaacaaca      60

gacggtgctt tccaggacgg cgctgtgctt tccaggatgg tgctgtgctt tcattcattt     120

gggtagctcc tctgtgagcc tcccagcgcc gactgcagag cccccactct ccagcctgca     180

agaccccgaa attcaagcca cacaaagaaa ggaggagggg gccgttggca tttactgaac     240

cttataaaac tgtcagcaaa acagccctta ggcttggact ccctgctagc cgggttttac     300

ggtgctgaag tcagcatctt gattcagctg cataaataat ctcctgcagt cctgcaaggc     360

ctggggtagg agagggtatg gggaccaggg cactctgtaa gggctggnat aggaacccca     420

gggaataaga cagaccaant gcgggacttc agactccact gcagccggga tcgggttgtt     480

gttaatttct taagcaattt ctaaattctg tattgactct ctcatgc                   527

<210>  222
<211>  310
<212>  DNA
<213>  Homo sapiens

<220>
<221>  misc_feature
<222>  (43)..(43)
<223>  n is a, c, g, t or u

<220>
<221>  misc_feature
<222>  (110)..(110)
<223>  n is a, c, g, t or u

<220>
<221>  misc_feature
<222>  (115)..(115)
<223>  n is a, c, g, t or u

<220>
<221>  misc_feature
<222>  (189)..(189)
<223>  n is a, c, g, t or u

<220>
<221>  misc_feature
<222>  (236)..(236)
<223>  n is a, c, g, t or u

<220>
<221>  misc_feature
<222>  (252)..(252)
<223>  n is a, c, g, t or u

<400>  222
   atacatgtgg ttatcttttg ccctgttgtg atggataatt tgnaaagaag tgggtttatg      60
   tcaccttctc accttcttat aagaaagctc tgagaatggg catttttgtn ttttnttgtt     120
   gttgttgaga tggagtctgc cacccaggct ggagtccagt ggcgtgatca tacctcactg     180
   cagcttcanc ttcctgggct caagtaatcc tcccacccca gcctcccagg tagctngtac     240
   tataggtgtg cnccaccacg cccagcaaat ttttaaattt attatagagt gggaggcagg     300
   gtgcggtggc                                                            310

<210>  223
<211>  283
<212>  DNA
<213>  Homo sapiens


<220>
<221>  misc_feature
<222>  (169)..(169)
<223>  n is a, c, g, t or u

<400>  223
cactgtctgt gtgagtccat tcacttcaat accagagcca cctctttgtt tcctatttac      60

taagaagcca taccagcatg agatctcctt gatagtgtta aatcccactg tggaaagatt     120

gaaaaatatc tcccagcctt accagaggtt acgatctagt gtggaggcna aagacattga     180

gaagaaaaaa gcaggtgcct cctcctggct ctcctgttag gttaacataa tcataattcc     240

cctttgaaat gtctcccaca tttgcccttt aacttcctat tgc                       283


<210>  224
<211>  499
<212>  DNA
<213>  Homo sapiens

<400>  224
gacgactacg gtctggacaa ctttgacaca cagttcacca gcgagcccgt gcagctgacc      60

ccagacgatg aggatgccat aaagaggatc gaccagtcag agttcgaagg ctttgagtat     120

atcaacccat tattgctgtc caccgaggag tcggtgtgag gccgcgtgcg tctctgtcgt     180

ggacacgcgt gattgaccct ttaactgtat ccttaaccac cgcatatgca tgccaggctg     240

ggcacggctc cgagggcggc cagggacaga cgcttgcgcc gagaccgcag agggaagcgt     300

cagcgggcgc tgctgggagc agaacagtcc ctcacacctg gcccggcagg cagcttcgtg     360

ctggaggaac ttgctgctgt gcctgcgtcg cggcggatcc gcggggaccc tgccgagggg     420

gctgtcatgc ggtttccaag gtgcacattt tccacggaaa cagaactcga tgcactgacc     480

tgctccgcca ggaaagtga                                                  499


<210>  225
<211>  562
<212>  DNA
<213>  Homo sapiens

<400>  225
tcttctgtgg aggaatggca tcccaggcct tcacccctcc aggtcagccg tggctgccgg      60

ccaagatggc cgcgtgggca gcctcacatt ccttctcggc ttttggcccc atgtcctcgg     120

cactcaggtc tgcagttcag cccaagtgtt gagactcagg tatgcagctc agggcggcct     180

taattaaccc tcccatgggc ctgggcaccg cctgcgcctc atcaactctg ggctgctggt     240

tttgttcctg acgctgcagc ctgacactgt gggcgggggt gcagtttgcg atggaaggct     300

gcctccgaat cgaggaagcc ttgaccttgg gaggggcctg ccttttcgct gggcttgcct     360

ttctctgggc agcgttcgct cagcacttca gtgcggccga ttcccctggg actgaattca 420

caccagccac gacgacttcc cggctacttc acgttctcta tgtttgcagc tgttctttgg 480

tggcagaaaa agatgatttt tcttcccccc actcccattc ccttttgtta gtttctctcc 540

ctgaaccaca ttttgagctg ag 562

<210> 226
<211> 47
<212> DNA
<213> Homo sapiens

<400> 226
ttccagaatt tcttccgagg tagtatggtt ttcttcatag gataaag 47

<210> 227
<211> 523
<212> DNA
<213> Homo sapiens

<220>
<221> misc_feature
<222> (476)..(476)
<223> n is a, c, g, t or u

<400> 227
aggcagcgct gcggagagga gcggcagagt gggttgtctg ccgcaggcaa ccaggcaagt 60

gtgtcggggc tggggtgtga atgccagcct gtgagtcccg gaactatgtg ggtaccccta 120

cccctcacag aagccaaggg catggaggag gtccctccac agtgacaacg gtgtggggta 180

ggggaggtgc attcaggaca ccacccaggg acagtgccta tgtgatcacc tcttaaaggc 240

taagcttagg ggcatttccc aaagtgggga cagagggcag gacgcccagg ctgggggctc 300

tcctcgcccg ccctggtgtc tgacagcctc aaggaaggag cagtgcctgt gtcagccatg 360

gggcccttgg agctgccgct ggtgcctagg gggcctgggt ttctgcccag gcagccagtg 420

gctgttggga gcctctgttt cccctgtgct gggggccttg agtgctatgc tagcangggc 480

ctggccccaa gtgtgagtga tgagcaataa acgtaccgtc ccc 523

<210> 228
<211> 138
<212> DNA
<213> Homo sapiens

<400> 228
aagtgcgaag tcagggatgg tctaagaggg ctgagaggag aattccggaa cctcaggacc 60

ttgctcactg gctgctggct ggggctgtga agctgtccag tctagaactc aaagagtgat 120

ggtacaggct ttagagcc 138

<210> 229
<211> 396
<212> DNA
<213> Homo sapiens

<220>
<221>  misc_feature
<222>  (198)..(198)
<223>  n is a, c, g, t or u

<400>  229
gggctgggta cctcttctgg ttgctgagtg gagtgcacca gcagccccac cccagagaag      60

ccctgttgga agcgctgtgg gaatccccca aggtagggga gtggacacca taaggaaggg     120

gaggagtgcc agctccatat gcggtctccc ccatcagtca ggccagcagc gggttcagct     180

gcctctgggc agccctancc catacagaca gggagacctc cctcccgatc ttctgtgaat     240

agtcccttat acccctgctt atgcctcagg ggctcctcca cccttttgtc ttcatactgc     300

atatgaaaac tgcccttgta tatgtggata tctgaatgtg tcagtgaagg cctatatgaa     360

tgtgcacatg tgggtatgtt ctcagccatg tgtata                               396

<210>  230
<211>  432
<212>  DNA
<213>  Homo sapiens

<220>
<221>  misc_feature
<222>  (39)..(39)
<223>  n is a, c, g, t or u

<400>  230
gaactaaagg agccatctct ctcccctctc ctccgttcnc gagaggaggg gtgggtctca      60

gacgtttttc ctatggactt atttcttcca tgtccaggac tttgcacaac tttggtttta     120

aaagctgttg aaaaatagga aaacaaaggg cattgttcac agatagggcc aagtctcccc     180

ttgcaagggt gcctctgttc tgtccctgcc cccacctcac cttctctact cctccagtaa     240

gttggcagtt ttggtgccaa accccaaatc tccaaagaga catgccaggc aagacaaacc     300

cccaaacacc tcctttccgg tggccttgga aacagattgc tccgagctgg agaatgtcgg     360

gtgaggtgta tgggagagga ggggagagtt agaacttgtg cctttgggag taaggggtaa     420

ctgcctggag gg                                                         432

<210>  231
<211>  549
<212>  DNA
<213>  Homo sapiens

<400>  231
atcagtgcca gaaattcctt acctaaagtg gcatatgcga cggccatgga ctggttcata      60

gccgtctgtt atgcctttgt attttctgca ctgattgaat ttgccactgt caactatttc     120

accaagcgga gttgggcttg ggaaggcaag aaggtgccag aggccctgga gatgaagaag     180

aaaacaccag cagccccagc aaagaaaacc agcactacct tcaacatcgt ggggaccacc     240

tatcccatca acctggccaa ggacactgaa ttttccacca tctccaaggg cgctgctccc     300

agtgcctcct caaccccaac aatcattgct tcacccaagg ccacctacgt gcaggacagc     360

ccgactgaga ccaagaccta caacagtgtc agcaaggttg acaaaatttc ccgcatcatc 420

tttcctgtgc tctttgccat attcaatctg gtctattggg ccacatatgt caaccgggag 480

tcagctatca agggcatgat ccgcaaacag tagatagtgg cagtgcagca accagagcac 540

tgtataccc 549

<210> 232
<211> 554
<212> DNA
<213> Homo sapiens

<400> 232
gatgagtcca tctcacttgc tcagaacttt gcctggtgag agcggttaca agcgaacaag 60

gtggaaatga aagaaaccct gactttccca ctaggaagga agagactgtt ccttcttgtg 120

atgtactctg aagaaaaatt ctaggatttg gacagatttc ttgggttata aaacatgatt 180

ttcttctctg tttcttgggc ttttataatg ggtactgttg ttttcttgca aagctttaat 240

gattccataa ggacttgtat aaagtttatg ggagaatttt caatgtagat gtgaatggca 300

gaaacccaag aatctgtgtg aggttgaata agatcctgtg tctccagaga ggtctgatgg 360

ggagacacag atctaaattt taaaggtggt ttgggccttc tcaatcatat attaaggtcc 420

ttttatgtta tagataagta aattaaggcc cagaaagatt aatagcccaa ggtcccaaga 480

cctgcttgag acctgtgccc catttctgac taatattctt catgatattg tatcactctg 540

tatcaaaacc aacc 554

<210> 233
<211> 539
<212> DNA
<213> Homo sapiens

<400> 233
gatggtgcag tacctgctag cactttgctg cagaatgcct ctgcactcag ttctgcaaat 60

gtactgtttt agtttcattt aaaacccctt tttttgtgag aagatttcaa acatcaggca 120

agtttgtaat gaattcaagc tgagttctct cgagggacaa acatgtataa ctacagttcc 180

agtgtcagtg ccagctgtca ggttttcact gtgcagctag ggctgcctgc atacccagtc 240

atgtaaacca aattcactct agaatcggcc aggtcttacc aaaatgcaaa tagaatacaa 300

agcaactgga aatatatttc gtaatttcat tttatgtgtg attttaaaag ttaagctact 360

tcaaaactca tctgtctaac ttattttcac taataagtgt aacttgcctg gaatttggca 420

gatctaagct gggcttgggc tagatggttt caagcctgag tcattaagat gtgaaattta 480

cagaaacaac agaggattga ggaacaagtt aaaggacact ctaatggtgc agtctgcat 539

<210> 234
<211> 431
<212> DNA
<213> Homo sapiens

<220>

<221>  misc_feature
<222>  (102)..(102)
<223>  n is a, c, g, t or u

<400>  234
gtgagcatgg aagtagatct tccccggtca agcccccaga aggacccagc cctgcggaca      60

ccttgaccga aacctgtgag agctccggaa atagaggaac cnagcattcc ctctggaata     120

catcagcact gttgcctttg aggctggcct gcttgaatgc acacctgagc tccggattca     180

cagtggagga agccagatgc catgtcatga gggtgctcaa gcaacttttt ggagatgtat     240

gtatggagag aaactgaggc ctcctgccaa cagccagcac taacttggca agcatgtttg     300

agagccacct gggaagtgga gccttcagcc ccagttaagc cttcagatga gactgcagtc     360

ctggccacca tctggactgc aacttcacaa gagctcctaa gccagagcca tgcagatgga     420

ttcttggccc c                                                          431


<210>  235
<211>  403
<212>  DNA
<213>  Homo sapiens


<220>
<221>  misc_feature
<222>  (139)..(139)
<223>  n is a, c, g, t or u

<400>  235
gatctcattg cctttttatg ccgattaaca tgcttttagc ccctactgag cttatagtta      60

acagaagttt ccaggtcttt cttcacctga actgtgtcta aagcaagttc cctccacctt     120

ctgtatttat acgcttgant ttttaaaacc taaatgttgg gcttcacatt tgttccttgt     180

aaatttcatc ttggtgattg cagtctaccc tctggccttt aaaaattgtc tgagccttga     240

ttcgatcatg aaaccagctt acccttcccc tgtgtgctgg ccccagtttt ctaaccaggt     300

gttgaatgaa ctggatggac tctgccagat ccctccgtgc aaggctggaa tcagtccatt     360

gttcaactgt gccctttggg gctgtggttc atttggctct gat                       403


<210>  236
<211>  257
<212>  DNA
<213>  Homo sapiens

<400>  236
ctgctggaaa ggcatccttg ctgcagctgt gagtgtgatg ggacagcaga gtcactcctg      60

catgggattc tagggctggg ggtcccagag gggtggcctc cgcccctcct gggggccgag     120

gactgtcacc atgtcactac ggcactctcc agctgctgac caaagccctc gctaaccgca     180

gccctgccat actctgggtc tttcctctgg agcaaggtga agagactgca gcgaggcgtg     240

gaattgggaa gctcttc                                                    257


<210>  237
<211>  446

<212>  DNA
<213>  Homo sapiens

<400>  237
actgtgactg cgcgcaggac gagaactgca agtccaccaa gcgcgccatt gagccgtgcc      60

tgccccggac gagcggcggc ggcgcgggcg gccccggcgc gggcggggtc atgggctgca     120

ccgaggcccg gcggcgctgc gaccgcgaca gccgctgcaa cctggcgctg agccgctacc     180

tgacctactg cggcaaagtc ttcaacgggc tgcgctgcac ggacgaatgc cgcaccgtca     240

ttgaggacat gctggctatg cccaaggcgg cgctgctcaa cgactgcgtg tgcgacggcc     300

tcgagcggcc catctgcgag tcggtcaagg agaacatggc ccgcctgtgc ttcggcgccg     360

agctgggcaa cggccccggc agcagcggct cggacggggg cctggacgac tactacgatg     420

aggactacga tgacgagcag cgcacc                                          446

<210>  238
<211>  340
<212>  DNA
<213>  Homo sapiens

<400>  238
ggaacagagg agagatgccg gctggaggac acagcaaatt tgaaccaaga ggagcttgga      60

ggaagcccga gcgacctgga ggggactggc tgaccttcct cattcttttc aagtgtgaat     120

aataaccaag cccagtttgg caactccttg agggtgagga cgaagcccca ttctcctttt     180

tggaacttgg tggggctcag gaagcaggtt ctctccagtc ggtggctttc ctttctgttg     240

cgggtctctt gagggcctgc cttcatgaag gcacatgagt gactcatcat ttgtgaatta     300

attgctatat gtgaagggca tctgagaaca aattatcttc                           340

<210>  239
<211>  560
<212>  DNA
<213>  Homo sapiens

<400>  239
tgaccgccat gtggctgtgt ctgaccgcct gcgatactcg gccatcatgc atggagggct      60

gtgtgctagg ttggccatca catcctgggt cagtggctcc atcaactctc ttgtgcagac     120

tgctatcacc tttcagctgc ccatgtgcac taacaagttt attgatcaca tatcctgtga     180

actcctagct gtggtcaggc tggcttgtgt ggacacctcc tccaatgagg ctgccatcat     240

ggtgtctagc attgttcttc tgatgacacc tttctgcctg gttctgttgt cctacatccg     300

gatcatctcc accatcctaa agatccagtc cagagaagga agaaagaaag ccttccacac     360

gtgtgcctct cacctcacgg tggttgccct gtgctacggc acaacgattt tcacttacat     420

ccagccccac tctggtccct cagtccttca agagaagctg atctctgtct tctatgccat     480

tgttatgcct ctgctgaacc ctgtgattta tagtctaagg aataaagagg tgaagggggc     540

ctggcataaa ctattagaga                                                 560

<210>  240

<211>  524
<212>  DNA
<213>  Homo sapiens

<400>  240
ggaaatagtt tgttcatatg gccaaattat aaagggactt agtaaaagaa agctatgttt      60

tctgattacg aaggaaatct atgctcacag tgggaaaaca agaaaatgtg gcaaagcaca     120

ggtaagaaaa taaaaatcaa taatatcaac attatgaata ttttaggtac ttaggaattt     180

ggggtagaat gatggaaagc aaactgttaa ttatagctgt atatttcagt gtagaggcta     240

caggtgcctt gcatttgttt tcttataaaa tctgttccca tacattttac ttactttatt     300

tgaatttagg aaactttcat taggtagcca tttttatttt ctgtttcttt aatcatttta     360

ctttgaaata attttaaatt tacagaaaat ttgcaaaaat agtgtagaaa tttcccattt     420

gcctttatcc agcttcctgt agtgttgcca ttttatgtaa ccatagtaca attattgaaa     480

ccaagacatt aactttgaga ggctgctact actctaagaa ccat                      524


<210>  241
<211>  504
<212>  DNA
<213>  Homo sapiens


<220>
<221>  misc_feature
<222>  (71)..(72)
<223>  n is a, c, g, t or u

<220>
<221>  misc_feature
<222>  (197)..(197)
<223>  n is a, c, g, t or u

<220>
<221>  misc_feature
<222>  (219)..(219)
<223>  n is a, c, g, t or u

<220>
<221>  misc_feature
<222>  (233)..(233)
<223>  n is a, c, g, t or u

<220>
<221>  misc_feature
<222>  (289)..(289)
<223>  n is a, c, g, t or u

<220>
<221>  misc_feature
<222>  (309)..(309)
<223>  n is a, c, g, t or u

<220>
<221>  misc_feature
<222>  (346)..(346)
<223>  n is a, c, g, t or u

<220>
<221>  misc_feature
<222>  (390)..(390)

<223>  n is a, c, g, t or u

<220>
<221>  misc_feature
<222>  (395)..(395)
<223>  n is a, c, g, t or u

<220>
<221>  misc_feature
<222>  (474)..(474)
<223>  n is a, c, g, t or u

<400>  241
tcctgtgtct tgacccagaa aattgtgaca tgtaaaaaga ataaattcct ggtttaagcc      60

agtaaggtta nnggtacatt gttacatctc agataattaa aaccttgaaa aactcatgag     120

agatcacaag tagaaccttg atctgaaaca tggcatgtgg cgatttatat tgagtattag     180

gttaaaaatg caagaangga gcatagttaa tattttacnt taaagctaaa acnataattg     240

cctacttaaa attttcagtt aattaggttg tcactttttg ttcttaacna agaaatcaac     300

tagttttant ccataaacag ttagaactga tgcacacatc cgtttntcct tactcatttt     360

aaacagctat ctgaaatagg aagtgtaatn taatntttaa agaatctgaa aacatgacag     420

aaatgtttaa actataaaca tatattgtat atgttagcat attgtataca ttgnatatta     480

acataagcta gaatcattga cata                                            504

<210>  242
<211>  317
<212>  DNA
<213>  Homo sapiens

<400>  242
cgaaccactc agggtcctgt ggacgctcac ctagctgcaa tggctacaga ggctggaaga      60

tggcagcccc cggactgggc agatcttcaa gcagacctac agcaagttcg acacaaactc     120

acacaacgat gacgcactac tcaagaacta cgggctgctc tactgcttca ggaaggacat     180

ggacaaggtc gagacattcc tgcgcatcgt gcagtgccgc tctgtggagg gcagctgtgg     240

cttctagctg cccgggtggc atccctgtga cccctcccca gtgcctctcc tggccttgga     300

agttgccact ccagtgc                                                    317

<210>  243
<211>  437
<212>  DNA
<213>  Homo sapiens

<400>  243
aatgccggct ggctcagtga tggctctgtg caatatccca tcacaaagcc cagagagccc      60

tgtgggggcc agaacacagt gcccggagtc aggaactacg gattttggga taaagataaa     120

agcagatatg atgttttctg ttttacatcc aatttcaatg gccgttttta ctatctgatc     180

caccccacca aactgaccta tgatgaagcg gtgcaagctt gtctcaatga tggtgctcag     240

attgcaaaag tgggccagat atttgctgcc tggaaaattc tcggatatga ccgctgtgat     300

gcgggctggt tggcggatgg cagcgtccgc taccccatct ctaggccaag aaggcgctgc     360

agtcctactg aggctgcagt gcgcttcgtg ggttttccag ataaaaagca taagctgtat 420

ggtgtctact gcttcag 437

<210> 244
<211> 389
<212> DNA
<213> Homo sapiens

<220>
<221> misc_feature
<222> (299)..(299)
<223> n is a, c, g, t or u

<400> 244
tagatcatgc cctcattggg cttacatgct gttgaaaaga taggatataa atccatgaaa 60

atttttacaa tgctatttat taacaataca tgacaagagt actagaaatg ttacttgtga 120

ctattttgtc tattctagcc aagctggatg cctggctgtt tctcagttat actaaatgag 180

ttctgctctc agggtcttca tacttgccct tccctctgcc tgcaacactc ttcctccagt 240

tttttttttt tttttttggc tctctccatc actttaggtc tccattaaaa ctgtcagcnt 300

tcagggaagt tgccttccct gaccacaacc acactaattc aaataccaat ccttccccgc 360

ctccgtttgg taactctcta gtctcttat 389

<210> 245
<211> 136
<212> DNA
<213> Homo sapiens

<220>
<221> misc_feature
<222> (68)..(69)
<223> n is a, c, g, t or u

<220>
<221> misc_feature
<222> (91)..(91)
<223> n is a, c, g, t or u

<400> 245
gccccaaggt ctttaagtat ctctgtcact tattagctca ccagagaaga cacaggaatg 60

agaggccnnt tgtttgtccc gagtgtcaaa naaggcttct tccagatatc agacctacgg 120

gtgcatcaga taattc 136

<210> 246
<211> 369
<212> DNA
<213> Homo sapiens

<400> 246
ggccctgggc taagtcgggg atgaaggcgg gagctgctgt gctggactgc agctcagcac 60

agagacagtg agcctagatt gcagagctgc ccagggaggg atgtcacctt gggggatgga 120

ggctgcaggt gctcctcaga ccttagggaa acatttggga gggagcttgt tgaggagata 180

caggcacctc agggtggctg ggctggatgg actttgatga cccttccttt tttgagacct 240

gatggttctc taatttggga atcatttcca aagatgggtc taaaaatcct tgtttcattg 300

gaaataatga gtttgctatg atgcttaaga ccaagcatgt caccatttgt tattactgca 360

cttttccct 369

<210> 247
<211> 444
<212> DNA
<213> Homo sapiens

<400> 247
gaggcttttg acacagttat tagttaaatc aaatgttcaa aaatacggag cagtgcctag 60

tatctggaga gcagcactac catttattct ttcatttata gttgggaaag tttttgacgg 120

tactaacaaa gtggtcgcag gagattttgg aacggctggt ttaaatggct tcaggagact 180

tcagtttttt gtttagctac atgattgaat gcataataaa tgctttgtgc ttctgactat 240

caatacctaa agaaagtgca tcagtgaaga gatgcaagac tttcaactga ctggcaaaaa 300

gcaagcttta gcttgtctta taggatgctt agtttgccac tacacttcag accaatggga 360

cagtcataga tggtgtgaca gtgtttaaac gcaacaaaag gctacatttc catggggcca 420

gcactgtcat gagcctcact aagc 444

<210> 248
<211> 394
<212> DNA
<213> Homo sapiens

<400> 248
ggggcggcgg aagcgagtag agtttgtgac atttgtgcca gcccctccag cccagtcacc 60

tgaggagcct gtaggggccc ctgctgtgca gtccatcctt gtggcaggcg aggaggacat 120

ccgctgggtg tgtgaggaca tggggctgaa ggaccctgag gagcttcgca actacatgga 180

gaggatccgg ggcagctcct gaccctccac agccacctgg tcagccacca gctggggcaa 240

cgagggtgga ggtcccactg agcctctcgc ctgcccccgc cactcgtctg gtgcttgttg 300

atccaagtcc cctgcctggt cccccacaag gactcccatc caggccccct ctgccctgcc 360

ccttgtcatg gaccatggtc gtgaggaagg gctc 394

<210> 249
<211> 414
<212> DNA
<213> Homo sapiens

<400> 249
tttgctttgg gtactgtgat aactactttt tatactttat cccatttaat tataaaaacc 60

actcttgaga agtaattttt attttcagaa ccattttaca gatttaaaat aaacaggttt 120

gaggaattag tttaacttat ccaaagtttc gtggctatta agttctagta tttggagtca 180

aatgcaagtc tgtctaaatc tagagcccat gttctttaac tgcaacacta taatgtctca 240

ccccgtccta gtcccaccaa ttagtcaact cttttagggc agaagtctgt ctaattcatc 300

tttgcttcct gttactttat atttaattaa aaattttagt gactttttaa cttgtaaatt 360

gtagctgatt ttacatttat cttcctgaag gaaactctgt atcattttgt cttt 414

<210> 250
<211> 268
<212> DNA
<213> Homo sapiens

<400> 250
cttttattag aatgccatgc ctgcttatgt tatgcatgta ttttataata atttaatcta 60

ttttacaatt ttaaactcaa atatgattta gtattatgca cataatacaa acagtagtgg 120

tgagcaaacg tgtgtttccc ccacatgtgc agaatatgat ggattttatg aaaataaata 180

ttcttaactc caggaaatat gatctatatg gttccttaaa agattttcca atacactgaa 240

aatttagttc cttatgttca ttgtataa 268

<210> 251
<211> 443
<212> DNA
<213> Homo sapiens

<220>
<221> misc_feature
<222> (131)..(132)
<223> n is a, c, g, t or u

<220>
<221> misc_feature
<222> (156)..(156)
<223> n is a, c, g, t or u

<220>
<221> misc_feature
<222> (187)..(188)
<223> n is a, c, g, t or u

<220>
<221> misc_feature
<222> (230)..(230)
<223> n is a, c, g, t or u

<400> 251
cgtgcagcag atcccaggag ttggaaaagt taaagctccc cttctcctcc agaagtttcc 60

aagcatccag caactgagta atgcttccat tggggaactg gagcaggtgg tcggacaagc 120

agtggcacag nnagatccat gccttcttca cgcagnccca ggtgagggct ggcctcaggg 180

ccacggnnat cttctcccga gaccacaaac accaggatct tgttttcagn tttaaaaacc 240

aagagaatgg gccgggtgca ctggctcacg cctctaatct cagcactttg ggaggccgaa 300

gacagcggat catctgaggt caggagttca agaccagcct ggccaacatg gagaaacccc 360

taaaaatagg aacaattagc caggcatggt gacaggtgcc tgtaatccca gctacttggg 420

aggccgaggc atgagaatca ctt 443

<210> 252
<211> 281
<212> DNA
<213> Homo sapiens

<400> 252
gagaaattcc cacactaaaa acactacaag tttttggaat cgtgccagat ggtacccttc 60

aactgttaaa ggaagccctt cctcatctac agattaattg ctcccatttc accaccattg 120

ccaggccaac tattggcaac aaaaagaacc aggagatatg gggcatcaaa tgccgactga 180

cactgcaaaa gcccagttgt ctatgaagta tttattgcag gatggtgtct cttctttaga 240

acagggaaaa taggcaggaa gcccaattgc tggagtactt a 281

<210> 253
<211> 249
<212> DNA
<213> Homo sapiens

<400> 253
ccaaatatct agattctgat cccttttgag gtcctagacc ctttgagaaa ctgatgaagc 60

caggcacctc cttcctcagg aaaatgctgg tgtacaaata cacacaaagc tcttcaggca 120

gctgatagat ttcccccaga gagctattca aggacttcct aaggtgggtg gactgcaggg 180

ttaggacacc tgctatagag gtgacatttt tccaaggaca agcagggact ttggtcttga 240

ctgttctct 249

<210> 254
<211> 259
<212> DNA
<213> Homo sapiens

<400> 254
agaagagcct gaacctcaac atcttcctga agcaatttaa gtgctccaac gaggaggtcg 60

ctgctatgat ccgggctgga gataccacca agtttgatgt ggaggttctc aaacaactcc 120

ttaagctcct tcccgagaag cacgagattg aaaacctgcg ggcattcaca gaggagcgag 180

ccaagctggc cagcgccgac cacttctacc tcctcctgct ggccattccc tgctaccagc 240

tgcgaatcga gtgcatgct 259

<210> 255
<211> 535
<212> DNA
<213> Homo sapiens

<400> 255
aaattctgca atgaacccta caccgaccgg acagaagaaa gggaagaatc caaagaggaa 60

gaagactggt ccctccgacc tgtcctttcg ggagctgaga aagatgacga agctgagcgt 120

ctcagagaaa caacagaaga cggagaagac ccgccaggct acaccaccga catgagaaca 180

gataaagaag ctgactcaaa tggcagaggg cagcctaaag gagaaacaac tggcaattat 240

cccgggtaat atgatcttgg ctgccttgat ggtaattacc gcggcggtaa gtctccctgc 300

tgtctggact gaagaaaatt ttacatactg gcttctgttc catttcctcc tttaattagg 360

ccagttactt ggatggattc ccctattgaa gtttatacaa atgatagtat tttggatgcc 420

tgggccgatt gatgatggct gtcctgcaca gcctgagaag gagggtatgt tgatgaatgt 480

aactggtatg aataccctcc aatttgttta ggaattgctc cttgatgttt accat 535

<210> 256
<211> 230
<212> DNA
<213> Homo sapiens

<400> 256
ggaagtaatg acttttttgc ccatttactc actgagtccc ataatgtggt aaatgtataa 60

tgctgacatt tgttccgtcc ttatagattg aggatagtac ggccctgaat tttgccttta 120

ctttagaaac ctgattcaac ttaaccgaac tctcaggaat ctgattccta agctgagtat 180

cacattttag attacttact aatttgtgca tctatccacc tagcaaatat 230

<210> 257
<211> 532
<212> DNA
<213> Homo sapiens

<220>
<221> misc_feature
<222> (97)..(97)
<223> n is a, c, g, t or u

<220>
<221> misc_feature
<222> (152)..(152)
<223> n is a, c, g, t or u

<400> 257
taaaaccaac cagctgaacc tttcaggcta caagagaacc cgggtcggta atgtcttttt 60

aagaataatt tttaattgct tataacaagc atatttngtg gcatttgaac tatatttact 120

gctccaatat ccgttatttt ccaaaggatt tngtatcttt ttgaaaatgt ttacatcatc 180

agatgatcca cagaattcac tttatgtgag atctcccgag agtttccatc ccaacataat 240

ggactttggt ttgaacacaa ttcgtttttt catttgaatt ggcatttccc aatatttgct 300

aaacatttgc tggagaaatc atttttcttt tttctttttt agaaaactca gaatgaaaat 360

tcattcccct gaaatattta ggtgtctata ttctatattt tgatctatta agggattagt 420

atttttccat gtttattgtg ttatcagagt gcattagaaa gattagtgat tcatcttcac 480

agcacatttt taatcaagca gttatttcaa ccagcacatt cgttttgttc at 532

<210> 258
<211> 489
<212> DNA
<213> Homo sapiens

<220>
<221> misc_feature
<222> (363)..(363)

<223>  n is a, c, g, t or u

<220>
<221>  misc_feature
<222>  (441)..(441)
<223>  n is a, c, g, t or u

<400>  258
atcaccccct gttcattatg tcaggcctca tgggagcctg gccttctcca gaagctggcc      60

ccggcgtcct cccaagctgg accacgtagg ccccagatca cacctggggg tccagatgta     120

ggggtcccgt gtgcacgccc aatcagaccg agcacttgtg acactacccc aacacctctc     180

ccagggctga atgaggaacg cgccactgga cacatgagga agaggctgcc ctgggagcta     240

ctgatgctgt gacctcacct ctctggcttt gggcggcagg tccctgcacc taggatgcct     300

gcctggaaat gtccttgcat tcgtggcctc cttcacagcc tcctcctcag agaagcctct     360

gcnagtgcac agggagtgtg tgcagccttg tgaagggctg ggaccacttg cccagactgg     420

ggcccctcag gcacaggcgt ngggtcctac tgacctgtct ccccagctcc cacacagaaa     480

gcatctaaa                                                             489

<210>  259
<211>  468
<212>  DNA
<213>  Homo sapiens

<400>  259
cagaaggaaa cggtgtctct cggctgtggc tctgagtgca aattgcatgg gcggaaaggc      60

gggggtggct gctcttcctg gcaggcctgg gccatcagcg aactgggccc cgtgaggagg     120

gcgggagtgt ggaggagggt gggcctctca cccaggcttt ctcggcccct ctcctcagct     180

tgcagagctg gccagccccc tccttagggg gtgggcgagg agcctctggg cagacccaag     240

aaccatgggg actggggtgg gttggtggca ccaatggcag ccctccccgc ccctctcctt     300

caaggagggt tcccgcagct ggggggtgtg cggaggcgca tggcctcccg ccacggggcc     360

gtgctgtgtt tatggctggc agaggcagcc agcgggtggg ggattctgct gctcgctcac     420

ctgcctggct cgctggtctc tcgaattttc ttccctctga aatcctat                  468

<210>  260
<211>  531
<212>  DNA
<213>  Homo sapiens

<400>  260
ctgcaccaac tcatgctgga ctgttggcag aaggaccgca accaccggcc caagttcggc      60

caaattgtca acacgctaga caagatgatc cgcaatccca acagcctcaa agccatggcg     120

cccctctcct ctggcatcaa cctgccgctg ctggaccgca cgatccccga ctacaccagc     180

tttaacacgg tggacgagtg gctgaaggcc atcaagatgg ggcagtacaa ggagagcttc     240

gccaatgccg gcttcacctc ctttgacgtc gtgtctcaga tgatgatgga ggacattctc     300

cgggttgggg tcactttggc tggccaccag aaaaaaatcc tgaacagtat ccaggtgatg     360

cgggcgcaga tgaaccagat tcagtctgtg gaggtttgac attcacctgc ctcggctcac 420

ctcttcctcc aagccccgcc ccctctgccc cacgtgccgg ccctcctggt gctctatcca 480

ctgcagggcc agccactcgc caggaggcca cgggcacggg aagaaccaag c 531

<210> 261
<211> 379
<212> DNA
<213> Homo sapiens

<220>
<221> misc_feature
<222> (210)..(210)
<223> n is a, c, g, t or u

<220>
<221> misc_feature
<222> (331)..(331)
<223> n is a, c, g, t or u

<400> 261
cctcggacac cagagacaat aactgagcgc ggaggacacg cctgccctgc ctgccatctg 60

tggcccgaag ccattgccat ccactgcaga cgcctggaga gggacaggcc gcttccgagt 120

gcagtcctgg cgcagcaccg actcccacgc acccggggaa ggacaccctc actcccacac 180

cccgggaaga acactagaac atcagcagan gggccctgcc cctccgcctg cagccgtgaa 240

aggaagctgg gtcatcagcc cagccccgcc caccccagcc cctatgtgtg tttccctcaa 300

taaggagatg ccttgttctt ttcaccatgc naataacatg cccagcaaaa acttgcttta 360

tgggtctgcc tggagaaaa 379

<210> 262
<211> 486
<212> DNA
<213> Homo sapiens

<400> 262
aaccacacca gaagacatcc tcaggaacaa aggctgctcc agctctacca gtgtcctcct 60

cacccttgac aacaacgtgg tgaatggttc cagccctgcc atccgcacta actacattgg 120

ccacaagaca aaggacttgc aagccatctg cggcatctcc tgtgatgagc tgtccagcat 180

ggtcctggaa ctcaggggcc tgcgcaccat tgtgaccacg ctgcaggaca gcatccgcaa 240

agtgactgaa gagaacaaag agttggccaa tgagctgagg cggcctcccc tatgctatca 300

caacggagtt cagtacagaa ataacgagga atggactgtt gatagctgca ctgagtgtca 360

ctgtcagaac tcagttacca tctgcaaaaa ggtgtcctgc cccatcatgc cctgctccaa 420

tgccacagtt cctgatggag aatgctgtcc tcgctgttgg cccagcgact ctgcggacga 480

tggctg 486

<210> 263
<211> 350
<212> DNA
<213> Homo sapiens

<400>  263
tctccgtgga ggctatggct tcagacaggc cccgaaggtc tgtcaccaat gtgctcggtt      60

gtgggtcaca taacgctctc tggagggctt gcctttcagc ttgggatcat gaaaagatga     120

tttgacgctg tttctcatgg tctccgacct aataaagcaa gataagagaa aacaaatgtt     180

attttaaaaa aatcaccctt tggcaaaaga aacatgtaaa attagaatct ggcacaaaca     240

aaacctgaat ctgggttgtg aactttcacc acccgccgca actctttgat aaaacctcaa     300

gtgatatcta ttaccattgt aaaaataaag cctgccccta tgcttagaat                350

<210>  264
<211>  507
<212>  DNA
<213>  Homo sapiens

<400>  264
ggcaaccggg gaagtattgt ggccttggag tttgctaaat ccaaatatga aaatcaaaag      60

ctttagtatt cctcatcttc tcttctggaa gatttgcgtt agagtttttg ttgggccttc     120

aaaaagctgt gttcagagtt aggagaatat atccaataaa agatggtttc gtctaccaat     180

tggggaagtt tcaccctctc cctatctgaa gaaaaaaatc aaaaacaaat gtccccggat     240

ctttcgatgc aagtcctgga ggcagggaga tcactgcctg cctggcccac gctgctggga     300

cggctcgtcc tccctgcttt ttgtttttca aacctcctgc ttctcccacc ttgggaagga     360

gaaatgtgaa acccggcagc ggccgaccta ggcggtcttg tggcccggag ccggcccggc     420

ccgaaaacca tagacctggt tgtactgtag cttgttgttt gggggaccaa attttctaga     480

gagaactaga gcacttttgt tgtgttt                                         507

<210>  265
<211>  192
<212>  DNA
<213>  Homo sapiens

<400>  265
cacaggcctt cagaggcgat ggctgggcga cagtgacgaa agcaaagcaa agcagggctg      60

tggagacact cctcgcattt gtctcttccc tccaaggatt atctgagcaa gtcgacttgt     120

tcattcaaag gcggggtctg ccaagccctg ctctatccaa tggggatagc ttctacgtaa     180

cggattccaa tt                                                         192

<210>  266
<211>  202
<212>  DNA
<213>  Homo sapiens

<400>  266
agagcaacag ctctatatct ggatcactgc agtgcctaga agatacaaca gcacaattta      60

caaatccaaa tttccaggaa gtctctgcac atacctctag tacaaaagat gtttcagaga     120

ctagagggtc agaaggcaaa gagaggcaat attcaactcc cagttcaggt caaaagggaa     180

gaaagcctgg tgttgaaaga aa                                              202

<210> 267
<211> 278
<212> DNA
<213> Homo sapiens

<400> 267
gaaccacgtt ctttgtatgg gcccaatgag ctgtcaagct gccctgtgtt catttcattt 60

ggaattgccc cctctggttc ctctgtatac tactgcttca tctctaaaga cagctcatcc 120

tcctccttca cccctgaatt tccagagcac ttcatctgct ccttcatcac aagtccagtt 180

ttctgccact agtctgaatt tcatgagaag atgccgattt ggttcctgtg ggtcctcagc 240

actattcagt acagtgcttg atgcacagca ggcactca 278

<210> 268
<211> 392
<212> DNA
<213> Homo sapiens

<220>
<221> misc_feature
<222> (302)..(302)
<223> n is a, c, g, t or u

<400> 268
ctcctggcct gatactctag ggatgcaggt gggagaagca ggggtcctgg gggctgcctg 60

gagctctggg aggcattctg aacggggtct actactgatc tcaggtgagc tctgccctcc 120

tctgaaagtc acttttctca tcagttaaat gggggcaagg gtccgtggtc cgaccaaggt 180

cttggcttca cagacatcac caggagcctg catgcccctg atcactcctt ctccttcctc 240

caggaaactc cagcctggcc tctgacccca gttcaatccg accatgccca agcccaagcg 300

gncctttcct ccagaactgc tccggggcct ggctgtgtga ctggagcaag gtgctaaacc 360

tctctgtgcc tcgctggtct aatctgtaaa at 392

<210> 269
<211> 417
<212> DNA
<213> Homo sapiens

<220>
<221> misc_feature
<222> (240)..(240)
<223> n is a, c, g, t or u

<400> 269
taatctcatc caaaaccatg ctcacagaca cacccagcat aatgtttgac caagtatctg 60

ggcaccttgt ggttcagtca aattaacaca tattaactac cttagcaaga tgaaaagcag 120

tgaatgcagg atggtggttg aaattttaaa tacgttggtt atatagtctc attgaaaaag 180

gaacatttga gtgaagactt gaaggggtgg tggaataaac catttatttg cttattgccn 240

gtctccctct atcagaatga aagcttcatg aagcgagaga cttaattttt atctgttata 300

tccctagtgc ctggtgcagg gtaagtactc aaaaatattt gttgagtgaa taagtaatga 360

ttgaggatgg ggactggttt gtatctggtt atatctcttg tccttagcac agtacct 417

<210> 270
<211> 412
<212> DNA
<213> Homo sapiens

<400> 270
ggggccctag ggattatagc caggactcta atctgcctac catgccattt aacaagagat 60

cccactctcc agctgccttg tgtccctagg gtcctggcca tgtgtttagt gtgctaaact 120

ttctcctttg ttctcaggcc ttccaggtag tccccttcct ggacttaaga gtgcaaactc 180

ttctctgtgg ttctagcctt gggcagaatt atatcccaga gaccacagag caactgtcaa 240

gctgcttacc ccctcaccca gggctacagc ctgtgcccag ccctctaatt tgtgcctctc 300

ttgtgttggg ggtggtgggg gttattcctt tccctttcct gctctggcct ccttgaaagt 360

tcagagtacc cagtacaagt cagccaccat gctgacgggt atttttcctc at 412

<210> 271
<211> 372
<212> DNA
<213> Homo sapiens

<220>
<221> misc_feature
<222> (76)..(76)
<223> n is a, c, g, t or u

<220>
<221> misc_feature
<222> (270)..(270)
<223> n is a, c, g, t or u

<220>
<221> misc_feature
<222> (347)..(347)
<223> n is a, c, g, t or u

<400> 271
tagccaggta tagtggcagg aacctgtaat cccagctaca ggggaggctg aggcaggaga 60

atcgcttgaa cccggnaggt gtaggttgca gtgagccgag attgcaccac tgcactccag 120

cctgggcgac agagcgagac tctgtctcga aaaaaaaaaa ggtccgtgcc aagctgctcc 180

ctgcccttgc cctttccctt tccctggggt ccaaaccaca tgtgtcctgc ctctcctggc 240

cctaccacat tctggtgctg tcctcactcn cccctggccc agaggctcct gaagatgctg 300

ggcggtcctg gcacagggag gagcagctct gtaaatctgt gcacatngcc actcttggcc 360

taataaagga gg 372

<210> 272
<211> 427
<212> DNA
<213> Homo sapiens

<400>  272
cctaccaccg tcttcgagag gatgtcatgc ggctctctcg cctagcactg ggctcagagg      60

cctggcgccg agtctggagc cgcagtctgc agctggcgag ttggccaaac cggggagggg     120

cacctggagc tccccagggt gaccctatga gggtattctc agttaggacc cggagacagg     180

acactcttcc tgaagcgggg cgcagatcag aggcagaaga ggaggaggcc aggaccatca     240

gagtgacacc tgtcaggggc cgagagaggc tcaatgagga ggagcctcca ggtgggcaag     300

acccttggaa attgctgaag gagcaagagg agcggaagaa gtgtgtcatc tgccaggacc     360

agagcaagac agtgttgctc ctgccctgcc ggcatctgtg cctgtgccag gcctgcactg     420

aaatcct                                                               427

<210>  273
<211>  526
<212>  DNA
<213>  Homo sapiens

<400>  273
gtccacattc ctgcaagcat tgattgagac atttgcacaa tctaaaatgt aagcaaagta      60

gtcattaaaa atacaccctc tacttgggct ttatactgca tacaaattta ctcatgagcc     120

ttcctttgag gaaggatgtg gatctccaaa taaagattta gtgtttattt tgagctctgc     180

atcttaacaa gatgatctga acacctctcc tttgtatcaa taaatagccc tgttattctg     240

aagtgagagg accaagtata gtaaaatgct gacatctaaa actaaataaa tagaaaacac     300

caggccagaa ctatagtcat actcacacaa agggagaaat ttaaactcga accaagcaaa     360

aggcttcacg gaaatagcat ggaaaaacaa tgcttccagt ggccacttcc taaggaggaa     420

caaccccgtc tgatctcaga attggcacca cgtgagcttg ctaagtgata atatctgttt     480

ctactacgga tttaggcaac aggacctgta cattgtcaca ttgcat                    526

<210>  274
<211>  429
<212>  DNA
<213>  Homo sapiens

<400>  274
tgtgtccact ggtttcagtc tgagttctct gcactttgag gatgcagaca gtgaagttct      60

cccatggtta tagggggaga gatcatagga atgctatgga aagaggcctg aagtcagagc     120

cagctagtgg ttattattta ttaattgcct gtgaggtgcc aggcgcacat attagaccat     180

atgtgattgc agtgagccac ccggatcccc ttcaagctgc tgctgcagct gatggaagtc     240

ctattggcag acagccttct ctcatcagcc ccttcaggac ttgcctcagt tgcagagagc     300

tgccttcccc aagatcacac ccttccctgg ggactcacaa ccaatggctg atccagaaga     360

atccataaag cccgtatcat ttcagcccaa tttaggacag ctttgttgag ccattagacc     420

tacatgcag                                                             429

<210>  275
<211>  434

<212>  DNA
<213>  Homo sapiens


<220>
<221>  misc_feature
<222>  (376)..(390)
<223>  n is a, c, g, t or u

<400>  275
gaagctctac ttgcctggtg gtaattccag gatgacccag gagaggctgg aaagagcgtt      60

caaacggcag ggcagccagc ccgcacctgt caggaaaaat cagttgctgc cgtctgacaa     120

ggtggatggt gagctgggtg ccctgcggct cgaggatgtg gaggatgagt tgataaggga     180

agaggtcatc ctgtcgccag tcccatcagt gctcaagttg cagacagcat caaaaccaat     240

tgacctctca gtagcaaagg aaataaagac ccttctgttt ggttccagct tttgctgttt     300

caatgaagaa tggaaacttc agagtttttc ctttagtaac acagcctcat taaaatacgg     360

catagtgcag aacaannnnn nnnnnnnnnn agtcctggca gctgtccaag gctgtgtcct     420

acagaaactc ctgt                                                       434

<210>  276
<211>  189
<212>  DNA
<213>  Homo sapiens

<400>  276
aaaatcactg ccactgactt ttaccctctt caggaagagg ccaaggagga ggaacgcctc      60

atagctttga agaaaatcct cagctcgggg gtgttctatt tctcatggcc aaacgatggg     120

tctcgctttg acctgactgt ccgcacgcag aagcaggggg atgacagctc tgaatggggg     180

aactccttc                                                             189

<210>  277
<211>  542
<212>  DNA
<213>  Homo sapiens

<400>  277
gaggagcagg caaggctacg tgggcagctg aaggagcaaa gcgtgcgctg ccggcgcctc      60

gctcacctgc tggcctcggc ccagaaggag cctgaggcag cagccccagc cccagggacc     120

gggggtgatt ctgtgtgtgg ggagacccac cgggccctgc agggggccat ggagaagctg     180

cagagccgct ttatggagct catgcaggag aaggcagacc tgaaggagag gccagggagg     240

gttctccccg tgacaacccc actgcacagc agatcatgca gctgcttcgt gagatgcaga     300

acccccggga gcgcccaggc ttgggcagca acccctgcat tccttttttt taccgggctg     360

acgagaatga tgaggtgaag atcactgtca tctaaaagcc ggctactgtc agcaaagcct     420

gaagaagtgg ggctggatac cctgccccca ccatatccct accatccctt ctcagtcaac     480

cctttaccct tacagtagca agcatagacc cctgtctaac gggggtagac aggtgcagat     540

ga                                                                    542

<210> 278
<211> 475
<212> DNA
<213> Homo sapiens

<400> 278
gacagtctac cgtcacgaga agcgggtgaa actgcagatc tgggacacag ctgggcagga 60

gcggtaccgg accatcacaa cagcctatta ccgtggggcc atgggcttca ttctgatgta 120

tgacatcacc aatgaagagt ccttcaatgc tgtccaagac tgggctactc agatcaagac 180

ctactcctgg gacaatgcac aagttattct ggtggggaac aagtgtgaca tggaggaaga 240

gagggttgtt cccactgaga agggccagct ccttgcagag cagcttgggt ttgatttctt 300

tgaagccagt gcaaaggaga acatcagtgt aaggcaggcc tttgagcgcc tggtggatgc 360

catttgtgac aagatgtctg attcgctgga cacagacccg tcgatgctgg gctcctccaa 420

gaacacgcgt ctctcggaca ccccaccgct gctgcagcag aactgctcat gctag 475

<210> 279
<211> 294
<212> DNA
<213> Homo sapiens

<220>
<221> misc_feature
<222> (225)..(228)
<223> n is a, c, g, t or u

<220>
<221> misc_feature
<222> (231)..(231)
<223> n is a, c, g, t or u

<400> 279
ttttttagat ctaccctctt gttgcccagg gggagtccag tggcgtgatc ttggctcact 60

gcaaccgccg cctcccgggt tcaagcaatt ctcctgcctc agtctcccga gtgtcttctg 120

tcttttgtaa aagtttttca tgcccaagtg agattaattg tttaaaaaaa aaaaaacaag 180

aagaaaacaa catagattta ccgcaagacc tattgatata ttatnnnnca nggtggtata 240

cccagggtgg gtgtgacaca gaccaaaaga ggctgtgtgt tctgttgttg ataa 294

<210> 280
<211> 421
<212> DNA
<213> Homo sapiens

<220>
<221> misc_feature
<222> (129)..(129)
<223> n is a, c, g, t or u

<220>
<221> misc_feature
<222> (136)..(137)
<223> n is a, c, g, t or u

<220>
<221>  misc_feature
<222>  (146)..(146)
<223>  n is a, c, g, t or u

<220>
<221>  misc_feature
<222>  (323)..(323)
<223>  n is a, c, g, t or u

<400>  280
ggaagcgtgt ctgctgggag tttgcgaccg atgactatga cattggcttt ggagtttatt      60

ttgactggac ccctgtaact agcactgaca taactgtgca ggtcagtgat tccagtgacg     120

atgaggatna agaagnnagg aagagnagga agagattgaa gaacccgttc cagctggaga     180

tgtggagaga ggctccagga gctccttgcg gggtcgctat ggggaggtca tgcctgtgta     240

ccggcgggac agccaccgag acgtgcaggc tggcagccat gactaccctg gtgagggcat     300

ctacctgctc aagttcgaca acncctactc cctgctgcgc aacaagactc tctacttcca     360

catctactac accagctgaa ggactgctgt gacaggggca ggctgtattt gctggctgaa     420

g                                                                     421

<210>  281
<211>  544
<212>  DNA
<213>  Homo sapiens

<400>  281
atgagaacgg cgtcttcatg tgcgccgagg gcaccggcaa gttctgtccc ctgaggtcct      60

tcccagacac tgtctacaag aagctggtcc agagagagaa gactttaaag gttagaggag     120

tggaccgcac tccctacctg ggggatgtcg ctgttgtcgt gcaccctggg aaaaaagaga     180

tgggaacccc actcgcagac actcctaccc ggcccgtcac ccggcatggg ggcatgaggg     240

accttcacga atccagcttc agcctctctg gctctcagat cgatgaccat gttccaaagc     300

gagcttcagc tcggatcctc gctcctcccg gaggcaggtc gagtggcatt tggtaaaggc     360

attgccaagc cccccgagtg aggacgcacc gccgccacca gcccgcaact ctccagccga     420

agctgcaggg gcaggagagg ctgggctggg tggcacacca cccgaggggg gccccgggac     480

ccacggagcc ctccctatgt ctgcaaagtg attcactgtg cttcgagcca actctaacag     540

gcac                                                                  544

<210>  282
<211>  430
<212>  DNA
<213>  Homo sapiens

<400>  282
ctgattctac ttctgcaggg ttccacagaa gtctccagtc ttcaaatctt cagtgtatga      60

aagcacagat tcctgaaaga atggcctcaa atgaccagga gtaggagctc tctatatccc     120

tgctcctgaa aaacaagcta actggagtct ccatcacctg ccaccagcta tacacactac     180

caactaccca actgaactcc atgactgatt tgccagctaa tcatgcccct gacccagccc     240

acatggacat gggaaggaca tcagtgaact gtgaaaagag gcagagactc actcccgttt 300

gtattatgaa aacacacgcc aataggacat aaaaagaagc aagagtactg ggctttacca 360

tgagttcaaa tctcatttct ggcaattcct atgtctaaaa aaagcttcgt aatctctttt 420

gagccctcac 430

<210> 283
<211> 219
<212> DNA
<213> Homo sapiens

<400> 283
ccagaggatg atagcacctg tcagtgccag gcgtgcgggc ctcaccaagc cgcgggtcca 60

gatcttggtt cctctaatga tggctgccct cagctgttcc aggagcggtc agtcatagtg 120

gagaactcct caggctctac cagcgcttct gagctcctca aacccatgaa gaagaggaag 180

cgcagggaat accagagccc atcagaggag gagtcggag 219

<210> 284
<211> 232
<212> DNA
<213> Homo sapiens

<400> 284
tttgcctgag gttgactata catacaaata ttgagcattt cctcctggtc tccgtgataa 60

acaaaggttt tgatattgtt cggcgagatg gaaagaaaat atcaaggagt gagctgaagc 120

cactgccctt gagaaccctc tcgaggagtc tggcctcatg aagatgccag aataaacggc 180

agatatatcc tgaatgaatg tgagattttt accctgtgaa tttcctgtga gg 232

<210> 285
<211> 249
<212> DNA
<213> Homo sapiens

<220>
<221> misc_feature
<222> (208)..(208)
<223> n is a, c, g, t or u

<400> 285
agtgcttcca gtggcccaaa aatgcttttt gaagtgtgtt ttgaaacagc ccccaccaac 60

atacacccca ccaggagtac tgatcctgcc tcccttcatg tctaggggaa gcattcgcct 120

ttgagcactt gtttgcaaat ctggggagtt tgagacctcc tagcatctct tcccttcttt 180

ccctgcagtc tattcactcc cgcagccnaa aaatctctgg cgttcaggtt agcagtttct 240

gggttggtt 249

<210> 286
<211> 510
<212> DNA
<213> Homo sapiens

<220>
<221>  misc_feature
<222>  (138)..(140)
<223>  n is a, c, g, t or u

<220>
<221>  misc_feature
<222>  (142)..(143)
<223>  n is a, c, g, t or u

<220>
<221>  misc_feature
<222>  (371)..(371)
<223>  n is a, c, g, t or u

<400>  286
gggaattacc ttttgtattg cttgaattta ctgctgtctg tatgaactct ttttcagata      60

aatttttaag aaatcagata agtgaagtga aagagagaga tcaaagtgtt gtggcagcac     120

aaaggagaga ctgactannn tnntgctggg gaatctgaaa gagtgctttg gtggaggtaa     180

catgagatca gggccttgaa gggtgagtca agtctgtcaa ggagacaaga gggagagaag     240

agcttgccag aggcccagag accagcgagg aggctgtggt gtcctggaat gagggcgaga     300

tacttggtgg gactggtcaa cacggcaatg aagagggata tggccgagga aaatggagag     360

gggcactgga nctgtgccag caaggactgg gatgcgtgga cttgatcctg tagataacgg     420

gaggaagaaa ggcctggatg cagcgccatg tcatgagcac atctgatcat gacagctcac     480

ctatgggagg attctccctc aacatttttc                                      510

<210>  287
<211>  555
<212>  DNA
<213>  Homo sapiens

<220>
<221>  misc_feature
<222>  (39)..(39)
<223>  n is a, c, g, t or u

<220>
<221>  misc_feature
<222>  (89)..(89)
<223>  n is a, c, g, t or u

<220>
<221>  misc_feature
<222>  (106)..(107)
<223>  n is a, c, g, t or u

<220>
<221>  misc_feature
<222>  (272)..(274)
<223>  n is a, c, g, t or u

<400>  287
aggatgtgac agtgactcgg ggcgaccagg ctatgtttnc ttgcatcgta aacttccagc      60

tgccaaagga ggagatcacc tattcctgna agttcgcagg aggagnnctc cggactcagg     120

acttgtccta tttccgagat atgccgcggg ccgaaggata cctggcgcgg atccggccgg     180

ctcagctcac gcaccgcggg acgttctcct gcgtgatcaa gcaagaccag cgccccctgg 240

cccggctcta cttctttctt aacgtgacgg gnnngccccc gcgggcggag acagagttgc 300

aggcctcgtt ccgggaagtg ctgcgctggg cgccgcggga tgccgagctg atcgagccct 360

ggaggcccag cctgggcgag ctgctggcca ggcccgaggc tctgacgccc agcaatctgt 420

tcctgcttgc agtcctcggg gccctcgcat cagcgagtgc gacagtgttg gcgtggatgt 480

tctttcgatg gtactgcagt ggcaactaac aaaggtatct ttcctccttc cctatcctat 540

ttccatcctg aaaat 555

<210> 288
<211> 381
<212> DNA
<213> Homo sapiens

<400> 288
atgtatccgc tgtcaactac gaatttgagg atgaatactt cagtaatacc agtgccctag 60

ccaaagattt cataagaaga cttctggtca aggatccaaa gaagagaatg acaattcaag 120

atagtttgca gcatccctgg atcaagccta aagatacaca acaggcactt agtagaaaag 180

catcagcagt aaacatggag aaattcaaga agtttgcagc ccggaaaaaa tggaaacaat 240

ccgttcgctt gatatcactg tgccaaagat tatccaggtc attcctgtcc agaagtaaca 300

tgagtgttgc cagaagcgat gatactctgg atgaggaaga ctcctttgtg atgaaagcca 360

tcatccatgc catcaacgat g 381

<210> 289
<211> 488
<212> DNA
<213> Homo sapiens

<400> 289
cacgctcctg gaacgtcaga tcattattga ggcaaatgat cgccatctag aatcagcagg 60

acagactgag atcttccgaa agcacccccg caaagcctcc atcctcaaca tgccactagt 120

gacaacactt ttctactcct gcttctatca ctacacagag gctgagggga cattcagcag 180

tcccgtcaac ctgaagaaga catttaagat cccagataaa cagtatgtgc tgacagccct 240

ggctgctcgt gccaagcttc gagcctggaa tgatgtagat gccctattca ccacaaagaa 300

ctggctgggc tataccaaga agagagcacc cattggcttc catcgggttg tcgaaatttt 360

gcacaagaac aatgcccctg tgcagatatt acaggagtat gtcaatctgg tggaagatgt 420

ggacacgaag ttgaacttag ccactaagtt caagtgccat gatgtcgtca ttgataccta 480

ccgggacc 488

<210> 290
<211> 306
<212> DNA
<213> Homo sapiens

<400> 290

tttcatgact tctccttcac ctaagcacct caaaacagat gatagcactt caggattgac 60

gcgaagcatc ttcaaatatt tggagagcta acaccatcaa aggtgccaaa atctacattg 120

agactgcttt gagaagtttc tagcactgaa agttggaatt gacactccag ccaatgatcc 180

ttccttcttt cataatcaat gcaataagat tgcagacaga aattccagtg atttctactg 240

cacagctctg gacatctctt ttcctagtat tattccctga attggccact gatttcaatt 300

ctgcag 306

<210> 291
<211> 348
<212> DNA
<213> Homo sapiens

<400> 291
ctcctgggtc cgcagtgtac tgcgagggag cacagatgtc catcccccgc tggggtggag 60

agcggcagca ggcctgatgg atgagggatc gtggcttccc ggcccagaga catgaggtgt 120

ccagggccag gccccccacc ctcagttggg gctgttccgg gggtgactgt gagcgatccc 180

accccaaacc tgagatgggg tagcccgtcc tgtgtcctcc acagggacaa gcagtgggag 240

gagtctgaat ggtcaccagg aagcccgggc tccatcttga cctccttttt cagggacagg 300

agcaacaggc ccctcttccc tgactctaag cccttccctg taaggtga 348

<210> 292
<211> 395
<212> DNA
<213> Homo sapiens

<220>
<221> misc_feature
<222> (343)..(343)
<223> n is a, c, g, t or u

<220>
<221> misc_feature
<222> (361)..(361)
<223> n is a, c, g, t or u

<400> 292
tctctgcttt ccctcttatg aaaatggcag atgccttttt gtgaaggtct caaagcccac 60

ttcatcctgg ctgcagcacc aaaaggacaa aggcccgctt ttgaagtgcc tgataaggca 120

ttcctttcac ccctccatga ggaaggtggc aaatcttgag actccctatt agagagcttc 180

gattttcctg aaattgtgtt aggaaaatag ggtgacttgg tttgatcttg gtttctatac 240

ctattatggc tgcctgactc tggtcatttg gcccctgcag gcctaagcca cttggttttg 300

cttcacatat tggggtttat tagaacagta cgtagggaag canatgccag aggcacccgt 360

nccttttccc tgccttctag gtgctcctgg gaaat 395

<210> 293
<211> 557
<212> DNA
<213> Homo sapiens

<400>  293
accaagatct ctgcctggca caataacgtg gagaaaaccc tgccctccac caaggccacg      60

cgggtgggcg tgcttctcaa ctgtgaccac ggctttgtca tcttcttcgc tgttgccgac     120

aaggtccacc tgatgtataa gttcagggtg gactttactg aggctttgta cccggctttc     180

tgggtatttt ctgctggtgc cacactctcc atctgctccc ccaagtaggc aggctgtagg     240

cacttgggct gactgcctgc agaagtccca agaccctagt gaaaatacag caggcagaac     300

tctccttgga taattccccc aagaggtccc caaggattgg gagcatggga ggggagctgg     360

cgggagggtg ggaggtggga tttagccagg aaaggggtga gagtgattgt gttgtgggcg     420

aggaggcgtt tccaccccct ggtgcctatc agggcagggt gacctactcc ccattgttct     480

ggaaatctcc aggctgctgg gcagctgggc agctgggcag agctctggga agtgaagtca     540

tgagtgcccg attcctc                                                    557

<210>  294
<211>  547
<212>  DNA
<213>  Homo sapiens

<400>  294
ggttcgggtg agggcactct atgactacgc tggccaggaa gctgatgagc tgagcttccg      60

agcaggggag gagctgctga agatgagtga ggaggacgag cagggctggt gccaaggcca     120

gttgcagagt ggccgcattg gcctgtaccc tgccaactac gtggagtgtg tgggcgcctg     180

agtgtcctga cagcccttct gcaacgttta cccaccctgg ttcagagccc agcttctcct     240

ggagagccgg accctcaggg ccctgaaccg tcgctctctg gctgctcctc tgtcccttga     300

gggaggaagt cctgggaccc agggagggga ggggcctttg tctagggaag ggactggtag     360

ggaagggacg agtctaggct gagggcaaga tgggaggtca gaggtgacag aagcgttcag     420

gggtgcctgg gcctccccag gagctgtgga ctcagttcct gacctctgct ttggggttcc     480

tggggtgggc ttggggtgag tgtagttctg gcctagcagc accctcttgt ggcttgttct     540

agcgtgt                                                               547

<210>  295
<211>  147
<212>  DNA
<213>  Homo sapiens

<400>  295
tgtatgtgac caaaggtagg tcctgggatg acagcaatgc tgacactggc ctaaggagtt      60

actcatccat ttaataagta ttccagcaga tacagatgtg aacagtcaag tctctgccat     120

ccacaatgct tgtgttctaa tgcaaga                                         147

<210>  296
<211>  83
<212>  DNA
<213>  Homo sapiens

<400>  296
atgtgttcaa ccaagcggga aactctccgg gtagagtgaa atccgaagtt gctatgctac      60

aagataacct gggccgtgcg ccg                                              83


<210>  297
<211>  545
<212>  DNA
<213>  Homo sapiens

<400>  297
gtctttctga gagtttcatt gccattatca acaagagaag ttgaaattta caagtcagga      60

ggttattttt ccagattgat aaccatagaa agtgaataaa cacttttaag gtcgcaaaca     120

tttgctaggt tgtccttctc aatgcatgtg caggctgcat cctgtccttg tttttaagcc     180

agggtttata aataagtaga tttataccaa tcttaataga attgtatatt ttatgcaaga     240

attaaatgct ttacaacatg aagtataact caacccattg taaactttgg tggcaatatg     300

gatttgaaac tcgacagttc tcttgtattt gcttcctagg tttctgcatg caagttatga     360

caggtaggac tgaaaaaaca ctgccttttg acttctagca tttagcaacc gagagtcgta     420

gagtcaataa agctgtaagt gtcttcactt aatctgtggt tctcctaaaa ctattatctg     480

aaacctacag catcccacca tgaaatattt ggtaaattta tgttgtgacg tgttgcagca     540

tgtaa                                                                 545


<210>  298
<211>  485
<212>  DNA
<213>  Homo sapiens

<400>  298
aatttgtctg tgacccagat gccctcttct ccatggcttt cccggataac cagcgtccgt      60

tcctgaaggc agagtccgag tgccacctca gcgaggagga caccctgccg ctgacccact     120

ttgaagacag ccccgcttac ctcctggaca tggaccgctg cagcagcctc ccctatgccg     180

aaggctttgc ttactaagtt tctgagtggc ggagtggcca aaccctagag ctagcagttc     240

ccattcaggc aaacaagggc agtggttttg tttgtgtttt tggttgttcc taaagcttgc     300

cctttgagta ttatctggag aacccaagct gtctctggat tggcaccctt aaagacagat     360

acattggctg gggagtggga acagggaggg gcagaaaacc accaaaggcc cagtgcctca     420

actcttgatt ctgatgaggt ttctgggaag agatcaaaat ggagtctcct taccatggac     480

aatac                                                                 485


<210>  299
<211>  409
<212>  DNA
<213>  Homo sapiens


<220>
<221>  misc_feature
<222>  (36)..(36)
<223>  n is a, c, g, t or u

<220>
<221>  misc_feature
<222>  (38)..(38)
<223>  n is a, c, g, t or u

<220>
<221>  misc_feature
<222>  (40)..(40)
<223>  n is a, c, g, t or u

<220>
<221>  misc_feature
<222>  (312)..(312)
<223>  n is a, c, g, t or u

<400>  299
acagcttagc gatggagaaa atggcatccc tgttgntntn tcaccagata aattgcctgg      60

atctctggga cacccccgtc cccaggagaa ggatgtttgg gaagagatgg atgccaacaa     120

aaacaagata aagcttggaa tttgtaaggc tgctactgaa gaggagaaca gccatggcca     180

ggcaaatggt cttctcaatg ctccaagcct tgggtcacca attcgtgtcc gctcagagat     240

tactcagcca gacagagata ttccactggt gcgaaagtta cgttccattc acagctttga     300

gctggaaaaa cntctgaccc tggagccaaa gccagacact gacaagttcc ttgagacctg     360

gtataaaata gtgtattttt ctttttaaag cttctaaggt accattatt                 409


<210>  300
<211>  430
<212>  DNA
<213>  Homo sapiens


<220>
<221>  misc_feature
<222>  (150)..(150)
<223>  n is a, c, g, t or u

<220>
<221>  misc_feature
<222>  (164)..(164)
<223>  n is a, c, g, t or u

<220>
<221>  misc_feature
<222>  (170)..(170)
<223>  n is a, c, g, t or u

<220>
<221>  misc_feature
<222>  (173)..(174)
<223>  n is a, c, g, t or u

<220>
<221>  misc_feature
<222>  (185)..(185)
<223>  n is a, c, g, t or u

<220>
<221>  misc_feature
<222>  (187)..(187)
<223>  n is a, c, g, t or u

<220>
<221> misc_feature
<222> (207)..(207)
<223> n is a, c, g, t or u

<220>
<221> misc_feature
<222> (210)..(210)
<223> n is a, c, g, t or u

<220>
<221> misc_feature
<222> (220)..(220)
<223> n is a, c, g, t or u

<220>
<221> misc_feature
<222> (390)..(390)
<223> n is a, c, g, t or u

<220>
<221> misc_feature
<222> (393)..(393)
<223> n is a, c, g, t or u

<220>
<221> misc_feature
<222> (395)..(398)
<223> n is a, c, g, t or u

<220>
<221> misc_feature
<222> (400)..(400)
<223> n is a, c, g, t or u

<220>
<221> misc_feature
<222> (403)..(403)
<223> n is a, c, g, t or u

<400> 300
gagggccaag agctagggac agggggaaga gactggccca ggtggtaggg aggaaagaac 60

tcccagagtt tcctttagcc aggaaacctg ctctactgac cccgtgactt ggacagtcag 120

acatcaccct gagagtgaca agtgtaaaan tgactccctt cctnccccgn ccnncggaag 180

tatantnaga tacttgaaag cagtccnttn ctaaaatggn cttacctatg tggcctgaac 240

gattaaaaga aagaactcag agttacaagg gaaaaagaaa aagagttaca agggaattgt 300

agtctttttc tgaatagaat attagtactg tggtattgca tttcatggga atggaaatgt 360

attggtaaag ctacctgatg gaagctttcn ctngnnnncn aanatggagg gtgtattatg 420

tgcagttatt 430

<210> 301
<211> 536
<212> DNA
<213> Homo sapiens

<220>
<221> misc_feature
<222> (68)..(69)
<223> n is a, c, g, t or u

<220>
<221>  misc_feature
<222>  (72)..(72)
<223>  n is a, c, g, t or u

<220>
<221>  misc_feature
<222>  (74)..(76)
<223>  n is a, c, g, t or u

<220>
<221>  misc_feature
<222>  (78)..(82)
<223>  n is a, c, g, t or u

<220>
<221>  misc_feature
<222>  (100)..(100)
<223>  n is a, c, g, t or u

<220>
<221>  misc_feature
<222>  (121)..(121)
<223>  n is a, c, g, t or u

<220>
<221>  misc_feature
<222>  (129)..(129)
<223>  n is a, c, g, t or u

<220>
<221>  misc_feature
<222>  (134)..(134)
<223>  n is a, c, g, t or u

<220>
<221>  misc_feature
<222>  (168)..(168)
<223>  n is a, c, g, t or u

<220>
<221>  misc_feature
<222>  (216)..(216)
<223>  n is a, c, g, t or u

<220>
<221>  misc_feature
<222>  (228)..(228)
<223>  n is a, c, g, t or u

<220>
<221>  misc_feature
<222>  (254)..(254)
<223>  n is a, c, g, t or u

<220>
<221>  misc_feature
<222>  (276)..(276)
<223>  n is a, c, g, t or u

<400>  301
atcgaagaac aaagagtgct ccaaaaaata ggtcattctt ttattttcat aaagtatcta      60

aactgtanna anannnannn nngtgtttca ttctaaattn gcagctgaaa taaatttatt     120

ngcgatagna gaantatctt attattcatc ctcagaaata aaggattnga agggatagag     180

attatatgat aaatttatag aagactttca gaattntgaa tgcatttngt ttagtgttat 240

gaaatgacaa tagnaaaaaa gtctcgactt caattnaaaa gttacacaaa caaacaaatc 300

tacaggcatg tctttatata ccatcaggtc taagttttca aagaaaatgg tagatataac 360

tgcagataac tcattacagt cataatctct gcccatgtgt attgagaggg ggcagttgtg 420

cacgaaaaaa gaatttatgt ggccatttta ataaattcag tttaaaatag acttgtgtat 480

atgcatgaat catcagagat gaaactggtt tgagagactc atgtgaacct tacgaa 536

<210> 302
<211> 371
<212> DNA
<213> Homo sapiens

<400> 302
ctggtacgct gctgctgcag ttaccagaaa aactcataag caaatacaac tggatcaagc 60

aatggaaact tggactgaaa tttgatggga agaatgagga cctggttgat aaaattaaag 120

agtcccttac tctgctgagg aagaaggttt ggaacctgta gtgtcctgtc tgataagggt 180

gaagctctcg ttcttgcttg ccccagaaga ccagttttta gtcttcactc agtggatttt 240

caaatgctct tggctgattt ttaggcaaaa tggttttaaa tgaattcaaa ctcttcccac 300

gagggcttta gtaaaatggg aagtaccaac attatatatt cttagagcag atgccatgta 360

ctagggtatc a 371

<210> 303
<211> 355
<212> DNA
<213> Homo sapiens

<220>
<221> misc_feature
<222> (223)..(223)
<223> n is a, c, g, t or u

<220>
<221> misc_feature
<222> (227)..(227)
<223> n is a, c, g, t or u

<400> 303
gaagctgtgt ggagtggaag atggacattg aggaagaagg gcaggtgtgg tctcacccag 60

aatggttcct gctgcttccg cggtgcccag gcttttctca cggcctctgc tgggttctcc 120

cctgggtgct gtggatgcat cctgcctgct ggaaattctg tgctctctgt ttccatccct 180

ttgtcgtggt aatgaccgta tacctctccc ctgtaccctc ctntgcntgc tctccgtgca 240

ggcccctctc cctctggttg tcccatcagc atttccccac agctcgttgt tcctccttcc 300

tcttttctgg tgacctttct actgattgca ttgtacctct ttccctgata ttaaa 355

<210> 304
<211> 362
<212> DNA
<213> Homo sapiens

<400>  304
gcctgtgtct tcgggctgaa tttgatctgg ccatcccagg gggtctcctc cctgagtgcc      60

cttgtgcccc tgaacatgtt cactgaactg ctgatcgagt actatgaaaa gatcttcagc     120

accccggagg cacctgggga gcacggcctg gcaccatggg aacaggggag cagggcagcc     180

cctttgcagg aggctgtgcc acggacacaa gccacgggcc tcaccaagcc taccctacct     240

ccgagtcccc tgatggcagc cagaagacgt ctctagtgtt gcgaacactc tgtatgtttc     300

gagctacctc ccacacctgt ctgtgcactt gtatgttttg taaacttggc atctgtaaaa     360

at                                                                    362

<210>  305
<211>  533
<212>  DNA
<213>  Homo sapiens

<400>  305
cgaagagcaa gacccactct gttccagaag ccctataagc tggaggtgga caactcgatg      60

taaatttcat gggaaaaccc ttgtacctga catgtgagcc actcagaact caccaaaatg     120

ttcgacacca taacaacagc tactcaaact gtaaaccagg ataagaagtt gatgacttca     180

cactgtggac agtttttcca aagatgtcag aacaagactc cccatcatga taaggctccc     240

acccctctta actgtccttg ctcatgcctg cctctttcac ttggcaggat aatgcagtca     300

ttagaatttc acatgtagta gcttctgagg gtaacaacag agtgtcagat atgtcatctc     360

aacctcaaac ttttacgtaa catctcaggg gaaatgtggc tctctccatc ttgcatacag     420

ggctcccaat agaaatgaac acagagatat tgcctgtgtg tttgcagaga agatggtttc     480

tataaagagt aggaaagctg aaattatagt agagtctcct ttaaatgcac att            533

<210>  306
<211>  434
<212>  DNA
<213>  Homo sapiens


<220>
<221>  misc_feature
<222>  (131)..(131)
<223>  n is a, c, g, t or u

<220>
<221>  misc_feature
<222>  (191)..(191)
<223>  n is a, c, g, t or u

<220>
<221>  misc_feature
<222>  (205)..(205)
<223>  n is a, c, g, t or u

<400>  306
ggaaccctcc tcttggcaag ggctttccga agttaacctg aaaaactggt tcaggccatg      60

acagcaaagg gttggatagc ctcattatcc ctcctccctt cagaactctg gaacagccag     120

cgttaacatc nacacaggcc ttcagtctga tgagaaacat ttaccatcta ttgtctcgga 180

agcctgctac ntggaggctt catcntgatg ataaagcctt ggtctccaca accccgtata 240

acccagacat tcctttctat tgataactct tgcaagcgat tgccaaccag aagatgttta 300

aatccaccta taacctggaa gcccccagtt ccagctgccc acctttctgg actaaaccaa 360

tgtatatctt caatatattt gattgatgtc tcatgtctcc ctaaaatggg taccatcaag 420

ctgtgcactg acca 434

<210> 307
<211> 157
<212> DNA
<213> Homo sapiens

<400> 307
cctccgcaca ctggatgaga atccatcttc cattcgagct gggaatagac tttgtgaaag 60

atattatgta atggagtctc gggaaccctg agacctctcc agcgaagctg aagtgaatta 120

attaagtgct ttaaacggtc ttggtgctgt gttacgg 157

<210> 308
<211> 367
<212> DNA
<213> Homo sapiens

<220>
<221> misc_feature
<222> (35)..(35)
<223> n is a, c, g, t or u

<220>
<221> misc_feature
<222> (38)..(40)
<223> n is a, c, g, t or u

<220>
<221> misc_feature
<222> (43)..(47)
<223> n is a, c, g, t or u

<220>
<221> misc_feature
<222> (52)..(53)
<223> n is a, c, g, t or u

<220>
<221> misc_feature
<222> (58)..(58)
<223> n is a, c, g, t or u

<220>
<221> misc_feature
<222> (60)..(60)
<223> n is a, c, g, t or u

<220>
<221> misc_feature
<222> (62)..(64)
<223> n is a, c, g, t or u

<220>

<221>  misc_feature
<222>  (67)..(68)
<223>  n is a, c, g, t or u

<220>
<221>  misc_feature
<222>  (71)..(71)
<223>  n is a, c, g, t or u

<220>
<221>  misc_feature
<222>  (73)..(73)
<223>  n is a, c, g, t or u

<220>
<221>  misc_feature
<222>  (75)..(75)
<223>  n is a, c, g, t or u

<220>
<221>  misc_feature
<222>  (79)..(79)
<223>  n is a, c, g, t or u

<220>
<221>  misc_feature
<222>  (82)..(82)
<223>  n is a, c, g, t or u

<220>
<221>  misc_feature
<222>  (84)..(86)
<223>  n is a, c, g, t or u

<220>
<221>  misc_feature
<222>  (89)..(91)
<223>  n is a, c, g, t or u

<220>
<221>  misc_feature
<222>  (93)..(93)
<223>  n is a, c, g, t or u

<220>
<221>  misc_feature
<222>  (289)..(289)
<223>  n is a, c, g, t or u

<400>  308
aggtgatgca ctatgcccag tacgtcctcc tggcnctnnn ctnnnnngcg tnncctgntn      60

cnnntcnntg ncntntgcna antnnngann nanaaccgtg taaaaccatt tttatgtggc     120

ttcaacgtca actataaatt agcttggtta tcttctagga gaaatgctat ttattttgga     180

gtagtagtaa aaagggctca aaggataagg aggccattca ggcctattct gaatccctga     240

tgacatcagc tcccaagggc tctgtgctgc aggaagcaaa actgtaggng ggtaccaggt     300

aatgccgtgc gcctccccgc cccctcccat atcaagtaga atgctggcgg cttacagact     360

gaagatg                                                               367


<210>  309
<211>  484
<212>  DNA

<213>  Homo sapiens

<400>  309
accccaccac gtaccagatg gatgtgaacc ccgagggcaa atacagcttt ggtgccacct      60

gcgtgaagaa gtgtccccgt aattatgtgg tgacagatca cggctcgtgc gtccgagcct     120

gtggggccga cagctatgag atggaggaag acggcgtccg caagtgtaag aagtgcgaag     180

ggccttgccg caaagtgtgt aacggaatag gtattggtga atttaaagac tcactctcca     240

taaatgctac gaatattaaa cacttcaaaa actgcacctc catcagtggc gatctccaca     300

tcctgccggt ggcatttagg ggtgactcct tcacacatac tccccctctg gatccacagg     360

aactggatat tctgaaaacc gtaaaggaaa tcacaggttt gagctgaatt atcacatgaa     420

tataaatggg aaatcagtgt tttagagaga gaacttttcg acatatttcc tgttcccttg     480

gaat                                                                  484

<210>  310
<211>  526
<212>  DNA
<213>  Homo sapiens

<400>  310
ccatggggcc atctgggcca ttcagagact ggagtgagat ttgggtgtgg agggggaggc      60

gccaaggtgg aggagcttcc cactccagga ctgttgatga aagggacaga ttgaggagga     120

agtgggctct gaggctgcag ggctggaagt ccttgcccac ttcccactct cctgccccaa     180

tctatctagt acttcccagg caaataggcc cctttgaggc tcctgagtgc cctcagatgg     240

tcaaaaccca gttttccctc tgggagccta aaccaggctg catcggaggc caggacccgg     300

atcattcact gtgataccct gccctccaga gggtgcgctc agagacacgg gcaagcatgc     360

ctcttccctt ccctggagag aaagtgtgtg atttctctcc cacctccttc cccccaccag     420

acctttgctg ggcctaaagg tcttggccat ggggacgccc tcagtctagg gatctggcca     480

cagactccct cctgtgaacc aacacagaca cccaagcaga gcaatc                    526

<210>  311
<211>  319
<212>  DNA
<213>  Homo sapiens

<220>
<221>  misc_feature
<222>  (264)..(264)
<223>  n is a, c, g, t or u

<400>  311
taaattgcct ggatctctgg gacacccccg tccccaggag aaggatgttt gggaagagat      60

ggatgccaac aaaaacaaga taaagcttgg aatttgtaag gctgctactg aagaggagaa     120

cagccatggc caggcaaatg gtcttctcaa tgctccaagc cttgggtcac caattcgtgt     180

ccgctcagag attactcagc cagacagaga tattccactg gtgcgaaagt tacgttccat     240

tcacagcttt gagctggaaa aacntctgac cctggagcca aagccagaca ctgacaagtt     300

ccttgagacc tggtataaa 319

<210> 312
<211> 234
<212> DNA
<213> Homo sapiens

<220>
<221> misc_feature
<222> (85)..(87)
<223> n is a, c, g, t or u

<220>
<221> misc_feature
<222> (89)..(90)
<223> n is a, c, g, t or u

<220>
<221> misc_feature
<222> (92)..(92)
<223> n is a, c, g, t or u

<220>
<221> misc_feature
<222> (94)..(95)
<223> n is a, c, g, t or u

<220>
<221> misc_feature
<222> (97)..(97)
<223> n is a, c, g, t or u

<220>
<221> misc_feature
<222> (186)..(186)
<223> n is a, c, g, t or u

<400> 312
gcgcttgcgc agtagctgaa cgcgggcgtt tctttcctcc ctttttttcg aattggtttt 60

gggggtagat tcgagttaca aaatnnncnn cngnngngtg ttcggcgcgg ttcccccagc 120

tgtctctggc tgaaccggcg ctctcgcctc cctgccgaac acagcgtgag gagccccccc 180

aggganatgg tgtttgagtc tctgggcttg ccgagcacta agtcctctga gttc 234

<210> 313
<211> 125
<212> DNA
<213> Homo sapiens

<400> 313
gtactgcaaa aatcaccctc ggcaagacga atgtctgacg tgccggaagg agtcatacgg 60

gtccatgctc cacttctctc caaggtgtcc atggccattc aactcaacaa tcaaaccaaa 120

gccaa 125

<210> 314
<211> 446
<212> DNA
<213> Homo sapiens

<220>
<221>  misc_feature
<222>  (53)..(53)
<223>  n is a, c, g, t or u

<220>
<221>  misc_feature
<222>  (93)..(93)
<223>  n is a, c, g, t or u

<220>
<221>  misc_feature
<222>  (130)..(130)
<223>  n is a, c, g, t or u

<220>
<221>  misc_feature
<222>  (205)..(205)
<223>  n is a, c, g, t or u

<220>
<221>  misc_feature
<222>  (211)..(211)
<223>  n is a, c, g, t or u

<400>  314
aagtcattcg tttaagcgtg gattattttg ccgaatgaat aatgatgatg gcngctttca      60

tctcttatga agttttcctg gccaagagcc agnagttgga agtttggatc attctttttt     120

cttttttaan catttcttct cttctttctc ttttttatca ctaaatgaat gacatgtgga     180

gaaactattc agcttttaaa gtatnctcca nttacttgtc tcaactacca ctatttattg     240

tgtttatcaa aatcataaaa agctcatttt tggcatttac cttcgtggtt gagactgctg     300

tctgtatgtc tgggaatgga agtcctcttc agggattcag caagggctgt acttttgctt     360

aatactagtg gttccttatt ctaagtgatg acatcatcca cctttcctag aaatgggtct     420

ttgtgcctag tatgatatct ttccaa                                          446


<210>  315
<211>  473
<212>  DNA
<213>  Homo sapiens


<220>
<221>  misc_feature
<222>  (207)..(207)
<223>  n is a, c, g, t or u

<220>
<221>  misc_feature
<222>  (264)..(264)
<223>  n is a, c, g, t or u

<220>
<221>  misc_feature
<222>  (375)..(375)
<223>  n is a, c, g, t or u

<220>
<221>  misc_feature
<222>  (395)..(395)

<223>  n is a, c, g, t or u

<220>
<221>  misc_feature
<222>  (405)..(405)
<223>  n is a, c, g, t or u

<400>  315
tgtttcaggc ccatccacag ttgaagcagt gtgtgcgtca ggcaattgaa cgggctgtcc      60

aggagctggt ccatcctgtg gtggatcgat caattaagat tgccatgact acttgtgagc     120

aaatagtcag gaaggatttt gccctggatt cggaggaatc tcgaatgcga atagcagctc     180

atcacatgat gcgtaacttg acagctngga atggctatga ttacatgcag ggaacctttg     240

ctcatgagca tatctaccaa cttnaaaaaa cagttttgcc tcagcccttc gtgtaagttg     300

gctatttcct tggtataggt acaaaacgta ttactgcttg tctgtaataa tttttttctt     360

tgtctatata tggcnctggg cgttaccact tattnttaat aatcnccata tttgtttgat     420

gtcttccatc attttagatt gtaattctgt gaggcaaagc atcatgtctg tgt           473


<210>  316
<211>  576
<212>  DNA
<213>  Homo sapiens


<220>
<221>  misc_feature
<222>  (63)..(63)
<223>  n is a, c, g, t or u

<220>
<221>  misc_feature
<222>  (351)..(352)
<223>  n is a, c, g, t or u

<220>
<221>  misc_feature
<222>  (395)..(395)
<223>  n is a, c, g, t or u

<220>
<221>  misc_feature
<222>  (496)..(496)
<223>  n is a, c, g, t or u

<400>  316
aggacaccag gctggtggcc acagtgctgc tgtccgtggt cgtgctgctc cacgccctcc      60

tgnccatggg ctgtaagttg tacttcttcc agtcgctgcc tccggagaac gtggctcctc     120

caccccaaat cacatctctg ccctcaaaca tcgcgctgtc ccctaccttg ccgcagtccc     180

tggccccctc ctaggaaggc ccgggtccca caggcaacac ctaagtggac caacccctct     240

gcctgtcctg ccccccagac gatgactgaa ggctcctttg acaccttgag atgattctgc     300

tactttccag acttttctta caaagcaaac acttttattt tctatgcaaa nntgattcag     360

agaatttata taaaggcggg cgaggggcag ccgancaggg agctttggga cagggctggg     420

gcccccatat ccccccgggg ccacctgctt tccctcctat ggctcccctg gaacaggagg     480

gagagccaag ggggcngccc agcctggaca gcgcccgctc ctgcctgggt gcacacacgg 540

cgggcctgag ctccagcatc tgagtttggg ggtatg 576

<210> 317
<211> 265
<212> DNA
<213> Homo sapiens

<400> 317
ccaggagcag ctgcgtgacg tcatgttcta cctggagaca cagcagaaga tcaaccatct 60

gcctgccgag acccggcaga aatccaggag ggacagatca acatcgccat ggcctcggcc 120

tcgagccctg cctcttcggg gggcagtggg aagttgccct ccaggaaggg ccgcagcaag 180

aggggcaagt gaccttcaga gcaacagaca tccctgagac tgttctccct gacactgtga 240

gagtgtgctg ggaccttcag ctaaa 265

<210> 318
<211> 515
<212> DNA
<213> Homo sapiens

<220>
<221> misc_feature
<222> (108)..(108)
<223> n is a, c, g, t or u

<220>
<221> misc_feature
<222> (115)..(115)
<223> n is a, c, g, t or u

<220>
<221> misc_feature
<222> (203)..(203)
<223> n is a, c, g, t or u

<220>
<221> misc_feature
<222> (241)..(241)
<223> n is a, c, g, t or u

<400> 318
atacgtgggt agtgttgcat ttcaaatgag gctcttctgg ttgaaatgat atatttataa 60

gaccagaata tcacaaatgg gtgatgtata atgtctcttt agtttttngg tattnggcct 120

cttttaaagc ctgtcggatg tatgggagaa aacaatgaac gtgctttgat ttcctatcag 180

tcactcttaa gaacatacat atngtttaag taactcggtc ttttttatct gattcttgag 240

ncactatggg tagcaagtaa ccacttacaa atttaaatgt aatatacact ccttttctgt 300

gtgtcaagtc cttattttta ggtgcatatt gacatttaaa tgttaattat tgtttggcat 360

ataatatcaa aaatctatta tttattttat gctgttacag ttaaaagatg tgatttatga 420

catactgaat caacttgcct tccaatttag tgtgtaatat ggtaagcatt tatactttta 480

gatatgtctt atttttattt ggatgcctgt ctacc 515

<210>  319
<211>  541
<212>  DNA
<213>  Homo sapiens


<220>
<221>  misc_feature
<222>  (136)..(136)
<223>  n is a, c, g, t or u

<220>
<221>  misc_feature
<222>  (141)..(141)
<223>  n is a, c, g, t or u

<220>
<221>  misc_feature
<222>  (147)..(147)
<223>  n is a, c, g, t or u

<220>
<221>  misc_feature
<222>  (159)..(159)
<223>  n is a, c, g, t or u

<220>
<221>  misc_feature
<222>  (161)..(161)
<223>  n is a, c, g, t or u

<220>
<221>  misc_feature
<222>  (167)..(167)
<223>  n is a, c, g, t or u

<220>
<221>  misc_feature
<222>  (172)..(172)
<223>  n is a, c, g, t or u

<220>
<221>  misc_feature
<222>  (181)..(181)
<223>  n is a, c, g, t or u

<220>
<221>  misc_feature
<222>  (188)..(188)
<223>  n is a, c, g, t or u

<220>
<221>  misc_feature
<222>  (191)..(191)
<223>  n is a, c, g, t or u

<220>
<221>  misc_feature
<222>  (195)..(195)
<223>  n is a, c, g, t or u

<220>
<221>  misc_feature
<222>  (220)..(220)
<223>  n is a, c, g, t or u

<220>
<221>  misc_feature

<222>  (260)..(260)
<223>  n is a, c, g, t or u

<220>
<221>  misc_feature
<222>  (509)..(509)
<223>  n is a, c, g, t or u

<400>  319
gagttaatgc agcactcgtc attcagaaat attggcgaag agtcttagca cagagaaaat      60

tattaatgtt aaaaaaggaa aagctggaaa aagttcaaaa taaagcagca tcacttattc     120

agggatattg gagaanatat nccactngac aaagatttnc ngaaatngaa anattattca     180

ntcatccngc naatntagga taagaatgat aattgctgtn acatcttata aacgatatct     240

ttgggctaca gttacaattn cagaggcatt ggcgtgctta tttaagaaga aaacaagatc     300

aacaaagata tgaaatgcta aaatcatcaa ctcttataat ccaatctatg ttcagaaaat     360

ggaagcaacg taaaatgcaa tcacaagtaa aagctacagt aatattgcaa agagctttta     420

gagaatggca tttaagaaaa caagctaaag aagaaaattc tgctattatc atacaatcat     480

ggtatagaat gcataaagaa ttacggaant atatttatat tagatcttgt gttgttatca     540

t                                                                     541

<210>  320
<211>  495
<212>  DNA
<213>  Homo sapiens

<220>
<221>  misc_feature
<222>  (144)..(145)
<223>  n is a, c, g, t or u

<400>  320
cttcggattt ttattgactc aaaatagtgc cattcccctt aatgaaatag attttgagtc      60

tttttttcat tgtaaccccc aaatgagaat catctacctg attcttgtac caaaaaaaaa     120

tttttttcag tctttttttt tttnnagaga gggtctcttg tcaacgcaag actgggagtg     180

gcagtggcac gatcttagct cactacaact tctggcctcc caggctcaag caattctcct     240

gcctcagcct cctgagtagc tggggattac aggcatgcac caccacgccc agctaatttt     300

ggtattttta gtagagacag ggtttcacca ttgtttggcc aggctggtcc cgaactcctg     360

acctcaggtg atccacccac ctcggcctcc caaagtgctg ggattatagg tgcgagccat     420

tgcgcccagc ctcagttatt ttatttaaca gtgtaagtac ttagaaagta agaaaatggc     480

gtgattagtt ttttg                                                      495

<210>  321
<211>  429
<212>  DNA
<213>  Homo sapiens

<400>  321
ggctgaggag gctggtctga acatcactca catttgcctc cctccagata gcagtgaagc      60

cgagattata gatgaaatct taaagatcaa tgaagatacc agagtacatg gccttgccct 120

tcagatctct gagaacttgt ttagcaacaa agtcctcaat gccttgaaac cagaaaaaga 180

tgtggatgga gtaacagaca taaacctggg gaagctggtg cgaggggatg cccatgaatg 240

ttttgtttca cctgttgcca aagctgtaat tgaacttctt gaaaaatcag taggtgtcaa 300

cctagatgga aagaagattt tggtagtggg ggcccatggg tctttggaag ctgctctaca 360

atgcctgttc cagagaaaag ggtccatgac aatgagcatc cagtggaaaa cacgccagct 420

tcaaagcaa 429

<210> 322
<211> 467
<212> DNA
<213> Homo sapiens

<400> 322
tctgagggtg ccttgatgct ggctcatcac acattgagta tcttgggcat tatcatggcc 60

cttgtgcttg gggagtctgg cacagaggtc aatgcagtcc tctttggaag tgagcttacc 120

aaccccttgc tacagatgcg ctggtttctc cgggaaacag ggcactatca cagtttcact 180

ggagatgtag tggacttcct ctttgtggct ctgttcacag gagtgaggat tggtgtggga 240

gcttgcctcc ttttctgtga aatggtctcc cccacgccta agtggtttgt gaaggctggg 300

ggagtagcga tgtatgctgt gtcttggtgt ttcatgttta gcatctggcg ctttgcatgg 360

aggaagagca tcaagaagta ccatgcttgg agaagcaggc ggagtgagga acggcagctg 420

aaacacaacg gacatctcaa aatacactag ccaaggcttg ctccaga 467

<210> 323
<211> 504
<212> DNA
<213> Homo sapiens

<400> 323
ttggcacttc agaagtctcc ccaatcttga caaagccctg gagaaagggc cgggcctccc 60

gttgataaga atatcactgc agataaatgg aggtttcaaa ttgaaagaaa ggaggagggc 120

ctcctgttga taagattatt gtcactgcag gtaaatggag gcttcaaata gaaatacatt 180

tcagttacag aaaaaaaaat tatctttgtt acacatttga gtttgcaggc ctaaggttac 240

tcccgctaca ctatcatctg taaccataac gcactcaaca ttttaagcta actataagga 300

ttgttgcttc actcaaagat cctgaggttt tattcactaa catttttatt tggtgactat 360

agttgacaag aacaaagctg tggggaacca acaaacactg caatgcctgg cattgtcacc 420

tcactagatt gtgagttcct ctgggacagg gtccgtacat tttcttagaa tccctcactt 480

agccattagc ctgcacagtg cttg 504

<210> 324
<211> 163
<212> DNA
<213> Homo sapiens

<400>  324
catggaggag tgcatttcct tggctattcc agaagtccta cctcccttct gagattttat      60

aatggtattt cttatggtta tcccaaatat acttggcaag tcgtcttata aaccaccaat     120

aatagcctct taaaaattca aaaattactc ctcttggcta aca                       163


<210>  325
<211>  441
<212>  DNA
<213>  Homo sapiens

<400>  325
cctccgcgga aggcgtggca gggaggcagt cgccctgcgg tgcaagctgc tgctccagag      60

cataccgtgg cccaggtggt atccccaagg cctcgtgccg tggctggggt cctgggaggt     120

ggtcgccctg cagtgcaagc tgctgctcca gagcgtaccg tggcccagac tgatcctcga     180

ggcctcctgc cgtggctggg gtcatggtcg gctgcgcatg tccagaagca tttccttcct     240

gcgaccatcc cggcgcccct agggggagaa gccaggacag cagcttccgc tgtctccaca     300

gcagacacgg gacggattcc acagacggga gcctcattcg taccatgcca aacgcattca     360

ctcggggcag tattaaccgt tctagaaagc cactgtttta tagcaaaaca ggaaaggaaa     420

agctaccagt tttttattca g                                               441


<210>  326
<211>  457
<212>  DNA
<213>  Homo sapiens

<400>  326
tttcccctag ttgacctgtc tataagagaa ttatatattt ctaactatat aaccctagga      60

atttagacaa cctgaaattt attcacatat atcaaagtga gaaaatgcct caattcacat     120

agatttcttc tctttagtat aattgaccta ctttggtagt ggaatagtga atacttacta     180

taatttgact tgaatatgta gctcatcctt tacaccaact cctaatttta aataatttct     240

actctgtctt aaatgagaag tacttggttt tttttttctt aaatatgtat atgacattta     300

aatgtaactt attatttttt ttgagaccga gtcttgctct gttacccagg ctggagtgca     360

gtgggtgatc ttggctcact gcaagctctg ccctccccgg gttcgcacca ttctcctgcc     420

tcagcctccc aattagcttg gcctacagtc atctgcc                              457


<210>  327
<211>  438
<212>  DNA
<213>  Homo sapiens


<220>
<221>  misc_feature
<222>  (65)..(65)
<223>  n is a, c, g, t or u

<220>
<221>  misc_feature

<222>  (96)..(96)
<223>  n is a, c, g, t or u

<220>
<221>  misc_feature
<222>  (112)..(112)
<223>  n is a, c, g, t or u

<220>
<221>  misc_feature
<222>  (218)..(218)
<223>  n is a, c, g, t or u

<220>
<221>  misc_feature
<222>  (229)..(229)
<223>  n is a, c, g, t or u

<400>  327
ttgtccttta tgtatcttct ttccatagtg cttactggag ccttccaaaa taatgtctcc      60

tcaangtgac agcccctcag gaatttgaag gcaatngtca caccctcacc cnctttcctg     120

agttttttct ggtttattaa cgtcagtctt tacagtcagt gctcattgac ggtggttttc     180

tctggttgtt tcctgaacac gtagtgctct taaagcantg ccctgaggng aatacaattc     240

tccaggggca ttctgattgg caggtgaagc acagtgccat gttcccagca ctgatttggg     300

aagtggcttg tcacatccca cagtgaactc agtcaactgg aatgcctaac tctctttcat     360

aagacctcct gctacattat gtttctccca gactgtactc aggtccaaga acagaattta     420

ctagtctatc cttctcaa                                                   438

<210>  328
<211>  535
<212>  DNA
<213>  Homo sapiens

<220>
<221>  misc_feature
<222>  (40)..(40)
<223>  n is a, c, g, t or u

<400>  328
cccttcttgc tgccacagga tgaataaagt gttgagattn gtctatggag aaagctgtgt      60

gtctgttttt atctcccctc tcaggaccag tcagccactg gtcaatcagg ctgatcatgg     120

aacattagga attctccaat taagggagaa aaagtccagg gacttagtta tatcttcaga     180

ccagtgcagc tgggacacac aaagttctcc tgtctcacca tctgatatgg tttggatgct     240

cgtcccctcc aaatctcatg ttgaaatgta attcccagtg ttggaagtgg agcctggtgg     300

gaagtatttg gatcatgaga gaggatcctt catgaatggc tcagcaccat ctccttggtg     360

atgagtgagt tctcactcaa ttcacataga tatggttgtt taaaagagtc tgagacctct     420

cccctctttc tcgccatgtg atatgcctgc tcccccttca ccttccgcct ttactgtaag     480

cttcctgagg ccctcaccag aagctgagca aatgttggtg ccatgccagt acagc          535

<210>  329

<211>  432
<212>  DNA
<213>  Homo sapiens

<400>  329
gccacagact gaactcgcag ggagtgcagc aggaaggaac aaagacaggc aaacggcaac      60

gtagcctggg ctcactgtgc tggggcatgg cgggatcctc cacagagagg aggggaccaa     120

ttctggacag acagatgttg ggaggataca gaggagatgc cacttctcac tcaccactac     180

cagccagcct ccagaaggcc ccagagagac cctgcaagac cacggaggga gccgacactt     240

gaatgtagta ataggcaggg ggccctgcca ccccatccag ccagacccca gctgaaccat     300

gcgtcagggg cctagaggtg gagttcttag ctatccttgg ctttctgtgc cagcctggct     360

ctgcccctcc cccatgggct gtgtcctaag gcccatttga gaagctgagg ctagttccaa     420

aaacctctcc tg                                                         432

<210>  330
<211>  234
<212>  DNA
<213>  Homo sapiens

<400>  330
agcaaatcta gctttcagta ttcctaattt ttacctaagc tcattgctcc aggctttgat      60

tacctaaaat aagcttggat aaaattgaac caacttcaag aatgcagcac ttcttaatct     120

ttagctcttt cttgggagaa gctagacttt attcattata ttgctatgac aacttcactc     180

tttcataata tataggataa attgtttaca tgattggacc ctcagattct gtta           234

<210>  331
<211>  317
<212>  DNA
<213>  Homo sapiens

<400>  331
acttaggagt ggtgcttttt ctcagaaaac aggccacggt gtttcataca gaatgtcttc      60

atatcatctg aaatggtatg gctgaagttc atttgtttac agggtcggga atgtcttcag     120

ttcttgagag tcaacagtaa tgattggttg taagccaagg gacattttaa gctagtgaag     180

agttttttct ggaattgatt tttcccaaaa gaatatatta attgaggtta agaagtcagt     240

gggaaacaca cagaaatttg ttttaaaatc tttcaggagc tttactgaaa gacttggtta     300

tcaagtcttt tggggag                                                    317

<210>  332
<211>  415
<212>  DNA
<213>  Homo sapiens

<400>  332
gacttacttt aacaaccagc caatccctac ctaagcctag tagccatggt ttggctaaga      60

ccgcagcgac tgtatttagt aaatcctttg aacaagtcag tggtgtcaca gtcccacata     120

acccgtcatc tgctgttggt tgtggggctg ggacagatgc caataggttt tccgcttgta     180

gtctccaaga agaaaagctt atttacgttt cagaaagaac tgaacttcca atgaagcatc 240

aatcaggtca gcagagacct cctagtatta gcattactct gtccacagat taattagtaa 300

catatttttc tcccataacc tagtgaacct ggaaatacaa ctttgcttct ttatgaaagt 360

accctgggtc tttcatccgt attcctgaca ggagccctga tgtcttaaat tctga 415

<210> 333
<211> 489
<212> DNA
<213> Homo sapiens

<400> 333
gacgggtcca ttaacaaagc gggctttgcc gtcaactttt tcaaagaggt ggacgagtgc 60

tctcggccca accgcggggg ctgtgagcag cggtgcctca acaccctggg cagctacaag 120

tgcagctgtg accccgggta cgagctggcc ccagacaagc gccgctgtga ggctgcttgt 180

ggcggattcc tcaccaagct caacggctcc atcaccagcc cgggctggcc caaggagtac 240

ccccccaaca agaactgcat ctggcagctg gtggccccca cccagtaccg catctccctg 300

cagtttgact tctttgagac agagggcaat gatgtgtgca agtacgactt cgtggaggtg 360

cgcagtggac tcacagctga ctccaagctg catggcaagt tctgtggttc tgagaagccc 420

gaggtcatca cctcccagta caacaacatg cgcgtggagt tcaagtccga caacaccgtg 480

tccaaaaag 489

<210> 334
<211> 239
<212> DNA
<213> Homo sapiens

<400> 334
   cacagataga acctgcacat tgcccattaa tgcacacttg tgtatgccta ttacagtctg 60
   tgaagtttgg tttagggtca gatgctgggc agagagctgt gaagccatta catattcctt 120
   cccttgcaca gtctgaatca tcccgacact tctcagactt tgacttgaat gcacactgtg 180
   ctgtacaaca aggaccttga cttggactgc actgtttccc aggtttcagt ttgcatttt 239

<210> 335
<211> 432
<212> DNA
<213> Homo sapiens

<400> 335
gccctgactg actgtattct ctggccacat tcaagtcccc cattggtggg ggcagagaag 60

taggaccagg ccatccttgg ctacagagct cgaagacccc aagacagccc tctgctctca 120

gcggcgccac agagagcctg ggctcagcct tctgcatcag gacatggcct cgtccactga 180

gggcacgatt taaacatttg acatcagaag ctttatttgt aaacctcaca cagataagga 240

ccaagggctg gcggtgtggc cagaggacag gggaagctga aggccccgtg cttgagctcg 300

gcagtcctgc tccttgcagt gaagccacca tgggtgaccg tccagcctca cccggtggcc 360

tgcacagtga gggaagggct tcagggccat ctgctcccag ggcaggggac aggccaccaa 420

ggacctttgg ca                                                          432

<210>  336
<211>  380
<212>  DNA
<213>  Homo sapiens

<400>  336
aatgttgaca tatttcctct atctcataga tggtaaaagt gttgctttta aactggcaaa      60

tgcactcttc agaaatcctt ttctatctga tccacatgga gaggttaaag gttcaatttc     120

atgacctcta tgcaggcagc gctctcattg gatgtaagaa tattacctgc aaggatagaa     180

tgcagttgtg caacagagac acattcttat ttcttttttt tcacaatttt gttttgtttt     240

taatgaccct tttattgaat attggactga aatataaatt ttaaaaaaca cgttggaaag     300

gatgtacaac agaaggctat gtatgtatat acagtatgtc aaaagccttt tatttttata     360

cttcaaatgc tctaaattaa                                                 380

<210>  337
<211>  544
<212>  DNA
<213>  Homo sapiens

<400>  337
gagtctctgc ttgataagtg cctctatacc aaccgctctc ctcatcctga catcttgata      60

cggacttctg gagaagtgcg gctgagtgac ttcttgctat ggcagacctc tcactcctgc     120

ctggtgttcc aacccgttct gtggccagag tatacatttt ggaacctctt cgaggccatc     180

ctgcagttcc agatgaacca tagcgtgctt cagaaggccc gagacatgta tgcagaggag     240

cggaagaggc agcagctgga gagggaccag gctacagtga cagagcagct gctgcgagag     300

gggctccaag ccagtgggga cgcccagctc cgaaggacac gcttgcacaa actctcggcc     360

agacgggaag agcgagtcca aggcttcctg caggccttgg aactcaagcg agctgactgg     420

ctggcccgtc tgggcactgc atcagcctga atgaggctgg ccacctgcca ctttgccctg     480

ccctctgcct ccagggctcc actccccttc cttttcttgg tgaaaggcac ctcctttcct     540

gata                                                                  544

<210>  338
<211>  530
<212>  DNA
<213>  Homo sapiens

<400>  338
tcaaagaacg cgtactgcag accccaaatg accttctggc tgctggcttt gaggagcaca      60

agttcagaaa cttcttcaat gctttttaca gtgtggtgga actggtagag aaggacggct     120

cagtgtccag cctgctgaag gtgttcaacg accagagtgc ctcggaccac atcgtgcagt     180

tcctgcgcct gctcacgtcg gccttcatca ggaaccgagc agacttcttc cggcacttca     240

ttgatgagga gatggacatc aaagacttct gcactcacga agtagagccc atggccacgg     300

agtgtgacca catccagatc acggcgttgt cgcaggccct gagcattgcc ctgcaagtgg 360

agtacgtgga cgagatggat accgccctga accaccacgt gttccctgag gccgccaccc 420

cttccgttta cctgctctat aaaacatccc actacaacat cctttatgca gccgataaac 480

attgattaat tttaggccat gcagtggaac ctgtcaccta atgggactgc 530

<210> 339
<211> 75
<212> DNA
<213> Homo sapiens

<400> 339
agtcatgcga ccaggtgagg gtccacgtcc ccaagcttcc actccctctg gtgtttccca 60

tttaagtata ctgtt 75

<210> 340
<211> 376
<212> DNA
<213> Homo sapiens

<400> 340
gatgctcacg tcacttggtg taggtttcag gatcgcctct ttgaggaagg acttcaggac 60

caactggggc ctgcataaga aaacttatct cattattaga gtactcacag cttgtatctc 120

ccagctacat cctagaaccc cattgtcctt tattccacca aaccagctcc aggtgaccag 180

actctactca gaaagcaaat tcgtcatcaa agaacagaga ctggccacca caaggacatg 240

caggagaact gtcgggacca ggaagactca ttccaaaaag cccaggccgg gcacagtcgt 300

caagcctgta atcccaacac tttgggagac cgaggtgggg gtatcgattg agcctcggag 360

gtcgagatca gcctgg 376

<210> 341
<211> 499
<212> DNA
<213> Homo sapiens

<400> 341
ccccgcctgt ggcattttct atgggctcag gttacacctt cccagctggt gtttctgtcc 60

caggaacctt tcttcagcct acagctcact ctccagcagg aaaccaggtg caagctggga 120

aacagtccca cattccttac agccagcaac ggccctctgg accagggcca atgaaccagg 180

gacctcaaca atcacagcca ccttcccagc aaccccttac atctttacca gctcagccaa 240

cagcacagtc tacaagccag ctgcaggttc aagctctaac tcagcaacaa caatccccta 300

caaaagctgt gccggctttg gggaaaagcc cgcctcacca ctctggattc cagcagtatc 360

aacaggcaga tgcctccaaa cagctgtgga atccccctca ggttcaaggc ccattaggga 420

aaattatgcc tgtgaaacag ccctactacc ttcagaccca agaccccata aaactgtttg 480

agccgtcatt gcaacctcc 499

<210> 342
<211> 183

<212>  DNA
<213>  Homo sapiens


<220>
<221>  misc_feature
<222>  (75)..(75)
<223>  n is a, c, g, t or u

<400>  342
cacccgagac tgacacactg aactccactt cctcctctta aatttatttc tacttaatag      60

ccactcgtct ctttntttcc ccatctcatt gctccaagaa tttttttctt cttactcgcc     120

aaagtcaggg ttccctctgc ccgtcccgta ttaatatttc cacttttgga actactggcc     180

ttt                                                                   183


<210>  343
<211>  558
<212>  DNA
<213>  Homo sapiens


<220>
<221>  misc_feature
<222>  (72)..(72)
<223>  n is a, c, g, t or u

<220>
<221>  misc_feature
<222>  (239)..(242)
<223>  n is a, c, g, t or u

<220>
<221>  misc_feature
<222>  (400)..(400)
<223>  n is a, c, g, t or u

<220>
<221>  misc_feature
<222>  (405)..(409)
<223>  n is a, c, g, t or u

<220>
<221>  misc_feature
<222>  (411)..(416)
<223>  n is a, c, g, t or u

<400>  343
tgggccttcc cttaaacatc agaacaatga gatttgtccc tattttacag gggttagaat      60

agactattaa gngacaactg agaaaggaca gagaagtgac agccagaggt tgagaggggc     120

cataaaaaca tacaatcaga catatatctg ctaccacttt gtagcaagat ggttcctatc     180

ataactctgg gtcaaaaaga tagtaatttg gtttataatg ttgaaagaaa gcagaaagnn     240

nnagatgggg tctcactgtc gttctggagt gtagtggttc aatcatctct cactgcagcc     300

ttgaacccct aggctcaaag gatcctccca cctcagcctc ctgaatagct gggactagag     360

gcatgagcca ctatgtcttg ctgattaaaa attgtttttn caaannnnna nnnnnnactt     420

tactgcctaa gctggtcttg aaatcctggc ttcaagcaat cctttcactt tggcctccca     480

aaatgctggg attacaggca tgagtcaata tgcccagtct cttttctttc ttagttactc     540

tagaaaatgg cttgttga 558

<210> 344
<211> 526
<212> DNA
<213> Homo sapiens

<400> 344
aataatgttc tgtcacgtga aatatttaag tatatagtat atttatactc tagaacatgc 60

acatttatat atatatgtat atgtatatat atatagtaac tactttttat actccataca 120

taacttgata tagaaagctg tttatttatt cactgtaagt ttattttttc tacacagtaa 180

aaacttgtac tatgttaata acttgtccta tgtcaatttg tatatcatga aacacttctc 240

atcatattgt atgtaagtaa ttgcatttct gctcttccaa agctcctgcg tctgttttta 300

aagagcatgg aaaaatactg cctagaaaat gcaaaatgaa ataagagaga gtagtttttc 360

agctagtttg aaggaggacg gttaacttgt atattccacc attcacattt gatgtacatg 420

tgtagggaaa gttaaaagtg ttgattacat aatcaaagct acctgtggtg atgttgccac 480

ctgttaaaat gtacactgga tatgttgtta aacacgtgtc gataat 526

<210> 345
<211> 435
<212> DNA
<213> Homo sapiens

<220>
<221> misc_feature
<222> (334)..(334)
<223> n is a, c, g, t or u

<400> 345
ttgtgtacac ataatctcat tttgagatat ataactattt ttgtctttca gaagtgaatc 60

aaaatatttc aaaatgctgt cttatgaaac tacaatattc tcacagatta gaaaagtttt 120

tctgtaaaag tcagatagta aatattttag gttttgcagt gtcttttgca actactcaac 180

tttcctactg tagcacaaga gtagctgtgg tactgtgcaa ataaattgct tgtgttccaa 240

taaagcttca tttacaaaaa catgccatgg gccatatttg gcctgtacac tgttgtttgc 300

caagtcctaa tatagttgct tagcaagtat tgtnagctat ttgaggaaga catgaaagtt 360

cattgggttg ctaaaaagta tgtagaaatt caaaggaaaa ttaaaattta ggctaagtta 420

taatacactg tttta 435

<210> 346
<211> 343
<212> DNA
<213> Homo sapiens

<220>
<221> misc_feature
<222> (95)..(95)
<223> n is a, c, g, t or u

<220>
<221>  misc_feature
<222>  (227)..(227)
<223>  n is a, c, g, t or u

<400>  346
tctcatttac cttctctctt gagcaacgtc agtaattgat cttgcatctc agagagagag      60

aaagagcatg tgtgagagag aaactggttt ctatngccag cactcctgaa accccttact     120

gtaaggatat tttctcttac cccttgggat ccaggctctg agtctcttct ctttgggagt     180

atccatcaaa atgacttttt ttaaaaacag attttccccc aaccagnaga atctgcacaa     240

acttggcagc gtttttactt gtttaatgag tttaagacat tacatggtga aagagaagca     300

ttttggactc ctgcattttt atttaccatt cccagactga cga                       343


<210>  347
<211>  534
<212>  DNA
<213>  Homo sapiens


<220>
<221>  misc_feature
<222>  (34)..(34)
<223>  n is a, c, g, t or u

<220>
<221>  misc_feature
<222>  (74)..(74)
<223>  n is a, c, g, t or u

<400>  347
gcctaacaat caaatctctt tcttttaaag cacnaccttc taggcaggga caggagctca      60

ttttccacac catnctttgt caactctcat agaaagtttt ccttgtatcg agctcaaatc     120

tgcctcctgg aaattcttct tcttcttccc tccctgttgg taccagctct gctgtcagag     180

acttcacagt ctgtgctccc tctgccctgt gacgtcttca gactatttga gaacaggaat     240

catgactcct gggacttgcc ttttctctag gtcaaatacc tctataattc catctgctgt     300

tcttcatagg gtcttctccc tatcctgccc ttttcctcca atccatcttt taactgctct     360

tgagcagtct aactgagaag tatgattcaa agcaaaataa atcttaaggt ggcatgactc     420

tgaaaaaatt gagaaaattg aactcagaga tcccgatccc aacccctttc tcctgggagt     480

gaaaccttag tttctaccag agagtgtggg aaaccacttc tggtggaagc ccct           534


<210>  348
<211>  580
<212>  DNA
<213>  Homo sapiens


<220>
<221>  misc_feature
<222>  (109)..(109)
<223>  n is a, c, g, t or u

<220>

<221>  misc_feature
<222>  (241)..(241)
<223>  n is a, c, g, t or u

<400>  348
aacattccct tgtcaaccaa gaatactcaa agctacttgt attggaaatg gcagaaggcc      60

taaatccaaa tttcttattt tttataattt accatagaag ttttgtgant aaattcttac     120

ttctgccagt ggaggtttat gcctgaaagg tcatggggtc ctgtctgtaa atagacctaa     180

agagaagtgc agtatttatt ctttgtaggc ataatgtgtt tgtcactgac aagcattcat     240

nttcatccca ctagtctttt attgcagtct tttattgtca ttttcagcct tatgttggag     300

agctttgctt tctcatcatg ttcacattgt cttaagtttt gtgagcttct gagaaagagc     360

ttggtaaagg tttaaagggg actttgttcc accagggagc attttatttg ggcgtctcac     420

ccttttctaa tgaaagctgt tgtaagccac ctctgacttg gaaattctga aagtatgaat     480

attttttata tcttaattgt aaaatgccag ttctccatta tttagatgaa tagtagaaca     540

ctgcaccctt tgtgcagtgt ttttgtttct ctactgcatt                           580

<210>  349
<211>  541
<212>  DNA
<213>  Homo sapiens

<400>  349
ccagtcttcc tggcaagggt aaacagatcc cctctcctca tccttcctct ttcctgtcaa      60

gtgcctcctt tggtgaaggt gacacatcat gtgacctctt cagtgaccac tctacggtgt     120

cgggccttga actactaccc ccagaacatc accatgaagt ggctgaagga taagcagcca     180

atggatgccg aggagttcga acctaaagac gtattgccca atggggatgg gacctaccag     240

ggctggataa ccttggctgt accccctggg gaagagcaga gatatacgtg ccaggtggag     300

cacccaggcc tggatcagcc cctcattgtg atctggggta tgtgactgat gagagccagg     360

agctgagaaa atctattggg ggttgagagg agtgcctgag gagagccctc accgtctggc     420

accctagtca ttggagtcat cagtggaatt gctgtttttg tcgtcatctt gttcattgga     480

attttgttca taatattaag gaagaggcag ggttcaagag gagccatggg gcactacgtc     540

t                                                                     541

<210>  350
<211>  415
<212>  DNA
<213>  Homo sapiens

<400>  350
gaataaatct ctgggaccgg gtctcaccat attgctctgg ctggtttcaa actcctgggc      60

tcaagcgatc ctcctgcctc agccttccaa aaccaggtgt ttaacttggg actaacatga     120

agcacttaga agactacgtg gaacatagca atgactatat atgtactaca acgtaaacag     180

cacctcctgg attgaataga acataactga catgaccagc agagacaggc taaagacact     240

gagctgaaaa ccctggactc tattgctaaa ttgaggctcc tgaatccgtt cgctctgagc     300

aactgttgct gtggtgctgc cttcacaagc actctgctga gcactcagat agaggggctg 360

tgctatccgt caacagacaa gctgcagcca gaactgctca gctgacaaac tggta 415

<210> 351
<211> 438
<212> DNA
<213> Homo sapiens

<400> 351
gtggggaagc ctgaacacag tcctataaac taaaggccac tgcagacttt tagcacaagg 60

agatccttac agggaacatg tgccatcagc tctttggagt gaacaaggaa ttagaccccc 120

atcatgccaa aaaactagga tttttaggtg gtctttccat cccttcagat ttaagtattc 180

aaagaaagag agacagacct acattccaag ggtcttctga gtgcaaggcc ttgtgttgtt 240

tgtttattta ggggagggcc tggtgctctt ctctgtttta tgctttacct tcttttattt 300

ctcagatctc atgttagcac tatgttctga attccctaat aatggctctt gagaactgat 360

ttacattttg ttggtttgtt tacttcttga gcacataaaa ggaccccaaa ttagagatac 420

tatcccttgg gcttctga 438

<210> 352
<211> 224
<212> DNA
<213> Homo sapiens

<400> 352
   gtccactgct ggaaatagaa gttttttcgc tgcagggcaa ttctgtaaat gtgcttccca 60
   gctttaggag gtctgaggct actcttctcc aataaccttc cttcccactg gaccttctca 120
   ctcacagcac tgctgccctc tggacaagcc acagtggaca aatatgtcaa gctgaagatg 180
   cacaaataat ttcaagttca gttctcaggg attcaaagga catg 224

<210> 353
<211> 415
<212> DNA
<213> Homo sapiens

<220>
<221> misc_feature
<222> (177)..(177)
<223> n is a, c, g, t or u

<400> 353
tgtcctgggg atcttggagc ctgaattcat tggcacaaaa ggcagcagca tcctcactgt 60

atctgcagtc catttggact caataaaaac tttgaaagtc acatgtgtta tggaattcct 120

tctcagtgac acattcatct gtgctcagtt gtcccagcaa gggtcagccc ctcatanccc 180

tgcagcatcc gctgctatga agcagagctg taaacgccct ccctgtgtat aggaaaagct 240

acatggagca aatcctcctg cctgaagaag tgcatctcag catcacttca gctgtcgggg 300

catttgtggg gagaaccaga ccacctctgc ggaaggcagc agaccctctt ccagccatgg 360

atggagttga attctctata aacggttcac cagcaaacca ccaatacatt ccatt 415

<210> 354
<211> 186
<212> DNA
<213> Homo sapiens

<400> 354
gccaggttaa tggtatcgat cctaatgggg attcggcaga gtttgatttg ttgtttgaaa 60

atgcttttga ccagtgggta gccagcacag cgtcagaaaa atgcaccttc ttccagatcc 120

tccaccatac ctgccagagg tacctcacgg acaggaagcc agagtttatt aactgccaat 180

ccaaaa 186

<210> 355
<211> 457
<212> DNA
<213> Homo sapiens

<400> 355
ctttacccta ggtcaggggt cagcaaacta ctgcctgtgg gccaaatttg cccaccacct 60

gtatctgtaa ataaggtttc attggaacac agctgtggcc atatgtttgt atattgtgtg 120

tggctgcttt tgcattagga tgacagaggt gaatagttgc aacagagact ggctggtctg 180

caaagcctaa aatatgtcct gtgtggccct ttacagaaaa agttttctaa cccctgctct 240

aggttacgga gaaaaaaaaa tggaataatg ttctctgcta cttttaacct gattttcttt 300

gtacctaaat aggcagctag aatgctgcct atattttaat aaggatttgg atctcacaag 360

acaccttagg cctacacaag ttgttcagat tctttgcccc agttctaatc tagtgacaaa 420

ggcatagaat tctcctccca caggaatgta tttctat 457

<210> 356
<211> 373
<212> DNA
<213> Homo sapiens

<400> 356
cagtctcctg ctcgtttaga agtaagggat aataatgtat ccatagctaa atgcccagtc 60

gttatatttt ctagatcaag atgcttgttg tgtacagttt cacagagcct tcggattttt 120

tctttaattt tgttcatgtc tttttcattc agtagcttgg ctgatgaagc atcttgttcc 180

agttccaaaa gtcgaatcat tagatccaag ctagctctat caagatccat gttcaaacga 240

tctctactca gtatatacat gagggcagct gtacagaggg acagattctg atggtgctgg 300

gaatcatcca aggttttaaa gaccattgct accatcccat gtgctctcag gtgcattcgc 360

cagtaggcca aca 373

<210> 357
<211> 116
<212> DNA
<213> Homo sapiens

<400> 357

tttgctccta acttgctctt ggacaggaac cagggaaaat gtgtagaggg catggtggag     60

aggctagaga tcctgatgat tggtctcgtc tggcgctcca tggatgcagg gagagg         116


<210>  358
<211>  522
<212>  DNA
<213>  Homo sapiens


<220>
<221>  misc_feature
<222>  (297)..(297)
<223>  n is a, c, g, t or u

<220>
<221>  misc_feature
<222>  (418)..(418)
<223>  n is a, c, g, t or u

<220>
<221>  misc_feature
<222>  (483)..(483)
<223>  n is a, c, g, t or u

<220>
<221>  misc_feature
<222>  (486)..(486)
<223>  n is a, c, g, t or u

<220>
<221>  misc_feature
<222>  (490)..(490)
<223>  n is a, c, g, t or u

<400>  358
gggcatctgg aattgacaca ccattacatt ctgtttgcag gatttttttt gtaaccatga     60

aattgaacat ttccaaatta taaactatgt taatacctat aaaatatata gccaggaacc    120

atttatcatc aagaaaagtg taagaaatta tttttgagat gtaatttaag attgttttat    180

gtaaaaggaa aatcttgtat ggcatcgaat agccttaatg aatttaattc tttcacaaaa    240

atgatttcaa attatcctag agtataacat ttttatcaaa gatattattt ccggagntct    300

tctttctttc tttttttttt ttttttagta atttagcaaa aacattactg ttctaatgct    360

gaagtgactt ttgccagtgc catgtccagg gggggaggta taagttactt gctcttanca    420

tttgggctgg attttttggt ttgggggaca cctttgggag tattcccaaa gcatgtctca    480

agnggnggcn cccgagagca tggtttaaaa gcttggaccc ct                        522


<210>  359
<211>  369
<212>  DNA
<213>  Homo sapiens


<220>
<221>  misc_feature
<222>  (121)..(121)
<223>  n is a, c, g, t or u

<400>  359

gctgggccag tgcatctaac agccctgtgc agcagcttcc cttgcctcgt gtaacatgag 60

gcccattctt cactctgttt gaagaaaata gtcagtgttc ttagtagtgg gtttctattt 120

ngttggatga cttggagatt tatctctgtt tccttttaca attgttgaaa tgttcctttt 180

aatggatggt tgaattaact tcagcatcca agtttatgaa tcgtagttaa cgtatattgc 240

tgttaatata gtttaggagt aagagtcttg ttttttattc agattgggaa atccgttcta 300

ttttgtgaat ttgggacata ataacagcag tggagtaagt atttagaagt gtgaattcac 360

cgtgaaata 369

<210> 360
<211> 378
<212> DNA
<213> Homo sapiens

<400> 360
agatactcag cactagacta acataacagg tcactacacg ggtgcagaat cactttacaa 60

aagaagactc tgttttacga aggggattca ctacagggac ttagagaaca gtctcttttc 120

tgcctttaaa atgagagttc ctccatttac caaaatttga cacgcacaca ttcttcaggg 180

gcatgccaat tgcgtaaagt gaggctcgcc tgcatagcta atcctgttaa agacaacttc 240

tcaaagcaca acgtgcttgt ttcctatcgg gctccctgcg gggctttctc tcactacaag 300

tcaagcttgg gctctcaaag ccctgcgcct gttaccacgg atgcccacag ggcctgggca 360

gttgctgtgg cgacagga 378

<210> 361
<211> 291
<212> DNA
<213> Homo sapiens

<400> 361
acagtggatc aaatttaggc ttcttgatgc aggcatggtg tagattacta cttctgtatt 60

gtcccaggag ctcagcacat tccttgccag agatgataag gagctcaatc ttgaatactt 120

gttcaagctt ttgaataaaa aaccacagtt cctcaaagaa gaagaagaat tgcgaaatca 180

ccggaaataa ccgaaaactt ccccctgttt gactttcaac attcttgaat gcaccaagat 240

agcctctttc tgtgagatta ataaatgaat aaatgcctcc atatttttca a 291

<210> 362
<211> 313
<212> DNA
<213> Homo sapiens

<220>
<221> misc_feature
<222> (200)..(200)
<223> n is a, c, g, t or u

<400> 362
aagccggatg gcaaaagagc ccagaaccta ttggaactga caaaatcaag tcacggcgcc 60

tacaaagatg aggggcagat tctggctgcc ttttaatttc gtccttcacc tgatatctgt 120

gccagagaat gtcttccagg agttctgcta cagagaagag agtaaccccc atccatcatg 180

gccaaagcac ccagtcaggn tccgctctgg atccagcccg acaaatgcaa cccttgaata 240

gggtttgtgc aagcaaactg gatgacgacc gaagaaaccc tgtcgcttct gagaagacac 300

ccaatccaag aat 313

<210> 363
<211> 318
<212> DNA
<213> Homo sapiens

<400> 363
cctggaccca actttgttac tgtgagaaag ggtcttcatt cattcaagat ggcatttgtt 60

aagcacctac tgctggagtg cagtggttca atcacggatc actgcagcct ccacctccca 120

gttcaagaaa ttctcatgtc tcagcctcct gagcagctag gattacagac aaaccttgga 180

aatcaagaaa gttctggaat gatgaagctg ttcatgccaa gaccgaaagt gctggcccag 240

tatgagtcca ttcagttcat gccgtgacaa ttttcttgga actccttttt attgttagtt 300

ctcacttgtt tccatatt 318

<210> 364
<211> 531
<212> DNA
<213> Homo sapiens

<220>
<221> misc_feature
<222> (117)..(117)
<223> n is a, c, g, t or u

<220>
<221> misc_feature
<222> (119)..(119)
<223> n is a, c, g, t or u

<220>
<221> misc_feature
<222> (122)..(122)
<223> n is a, c, g, t or u

<220>
<221> misc_feature
<222> (153)..(153)
<223> n is a, c, g, t or u

<220>
<221> misc_feature
<222> (155)..(155)
<223> n is a, c, g, t or u

<220>
<221> misc_feature
<222> (241)..(241)
<223> n is a, c, g, t or u

<400> 364
ttagcatctt ggttactgga gaactataac ttttatgtag tcatgcttgg aaaacactaa 60

aagggaaatc gagtctgttt gacaatattc tgtcttcact gttgttcact tcataangng 120

tnggaatata aagttctata cagttaatat gangntctct ttagcattta aaacatgatt 180

tgcattttca tgaggcattt tggctaattt tattgatttc cttatatttc atagtcctta 240

nccttatgag aatcttatgt ttctgtgtgt tttctatcat gtagcacaat ttctgacaca 300

caaaacatac aataaacttg tgttaatttt tctatcaaag tcagaattta ttcataagga 360

atctgaagta aggtgtacta agcttgttta tgggttaagt gatatagcca aattcaaaac 420

tttacttttt atgtcagtct agaaatatct cagattaaaa catatcactt cttagttcca 480

attagataag ggaaatcttt tataataatg ccaggattgc tataatctga t 531

<210> 365
<211> 525
<212> DNA
<213> Homo sapiens

<220>
<221> misc_feature
<222> (35)..(36)
<223> n is a, c, g, t or u

<220>
<221> misc_feature
<222> (39)..(39)
<223> n is a, c, g, t or u

<220>
<221> misc_feature
<222> (78)..(78)
<223> n is a, c, g, t or u

<220>
<221> misc_feature
<222> (86)..(86)
<223> n is a, c, g, t or u

<220>
<221> misc_feature
<222> (92)..(92)
<223> n is a, c, g, t or u

<220>
<221> misc_feature
<222> (234)..(234)
<223> n is a, c, g, t or u

<400> 365
aggccatagt aatcatcctg ctgatattgc aagtnngtng ctagaatgag gttatataat 60

atatacaaaa acattttntc aactgntaaa gntgccttag taatataggg taataccagc 120

aacattatgg atatataatt atagtctatt gggccacact taagtttgga gtctaataaa 180

gtcacaatca aattctgcaa tttcaattga agataacctt gtctttatat tatnaattag 240

aagctaaagt tgatttttct aagagttctt tatttaaatg aagtactctg ggactgacct 300

tttcggaaat ggaatcttca ttggtcaggt gattcaacat ttttatacaa tttatccatc 360

ctcatctctt caggatttgc ataccttgcc agtttctact ggccattgtt gaaaatacat 420

ttatttggag aagtccaaag ccaaggggct catggggctg tgaggtcctt cttgctgcat 480

cgtcctgtgg tagaaggtgg aggagtcaag agagtgcccc agagt 525

<210> 366
<211> 267
<212> DNA
<213> Homo sapiens

<400> 366
gggccaatga aagcagggtc aaggacagga ccagcgcagg ccaaggaagg gaatatctga 60

cagcgcccac ccagccaaac cctcagccca aggacaggaa tgaggagatg ctggtgaact 120

agccatccat cagtacctgc cttcccccga ggctgcagcc ccactcccag gcgcctggcc 180

aggggagttt tctaggttct gagagccacg ttgtcatccc tgggctttga agttaaacat 240

cacacagctg tctataaaca agatttt 267

<210> 367
<211> 199
<212> DNA
<213> Homo sapiens

<220>
<221> misc_feature
<222> (67)..(67)
<223> n is a, c, g, t or u

<220>
<221> misc_feature
<222> (107)..(107)
<223> n is a, c, g, t or u

<220>
<221> misc_feature
<222> (120)..(120)
<223> n is a, c, g, t or u

<400> 367
gattcaggga ttggatgagt ctctatggtt tgttttgccc tgaagagcag aaggcttctg 60

tcccaantgg tgttgccaaa gcaacatatt aattccatgc catgatnctg ggtcaagatn 120

tgcacaatct gattgggcat gtcacctcgg atggcaaggg agtggaagtg gtcaaaatca 180

tggagtccca gctttcgga 199

<210> 368
<211> 372
<212> DNA
<213> Homo sapiens

<400> 368
gccccatgtt gcataggtgg cctataacca gtcagacaca ggagacaaca tgaagcccca 60

tctgtgcttc cctttctgac attaccacat ttgcctgatg gagtggccag ctccctttca 120

ctgctggaat gaatacaatc cagaaaacct accttctatt gctttaccta atggggtaag 180

gaaatttaag tagaaattgc taaccgaaga ctttgctaag caaacccagg tctgcttgat 240

gtcagagccc ttgctgttaa ccccatttac tgcttagcct ccaaagagaa gcaatagcat 300

cacatgggga aatgtcaaca gcataagagg actttcataa tcagaattta aactggctat     360

tatccctctg ga                                                         372


<210>  369
<211>  296
<212>  DNA
<213>  Homo sapiens

<400>  369
gaccgtgact cctgaagctt ttcagcgcag gtgtagccgg cttggcgtcg ccgcagtgag      60

gtttggagcc gctttggatt gctgagtcac tttcttcagc cacttaggga aaccgaaagt     120

ggaaactcgt ggggcttgaa atagtgtgtt ctcttgagaa ccaccgaggc agtgagattt     180

gggattccgg ggtctggaga tcgtgctttt tgtggactgc gtttgcagtt cctagggtgc     240

tgctgattca caggccttct ctgtctttaa gtgtgcagat cattgaccgc tcagtt         296


<210>  370
<211>  228
<212>  DNA
<213>  Homo sapiens

<400>  370
aaacccagag ccttctggat gtgtgaggta gtaggcttca accctcattc atgcataggt      60

cacacttctc caaagttggt atggcctgtc tccttggcat gttcccttgc ttctgcttgt     120

ccagttaatc ctttctgaca taccatgcat ctcagggtga agcggttgac atcagtaaac     180

tgtctccttc ttctagcttc atctgctaat tccagtgctt gtacaaga                  228


<210>  371
<211>  206
<212>  DNA
<213>  Homo sapiens

<400>  371
cctcctgatc accatagctt tatgcaacaa caagaaacaa atttattagc taacctaacc      60

actaatgacg caagagacaa ttctaaggac tttcaaaaca gcaaagtagg agcagctgct     120

acctctaggg atgagggatg caattgtcca attattggtg aaattgtcat ttcatgctat     180

tggctatttg aaattcctcc tctaat                                          206


<210>  372
<211>  463
<212>  DNA
<213>  Homo sapiens


<220>
<221>  misc_feature
<222>  (94)..(94)
<223>  n is a, c, g, t or u

<220>
<221>  misc_feature
<222>  (121)..(121)
<223>  n is a, c, g, t or u

<220>
<221>  misc_feature
<222>  (382)..(382)
<223>  n is a, c, g, t or u

<400>  372
ccctgcctgt actaatgatc caaaaattag ccaggtgtgg tggtgcgtgt ctgtagtgcc      60

agctactcgg gaggctgagg caggagaatc tcangaaccc gggaggcgga ggttgcagtg     120

ngccgaggtt gcactactgc agtccagcct ggctctgtct tggtgttcag ccatgttccc     180

atgctcactc ccaaggtgac tctgggaagg tctcagcctt tttgtcttcc cagttaggat     240

ggtcccatgc ccctgttacc atcagacttg gtaagtttcc cgaggagact ctgcaagagg     300

cactgttctg gatggtggag gagagactag ttgttctgct ctcctggcca cagtgggtgc     360

agtggacccc atcatggaga anttcaacac atccagccta cgaccagcac ctgtgggagg     420

tggatattca aggcagcaga gcctacagcc ggggcatgga gaa                       463


<210>  373
<211>  451
<212>  DNA
<213>  Homo sapiens


<220>
<221>  misc_feature
<222>  (38)..(38)
<223>  n is a, c, g, t or u

<220>
<221>  misc_feature
<222>  (87)..(88)
<223>  n is a, c, g, t or u

<220>
<221>  misc_feature
<222>  (231)..(231)
<223>  n is a, c, g, t or u

<220>
<221>  misc_feature
<222>  (406)..(408)
<223>  n is a, c, g, t or u

<220>
<221>  misc_feature
<222>  (421)..(421)
<223>  n is a, c, g, t or u

<400>  373
agggtctcaa atgaactctg agttaccatc tttggacnga cttttaatat aaagctgtaa      60

tccttaaatc tgtgtcagta gtcccannta ctatgtcact ttaattggat gaatgcgtta     120

atgaaaagtt tgttttcaaa cctcactaaa ctgctactta agatcacagt taatgtgagt     180

cctgcttaat ttggaaagca tttaaaaaat ggaaaagttt cttagggaag naaaaatttt     240

gcaactctgc ctacaaggta cagtaattgg ctaggttctt ttgaagagca gtgttgacta     300

gagttaagga aaagtcagtt gtgaaaaatg gacattttta atagcaaaat gatgtgcttt     360

actgtagaaa caggaggaag ggtgcattat cctggggaaa atgaannntt cttcagttat 420

nttttatgct gctctacttt attgcaaaac g 451

<210> 374
<211> 46
<212> DNA
<213> Homo sapiens

<400> 374
cagtcaccga ccttccctga gattgctacc tggaagctct ttctat 46

<210> 375
<211> 519
<212> DNA
<213> Homo sapiens

<400> 375
gaataagtac acagagtccc caaagactag tgaggccaag atgtgtgagt cattttccat 60

cacacacaaa aaacccaatt gttctaagta tgtattttac caagcagctt tatagaaaga 120

aaaacaaaca aacaaaccaa acaacaacaa caacaaaaaa ccttggccag gcacagtggc 180

ttacacctgt aatcccagca ttttgggaga ttcaggcggg tggatccttt gagcttggga 240

gtttgagatc agcctgggta atgtggcgaa acctcatctc taccaaaaat ataaaaacta 300

gccaggtgtg gtggtgcacg cctgtagtcc cagctgctta ggaaactgag gtgggaagat 360

tgcctgagcc caagaggtag aggtttcagt gagccgtggg aagattgcct gagcccaaga 420

ggtagaggtt tcagtgagcc gtgggaagat tgcctgagcc caagaggtag aggtttcagt 480

gagccaagat tgtatcactg cacaactgtt gcctgggca 519

<210> 376
<211> 222
<212> DNA
<213> Homo sapiens

<400> 376
cctgctggac agccgcgcag gatgagccgg agaccccgag ggccgtggcc ttccaggact 60

gccccgtgga cctgttcttt gtgctggaca cctctgagag cgtggccctg aggctgaagc 120

cctacggggc cctcgtggac aaagtcaagt ccttcaccaa gcgcttcatc gacaacctga 180

gggacaggta ctaccgctgt gaccgaaacc tggtgtggaa cg 222

<210> 377
<211> 460
<212> DNA
<213> Homo sapiens

<400> 377
atagtagggg caattttgtc tgtagatggc agtatgacaa ttcttgctag agaatatatt 60

gaaaaaaact tcaacacaaa gggttgtagc actgtcctca gtaccattgt gtgcatgagg 120

atcagaatag tctgggctag atacatcaca ttaaagcttt tcagaatctg ataaatagct 180

ctaaatacta atgatattga gaagcctagc ttcacttggg aaaatctgtg gctgttcaca 240

gaaattcagc accaagttat tccccccata ctctaccagg ccttcaggtc ctcataaaga 300

aaagtgtcgt tttcagatta ggaactcaaa attattttgg tgcatcaaat ctacagtcac 360

acaatataac aagaatggga ttagaaaaat gaaagcctac tcattctcat ctttaagcca 420

gagaatgaaa tatatatgag gtctctggat agctatttaa 460

<210> 378
<211> 544
<212> DNA
<213> Homo sapiens

<400> 378
cgccgcatca agccgtggcg gagatcgacg cgctctacga cgtgtacctg gacgtgatcg 60

acaagtgggg caccgacgac atgctgttcc tgggcgactt caacgccgac tgcagctatg 120

tgcgggcgca ggactgggcc gccatccgtc tgaggagcag tgaggtcttc aagtggctca 180

tccctgacag cgccgacacc acggtgggca actcagactg cgcctacgac cgcattgtgg 240

cctgtggcgc ccgcctgcgc cggagcctga agccccagtc ggccaccgtg cacgacttcc 300

aggaggaatt cggcctggac cagactcagg ctcttgccat cagcgaccac tttccagtgg 360

aggtgaccct caagttccac cgatgactcg aggcctgact ggggcatgcc acctgcagac 420

cctggctctg aggaatggcc caacagtggc cccttcaggg tggcagccac ccttcagtga 480

ggccccaagg cagagtcggc tgggcgtgga ccaggggcat ggacacgtga tgtgctgctc 540

tgta 544

<210> 379
<211> 254
<212> DNA
<213> Homo sapiens

<400> 379
gaagtttgtc ttcctacaac cacgtgatcc tctctctggg atttccccac tcaaccaggg 60

acaagaggtc aaagttgacc tgattatgtg tccatcaagg aagtgcccct ggaaggcaaa 120

taaagaaggc accatttaca ttacagtctc ctaagtgcag gcaatgatac cccaaggtgg 180

ggctctgcag accctccagc aaagagcttt tgaaaataaa tgtgaagctg ggcttaggag 240

ctcatgcctg caat 254

<210> 380
<211> 398
<212> DNA
<213> Homo sapiens

<220>
<221> misc_feature
<222> (140)..(140)
<223> n is a, c, g, t or u

<220>
<221> misc_feature
<222> (295)..(295)
<223> n is a, c, g, t or u

<400>  380
aacctgctaa ccaagaatgc tttacctggc aaagctgtcc ttcagaaatg agggagaaat      60

gaaagctttc tcagacaaac aaaaacaaag gaaacatgta aaagtgaaaa aataaatggt     120

ataagtaata tatagtcccn actcagaatt ctctaatact gttaaggtgg tgtgtgaagc     180

aatcttatta ctactaggag ggttaagaga caaaactatt aaaaacaact gcagctacag     240

tatattgtta aaggacacaa attttaagtt tacatcaaaa tcagaaaaca tgggnaagga     300

aggaatgaaa gtgcagagtt tttgtatgtg attaaaggca aattgttatc agtttaaagc     360

ctgttttaag gataaaatat tttatgtaag cctcatgg                             398

<210>  381
<211>  276
<212>  DNA
<213>  Homo sapiens

<400>  381
cccgccgcgc gagattaaag gacagaccaa gagggcgcgg gagctaccag cttggagggg      60

aggacagatg gggacccagg gctggccagg gctggtctct ggagctgttc tgccagagtg     120

atgggggcgc ttggcgaggc caaggatttg gttgggtcct atctctgaga cattttgaag     180

tctcacaccc cttccatttg ttgcctattc cacttaactt tgtatttgtt tgaaatctac     240

tgttcggatg ctggactaga agagggacac ttggcc                               276

<210>  382
<211>  119
<212>  DNA
<213>  Homo sapiens

<400>  382
aaacataaca gaggagttgc gaattttatg aaatttctga gtcttacaaa cttctcttta      60

agactatgag gaaatgctga cttgtattat ttatatcatt aaatttgctt gtgtatggt      119

<210>  383
<211>  490
<212>  DNA
<213>  Homo sapiens

<400>  383
gtccctgctg tttagtatgc tggagtggag gttctgtgac ttcctgttta gtggtgctga      60

ttctagttgg tgtgaaacgt cagatttcat cccagtcgcg tggctgattt ttttatgtgt     120

ggttctctgt gtttccagcc tggtcctgct ggtcaggatc ctctgtggat cccggaagat     180

gccgctgacc aggctgtacg tgaccatcct gctcacagtg ctggtcttcc tcctctgcgg     240

cctgcccttc ggcattctgg gggccctaat ttacaggatg cacctgaatt tggaagtctt     300

atattgtcat gtttatctgg tttgcatgtc cctgtcctct ctaaacagta gtgccaaccc     360

catcatttac ttcttcgtgg gctcctttag gcagcgtcaa aataggcaga acctgaagct     420

ggttctccag agggctctgc aggacaagcc tgaggtggat aaaggtgaag ggcagcttcc     480

tgaggaaagc                                                            490

<210>  384
<211>  458
<212>  DNA
<213>  Homo sapiens


<220>
<221>  misc_feature
<222>  (72)..(73)
<223>  n is a, c, g, t or u

<220>
<221>  misc_feature
<222>  (229)..(229)
<223>  n is a, c, g, t or u

<400>  384
gatacctcat tatacatctt acagagagca tcattggtgt ttccaaggtc acagggctag      60

gcaagggtgg anncctgagt ctgcttgtct gtttgcccca tgacagccca ggggtggtgg     120

cctcactcca cctccaggca cccacaagaa tataaaatct tgtacaagga tgtcgatatt     180

actattgcca ttcccaagtg cacctgcacc tgtagtatca ggtggtttnc agccttggct     240

gcatagctgc atatgagaat cacctgggaa gcttttaaag atcccagtat ccccacctct     300

tccccagtta cagtggagtc ttgcgggtgg tgggggacat cattattttt gaagcttcca     360

agtaattctg gtgtgcagtg gggtgaccag ctgtcccagg gacctccttt aaaaaataat     420

atcccgggca catgacaggc caattgccct aatgcaac                             458


<210>  385
<211>  510
<212>  DNA
<213>  Homo sapiens


<220>
<221>  misc_feature
<222>  (343)..(343)
<223>  n is a, c, g, t or u

<220>
<221>  misc_feature
<222>  (467)..(467)
<223>  n is a, c, g, t or u

<220>
<221>  misc_feature
<222>  (471)..(476)
<223>  n is a, c, g, t or u

<400>  385
cacctctgca cttttgtagg ctcaacaagt actggggagc ctgccaccac tgtatgcctt      60

tgaggcccct gccctgcctc cctggctggc cacggagctc gccctccctg gtagggggtg     120

agtttggaag tgagaggctg gtgtgggtct gtcccatgag ctgactcaca cttgcctcac     180

cacacatacc atcagaagac ccacgtggtg gagctaccgc tgctgctccc cacagtgcac     240

ctaggcaccc tcctgtcctt cccatggcac tcggttgacc tgggggttcc tgtccaacag     300

gtgaggcctg gtgtgcacag acactctgcc attgctagaa ggnggctgtg ccccctgcta 360

agatatcagt aggtccttca cagcctcacc ttgttcctcc catttgtttt taaaaattgt 420

ttcttatata tacagtttat ttagcttacg taaacatttg gtgcacntaa nnnnnntcaa 480

agatcatgat gtctcttttg tggttttata 510

<210> 386
<211> 92
<212> DNA
<213> Homo sapiens

<400> 386
cctctgccat tgcccaaaga aagtacgcag gagggaaggc gccgggggcg caggagtcgg 60

ggggaagtga aatctcggca ttagaacccc cg 92

<210> 387
<211> 394
<212> DNA
<213> Homo sapiens

<400> 387
aaggcgccgt caagtcaaat aaataaatgc cctacaacac caacccagga ctgagatctg 60

catgctggaa tgacggtggt ggtggtggct ttcagtattc cccaggtttt gtccggagca 120

ccggcacgcc ctctcttgaa gtccgctctc cgcacagtgg ttagacggga agatccggag 180

ctgtccagtg tcttgggtaa tgcacggcat cgcctgatgt ctgacgctag aacaccacgt 240

aaagtcaagc agagggaagt gaatgcgccc taggcccctg caggccacca agaagagcta 300

gagggagttg gtgcaatcct agagatgccg gcaggtgcac caatctgtgg cacacgtacg 360

ctctccaatg gaagacaact caagaccaca ccaa 394

<210> 388
<211> 289
<212> DNA
<213> Homo sapiens

<400> 388
actataatgc acttcgcaaa atgtaagggg ccggcttcac gccagcgggg ccttctggga 60

ctttgaattc aaccaggtga gcgctccagg tgccccgaca ggcgcactgt agccactggg 120

tgttaggggc gggagtctgg aaggtgacgg tagacggcca cttgggccct tctgggggcg 180

agcctactgg tggggtcagg gctctccgtg ctcagagcaa ggtagaggag caaggcccta 240

cttttggggg gcagggtcca gaccaaggac cctatgcgcg gagggtggc 289

<210> 389
<211> 139
<212> DNA
<213> Homo sapiens

<400> 389
aggcctgacc gaagagaact ttaaggaact aaagcaagac atttctagtt tccgctttga 60

agtcctggga ttactaagag gaagcaaact ttccacaata caatctgcga atgcctcgaa 120

ggagtcttca aattcggca                                                    139

<210>  390
<211>  528
<212>  DNA
<213>  Homo sapiens

<400>  390
caggttcttg aagttctcca ccatgacatc tcggtacagc ttcctctggg taagatcgag      60

cagtcgcagt tcctccctgg agaagaccac agccacatcc ttgaatgtca cagcctccta     120

caatatcaaa cacatgtaac ctcaatctta caaccaacct tcactagaag aagggtggca     180

tcaagaagga aaagagcacc acaaaaaagt tgttatagat tccaagagat ctcagtcaat     240

tttcagctgt tacagttttc cctgtctcac tatctcctac gctcatcccc ataaagcctg     300

tagtttatca ctgttttttg tttttttctt ttttgagatg gagtctcact ctgtcaccca     360

ctgcactcca gcctgggtga caggggtgag acactgtctt aaaataaata aatttttaga     420

attaaaataa atagatcata aagtgtttga aaggatcaga tgaatgaata tatgtcaagc     480

acttagaagt gcctagcaca ccatacatgc tcaataaact cgaacaac                  528

<210>  391
<211>  443
<212>  DNA
<213>  Homo sapiens

<400>  391
gccaggggtc gccaatcctg gaaccccact ggcttagagg gctgggggag agaaacatgc      60

tgccctcttt gtagcagtca ggcgctgacc caagagaact caccttattc ttcatttcgc     120

ctggtaatcc tccaggccct tctctacacc ctgaagggga gggaggaaaa tggatgaatg     180

agagagggag ggaacagtgc ccaagcgctt ggcctctcct tctcttcctt cactttgcag     240

aggctggaag acggcagccg ccggactggg cagatcctca agcagaccta cagcaagttt     300

gacacaaact cacacaacca tgacgcactg ctcaagaact acgggctgct ctactgcttc     360

aggaaggaca tggacaaggt cgagacattc ctgcgcatgg tgcagtgccg ctctgtagag     420

ggtagctgtg gcttctaggt gcc                                             443

<210>  392
<211>  463
<212>  DNA
<213>  Homo sapiens

<400>  392
tattggcacg tagcagtaca aggatggtga ggggtgggta gggggcagac agctaggcac      60

ttgaaaggaa agctcatctg gaaagattgg atcgtctcaa atgcacatac tcgtacactc     120

gattgaagcg tactctgtgc ctactagatc ttttcacagc caaaaacacc tggcaaccct     180

tggagaagta actattcctt tttttcacaa gtaagaaaat agagcctcag aaaatttaac     240

agttgtctaa gctagaaagt agcaggactg gactttgaag tagtctttag gttgtgctgt     300

acattttgtg gatatgctta aatcacagtt tagcttgtac acattttcct ttattagaat     360

tggaagtaag tattaatgtt tgaaaaaata ttttagcctg acaatattta ttctatcttc 420

atatgttttt gaaattagat attttaaact aggcacggtg gct 463

<210> 393
<211> 376
<212> DNA
<213> Homo sapiens

<220>
<221> misc_feature
<222> (26)..(26)
<223> n is a, c, g, t or u

<220>
<221> misc_feature
<222> (65)..(65)
<223> n is a, c, g, t or u

<220>
<221> misc_feature
<222> (99)..(99)
<223> n is a, c, g, t or u

<220>
<221> misc_feature
<222> (123)..(123)
<223> n is a, c, g, t or u

<400> 393
agctcatttt agtctcattt ctctcnctcc cttcttccct gatgaataaa gtttattggg 60

atggntttca gatgctcagc ttttccatat gattaggtna gtgatccaga acccttccaa 120

agnaccctgt ggactcaacc ctctgtttga acaacataca agataatatg agacatttat 180

ttatcgagga ccctctgagc acctggcact gtgccagatt ctttcagata tataaaattt 240

cacttgctcc tgttgattct ggaaaggagc aacggcatct tatgaagctg tagcagatac 300

tgtcctggcc tcgctcatgt gtgtcagatg tgttggagtg ccctggctgc tgctctgcat 360

gtgtagctga ggtcct 376

<210> 394
<211> 220
<212> DNA
<213> Homo sapiens

<400> 394
tggattcatg ccaaaggaaa ctgaaagcct gcctttcttt ttttcccagt gcacatctca 60

gattatttgg cctttgtccg aggactgaaa acagttctgt gtccaagtat gtttttaata 120

cctgatattt atttcacaaa aaaactgaaa ttgctttgtg tgtccaggct tgaatgttta 180

aggcatactt gattaataca tgtgtgctga gtgcttcctg 220

<210> 395
<211> 553
<212> DNA
<213> Homo sapiens

<400>  395
caaccgccac atagtcacat tgtcaaatag cgtattcacc ttctcttata agaaggctca      60

gcgagatctg gcgtataagc cactctacag ctgggaggaa gccaagcaga aaacggtgga     120

gtgggttggt tcccttgtgg accggcacaa ggagaccctg aagtccaaga ctcagtgatt     180

taaggatgac agagatgtgc atgtgggtat tgttaggaga tgtcatcaag ctccaccctc     240

ctggcctcat acagaaagtg acaagggcac aagctcaggt cctgctgcct ccctttcata     300

caatggccaa cttattgtat tcctcatgtc atcaaaacct gcgcagtcat tggcccaaca     360

agaaggtttc tgtcctaatc atataccaga ggaaagacca tgtggtttgc tgttaccaaa     420

tctcagtagc tgattctgaa caatttaggg actcttttaa cttgagggtc gttttgacta     480

ctagagctcc atttctactc ttaaatgaga aaggatttcc tttcttttta atcttccatt     540

ccttcacata gtt                                                        553


<210>  396
<211>  357
<212>  DNA
<213>  Homo sapiens


<220>
<221>  misc_feature
<222>  (90)..(90)
<223>  n is a, c, g, t or u

<220>
<221>  misc_feature
<222>  (103)..(103)
<223>  n is a, c, g, t or u

<220>
<221>  misc_feature
<222>  (111)..(111)
<223>  n is a, c, g, t or u

<220>
<221>  misc_feature
<222>  (114)..(114)
<223>  n is a, c, g, t or u

<220>
<221>  misc_feature
<222>  (124)..(124)
<223>  n is a, c, g, t or u

<220>
<221>  misc_feature
<222>  (132)..(132)
<223>  n is a, c, g, t or u

<400>  396
ctagaaactc actcagtcct gtggttgcca acctttctcc atctcccgca gacgttttac      60

tgcatgccag ataccatgtg cagtaacttn tgaatcctct cancccccta nctnccagaa     120

cacnggacta tnagttactt gaaagctgag gcttggtaga gggctggagc caattgcgtt     180

aaactaacta acattattgc aaaatatatt ctagggcttt tactctaata aaaatgactc     240

ctggaactgc agtactatat tcttggaacc ccaagaaacc aggtgacaac ccataaattt     300

accatcactt ttcagatgag gaaggcaaat ctggaaggcc aaattacttg tccaaag 357

<210> 397
<211> 423
<212> DNA
<213> Homo sapiens

<220>
<221> misc_feature
<222> (184)..(184)
<223> n is a, c, g, t or u

<220>
<221> misc_feature
<222> (195)..(195)
<223> n is a, c, g, t or u

<220>
<221> misc_feature
<222> (350)..(350)
<223> n is a, c, g, t or u

<400> 397
gttagcacat accattgaat tcactgagac acatgagaaa atatgggaaa gtcggagagt 60

ggaagtaaat gtaaagaccc ccctcctccc caaagagtac gttgtgtagt ggggtagagt 120

ggaaaatcaa tccaagaaaa gtagcaaacg gacccaaaga tgaagaggaa gaaaagaaac 180

agcnacacga aacgnaaaaa aaaagccacc agatttgttg caacgttgat gtaaacctgg 240

ccgtcttcct gaaccagtga cccagggttt ccgcttccct ttgctgtcat cttgctcaag 300

tctagaagct gaaatatcat catcaactcg acatgagggg ataacctctn gatccactca 360

tcagatgctc atcagacgtt ccaattacaa aactgaacct cttcttagtg ctggggcggt 420

tag 423

<210> 398
<211> 515
<212> DNA
<213> Homo sapiens

<220>
<221> misc_feature
<222> (132)..(132)
<223> n is a, c, g, t or u

<220>
<221> misc_feature
<222> (150)..(150)
<223> n is a, c, g, t or u

<400> 398
ggacaaaaac tttcccaagt cagcttttta ctatgattac gtcctagcct cagatgtggt 60

ctaccatcac tacttcctgg acaagctgct caccaccatg gtgtaccttt cccagccagg 120

gacggtgctg cnttgggcaa acaaattcan ggttcagcac cgactatgaa tttttagata 180

aattcaagca agtttttgac acaacactgt tggctgaata tccagagtca tcagtcaaac 240

tttttaaggg gatactaaaa tgggactaaa tccaacaaaa tgcctttcac aacgttactg 300

tgtcttttga gcaatgtgtt agaaattgct ttggtaatag acttctttca caggattgag 360

aaggtagtgc atagaaacaa cttgtatact tggaacaaat gtaacaatac tgcagaaact 420

ttctaatttc taagataatt taagattatc tggttaatct aaatatctaa aaagaacaac 480

ataaaaacat gaaagtagct ttgttggttc caacg 515

<210> 399
<211> 483
<212> DNA
<213> Homo sapiens

<220>
<221> misc_feature
<222> (55)..(60)
<223> n is a, c, g, t or u

<220>
<221> misc_feature
<222> (62)..(63)
<223> n is a, c, g, t or u

<220>
<221> misc_feature
<222> (65)..(65)
<223> n is a, c, g, t or u

<220>
<221> misc_feature
<222> (85)..(85)
<223> n is a, c, g, t or u

<220>
<221> misc_feature
<222> (88)..(88)
<223> n is a, c, g, t or u

<220>
<221> misc_feature
<222> (90)..(90)
<223> n is a, c, g, t or u

<220>
<221> misc_feature
<222> (93)..(93)
<223> n is a, c, g, t or u

<220>
<221> misc_feature
<222> (134)..(134)
<223> n is a, c, g, t or u

<220>
<221> misc_feature
<222> (278)..(278)
<223> n is a, c, g, t or u

<220>
<221> misc_feature
<222> (304)..(304)
<223> n is a, c, g, t or u

<400> 399

gactgccatc tgatcaacag ctacccttca gccaatttca cctttctgtt acttnnnnnn 60

anngngccct tgtgtggata atgtncancn cantaaggaa tgcatgttag gtactttaag 120

tcccagtaga atancagttc ataatatcac acatgtggtg aaatctaaaa aggcaatagg 180

gctaattttg gcaggagttg gaatagcccc ttgggatggc tttgcatact gtcagacagc 240

tttgagaaac tttcaaaccc ttgaaacact ggcaactnag tacaggcaga gccataaaag 300

gacntcaagc ctccctgcac tccctagcca atgctgtctt ggataacaga tttgccctgg 360

aatatcttct ggctgaacaa gggcgggtat gcacagtaat aaaccacatc tgttgttctt 420

acattaacag ttcaggattg gctaaactgc aagttcaaaa gatttaccaa gaccaggcac 480

aat 483

<210> 400
<211> 555
<212> DNA
<213> Homo sapiens

<220>
<221> misc_feature
<222> (483)..(483)
<223> n is a, c, g, t or u

<400> 400
ggagcaaaac acttggaacc cacaagactc ccagaaggtg aagttaagag ctcccagact 60

cataaggtta ttagaacagc aaactggcac cccaaagaac tttacggaga cttgcaacct 120

atcaacaagt tggatgaggg attaaaagcc ttcaacaacc aacaacccca agcatcaaac 180

tgaaggaaac attctaacct tcacagacag actggaggct ggatggggac ctggctgaag 240

acatctggag aatgaaagtt aagtaccagc ttgcattttt gtgcccctag attatttttg 300

cattttaaaa taagaagcat caaattgcgt gtctctgtgt aaaagttcta gcaatttgtt 360

ttaaggtgaa cttattttgg cttagggact acaaaaagag aaggtaattc ctagggaagg 420

aagaagagaa agaaatgaaa attagagaat aagattattt tgaatgactt caggtagcga 480

ggngtgtgtg tttgtgagtg tgtatttgag agacttggct catgcctgtg ggtcttctct 540

tctagtatca gtgag 555

<210> 401
<211> 327
<212> DNA
<213> Homo sapiens

<400> 401
ggctgagaaa ctactggagc accagggaca gtctgtaaag ttggatggac caccaatggg 60

aaaatgagag ctgcccaccc tggccttaca ctccttcaat taatacataa acagaaagga 120

ggatatacag agagccaaag gcccatggga cgtgaccaac attccactga gtctatacga 180

tcaaacagca aactgtttat catgaataca gaatgtgggc aaactcatga ctgtgcctgc 240

cccagaaggt ttgctgaggg caattgcttc ctgacgccaa gctccttgag gttatctatt 300

gggacatcca gagaatgcag tcttgca                                          327

<210>  402
<211>  497
<212>  DNA
<213>  Homo sapiens

<400>  402
gggtggcctg gggatagtgg cttcatcttt tggggcttca agattctttg tctttaaaat      60

caggggttat atcaagatca tcaaagttcc cattccatta aagaaaaccc tgcatgtatc     120

cataatgatg cttcttcctg ttaaatttac aatgaaggga aacccatcac ttaactgtag     180

gaatttccca aaatgaactg atgaccagtg atctctctat cagaaaatgg cagatttcta     240

gccttccaga actttgattt tcttggacat tcaatggttc ctttttccca aatatttttc     300

aactgatgcc aaaccttgga tttggtttaa tccacctttg gtttaggttt ggggaccctt     360

ttcctggacc gtcccagttt tgggttaaac cgatttggat gaccctgtga gtcgccactg     420

gataccgaca gtctgctgtg gtgcttagaa gccactgaaa cattggtgaa tgtgaagtca     480

cttttggggt gcctgcc                                                    497

<210>  403
<211>  512
<212>  DNA
<213>  Homo sapiens

<400>  403
gaaagctcca aatccatgac agtcgaagtc tctgctcctt caggaacagg acatcttcct      60

ggccttaatc cattatagca gccgtgatgt catttctgta tttcaggaag actggcagac     120

agttgctttc attcttcctc aaagtattta ccatcagcta cagtccaaaa ttgctttttg     180

ttcaaggaga tttatgaaaa gactctgaca aggactcttg aatacaagtt cctgataact     240

tcaagatcat accactggac taagaacttt caaaatttta atgaacaggc tgatacttca     300

tgaaattcaa gacaaagaaa aaaacccaat tttattggac taaatagtca aaacaatgtt     360

ttcataattt tctatttgaa aatgtgctga ttctttgaat gttttattct ccagatttat     420

gcactttttt tcttcagcaa ttggtaaagt atacttttgt aaacaaaaat tgaaacattt     480

gcttttgctc cctaagtgcc ccagaattgg ga                                   512

<210>  404
<211>  229
<212>  DNA
<213>  Homo sapiens

<400>  404
caccccattc aaactcaagc acagtatgcc tccccagtct ttatgcagcc tgtatataat      60

cctcaccaac agtactcggt ctatagtatt gtgcctcagt cttggtctcc aaatcctaca     120

ccttactttg aaacaccact ggctcccttt cccaatggta gttttgtgaa tggctttaat     180

tcgccaggat cttataaaac aaatgctgct gctatgaata tgggtcgac                 229

<210> 405
<211> 495
<212> DNA
<213> Homo sapiens

<400> 405
acagctcagg ttttatcacc gactgggaat agacaacctc aatgctgaac cgcactggag 60

aaaaggggca aggtacccct gctgaggtgt atgggctgcc atctcaggct gtcttgagga 120

cctgggctcc ctctgctact cccaggaaat gggctcctga cacagcagtc tgccaccaca 180

gccccaggag ggtgtcaaca ccagcaaatg ctgtatttgc agcatgtcca agatgaccct 240

tctcccctac ctctacctag ccactggcag ggaggggaga cagtggtgat agcagcagca 300

ctctaggcat ggtgaacgcc tgggaccaag ccatgtggcg tttttatttt tgcctttctg 360

gaagactcaa gatatgtctc ttcattctct ctcagtattt gtttactttg gtttttttgt 420

ttttaatctc agagagaggt gtgtttagtg ggcacaagct gtaatattca gcaaaacttt 480

gtcgactggc actgt 495

<210> 406
<211> 472
<212> DNA
<213> Homo sapiens

<220>
<221> misc_feature
<222> (77)..(77)
<223> n is a, c, g, t or u

<220>
<221> misc_feature
<222> (79)..(79)
<223> n is a, c, g, t or u

<400> 406
ttcctcttgc tgagaaaacc caccctgctc acctaaaccc tggccttgcc tggtaattcc 60

atccatgcgc ctggaangnc ccagacatca aggctctgag gggccaggca cggggagaac 120

ccagcagtgc cctgccctgc agtctgagct accagattcc ttgtgaagat aatttgagga 180

ccatgactca cccaaccaca tttcctgggg cctcaaattg aaaattcagg atgggctttt 240

ctatatgact ggctgatatc caactatgcc atggtcttta catgccatga acattctttc 300

ctgccagagt tctaagaatc tgtgttctct gccttagacc ttctgcagat gagcccacag 360

gaagctccac gtgtagctga gctacatgca ccaggcctca gtttgcccca agtcccctgt 420

gtactctctc atggcctgtg gccaagaaat gtattctctc actttggact ta 472

<210> 407
<211> 395
<212> DNA
<213> Homo sapiens

<400> 407
agcagatgga ccctactgga agtcagttgg attcagattt ctctcagcaa gatactcctt 60

gcctgataat tgaagattct cagcctgaaa gccaggttct agaggatgat tctggttctc 120

acttcagtat gctatctcga caccttccta atctccagac gcacaaagaa aatcctgtgt 180

tggatgttgt gtccaatcct gaacaaacag ctggagaaga acgaggagac ggtaatagtg 240

ggttcaatga acatttgaaa gaaaacaagg ttgcagaccc tgtggattct tctaacttgg 300

acacatgtgg ttccatcagt caggtcattg agcagttacc tcagccaaac aggacaagca 360

gtgttctggg aatgtcagtg gaatctgctc ctgct 395

<210> 408
<211> 397
<212> DNA
<213> Homo sapiens

<400> 408
attttcctca taaagcattg ctccagctaa tcttatctat ttttctccag aatctccatc 60

cccttcccgt cagatacatc taaaactttt tttgtatctt tgtttttcct cgtgttgtat 120

catcttccta aaacatgttc tacttgtgaa aaccctaaga aattctctct gtcttattga 180

aattctatct ccactgtgaa gcattatcat ggtgtggcca tatatgatct atccctatct 240

gaagtcactg catttattcc ctgatcctca tttgcaggtc cagtaccttg tacaagtttc 300

tttttgtgcc atattagact gtaagctcca agagggcagg gcccaagtct tatgaatttg 360

tgtctgcata gtgtctagta cttgtctgag gcccaca 397

<210> 409
<211> 48
<212> DNA
<213> Homo sapiens

<400> 409
aggacgtacc ttgtgagatg cgagccggcc aacagcttgc aagcatgc 48

<210> 410
<211> 459
<212> DNA
<213> Homo sapiens

<400> 410
gcaagtcgcg tgatttctac cacacctgct actgcctgag cggcctgtcc atagcccagc 60

acttcggcag cggagccatg ttgcatgatg tggtcctggg tgtgcccgaa aacgctctgc 120

agcccactca cccagtgtac aacattggac cagacaaggt gatccaggcc actacatact 180

ttctacagaa gccagtccca ggttttgagg agcttaagga tgagacatcg gcagagcctg 240

caaccgacta gaggacctgg gtcccggcag ctctttgctc acccatctcc ccagtcagac 300

aaggtttata cgtttcaata catactgcat tctgtgctac acaagcctta gcctcagtgg 360

agctgtggtt ctcttggtac tttcttgtca aacaaaacca atggctctgg gtttggagaa 420

cacagtggct ggttttaaaa ttctttccac acctgtcaa 459

<210> 411
<211> 275
<212> DNA

<213>  Homo sapiens

<400>  411
agagggcaag gggctggatg caggcagaga atgactttaa gaaaagattc tatgatccct      60

tcctttagta tggagctcga ttttccagct ggcgcttggt gagaaagtac ttgaagaact     120

catagacaga ccaagaaatg gcggtggagg gcatctggta gatgacacgc gcctggatgc     180

ctttgaagta gccggccagg ccgttgagct ggtacaccgt ccggaaggca ttggccatac     240

ccgacagccg gccgctgatg ttggccagcg agagg                                275


<210>  412
<211>  536
<212>  DNA
<213>  Homo sapiens

<400>  412
gcagataagc tccgtctgca gttccaggcc agccagaaac tcctgtgtcc acatagagct      60

gacgtgagaa atatctttca gcccaggaga gaggggtcct gatcttaacc ctttcctggg     120

tctcagacaa ctcagaaggt tggggggata ccagagaggt ggtggaatag gaccgccccc     180

tccttacttg tgggatcaaa tgctgtaatg gtggaggtgt gggcagagga gggaggcaag     240

tgtcctttga aagttgtgag agctcagagt ttctggggtc ctcattagga gcccccatcc     300

ctgtgttccc caagaattca gagaacagca ctggggctgg aatgatcttt aatgggccca     360

aggccaacag gcatatgcct cactactgcc tggagaaggg agagattcag gtcctccagc     420

agcctccctc acccagtatg ttttacagat tacgggggga ccgggtgagc cagtgacccc     480

ctgcagcccc cagcttcagg cctcagtgtc tgccagtcaa gcttcacagg cattgt         536


<210>  413
<211>  286
<212>  DNA
<213>  Homo sapiens


<220>
<221>  misc_feature
<222>  (63)..(63)
<223>  n is a, c, g, t or u

<220>
<221>  misc_feature
<222>  (121)..(121)
<223>  n is a, c, g, t or u

<400>  413
ttaatttctg tgaagagtgc ccctggtgtt tcatcttggc ctgttttgat gagaatgtta      60

tcntttgtgt ctggataacg cgtcagcttc ttaaagtaca tataaagata ttctgtcacc     120

nccccacatg cacacacttt taaaatctat ttttattctc ttgctaaagt tgtaattatg     180

tcaagaattt tccagctcta actgccttct tagtacatgt ctttctgcct ttgaagcata     240

tgagtttgcc aaagtcattc tcccctaatg acatattgtg gactta                    286


<210>  414

<211>  166
<212>  DNA
<213>  Homo sapiens


<220>
<221>  misc_feature
<222>  (27)..(27)
<223>  n is a, c, g, t or u

<220>
<221>  misc_feature
<222>  (74)..(74)
<223>  n is a, c, g, t or u

<400>  414
gaaagacgga ggaaacaatc aaaatcncca ttctattgct ttgacacctt tactaggtga      60

attggtggca ttcncaaagc taatagggac gtttatatca agaaacattt ctgtatatat     120

tgttgaattt tagttgtaca tatactttgt atgtttttgt cttctt                    166


<210>  415
<211>  552
<212>  DNA
<213>  Homo sapiens

<400>  415
tgcaggctag gggaggagcc acccccgctt ccctattgtg accaggccta tggggaggag      60

ctgtccatac gccaccgtga gacctgggcc tggctctcaa ggacagacac cgcctggcct     120

ggtgctccag gggtgaagca ggccagaatc ctgggggagc tgctcctggt ttgagctgca     180

ttcaggaagt gcgggacatg gtaggggagg caaaaagcct tgggcactac cctccctgtg     240

gagctgttcg gtgtccgtcg agctagccac accctgacac catgttcaag ggtaccggaa     300

gagaagggtg tctgccccca acctcccctg tgggtgtcac tggccagatg tcatgaggga     360

agcaggcctt gtgagtggac actgaccatg agtccctggg gggagtgatc ccccaggcat     420

cgtgtgccat gttgcacttc tgcccaggca gcagggtggg tgggtaccat gggtgcccac     480

ccctccacca catggggccc caaagcactg caggccaagc agggcaaccc cacacccttg     540

acataaaagc at                                                         552


<210>  416
<211>  524
<212>  DNA
<213>  Homo sapiens

<400>  416
acgccgcgcg aaggtgatga gctcgcccgg ctgccctacc tacggacctg gttccgcacc      60

cgcagcgcca tcatcctgca cctcagcaac ggcagcgtgc agatcaactt cttccaggat     120

cacaccaagc tcatcttgtg cccactgatg gcagccgtga cctacatcga cgagaagcgg     180

gacttccgca cataccgcct gagtctcctg gaggagtacg gctgctgcaa ggagctggcc     240

agccggctcc gctacgcccg cactatggtg gacaagctgc tgagctcacg ctcggccagc     300

aaccgtctca aggcctccta atagctgccc tcccctccgg actggtgccc tcctcactcc     360

cacctgcatc tggggcccat actggttggc tcccgcggtg ccatgtctgc agtgtgcccc 420

ccagccccgg tggctgggca gagctgcatc atccttgcag gtgggggttg ctgtataagt 480

tatttttgta catgttcggg tgtgggttct acagacttgt cccc 524

<210> 417
<211> 378
<212> DNA
<213> Homo sapiens

<400> 417
aaatgactgc attcgtctct tttttaaagg tagagattaa actgtataga cagcataggg 60

atgaaaggaa ccaagcgttt ctgtgggatt gagactggta cgtgtacgat gaacctgctg 120

ctttgttttc tgagaagagg tttgaagaca ttttattaac agcttaattt ttctctttta 180

ctccatagga acttatttta atagtaacat taacaacaag aatactaaga ctgtttggga 240

attttaaaaa gctactagtg agaaaccaaa tgataggttg tagagcctga tgactccaaa 300

caaagccatc acccgcattc ttcctccttc ttctggtgct acagctccaa gggcccttca 360

ccttcatgtc tgaaatgg 378

<210> 418
<211> 116
<212> DNA
<213> Homo sapiens

<400> 418
agtatggaag ctgagaagag ttattggaat cacccccacc gttgacagag gaaggcaggg 60

ggtgagaatt aactgcttga gggtaggaga gtctgagatg tgggggccct attccg 116

<210> 419
<211> 147
<212> DNA
<213> Homo sapiens

<400> 419
cctgagccac cacgcagaag aggcactttc caagttgttt accaagaatt tacattaaaa 60

taacaagcta ttgtttggct atacattgtt ctttgtatca catattccag gaactacagg 120

aaaataatgg gtgaggcagc tagttag 147

<210> 420
<211> 310
<212> DNA
<213> Homo sapiens

<400> 420
gaaattccat caatacatct agacagatgt ttgcttgtag tttttggtat ccaaaacctt 60

ttttccacac atcgcacaga tgcctttttt gtaggcacag ccctggcagt aatgagaacc 120

tggttggtgc acagaacttt tacaaattct acaagtggag aacttattct ttccatatgg 180

atcaaatctt gctttttttg aagtcaaagc tttattttca ttcagctttc ttccaccact 240

ttctgtggta ttcctagcac cacctttcca tgtatctgga gtgataacag taccaagttt 300

cttttcacat 310

<210> 421
<211> 154
<212> DNA
<213> Homo sapiens

<220>
<221> misc_feature
<222> (68)..(68)
<223> n is a, c, g, t or u

<400> 421
agatataact ggtagagcac gtcaaagata tacagaaata accagagaaa agtttgaggc 60

attaaaanaa gaaaatatgg acctaaacaa tatgaatcaa agccttaccc ttgaactaaa 120

cacaatgaaa caagcaatga aagaactaca gtta 154

<210> 422
<211> 444
<212> DNA
<213> Homo sapiens

<220>
<221> misc_feature
<222> (92)..(92)
<223> n is a, c, g, t or u

<220>
<221> misc_feature
<222> (264)..(264)
<223> n is a, c, g, t or u

<400> 422
tttttgtgca tgattacact ccactgacat cttccaagta ctgcatgtga ttgaataaga 60

aacaagaaag tgaccacacc aaagcctccc tnggctggtg tacagggatc aggtccacag 120

tggtgcagat tcaaccacca cccagggagt gcttgcagac tctgcataga tgttgctgca 180

tgcgtcccat gtgcctgtca gaatggcagt gtttaattct cttgaaagaa agttatttgc 240

tcactatccc cagcctcaag gagnccaagg aagagtcatt cacatggaag gtccgggact 300

ggtcagccac tctgactttt ctaccacatt aaattctcca ttacatctca ctattggtaa 360

tggcttaagt gtaaagagcc atgatgtgta tattaagcta tgtgccacat atttattttt 420

agactctcca cagcattcat gtca 444

<210> 423
<211> 510
<212> DNA
<213> Homo sapiens

<220>
<221> misc_feature
<222> (357)..(357)
<223> n is a, c, g, t or u

<220>

<221>  misc_feature
<222>  (454)..(454)
<223>  n is a, c, g, t or u

<220>
<221>  misc_feature
<222>  (462)..(462)
<223>  n is a, c, g, t or u

<400>  423
gctttggact ggctcgcatg gaaaaccagg catttgatcc cgagaaaggg aacttcaaca      60

ctttgttttg caggctctgc gtgctgctgc tggtgtgtgc cgcccaggcc tggctcatgt     120

ggcgcttcat ccactcccag ctgcggcact ggcgggaata ctggaatgag cagagtgcaa     180

agcggagagt cccagccaca cccagactac cagccaggct catcaagagg gaatctggtt     240

accatgaaaa tggagtggtg aaggcagaga acggaacctc cccacggact aagaaactca     300

agtctcccta aggccaaagt gctaagaaca ggaatcctct tggtgggggc cgagcanggg     360

gcaaggagcc caggccccct ccctgcctcc tccttcctgc ctgtgatgct ccgtctcaaa     420

cagccgaaac ctgtcttgca atggggggag gggngcgttt cnctttcctt cttcttggct     480

tcctcttatt cttccacaaa ccattctcaa                                      510


<210>  424
<211>  191
<212>  DNA
<213>  Homo sapiens

<400>  424
acattgtgcc tcaggatttt gataataatt ctggatattg gaacagaata gaaatgtact      60

gtcgagagct gacagaaagg tttgaagatg tttgggtggt atctgggcct ttgaccttac     120

ctcagactag aggcgatgga aagaaaatag ttagttacca ggtgattggc gaggacaacg     180

tggcagtccc c                                                          191


<210>  425
<211>  186
<212>  DNA
<213>  Homo sapiens

<400>  425
gcggtgtgga ccgaggaaca acttggaaga tctacctgca acacaacatt tgtgtcactg      60

tacagttttg tggactgagc gaggaaaaac aacaaataat ttaagttggc tagagcttct     120

gtattttcaa agactgccac gtgccttagg aatactgttt tatctccata ctttggatga     180

cttgtt                                                                186


<210>  426
<211>  465
<212>  DNA
<213>  Homo sapiens

<400>  426
gttttggacc aacaagtgcc tcctttggtg aaggtgacac atcatgtgac ctcttcagtg      60

accactctac ggtgtcgggc cttgaactac tacccccaga acatcaccat gaagtggctg     120

aaggataagc agccaatgga tgccaaggag ttcgaaccta aagacgtatt gcccaatggg 180

gatgggacct accagggctg gataaccttg gctgtacccc ctggggaaga gcagagatat 240

acgtgccagg tggagcaccc aggcctggat cagcccctca ttgtgatctg ggagccctca 300

ccgtctggca ccctagtcat tggagtcatc agtggaattg ctgtttttgt cgtcatcttg 360

ttcattggaa ttttgttcat aatattaagg aagaggcagg gttcaagagg agccatgggg 420

cactacgtct tagctgaacg tgagtgacac gcagcctgca gactc 465

<210> 427
<211> 480
<212> DNA
<213> Homo sapiens

<400> 427
tcctttgtgt agcattatca gcctcggtct ggcctctggc acctcaccct tgccatggct 60

gaccccaccc attccaaggc ggggtcacgg taccagcagc acttggggtg aggcctccaa 120

agcttcctca gaattgtggc tgtgccacgc tggaccacag ggtccccctc aagcatctcg 180

gggccctatt ctctctgagc acctggaggg ctggactcag gcttgtgcca gggcctgact 240

tgggcctggg ggccctagaa cactcctcct cctgagccta ctgccaaacg tcctcagtgt 300

tgtctgcacc tgctccgact ccttcagccg ccccattcag cgcccgctcc gtccagtgcc 360

cgccctgtgg ggccaaggcg gccgtgcctt actactctgt gtcttctgcc tcctctgagg 420

aatctggccc tgtctgacag tcccagaccc cccgttctct cctctttagt tgcatgagtt 480

<210> 428
<211> 533
<212> DNA
<213> Homo sapiens

<400> 428
ttcattcaca aacttccgct gtacctgcgt ctaaaaaggc ccaaacccga gagagacctg 60

atgccggagc cccctcactg ttcttctcca ggaagtggct ggggtcgggg aacagatgaa 120

tatttcatcc ggaagccgcc aagtgatttt ctcttcccca aacccaatag gttccagcct 180

gaactgtctg cccctgatct gcggcgattt atcgatggtc caaaccgggc tgtggccctg 240

cttccggagc tacgggaggt cgtctcctct atcagctaca tcgctcgaca gctgcaggaa 300

caggaggacc acgatgcgct gaaggaggac tggcagtttg tggccatggt agtggaccgc 360

ctcttcctgt ggactttcat catcttcacc agcgttggga ccctagtcat cttcctggac 420

gccacgtacc acttgccccc tccagacccc tttccttgaa gactggaggg ttgagaccag 480

gccccctgcc agttgaagtg agagagtttg gtgatactgt caagccctat cct 533

<210> 429
<211> 486
<212> DNA
<213> Homo sapiens

<400> 429

gtgacctttc acgaacatgg gcatggctgc ggctccctcg tcatcaggtg catagcaagt 60

gaaagcaagt gttcacaacg gtgaaacttg agcgtcattt ttcttagtgt gccaagagtt 120

cgatgttagt gtttccattg tattttctta cagtgtgcca ttctgttaga tactatcctt 180

ataattgatg agcaagacat actgaatgca tatttcggtt tgtgtatcca tgcacctacg 240

tcagaaaaca agtattgtca ggtattctct ccatagaaca gcactatcct catctctccc 300

cagatgtgac tactgagggc agttctgagt gtttaatttc agactttttc ctctgcattt 360

acacacacac acacacacac acgcacacac acacaccaag taccagtata agcatctccc 420

atctgctttt cccattgcca tgcgtcctgg tcaagccccc ctcactctgt ttcctggtca 480

gcatgt 486

<210> 430
<211> 97
<212> DNA
<213> Homo sapiens

<400> 430
tattagttaa ttagtgattt cacagtatcc tttcgcaggc cgatccccac tccaaccgtt 60

ccctcagcaa ccccaggggt gtcagacggg gcaccct 97

<210> 431
<211> 241
<212> DNA
<213> Homo sapiens

<220>
<221> misc_feature
<222> (88)..(88)
<223> n is a, c, g, t or u

<400> 431
gctgcctttg cactggcgaa gggaggggca ctggttatgt tgtttccatt cgacagtcct 60

tccaaaggct tccctccagc gccactancc aaatccagaa aagcgtcctc ctccagaagg 120

taccaccaaa cctttaaaac ctttaaaggc tcctccagtg tcagattcaa atccaacatt 180

tctgcgcttt gctttcttta tggctctatt cttcaagact tcctcactgg ccatggagaa 240

t 241

<210> 432
<211> 537
<212> DNA
<213> Homo sapiens

<400> 432
tgagcctgtg cgttttgcat actgggttgg tttgctgggg ctgcggtgac agcatatgcc 60

gcgagctggg ctttaacaga gatgtgtgct ctcacagctt tgcaggcggg ggtctgagat 120

cagggtgtcg cgggtggggg gtcactgctg aggccgtgag gggaatctgc tcaggcctgt 180

ccctggcttc tgggggctgc tggtggtatt ttcagttcct tggtgtgtgg atacttcgcc 240

ccatctctgc cttcacctgt gtcctccctg tgtgggtgct ggtgtccaaa atttcccctt 300

ttcgtagtga caccagctgt gttggattgg ggcccaccct gctccagcat ggcctaatct 360

taactaatta catttgcaag gatcttatgt ccacaaaagt cacagtctga ggtgctgggg 420

gttaggactt caatatataa attttgcggt tacacaattc aatccatgac agaatccaaa 480

ggtttactct ggttataaaa acagtacaat aaaatattgt ttatagcctt ccctgta 537

<210> 433
<211> 355
<212> DNA
<213> Homo sapiens

<220>
<221> misc_feature
<222> (56)..(56)
<223> n is a, c, g, t or u

<220>
<221> misc_feature
<222> (109)..(109)
<223> n is a, c, g, t or u

<220>
<221> misc_feature
<222> (186)..(186)
<223> n is a, c, g, t or u

<400> 433
gaaaacccgt tatgagacac aacttgaatt aaatgatgaa ctagaaaagc aaattnttta 60

tctcaaggag aaagtggaaa aaatccatgg aaactcttca gatagactnt cttctattcg 120

tgtctatgaa cgaatgccag tggaatcctt aaacacatta cttaaacagc tagaagaaga 180

aaagangact cttgaaagtc aagtgaaata ctatgcactt aaactggaac aagaatcaaa 240

ggcttaccag aagatcaaca atgaacgccg tacataccta gctgaaatgt ctcagggttc 300

tggtttacat caagtttcta aaaggcaaca ggtggatcaa ctgcctagga tgcaa 355

<210> 434
<211> 319
<212> DNA
<213> Homo sapiens

<400> 434
ggcaagaagc caggtaaggc atgcagtctt tctgttcccc gttgggggag tggtattaag 60

gaactgtgtc ttcaggatac agtgagctgt aaaaatagac aacaagaaca cggaaactat 120

ggtagacgaa tgggctgagg acacagttca tgaaagagaa atatactcaa gatagaagaa 180

cctgcttcat cttagtggtg atttttgtaa aatgtaattt aaaatattcc ccgatgctgg 240

gagctaagta aaaaataaat aagtaaataa aatacaaaat tacatgtaca tttaaatgtt 300

ttttctctat caagtttat 319

<210> 435
<211> 511
<212> DNA
<213> Homo sapiens

<400>  435
   cacgatgacc ccagacatga gatccatcac taataatagc tcagatcctt tcctcaatgg      60
   agggccatat cattcgaggg agcagagcac tgacagtggc ctggggttag ggtgctacag     120
   tgtccccaca actccggagg acttcctcag caatgtggat gagatggata caggagaaaa     180
   cgcaggacaa acacccatga acatcaatcc ccaacagacc cgtttccctg atttccttga     240
   ctgtcttcca ggaacaaacg ttgacttagg aactttggaa tctgaagacc tgatccccct     300
   cttcaatgat gtagagtctg ctctgaacaa aagtgagccc tttctaacct ggctgtaatc     360
   actaccattg taacttggat gtagccatga ccttacattt cctgggcctc ttggaaaaag     420
   tgatggagca gagcaagtct gcaggtgcac cacttcccgc ctccatgact cgtgctccct     480
   cctttttatg ttgccagttt aatcattgcc t                                     511


<210>  436
<211>  515
<212>  DNA
<213>  Homo sapiens


<220>
<221>  misc_feature
<222>  (89)..(89)
<223>  n is a, c, g, t or u

<220>
<221>  misc_feature
<222>  (91)..(92)
<223>  n is a, c, g, t or u

<220>
<221>  misc_feature
<222>  (96)..(98)
<223>  n is a, c, g, t or u

<220>
<221>  misc_feature
<222>  (100)..(100)
<223>  n is a, c, g, t or u

<400>  436
taagatccag ggttccagga ctgccaccaa ctcctgtcca gctgctctat ccagtgtccc      60

gattcagcaa tgtcaaatcc ctccagcanc nntgcnnntn ccggatacga cagctcgtca     120

ggatagatca catcccagat ctcccactgc ctaaacctct gatctcttat atccgaaagt     180

tctactacta tgatcctcag gaagaggtat acctgtctct aaaggaagcg cagctcattt     240

ccaaacagaa gcaagaggtg gaaccctcca cgtagcgagg ggctccctgc tggtcaccac     300

caagggcatt tggttgccaa gctccagctt tgaagaacca aattaagcta ccatgaaaag     360

aagaggaaaa gtgagggaac aggaaggttg ggattctctg tgcagagact ttggttcccc     420

acgcagccct ggggcttgga agaagcacat gaccgtactc tgcgtggggc tccacctcac     480

acccacccct gggcatctta ggactggagg ggctc                                515


<210>  437

<211>  489
<212>  DNA
<213>  Homo sapiens

<400>  437
gctttgagga aaccactgtg caacttgaga tgtctgtggt tgtggggatg ttccatccct      60

ccgttcagtt gtgaagacct ctgctctgcc ctcagcaacc agagcctcgt cactctggac     120

ctgggtcaga atcccttggg gtctagtgga gtgaagatgc tgtttgaaac cttgacatgt     180

tccagtggca ccctccggac actcaggttg aaaatcgatg actttaatga tgaactcaat     240

aagctgctgg aagaaataga agaaaaaaac ccacaactga ttattgatac tgagaaacat     300

catccctggg cagaaaggcc ttcttctcat gacttcatga tctgaatccc cccgagtcat     360

tcattctcca tgaagtcatc gattttccag gtgttggtga actgcctgtg actcctctcc     420

tccccggccc ctacccctca gggataatga gttcattgct gggctagatg ttttagccat     480

gattctgcc                                                             489


<210>  438
<211>  580
<212>  DNA
<213>  Homo sapiens


<220>
<221>  misc_feature
<222>  (275)..(275)
<223>  n is a, c, g, t or u

<220>
<221>  misc_feature
<222>  (284)..(285)
<223>  n is a, c, g, t or u

<220>
<221>  misc_feature
<222>  (293)..(294)
<223>  n is a, c, g, t or u

<220>
<221>  misc_feature
<222>  (303)..(304)
<223>  n is a, c, g, t or u

<220>
<221>  misc_feature
<222>  (313)..(314)
<223>  n is a, c, g, t or u

<220>
<221>  misc_feature
<222>  (322)..(323)
<223>  n is a, c, g, t or u

<220>
<221>  misc_feature
<222>  (327)..(327)
<223>  n is a, c, g, t or u

<220>
<221>  misc_feature
<222>  (332)..(333)

<223>  n is a, c, g, t or u

<220>
<221>  misc_feature
<222>  (350)..(351)
<223>  n is a, c, g, t or u

<220>
<221>  misc_feature
<222>  (369)..(370)
<223>  n is a, c, g, t or u

<220>
<221>  misc_feature
<222>  (386)..(386)
<223>  n is a, c, g, t or u

<220>
<221>  misc_feature
<222>  (389)..(389)
<223>  n is a, c, g, t or u

<220>
<221>  misc_feature
<222>  (408)..(408)
<223>  n is a, c, g, t or u

<220>
<221>  misc_feature
<222>  (427)..(427)
<223>  n is a, c, g, t or u

<220>
<221>  misc_feature
<222>  (446)..(446)
<223>  n is a, c, g, t or u

<220>
<221>  misc_feature
<222>  (523)..(523)
<223>  n is a, c, g, t or u

<400>  438
   agcgagaccc agactcgtac aacaaacacc tcttcgtgca cattgggcat gccaaccatt      60
   cttacagtga cccattgctt gaatcagtgg acattcgtca gatttatgac aaatttcctg     120
   aaaagaaagg tggcttaaag gaactgtttg gaaagggccc tcaaaatgcc ttcttcctcg     180
   taaaattctg ggctgattta aactgcaata ttcaagatga tgctggggct ttttatggtg     240
   taaccagtca gtacgagagt tctgaaaata tgacngtcac ctgnnccacc aanntttgct     300
   ccnntgggaa gcnngtagta gnnaaantag anncggagta tgcaaggttn nagaatggcc     360
   gatttgtann ccgaataaac cgctcnccna tgtgtgaata tatgatcnac ttcatccaca     420
   agctcanaca cttaccagag aaatanatga tgaacagtgt tttggaaaac ttcacaattt     480
   tattggtggt aacaaacagg gatacacaag aaactctact ctngcatggc ctgtgtgttt     540
   gaagtttcaa atagtgaaca cggagcacaa catcatattt                           580


<210>  439
<211>  581
<212>  DNA
<213>  Homo sapiens

<400> 439
gcacggacac ctatgaagac cagcagtgga gaccccccaa gcccactggt gaaacagctg 60

agtgaagtat ttgaaactga agactctaaa tcaaatcttc ccccagagcc tgttctgccc 120

ccagaggcac ctttatcttc tgaattggac ttgcctctgg gtacccagtt atctgttgag 180

gaacagatgc caccttggaa ccagactgag ttcccctcca aacaggtgtt ttccaaggag 240

gaagcaagac agcccacaga aacccctgtg gccagccaga gctccgacaa gccctcaagg 300

gaccctgaga ctcccagatc ttcaggttct atgcgcaata gatggaaacc aaacagcagc 360

aaggtactag ggagatcccc cctcaccatc ctgcaggatg acaactcccc tggcaccctg 420

acactacgac agggtaagcg gccttcaccc ctaagtgaaa atgttagtga actaaaggaa 480

ggagccattc ttggaactgg acgacttctg aaaactggag gacgagcatg ggagcaaggc 540

caggaccatg acaaggaaaa tcagcacttt cccttggtgg a 581

<210> 440
<211> 449
<212> DNA
<213> Homo sapiens

<400> 440
ggcgtataat tcagccctgt ttaaatatac ttgcctttca aattcttcaa gtaacatggg 60

aagtattctt gaaatgtcac attttctgcc ttccctctaa gtatgctttc tgaagaagtc 120

agggaaagtt agagtctgtg gcctgaggtg tctgctctgg gtggcgatag tgggcacctc 180

aggcaggtcg gtgacgttta gcacaggtgc cagggctcct gcctgctcct cctgtgttag 240

ctctgtgaag ttcatttagg aatttttttt tcctatgcag tttaagaaat aatcctaatt 300

gtttttcttt attacctaag caatatattt ttattatagc aacctcagaa aagaaaaata 360

aaaggataat ttaaaaaact cattcatagt ctcagttacc cagataacct cggttgtcac 420

cttggagtat cttgttgtag tccctttac 449

<210> 441
<211> 457
<212> DNA
<213> Homo sapiens

<400> 441
   agcagaggct catccgggag cagatacgcc aggagcgtga ccagaggttg agaggaaagg 60
   cagaaaatac tgaaggccaa ggaaccccca aactaaagct aaaatggaag tgcaagaagg 120
   aggatgagtc aaaaggtggc tactccaaag acgtcctcct acggcttttg cagaagtatg 180
   gtgaggttct caacctggtg ctttccagta agaagccagg cactgctgtg gtggagtttg 240
   caaccgtcaa ggcagcggag ctggctgtcc agaatgaagt tggcctggtg gataaccctc 300
   tgaagatttc ctggttggag ggacagcccc aggatgccgt gggccgcagc cactcaggac 360
   tgtcaaaggg ctcagtgctg tcagagaggg actacgagag cctcgtcatg atgcgcatgc 420
   gccaggcggc cgagcggcaa cagctgatcg cacggat 457

<210>  442
<211>  498
<212>  DNA
<213>  Homo sapiens

<400>  442
aaggctatta acgacgcgat ttcacaaagt cggcagagtt ctgcgggaaa tcccctggaa      60

agactcaatt aaagagcagt gaagagagtg cagatcccgt cactggaagt tcggaaaatg     120

cagtgtcatc ttcagaactg atgtcccaga ctcccagtga agttctgggt accaacgaga     180

atgagaaact gagccctaca agtaatacct catatagttt agaaaaaatc tccagtctgg     240

cccctcctag catggagtac tgcgttttac tcttctgctg ttgtatttgt ggttttgaat     300

caaccagcaa agaaaacctc ttggatcata tgaaagagca cgagggtgaa attgtaaaca     360

tcatcctgaa taaggaccac aatacagctc taaacacaaa ttaggtggaa taatgactcg     420

agcaggaaag cagtagaaga ggattccttc accacagttt cacctttacg ctgtcagaca     480

acttcctgcc acagaaga                                                   498


<210>  443
<211>  476
<212>  DNA
<213>  Homo sapiens


<220>
<221>  misc_feature
<222>  (73)..(73)
<223>  n is a, c, g, t or u

<220>
<221>  misc_feature
<222>  (243)..(243)
<223>  n is a, c, g, t or u

<220>
<221>  misc_feature
<222>  (245)..(245)
<223>  n is a, c, g, t or u

<220>
<221>  misc_feature
<222>  (269)..(269)
<223>  n is a, c, g, t or u

<220>
<221>  misc_feature
<222>  (320)..(320)
<223>  n is a, c, g, t or u

<220>
<221>  misc_feature
<222>  (362)..(362)
<223>  n is a, c, g, t or u

<220>
<221>  misc_feature
<222>  (370)..(370)
<223>  n is a, c, g, t or u

<220>

<221>  misc_feature
<222>  (390)..(390)
<223>  n is a, c, g, t or u

<220>
<221>  misc_feature
<222>  (398)..(398)
<223>  n is a, c, g, t or u

<220>
<221>  misc_feature
<222>  (419)..(420)
<223>  n is a, c, g, t or u

<220>
<221>  misc_feature
<222>  (429)..(429)
<223>  n is a, c, g, t or u

<220>
<221>  misc_feature
<222>  (438)..(438)
<223>  n is a, c, g, t or u

<400>  443
caaccgagag ggccggcagg agcttgaaat cattattgga gatgaacaca tttcttttac      60

aacatcaaaa atnggttccc ttattgatgt cagtcaatcc aaggatccag aaggcttatg     120

agtattttat tatcctgtcc aggaccctga agtgtttggt cttcagtctt actggattac     180

acttcaagat taaaccaatc taaactgaat attgatgtgg acatgggggg gtgggagtag     240

ttntnaatta ccattatcaa gaacatttng tgtcagggca gtatattttt ataaactata     300

tatgattatc tttaataaan tatgtgataa aatttaaaaa aagcaaaaca aaacttctag     360

angaataccn tcaaaacctt ggtgagggan attcttanac agcacaaaaa tcattaggnn     420

aagatcaant ttaacatngt caaattaatc aatgacttct cttcctcaaa agacat         476


<210>  444
<211>  133
<212>  DNA
<213>  Homo sapiens

<400>  444
ttccagagct acccagacca tatggtgcac ccacagatcc agctgcagct ggtcctttag      60

gtccatgggg atccatgtct tctggacctt gggcgccagg aatgggaggg cagtatccta     120

cccctaatat gcc                                                        133


<210>  445
<211>  353
<212>  DNA
<213>  Homo sapiens

<400>  445
cgccgctgcg aattctcgga caaaactgtc aacagcccgg gcgcgccttt tggctctgcg      60

ggtccctcta tttatgcaaa gccgacctat gctacagccc cccaaccccc gacctggggt     120

agggaggaag agggtgccgg ggaagggagt ccgccctgtc caggcactag aggctccctt     180

gacgtttggc agatgaaaaa caactaagcc tttttgaggt gtagagattc tcaggtccag     240

gcgttaaaaa ataatggtca aaagaataat acaaaaatag taaaggtctt gaagaatgcc 300

agcgaagcaa ttctttttta tttgaggaca cttgtctggt gtactttttc atg 353

<210> 446
<211> 416
<212> DNA
<213> Homo sapiens

<220>
<221> misc_feature
<222> (275)..(278)
<223> n is a, c, g, t or u

<400> 446
gaggaagata tcctggctgg cactctttca gttgacagag agtgacctca ggctggggcg 60

gctcctcctc cgtgtggccc cggatcagca caccaggctg ctgcctttcg ctttttacag 120

tcttctctcc tacttccatg aagacgcggc catcagggaa gaggccttcc tgcatgttgc 180

tgtggacatg tacttgaagc tggtccagct cttcgtggct ggggatacaa gcacagtttc 240

acctccagct ggcaggagcc tggagctcaa gggtnnnnca gggcaacccc gtggaactga 300

taacaaaagc tcgtcttttt ctgctgcagt taatacctcg gtgcccgaaa aagagcttct 360

cacacgtggc agagctgctg gctgatcgtg gggactgcga cccagaggtg agcgcc 416

<210> 447
<211> 409
<212> DNA
<213> Homo sapiens

<400> 447
gctccccaca tgctggtggt gtactctgct aatggagaga tgtttaaact gagagctgct 60

gatgcaaaag agaaacaatt ctgggtgact cagcttcgag cttgtgccaa ataccacatg 120

gaaatgaatt ctaagagtgc tccaagctcc cgaagccgaa gtctcacttt gctcccacat 180

ggaacaccca attctgcgtc tccctgtagc cagagacacc tcagtgtggg ggcccccggt 240

gttgtcacaa tcacgcatca caagtcgcct gcagccgccc gaagagccaa gagtcagtat 300

tccggccagc ttcacgaagt cagagaggta cacactctcc tgacagagga aagctgtttg 360

ctgcactggt ttactggata gattaactgg gttgaggctg tgtaattta 409

<210> 448
<211> 316
<212> DNA
<213> Homo sapiens

<400> 448
gaggggcaca tgcaagtcac caaagtggga agccttcacc aaggccacac ccaaagtcta 60

ctgattgtct gtccaaagtt cgttgattcc tggccatgaa caagcacaat agaaaaagac 120

acagggtcct agtggctaca agtcaatgtg aattggcaca tggtctagca gttttaaaat 180

ctgacagtag agtatggcaa tgggcaaggg ccaagaagtc ctgagatggg aggtcagcgc 240

tctaactggg ctcagtggag gtctgtgacc agtgtctgga cactagctac aggggaccgg 300

gcagaggatt ctgggc 316

<210> 449
<211> 473
<212> DNA
<213> Homo sapiens

<220>
<221> misc_feature
<222> (241)..(241)
<223> n is a, c, g, t or u

<220>
<221> misc_feature
<222> (341)..(341)
<223> n is a, c, g, t or u

<220>
<221> misc_feature
<222> (384)..(385)
<223> n is a, c, g, t or u

<220>
<221> misc_feature
<222> (417)..(417)
<223> n is a, c, g, t or u

<220>
<221> misc_feature
<222> (420)..(420)
<223> n is a, c, g, t or u

<220>
<221> misc_feature
<222> (427)..(427)
<223> n is a, c, g, t or u

<220>
<221> misc_feature
<222> (430)..(430)
<223> n is a, c, g, t or u

<400> 449
gcactttagt gattgctttt attacattag ttaagatgtc ttgagagacc atctcctatc 60

ttttatttca ttcatatcct ccgccctttt tgtcctagag tgagagtttg gaaggtgtcc 120

aaatttaatg tagacattat cttttggctc tgaagaagca aacatgacta gagacgcacc 180

ttgctgcagt gtccagaagc ggcctgtgcg ttcccttcag tactgcagcg ccacccagtg 240

naaggacact cttggctcgt ttgggctcaa ggcaccgcag cctgtcagcc aacattgcct 300

tgcatttgta ccttattgat ctttgcccat ggaagtctca nagatctttc gttggttgtt 360

tctctgagct ttgttactga aatnngcctc gtggggagca tcagagaagg ccaggangan 420

tggtgtnttn ccctagactc tgtaaccacc tctctgtctt tgtccttcct gag 473

<210> 450
<211> 512
<212> DNA
<213> Homo sapiens

<220>
<221>  misc_feature
<222>  (363)..(363)
<223>  n is a, c, g, t or u

<400>  450
gggaagtagg tgatgccagc cctcaagtct gtcttcagcc agggacttga gaagttatat      60

tgggcagtgg ctccaatctg tggaccagta tttcagcttt ccctgaagat caggcagggt     120

gccattcatt gtctttctct cctagccccc tcaggaaaga aggactatat ttgtactgta     180

ccctaggggt tctggaaggg aaaacatgga atcaggattc tatagactga taggccctat     240

ccacaagggc catgactggg aaaaggtatg ggagcagaag gagaattggg attttagggt     300

gcagctacgc tcaccctaaa cttttggtgg cctggggcat gtcttgaggc ccagactgtt     360

aancaggctc tgctggcctg tttactcgtc accacctctg cacctgctgt cttgagactc     420

catccagccc caggcacgcc acctgctcct gagcctccac tatctccctg tgacgggtga     480

acttcgtgta ctgtgtctcg ggtccatata tg                                   512

<210>  451
<211>  397
<212>  DNA
<213>  Homo sapiens

<400>  451
gtgaacattt caaccagcct tatagctgtt ctcatcatca ccttctgcat tgtgaccgtg      60

cttggaaggg aggctctcac caaaggggcg ctgtgggcag tctttctgct cgcagggtct     120

gccctcctct gtgccgtggt cacgggcgtc atctggaggc agcccgagag caagaccaag     180

ctctcattta aggttccctt cctgccagtg ctccccatcc tgagcatctt cgtgaacgtc     240

tatctcatga tgcagctgga ccagggcacc tgggtccggt ttgctgtgtg gatgctgata     300

ggcttcatca tctactttgg ctatggcctg tggcacagcg aggaggcgtc cctggatgcc     360

gaccaagcaa ggactcctga cggcaacttg gaccagt                              397

<210>  452
<211>  426
<212>  DNA
<213>  Homo sapiens

<220>
<221>  misc_feature
<222>  (32)..(32)
<223>  n is a, c, g, t or u

<400>  452
gactgtaggt gcgtgggaga aactttgcag gntggggacc cggcggctgc tggccggtag      60

tgactggtgg gcgcgctcga ggactccaag gggcgcagcc cgggggcaga cccttgggtc     120

gggcggggat cttacgcttc ccttacccgc ccccttttgt ctttcacctc agccccgccg     180

gctgctgtgg gagcggcggc cgtccctctc ctggaggtcg tctcctggca tcctcggggc     240

cgcaggaagg aagaggaggc agcggccgga gccctggtgg gcggcctgag gtgagagccc 300

gaccggcccc tttgggaata tggcgaccgg tggctaccgg accagcagcg gcctcggcgg 360

cagcaccaca gacttcctgg aggagtggaa ggcgaaacgc gagaagatgc gcgccaagca 420

gaaccc 426

<210> 453
<211> 384
<212> DNA
<213> Homo sapiens

<400> 453
ctaaagaaag tacacacact ctctcgctct ctctcggtct tataaaactc gttggtgtct 60

tataaaacaa acagtgataa tctcaagtta gaaaacagta ggtcctgaga accataagaa 120

aaatgactgg tgtgatgttg agtaacaagt tggtacagtt actttagcta tttattaact 180

tgctcatctc atagaacatt ttaatagatt tttcacacac ctcattatta aaaaaaaaca 240

aacatgctgg tgtcttggtt acccattatt cctctgtacc tgaattcagg ttggtttttc 300

tatttggaaa agactttata aatgttggct taaaaagagg ttgagcacca gaatctcaga 360

atttaccacc aaagaactca tcca 384

<210> 454
<211> 407
<212> DNA
<213> Homo sapiens

<400> 454
agcataatga agcctgcatg tgcccagctt caataattac caatatcttg ccagttttgt 60

ttcgtttctc ctttgattct ctgtattgag caagtcttag acatcatacg tttcccgcgt 120

aagtacctta ttctacatca ttaaccagta aggacttttt aattaaccac aataccacta 180

tcacacctaa taatagtaat tccttatgga tcttttcttt agacctattt ttgaaggcat 240

aaaagcagtt gagtttctgg agaatttttg gatggtgatt aatgacttga ctggctgctc 300

ttcccagagc tgtggcagct ctccccccgt agaagatggg gtttgtattg gcgcaccaag 360

atctccaaca gccagtgtgt gtttcccatt tcctgtaggt tccatca 407

<210> 455
<211> 223
<212> DNA
<213> Homo sapiens

<400> 455
tagtcagagt gacccatgta tctgggaaga ctctagtctg gactgtggcc cagcttgggg 60

accttgtgtg ctcagatcat cttcaggaag gaaaaggcat cctggagaca ggagtccatt 120

cactcctctg ctctctaccc actcatttgc ttgccaaact tagctttgcc agtgatagtc 180

aatattaaag tgtacttttt tcccctttaa tccaatatag ttg 223

<210> 456
<211> 160

<212>  DNA
<213>  Homo sapiens

<400>  456
tataattata accttaccgc atggacagtt ttgaatccta tgctaattgg ggtaattaag      60

tcaattattt catatgttat gttctcttca tgtgcatttt tcaatgatat attatgttcc     120

attgtgttgg aatgtgaatg ttcaattact tttccctata                           160


<210>  457
<211>  465
<212>  DNA
<213>  Homo sapiens

<400>  457
ccacatccat ggcctaggag ctactgggca ggttcccggc cacacatctg gtgggctgtt      60

ttgttttttt ttttcctctt cccccagatg tcttgacggg atcactgggg ctctttgtga     120

gtgagggtgg ccaaactacc gccggaggag atggggtctc agagcgagag ctgcggaggg     180

ggaggggaag aagaaggcct cacttttgct gctgcggggc ccacacagcc gctgctactt     240

tggggggtgg ggaaggggcc aagctgcaga cacacacagt cattcatttc tgtccacacc     300

cctgtgggtg gcgggtgtgc gtgtgtgtgc ttgtgtgtgc gcacgtgtcg gcgctcacac     360

acacatgcta gcccactgat gcacccagcc cagggctggc agtctttgca gcgtggggcc     420

gtctcaccct ggagcctgga gaggatctat gcttgtttgt ttttg                      465


<210>  458
<211>  212
<212>  DNA
<213>  Homo sapiens


<220>
<221>  misc_feature
<222>  (122)..(122)
<223>  n is a, c, g, t or u

<220>
<221>  misc_feature
<222>  (158)..(158)
<223>  n is a, c, g, t or u

<400>  458
gtgccgctgg cacccgggaa gacgctgggg gccggcgctg tagagccggg catgggctgg      60

gatgtgtttg gattccaatc cgggcctgac accagttcag tgacctcggg aagttcccca     120

ancctccggg cctgtttcct ccctctgaag tggcgacnag tagtagaacc gacctcgtag     180

gctcatcggg aggtcctgat gggagaaccc at                                   212


<210>  459
<211>  342
<212>  DNA
<213>  Homo sapiens


<220>
<221>  misc_feature

<222>  (161)..(162)
<223>  n is a, c, g, t or u

<400>  459
ggttgtactc aagatgtttt cctggaaaaa ttcattctgc tttctgacca ggatttccag      60

aaactctgac ccttctaaga ggtctgggtg gaattgtgat ggtgattctg ctagtagaca     120

gtgtaacttc tgcgtctaca aaaagaggat aggccgtcac nnctcacatg gctttgcgtg     180

aaagcccaat ggtactgtct ctatggcaga gatgaggaag gaacaccagc gtcctccaac     240

tttcctgttc ttcctttggg ttaatggcca ctgtaaggaa acagttttct gccacgtgtg     300

gggtgatttg aatgtaaaat gcccaactct catagcaggc tg                        342

<210>  460
<211>  519
<212>  DNA
<213>  Homo sapiens

<400>  460
aaggggaaga tttgctgctg ctgccgggcc aagttcccgc tgttctcgtg gccgcccagc      60

tgtctcttct gcaagagagc cgtctgcact tcctgtagca taaagatgaa gatgccttct     120

aagaaatttg gacacatccc tgtctacaca ctgggctttg agagtcctca gagggtatca     180

gctgccaaaa ccgcgccaat ccagagaaga gacatctttc agtctctgca agggccacag     240

tggcagagcg tggaggaggc gttcccccac atctactccc acggctgtgt cctgaaggat     300

gtctgcagtg agtgcaccag ctttgtggca gacgtggtgc gttccagccg caagagcgtg     360

gacgtcctca acactacgcc acgacgcagt cgccagaccc aatccctcta catccctaac     420

accaggactc ttgacttcaa gtgacagccc caggtggcca ggcctccagg aggcaccagg     480

caggccctgt atcaggctag gacgctctga gctgtgcat                            519

<210>  461
<211>  208
<212>  DNA
<213>  Homo sapiens

<400>  461
   tcccccctct gaattttact gatgaagaaa ctgaggccac agagctaaag tgacttttcc      60
   caaggtcgcc cagcgaggac gtgggacttc tcagacgtca ggagagtgat gtgagggagc     120
   tgtgtgacca tagaaagtga cgtgttaaaa accagcgctg ccctctttga aagccaggga     180
   gcatcattca tttagcctgc tgagaaga                                        208

<210>  462
<211>  532
<212>  DNA
<213>  Homo sapiens

<400>  462
   ctcagcattt agtgaaggta attccaaaat actggtatca gtactcttat ttataagtgt      60
   acggaatgca taacatgaac attagtcaaa gaacttttaa tataattcac tttttaagtg     120
   ttaaaattta aaggtcaagt aaaattgtaa atttgtaata tggaaacatt aagcgtcatt     180
   atcatacaaa ttattagcag ataaccttaa taaaaataaa cgtttgcggg ttttttttga 240
   gacagggtct cgctttgtca cctaagctgg agtgcagtgc gcgatctcgg ctcactgcaa 300
   cttccgcctc ctgggatcaa gtgattctcc tgccttagcc tcctgagtat ctgggtttac 360
   aggtgtgtac cgccacaccc gtctctacta aaaatacaaa aaacaaaaaa agattagctg 420
   ggcgtggtgg caggtgcctg tggtcccagc tgctcgggag gctgaggcag gagaatagca 480
   tggacctggg aggcggagct tgcagtgagc tgaaatggtg ccactgcact cc 532

<210> 463
<211> 542
<212> DNA
<213> Homo sapiens

<400> 463
attatcgatc atgtctattg ctccccgtcc cttcgctgcg ttcagactgc acacaatatc 60

ttgaaaggtt tacaacaaga aaatcacttg aagatccgtg tagagcccgg cttatttgag 120

tggacaaaat gggttgctgg gagcacatta cctgcatgga tacctccatc agagttagct 180

gcagccaacc tgagtgttga tacaacctac agacctcaca ttccaatcag caaattagtt 240

gtttcagaat cctatgatac ttatatcagt agaagtttcc aagtaacaaa agaaataata 300

agtgaatgta aaagtaaagg aaataacatc ctgattgtgg cccacgcatc ttcccttgaa 360

gcgtgtacct gccaacttca gggcctgtca cctcagaact ccaaggactt cgtacaaatg 420

gtccgaaaga tcccatatct gggattttgt tcctgtgaag aattaggaga aactggaata 480

tggcagctga cagatccacc aatccttcct cttacccatg gaccaactgg gggcttcaac 540

tg 542

<210> 464
<211> 451
<212> DNA
<213> Homo sapiens

<220>
<221> misc_feature
<222> (368)..(368)
<223> n is a, c, g, t or u

<400> 464
cagccccatg acagcgaagg gacctttctg tccccgcccc tgtccctgtg ctgggcccac 60

gtactcaccc acgtactggt gcccggctcc cctgggcacc cagagccccc cagataggcc 120

ggtggaggag gtggaggagc tgtcccccca aaactactgg cctgtggtct ggactccagg 180

gccccatttc tgatgtcgcc aggtgtgcct gagcccatcg gggccaggcc tgaggaagtg 240

tttcttggga ggatgggatg accccctgtt cccaagagat ggcagcacag tggaggccat 300

ggtggaaaag gccctgccat ggggtccttg agggccagga cagcctgagg gagggatggt 360

ggccactncc cacaaggggc ctggtgggaa cgggtcccag gacagactca tagctagacc 420

ccgttggcgg cctctgtgtt gaaccagaac t 451

<210> 465
<211> 467
<212> DNA
<213> Homo sapiens

<400> 465
ggccccaggc agttttatga tgacacctgt gttgtcccag aaaaattcga tggagacatc 60

aaacaagagc caggaatgta tcgggaagga cccacatacc aacggcgagg atcacttcag 120

ctctggcagt ttttggtagc tcttctggat gacccggcaa attctcattt tattgcctgg 180

actggtcgag gcatggaatt taaactgatt gagcctgaag aggtggcccg acgttggggc 240

attcagaaaa acaggccagc tatgaactat gataaactta gccgttcact ccgctattac 300

tatgagaaag gaattatgca aaaggtggct ggagagagat atgtctacaa gtttgtgtgt 360

gatccagaag cccttttctc catggccttt ccagataatc agcgtccact gctgaagaca 420

gacatggaac gtcacatcaa cgaggaggac acagtgcctc tttctca 467

<210> 466
<211> 405
<212> DNA
<213> Homo sapiens

<220>
<221> misc_feature
<222> (162)..(162)
<223> n is a, c, g, t or u

<220>
<221> misc_feature
<222> (301)..(301)
<223> n is a, c, g, t or u

<400> 466
catacaccta ttaccataca ggggaagtcc ccaagctctc cggcctcaca gactctcacc 60

cacgggcaga gcattcttgg ctgattgagg ggaagttcca gcaatcagca caagtgttct 120

ttatacccca aatcactaaa acatatagag gggtctatgt cngtttcatc cataactcag 180

ccactggtgg aacaaatctc ataatcaaga ggatcatagt ccctggtaag tggatccctg 240

gagcattggc accatgtttt ccagtaaagt ctatctagct gtcagggaag agccacctgc 300

nctctgcaaa gggagaggga aaatcaaaac ccaggaaagg gaatatgttt ctgctccaaa 360

accaccagct tctgcctgtc cccttcactc tttctagatc attct 405

<210> 467
<211> 110
<212> DNA
<213> Homo sapiens

<400> 467
gaaagagcga gagaagggga aagacaagtc gggagaggcc ggtaggcgtg aggcgggcct 60

gaagcggcag cgggcggcct tcgtccggcg agagctaggc cgaggacccg 110

<210> 468
<211> 204
<212> DNA
<213> Homo sapiens

<400> 468
ctgcccccca gggctagtga agtggcctct tggataccag ctcaggggac actggcccca 60

caggagttgt gagccctcta gggcagggtg ggagccggga ccctcaggtg tagctgagct 120

gtgacattgc tggtcatcct tggtgctctt gcttttttga aagatgcttt tttttttttt 180

aactgacgta gaatgaagaa ctgc 204

<210> 469
<211> 139
<212> DNA
<213> Homo sapiens

<400> 469
tcagatagga aggatggata tgtctttatc tacagcagaa gttagttacc ctttcatgag 60

gtgattagtt tacttctagg tggaaaaaga gaggactttg aacttggtgt tgtcacagga 120

gctgctctca tggacaaga 139

<210> 470
<211> 115
<212> DNA
<213> Homo sapiens

<220>
<221> misc_feature
<222> (81)..(81)
<223> n is a, c, g, t or u

<400> 470
ctcagagatt actcagccag acagagatat tccactggtg cgaaagttac gttccattca 60

cagctttgag ctggaaaaac ntctgaccct ggagccaaag ccagacactg acaag 115

<210> 471
<211> 475
<212> DNA
<213> Homo sapiens

<400> 471
cagcgcctcc ggttataagt tgaagaaata agaccagttt ccaaataaat gacaaagagc 60

ttggtattcc tgcaggcatc agaatcacct ggaggaggag atgctgctgc tggtggtggc 120

ccagagacca cacattgaga accactgctc tagaaaacca tttgtctttg ctgatggaga 180

aacctggctc taatagaagg gcttgtatgt gtccaggaag tctagtgaat tcgaccatga 240

atccagacat ggccagtggc taaatcctgt gggaagacac tgtgcttctc tctgacccat 300

gaacactctg ctagtcaagc tctctgtcac aaagacaact tgaagagaca gagtggacct 360

cacagaagat accatcgtca ctcttaccaa tgcaactgtg gtgaacagga ccactattat 420

tccttagatc aaaaggacag cacattcaac agcatcctca tggcatgcca gcaat 475

<210> 472
<211> 446
<212> DNA
<213> Homo sapiens

<400> 472
cggcttgttg ggaccaccaa ccaaggggac cagcgcatcc tgcgcagcag cgcccctccc 60

tccctggctg gccctgctgt tagtcacaga ggccgcaagg ccaagacgtg agtgggctgc 120

ccctccacct aggctttcca ccgtggccac tccctccatg accaggcctg actctgttaa 180

ccactacttg aagtcttgag ggggaaagcc tccagggaga cataggggcc ttctcccttc 240

ttcccaccaa agtagggggt aggcaactgg ttgtcatgga aatggggatc atcacagtcc 300

ccttcccctt caccccacgt ggctgggcag tgttaagggt ggcaagatag tctctgtccc 360

cacccccttg tacttgattc cccagctgtc tttcacacag ccccccaccc ttaggggaag 420

ggggaggggc ttctctacaa tgaggt 446

<210> 473
<211> 443
<212> DNA
<213> Homo sapiens

<400> 473
gagacttggt ggtctgagct gtcccaagtc ctccggttct tcctcgggat tggcgggtcc 60

acttgccagg gctctggggg cagatttgtg gggacctcag cctgcaccct cttctcctct 120

ggcttccctc tctgaaatag ccgaactcca ggctgggctg agccaaagcc agagtggcca 180

cggcccaggg agggtgagct ggtgcctgct ttgacgggcc aggccctgga gggcagagac 240

aatcacgggc ggtcctgcac agattcccag gccagggctg ggtcacagga aggaaacaac 300

atttcttgga aaggggaaac gtctcccaga tcgctccctt ggctttgagg ccgaagctgc 360

tgtgactgtg tccccttact gagcgcaagc cacagcctgt cttgtcaggt ggaccctgta 420

aatacatcct ttttctgcta acc 443

<210> 474
<211> 465
<212> DNA
<213> Homo sapiens

<400> 474
cctaattcac acaaagactc cttgtggact ggctgtgccc ctgatgcagc ctgtggctgg 60

agtggccaaa taggagggag actgtggtag gggcagggag gcaacactgc tgtccacatg 120

acctccattt cccaaagtcc tctgctccag caactgccct tccaggtggg tgtgggacac 180

ctgggagaag gtctccaagg gagggtgcag ccctcttgcc cgcacccctc cctgcttgca 240

cacttcccca tctttgatcc ttctgagctc cacctctggt ggctcctcct aggaaaccag 300

ctcgtgggct gggaatgggg gagagaaggg aaaagatccc caagaccccc tggggtggga 360

tctgagctcc cacctccctt cccacctact gcactttccc ccttcccgcc ttccaaaacc 420

tgcttccttc agtttgtaaa gtcggtgatt atatttttgg gggct 465

<210> 475
<211> 443
<212> DNA
<213> Homo sapiens

<400> 475
agaatgcaaa gaggccgctt ccctaagagg cttggaggag ctgggctcta tcccacaccc 60

acccccaccc cacccccacc cagcctccag aagctggaac catttctccc gcaggcctga 120

gttcctaagg aaaccaccct accggggtgg aagggagggt cagggaagaa acccactctt 180

gctctacgag gagcaagtgc ctgccccctc ccagcagcca gccctgccaa agttgcatta 240

tctttggcca aggctgggcc tgacggttat gatttcagcc ctgggcctgc aggagaggct 300

gagaccagcc cacccagcca gtggtcgagc actgccccgc cgccaaagtc tgcagaatgt 360

gagatgaggt tctcaaggtc acaggcccca gtcccagcct gggggctggc agaggccccc 420

atatactctg ctacagctcc tat 443

<210> 476
<211> 458
<212> DNA
<213> Homo sapiens

<400> 476
gactcagtgg gcactagaac gcctgaggct gcagctgggc tccccggggt ccttgcagag 60

gaaactcagt ctgctggagc aggaatccca gcagcaggag ctgcagatcc agggcttcga 120

gagtgacctc gccgagatcc gcgccgacaa acagaacctg gaggccattc tgcacagcct 180

gcccgagaac tgtgccagct ggcagtgagg gctgcccaga tccccggcac acactccccc 240

acctgctgtt tacatgaccc agggggtgca cactacccca caggtgtgcc catacagaca 300

ttccccggag ccggctgctg tgaactcgac cccgtgtgga tagtcacact ccctgccgat 360

tctgtctgtg gcttcttccc tgccagcagg actgagtgtg cgtacccagt tcacctggac 420

atgagtgcac actctcaccc ctgcacatgc ataaacgg 458

<210> 477
<211> 475
<212> DNA
<213> Homo sapiens

<220>
<221> misc_feature
<222> (342)..(342)
<223> n is a, c, g, t or u

<400> 477
agcatcctga accagctgtg ttttattatg cacagatatc gtaaaaattt gactgccgca 60

aagaaaaatg agttggtaca aaagacaaaa tcagagttca atttcagcag caagacttat 120

caagaattta attactattt gacatcaatg gttggttgcc tgtggacgtc caaacccttt 180

gcgaaaggaa tatatattga ccctgaaatc ctagaaaaaa ctggagtggc tgaatataaa 240

aacagtttaa atgtagtcca tcatccttct ttcttgagtt acgctgtttc ctttttgcta 300

caggaaagcc cagaagaaag gacagtaaac gtgagctcta tncggggaaa gaaatggagc 360

tggtatttgg actatttatt ttcacagggg ttacaaggct tgaaactttt tataagaagt 420

agtgttcatc attcttccat tcccagagca gagggcataa actgcaacaa tcaat 475

<210> 478
<211> 490
<212> DNA
<213> Homo sapiens

<400> 478
ctcgcagagt tccgtcgatc aggactggag gaagccacgt ttcaacagat atatagtcaa 60

catgtggcac tgtgcagaat ggagggactg ccgtacccca ccatgtcaga gaccatggcc 120

gtgtgttctc acctgggctc ctgtcgcctc ctgcttgtgg agcccagcag gaacgatctg 180

ctccttcggg tgcggctcaa cgtcagccag gatgatgtgc tgtatgcgct gaaagacgag 240

taaaggggct tcacaagtta aaagactggg gtcttgctgg gttttgtttt ttgagacagg 300

gtcttgctct gtcgcccagg ctggagtgca gtggcacgat catggctcac tgcagccttg 360

acttctcagg cttaggtgac cccccaacct catcctccca ggtggctgaa actacaggca 420

catgccacca tgcccagctg attttttgta gagacagggc ttcaccatgt tgccaagcta 480

gtctacaaag 490

<210> 479
<211> 460
<212> DNA
<213> Homo sapiens

<220>
<221> misc_feature
<222> (72)..(77)
<223> n is a, c, g, t or u

<220>
<221> misc_feature
<222> (364)..(364)
<223> n is a, c, g, t or u

<400> 479
tttttagggg actctcaacc tcctggcagg gttaaaggga gagtacttta aacccatata 60

ccagctgtgc tnnnnnntct ctcactttgc cctgggtaag ctgctgtagg gtcagaagta 120

accctttctg tgccagttga gaatgagcct gtgtggtagc tgatgtcaga ggacaaagct 180

ctctgcaagg gctggacaca gagctgcaga gtcctgaaca tccctccttt caggctgcag 240

aagggagagg caatgaagac aggtgctccg gaagcagcat cagggctctt ggaggggact 300

ggtggggact caggctgggt gcagcctcca aacagagaac ggaacttagg tgtgtctcta 360

cagnctaggc ccagcctagc ccagcccaga acaaacaccc ttcagagcct aaccaaagaa 420

cataagctgc aaaatgtgca cccatatttt aagctgcttt 460

<210> 480

<211>  492
<212>  DNA
<213>  Homo sapiens


<220>
<221>  misc_feature
<222>  (77)..(77)
<223>  n is a, c, g, t or u

<400>  480
cctgtctcct acatttagcc aatgaaaaga atctaaaact ggaaggaaca gaggacctct      60

ctgatgttct tgtgagncaa ggagattgag ttcactatgg agaagtcagc agcaggaggc     120

ccatccctta ctcagttgcc gggacatccc cagtctcggg ggaagaagat gccatgggct     180

tatacccagg ctgtagccaa ctaccaacgt gcctgtttgt ttgttgctct ttccttctct     240

ccatcatagt ctgggtgcca gcgccctgaa gctccgtgct caactgatta aactttactg     300

ccctatggtg accatctagg agaggggagg gcagaggggg tgagggtact attctggatt     360

gagaaaacct atatccattc tttatatcaa tgtatagttt tagtctccta aattgatctg     420

ttattttcca aactattctc ttgtagaaaa ttttccagtg ggcacttaat ggtgcccttg     480

aagaacttcc ta                                                         492


<210>  481
<211>  501
<212>  DNA
<213>  Homo sapiens


<220>
<221>  misc_feature
<222>  (197)..(197)
<223>  n is a, c, g, t or u

<220>
<221>  misc_feature
<222>  (247)..(247)
<223>  n is a, c, g, t or u

<400>  481
ggagggagag gtccctgcaa ggtcccttcc cgggcagggg agggatggaa atgccgtcac      60

agtagtaggg actggagcgt ctacaaggat ggaggggagc tactcaggcc taacgttagc     120

tacaaggaaa aaggacgcct tccgtgacag atccttgagg tgtctgtgtc tgccccaagt     180

ggccggcagt ggccttncct ccgggcccaa ggcctgcagc cacctgctct aactcttgag     240

tgggggngcg gggggggacc tgcaggggct cggggacagg acagcagcaa gaggcagggg     300

ccgaggacgg aggccttccc gacagtgggg tgggttgtac attcaagtgt gaggtgaacc     360

ctttggtggg gagggggccc ctgaagcctc ggcggggcca cccctccccg cggcgcctct     420

gagtctaggg agaggggctg ctggctcggc ccggccggcc tggcttcaca gagggtctgc     480

ggattgacac tggttctttt c                                               501


<210>  482
<211>  490

<212>  DNA
<213>  Homo sapiens


<220>
<221>  misc_feature
<222>  (120)..(120)
<223>  n is a, c, g, t or u

<220>
<221>  misc_feature
<222>  (122)..(122)
<223>  n is a, c, g, t or u

<220>
<221>  misc_feature
<222>  (185)..(185)
<223>  n is a, c, g, t or u

<220>
<221>  misc_feature
<222>  (271)..(271)
<223>  n is a, c, g, t or u

<220>
<221>  misc_feature
<222>  (306)..(306)
<223>  n is a, c, g, t or u

<220>
<221>  misc_feature
<222>  (313)..(313)
<223>  n is a, c, g, t or u

<220>
<221>  misc_feature
<222>  (352)..(354)
<223>  n is a, c, g, t or u

<220>
<221>  misc_feature
<222>  (357)..(357)
<223>  n is a, c, g, t or u

<220>
<221>  misc_feature
<222>  (359)..(359)
<223>  n is a, c, g, t or u

<220>
<221>  misc_feature
<222>  (361)..(362)
<223>  n is a, c, g, t or u

<220>
<221>  misc_feature
<222>  (365)..(365)
<223>  n is a, c, g, t or u

<220>
<221>  misc_feature
<222>  (367)..(367)
<223>  n is a, c, g, t or u

<220>
<221>  misc_feature
<222>  (371)..(371)
<223>  n is a, c, g, t or u

<220>
<221> misc_feature
<222> (373)..(374)
<223> n is a, c, g, t or u

<220>
<221> misc_feature
<222> (376)..(377)
<223> n is a, c, g, t or u

<400> 482
gtgaggagct gttttcatct gtgtctgttg gagatcaaga tgattgctat tccctgttag 60

atgatcagga cttcacttct tttgatttat ttcctgaggg gagtgtctgc agtgatgtcn 120

cntcttctat tagcacttac tgggattggt cagatagcga gtttgaatgg cagttaccag 180

gcagntgaca ttgccagtgg gagtgatgta ctttctgatg tcatacccag tattccaagt 240

tcaccttgcc tgcttcctaa aaagaaaaac nagcaccgga atttagatga actcccttgg 300

agtgcnatga canatgatga gcaggtggaa tatattgagt atctgagtcg gnnngtnant 360

nntgngntgg ncnncnntac tgtcctgtgg tctagtgggc agggacctgg gggccatcag 420

tggctgtagg acttttttac ccctctgttc ctggcctaaa tatgtgatgg gtatgcttca 480

ccttaagtgg 490

<210> 483
<211> 231
<212> DNA
<213> Homo sapiens

<220>
<221> misc_feature
<222> (63)..(63)
<223> n is a, c, g, t or u

<400> 483
ctttcacact gtggcagccc agtgaagcag actgggccat gaactctcct agccctgggg 60

ccnagcctgt tccacaggca cccctgcagg aggcgctgcc aggagagcct tccatctcgg 120

ggctctttga ggttccctcc ttctgggtgt tcttcaggct gagcagagag gctcctgtac 180

cctctctctc ggaatctgaa gagccagatt taggccgggc aaaggggctc a 231

<210> 484
<211> 414
<212> DNA
<213> Homo sapiens

<400> 484
ggtgctggaa aaactactat cttgtttaag ttaaaacagg atgaattcat gcagcccatt 60

ccaacaattg gttttaacgt ggaaactgta gaatataaaa atctaaaatt cactatttgg 120

gatgtaggtg gaaaacacaa attaagacca ttgtggaaac attattacct caatactcaa 180

gctgttgtgt ttgttgtaga tagcagtcat agagacagaa ttagtgaagc acacagcgaa 240

cttgcaaagt tgttaacgga aaaagaactc cgagatgctc tgctcctgat ttttgctaac 300

aaacaggatg ttgctggagc actgtcagta gaagaaatca ctgaactact cagtctccat 360

aaattatgct gtggccgtag ctggtatatt cagggctgtg atgctcgaag tgtt 414

<210> 485
<211> 508
<212> DNA
<213> Homo sapiens

<400> 485
tcctctgtcc tctatattca gcatgttcct tgtcagctgc tgggccggcc ctgccttgcg 60

ctagcagagc ctctcctggc agcttctcag gtctccctaa tggagacacc aggctactag 120

gacactggct ggggccaccc cctcctgcct aatgcctcac cttacagctg gggaaactga 180

ggcctggaat ggcccagagt caccaaggca aagttggggc tggtcccagc ctgaggctcc 240

agctgatgcc ctcagctccc agagaggggg tgccccatct agctgggtgc aggggtcact 300

gcttgtcagc tcagggccct gtgcccgctt gcctgttccc ctacatctgt gcctgcacat 360

ccagaactgc ctccttgccg ctgcctccag gaagcccacc ttgagccaga gtcaagggct 420

gcagcactgc ccgatagaac acgcccgccc tcactgctgt tcttgcctta cagccaccat 480

gggaaagctg caacctttct gttttatt 508

<210> 486
<211> 555
<212> DNA
<213> Homo sapiens

<220>
<221> misc_feature
<222> (400)..(401)
<223> n is a, c, g, t or u

<220>
<221> misc_feature
<222> (410)..(410)
<223> n is a, c, g, t or u

<400> 486
tgtcaacttg tcatatacac ctccagggac caaaaacaaa agcagctcgg agtctgtgtt 60

gcctgattgg aaagtagaag ctctggtgta tgctacagca cataacacat ttttactaaa 120

ggaaaaaagc taattatgtc catgcctctc gtaaaactgg ggggaacctt aaagagaaag 180

aactaaggct taagttatct gtagtataat caattagaag taatgaatgg atgcatgtaa 240

aatggatgtg attttttttc aagcttattt tgaaatctta aaaatcaggt tacaccatag 300

ctactcaaaa gttttacaca cttaaaactc agatcagtaa gtgttggtac cttttagact 360

cataaaattg aataaaccat tgcaatgctt taaaaaaaan naaaaaaaan ggttttattg 420

ctatgatttt atggcagaca catccaagca aaaccatttt ccaaatgcag accttcctga 480

tgttatctga aatctgataa aatgacccta ctctctgctg tggttcattc ttgctccatg 540

ctgtccatat ttatg 555

<210>  487
<211>  541
<212>  DNA
<213>  Homo sapiens

<400>  487
gtggcactta ggcactatat tattgatatc tacaatggcc tcctggatgc acaaaagacc      60

ctgaagggct tttttgatca gcaaaacaaa aacagaaaag caaaaaacag ttaatttttg     120

tttggtcaag tttactcaac cagaccacct tgataccaac aatgctggag agcatttggc     180

aagagcaggg ccacaatgcc aaattccttg gaaaggtaga cttcctatga tactttcatg     240

gattggcaaa tttgtggggt ttttttggta gtagcttttg agaatgttag tttctggctg     300

gggtagtgac ttacatctgt aatcccagca cttcgggagg cgaaggcagg tggattgctt     360

gtgcccagga gtttgagacc agcctgggta acatggtgag accccatctc tatttttata     420

aaattaaaaa aaaaaaaaaa gatagagaat gttactttcc tataaagcca tgatacccta     480

agtactaaga catgtctgtt gttgtccttt ccttcataac atttctcata acccgtaatt     540

t                                                                     541

<210>  488
<211>  523
<212>  DNA
<213>  Homo sapiens

<220>
<221>  misc_feature
<222>  (86)..(86)
<223>  n is a, c, g, t or u

<220>
<221>  misc_feature
<222>  (106)..(106)
<223>  n is a, c, g, t or u

<220>
<221>  misc_feature
<222>  (140)..(140)
<223>  n is a, c, g, t or u

<400>  488
cagccctgac gtgaactcat tttattttgg ccaggaccca gaaaggagtc tactgctaag      60

atttcagcat gtcctgtggc tgagtnaatc agagttatga cagganggta ccgggcacac     120

catcgcaatg ctccatcaan gctagtatgt tgtgttcttt ccttcatatc aagtcaactc     180

aagcttgctc tacttacctg gtgtacacag tctaagaact gtaagaagac tggagcaaaa     240

ccactcccct gacagttgag ggtcaagctg ctcctctgac tgaatttgtg accaaaagag     300

agccactctt tttcaaccaa catctggaag ccttcaagtg tcctataaaa gggatcactg     360

agtaactgaa ccagggatgt cacctagggc ataagcagga tggattgtca ttaattttag     420

ttctgaaaaa ggcctattac taagataaaa gcacttcctt ctgatgatag ctaattcaca     480

aatttacctg gacagcaaat ttgttcacta accattccag gat                       523

<210>  489
<211>  306
<212>  DNA
<213>  Homo sapiens

<400>  489
cggctgtacg actccataat gggcatgggg actcaagata aggtcctgat cagaatcatg      60

gtctcccaca atgaagtgga catgttgaaa attaggtctg aattcaagag aaagtatagc     120

aagtccctgt actactatat ccagcaagac actaagggtg ctgtacctgt gtggtggaga     180

tggctgaagt ccgacacagc acgagcgtcc agaaatggtg ctccccatgc ttccagctaa     240

caggtctaga aaacccgctt gtgactagca gtccctgtgg ctgttcctgt gaggatgacg     300

ttagca                                                                306


<210>  490
<211>  170
<212>  DNA
<213>  Homo sapiens

<400>  490
agaagattcc cttgaagcct tctccttcca aaaagtttcg gtctggctca tctttctctc      60

ggcgagcagg ctccagtggc aactcctgca ttacttacca gccatcggtc tctggggaac     120

acaaggcaca agtgacaaca aaggcagaag tggagccagg cgttcacctt                170


<210>  491
<211>  532
<212>  DNA
<213>  Homo sapiens

<400>  491
tgggggtgac tgctgcttat taagatgatt catttcattt ccactcgtgg ttgtgatttt      60

caccttctca aaactgagtc agcaagagaa aatcttgtct tagaagggcc agataacact     120

tcgctgtgag aacaggaggg ataatggatt ggagatggct atgtgtaaag cagccctgcc     180

tgctgattta acacactttc aaaatagatg tgtcagtatt catttaaagc aagactctga     240

tgacagaagg aaccttgaaa actacctgat attgaaatgg ttgtgccctt tatagccctt     300

ttgcatctcc ttgactttcc agtcatgcct cctaaatcag aagaaaagct gcaaagaaaa     360

tgttttgtgt ggttctgggc ttatttgaat aatgttcatg accacaggct gccatagcac     420

aagtgagaat ttcagaccac aagggtttaa ggagcagtgc tctcttctct caaagctcag     480

aacggtctct ggatccatgg tatcgtacac ccagtgtgga tattaacatt ct             532


<210>  492
<211>  559
<212>  DNA
<213>  Homo sapiens


<220>
<221>  misc_feature
<222>  (232)..(232)
<223>  n is a, c, g, t or u

<220>
<221> misc_feature
<222> (259)..(259)
<223> n is a, c, g, t or u

<400> 492
aagggaagtt agcaccttcc tcttggaggt gctgaggatt aaatgagata atacgtggaa 60

agcattaggc atgtagcaca gttagcagat ggtggttggc tccctctgct tttccatcag 120

tctgtggcct agtttaaatg gtgggaggaa gggtgtgaga tttaaggctg gttgtaaggg 180

atcagtcagt gtagttggaa aaattgtaag atgaagttat aggatataga cncaaacctt 240

cctggaaggc cagaaagtnt gcatagcttc aataaaggat ttggctgaaa gcagcgtaat 300

cccctttacc ttgagttgat agcaatagag caaataacat gggaacgtgg gggagtttat 360

tgaatagctt gtttactcat gtggtcctaa gaccaacctt tgattatcca cgggtgcatg 420

attgctctct actcggtggt cggcaaattt aattacccac aggtgtgttg actcaaagcc 480

tctgtcatta aatctatgct gaataaatgc cgtcaggcca gctagtcaag gtgcacaact 540

ctttttgtgc gtggtgtgg 559

<210> 493
<211> 287
<212> DNA
<213> Homo sapiens

<400> 493
gtaagtctca gtcctttaaa actcagaaaa aggtgtgttt tccaaattta atatttcctt 60

tctgtaagtc tcagtgtctg cactatttgt cttggagact taaaattatc ccttgaaagc 120

ataagaagta caccccaaac cagctttgtc cttcctgtcc tcttctagtt tacattttat 180

gtggttagta attttgtacc taaaagtatt tgaaattcta taaatttgga cttgacgtga 240

gcaaaagaaa atttctacgt aagcgaaact aataaaacta cagtcac 287

<210> 494
<211> 476
<212> DNA
<213> Homo sapiens

<400> 494
ctgtggcatc tataacctga gttcagtcac ttaataccga ggtcctgcgc tctgctgtgt 60

gcctggccct gggctgggca ctggggacat agcagtgacc gagacagaca ggctcacaag 120

gagacatacg acaaccaggt aaacatggca gacaagagca tgtcagatgc gctgtgaaga 180

acactgcggg gcccctccta ggaggtggca tgagttacat gcagacagag acgatccggg 240

ggcagacgga gttccatgtg gggcagtggt gagggcagac gctctggggc tgggatccct 300

gggagtgttc gagaagcacc gagaaggctt ctgtggctgg agccggccag ctgggggaga 360

tggggccagg gagatggcag gggcctctcc ctgtcccagg acccagagcc aagggaggct 420

ttaagcccag gaccaggggt ctgaaaacga aaagcactca cagtccttga acattg 476

<210> 495

<211>  542
<212>  DNA
<213>  Homo sapiens

<400>  495
ggcaacctcc tggacaagga cgacctggcc atcccacccc ccgattacgg cgccgcctcc      60

cgggccttcc ccgcccagac ggccagcggc ttcaagcaga ggccctacag tgtggccgtg     120

cccgccttct cccagggcct ggatgactat ggagcgcggt ccatgagcag tggcagcggc     180

acgctggtgt ccacagtgtg aggacgctga ccccgggcag ccgctgctct gaagagcttc     240

cgcgccttcc ccctggtctc gtccgttttc ctcctcagct ctcgctggtt tgttcttggg     300

ttgtttttct tttccacctg ccccatgcct tttggttggt gaccccagac tctgtgatcc     360

cccagggtcc atggtgctgc tccatccgcc ccccctcccc tgtgtttacg cgccccatcc     420

tgtgtgtccc agccttttga gcagaaactg ccaggcagga cctgctgggc cgtgcggggc     480

accctcggcc tcaccctgca gtgtctgtgg cactcactgc ttttctaagg ctcgccgtga     540

gc                                                                    542

<210>  496
<211>  438
<212>  DNA
<213>  Homo sapiens

<400>  496
gagaggtatt atcgagacat tgcaaagatg gcatccatca gcgaccagga catggatgcc      60

tacctggtgg agcagtcccg cctccacgcc agcgacttca gcgtcctgag tgcgctcaac     120

gagctgtatt tctatgtcac caagtaccgc caggagattc tcacggctct ggaccgagat     180

gcctcttgtc ggaagcataa gttgcggcag aaactggaac agatcatcag cctcgtgtcc     240

agcgacagct aaggtggtgg aatcggtgag gagggggctt ctcagtcctg tgccgtcctc     300

ccatccaggg gagtggctgg ctcaagcctg ggtccccggg ctgagccctg gattgggtat     360

cgtggggcag gtcaccctgg ccacgatgcc cccggcacac ccaggccccc ttcattagtg     420

ccttgctttg ggccctgc                                                   438

<210>  497
<211>  419
<212>  DNA
<213>  Homo sapiens

<220>
<221>  misc_feature
<222>  (248)..(251)
<223>  n is a, c, g, t or u

<400>  497
taagttctca tccaacattt ctcctggcca tccattctcc atctttaaag gcaatcacca      60

ttgccagttt cttctgtatc cttctggaaa tacaatatat tacataaatg acagcattct     120

atattctctc ttctatatct tacctatttc tgtgaataat ttattttgga cagcatttta     180

tgtatgaata ttcacaaatg tgcttcctta tttcagaggc tgaactaata aaaattttgt     240

ttattttnnn nttgaggcaa tatttttata tggtacccta atctttaata cttaacctgc 300

cagactttaa ccgtaacaca ataatgtatt gccaaatagc accattcttc ttctctcact 360

ctcttgccat gggggctctt aaaaaaaaaa gtatacatct aaggtgtaca acatgctgt 419

<210> 498
<211> 477
<212> DNA
<213> Homo sapiens

<400> 498
accagtttac ctaggccttg gactgccaaa tagctacaca actgcttaag ctggcctata 60

aggacagacc agagacaaag caagaagatc attggtccag actgagaaga aagttgccag 120

agggatgtct ccactaaggc ctttgagcag ggattaatgc tgtcaccacc ttggtggaga 180

acaagaaagc tcagctggtg gtgactgcac gtgacaatgg atctcataga gctagctgtc 240

ttcctgcctg ccctgcatca taaaatacaa agggaagaga agactgggat gtctagtcca 300

caggaagact tgcaccactg tcgccttcac acagattaac ttggcagaca aaggagcttt 360

ggctaagctg gtggaagcca tcagaaccaa tgacaatgac agacaggatg agatccactg 420

tcactaggga ggcaatatcc tgggtccaaa atctctggct ctcattgcca agctgga 477

<210> 499
<211> 366
<212> DNA
<213> Homo sapiens

<400> 499
tgagggaggg atgtgcctct ggccacgtgg ttaccttgca gtgcacagcc tgtggtcata 60

gaaggggcta cagctcacgc atcgtgggtg gaaacatgtc cttgctctcg cagtggccct 120

ggcaggccag ccttcagttc cagggctacc acctgtgcgg gggctctgtc atcacgcccc 180

tgtggatcat cactgctgca cactgtgttt atgacttgta cctccccaag tcatggacca 240

tccaggtggg tctagtttcc ctgttggaca atccagcccc atcccacttg gtggagaaga 300

ttgtctacca cagcaagtac aagccaaaga ggctgggcaa tgacatcgcc cttatgaagc 360

tggccg 366

<210> 500
<211> 537
<212> DNA
<213> Homo sapiens

<220>
<221> misc_feature
<222> (193)..(193)
<223> n is a, c, g, t or u

<220>
<221> misc_feature
<222> (210)..(210)
<223> n is a, c, g, t or u

<220>
<221> misc_feature
<222> (379)..(379)
<223> n is a, c, g, t or u

<220>
<221> misc_feature
<222> (443)..(444)
<223> n is a, c, g, t or u

<220>
<221> misc_feature
<222> (446)..(446)
<223> n is a, c, g, t or u

<220>
<221> misc_feature
<222> (448)..(451)
<223> n is a, c, g, t or u

<400> 500
gaacaatcgt cttttgaact tccagtaggc ccacagttgt tggttgttcc tcaaaacagg 60

ttgtggctcc tgttgaataa gatgatccat taaaaactga acaaggttga ggagaaatag 120

tgcttacgtt gaaaaatctt taagtctttg tccccgttct ctaacttcct tacgttttcg 180

tttatttagc tcnatcccca ctatctactn gaatttctca tatttaaacc aagatgggag 240

actaggtcat taggaaaata ttaccgtcta caattttctt atactttgat ctgtctttta 300

tttgattgta agttgctgat ggacagtgat cattagaaac tgaattttgt ataatactag 360

ttttatatga aactagatnt ttattgcgct caggttatgt tccttttacc tccttcctta 420

ataaagagac cacttgaaat aannanannn nttccaagta ctgtctgcac cttatcccac 480

ctctttccca tttatgagat agtgcaaaac cctagcacag tcttttccat ttagtaa 537

<210> 501
<211> 332
<212> DNA
<213> Homo sapiens

<400> 501
aagtatctcc atacaaaata cggttgaatt acaaaaagaa aattgtaaca ttagcatgga 60

caaacctggc aggtactcct taactctcct aagtaataaa aactgtaaaa tgcaaataag 120

ccttcgatga catttactaa cctttactaa agtatcaatg atgacttggt tgtttaaaca 180

gctgacattt gggcaatttg agtatgtcaa actcaataat actggttttc atttgcaaga 240

tccacttaaa acttaaggag gccaaaaaac atcatttaaa ataccctata aattataatc 300

atacatatga tacgaaaaat atcctacttc ag 332

<210> 502
<211> 375
<212> DNA
<213> Homo sapiens

<400> 502
agggtaactt ccagtgtcac aatgagcagt tctgtaagtg ggtgcctctc agcacatttc 60

tatgaatata ttatgtagat aggctgtatt gattttggta gcattgacac cttcttaggc 120

aattagttga agaaaactgc aaaatatttt cttatgtaat agctgtatag agcaatagca 180

atcaaagcat gagaaggcac taacgctggg atgaaagatg agattcagag gtgactgaga 240

atcatgtgag tgatggctgt atattttgtg taaaatatat gtgtgaaaat gaactaagag 300

tgagttactc agcactctca agaattatgc agattctgca tttttcttat gccgtgtgcc 360

taaaaaccta cttga 375

<210> 503
<211> 468
<212> DNA
<213> Homo sapiens

<220>
<221> misc_feature
<222> (30)..(30)
<223> n is a, c, g, t or u

<400> 503
gggacaggat gaccttcccg aggaactcan tggcctgggg tagtttaaga agtaatgttc 60

tttctttctt tctcttttcc ctacctcctg ctaacccaac cagagatccc cttccttgct 120

gagagggttg ggggcaggag gagatttggc agtgcctgca ggttgcctgg ccaggtggag 180

agggggaaag aggaagggca ccgtgggtgt aagatgcctt tctcctccac ccatcgaaac 240

cagccacccc ttccctgtgc caccaagaca gccttttcca gtggccatcc taaggggaac 300

tcccaaatgg gtgttgctgg tggacacaga tgctcccccc aatggaagcc ccaagctctg 360

aggtatgcgg gtagaggctt tggataggtt ttcttctgct cccctctttt atagatctag 420

gctgcttggc tgcctgtctt tctaggcagt ccccctagag gaaaaatg 468

<210> 504
<211> 484
<212> DNA
<213> Homo sapiens

<400> 504
accccaccac gtaccagatg gatgtgaacc ccgagggcaa atacagcttt ggtgccacct 60

gcgtgaagaa gtgtccccgt aattatgtgg tgacagatca cggctcgtgc gtccgagcct 120

gtggggccga cagctatgag atggaggaag acggcgtccg caagtgtaag aagtgcgaag 180

ggccttgccg caaagtgtgt aacggaatag gtattggtga atttaaagac tcactctcca 240

taaatgctac gaatattaaa cacttcaaaa actgcacctc catcagtggc gatctccaca 300

tcctgccggt ggcatttagg ggtgactcct tcacacatac tcctcctctg gatccacagg 360

aactggatat tctgaaaacc gtaaaggaaa tcacaggttt gagctgaatt atcacatgaa 420

tataaatggg aaatcagtgt tttagagaga gaacttttcg acatatttcc tgttcccttg 480

gaat 484

<210> 505
<211> 277

<212>  DNA
<213>  Homo sapiens


<220>
<221>  misc_feature
<222>  (136)..(136)
<223>  n is a, c, g, t or u

<400>  505
ctgcacagtc tccagtgtgg aaagctgtgg gaaaggaagg agcaggttct aggtcttcag      60

gattttctgc atcttaaagc agctcatctc ctttgccctc ctagggagca gggggggcta     120

gctttgggat cgtccnccta gcctcagaaa taattgttca agaaataaca tttctcacac     180

aaaggataaa tgtttgaggg gatggatacc ccatcttcca tgatttgatt attacacatt     240

gcatgcctgt atcaaaaatc tcatatatac acctact                              277


<210>  506
<211>  515
<212>  DNA
<213>  Homo sapiens


<220>
<221>  misc_feature
<222>  (380)..(380)
<223>  n is a, c, g, t or u

<400>  506
gggggtgatt agtatgttgg gacaaacacg ctgttgctaa atggaaacac tgacctcaca      60

gtgcatcctc ctgccaacac acacacacac acacctctca cacatgcacg cttacacaca     120

cacacacaca cacacacaca cacacacata cacacacaca cacacacgct ctctctctct     180

ctctctctct ctctctgtca gtgtgttatc ggtgtggagc ggaggccgcg gaggctcctc     240

ggtccttcag cacccctcgg cccgacgcac ccacgcccct caccccccga gagccgaacg     300

ctccccgcac cgcccccggt cccttccctc ggccgggagc gacttctgca gctcgttctt     360

ccgaatcgca ccagcaatgn cggccagccg tagagggagg aagagcccgg ggagcccgag     420

catagcgtaa acggctctct gaccttaatt tcatcctgca tggcgaatct ctgccgtctc     480

tctgaacgca gaagggtctg agactggccg tctcc                                515


<210>  507
<211>  259
<212>  DNA
<213>  Homo sapiens

<400>  507
ttcagtttat actcaaagcc ctgcagtttc ctgacagcac agagcacacc tgtcacgcga      60

gcaggatgaa gcccagaggc tgcctggtga agtgggcggc gcgctggaaa atccacgtag     120

ctttgttccc tccacgggga gcgtgcaagg ccctctcgag cactacggga gcctcgcctt     180

ctgcacagac ttcggagcca ggtgctggag cggcagcaac tgaggggcgt ggatgtcttt     240

gcatggttcc catacgttt                                                  259

<210> 508
<211> 285
<212> DNA
<213> Homo sapiens


<220>
<221> misc_feature
<222> (189)..(189)
<223> n is a, c, g, t or u

<400> 508
atagcagtgg actgtcactc atcagtatct gcagttctgt ttaccaaagc ctgcttgcta 60

gagacgtttc agggcctcct tccctcaaag cgtccactgt acctccatct ggatacaatt 120

agctggctcc ccacttcctg gactgacggt aaccaccttt tccaatgacc ctgaagaaaa 180

catgcaatnt aagctgcttt aagagtaacc tacaactgag gacaaatttt ctatcaactc 240

ccagtacccc tctctgccgt ggctgatttg ttactggttt tcctt 285


<210> 509
<211> 274
<212> DNA
<213> Homo sapiens

<400> 509
gaggtgcatg ggatcaatgg gacccaatgg ggccagactc tgaggatggg atggtagtag 60

tgaaggacat aggatggggg tagagtgtgg agactttttg aaatagtata gatgaatgcc 120

ctgaggggac tgtgaacaag ctctgcccct cttaggaaat caatggggaa tcaactaaat 180

taaataaaaa atggggtcaa gattaagagg cagggtcacc cagggaatgg tttaggtcct 240

ggcaactctg aaggggttgg aagggctggc agga 274


<210> 510
<211> 470
<212> DNA
<213> Homo sapiens

<400> 510
gcgtgggttt ttgtatccag agctgtttgg atacagctgc tttgagctac aggacaaagg 60

ctgacagact cactgggaag ctcccacccc actcagggga ccccactccc ctcacacacc 120

cccccccaca aggaaccctc aggccaccct ccacgaggtg tgactaacta tgcaataatc 180

caccccaggt gcagccccag ggcctgcgga ggcggtggca gactagagtt tagatgcccc 240

gagcccaggc agctatttca gcctcctgtt tggtggggtg gcacctgttt cccgggcaat 300

ttaacaatgt ctgaaaaggg actgtgagta atggctgtca cttgtcgggg gcccaagtgg 360

ggtgctctgg tctgaccgat gtgtctccca gaactattct gggggcccga caggtgggcc 420

tgggaggaaa atgtttacat ttttaaaggc acactggtat ttatatttca 470


<210> 511
<211> 193
<212> DNA
<213> Homo sapiens

<400>  511
gaaaatgaat tccatgttct tgaaggaaag actgtaacta tgtacattca tgatgttcct      60

ttggtgtgtg gtttctgtga gtaacaggta gatgtcattt ctggaaatgg tatgtttatg     120

tctatacatt gttttataaa actccatgga gaaagaaggg gtttacttgc tttgtatcac     180

atagcaataa cat                                                        193

<210>  512
<211>  452
<212>  DNA
<213>  Homo sapiens

<400>  512
ctggcccacc caggaacagt gagggcgacg agaactacat ggagttcctc gaggtgctga      60

ccgagggcct tgagcgggtg ctgttggtgc gcggtggtgg ccgtgaagtc atcaccatct     120

actcctgagc ccagtgtcat cttgtggcct ggagtcgagg tcttggccag gacataacaa     180

gctgtggtct ggggtaacag cctcttccca gcacccacct gccagccctg cttgcctggc     240

cctgtcctgg acccagcttt gctaggtctc cttggaaacc aggcctgggc ctcaaaatgg     300

agatggatcc caggtcttgt gggaccctgg gatgtttggg gactttacta tctagcaccc     360

cagtaggcct gtcctggcca gagaagactg gtaggggccg agtggggttt gaaggcagcc     420

ggcccggccc agcccaggag cgctatttat tg                                   452

<210>  513
<211>  411
<212>  DNA
<213>  Homo sapiens

<400>  513
ttggaggcct ttgcagcggc ctacaaaggc acgcggccgt ttgccagtgc caacagcgtg      60

ctggacccca tcctcttcta cttcacccag aagaagttcc gccggcgacc acatgagctc     120

ctacagaaac tcacagccaa atggcagagg cagggtcgct gagtcctcca ggtcctgggc     180

agccttcata tttgccattg tgtccggggc accaggagcc ccaccaaccc caaaccatgc     240

ggagaattag agttcagctc agctgggcat ggagttaaga tccctcacag gacccagaag     300

ctcaccaaaa actatttctt cagccccttc tctggcccag accctgtggg catggagatg     360

gacagacctg ggcctggctc ttgagaggtc ccagtcagcc atggagagct g              411

<210>  514
<211>  423
<212>  DNA
<213>  Homo sapiens

<220>
<221>  misc_feature
<222>  (110)..(111)
<223>  n is a, c, g, t or u

<400>  514
tcgtttctct gaacacacaa cacccatcgt cctcttttat gttacttgaa atatcaaaag      60

aattattaca gctgaaaaca aatctatgta aatcggatct tgaaagagan naagctttct 120

ccagttttga aaggcgccat ttttaacttt gatcttgtaa tgacaaataa gaatgttgaa 180

tcggctggct tttttctatc ctaggtaatg tggactgtgg agctctgtgc tggtcacttt 240

caaccctgaa cctgatgcta cttattttgc agttctaagt gcaaagtcgg cctggtggat 300

gcttcccatt ataatattaa atttgcttct tcgtgaggtc acacctcaca tccccagtgt 360

cactttaata actagtgttt tttacatggt gggccatgac ccattagtgg actctgcatt 420

taa 423

<210> 515
<211> 230
<212> DNA
<213> Homo sapiens

<400> 515
ccctggcaag gcccgggaca ggaaggccta cacggtcctc ctatacggaa acggtccagg 60

ctatgtgctc aaggacggcg cccggccgga tgttaccgag agcgagagcg ggagccccga 120

gtatcggcag cagtcagcag tgcccctgga cgaagagacc cacgcaggcg aggacgtggc 180

ggtgttcgcg cgcggcccgc aggcgcacct ggttcacggc gtgcaggagc 230

<210> 516
<211> 426
<212> DNA
<213> Homo sapiens

<400> 516
atgaccttcg aatgcatagg cctttaatgg tgcagacaga ggaccagtat gttttcctca 60

atcagtgtgt tttggatatt gtcagatccc agaaagactc aaaagtagat cttatctacc 120

agaacacaac tgcaatgaca atctatgaaa accttgcgcc cgtgaccaca tttggaaaga 180

ccaatggtta catcgcctaa ttccaaagga ataacctttc tggagtgaac cagaccgtcg 240

cacccacagc gaaggcacat gcccgatgtc gacatgtttt atatgctaat atcttaattc 300

tttgttctgt tttgtgagaa ctaattttga gggcatgaag ctgcatatca tagatgacaa 360

attggggctg tcgggggctg tggatgggtg gggagcaaat catctgcatt cctgatgacc 420

aatggg 426

<210> 517
<211> 448
<212> DNA
<213> Homo sapiens

<400> 517
gagcaagttg taaattgtct cttatcggac ttaaaagggt gcctggctct tacttagttg 60

attatctcct ggatctggaa agaaaggaag gaaaacaaag gcggaagggg aatctctata 120

gaatgtggat ttttcccaca agagactttg cagggcaatt tcaaggtatg gcacggaaat 180

atattttggg gttaaatatt tttttccttg tctcataatg ttatgccaga gtcagattga 240

aaagtaaatc acaacatata gggtcaaata aaacccatct gatgagaatg tgtggtttgt 300

agggcatgac ttcctagacc tcttaggtag gaatctgggt aagacagaat atcagactta 360

gtcctcaatt cctaatgcaa agttctgaga tccaaaatgc tccaaaatct aaaacatttt 420

ttagcaccga cataatgcca caagtgga 448

<210> 518
<211> 148
<212> DNA
<213> Homo sapiens

<400> 518
aattaacacc aggaacagca ccttgaatat tcctttttca agttcctctt cctcaggaga 60

tattcaaggt cgaaacacaa gccccaatgt ttctgtacag aaatccaatc ccatgaggat 120

tactgagagt catgccacca agggccac 148

<210> 519
<211> 173
<212> DNA
<213> Homo sapiens

<220>
<221> misc_feature
<222> (141)..(141)
<223> n is a, c, g, t or u

<400> 519
gaaaatcaca actctaacca taatcatctg cactatatgc ctcgcatcag gtaatgtgtc 60

taaaataata agtaacattt agcatttctg accttatccc aaagtatttt aatagtatct 120

gttaatgttt taattaatgg nttttgtatt gcatctcctg gataacaaag tag 173

<210> 520
<211> 441
<212> DNA
<213> Homo sapiens

<220>
<221> misc_feature
<222> (26)..(26)
<223> n is a, c, g, t or u

<220>
<221> misc_feature
<222> (28)..(28)
<223> n is a, c, g, t or u

<220>
<221> misc_feature
<222> (54)..(54)
<223> n is a, c, g, t or u

<400> 520
catgagtgtg agctgatttg cacccnanca ccctctgtaa gtgcctgctg tggntttggt 60

tttgattatt ccgttaatgc tgagtctgtt tcacaaacga gattagcaga attaattatt 120

gaagatgcag tatgctttat ggttttaata acactgttaa aaactaaaca aggaagttaa 180

atatgttgat gattatcggt gactgctcac cacacagcat ccctcaggcc gagtcagttg 240

gcccagtgac tcccacatca caaactgccc tttcttggtc agaagaagca gagtggagcc 300

ttctcatccc cacgcgcgca gctgtggggc cccgtggtca cctggccaca tgggagtttg 360

catactgagt ggttcatctt ttccaatgtg ttgtgtcctt taatttacat ttatatttca 420

ttgccctttc taatgatcag a 441

<210> 521
<211> 488
<212> DNA
<213> Homo sapiens

<220>
<221> misc_feature
<222> (456)..(456)
<223> n is a, c, g, t or u

<400> 521
tttgagttct gctctggcca atccccaagc tccacgctgt cagccacccc gctctcctac 60

ctcccagagg agcaggctac actcctgttc cttttagaga gagaaatatt gcggccgggc 120

gcggtggctc acgtctgtaa tcccagcatt ttggcaggcc aagggttttg ccatgttcgt 180

ggggctggtc tcaaactaat tacctcagat gatccgccca cctcggcctc ccaaagtgct 240

gggattacag ccgtcctggg ccgccggaca cccccgctgg ggccgatgcc caacagtgac 300

atcgacttga gcaacctgga gcggctggag aagtaccgga gcttcgaccg ctaccggcgc 360

cgggcagagc aggaggcgca ggccccgcac tggtggcgga cctaccgaga gtatttcggg 420

gagaagacag agttccagct tctaaaatat ttgctnctaa aatcttgacc acctgacttt 480

ccggattg 488

<210> 522
<211> 339
<212> DNA
<213> Homo sapiens

<220>
<221> misc_feature
<222> (117)..(119)
<223> n is a, c, g, t or u

<220>
<221> misc_feature
<222> (228)..(228)
<223> n is a, c, g, t or u

<400> 522
aaaatggatc ctgtctttct tagccaagga ctggtctctt ttctccaatg tgtccctaac 60

agagtggtga ggctggctct tcccaccagt acaggaagat cattccttaa aagaaannnc 120

catatggctt ataagtgttc tttcctgtat gaagcccaag ctgtccactt ggagagacat 180

ctggccagcc ccccgttgtt ccagccatcc ccagttcagg catcaganat gtggtgaaga 240

agccatccta gatgcccagc cccagctacc atctgatgca accacactgc tcaccccgag 300

caagaactgc ctgcaggagc ctagtattat cctctctca 339

<210> 523
<211> 396
<212> DNA
<213> Homo sapiens

<400> 523
gcggcagcaa ccggaaccgg aactcgtcgc ggccaccacc actgagcgct gcggggaggg 60

ggagcaagga ccggacgaga cgctacgcct gaaaacaggc ggcgggcgag ggacgaggct 120

taccacggca ccacgcgagt ggaaagggtc gtctccgcta gcggcggccc acaccagctc 180

accgaggggc ggcagcgcgc ggcccggctg ccggaccgta ccatcccggg cggtggagcc 240

gccgcggagg ggcgcgcgcg agccgaaggc gcacccggga ggcccaggta gcccgggggc 300

cggtgctggg gcgccgggca ggcccggctc ccgcctcgac ccacccggag ccagccccct 360

ctgcggacac gacatcccca tggggacggt ggcgcg 396

<210> 524
<211> 194
<212> DNA
<213> Homo sapiens

<400> 524
ccccacaggt gttcctctgt gagctggtcg ggcggccggg gccggggccg ggcttcgctg 60

ctccgtgcct tccacctccc tggcggtgcg gggcctcagg gtgggcctgg gaagctggaa 120

acacctttgg aaacagccgc ctgaggcagc tgtggacaga agaccctgcc cagcagccaa 180

gggagctggc ctct 194

<210> 525
<211> 526
<212> DNA
<213> Homo sapiens

<220>
<221> misc_feature
<222> (424)..(430)
<223> n is a, c, g, t or u

<220>
<221> misc_feature
<222> (443)..(443)
<223> n is a, c, g, t or u

<220>
<221> misc_feature
<222> (445)..(445)
<223> n is a, c, g, t or u

<400> 525
caagggcacg aggcagtacc tttgctccat gcctttgctt ggactagtcc taccaccagc 60

aattcctgca tttctgtgtt tggcaagttt ctgctcagcc tccaaagcct taaccaagtg 120

tcaccttttc tctgcagcat tttctgccac cctccccatt tcttccaata gaaccaggga 180

tcttttactt gggatccaga agcactgtgg acatattgcc atcacaacac ctttcatgtc 240

acaatggcaa ggtttgcact gtcttggagg agaggaagga agccatattc atccctgaac 300

cctcatctcc cagcactggt tgtaaaactg aaacaaaaat ggaaaacctt gatgaaattc 360

attgttggtg tggctatggg gaaacagatt ttccatttct gatagtaaat gaaataggca 420

ccannnnnnn aaaaaaaaaa aananattat taacactgaa aatgcacaca tctttcaacc 480

cagcaatttt atttcttgct ttctagagga atgtttgccc atgtgc 526

<210> 526
<211> 197
<212> DNA
<213> Homo sapiens

<400> 526
cattattaat tataccaatc ctttcatata tgtagaaaaa atgtttgagt tggtcatctg 60

tcttttattg aagatgcatt tcaaatatca aatatatttg aaagataaaa tagcatctgt 120

gaaattgaat attattttat gtgcgcttgg ctatgcccta aaatgtcagt ttattgtccc 180

taaagacgta tttattg 197

<210> 527
<211> 275
<212> DNA
<213> Homo sapiens

<400> 527
ggatgaacgg gtgggctgaa gaacagctga atccaatagc ttggcagaac atgaagacag 60

gtttgttttc cagattctta aaactccaaa cttgatatta ttacagacac aaagtaaatg 120

gcacataaca agaggaagga gatcacagtt tgcaaaactt ttatgtggac cttggtactg 180

ggatcttgag atcctttgcc atggaggtgc atcttcttga gatgtttaca cagagaacag 240

actaacagca gaaaagatat cagggttaca gtaaa 275

<210> 528
<211> 496
<212> DNA
<213> Homo sapiens

<220>
<221> misc_feature
<222> (43)..(43)
<223> n is a, c, g, t or u

<220>
<221> misc_feature
<222> (46)..(46)
<223> n is a, c, g, t or u

<220>
<221> misc_feature
<222> (48)..(48)
<223> n is a, c, g, t or u

<220>

<221>  misc_feature
<222>  (51)..(51)
<223>  n is a, c, g, t or u

<400>  528
aataaatcct gcgagttcac gcccgcgtag ttcgccccct ganttntnga ngcgactcct      60

ttcgcatggg atctacaaaa ccgaactgcc ttaaagacct ctttcacacg gacgtgaagt     120

cacagaactg acaaaatccc atcctgtcaa agtgcacggg tctttgaaat ctaacacaaa     180

aagccataga aagattctct aaacaccctg tactaagagg aacacggaca gggcactgcg     240

ttctgaagta gaggccaggg cactggccct tagacacgtc tcgctgtcac cgggctaaca     300

acattggcaa gggcggcggc agcagcactg atatttgcag cccccaaggg ctctggcgaa     360

accccctcta ttactctgta tcctgcctgc ttccaagatg aacctgttgc tgggaaagaa     420

caggctaaat tagaaaaggg agtattttgt caaagttgaa ggtgagtgat agcctgcccg     480

cctcaaatag gatggg                                                     496

<210>  529
<211>  524
<212>  DNA
<213>  Homo sapiens

<400>  529
agcgcagtgg cgaggcgagt gtggaaggac tcctgaacca gctcgtcctg gagcacctgc      60

agctggcgcc tctgcagtgg gatgtgctgg tggacggaca gccatgtgac cgcgaggctg     120

tggcggcctg ccaggtgggc gaccccgtgc gcctggaggt gcggctgacc aaccggagcc     180

cgcgcagcgt agggcccttc gccctcactg tggtcccctt ccaggaccac cagaacggcg     240

tgcacaacta cgacctgcac gacaccgtct ccttcgtggg ctccagcacc ttctacctcg     300

acgcggtgca gccgtccggc cagtcggcct gcctcggggc cctcctcttc ctctacacgg     360

gagacttctt cctccacatc cggttccacg aggacagcac cagcaaggag ctgccaccct     420

cttggttctg cctgcccagt gtgcacgtgt gtgccctgga ggcgcaggcc tgagcccgcc     480

tacttccgtc cctctttctg cagggccaga ggtgaccctg cctg                      524

<210>  530
<211>  497
<212>  DNA
<213>  Homo sapiens

<400>  530
aggtcaatct cgtattctct atgtgatatt gctgacaaag tcaaagtaag gaaagacata      60

tcaagggaag gcaatggaag caccttttct ttatagtaca ttcacctacc ttaacagacc     120

aagataacat aggagagaaa ctggggctta agtccttgat agagcttctg ggggcacagt     180

agttataggg ccaggtcaga aaatgtcctc acacactaag aaggcatttt aaaatcagaa     240

aagacagtca cactcacttt ggtcaccaag tcatttagcc atcctgtctg gaaagcatgt     300

tttcctctgg ggtcttcctc tggggtatct tgggaaaggg tagagttttg aggagctaga     360

gaagagaaag aggtcatgag ggagattagt cctttctgaa tagcctagga aacccctcac     420

caaatagatg cctacacttt cttaaatcga gaagtaagaa ggaaatcaaa aacagcactc 480

ctacttcaaa gcatcag 497

<210> 531
<211> 253
<212> DNA
<213> Homo sapiens

<400> 531
gtgaaaagca accaaaggca acagagtcta gctcatggcc accagaccaa aagcatccag 60

cttctgtgca cctcctgcaa agctggcaga ggccctggaa ttccagatca cctgagggga 120

aagggttgtc tctctccttt ctgttggggg agggggatgg gggacttttg ttggtggctc 180

ccacccatat atccctcctt taccatagta ctcccaccca cttccatcac ccatccaata 240

aaatgcagcc agg 253

<210> 532
<211> 567
<212> DNA
<213> Homo sapiens

<400> 532
cacctcggtc accagtgtga accaagccag cacatcccgc ctggagggcc tacagtcaga 60

aaaccatcgc ctgcgaatga agatcacaga gctggataaa gacttggaag aggtcaccat 120

gcagctgcag gacacaccag aaaagaccac ctacattaaa cagaaccact accaagagct 180

caatgacatc ctcaacctgg gaaacttcac tgagagcaca gatggaggaa aggccatttt 240

aaaaaatcac ctcgatcaaa atccccagct acagtggaac acaacagagc cctctcgaac 300

atgcaaagat cctatagaag atataaactc tccagaacac atccagcgtc ggctgtccct 360

ccagctcccc atcctccacc acgcctacct cccatccatc ggaggcgtgg acgccagctg 420

tgtcagcccc tgcgtcagcc ccaccgccag cccccgccac agacatgtgc caccctcctt 480

ccgagtcatg gtctcgggcc tgtaagggtg gggggcctgg gcccggggcc tcccccgtga 540

cagaaccaca ctgggcagag gggtctg 567

<210> 533
<211> 402
<212> DNA
<213> Homo sapiens

<400> 533
cagtattctg taccatagcg ctgctcttat gccatttgtt tatttttata tagcttgaaa 60

catagaggga gagagggaga gagcctatac cccttactta gcatgcacaa agtgtattca 120

cgtgcagcag caacacaatg ttattcgttt tgtctacgtt tagtttccgt ttccaggtgt 180

ttatagtggt gttttaaaga gaatgtagac ctgtgagaaa acgttttgtt tgaaaaagca 240

gacagaagtc actcaattgt ttttgttgtg gtctgagcca aagagaatgc cattctcttg 300

ggtgggtaag actaaatctg taagctcttt gaaacaactt tctcttgtaa acgtttcagt 360

aataaaacat ctttccagtc cttggtcagt ttggttgtgt aa 402

<210> 534
<211> 279
<212> DNA
<213> Homo sapiens

<220>
<221> misc_feature
<222> (178)..(178)
<223> n is a, c, g, t or u

<400> 534
tgcattgtac ctgtagccat tccattgtga ataacacaaa aagtggagga aatatttttc 60

tcgcatttgg aaattattct gtgattcagc aaagaagttg ttcatgtcat taacaagttc 120

agaaatacat gctgccaaag ccaaaaagag tcttcagttt aataaaaata attaacanga 180

aggtgagaaa tggtttacca gctgttcact tactggattt aaggttactt gttggggaaa 240

gagcagagta agatgcaact ctgtcaaatc atggctgaa 279

<210> 535
<211> 354
<212> DNA
<213> Homo sapiens

<400> 535
tagcaaagga catggaagcc tggaaagatg taaccagtgg aaatgctaaa atttaccagc 60

ttccaggggg tcacttttat cttctggatc ctgcgaacga gaaattaatc aagaactaca 120

taatcaagtg tctagaagta tcatcgatat ccaattttta gatattttcc ctttcacttt 180

taaaataatc aaagtaatat catactcttc tcagttattc agatatagct cagttttatt 240

cagattggaa attacacatt ttctactgtc agggagattc gttacataaa tatatttacg 300

tatctgggga caaaggtcaa gccagtaaag aatacttctg gcagcacttt ggga 354

<210> 536
<211> 497
<212> DNA
<213> Homo sapiens

<220>
<221> misc_feature
<222> (302)..(302)
<223> n is a, c, g, t or u

<220>
<221> misc_feature
<222> (304)..(306)
<223> n is a, c, g, t or u

<220>
<221> misc_feature
<222> (308)..(309)
<223> n is a, c, g, t or u

<220>
<221> misc_feature

<222>  (311)..(313)
<223>  n is a, c, g, t or u

<220>
<221>  misc_feature
<222>  (315)..(317)
<223>  n is a, c, g, t or u

<220>
<221>  misc_feature
<222>  (354)..(354)
<223>  n is a, c, g, t or u

<220>
<221>  misc_feature
<222>  (361)..(361)
<223>  n is a, c, g, t or u

<400>  536
ttccctgatg actcacttac aagttagtga actccttgtt taagtattac aaactgcaca      60

ccttctccct tctcaatcta gcttcacatc aggccttcct gccaaagcgg caaacttgcc     120

acatggggca aggtactccc caagcagaca aggcccatct gtgtcatgag tgatacccaa     180

tgctaatgcc atgctctgaa atgtagtgcc caccttggct tcccaaagtg ctgggattgc     240

agacgtgagt cactgcgccc agccattcca tgtctcttaa gtctcagaat ctcccctagc     300

tncnnncnng nnncnnnagt ggttgtcccc tcaaagctgt cccacaccct cctncgagga     360

ncctttgtgt atctcctcca gctaccgcag agcccacaaa cccaggcatc tatcaaagtc     420

cctcattcat gagggtggtg aggacacaga ctgcgaccag aacagaaata tgaaaatgtg     480

aatgacagcg tcccccg                                                    497


<210>  537
<211>  340
<212>  DNA
<213>  Homo sapiens


<220>
<221>  misc_feature
<222>  (68)..(68)
<223>  n is a, c, g, t or u

<220>
<221>  misc_feature
<222>  (81)..(81)
<223>  n is a, c, g, t or u

<220>
<221>  misc_feature
<222>  (115)..(115)
<223>  n is a, c, g, t or u

<220>
<221>  misc_feature
<222>  (118)..(118)
<223>  n is a, c, g, t or u

<220>
<221>  misc_feature
<222>  (120)..(120)
<223>  n is a, c, g, t or u

<220>
<221> misc_feature
<222> (122)..(122)
<223> n is a, c, g, t or u

<400> 537
tggagttagc aaaccttttc atgcctgtga cctcactgga gttctttgat gttgggactt 60

cataagtntg ccaaatcctg ncacttactg ctttatgacc ttgaccattt accgnttntn 120

tntggacctc agtgttctca ggatgcaaaa ggagggtcag gggtaaaata gcgactttcg 180

aactgtcagg ggtaaaatag cgactttcaa acttttcaaa cttctgggac aagggtgaag 240

ggcaggactc tgcctctctc cttcccttca ccttattcca cttaaattgt gtgattctac 300

aagcttatgt ttaaaggaat atgttcctcc attacaaaga 340

<210> 538
<211> 527
<212> DNA
<213> Homo sapiens

<220>
<221> misc_feature
<222> (133)..(133)
<223> n is a, c, g, t or u

<220>
<221> misc_feature
<222> (148)..(148)
<223> n is a, c, g, t or u

<400> 538
tgggaccacg ggcatttttg gcatgtacaa gggtataggt gcctcctact tccgcctcgg 60

cccccacacc atcctctccc tcttcttctg ggaccagctg cgctccctct actacacaga 120

cactaaataa canccgcttt cccagtcntc caccaaatga gcactccttg gccacttgtg 180

cctccaccac tatgtcctgg tgactactga ttaggtgacc tttcatccat ccatggggga 240

cagccaaccc cactccccat ctgttctcag ggttgaatca ctacaagaga tgagtttccc 300

ttctttcctt gggtgttgct ttaaaccttc cctacccatt ccctgggtaa ctcacacccc 360

tctctcaggg ctgaacgagt catcccaaag tgtatttcct cccactcacc actgccaccc 420

ttgagtccct cctgctccca tgcacagttt taaactcctc cctccaaaac caaagggaat 480

tgagagaccc aattcccagg cgtctgggac ccaggtgtcc tgttaga 527

<210> 539
<211> 532
<212> DNA
<213> Homo sapiens

<400> 539
gacatgtttt ctagccttag ttccccatct acaaaatggg cctcatggaa tggaatgtct 60

ccacttcact ccagcatcaa caagtgggga attctgatgg attcaattcg acttctttcc 120

atgggcgtgt tctaagcagc ctctttgttc cagaagctgc cctcagccag agttggataa 180

gccaatcctc actccccagc ctcctctgga tagggatgaa gaccccactg gggttggaag 240

tgcagaggca gacaggtgta tggagtcacc tgtaaattga ttcaagtgag ccaggaaagc 300

agcaaaggaa agagaaacct gagtgacgac gtggtggagg aacagggctg gaaagaggct 360

gctggctgtc tggcttcgca gctctggcct cctaatcagc ctcgctcttg tctctggtgt 420

tctctggctc ttgtccatct gtctgtgttt ctttttgcca gctattgact aatctttgct 480

gaagctgagc tagaattctg gtgtttataa gcaggtaact agctgagcac ta 532

<210> 540
<211> 811
<212> DNA
<213> Homo sapiens

<400> 540
ctttgggagg ctgaggcagg cagatcacct aaggccagga attcgacacc agcctggcca 60

acgtggcaaa acccgtctct actaaaaata caaaaattag ccgggcgtgg tggtgtgcgc 120

ctggaatccc agctacccag gaggctgagg caggagaaat gctggaaccc gggaggcaga 180

ggctgcagtg agctgagatc atgccactac tgcactccag cctgggtgac acagcaagac 240

tccctctaaa aaaagaaaaa aagaaaagaa aagaaaagaa aatgatatat ccatgatgaa 300

ttaaaatgga gtggaaccca ctgatgggaa agccacagaa ggtaccagtt atccactcac 360

tgacttaggt gcctccacta gaattctcag cacgtttttg cagaacctgg gcaacaagag 420

cgaaacccca tctcaaaacc acaacaacaa caacaggaca acagagatgg acgacggatc 480

gggaaagcca accagacagc gtgaggccag gacggaaaga ggcacaggga gctctgctca 540

gtgtcgctac aggggatctc tcaggctcac aacgggccac tcctctaggg aagttctggt 600

ctcatcatga tccttgtttg gtctcactcc ccatgtcctt ctctgtccct cctccaactg 660

ccatttattt atttaactga aaaagtacca atcacccaca taggcatgac atactcatcc 720

atgtacccat ttcttaaaat tgatcattgt taacatttgg tgtaatttgc tttatttatt 780

tttaatgaaa taaataaaac tttacagaaa a 811

<210> 541
<211> 3874
<212> DNA
<213> Homo sapiens

<400> 541
aaaaaggtac aactaccttg ctgatgctgt acatatggct cacttgtgcc cagagagaga 60

ataaagccat gtcgaaacta tctacgattc cttgagtgtt tttccagcta cctgccactt 120

gcccacccac tcccctcaga tctcagttag aacatgacaa ttgggctcat gaacaggatc 180

ctgagtggtt gcaggtgaac aagcagttgg cacaagggca aagtgatcac atcctgattg 240

agtggctatg gacagccata cagactgtgt ggaacaacgc tggtgaaata cccaaaccat 300

ttagaagcag taatgcctca cttgcctggg actgggatgg tgtggctgag actgccttac 360

tggcagccag gtgcgctatt cagcagccac aagccctaca agtaattaac caggggcacc 420

tgtttgagct ggaggtgcat gtggccacag acggttttgg ttgaggcttg tggcaatgca 480
cagagcgcct aagaatgcca gtaggctttt ggtcccaact atggaaagga gctgaactcc 540
ggtattcatt gatagagaaa cagctagcag ctgtatatgc tggccttcgg gctcatgaga 600
gcatgacagg acaggctgca gtcatcatat ggacaactta cccaataaca ggatggatgc 660
gtctatgtgt aatgaccacc tggagtggga tagcacagat gtccactttg gcaaaatggg 720
gcgactcctt gcagcagtgg agtaagctga gtacaagtcc catagcagca gagttgcaag 780
aggtcttggg acgtgtagtc ctaatgcaag ataaggccat gcggcctgag gcacccctag 840
atcctgagtc ttcaccattt aaggaagggc atcccaggat tcctgagggg gcatggtaca 900
cagtagatga ggtgctactg ctgcctggac cactgttgca gtccaaccta gtactgacac 960
catatggttt gaaactgggt gcggacaaag tagccaatgg gctgaactca gagcagtgtg 1020
gatggtaatc accaaggagg tgacacctgt ggtaatctgt actgatagct gggcagtcta 1080
ctgaggctta accttgtggt taactacttg gaaaatacag aattggctag tgagccacag 1140
acccatttga ggccaagcca tatggcaaga cctttgggga ataggtcatc aaaaagaggt 1200
aactatttat catgtgtcag gccatatgcc tttggccacc cctagtaatg atgaggcaga 1260
tgccttggct aaggtcagat ggtcagagtc agcaccaaca caagatgtga ccttgtggct 1320
acaccggaaa ctgggacatg cagggggtaa actgatgtaa caattcaata agtgttgggg 1380
tctgtccctt cccaagcaag acatttgtga ggcttgtcag aaatgcctgg catgtgttca 1440
gacatatcct aaaaagaggc agctgcccgg tgttatacaa caagtaacaa tagggtgagt 1500
gcccttgacc aggtgggaag tagactacat cgggccgccg ccaaagtcgc gagggtatac 1560
gcatgcacta acggctgtag acatggccac aggcctgttg ttcacctacc cttgcagggt 1620
ggccaaccaa cagaacacca tccaggccct gcaacactta tgttccctgt atggttgtcc 1680
tctggccatt gagagtgata ggggaacaca tttcactgga caacaggtac aatgatgggc 1740
acagcaaatg gacataaagt ggggattcca tgtgccatac agcccacaag ctgaggtatt 1800
attgaatgat ataatgggat cttgaagaat ggattacgct tgcatgtcaa acccctgtct 1860
ttgcggagct ggagttccag gctggacctg gtgctccaaa ccttaaatga atggccacag 1920
aaaggtggcc cggccccagt ggaggctttg tttcactagg ccaccacccc cattcaattg 1980
gagatacata ccaaggatga cctcctccga tcaggtatgg ggacaaatgg taacctgttg 2040
ttgcctgccc caacaaccct gaaggcaggg gaacagaaaa cctggctgtg gccatggacc 2100
ctccaagctc tccactgctg gtggttggcc atcatagctc cctgtgggga gggcctacag 2160
tatgacttgc atgtcacttt ttgagtgttc aatacatggc ttccaaggtt gactgtttgt 2220
agaggaacag ccagggaagg aaccctcctc tgagggacat atgtactatc tgatgggcct 2280
attatgagct acgctgtgac tttggcatgg atacaggatt ctaaggaacc atggagattt 2340
gagaaggtgt ggtaccatca cccagggcaa aagcccttgg tggctgcatt gttatccagg 2400
gatggaaagt tagcctatat tttgcctgag ggatgtgatt tacctctgtt agtacctgtg 2460

cctgctctgt catttcaacc gtaggttaac atgctccaat tgcattgtgg actgacccca 2520
cacctatgct gaggtgacca atgtttccaa ctgttggacc tgcactgcct ttccagcagc 2580
agctgcagac agcttgcccc gacacataca tcccgtgtct gcagagaact ggacatgcct 2640
ggagacttga gatcccatgg ctgatgcctg gaacacaatg tggcaagctt tggacaaagg 2700
acacagcaag acccatggct ggaccgtagc attcgtgatg agtggggctg gctagtaggg 2760
gaacatgtag tagcccaagc ccaggcattg cagtgcacag agcaacattg gggtaacagg 2820
atgggtacct gtcacggcct gtgcaaacat aacatgtgtc accacactga aggtatggta 2880
gaacaagtgg cctcaccaag gtcggacccc aatggacttt ttgcctcttg ggagcttatg 2940
ggtctatgag gacacagtag cctttcctat cagcaaactg gagtggatgt tgtatctggg 3000
ggtggcctta tgtacctgct actgttctcc ccacattgcc cagatgcctg tataactggg 3060
aggcactgtg ctctcagttt ttgcgaatgt gatgagcccc ctggtgtttc taccctttgg 3120
caatgactat ccctggagca ggtgtcaaaa ctgtagaagc acaatttact gctcttgcgg 3180
agcacaccgc tcaggctctg aattacacct gagtgtccct cctcctgtta atgaatgagg 3240
ttgatcagat caaaaagtgg tgttgcaaaa ccaagtggcc ttagacataa ctgctgccca 3300
aggagccacc tgtgcccttt taggaacaca atgttgtacc ttatccctga caatcagcag 3360
aacataacag cagccctgca aaggggtctt ccaggagatt aaggtgactg agagcctcac 3420
tgtcaacccc ctgcagagat ggtgagcatc cctaggttct ggcgtacatt gggccctaat 3480
agtcataagt atcatagctg agatcctagt agtgagctgt tgctctctgt attgttgttg 3540
tgggttatgg actcagggct ccgccatata ggcatgtgtc cctgcctgga ggacgccctc 3600
agcctagggg gtgtagtgta agggaaatgg ctgtgcttta gtcaggagta ggctgaggca 3660
gccttctggt gcagcatgac tcagtgggtt tggagtgcaa gcacacaacc ttgctcgtta 3720
tgtaaccaca ccacatgagg cccattaggt aacaactcac atgagctcgt gtttggctca 3780
gagccactat tgtctgtaaa aggtatacct tgctgatgct gcacatatgg ctcgcttgtg 3840
cccagagaga gagtaaagcc atgttgaaac tgtc 3874

<210> 542
<211> 177
<212> PRT
<213> Homo sapiens

<400> 542

<210>  543
<211>  21
<212>  DNA
<213>  artificial

<220>
<223>  Description of artificial sequence: Oligonucleotide

<400>  543
catgaacagg atcctgagtg g                                                21

<210>  544
<211>  24
<212>  DNA
<213>  artificial

<220>
<223>  Description of artificial sequence: Oligonucleotide

<400>  544
tgaggcatta ctgcttctaa atgg                                             24

<210>  545
<211>  2863
<212>  DNA
<213>  Homo sapiens

<400>  545
cacgccaaac acgcagcccc ctcccgctgg agtgacaact ggccagcata ctctaggctg      60

ttgtcccttt aaaacttgaa tccaaggggg taatgattta tcaaacttgt attatcaaga 120
aaatgtcaaa ccaagggcac cttgctttgc actgacgcaa acccggcctt tcccaaggag 180
atatagaaag cgcctctcct gcctgagcca aacccagtct tgtcaatagc gggtttcacc 240
ctccaccagt tcagtctgtt gcctgtgtca gacatggatt gcagtgctcc caaggaaatg 300
aataaactgc cagccaacag cccggaggcg gcggcggcgc agggccaccc ggatggccca 360
tgcgctccca ggacgagccc ggagcaggag cttcccgcgg ctgccgcccc gccgccgcca 420
cgtgtgccca ggtccgcttc caccggcgcc caaactttcc agtcagcgga cgcgcgagcc 480
tgcgaggctg agcggccagg agtggggtct tgcaaactca gtagcccgcg ggcgcaggcg 540
gcctctgcag ctctgcggga cttgagagag gcgcaaggcg cgcaggcctc gccccctccc 600
gggagctccg ggcccggcaa cgcgctgcac tgtaagatcc cttttctgcg aggcccggag 660
ggggatgcga acgtgagtgt gggcaagggc accctggagc ggaacaatac ccctgttgtg 720
ggctgggtga acatgagcca gagcaccgtg gtgctggcca cggatggaat cacgtccgtg 780
ctcccgggca gcgtggccac cgttgccacc caggaggacg agcaagggga tgagaataag 840
gcccgaggga actggtccag caaactggac ttcatcctgt ccatggtggg gtacgcagtg 900
gggctgggca atgtctggag gtttccctac ctggccttcc agaacggggg aggtgctttc 960
ctcatccctt acctgatgat gctggctctg gctggattac ccatcttctt cttggaggtg 1020
tcgctgggcc agtttgccag ccagggacca gtgtctgtgt ggaaggccat cccagctcta 1080
caaggctgtg gcatcgcgat gctgatcatc tctgtcctaa tagccatata ctacaatgtg 1140
attatttgct atacactttt ctacctgttt gcctcctttg tgtctgtact accctggggc 1200
tcctgcaaca acccttggaa tacgccagaa tgcaaagata aaaccaaact tttattagat 1260
tcctgtgtta tcagtgacca tcccaaaata cagatcaaga actcgacttt ctgcatgacc 1320
gcttatccca acgtgacaat ggttaatttc accagccagg ccaataagac atttgtcagt 1380
ggaagtgaag agtacttcaa gtactttgtg ctgaagattt ctgcagggat tgaatatcct 1440
ggcgagatca ggtggccact agctctctgc ctcttcctgg cttgggtcat tgtgtatgca 1500
tcattggcta aaggaatcaa gacttcagga aaagtggtgt acttcacggc cacgttcccg 1560
tatgtcgtac tcgtgatcct cctcatccga ggagtcaccc tgcctggagc tggagctggg 1620
atctggtact tcatcacacc caagtgggag aaactcacgg atgccacggt gtggaaagat 1680
gctgccactc agattttctt ctctttatct gctgcatggg gaggcctgat cactctctct 1740
tcttacaaca aattccacaa caactgctac agggacactc taattgtcac ctgcaccaac 1800
agtgccacaa gcatctttgc cggcttcgtc atcttctccg ttatcggctt catggccaat 1860
gaacgcaaag tcaacattga gaatgtggca gaccaagggc caggcattgc atttgtggtt 1920
tacccggaag ccttaaccag gctgcctctc tctccgttct gggccatcat ctttttcctg 1980
atgctcctca ctcttggact tgacactatg tttgccacca tcgagaccat agtgacctcc 2040
atctcagacg agtttcccaa gtacctacgc acacacaagc cagtgtttac tctgggctgc 2100

tgcatttgtt tcttcatcat gggttttcca atgatcactc agggtggaat ttacatgttt 2160
cagcttgtgg acacctatgc tgcctcctat gcccttgtca tcattgccat ttttgagctc 2220
gtggggatct cttatgtgta tggcttgcaa agattctgtg aagatataga gatgatgatt 2280
ggattccagc ctaacatctt ctggaaagtc tgctgggcat ttgtaacccc aaccatttta 2340
acctttatcc tttgcttcag cttttaccag tgggagccca tgacctatgg ctcttaccgc 2400
tatcctaact ggtccatggt gctcggatgg ctaatgctcg cctgttccgt catctggatc 2460
ccaattatgt ttgtgataaa aatgcatctg gcccctggaa gatttattga gaggctgaag 2520
ttggtgtgct cgccacagcc ggactggggc ccattcttag ctcaacaccg cggggagcgt 2580
tacaagaaca tgatcgaccc cttgggaacc tcttccttgg gactcaaact gccagtgaag 2640
gatttggaac tgggcactca gtgctagtcc agtggtgtgg gatggtccag acttgatcct 2700
gtttttcctc tctgcctcct cctaatgttt tccatagctc tcctcccatt tttcttcatc 2760
tttcttccta catcttggtt cacatccacg catgagagtg attatgtaga aaagtaggca 2820
tagtgtcgca tgctgcagta aagagctaca tagaccacct gaa 2863

<210> 546
<211> 797
<212> PRT
<213> Homo sapiens

<400> 546

<210>  547
<211>  24
<212>  DNA
<213>  artificial

<220>
<223>  Description of artificial sequence: Oligonucleotide

<400>  547
ggatttgcaa gttgtgtagt gtgc                                              24

<210>  548
<211>  21
<212>  DNA
<213>  artificial

<220>
<223>  Description of artificial sequence: Oligonucleotide

<400>  548
aagcagatgg tcatcttcca g                                                 21

<210>  549
<211>  2426
<212>  DNA
<213>  Homo sapiens

<400>  549
ctctttcaac tcaagagctc agtcctgtgt ctctcatgga ggcgtctcta accaggaggc      60

tactctttaa agacaggcat tttacttgca gcaaaataat aggaaggaga ttcgcttgct     120

ttgcacagag gctgagccac aggagaaagc aaagccaatg tgatttattg aatgaaagca     180

ctggacaatt accaacaact tgttcctctg ctgcctcgaa cagcataaac tggaattgtc     240

gtgtgaaaat gacgcaacaa atgcaaaatt tacatctctg tcagtcaaaa aaacatagtg 300
ctccctcatc tcccaacgca gccaaacgcc tgtacaggaa cctctctgag aaactgaaag 360
ggagccactc ttccttcgat gaggcctatt ttaggacaag aactgatcgg ctgagtctca 420
ggaagacctc ggtgaatttc cagggcaatg aagccatgtt tgaggcagtc gaacagcagg 480
acatggatgc tgtgcagatc ctcctgtatc agtacacacc agaagaactt gacctcaaca 540
cacctaacag cgagggcttg acacccctgg atattgccat catgaccaac aatgtgccca 600
ttgcaaggat tcttctgagg acaggggccc gagaaagtcc acactttgtc agcctggaaa 660
gccgagcaat gcacctcaac acactggtcc aggaagccca ggagagggtg agtgaactgt 720
ctgcccaggt ggagaatgaa ggattcactc tggacaacac agagaaagag aagcagctga 780
aagcttggga gtggaggtat cggctctaca gacgcatgaa aacaggcttt gagcatgcca 840
gagcccctga gatgccaacc aatgtctgtc tcatggtaac cagcagcaca tcactcactg 900
tcagcttcca agagcctctt agcgtcaatg cagctgtagt aaccaggtat aaagtggaat 960
ggagtatgtc cgaagacttt tctcctttgg ctggagaaat catcatggat aatctgcaga 1020
ctctgagatg cacaatcaca ggacttacaa tgggccaaca gtattttgtt caagtctcgg 1080
cttacaatat gaaaggatgg ggacctgctc agaccacgac accggcatgt gcctctcctt 1140
ctaactggaa agactatgac gacagagagc ccagacacaa gggacagagt gaagttttgg 1200
aaggtctgct gcagcaggtc cgagcccttc atcagcatta cagttgccgg gaaagcacaa 1260
aattacaaac cacaggccgc aagcagtcag tctcaagaag cctgaaacac ctgttccatt 1320
cctcgaacaa gtttgtgaag accttaaaac ggggactcta catagccgtt atattttatt 1380
acaaagacaa tatcttagtc accaatgaag atcaagtacc aattgttgaa atagatgact 1440
ctcacaccag ttctattaca caagattttc tgtggttcac gaagctgtct tgtatgtggg 1500
aagatataag gtggctgagg caaagcatac caatatcctc atcctcatcc acagtgctgc 1560
aaactcggca gaagatgctc gcagcaacag cacagctaca gaatttactt gggacacaca 1620
acttgggaag agtttactat gagcccatta aagatcgaca tggaaacata ctcatagtca 1680
ccatcaggga ggtggagatg ctttattcat tttttaatgg caaatggatg cagatctcaa 1740
agctgcaaag ccagagaaag tctctatcaa cacctgagga gccaacagct ttagacattc 1800
tactgataac catccaggat attctatcct atcacaaaag gagtcatcag cgtctctttc 1860
ctggattata tctgggttac ctaaagctct gtagctctgt ggatcaaatc aaagttcttg 1920
ttacccaaaa gttgcccaac attctctgcc acgtgaagat ccgtgaaaac aataatattt 1980
ctagagagga atgggaatgg atccaaaagc tttctggctc tgaatctatg gaaagtgtgg 2040
atcatacttc tgactgcccc atgcaattgt tcttctacga gctccagatg gcagtgaaag 2100
ctctccttca gcagatcaat atacctctac accaggcaag gaacttccgc ctctacacac 2160
aggaggtgtt ggaaatgggt cacaatgtgt cctttcttct cctgctccct gcctcagacg 2220
acgtctgtac agccccagga cagaataatc cttacacccc acactcaggg tttcttaacc 2280

tccctcttca gatgtttgaa cttggtatag tagcttgttt cacctagaaa tattaaccca 2340

gcctccttat aataaaatca caaagttata tctgttcccc cttgtcccag tggagggtca 2400

ataaatcaca tgatggcttt ggcaac 2426

<210> 550
<211> 763
<212> PRT
<213> Homo sapiens

<400> 550

Met Glu Ala Ser Leu Thr Arg Arg Leu Leu Phe Lys Asp Arg His Phe
1               5                   10                  15

Thr Cys Ser Lys Ile Ile Gly Arg Arg Phe Ala Cys Phe Ala Gln Arg
            20                  25                  30

Leu Ser His Arg Arg Lys Gln Ser Gln Cys Asp Leu Leu Asn Glu Ser
        35                  40                  45

Thr Gly Gln Leu Pro Thr Thr Cys Ser Ser Ala Ala Ser Asn Ser Ile
    50                  55                  60

Asn Trp Asn Cys Arg Val Lys Met Thr Gln Gln Met Gln Asn Leu His
65                  70                  75                  80

Leu Cys Gln Ser Lys Lys His Ser Ala Pro Ser Ser Pro Asn Ala Ala
                85                  90                  95

Lys Arg Leu Tyr Arg Asn Leu Ser Glu Lys Leu Lys Gly Ser His Ser
            100                 105                 110

Ser Phe Asp Glu Ala Tyr Phe Arg Thr Arg Thr Asp Arg Leu Ser Leu
        115                 120                 125

Arg Lys Thr Ser Val Asn Phe Gln Gly Asn Glu Ala Met Phe Glu Ala
    130                 135                 140

Val Glu Gln Gln Asp Met Asp Ala Val Gln Ile Leu Leu Tyr Gln Tyr
145                 150                 155                 160

Thr Pro Glu Glu Leu Asp Leu Asn Thr Pro Asn Ser Glu Gly Leu Thr
                165                 170                 175

Pro Leu Asp Ile Ala Ile Met Thr Asn Asn Val Pro Ile Ala Arg Ile
            180                 185                 190

Leu Leu Arg Thr Gly Ala Arg Glu Ser Pro His Phe Val Ser Leu Glu
        195                 200                 205

Ser Arg Ala Met His Leu Asn Thr Leu Val Gln Glu Ala Gln Glu Arg
    210                 215                 220

Val Ser Glu Leu Ser Ala Gln Val Glu Asn Glu Gly Phe Thr Leu Asp
225                 230                 235                 240

Asn Thr Glu Lys Glu Lys Gln Leu Lys Ala Trp Glu Trp Arg Tyr Arg
                245                 250                 255

Leu Tyr Arg Arg Met Lys Thr Gly Phe Glu His Ala Arg Ala Pro Glu
            260                 265                 270

Met Pro Thr Asn Val Cys Leu Met Val Thr Ser Ser Thr Ser Leu Thr
        275                 280                 285

Val Ser Phe Gln Glu Pro Leu Ser Val Asn Ala Ala Val Val Thr Arg
    290                 295                 300

Tyr Lys Val Glu Trp Ser Met Ser Glu Asp Phe Ser Pro Leu Ala Gly
305                 310                 315                 320

Glu Ile Ile Met Asp Asn Leu Gln Thr Leu Arg Cys Thr Ile Thr Gly
                325                 330                 335

Leu Thr Met Gly Gln Gln Tyr Phe Val Gln Val Ser Ala Tyr Asn Met
            340                 345                 350

Lys Gly Trp Gly Pro Ala Gln Thr Thr Thr Pro Ala Cys Ala Ser Pro
        355                 360                 365

Ser Asn Trp Lys Asp Tyr Asp Asp Arg Glu Pro Arg His Lys Gly Gln
    370                 375                 380

Ser Glu Val Leu Glu Gly Leu Leu Gln Gln Val Arg Ala Leu His Gln
385                 390                 395                 400

His Tyr Ser Cys Arg Glu Ser Thr Lys Leu Gln Thr Thr Gly Arg Lys
                405                 410                 415

Gln Ser Val Ser Arg Ser Leu Lys His Leu Phe His Ser Ser Asn Lys
            420                 425                 430

Phe Val Lys Thr Leu Lys Arg Gly Leu Tyr Ile Ala Val Ile Phe Tyr
        435                 440                 445

Tyr Lys Asp Asn Ile Leu Val Thr Asn Glu Asp Gln Val Pro Ile Val
    450                 455                 460

Glu Ile Asp Asp Ser His Thr Ser Ser Ile Thr Gln Asp Phe Leu Trp
465                 470                 475                 480

Phe Thr Lys Leu Ser Cys Met Trp Glu Asp Ile Arg Trp Leu Arg Gln
                485                 490                 495

Ser Ile Pro Ile Ser Ser Ser Ser Ser Thr Val Leu Gln Thr Arg Gln
            500                 505                 510

Lys Met Leu Ala Ala Thr Ala Gln Leu Gln Asn Leu Leu Gly Thr His
        515                 520                 525

Asn Leu Gly Arg Val Tyr Tyr Glu Pro Ile Lys Asp Arg His Gly Asn
    530                 535                 540

Ile Leu Ile Val Thr Ile Arg Glu Val Glu Met Leu Tyr Ser Phe Phe
545                 550                 555                 560

Asn Gly Lys Trp Met Gln Ile Ser Lys Leu Gln Ser Gln Arg Lys Ser
                565                 570                 575

Leu Ser Thr Pro Glu Glu Pro Thr Ala Leu Asp Ile Leu Leu Ile Thr
            580                 585                 590

Ile Gln Asp Ile Leu Ser Tyr His Lys Arg Ser His Gln Arg Leu Phe
        595                 600                 605

Pro Gly Leu Tyr Leu Gly Tyr Leu Lys Leu Cys Ser Ser Val Asp Gln
    610                 615                 620

Ile Lys Val Leu Val Thr Gln Lys Leu Pro Asn Ile Leu Cys His Val
625                 630                 635                 640

Lys Ile Arg Glu Asn Asn Asn Ile Ser Arg Glu Glu Trp Glu Trp Ile
                645                 650                 655

Gln Lys Leu Ser Gly Ser Glu Ser Met Glu Ser Val Asp His Thr Ser
            660                 665                 670

Asp Cys Pro Met Gln Leu Phe Phe Tyr Glu Leu Gln Met Ala Val Lys
        675                 680                 685

Ala Leu Leu Gln Gln Ile Asn Ile Pro Leu His Gln Ala Arg Asn Phe
    690                 695                 700

Arg Leu Tyr Thr Gln Glu Val Leu Glu Met Gly His Asn Val Ser Phe
705                 710                 715                 720

Leu Leu Leu Leu Pro Ala Ser Asp Asp Val Cys Thr Ala Pro Gly Gln
                725                 730                 735

Asn Asn Pro Tyr Thr Pro His Ser Gly Phe Leu Asn Leu Pro Leu Gln
            740                 745                 750

Met Phe Glu Leu Gly Ile Val Ala Cys Phe Thr
        755                 760

<210>  551
<211>  21
<212>  DNA
<213>  artificial

<220>
<223>  Description of artificial sequence: Oligonucleotide

<400>  551
agctctgtag ctctgtggat c                                                21


<210>  552
<211>  21
<212>  DNA
<213>  artificial

<220>
<223>  Description of artificial sequence: Oligonucleotide

<400>  552
aggcggaagt tccttgcctg g                                                21


<210>  553
<211>  2281
<212>  DNA
<213>  Homo sapiens

<400>  553
gcgggccgca gccagcgcac ccagaccctg cgctgccctc ggacggccgg gcgcggagcc      60

ccagctgcgg aggccgacgg cacccggccc cgagcgcctc gacgccgagc cgcgcgcgcc     120

ttctccgcca ggcccggcgg gcgggagcgg gggcgaggga gcaggagcgg ccagtgcccc     180

cgacaccccc ggcccggcac ccccggcccg gcatcccccg ccgccgccgc cgccgcctca     240

aggccgcccg ctccccgcag gtggacgcgg ccatgggccg aggggtgcgc gtgctgctgc     300

tgctgagcct gctgcactgc gccgggggca gcgagggcag gaagacctgg cggcgccggg     360

gtcagcagcc gcctcctccc ccgcggaccg aggcggcgcc ggcggccgga cagcccgtgg     420

agagcttccc gctggacttc acggccgtgg agggtaacat ggacagcttc atggcgcaag     480

tcaagagcct ggcgcagtcc ctgtacccct gctccgcgca gcagctcaac gaggacctgc     540

gcctgcacct cctactcaac acctcggtga cctgcaacga cggcagcccc gccggctact     600

acctgaagga gtccaggggc agccggcggt ggctcctctt cctggaaggc ggctggtact     660

gcttcaaccg cgagaactgc gactccagat acgacaccat gcggcgcctc atgagctccc     720

gggactggcc gcgcactcgc acaggcacag ggatcctgtc ctcacagccg gaggagaacc     780

cctactggtg gaacgcaaac atggtcttca tcccctactg ctccagtgat gtttggagcg     840

gggcttcatc caagtctgag aagaacgagt acgccttcat gggcgccctc atcatccagg     900

aggtggtgcg ggagcttctg ggcagagggc tgagcggggc caaggtgctg ctgctggccg     960

ggagcagcgc gggggggacc ggggtgctcc tgaatgtgga ccgtgtggct gagcagctgg    1020

agaagctggg ctacccagcc atccaggtgc gaggcctggc tgactccggc tggttcctgg    1080

acaacaagca gtatcgccac acagactgcg tcgacacgat cacgtgcgcg cccacggagg 1140

ccatccgccg tggcatcagg tactggaacg gggtggtccc ggagcgctgc cgacgccagt 1200

tccaggaggg cgaggagtgg aactgcttct ttggctacaa ggtctacccg accctgcgct 1260

gccctgtgtt cgtggtgcag tggctgtttg acgaggcaca gctgacggtg gacaacgtgc 1320

acctgacggg gcagccggtg caggagggcc tgcggctgta catccagaac ctcggccgcg 1380

agctgcgcca cacactcaag gacgtgccgg ccagctttgc ccccgcctgc ctctcccatg 1440

agatcatcat ccggagccac tggacggatg tccaggtgaa ggggacgtcg ctgccccgag 1500

cactgcactg ctgggacagg agcctccatg acagccacaa ggccagcaag acccccctca 1560

agggctgccc cgtccacctg gtggacagct gcccctggcc ccactgcaac ccctcatgcc 1620

ccaccgtccg agaccagttc acggggcaag agatgaacgt ggcccagttc ctcatgcaca 1680

tgggcttcga catgcagacg gtggcccagc cgcagggact ggagcccagt gagctgctgg 1740

ggatgctgag caacggaagc taggcagact gtctggagga ggagccggca ctgaggggcc 1800

cagacacccg ctgccccagt gccacctcac cccccaccag caggccctcc cgtctcttcg 1860

ggacagggcc ccagccgtcc cccctgtctg ggtctgccca ctgccctcct gccccggctt 1920

tccctgcccc tctcccacag cccagccaga gacaagggac ctgctgtcat ccccatctgt 1980

ggcctggggg tccttcctga caacgagggg gtagccagaa gagaagcact ggattcctca 2040

gtccaccagc tcagacagca cccaccggcc ccacccatca agccctttta tattatttta 2100

taaagtgact tttttattac tttaattttt taaaaaaagg aaaataagaa tatatgatga 2160

atgatattgt tttgtaactt tttaaaaatg attttaaaga gacaaaaaag aacctcaaaa 2220

aaaaaaaaaa aaaaaaaaaa aaaaaaaaaa aaaaaaaaaa aaaaaaaaaa aaaaaaaaaa 2280

a 2281

<210> 554
<211> 496
<212> PRT
<213> Homo sapiens

<400> 554

Met Gly Arg Gly Val Arg Val Leu Leu Leu Leu Ser Leu Leu His Cys
1               5                   10                  15

Ala Gly Gly Ser Glu Gly Arg Lys Thr Trp Arg Arg Arg Gly Gln Gln
            20                  25                  30

Pro Pro Pro Pro Pro Arg Thr Glu Ala Ala Pro Ala Ala Gly Gln Pro
        35                  40                  45

Val Glu Ser Phe Pro Leu Asp Phe Thr Ala Val Glu Gly Asn Met Asp
    50                  55                  60

Ser Phe Met Ala Gln Val Lys Ser Leu Ala Gln Ser Leu Tyr Pro Cys
65                  70                  75                  80

Ser Ala Gln Gln Leu Asn Glu Asp Leu Arg Leu His Leu Leu Leu Asn
                85                  90                  95

Thr Ser Val Thr Cys Asn Asp Gly Ser Pro Ala Gly Tyr Tyr Leu Lys
            100                 105                 110

Glu Ser Arg Gly Ser Arg Arg Trp Leu Leu Phe Leu Glu Gly Gly Trp
        115                 120                 125

Tyr Cys Phe Asn Arg Glu Asn Cys Asp Ser Arg Tyr Asp Thr Met Arg
    130                 135                 140

Arg Leu Met Ser Ser Arg Asp Trp Pro Arg Thr Arg Thr Gly Thr Gly
145                 150                 155                 160

Ile Leu Ser Ser Gln Pro Glu Glu Asn Pro Tyr Trp Trp Asn Ala Asn
                165                 170                 175

Met Val Phe Ile Pro Tyr Cys Ser Ser Asp Val Trp Ser Gly Ala Ser
            180                 185                 190

Ser Lys Ser Glu Lys Asn Glu Tyr Ala Phe Met Gly Ala Leu Ile Ile
        195                 200                 205

Gln Glu Val Val Arg Glu Leu Leu Gly Arg Gly Leu Ser Gly Ala Lys
    210                 215                 220

Val Leu Leu Leu Ala Gly Ser Ser Ala Gly Gly Thr Gly Val Leu Leu
225                 230                 235                 240

Asn Val Asp Arg Val Ala Glu Gln Leu Glu Lys Leu Gly Tyr Pro Ala
                245                 250                 255

Ile Gln Val Arg Gly Leu Ala Asp Ser Gly Trp Phe Leu Asp Asn Lys
            260                 265                 270

Gln Tyr Arg His Thr Asp Cys Val Asp Thr Ile Thr Cys Ala Pro Thr
        275                 280                 285

Glu Ala Ile Arg Arg Gly Ile Arg Tyr Trp Asn Gly Val Val Pro Glu
    290                 295                 300

Arg Cys Arg Arg Gln Phe Gln Glu Gly Glu Glu Trp Asn Cys Phe Phe
305                 310                 315                 320

Gly Tyr Lys Val Tyr Pro Thr Leu Arg Cys Pro Val Phe Val Val Gln
                325                 330                 335

Trp Leu Phe Asp Glu Ala Gln Leu Thr Val Asp Asn Val His Leu Thr
            340                 345                 350

Gly Gln Pro Val Gln Glu Gly Leu Arg Leu Tyr Ile Gln Asn Leu Gly
        355                 360                 365

Arg Glu Leu Arg His Thr Leu Lys Asp Val Pro Ala Ser Phe Ala Pro
    370                 375                 380

Ala Cys Leu Ser His Glu Ile Ile Ile Arg Ser His Trp Thr Asp Val
385                 390                 395                 400

Gln Val Lys Gly Thr Ser Leu Pro Arg Ala Leu His Cys Trp Asp Arg
                405                 410                 415

Ser Leu His Asp Ser His Lys Ala Ser Lys Thr Pro Leu Lys Gly Cys
            420                 425                 430

Pro Val His Leu Val Asp Ser Cys Pro Trp Pro His Cys Asn Pro Ser
        435                 440                 445

Cys Pro Thr Val Arg Asp Gln Phe Thr Gly Gln Glu Met Asn Val Ala
    450                 455                 460

Gln Phe Leu Met His Met Gly Phe Asp Met Gln Thr Val Ala Gln Pro
465                 470                 475                 480

Gln Gly Leu Glu Pro Ser Glu Leu Leu Gly Met Leu Ser Asn Gly Ser
                485                 490                 495

<210>  555
<211>  21
<212>  DNA
<213>  artificial

<220>
<223>  Description of artificial sequence: Oligonucleotide

<400>  555
gagatcatca tccggagcca c                                                21

<210>  556
<211>  21
<212>  DNA
<213>  artificial

<220>
<223>  Description of artificial sequence: Oligonucleotide

<400>  556
tagcttccgt tgctcagcat c                                                21

<210>  557
<211>  522
<212>  DNA
<213>  Homo sapiens

<400>  557

atgtaagcaa agtagtcatt aaaaatacac cctctacttg ggctttatac tgcatacaaa 60

tttactcatg agccttcctt tgaggaagga tgtggatctc caaataaaga tttagtgttt 120

attttgagct ctgcatctta acaagatgat ctgaacacct ctcctttgta tcaataaata 180

gccctgttat tctgaagtga gaggaccaag tatagtaaaa tgctgacatc taaaactaaa 240

taaatagaaa acaccaggcc agaactatag tcatactcac acaaagggag aaatttaaac 300

tcgaaccaag caaaaggctt cacggaaata gcatggaaaa acaatgcttc cagtggccac 360

ttcctaagga ggaacaaccc cgtctgatct cagaattggc accacgtgag cttgctaagt 420

gataatatct gtttctacta cggatttagg caacaggacc tgtacattgt cacattgcat 480

tatttttctt caagcgttaa taaaagtttt aaataaatgg ca 522

<210> 558
<211> 21
<212> DNA
<213> artificial

<220>
<223> Description of artificial sequence: Oligonucleotide

<400> 558
actacggatt taggcaacag g 21

<210> 559
<211> 24
<212> DNA
<213> artificial

<220>
<223> Description of artificial sequence: Oligonucleotide

<400> 559
gagatctcga gatctcgatc gtac 24

<210> 560
<211> 2383
<212> DNA
<213> Homo sapiens

<400> 560
cttttctctt gttgagtgca aatggagaac agctgctcac gctcgtcgtc tgacatcagc 60

tatttctcag gatgaccctg cgagacaggc cagggtcatt agacccaatt tggttctcag 120

caaatatgtg tttattcctg catgcgtggg ccacaggctg gtttcttggg tgcaatgaat 180

agctgcaggt ttattagggt gtctttttag atggatgtat gtttcccgat gtctatagaa 240

cactccggac cccggagagt gaagactctg cctgtcggac ttgctttgag aagatccttc 300

tccacctccc catggcagaa gttgcttcac agaggggaac agttttatgg atgtggctga 360

gaccttaaac ttgaggcaac ccatctgagg tggcatccag aggagactgg ctggcccctc 420

cttcaccttg gatgtagtgc tgtttctagg atctcttttc aatcagcaaa acaggggatg 480

ttccaagagg gtgtggattc cctgccatcc cacatggtca agtggagggg acgggaaaaa 540

gctatgaagg gtttgtgacc acacagactc tcctggcccc ctgtcctttt ggaaagaaga 600

cagggatgaa atataatcaa gcaattaacc acccccatca tcaccaagaa caacagtatc 660
aacaagaaga acagggacaa caaaacccac ggatgaaaca ttcctttctc agctcagatc 720
ttatctggtg cgttctctct ctgctctgtc ttggtgtgtg gtttagagaa acatggacaa 780
cgctgtttgg aagaacaggt gagcgagggt ggggaatttc agaggcctgg gcccaccgcc 840
tccacccctt ccccagttta acctttgaca ggatcttcac ctctctctga tcagcattgc 900
ttcttgttca aaggcctcag ccacccagct gtgtcccttt ccccagaaag caagggcaga 960
tggcagtggg tctgttgatg agagaacttt aagggcccaa tcagtccctg ggcaccccct 1020
cctgggctcg ttttctccag gaggctgcat tctgatccat aaaccttctc ctcggggttt 1080
agggtcgagc tgttcctgat gtttatcgga gactgggatc aaagctatcc aggtcataaa 1140
tctctctctg tggctgttgg gccccagggc agctgaagag ggttgacagc cctttggacc 1200
tcaaaggaaa aaatgtgctc tactccaccc actcccagct ctgccaagaa gctgtcctct 1260
gagaagccat ggctgggccg ttccattctg gggagctgct gaaaagagct gggaggccga 1320
gaagaacttg cgtgtgctgg gggagaggaa gcctggcctt gagggagggg tgcaggtgtg 1380
gctcctctgt gtgtgggggc tgggggacct tgtgtgcctt ttccttgtgg ctgtgaaatg 1440
ctttatgagt acttccatag gaggatggac agggagtcgg ggagataaac tcagccacaa 1500
ggccccaggg cctcaggaaa cttgcaccca accctctcat tttacagaag aaaactgtgc 1560
ctggaaggtt gaagggtttg ttcccagtca cacaaccagg gatccttagg acagccagac 1620
caggaaacca tttccaaact gccaagccat ggcagagtat caagacctca ggaaccatcg 1680
agacaccatg gaagcattgg gaaaagcctc cttagctttt gaagctcctc attgttcttg 1740
agtgtgcatg gagcccatga ctgcggggtt ttgtagacac ctcagggatt acatgactgg 1800
tacccctgac aaagtcaagg ctgctggaca aaatgagtcc gaggatttca ggggcagctg 1860
ggcgcaggag ctggtgggct gttgggagtg cccctttact gggcaggctt ccttcctcct 1920
ggtgatgggg ggttcctcag cacaaaagtg aaggggtgga ggggctggag gagcaggaat 1980
ctctcttgtt gataggtatg aggccttgaa gtccttttct ttgtcccagg attcatggac 2040
gcttcggggc tgatctttga gttttcaagc atggggtgca gagacgttta ggtaaactct 2100
taccgtcctc tctcttcgtc agggcttccc aggaatcaac aatgcccaag aaggaaggga 2160
ttgtagaaat agcttaaccc tttcatttac caacgtggaa attgaagccc agggaaggga 2220
agggaccggt cgtggaaggg agagccatca gcagaaagag accctgagat cttcgcctgg 2280
gattcccagg aagtccagcc cgagctgatt cacagaacaa atgcatgcaa accttgctat 2340
caataaatta cacatgcact tacgtaaaaa aaaaaaaaaa aaa 2383

<210> 561
<211> 21
<212> DNA
<213> artificial

<220>

<223>  Description of artificial sequence: Oligonucleotide

<400>  561
cagagacgtt taggtaaact c                                                21


<210>  562
<211>  21
<212>  DNA
<213>  artificial

<220>
<223>  Description of artificial sequence: Oligonucleotide

<400>  562
taccaacgtg gaaattgaag c                                                21


<210>  563
<211>  2336
<212>  DNA
<213>  Homo sapiens

<400>  563
aaaaaaacct atctaaggag gagccaagat ggccgcatag gaacagctcc agtccacagc      60

tcccagcgtg agcgacgcag aagacgggtg atttctgcat ttccatctga ggtaccgggt     120

tcttctcact agggagttcc agacagtggg cgcagcaggc ttttcttctt ggagctgaaa     180

caggcgcact ggcgagggtt gtgaggaaag gtttgcagcc ccctgccttg gtctgctata     240

gccagtgaca cgatcaatac cagggtgagc agcagaggaa gctcagggaa gattatgaag     300

atgagaattc attacctatc aaagaaaatg cgtaaactct agaagtattg cttgctcctt     360

tgccacaaag tgtacattca agagtaaatt gtttaaagcc aaagggcctt gcgccacgtc     420

cttagcctca gctttctact tgctaaaatg gaaataacaa cagtacctac cttacagacc     480

tggcgtgagg attaaattat accagcaaag tgcctggcac ctagaatttg cctgagttct     540

gatcaatgct aaaaacacca tttaacagtg ccctttcctg cctgggaagc ccacaaagat     600

ttcgcttttc acttattact catcaaactg actctggttc acagtggaaa agagaagaaa     660

agctgatgga gaaatggcag tgaagaagga gaacaaaatg tcagagcaat acttttgagc     720

gacttatcct ctgttctgca atatttgcaa aagtcagtct atagacgtga agccaacagg     780

gaccctcagg cctgtgcatc aggatggtgg gctgcctaag tcctctgggg acagcagtgc     840

cagaaggagt attggacaca gtgacccgac tgtatgagat gagaaaaaac aaaaacagga     900

ggtccgcagt actgatgaac taatctgtca ctcacaagct caggtctgca aaaaaaagaa     960

acgaagcact aaacatggcc ataagagatg gaaatgcaag tcttcatttc taaatgataa    1020

tcaaaccaac gatcagaaac tgattaactg tgtaattgaa ttgaattgaa aatcatccca    1080

tgaataacaa tccatcctac cttcaagggg ttaggaagct aactacaggt aattgctatc    1140

agaaatctga tttgatttcc aaaaattgtg tgaatgaacc aagtttcttc atcttgatat    1200

actaggcagg gagtttgttc ttccaagtac tagactgctt aattgcttgc ttgggggagg    1260

agaaatccta ggggaaaggc atatatgagc aatttctact ctgtgaagcc agcgctgtgt    1320

cctgagctgg atcatggcca gaaacagaaa agtctactct tccctacagt ggaagcaact 1380

gtggatattt catcctagga gtgaatgaaa aaacctaaag ctcatacttc atgggaatct 1440

ttcaatattc tgactgaaaa ctggttattt gctcctccaa cccaaagcca tctaggaaca 1500

gcactcagaa caggaaaaaa aaaagacaaa aataataatt attccaaaac gtatttgagc 1560

agaaacaaac acaaacattt gcattattaa atgggcttgt tcacacctgc tgagtagata 1620

taagacgata tttaagacaa gagctaaaaa ataaaccatc cctttctggt tttgagtgac 1680

agcagagcaa taaaaattat tttcacattc ttttccctat tgttagaagt aatcatttga 1740

gtaaatacac ttatctgtgc tgtaactatt gaaatgaatc cacttcaaat atgtatacca 1800

cctttctttt ttatatttct agatatggtt tcaatataga ctttctgact tttatggtat 1860

acatatagga caatattcta ttcttctttc cttttaaata cttactgttt caatttcaaa 1920

taaaaaatca gcattctagt ttgtacattt tagcacagaa atgtttacaa ccttcagcac 1980

aattgctttt gtaatttact gacttggcat tttgaggcgt ttttaacaaa ttatgagaaa 2040

taacaccttc agaaagcatg tgactacttt gatgcaacta tttacaatgt attcataaga 2100

agtcattaac ctgtagagtt cttagacatg tggaaccttt aacaattata ctaaagagta 2160

catacaaaat acagagctat gtaataataa ctaattttaa atcctgacaa attagaagtt 2220

aagcctacta tctgtaaaaa tatgtcctga ttcatttttt taagtatata cctgagcctt 2280

taaaaagtat atgcctttac aattgatttc caataaacaa tactgaataa catact 2336

<210> 564
<211> 24
<212> DNA
<213> artificial

<220>
<223> Description of artificial sequence: Oligonucleotide

<400> 564
gcaatacttt tgagcgactt atcc 24

<210> 565
<211> 23
<212> DNA
<213> artificial

<220>
<223> Description of artificial sequence: Oligonucleotide

<400> 565
gatagcaatt acctgtagtt agc 23

<210> 566
<211> 1187
<212> DNA
<213> Homo sapiens

<400> 566
gaacaacatg gcttccccca gcgtgagact cgcttgtcct cccaccgcct gctctctcct 60

gatgaccagg ttccaggagt tatcaaagaa cagcctctga gctgcgtgga gaaagccatg 120

gagacagcca gaagaaaacg gcaggattag attaacctgt gattcctggc tggccacgag 180
gtcacccatg gcatggagct gcccacaacg cccttctcag catgaagcat cctgaaagat 240
ccagggccag gttccccagg attggggagt tggaagctca ttggcactgt caaatttgaa 300
gaagaggcgt gctctgactg cctggacagg acccggaatc aaaccgcagg ccctgggtca 360
ccgctgccgg aaagagccag ttcctgtccg tccatgcacc caccaccaaa acccaggcct 420
tcctggaggt gctaggggag gccatgcccc ttttctgagt gcttggaagt gactgctgca 480
agtgacaagt gaccacgcct tttcccccgc gggtataaat tcagaggcgc tgcgctccga 540
ttctggcagt gcagctgtgg gaacctctcc acgcgcacga actcagccaa cgatttctga 600
tagatttttg ggagtttgac cagagatgca aggggtgaag gagcgcttcc taccgttagg 660
gaactctggg gacagagcgc cccggccgcc tgatggccga ggcagggtgc gacccaggac 720
ccaggacggc gtcgggaacc ataccatggc ccggatcccc aagaccctaa agttcgtcgt 780
cgtcatcgtc gcggtcctgc tgccagtgag tccccgccgc ggtccctggc tggggaagag 840
cgcacctggc gccgggaggg ggcagggaga cggggacacg gcagggatgc ctggccctgg 900
tcacctgcgg ccgggcatgt ccgggcagga cgaactcgcc gtcggagtca ggggaagaac 960
tgggtccccg ggctgggcag gagggacccg gccgcgaggg agcagagagg cggtccccct 1020
ggctgccccg agcccgcgaa gggagggaag ttccagaatc gagagaggga gggagtcaag 1080
gtggaaccca tagagtgagc ctcctgaaga cacagagcgg ttgcctctct cattaattaa 1140
ttaattagtt aataaaatta accccatgtt taaaaaaaaa aaaaaaa 1187

<210> 567
<211> 155
<212> PRT
<213> Homo sapiens

<400> 567

<210>  568
<211>  21
<212>  DNA
<213>  artificial

<220>
<223>  Description of artificial sequence: Oligonucleotide

<400>  568
ggattgggga gttggaagct c                                                21

<210>  569
<211>  21
<212>  DNA
<213>  artificial

<220>
<223>  Description of artificial sequence: Oligonucleotide

<400>  569
agaaatcgtt ggctgagttc g                                                21

<210>  570
<211>  857
<212>  DNA
<213>  Homo sapiens

<400>  570
   catccctctg gctccagagc tcagagccac ccacagccgc agccatgctg tgcctcctgc      60
   tcaccctggg cgtggccctg gtctgtggtg tcccggccat ggacatcccc cagaccaagc     120
   aggacctgga gctcccaaag ttggcaggga cctggcactc catggccatg gcgaccaaca     180
   acatctccct catggcgaca ctgaaggccc ctctgagggt ccacatcacc tcactgttgc     240
   ccacccccga ggacaacctg gagatcgttc tgcacagatg ggagaacaac agctgtgttg     300
   agaagaaggt ccttggagag aagactgaga atccaaagaa gttcaagatc aactatacgg     360
   tggcgaacga ggccacgctg ctcgatactg actacgacaa tttcctgttt ctctgcctac     420
   aggacaccac cacccccatc cagagcatga tgtgccagta cctggccaga gtcctggtgg     480
   aggacgatga gatcatgcag ggattcatca gggctttcag gcccctgccc aggcacctat     540
   ggtacttgct ggacttgaaa cagatggaag agccgtgccg tttctaggtg agctcctgcc     600
   tggtcctgcc tcctggctca cctccgcctc caggaagacc agactcccac ccttccacac     660
   ctccagagca gtgggacttc ctcctgccct ttcaaagaat aaccacagct cagaagacga 720
   tgacgtggtc atctgtgtcg ccatcccctt cctgctgcac acctgcacca cggccatggg 780
   gaggctgctc cctgggggca gagtctctgg cagaggttat taataaaccc ttggagcatg 840
   aaaaaaaaaa aaaaaaa 857

<210> 571
<211> 180
<212> PRT
<213> Homo sapiens

<400> 571

Met Leu Cys Leu Leu Leu Thr Leu Gly Val Ala Leu Val Cys Gly Val
1               5                   10                  15

Pro Ala Met Asp Ile Pro Gln Thr Lys Gln Asp Leu Glu Leu Pro Lys
            20                  25                  30

Leu Ala Gly Thr Trp His Ser Met Ala Met Ala Thr Asn Asn Ile Ser
        35                  40                  45

Leu Met Ala Thr Leu Lys Ala Pro Leu Arg Val His Ile Thr Ser Leu
    50                  55                  60

Leu Pro Thr Pro Glu Asp Asn Leu Glu Ile Val Leu His Arg Trp Glu
65                  70                  75                  80

Asn Asn Ser Cys Val Glu Lys Lys Val Leu Gly Glu Lys Thr Glu Asn
                85                  90                  95

Pro Lys Lys Phe Lys Ile Asn Tyr Thr Val Ala Asn Glu Ala Thr Leu
            100                 105                 110

Leu Asp Thr Asp Tyr Asp Asn Phe Leu Phe Leu Cys Leu Gln Asp Thr
        115                 120                 125

Thr Thr Pro Ile Gln Ser Met Met Cys Gln Tyr Leu Ala Arg Val Leu
    130                 135                 140

Val Glu Asp Asp Glu Ile Met Gln Gly Phe Ile Arg Ala Phe Arg Pro
145                 150                 155                 160

Leu Pro Arg His Leu Trp Tyr Leu Leu Asp Leu Lys Gln Met Glu Glu
                165                 170                 175

Pro Cys Arg Phe
            180

<210> 572
<211> 21
<212> DNA

<213>  artificial

<220>
<223>  Description of artificial sequence: Oligonucleotide

<400>  572
agttcaagat caactatacg g                                                21


<210>  573
<211>  21
<212>  DNA
<213>  artificial

<220>
<223>  Description of artificial sequence: Oligonucleotide

<400>  573
tagaaacggc acggctcttc c                                                21


<210>  574
<211>  4415
<212>  DNA
<213>  Homo sapiens

<400>  574
   agagaagcaa catctttaag gtactgaggg caggagaagt taatgtagaa tactatgcca      60
   gaaaaaataa attcccaaaa gtggaagtga aataaggaca tttagagatg tacaaaagct     120
   gaccgaattc actaccagtc aacccacact acaagaaaca tcaaatgagt cctccaagca     180
   gaaggaaccc aataccagat gaaaatccag atctccacga ggaaatgaag aacaccagaa     240
   atggatcggc cctttcttca aataagagca gttggaataa caaagctgtt cagttgtacc     300
   cttggaatcc actgaaatcc tgggtaggga agctccagta ccaccaactg gaaagactgg     360
   gaatgcctaa tagctggtac tggccattgt cgtaggcttt gtccactctg acaaactgaa     420
   gatggggact cgactcacct tcgccagcca caggaggacc tccagacgag gttaggtcga     480
   cttcccgata actttagatc ctgaaacctc acgggatttt tcttctcttc cctttgatct     540
   ctcttccgct tgctcaacag gacaggactc gctgcctttc tttcccgtca gaaagggatc     600
   ccttgcggac aggacctaag tgagtagctg gtttccccta cttgtccttc cgggcctggg     660
   tgtctcggga gctcaggctg acgggagacc taactaccgg cgagtgagac cagcaggagc     720
   ctggaggggc gcgcaccagg gtggaggttt ggtgccgggg gttgagaaca acagtcaaac     780
   cctcttcttc ccctggcacc acgcacctgc cccccgggac gccgaacgaa gtggtcccta     840
   aagctcctct gcaggcccaa ccgaaacagg cctgaagctc caggatgggc gagaggatcc     900
   tctttgagcg aaaccagcct tctgcctggc tggccctggt caacaccctg ggaagaggcc     960
   gatttggcgg acagaacgga agaaaagacc taaaggtaga atctcatgat gtcgagatgt    1020
   taaaacactc aaattttaag gttcgactgt gagggggaga tagggggtct cgagcaggat    1080
   cgacccctga gccttcatct gcagagtcct gtgcaccagc tcagaggaca ggactatgtg    1140
   caccaatggt tctcatcagg cggcaacttc accctcacat gcctccccca tccctgctgg    1200
   tacacaagac cacgactagg ggaagcccgg agggagaatg ttaacccctg gcatctatct    1260
   agtcagcaga ggtgagggat gctgctaaac accttacaat ccaccggagg acacccgccc 1320
   ccaccgaccc cgaagtagcc attccctgga ggtggggaaa ctcgcctgta gatcaatgcc 1380
   cacgcacttg gcggacagga aatcacgaat tggccactaa ctggatcttg gatctgagga 1440
   aaaaattcca gcgtcagagg gaactctcgg agatttgccc agagcataag gaacgtactc 1500
   cttccctcag tgatggatca tcacatctgg gggaaatcat agacaatttc ttttgtaggg 1560
   cgaactctgc tatacagttt atgatgtcag agtgaatact ttctttgagt tgcagtcaga 1620
   aactgtagat ttttaaaaat ttaaaattca ttattctctg tcagtatttc aaagtgtata 1680
   cagaaagcta ttgcactgtt caggagatgg cgcctaacat tttggaaatt caaggtgatg 1740
   aatgtccaga taagactatc tctcctggta caaagtttga caatgctgaa catttttaaa 1800
   ggttcttttt gatatacaaa gtgcaccaat gagtgctttt taattcttac aataattctg 1860
   ggtgaggtag gtatttttcc aattcccatt ttatgcttcg gtagcccttt gtatttatac 1920
   ttcaaaacac ttggctctct tgtaattatt taagaaatta gttgtgatta tttgtttaat 1980
   gtgcaggagt tacaaaaggc aagcgttaga acaagacaga cctggttatg attcctggct 2040
   ctgaaagctg tacaccctgt gaccctagac aggtgtttta atgcctcgct gcctctgttt 2100
   cttgctctgt aaaatgtgaa caataacagt attggcctca tgcttttttt gggttttaaa 2160
   agtaataatg tggacaaaga tcagtggagt gcctggcatg ctgaacccat tccatgactg 2220
   atagctatag ttgttatgat ttgtatcaat ccattttcac actgctataa ggaactacct 2280
   gagactgggt aatctatgaa gaaaagaggt ttaattgact cacagttctg catggctggg 2340
   agtcctcagg aacttacaat catggcagaa ggggaagcaa gacatttctt atatggcagc 2400
   aggacagaaa gagagagagt gaagggggaa gtgccacaca cttccaaaca accatatctt 2460
   gtggttaatt aaaaagtact cattggtgtg ccttgtatag aaaaaaatat acactcacta 2520
   tcatgagaac agcaaggagg gagtctgccc ccaaggttca atcacctccc agtagacccc 2580
   tcccctgaca tgtggggatt acaattcaag atgagatttg ggtggggaaa cagagtcaaa 2640
   ccatatcgtg attgttctat aataaagaga tgcccacatg tgtttcatca gggacagtgc 2700
   tcattaacca gttgtcctgc cgtaattatt aatagtatcc cctttgcttt caaaagtgtc 2760
   ctagtttaca aaaagtatag aaatggagga cagaatagtg gttgcccaag attggaaaag 2820
   ggtaagggta aagggtgcag aggtggatgt ggttataaaa ggcaacatgg gagatcctcg 2880
   tagtgaagga accgtttagt atctccactg tggtggtaga tacccgaacc taaacatgtg 2940
   aaaaattgca tgaaactaaa cacacacacc aacaagtaca agttaagtta ggaaaatcca 3000
   aataagattt ctacattgta tcaataggta tatcttgatt atgatattgc aagatggtac 3060
   tattcaagga aactgggtag aggctacatg agactcccct gtattatttc ctataactcc 3120
   atgtgaatct acaaggatct caggattaag gaagatatcc tagtttggaa gataaaaaat 3180
   atatcccagt agtaatatcc actgtcccac cagggcctga ctaccttcta taaaaagaag 3240
   tgcctttgtt cccctcaagt tcctttattt ggttttattc ttcttcacag tacctacctc 3300
   cacttggcag attacattta ttttttcatc tttcaacagc tatttactga atgcctacta 3360
   gatgccaggc ttgagatcta gcaatgaaca agatctctgt gaaacttaca ttccaggagg 3420
   agaaataaat aataaaccaa aaatataatc agtaaattat ttaatatgct gggaaacaat 3480
   atgtgtaatg gaagaaatat gtaaagtgat ggattagggt tctccagaga aacagaacca 3540
   acaattgact catgtgatta tggaggctga gaagtctcaa gatcacagtt ggcaagcttg 3600
   agacacagga gagcccatgg tgtgtttctg atttgagtcc aaaggcctga gaaccaggag 3660
   agatgatggt gtgattacag ttcaaaagct ggcaggcttg aggcccagga agagccagtg 3720
   ttgcagttca attccaaagg caggaaaagg ctgatatctt agctgaagca atcaggcaga 3780
   aggagctctc tcttactcat gggcaggtca gacttttggt tctattcagg cctttaagtg 3840
   attggatgag gatcatctac tgtggaaaga aataagcttt attcagtgta ctgattcaaa 3900
   tgttaatctc atccaaaacc atgctcacag acacacccag cataatgttt gaccaagtat 3960
   ctgggcacct tgtggttcag tcaaattaac acatattaac taccttagca agatgaaaag 4020
   cagtgaatgc aggatggtgg ttgaaatttt aaatacgttg gttatatagt ctcattgaaa 4080
   aaggaacatt tgagtgaaga cttgaagggg tggtggaata aaccatttat ttgcttattg 4140
   cctgtctccc tctatcagaa tgaaagcttc atgaagcgag agacttaatt tttatctgtt 4200
   atatccctag tgcctggtgc agggtaagta ctcaaaaata tttgttgagt gaataagtaa 4260
   tgattgagga tggggactgg tttgtatctg gttatatctc ttgtccttag cacagtacct 4320
   ggcacatcct aagccatcca aaagagttgg ttatatgatt gtctttgaat tctatgactg 4380
   tttataatat acagtaaact tcactgaaga cactg 4415

<210> 575
<211> 22
<212> DNA
<213> artificial

<220>
<223> Description of artificial sequence: Oligonucleotide

<400> 575
gaagaacacc agaaatggat cg 22


<210> 576
<211> 21
<212> DNA
<213> artificial

<220>
<223> Description of artificial sequence: Oligonucleotide

<400> 576
cttcagtttg tcagagtgga c 21


<210> 577
<211> 484
<212> DNA
<213> Homo sapiens

<400>  577
tgtcagcttt gtctgtgcct cgcaaatcag aggcaaggga gaggttgtta ccaggggaca      60

ctgagaatgt acatttgatc tgccccagcc acggaagtca gagtaggatg cacagtacaa     120

aggagggggg agtggaggcc tgagagggaa gtttctggag ttcagatact ctctgttggg     180

aacaggacat ctcaacagtc tcaggttcga tcagtgggtc ttttggcact ttgaaccttg     240

accacaggga ccaagaagtg gcaatgagga cacctgcagg aggggctagc ctgactccca     300

gaactttaag actttctccc cactgccttc tgctgcagcc caagcaggga gtgtccccct     360

cccagaagca tatcccagat gagtggtaca ttatataagg atttttttta agttgaaaac     420

aactttcttt tctttttgta tgatggtttt ttaacccagt cattaaaaat gtttataaat     480

caaa                                                                  484


<210>  578
<211>  21
<212>  DNA
<213>  artificial

<220>
<223>  Description of artificial sequence: Oligonucleotide

<400>  578
cagccacgga agtcagagta g                                                21


<210>  579
<211>  24
<212>  DNA
<213>  artificial

<220>
<223>  Description of artificial sequence: Oligonucleotide

<400>  579
ccactcatct gggatatgct tctg                                             24


<210>  580
<211>  592
<212>  DNA
<213>  Homo sapiens


<220>
<221>  misc_feature
<222>  (62)..(62)
<223>  n is a, c, g, t or u

<400>  580
ggtgcatgtt cattgggcat cttccattcg acccctttgc ccacgtggtg accgctgggg      60

anctgtgaga gtgtgagggg cacgttccag ccgtctggac tctttctctc ctactgagac     120

gcagcctata ggtccgcagg ccagtcctcc caggaactga aatagtgaaa tatgagttgg     180

cgaggaagat caacatatag gcctaggcca agaagaagtt tacagcctcc tgagctgatt     240

ggggctatgc ttgaacccac tgatgaagag cctaaagaag agaaaccacc cactaaaagt     300

cggaatccta cacctgatca gaagagagaa gatgatcagg gtgcagctga gattcaagtg     360

cctgacctgg aagccgatct ccaggagcta tgtcagacaa agactgggga tggatgtgaa 420

ggtggtactg atgtcaaggg gaagattcta ccaaaagcag agcactttaa aatgccagaa 480

gcaggtgaag ggaaatcaca ggtttaaagg aagataagct gaaacaacac aaactgtttt 540

tatattagat attttacttt aaaatatctt aataaagttt taagcttttc tc 592

<210> 581
<211> 21
<212> DNA
<213> artificial

<220>
<223> Description of artificial sequence: Oligonucleotide

<400> 581
attggggcta tgcttgaacc c 21

<210> 582
<211> 21
<212> DNA
<213> artificial

<220>
<223> Description of artificial sequence: Oligonucleotide

<400> 582
tttcccttca cctgcttctg g 21

<210> 583
<211> 2514
<212> DNA
<213> Homo sapiens

<400> 583
actggggtct tctccatgcg gctcgggcta tgacagcctc cgtgctcctc cacccccgct 60

ggatcgagcc caccgtcatg tttctctacg acaacggcgg cggcctggtg gccgacgagc 120

tcaacaagaa catggaaggg gcggcggcgg ctgcagcagc ggctgcagcg gcggcggctg 180

ccggggccgg gggcgggggc ttcccccacc cggcggctgc ggcggcaggg ggcaacttct 240

cggtggcggc ggcggccgcg gctgcggcgg cggccgcggc caaccagtgc cgcaacctga 300

tggcgcaccc ggcgcccttg gcgccaggag ccgcgtccgc ctacagcagc gcccccgggg 360

aggcgccccc gtcggctgcc gccgctgctg ccgcggctgc cgctgcagcc gccgccgccg 420

ccgccgcgtc gtcctcggga ggtcccggcc cggcgggccc ggcgggcgca gaggccgcca 480

agcaatgcag cccctgctcg gcagcggcgc agagctcgtc ggggcccgcg gcgctgccct 540

atggctactt cggcagcggc tactacccgt gcgcccgcat gggcccgcac cccaacgcca 600

tcaagtcgtg cgcgcagccc gcctcggccg ccgccgccgc cgccttcgcg gacaagtaca 660

tggataccgc cggcccagct gccgaggagt tcagctcccg cgctaaggag ttcgccttct 720

accaccaggg ctacgcagcc gggccttacc accaccatca gcccatgcct ggctacctgg 780

atatgccagt ggtgccgggc ctcgggggcc ccggcgagtc gcgccacgaa cccttgggtc 840

ttcccatgga aagctaccag ccctgggcgc tgcccaacgg ctggaacggc caaatgtact 900
gccccaaaga gcaggcgcag cctccccacc tctggaagtc cactctgccc gacgtggtct 960
cccatccctc ggatgccagc tcctatagga gggggagaaa gaagcgcgtg ccttatacca 1020
aggtgcaatt aaaagaactt gaacgggaat acgccacgaa taaattcatt actaaggaca 1080
aacggaggcg gatatcagcc acgacgaatc tctctgagcg gcaggtcaca atctggttcc 1140
agaacaggag ggttaaagag aaaaaagtca tcaacaaact gaaaaccact agttaatgga 1200
ttaaaaatag agcaagaagg caacttgaag aaacgcttca gaactcgttg ctttgcccag 1260
ataatgataa taatgcttaa taataattga agaatgggaa agagaaagag acagagactg 1320
gcattttcct ctcccgaagg agatctcttt ctctttaatg gaatctacaa ctgttttaaa 1380
actttaagaa aggtaaagac tgccagttct tccgccaacc ccatcagccc agcccgttaa 1440
atgtcaaacg tcaaccccca aaatacgcaa tttcagataa gttacgcagt tactgaaatc 1500
ttgtaagtat ttaagtgatc gttacatttt aggacactgc gttagatggt aataatctgg 1560
aagttggtta caaacgcaag aggccattgt aaacatctgc ttgtccttct taggtcgcca 1620
ttccctttgc atgttaagcg tctgctcagg taaatcttag tgaaattcct accgttgttg 1680
tacgttctgc aaaacatttt atgtatagat ttagagggga aacgagaagg tactgaaata 1740
atgatcttgg aatatttgct gtgaagggag aaagggagag aaaactcttc tgaggatcat 1800
ttgtcttggt agtatagtaa aaccaaccag ctgaaccttt caggctacaa gagaacccgg 1860
gtcggtaatg tctttttaag aataattttt aattgcttat aacaagcata ttttgtggca 1920
tttgaactat atttactgct ccaatatccg ttattttcca aaggattttg tatctttttg 1980
aaaatgttta catcatcaga tgatccacag aattcacttt atgtgagatc tcccgagagt 2040
ttccatccca acataatgga ctttggtttg aacacaattc gttttttcat ttgaattggc 2100
atttcccaat atttgctaaa catttgctgg agaaatcatt tttctttttt cttttttaga 2160
aaactcagaa tgaaaattca ttcccctgaa atatttaggt gtctatattc tatattttga 2220
tctattaagg gattagtatt tttccatgtt tattgtgtta tcagagtgca ttagaaagat 2280
tagtgattca tcttcacagc acatttttaa tcaagcagtt atttcaacca gcacattcgt 2340
tttgttcata ttcactatag aatgatatct tgtaaataaa gacattcagc acactgtgaa 2400
aatgtatttg tgcacctgct ttttaaatat ttctactaaa aatgaaaaaa aaaaaccctt 2460
agacctgtag atagtgatat cgtaatatta attgttaata aaatagtcac tgcc 2514

<210> 584
<211> 388
<212> PRT
<213> Homo sapiens

<400> 584
   Met Thr Ala Ser Val Leu Leu His Pro Arg Trp Ile Glu Pro Thr Val
   1 5 10 15
   Met Phe Leu Tyr Asp Asn Gly Gly Gly Leu Val Ala Asp Glu Leu Asn
               20                  25                  30
   Lys Asn Met Glu Gly Ala Ala Ala Ala Ala Ala Ala Ala Ala Ala Ala
           35                  40                  45
   Ala Ala Ala Gly Ala Gly Gly Gly Gly Phe Pro His Pro Ala Ala Ala
       50                  55                  60
   Ala Ala Gly Gly Asn Phe Ser Val Ala Ala Ala Ala Ala Ala Ala Ala
   65                  70                  75                  80
   Ala Ala Ala Ala Asn Gln Cys Arg Asn Leu Met Ala His Pro Ala Pro
                   85                  90                  95
   Leu Ala Pro Gly Ala Ala Ser Ala Tyr Ser Ser Ala Pro Gly Glu Ala
               100                 105                 110
   Pro Pro Ser Ala Ala Ala Ala Ala Ala Ala Ala Ala Ala Ala Ala Ala
           115                 120                 125
   Ala Ala Ala Ala Ala Ser Ser Ser Gly Gly Pro Gly Pro Ala Gly Pro
       130                 135                 140
   Ala Gly Ala Glu Ala Ala Lys Gln Cys Ser Pro Cys Ser Ala Ala Ala
   145                 150                 155                 160
   Gln Ser Ser Ser Gly Pro Ala Ala Leu Pro Tyr Gly Tyr Phe Gly Ser
                   165                 170                 175
   Gly Tyr Tyr Pro Cys Ala Arg Met Gly Pro His Pro Asn Ala Ile Lys
               180                 185                 190
   Ser Cys Ala Gln Pro Ala Ser Ala Ala Ala Ala Ala Ala Phe Ala Asp
           195                 200                 205
   Lys Tyr Met Asp Thr Ala Gly Pro Ala Ala Glu Glu Phe Ser Ser Arg
       210                 215                 220
   Ala Lys Glu Phe Ala Phe Tyr His Gln Gly Tyr Ala Ala Gly Pro Tyr
   225                 230                 235                 240
   His His His Gln Pro Met Pro Gly Tyr Leu Asp Met Pro Val Val Pro
                   245                 250                 255
   Gly Leu Gly Gly Pro Gly Glu Ser Arg His Glu Pro Leu Gly Leu Pro
               260                 265                 270
   Met Glu Ser Tyr Gln Pro Trp Ala Leu Pro Asn Gly Trp Asn Gly Gln
           275                 280                 285
   Met Tyr Cys Pro Lys Glu Gln Ala Gln Pro Pro His Leu Trp Lys Ser
       290                 295                 300
   Thr Leu Pro Asp Val Val Ser His Pro Ser Asp Ala Ser Ser Tyr Arg
   305                 310                 315                 320
   Arg Gly Arg Lys Lys Arg Val Pro Tyr Thr Lys Val Gln Leu Lys Glu
                   325                 330                 335
   Leu Glu Arg Glu Tyr Ala Thr Asn Lys Phe Ile Thr Lys Asp Lys Arg
               340                 345                 350
   Arg Arg Ile Ser Ala Thr Thr Asn Leu Ser Glu Arg Gln Val Thr Ile
           355                 360                 365
   Trp Phe Gln Asn Arg Arg Val Lys Glu Lys Lys Val Ile Asn Lys Leu
       370                 375                 380
   Lys Thr Thr Ser
   385

<210>  585
<211>  24
<212>  DNA
<213>  artificial

<220>
<223>  Description of artificial sequence: Oligonucleotide

<400>  585
tctggaagtc cactctgccc gacg                                              24

<210>  586
<211>  21
<212>  DNA
<213>  artificial

<220>
<223>  Description of artificial sequence: Oligonucleotide

<400>  586
tgtgacctgc cgctcagaga g                                                 21

<210>  587
<211>  8769
<212>  DNA
<213>  Homo sapiens

<400>  587
atttagaggc ggcgccaggg cggccgcgga gaaacgtgac acaccagccc tctcggaggg      60

gtttcggacc gaagggaaga agctgcgccg tgtcgtccgt ctccctgcgc gccgcgggca     120

cttctcctgg gctctccccg aactctcccg cgacctctgc gcgccctcag gccgccttcc     180

ccgccctggg ctcgggacaa cttctggggt ggggtgcaaa gaaagtttgc ggctcctgcc     240

gccggcctct ccgcctcttg gcctaggagg ctcgccgccc gcgcccgctc gttcggcctt     300

gcccgggacc gcgtcctgcc ccgagaccgc caccatgaac aagctttaca tcggcaacct 360
caacgagagc gtgacccccg cggacttgga gaaagtgttt gcggagcaca agatctccta 420
cagcggccag ttcttggtca aatccggcta cgccttcgtg gactgcccgg acgagcactg 480
ggcgatgaag gccatcgaaa ctttctccgg gaaagtagaa ttacaaggaa aacgcttaga 540
gattgaacat tcggtgccca aaaaacaaag gagccggaaa attcaaatcc gaaatattcc 600
accccagctc cgatgggaag tactggacag cctgctggct cagtatggta cagtagagaa 660
ctgtgagcaa gtgaacaccg agagtgagac ggcagtggtg aatgtcacct attccaaccg 720
ggagcagacc aggcaagcca tcatgaagct gaatggccac cagttggaga accatgccct 780
gaaggtctcc tacatccccg atgagcagat agcacaggga cctgagaatg ggcgccgagg 840
gggctttggc tctcggggtc agccccgcca gggctcacct gtggcagcgg gggccccagc 900
caagcagcag caagtggaca tcccccttcg gctcctggtg cccacccagt atgtgggtgc 960
cattattggc aaggaggggg ccaccatccg caacatcaca aaacagaccc agtccaagat 1020
agacgtgcat aggaaggaga acgcaggtgc agctgaaaaa gccatcagtg tgcactccac 1080
ccctgagggc tgctcctccg cttgtaagat gatcttggag attatgcata aagaggctaa 1140
ggacaccaaa acggctgacg aggttcccct gaagatcctg gcccataata actttgtagg 1200
gcgtctcatt ggcaaggaag gacggaacct gaagaaggta gagcaagata ccgagacaaa 1260
aatcaccatc tcctcgttgc aagaccttac cctttacaac cctgagagga ccatcactgt 1320
gaagggggcc atcgagaatt gttgcagggc cgagcaggaa ataatgaaga aagttcggga 1380
ggcctatgag aatgatgtgg ctgccatgag cctgcagtct cacctgatcc ctggcctgaa 1440
cctggctgct gtaggtcttt tcccagcttc atccagcgca gtcccgccgc ctcccagcag 1500
cgttactggg gctgctccct atagctcctt tatgcaggct cccgagcagg agatggtgca 1560
ggtgtttatc cccgcccagg cagtgggcgc catcatcggc aagaaggggc agcacatcaa 1620
acagctctcc cggtttgcca gcgcctccat caagattgca ccacccgaaa cacctgactc 1680
caaagttcgt atggttatca tcactggacc gccagaggcc caattcaagg ctcagggaag 1740
aatctatggc aaactcaagg aggagaactt ctttggtccc aaggaggaag tgaagctgga 1800
gacccacata cgtgtgccag catcagcagc tggccgggtc attggcaaag gtggaaaaac 1860
ggtgaacgag ttgcagaatt tgacggcagc tgaggtggta gtaccaagag accagacccc 1920
tgatgagaac gaccaggtca tcgtgaaaat catcggacat ttctatgcca gtcagatggc 1980
tcaacggaag atccgagaca tcctggccca ggttaagcag cagcatcaga agggacagag 2040
taaccaggcc caggcacgga ggaagtgacc agcccctccc tgtcccttcg agtccaggac 2100
aacaacgggc agaaatcgag agtgtgctct ccccggcagg cctgagaatg agtgggaatc 2160
cgggacacct gggccgggct gtagatcagg tttgcccact tgattgagaa agatgttcca 2220
gtgaggaacc ctgatctctc agccccaaac acccacccaa ttggcccaac actgtctgcc 2280
cctcggggtg tcagaaattc tagcgcaagg cacttttaaa cgtggattgt ttaaagaagc 2340

tctccaggcc ccaccaagag ggtggatcac acctcagtgg gaagaaaaat aaaatttcct 2400
tcaggtttta aaaacatgca gagaggtgtt ttaatcagcc ttaaaggatg gttcatttct 2460
tgaccttaat gtttttccaa tcttcttccc cctacttggg taattgatta aaatacctcc 2520
atttacggcc tctttctata tttacactaa tttttttatc tttattgcta ccagaaaaaa 2580
atgcgaacga atgcattgct ttgcttacag tattgactca agggaaaaga actgtcagta 2640
tctgtagatt aattccaatc actccctaac caataggtac aatacggaat gaagaagagg 2700
ggaaaatggg gagaaagatg gttaaaatac ataataatcc acgtttaaaa ggagcgcact 2760
tgtggctgat ctatgccaga tcaccatctt caaattggca caactgaaat ttccccactc 2820
tgttggggct tccccaccac attcatgtcc ctctcccgtg taggtttcac attatgtcca 2880
ggtgcacata ggtggtattg aatgctcagc agggtagggg ctgaccactg tccctgattc 2940
ccatcgttct caggcggatt ttatattttt ttaaagtcta ttttaatgat tggatatgag 3000
cactgggaag gggacgctaa ctccccttga taaagtctcg gttccatgga ggacttgagt 3060
ggccccaaag gctgccacgg tgccctcacc ccagcccatg tgctcccata agggctggtt 3120
cctagaggca ggggttgtgg ggcactccca gccacggcac tgttaccttg gtggtgggac 3180
ttggaaccca accctgagct cccgataaag ctaaagtcca tcatctggca aattcagtaa 3240
attggagagt acttgcttct gtttgtatct gagaggaatt tttaactgac ggcttctgtc 3300
tccatgaatc attatcagca tgatgaaagg tgtgtctaaa aaacaattca gaataccagc 3360
agcattgtac agcaaggggt aaataagctt aatttattaa tttaccaggc ttaattaaga 3420
tcccatggag tgtttagccc ttgtgggaga cagaagccat cagttaaatg aggttaggcc 3480
tctcctccta atatactgat tgacaatgca tattagccag gtaatgcact ttagctaccc 3540
tggacaatgc tatcaagtgt gctgggaagg gaggaaggcc tctctacata tggaaaagcc 3600
catgcgtgga gttcccctcc tttcaacatt gcaacaacag taacaacaag acaaccgcaa 3660
catgtgggcg tagtcaggca atgctgtgtg cgaagtaaac tacctcaagg tatgaagtta 3720
cctcagcaat tattttcctt tttgttcccc ccaaccccat taaaaaaatt tttttttgat 3780
ttttgttttt ttgcagcttg ctgatatttt atataaaaaa gaaaagcaaa gcaaaagaga 3840
agctgatagt cttgaatatt ttattttttt aatgaaaaga aaaaacaaga aagttatgtt 3900
tcataatttc ttacaacatg agccagtaac cctttaggaa ctctctatgg agaacaggcc 3960
tggtgggaaa ggctttgggg gctgccccct taggaggagg ctagtgctaa gagggaaggc 4020
ccaggtttga gagagcccag aggggcagag cccagagcct tgtttggccc tgatctctga 4080
cttctagagc cccagctgct ggcggctgct ggaatatcct acctgatagg attaaaaggc 4140
ctagtggagc tgggggctct cagtggttaa acaatgccca acaaccaacc agctggccct 4200
tggtctcctc tctttcctcc tttggttaaa gagcatctca gccagctttt cccaccagtg 4260
gtgctgttga gatattttaa aatattgcct ccgttttatc gaggagagaa ataataacta 4320
aaaaatatac cctttaaaaa aacctatatt tctctgtcta aaaatatggg agctgagatt 4380

ccgttcgtgg aaaaaagaca aggccaccct ctcgccctca gagaggtcca cctggtttgt 4440
cattgcaatg cttttcattt tttttttttg ttattgtttc atttcagttc cgtcttgcta 4500
ttcttcctaa tctatatcca tagatctaag gggcaaacag atactagtta actgccccca 4560
cctctgtctc cctgtcttct ttagatcggt ctgattgatt ttaaaagtgg acccaaactt 4620
agggaattct tgatttaggg tggctggtgg caaggagggg caggggatat ggggacgtga 4680
ctgggacagg ttcctgcctt atcattttct ccctaggaca ttcccttgta gcccccagaa 4740
ttgtctggcc caaattgaat agaagcagaa aaacatttag ggataacatc aggccagtag 4800
aattaagcct ctccacctgt cccaaccata aaaagggtct cccagctttc catctctggc 4860
tctatatgct ttatcccaaa acaaagcaga taacgttcag acgtcggcca tttagtaatt 4920
taaagcgaat ttccagcagc aagcatgctt tgatatctgg ttcagactat catcaggaag 4980
aaaaaaaaat cccacagtac ctgaaatgtg attgttgcag tgttcagttt ccttgggggc 5040
ctgctccctt cacaccttga gcccaagtcc ttttccgttg gctgattcag ctcccagaag 5100
agacgaggaa gtgtgtggca agggactgga aaacttcact tgcttggatt aggcaaggct 5160
ccactcattg ttgatatttg cccagcagga aaatcatgta agttatacca ccagaaagca 5220
aaaggagcat ggtttggtgg ttaaggttta gtgggatgaa ggacctgtct tggtgggccg 5280
ggccctcttg tgccccgtag gctaggtctt agggcaactc cttgccctcc tgctcagcac 5340
ctccatttcc ccatccttgg tgagataaca agctatcgcg aaaagcactt gggagatttg 5400
gatgatttga gaagagtgac ttaaaaaaaa tgcttctgtg ctctaagata tatatgtgtg 5460
tgtgtgtgct acatatatat ttttaagaaa ggaccatctc tttaggatat atttttaaat 5520
tctttgaaac acataaccaa aatggtttga ttcactgact gactttgaag ctgcatctgc 5580
cagttacacc ccaaatggct ttaatcccct ctcgggtctg gttgcctttt gcagtttggg 5640
ttgtggactc agctcctgtg aggggtctgg ttaggagaga gccattttta aggacaggga 5700
gttttatagc ccttttctac tttcctcccc tcctcccagt ccttatcaat cttttttcct 5760
ttttcctgac cccctccttc tggaggcagt tgggagctat ccttgtttat gcctcactat 5820
tggcagaaaa gaccccattt aaaacccaga gaacactgga gggggatgct ctagttggtt 5880
ctgtgtccat tttcctctgt gccaaagaca gacagacaga ggctgagaga ggctgttcct 5940
gaatcaaagc aatagccagc tttcgacaca tacctggctg tctgaggagg aaggcctcct 6000
ggaaactggg agctaagggc gaggcccttc ccttcagagg ctcctggggg attagggtgt 6060
ggtgtttgcc aagccaaggg gtagggagcc gagaaattgg tctgtcggct cctggttgca 6120
ctttggggaa ggagaggaag tttggggctc caggtagctc cctgttgtgg gactgctctg 6180
tcccctgccc ctactgcaga gatagcactg ccgagttccc ttcaggcctg gcagacgggc 6240
agtgaggagg ggcctcagtt agctctcaag ggtgccttcc cctcctccca acccagacat 6300
accctctgcc aaactgggaa ccagcagtgc tagtaactac ctcacagagc cccagagggc 6360
ctgcttgagc cttcttgctc cacaggagaa gctggtgcct ctaggcaacc ccttcctccc 6420

acctctcatc aggggtgggg gttctccttt ctttcccctg aagtgtttat ggggagatcc 6480
tagtggcttt gccattcaaa ccactcgact gtttgcctgt ttcttgaaaa ccagtagaag 6540
ggaaacagca cagcctgtca cagtaattgc aggaagattg aagaaaaatc ctcatcaatg 6600
ccaggggaca taaaagccat ttcccttcca aatactcgac aatttagatg cagaacattt 6660
ctctgtattc agacttagag taacaccagc tgaaaactgc agtttctttc ctttggatac 6720
ataaggcttc tctatcgggg tacgggacag ggaggaggcc tcatgtctga agggggattt 6780
aggggcgaga gccccagccc tgaccctcgg tcctgtgcac cgctttgggg cacagtctga 6840
tggcgccttt gctggcgcct tagtatggtt gactccggat ggacaaaaga aaaaaaattt 6900
tttttcttga atgaaatagc aggaagctcc tcgggagcat gtgttttgat taaccgcagg 6960
tgatggatgc tacgagtata aatggattaa ctacctcaat ccttacagta agattggaac 7020
taagggcagg gactcatgca taagggtatg aatcccagcc aggacaagtg agttgaggct 7080
tgtgccacaa aaggtttgtc cttggggaac aggcaggcct gccaggatcc cccccatatc 7140
gattgggctg ggagggctgg ccatgaggtc cccactttct gctttccttg cccatgtgtc 7200
acccctttgg cctccagctt gtccctctct cactttctat agctttgttg gaccagatgg 7260
tgaggaaagg aatggcctct tcccttctag agggggctgg ctggagtgag acctggggct 7320
tggcctggaa cccaccacac agccccaaag tcaggaagcc tggggaaacc agagctgaga 7380
cctcttcaac agggtttctt tgagatccta cacctccatt gggccctttt tcagtcttca 7440
atgggggccc agttggctct agaaggagaa gaggtgaagc aggatccttt gccctggggg 7500
agtctgaggg cgcggtcctt ggactcattc aggccgtctt tgtagttggg ggagttccac 7560
tgggcgatcc cagcccctcc ccacccaccc tctaatggac ctcctcatag aagccccatt 7620
tcacttttgt tttatctacc tcttagcaaa acaatagata aattaggtag tggcagctcc 7680
acttgcttag gttagggggg gaaaaagatt tctttttcca aaggaaaaaa atattacctt 7740
gagaatactt tccaaaaaat aaaattaaaa aaaaaaaaac caaaaaaaaa aatttttttt 7800
taaaagggag acattttcca gtgaccactg gattgtttta atttcccaag cttttttttc 7860
ccccataaat aagtttcact ctttggcgat tttcttcact tgtttaagat aacgtgctag 7920
ctattccaac aggtaacagc tttcacagtc tgcccctggc ctgtctcacc ccatccccca 7980
ccctattcct gccagtgagt ccttcctgtg cttctctccc ttctcccctc ccagccagct 8040
gacttcagtc acccctgtcc cccctcccct gccaataagc tcccccagga ataaaggctt 8100
tgttttgggg atgcttaaat cttgactggc acttcccggc tgtgggggct ggggagccac 8160
ttgtaacatt tctgtgcaga ttttatgtta gccactgcta tgtaaaagca cgttcaaaat 8220
gaatttcagc agattatgtg ttaccataat gaataaacgt cctctatcac catttggagt 8280
ctcccttttc tccaggatct tgatcctggt ccccaaaacc agagtgaatc aaaagagctt 8340
cctcccctga ggcaaagtgg atttgtaagc agttctgaaa catcacttac tcagaagagg 8400
gaacgatgta ttttgatgag tgcaaattgg gaagagctgg aggcctactg cttgggacag 8460

tttttttttt tttttttttt ttaaatatga gtgctagctt attctgtaat tgcggcaact 8520

ttgaaaattg tattttactg gaaatctgcc agccatcacc acccgatttt gattgtatcc 8580

ttcctcccat cctttaatct gttcattgct ttgggggagg tggggcagct ggctcacacg 8640

ttggagtttg ttctttgatg gatgaacgaa cactccagtt ttctttcccg tgaaggttgt 8700

ttcagccaca aaccacttca ttttgctgtt tcaatttcaa aataaaagga aacttatatt 8760

gaaagacaa 8769

<210> 588
<211> 577
<212> PRT
<213> Homo sapiens

<400> 588

Met Asn Lys Leu Tyr Ile Gly Asn Leu Asn Glu Ser Val Thr Pro Ala
1               5                   10                  15

Asp Leu Glu Lys Val Phe Ala Glu His Lys Ile Ser Tyr Ser Gly Gln
            20                  25                  30

Phe Leu Val Lys Ser Gly Tyr Ala Phe Val Asp Cys Pro Asp Glu His
        35                  40                  45

Trp Ala Met Lys Ala Ile Glu Thr Phe Ser Gly Lys Val Glu Leu Gln
    50                  55                  60

Gly Lys Arg Leu Glu Ile Glu His Ser Val Pro Lys Lys Gln Arg Ser
65                  70                  75                  80

Arg Lys Ile Gln Ile Arg Asn Ile Pro Pro Gln Leu Arg Trp Glu Val
                85                  90                  95

Leu Asp Ser Leu Leu Ala Gln Tyr Gly Thr Val Glu Asn Cys Glu Gln
            100                 105                 110

Val Asn Thr Glu Ser Glu Thr Ala Val Val Asn Val Thr Tyr Ser Asn
        115                 120                 125

Arg Glu Gln Thr Arg Gln Ala Ile Met Lys Leu Asn Gly His Gln Leu
    130                 135                 140

Glu Asn His Ala Leu Lys Val Ser Tyr Ile Pro Asp Glu Gln Ile Ala
145                 150                 155                 160

Gln Gly Pro Glu Asn Gly Arg Arg Gly Gly Phe Gly Ser Arg Gly Gln
                165                 170                 175

Pro Arg Gln Gly Ser Pro Val Ala Ala Gly Ala Pro Ala Lys Gln Gln
            180                 185                 190

Gln Val Asp Ile Pro Leu Arg Leu Leu Val Pro Thr Gln Tyr Val Gly
        195                 200                 205

Ala Ile Ile Gly Lys Glu Gly Ala Thr Ile Arg Asn Ile Thr Lys Gln
    210                 215                 220

Thr Gln Ser Lys Ile Asp Val His Arg Lys Glu Asn Ala Gly Ala Ala
225                 230                 235                 240

Glu Lys Ala Ile Ser Val His Ser Thr Pro Glu Gly Cys Ser Ser Ala
                245                 250                 255

Cys Lys Met Ile Leu Glu Ile Met His Lys Glu Ala Lys Asp Thr Lys
            260                 265                 270

Thr Ala Asp Glu Val Pro Leu Lys Ile Leu Ala His Asn Asn Phe Val
        275                 280                 285

Gly Arg Leu Ile Gly Lys Glu Gly Arg Asn Leu Lys Lys Val Glu Gln
    290                 295                 300

Asp Thr Glu Thr Lys Ile Thr Ile Ser Ser Leu Gln Asp Leu Thr Leu
305                 310                 315                 320

Tyr Asn Pro Glu Arg Thr Ile Thr Val Lys Gly Ala Ile Glu Asn Cys
                325                 330                 335

Cys Arg Ala Glu Gln Glu Ile Met Lys Lys Val Arg Glu Ala Tyr Glu
            340                 345                 350

Asn Asp Val Ala Ala Met Ser Leu Gln Ser His Leu Ile Pro Gly Leu
        355                 360                 365

Asn Leu Ala Ala Val Gly Leu Phe Pro Ala Ser Ser Ser Ala Val Pro
    370                 375                 380

Pro Pro Pro Ser Ser Val Thr Gly Ala Ala Pro Tyr Ser Ser Phe Met
385                 390                 395                 400

Gln Ala Pro Glu Gln Glu Met Val Gln Val Phe Ile Pro Ala Gln Ala
                405                 410                 415

Val Gly Ala Ile Ile Gly Lys Lys Gly Gln His Ile Lys Gln Leu Ser
            420                 425                 430

Arg Phe Ala Ser Ala Ser Ile Lys Ile Ala Pro Pro Glu Thr Pro Asp
        435                 440                 445

Ser Lys Val Arg Met Val Ile Ile Thr Gly Pro Pro Glu Ala Gln Phe
    450                 455                 460

Lys Ala Gln Gly Arg Ile Tyr Gly Lys Leu Lys Glu Glu Asn Phe Phe
465                 470                 475                 480

Gly Pro Lys Glu Glu Val Lys Leu Glu Thr His Ile Arg Val Pro Ala
                485                 490                 495

Ser Ala Ala Gly Arg Val Ile Gly Lys Gly Gly Lys Thr Val Asn Glu
            500                 505                 510

Leu Gln Asn Leu Thr Ala Ala Glu Val Val Val Pro Arg Asp Gln Thr
        515                 520                 525

Pro Asp Glu Asn Asp Gln Val Ile Val Lys Ile Ile Gly His Phe Tyr
    530                 535                 540

Ala Ser Gln Met Ala Gln Arg Lys Ile Arg Asp Ile Leu Ala Gln Val
545                 550                 555                 560

Lys Gln Gln His Gln Lys Gly Gln Ser Asn Gln Ala Gln Ala Arg Arg
                565                 570                 575

Lys

<210>  589
<211>  21
<212>  DNA
<213>  artificial

<220>
<223>  Description of artificial sequence: Oligonucleotide

<400>  589
atttctatgc cagtcagatg g                                              21

<210>  590
<211>  21
<212>  DNA
<213>  artificial

<220>
<223>  Description of artificial sequence: Oligonucleotide

<400>  590
gtgggcaaac ctgatctaca g                                              21

<210>  591
<211>  5016
<212>  DNA
<213>  Homo sapiens

<400>  591
taacattctg ttcttccgcg tgatggattt tcttttggag attcgaactg aagcctgtac      60

ggaggaaatg ttgtttttaa gggaaatgaa tagaaacaat ccactttgaa gaagccatgg     120

cgaaatcaaa gacaaaacat agactttgtt ctcaggaatc ttcagtatct gccctgctgg     180

caagctgcac cctgagtggt agtaattcct ctaattctga tggctcgttt cactataaag 240
ataagctgta cagatctgct tctcaagctc tacaggctta tattgatgat tttgatctag 300
gccaaatata tcctggtgca agcactggaa aaattaacat tgatgaggat tttactaata 360
tgtcacagtt ctgcaactat atttacaaac caaacaatgc ttttgaaaac cttgatcacg 420
aaaagcactc aaacttcata tcctgtagaa gacacatcgt taatgacata gactccatga 480
gcctaacaac tgatgatcta ttaagactcc cagcagatgg atcattttct tatacttatg 540
ttggaccgag tcaccgaacg agcaagaaaa acaagaaatg ccgtggaagg ctgggttcat 600
tggacattga gaagaatcca cattttcaag gaccctacac ttccatgggc aaggataact 660
ttgttactcc tgttatacgc tcaaatataa atggaaagca atgtggtgac aaaattgaat 720
tgcttatctt gaaggccaag agaaatctag agcagtgtac tgaagaatta ccaaagtcca 780
tgaaaaagga tgacagtcct tgctcattag ataaacttga agcagacaga tcatgggaaa 840
atattcctgt tactttcaaa tctcctgttc ccgttaactc tgatgatagt cctcaacaaa 900
cttcaagggc aaagagtgct aaaggggttc ttgaagactt tctaaataat gataatcaga 960
gctgtactct ctctggaggc aaacatcatg gtcctgttga agccctgaaa caaatgttat 1020
ttaaccttca agcagtacaa gaacgtttta atcaaaataa gaccacagat ccaaaagaag 1080
agattaaaca agtttcagaa gatgatttct ctaaattaca gttgaaggaa agtatgattc 1140
ctattactag gtcacttcag aaggctttgc accatttatc tcgcctgaga gacctggttg 1200
atgatacgaa tggagaacgg tcaccgaaaa tgtgaagagg aaaatgaaac tgtcaccacg 1260
ataaatagtc accacagaac aaataggcat tttttctatt acttaaactg acaaagtaaa 1320
tataagccat acattatttt gtggttggtt caaggattat atatttctaa aacactaaac 1380
ttgaaaatac ccataggttt tgggacctat ctttattttg tgccaacata ctagaatgtg 1440
aactgcaagg acccacaatg tatcctgaag tcttactttc gccttctggc cagcaaatgt 1500
ctaatattta aagatggatg acttctgttc ttgaagctta cctggattta accttcttca 1560
gcatcctcaa cattttatta cctggttcag gatcattaag aaacttactg gtttttatcc 1620
aaaatctttt acgttaaata gactttttta aagatatagt tagcatcact tttaaacagc 1680
ttaaaggaat atcaaaattg ttattgtgta tctcatctat aaggaagtct gttactttga 1740
aattttcata aatttaatat ttaagataca ttgtatttga aaattgcatt aatagtgggg 1800
tgatactgtg ttaaaaggaa cgttgtgttg tgacattcaa gagaacctcc tcatttaatt 1860
agtactttga ttctgtgtaa gataatcttg gtagtgcttg acagtttcca aacctttttt 1920
tggagagata tttaagaatt taatattttg atattagatt gtttcccaga ttttaatttt 1980
ggggttggct caaactagtg aaaactatga ctcaatggcc aattgcttta tcaaatttga 2040
taactaaaac ttaaaatgaa tatggaaaat cagaaagcaa ctctatttta gagctatttt 2100
gtaagagttg tgctttcttt aacaccatct gtagtcttaa gtttgtctct agctagaact 2160
gaacaaagct ctataatttt taccaagcac ttattattaa tacttcttat aagtagtaag 2220

catctttact aacacaactg agaattaagt cataaaacat aactaataca gcacattact 2280
gcctgacaaa attaaagagt actgtgtgta tgtataacta ctacaggtta acacttcacc 2340
caaatgatag cgtttttcct cagtagatta ttgtcaaata ggaatttcta agcacattga 2400
gtcaaagcat tttttccagg ttaataaagt gttatttact atctttgtta gaggtgacat 2460
gtcaaacact acagtgagct ctgtggggtt tttttttttt tttttgcccg tgagtttttt 2520
accatgctgc tctgaccagt ttgagtggca attaccaata gatttgtttt ctttattcta 2580
tggagatgtt tttaccactg acactgtttt ctgattatag tctgcttcat agaaaatagc 2640
ctgcataatc aaacaaggag ttactttgaa attaaagtat gcctggctat taaaaatgca 2700
gattttaggt gggtaaacat caggtaggtc tgggtgggtc atgttctagg cctagaaaaa 2760
tacactatta gacaagttct aaagaaggca aggagataaa ggcatcaggt ggtaacttct 2820
aattgaatat tatatgttga tcatacataa tatatactat gcctggaaat tatgactgaa 2880
aagcacctat tcggttagtg ctcctattca tgagaacata tctccaatac taaatgagat 2940
aagcctgttc taaaatctta tagccagtat tttaagaaac ttgattatac ttaccaaagg 3000
aacattgttt gttttctctt gttttaaata tggagaggtt taatccttta cataacaaag 3060
gaattaattt tagcaaaatg attcattcca accttcttat aagaaatatc taggagagtc 3120
aagtaagaaa aataacgaat ctaagtgata aacattcaag aaattctcta aataagagat 3180
ttatttataa ttttaatatc tcagggttct ttttaggttt ccaggggaaa agagcaggat 3240
aacagtgtgg agactgctaa gttgagaatt taaaacaaat gagaacataa gatttttaaa 3300
attgcattgt gaatgtaaaa tttttatcaa tcctttgctc ttttagacat attgagaaaa 3360
tgttaaatag aaaaaattaa gaaattttaa taagatgttt cagatctttg agtatgaaaa 3420
acataacaaa aaagcctaat ttcaaaaaac tatttgagat caagggacaa tggtgtgacc 3480
aatatgaagg gtcaagactg aaatgtattg tctttactat caagaactct actttcagtt 3540
gtttctcaga cagttaattt cagcttcata gagatttctg agcaaattaa gaaacactgt 3600
tttcctgggt ttgttttggg tatatgtcat tatagttatg ttatttcttg ttgaaattta 3660
taattgtagg ttttttgtat tgttttggta tttaatggtg tataatgtgt tattacatta 3720
tatgtagtta taccaaaata ttgcctgaag agaaatcatg acaaggtccc ctgtttattc 3780
ctgtgttaca gacgcatgga attgctcctg tagatttgaa tttttgtttc atttttttct 3840
gtcccaccct tcactctctc tgtttcagaa catttttggt agaagtgcta tccagaagtg 3900
aacttgtcaa aaggcaagta gcatgaaaga agacagaaga agcaaaaggc taatacagtg 3960
gataatttct gagcacttga agtttcttca aatgtgcaag actgtgtgtc ttcctattag 4020
atgtataaat tggatatttc atgcctaatt aaatgttgcg ttggattgca gtgcctatca 4080
tacagtgatt ggagtaaatt gaggcctaat cctgaacaca tatagagcat attgttagat 4140
atttttcctg tgacatttga agttattatt ctcccatttc ctttttcttt ttttgtttat 4200
aatcatatgt ccctaagatt gttttccttt tttggaccaa aaaaaagaaa aaaaaaatct 4260

tagcttttca tcctcccagt gtattctgca ttgtccttac cctagatcag ccccttctgt 4320
gtaacagttt ttctcacaat gtagcaactt ttatccaccc ttcaggacct tcactgggac 4380
tagttcattc attttcaaat agctatttca acctttaaca tctactgtct tagtctttta 4440
cacagaagcc agagtgactg gtcttggcaa gactctgttg tgtatcacca ctctaacctt 4500
actgatttgt ttcagcaaat ttgctttagt taaattgctt tactcagatt cccccaaact 4560
ttatatgtgt attgtcatct ttgtgcatat tatttctcat gcatgaaata ctcaattttt 4620
attcttttat ctaacgctta ctcttacatt tctttaaagc tctggccaag tattttattt 4680
cgtccctaaa cattctaact atccaccaaa ctggtaagtt ggcttttctt tttcctcccc 4740
ctgtcattca tttagctgtt atatttcatt ttaatgtttt gggtggtgcc tcttatacta 4800
tgttgtattc ctagacaagg aaatgtatat caaaatatgt tagatgattg attgttttat 4860
ctccttgatg atagcacctc ttatactgct ttacagaatc aggaaaaagt aaactgcatt 4920
ttacatagtg gttttaaata ttgattgatt gatattctaa acctggtttc ctatataaag 4980
ttgtaagttc aagataaaaa aaaaaaaaaa aaaaca 5016

<210> 592
<211> 372
<212> PRT
<213> Homo sapiens

<400> 592

Met Ala Lys Ser Lys Thr Lys His Arg Leu Cys Ser Gln Glu Ser Ser
1               5                   10                  15

Val Ser Ala Leu Leu Ala Ser Cys Thr Leu Ser Gly Ser Asn Ser Ser
            20                  25                  30

Asn Ser Asp Gly Ser Phe His Tyr Lys Asp Lys Leu Tyr Arg Ser Ala
        35                  40                  45

Ser Gln Ala Leu Gln Ala Tyr Ile Asp Asp Phe Asp Leu Gly Gln Ile
    50                  55                  60

Tyr Pro Gly Ala Ser Thr Gly Lys Ile Asn Ile Asp Glu Asp Phe Thr
65                  70                  75                  80

Asn Met Ser Gln Phe Cys Asn Tyr Ile Tyr Lys Pro Asn Asn Ala Phe
                85                  90                  95

Glu Asn Leu Asp His Glu Lys His Ser Asn Phe Ile Ser Cys Arg Arg
            100                 105                 110

His Ile Val Asn Asp Ile Asp Ser Met Ser Leu Thr Thr Asp Asp Leu
        115                 120                 125

Leu Arg Leu Pro Ala Asp Gly Ser Phe Ser Tyr Thr Tyr Val Gly Pro
    130                 135                 140

Ser His Arg Thr Ser Lys Lys Asn Lys Lys Cys Arg Gly Arg Leu Gly
145                 150                 155                 160

Ser Leu Asp Ile Glu Lys Asn Pro His Phe Gln Gly Pro Tyr Thr Ser
                165                 170                 175

Met Gly Lys Asp Asn Phe Val Thr Pro Val Ile Arg Ser Asn Ile Asn
            180                 185                 190

Gly Lys Gln Cys Gly Asp Lys Ile Glu Leu Leu Ile Leu Lys Ala Lys
        195                 200                 205

Arg Asn Leu Glu Gln Cys Thr Glu Glu Leu Pro Lys Ser Met Lys Lys
    210                 215                 220

Asp Asp Ser Pro Cys Ser Leu Asp Lys Leu Glu Ala Asp Arg Ser Trp
225                 230                 235                 240

Glu Asn Ile Pro Val Thr Phe Lys Ser Pro Val Pro Val Asn Ser Asp
                245                 250                 255

Asp Ser Pro Gln Gln Thr Ser Arg Ala Lys Ser Ala Lys Gly Val Leu
            260                 265                 270

Glu Asp Phe Leu Asn Asn Asp Asn Gln Ser Cys Thr Leu Ser Gly Gly
        275                 280                 285

Lys His His Gly Pro Val Glu Ala Leu Lys Gln Met Leu Phe Asn Leu
    290                 295                 300

Gln Ala Val Gln Glu Arg Phe Asn Gln Asn Lys Thr Thr Asp Pro Lys
305                 310                 315                 320

Glu Glu Ile Lys Gln Val Ser Glu Asp Asp Phe Ser Lys Leu Gln Leu
                325                 330                 335

Lys Glu Ser Met Ile Pro Ile Thr Arg Ser Leu Gln Lys Ala Leu His
            340                 345                 350

His Leu Ser Arg Leu Arg Asp Leu Val Asp Asp Thr Asn Gly Glu Arg
        355                 360                 365

Ser Pro Lys Met
    370

<210>  593
<211>  21
<212>  DNA
<213>  artificial

<220>

<223>  Description of artificial sequence: Oligonucleotide

<400>  593
aatggaaagc aatgtggtga c                                                21


<210>  594
<211>  21
<212>  DNA
<213>  artificial

<220>
<223>  Description of artificial sequence: Oligonucleotide

<400>  594
tccagagaga gtacagctct g                                                21


<210>  595
<211>  2061
<212>  DNA
<213>  Homo sapiens

<400>  595
atgaggcgga caggccccga ggaggaggcc tgcggcgtgt ggctggacgc ggcggcgctg      60

aagaggcgga aagtgcagac acatttaatc aaaccaggca ccaaaatgct aacactcctt     120

cctggagaaa gaaaggctaa tatttatttt actcaaagaa gagctccatc tacaggcatt     180

caccagagaa gcattgcttc cttcttcacc ttgcagccag gaaagacaaa tggcagtgac     240

cagaagagtg tttcatctca tacagaaagt cagatcaaca aagagtccaa gaaaaatgcg     300

acccagctag accatttgat cccaggctta gcacacgatt gcatggcatc ccctttagcc     360

acttcaacca ctgcggacat ccaggaagct ggactctctc ctcagtccct ccagacttct     420

ggccaccaca gaatgaaaac cccattttca actgagctat ctttgctcca gcctgatact     480

ccagactgtg ctggagatag tcatacccca ctggcttttt ccttcaccga ggacttggaa     540

agttcttgtt tgctagaccg aaaggaagaa aaaggggatt ctgccaggaa atgggaatgg     600

cttcatgagt ctaagaagaa ctatcagagt atggagaaac acaccaaact acctggggac     660

aaatgctgtc agcccttagg caagactaaa ttggaaagaa aggtgtctgc caaagaaaac     720

aggcaggccc ctgtcctcct tcaaacatac agggaatcct ggaatggaga aaacatagaa     780

tcggtgaaac aaagccgtag tccagtttct gtgttttcct gggacaatga aaagaatgac     840

aaggactcct ggagtcaact tttcactgaa gattctcaag gccagcgggt cattgcccac     900

aacactagag ctccttttca agatgtaacc aataactgga attgggactt agggccgttt     960

cctaacagtc cttgggctca gtgccaggag gatgggccaa ctcaaaatct gaagcctgat    1020

ttgctcttta cccaggactc tgaaggtaat caagttatca gacaccaatt ctaaatgttt    1080

gaagctttgt ttctaaaagt accttgaaat gatagagatg taggaaaata tagttgtggg    1140

tggagagagg agtgagtttg tttaggtggg aaggtggcat gggatgaagt tgtcattact    1200

gagcatcttc tctgtgtaaa taaagggcag taccattgtt aagacagtgg gattggcatc    1260

atggctttcc ctcaggaagg tggtggctgg taaattccct gaatgagtct atgatgaaca    1320

ctgaggcagc acagtgggta tttatctcta tgaaagtgcc ttttactcag cctgcacaga 1380

gccatctctt tgcccttcca gatgtctgac tgggaccttg cttatggatg tgtttttttt 1440

tttttttttt tgagatggag tctcgctctg tcgccaggct ggagtgcagt ggtgcgacct 1500

cagctcactg caccctctgt gtcccggatt caagcgattc tcctgcctca gcctcccgaa 1560

tagcagggac tacaggcatg cgccaccacg cccagctaat tttttttgga tttttagtag 1620

agacgaggtt tcaccatatt agccaggatg gtctccatct cctgacctcc tgatccgccc 1680

acctcagcct cccaaagtgc tgagattaca ggcataagcc accgcgccca gccagatgtg 1740

tgagctttta atctctggct gatcttaacc cacatcagcc taagcttggg atgattactc 1800

ttgacccttt tttttcagtg attagcaaat ctccccacaa cccaggtgtg gagagaagag 1860

aggtagaatg gtgctagttt cctattttat ttttgtggta actgtacagc actttaaagt 1920

tatatactct atgtttaaat atctccctta aaaagcctga gctgtacaac aatctggatg 1980

tgactctgtt acccttttcc cacaagatag gagggaatcc cctttgtaaa actatgaatc 2040

caaataaatg tttacaaagt g 2061

<210> 596
<211> 357
<212> PRT
<213> Homo sapiens

<400> 596

Met Arg Arg Thr Gly Pro Glu Glu Glu Ala Cys Gly Val Trp Leu Asp
1               5                   10                  15

Ala Ala Ala Leu Lys Arg Arg Lys Val Gln Thr His Leu Ile Lys Pro
            20                  25                  30

Gly Thr Lys Met Leu Thr Leu Leu Pro Gly Glu Arg Lys Ala Asn Ile
        35                  40                  45

Tyr Phe Thr Gln Arg Arg Ala Pro Ser Thr Gly Ile His Gln Arg Ser
    50                  55                  60

Ile Ala Ser Phe Phe Thr Leu Gln Pro Gly Lys Thr Asn Gly Ser Asp
65                  70                  75                  80

Gln Lys Ser Val Ser Ser His Thr Glu Ser Gln Ile Asn Lys Glu Ser
                85                  90                  95

Lys Lys Asn Ala Thr Gln Leu Asp His Leu Ile Pro Gly Leu Ala His
            100                 105                 110

Asp Cys Met Ala Ser Pro Leu Ala Thr Ser Thr Thr Ala Asp Ile Gln
        115                 120                 125

Glu Ala Gly Leu Ser Pro Gln Ser Leu Gln Thr Ser Gly His His Arg
    130                 135                 140

Met Lys Thr Pro Phe Ser Thr Glu Leu Ser Leu Leu Gln Pro Asp Thr
145                 150                 155                 160

Pro Asp Cys Ala Gly Asp Ser His Thr Pro Leu Ala Phe Ser Phe Thr
                165                 170                 175

Glu Asp Leu Glu Ser Ser Cys Leu Leu Asp Arg Lys Glu Glu Lys Gly
            180                 185                 190

Asp Ser Ala Arg Lys Trp Glu Trp Leu His Glu Ser Lys Lys Asn Tyr
        195                 200                 205

Gln Ser Met Glu Lys His Thr Lys Leu Pro Gly Asp Lys Cys Cys Gln
    210                 215                 220

Pro Leu Gly Lys Thr Lys Leu Glu Arg Lys Val Ser Ala Lys Glu Asn
225                 230                 235                 240

Arg Gln Ala Pro Val Leu Leu Gln Thr Tyr Arg Glu Ser Trp Asn Gly
                245                 250                 255

Glu Asn Ile Glu Ser Val Lys Gln Ser Arg Ser Pro Val Ser Val Phe
            260                 265                 270

Ser Trp Asp Asn Glu Lys Asn Asp Lys Asp Ser Trp Ser Gln Leu Phe
        275                 280                 285

Thr Glu Asp Ser Gln Gly Gln Arg Val Ile Ala His Asn Thr Arg Ala
    290                 295                 300

Pro Phe Gln Asp Val Thr Asn Asn Trp Asn Trp Asp Leu Gly Pro Phe
305                 310                 315                 320

Pro Asn Ser Pro Trp Ala Gln Cys Gln Glu Asp Gly Pro Thr Gln Asn
                325                 330                 335

Leu Lys Pro Asp Leu Leu Phe Thr Gln Asp Ser Glu Gly Asn Gln Val
            340                 345                 350

Ile Arg His Gln Phe
        355

<210>  597
<211>  21
<212>  DNA
<213>  artificial

<220>
<223>  Description of artificial sequence: Oligonucleotide

<400>  597
caccttgcag ccaggaaaga c                                                21

<210> 598
<211> 21
<212> DNA
<213> artificial

<220>
<223> Description of artificial sequence: Oligonucleotide

<400> 598
cagcacagtc tggagtatca g 21


<210> 599
<211> 1907
<212> DNA
<213> Homo sapiens

<400> 599
aatgcacacg agcagacaga gaagcaacat ctttaaggta ctgagggcag gagaagttaa 60

tgtagaatac tatgccagaa aaaataaatt cccaaaagtg gaagtgaaat aaggacattt 120

agagatgtac aaaagctgac cgaattcact accagtcaac ccacactaca agaaacatca 180

aatgagtcct ccaagcagaa ggaatccaat accagatgaa aatccagatc tccacgagga 240

aatgaagaac accagaaatg ggtaactata ctagatcggc cctttcttca aataagagca 300

gttggaataa caaagctgtt cagttgtacc cttggaatcc actgaaatcc tgggtaggga 360

agctccagta ccaccaactg gaaagactgg gaatgcctaa tagctggtac tggccattgt 420

cgtaggcttt gtccactctg acaaactgaa gatggggact cgactcacct tcgccagcca 480

caggaggacc tccagacgag gacaggactc gctgcctttc tttcccgtca gaaagggatc 540

ccttgcggac aggacctaag caccacgcac ctgccccccg ggatgccgaa cgaagtggtc 600

cctaaagctc ctctgcaggc ccaaccgaaa caggcctgaa gctccaggat gggcgagagg 660

atcctctttg agcgaaacca gccttctgcc tggctggccc tggtcaacac cctgggaaga 720

ggccgatttg gcggacagaa cggaagaaaa gacctaaagg tagaatctca tgatgtcgag 780

atgttaaaac actcaaattt taaggttcga ctgtgagggg gagatagggg gtctcgagct 840

ggatcgaccc ctgagccttc atctgcagag tcctgtgcac cagctcagag gacaggacta 900

tgtgcaccaa tggttctcat caggcggcaa cttcaccctc acatgcctcc cccatccctg 960

ctggtacaca agaccacgac taggggaagc ccggagggag aatgttaacc cctggcatct 1020

atctagtcag cagaggtgag ggatgctgct aaacacctta caatccaccg gaggacaccc 1080

gcccccaccg accccgaagt ggccattccc tggaggtggg gaaactcgcc tgtagatcaa 1140

tgcccacgca cttggcggac aggaaatcac gaattggcca ctaactggat cttggatctg 1200

agaaaaaaat tccagcgtca gagggaactc tcggagattt gcccagagca taaggaacgt 1260

actccttccc tcagtgatgg atcctcacat ctgggggaaa tcatagacaa tttcttttgt 1320

agggcgaact ctgctataca gtttatgatg tcagagtgaa tactttcttt gagttgcagt 1380

cagaaactgt agatttttaa aaatttaaaa ttcattattc tctgtcagta ttccaaagtg 1440

tatacagaaa gctattgcac tgttcaggag atggcgctta acattttgga aattcaaggt 1500

gatgaatgtc cagataagac tatctctcct ggtacaaagt ttgacaatgc tgaacatttt 1560

taaaggttct ttttgatata caaagtgcac caatgagtgc tttttaattc ttacaataat 1620

tctgggtgag gtaggtattt ttccaattcc cattttatgc ttcggtagcc ctttgtattt 1680

atacttcaaa acacttggct ctcttgtaat tatttaagaa attagttgtg attatttgtt 1740

taatgtgcag gagttacaaa aggcaagctt tagaacaaga cagacctggt tatgattcct 1800

ggctctgaaa gctgtacacc ctgtgaccct agacaggtgt tttaatgcct cgctgcctct 1860

gtttcttgct ctgtaaaatg tgaacaataa cagtattggc ctcatgc 1907

<210> 600
<211> 21
<212> DNA
<213> artificial

<220>
<223> Description of artificial sequence: Oligonucleotide

<400> 600
ttgcggacag gacctaagca c 21

<210> 601
<211> 21
<212> DNA
<213> artificial

<220>
<223> Description of artificial sequence: Oligonucleotide

<400> 601
tagtcctgtc ctctgagctg g 21

<210> 602
<211> 2553
<212> DNA
<213> Homo sapiens

<400> 602
tgcgtgtcgg ggtccgctcg tgcgcgcctc tccggggtct gtgcgcgtgg ccctccgctc 60

gcgccggagg gcgtgggcgt ggcctcggcg tgggtgtggc cgctcgggga ggggcctccc 120

gggggcgggg ccggcctggt ccgcgcggtg acgcgccctg cagccccgag cgagcgagcg 180

agcgagcgag ttgccgagcg cgccccgtcc ctcgcgcgcg atgctcccct ggacggcgct 240

cggcctggcc ctgagcttgc ggctggcgct ggcgcggagc ggcgcggagc gcggtccacc 300

agcatcagcc ccccgagggg acctgatgtt cctgctggac agctcagcca gcgtctctca 360

ctacgagttc tcccgggttc gggagtttgt ggggcagctg gtggctccac tgcccctggg 420

caccggggcc ctgcgtgcca gtctggtgca cgtgggcagt cggccataca ccgagttccc 480

cttcggccag cacagctcgg gtgaggctgc ccaggatgcg gtgcgtgctt ctgcccagcg 540

catgggtgac acccacactg gcctggcgct ggtctatgcc aaggaacagc tgtttgctga 600

agcatcaggt gcccggccag gggtgcccaa agtgctggtg tgggtgacag atggcggctc 660

cagcgaccct gtgggccccc ccatgcagga gctcaaggac ctgggcgtca ccgtgttcat 720
tgtcagcacc ggccgaggca acttcctgga gctgtcagcc gctgcctcag cccctgccga 780
gaagcacctg cactttgtgg acgtggatga cctgcacatc attgtccaag agctgagggg 840
ctccattctc gacgcgatgc ggccgcagca gctccatgcc acggagatca cgtccagcgg 900
cttccgcctg gcctggccac ccctgctgac cgcagactcg ggctactatg tgctggagct 960
ggtgcccagc gcccagccgg gggctgcaag acgccagcag ctgccaggga acgccacgga 1020
ctggatctgg gccggcctcg acccggacac ggactacgac gtggcgctag tgcctgagtc 1080
caacgtgcgc ctcctgaggc cccagatcct gcgggtgcgc acgcggcccg gtgaggcagg 1140
gccgggggct tcgggcccgg agtcgggggc tgggccggcc cccacgcagc tcgccgccct 1200
ccccgcccca gaggaggccg ggccagagcg catcgtcatc tcccacgccc ggccgcgcag 1260
cctccgcgtg agttgggccc cagcgctggg ctcagccgcg gcgctcggct accacgtgca 1320
gttcgggccg ctgcggggcg gggaggcgca gcgggtggag gtgcccgcgg gccgcaactg 1380
caccacgctg cagggcctgg cgccgggcac cgcctacctg gtgaccgtga ccgccgcctt 1440
ccgctcgggc cgcgagagcg cgctgtccgc caaggcctgc acgcccgacg gcccgcgccc 1500
gcgcccacgc cccgtgcccc gcgccccgac cccggggacc gccagccgtg agccgtaagc 1560
cggcgtcccc gcccagccga gagggccggc gcctacctga gggcccctgt gtcccgaacc 1620
cggagcggag gcgcccaacc cggcagacgg gtgcaggccc ggcctttccc cacgcggact 1680
ccgcgcgacc ccggccctct ccctgcggcc gcagggcttc cccgcctggc gcctgccctc 1740
cagggctggg gcctcgcctg gcgggacccc gcagcagccc cggccccatc cccgcccaga 1800
gccgggcgtc gtgtgggtcc gtgggtgata attgagagcg tcagacccag gactgttcag 1860
ggaggagccc cggtcagact cccacgtgtg aagaccgggc cccaagtggc aagggctggc 1920
ctggggcggg cagcttgggt cctggacgtt gataggaagc ggaaggggaa tcgcgggaag 1980
ctggcccagg tcaggtccgc aaaggcttct gaagaagagg aagggcgagt aggggcacct 2040
ggacgctgat ggtggccagg atgctcagct ggccaggagg gcagcacctg ctggggacgg 2100
tggccctgcc ttcatgccca ggacaccagc tgggtccagc tagcagccac tgggaatcag 2160
aggaatgggg cagagctggg cattcaggac cttgaggaca cgtgacccca cccgcccacc 2220
gccactatca ggccccggga ccgcactgac aggaaacctt ccgtcgtgag ggagcacttc 2280
ccaggggccg cagggacgac actctccagg gaggccccag caaccacacc atcttcttgc 2340
tgtgagaggt ctcaccccgg gctacctcct gtcactactc actgccctgg ggtccgtggg 2400
caagttgccc agggtggggg tgcctagcca ggtgcagtcc ccgccccgcc tagtcctcgg 2460
cgtcacgcaa tgctcacctc gcctcttccc cactaacatc ccagacttta aaattcagta 2520
aatcagatgt acaccgaaaa aaaaaaaaaa aaa 2553

<210> 603
<211> 445

<212>  PRT
<213>  Homo sapiens

<400>  603

Met Leu Pro Trp Thr Ala Leu Gly Leu Ala Leu Ser Leu Arg Leu Ala
1               5                   10                  15

Leu Ala Arg Ser Gly Ala Glu Arg Gly Pro Pro Ala Ser Ala Pro Arg
            20                  25                  30

Gly Asp Leu Met Phe Leu Leu Asp Ser Ser Ala Ser Val Ser His Tyr
        35                  40                  45

Glu Phe Ser Arg Val Arg Glu Phe Val Gly Gln Leu Val Ala Pro Leu
    50                  55                  60

Pro Leu Gly Thr Gly Ala Leu Arg Ala Ser Leu Val His Val Gly Ser
65                  70                  75                  80

Arg Pro Tyr Thr Glu Phe Pro Phe Gly Gln His Ser Ser Gly Glu Ala
                85                  90                  95

Ala Gln Asp Ala Val Arg Ala Ser Ala Gln Arg Met Gly Asp Thr His
            100                 105                 110

Thr Gly Leu Ala Leu Val Tyr Ala Lys Glu Gln Leu Phe Ala Glu Ala
        115                 120                 125

Ser Gly Ala Arg Pro Gly Val Pro Lys Val Leu Val Trp Val Thr Asp
    130                 135                 140

Gly Gly Ser Ser Asp Pro Val Gly Pro Pro Met Gln Glu Leu Lys Asp
145                 150                 155                 160

Leu Gly Val Thr Val Phe Ile Val Ser Thr Gly Arg Gly Asn Phe Leu
                165                 170                 175

Glu Leu Ser Ala Ala Ala Ser Ala Pro Ala Glu Lys His Leu His Phe
            180                 185                 190

Val Asp Val Asp Asp Leu His Ile Ile Val Gln Glu Leu Arg Gly Ser
        195                 200                 205

Ile Leu Asp Ala Met Arg Pro Gln Gln Leu His Ala Thr Glu Ile Thr
    210                 215                 220

Ser Ser Gly Phe Arg Leu Ala Trp Pro Pro Leu Leu Thr Ala Asp Ser
225                 230                 235                 240

Gly Tyr Tyr Val Leu Glu Leu Val Pro Ser Ala Gln Pro Gly Ala Ala
                245                 250                 255

Arg Arg Gln Gln Leu Pro Gly Asn Ala Thr Asp Trp Ile Trp Ala Gly
            260                 265                 270

Leu Asp Pro Asp Thr Asp Tyr Asp Val Ala Leu Val Pro Glu Ser Asn
        275                 280                 285

Val Arg Leu Leu Arg Pro Gln Ile Leu Arg Val Arg Thr Arg Pro Gly
    290                 295                 300

Glu Ala Gly Pro Gly Ala Ser Gly Pro Glu Ser Gly Ala Gly Pro Ala
305                 310                 315                 320

Pro Thr Gln Leu Ala Ala Leu Pro Ala Pro Glu Glu Ala Gly Pro Glu
                325                 330                 335

Arg Ile Val Ile Ser His Ala Arg Pro Arg Ser Leu Arg Val Ser Trp
            340                 345                 350

Ala Pro Ala Leu Gly Ser Ala Ala Ala Leu Gly Tyr His Val Gln Phe
        355                 360                 365

Gly Pro Leu Arg Gly Gly Glu Ala Gln Arg Val Glu Val Pro Ala Gly
    370                 375                 380

Arg Asn Cys Thr Thr Leu Gln Gly Leu Ala Pro Gly Thr Ala Tyr Leu
385                 390                 395                 400

Val Thr Val Thr Ala Ala Phe Arg Ser Gly Arg Glu Ser Ala Leu Ser
                405                 410                 415

Ala Lys Ala Cys Thr Pro Asp Gly Pro Arg Pro Arg Pro Arg Pro Val
            420                 425                 430

Pro Arg Ala Pro Thr Pro Gly Thr Ala Ser Arg Glu Pro
        435                 440                 445

<210>  604
<211>  21
<212>  DNA
<213>  artificial

<220>
<223>  Description of artificial sequence: Oligonucleotide

<400>  604
aaccaacctg aggatttcac g                                                21

<210>  605
<211>  21
<212>  DNA
<213>  artificial

<220>
<223>  Description of artificial sequence: Oligonucleotide

<400>  605
agacagctgt tcgtgagaag c                                                21

<210>  606
<211>  1999
<212>  DNA
<213>  Homo sapiens

<400>  606
cactctgtaa gttcaccgcc ggtcgggtcc ggccgccgcg ctgtccagct cctgagacct      60
tgctgtccgc cggtctgccg tctgcgcgcc tcacgctcct cagccctgga ccggggacaa     120
gtaaccctcg gtgacaagac caaagtgcac tgctgcccac acagttccta cctttctggc     180
ttcaattctt cagaagagtt tgccgtcctt tggggagaac gtgatttttg ttatctcagc     240
ccactgactt cattgatctc taatcttttt taattccttg ggccaacttt gttcgtgccc     300
ccacactgta gccagaagcc cgttggcgag ctctggcacc tgcaaaccac cccgtggaac     360
gagtgtttcc tctggctgag ggttggagag gaggtgtggt ctcagcaggc ggcccgtagc     420
ctcacagcca ggcctggtgg tgaggtcacc atgtccacca aggtgcccat ctatctgaag     480
cgtggcagtc gcaagggcaa gaaggagaag cttcgggacc tgctgtcctc ggacatgatc     540
agcccaccgc tgggggactt ccgccacacc attcatattg gcagtggcgg cggcagtgac     600
atgtttggcg acatctcctt cctgcagggc aagttccacc tcctgccggg gaccatggtg     660
gaggggcctg aagaagatgg caccttcgac ctccccttcc agttcacccg caccgccacc     720
gtgtgtgggc gggagctccc ggacggccca tcccctctgc tcaagaacgc catctccctc     780
ccggttatcg gtggacccca ggctctcacc ctgcccacag cccaggctcc acccaagccc     840
cctcgcctgc acctggagac ccctcagcct tccccacagg agggagggag tgtggacatc     900
tggaggattc cagagactgg ctcccccaac agtggactga ccccggagtc aggggccgag     960
gagcccttcc tgtccaatgc cagctccctg ctgtccctgc acgtggacct ggggccttcc    1020
atcctggatg atgtcctgca gatcatggat caggacctgg acagcatgca gatccccaca    1080
taggacacga ggctgcctag gctggggtcc caggtggggc ccagccagga ggtggggtgt    1140
ggacccggcc ctggcggcgg agtcagggtc ccaagatccc acctgtatgg tcgctggcca    1200
gtgattctcc ttctgagccg tgtttcccct ctccctccct ctccacgtgg gcagggcagg    1260
ccccatcgct ttcctctgat aaccacatgg acacatcctg aagtcagccc aggcgccctg    1320
agcatcttgg ggcacctgga ccccatcaca atactccttc ttccttcagg tccctgggtg    1380
aaggctttgc tgaaaccgac cccccttttc acgtcccttc tgcctctgcc ccgttggatg    1440
ccctgactgg gggcagggga agagacaggg cacagctggc cacagggctc agccactgag    1500
caggctgttc cgggcctttg gctttgcatc ctggacgggg agtgtcctgt cagggaccag    1560
atgtgtcctg cctcatccct agctccaatc ccttccccac gtgaccgggg attctggttg    1620
caataaaaca tgctgctgct ggtggcggag ctccctgtcc ctttgcccca ggtttcctcc    1680
cggaggcaga cagtctccca gagctgaggg cttgcctctg gagaccccag ccccagaggg    1740

ctttgtggag gacaggcctt gccctcaaga acgtcgtacc tgacgctgag cctgtcatga 1800

gaatgcaaca ggagcaaacc aagtgttgct gtgacattga ttcagatgtt tggcaagagg 1860

tggctgagca ctggggtggg cttggcactg tgccaagcct ggggccaatc cctgcccagt 1920

cagctggggt ctggtggggg acacccaaga ataaaagaat aaccacaaag tgtgcaaggg 1980

aaaaaaaaaa aaaaaaaaa 1999

<210> 607
<211> 210
<212> PRT
<213> Homo sapiens

<400> 607

Met Ser Thr Lys Val Pro Ile Tyr Leu Lys Arg Gly Ser Arg Lys Gly
1               5                   10                  15

Lys Lys Glu Lys Leu Arg Asp Leu Leu Ser Ser Asp Met Ile Ser Pro
            20                  25                  30

Pro Leu Gly Asp Phe Arg His Thr Ile His Ile Gly Ser Gly Gly Gly
        35                  40                  45

Ser Asp Met Phe Gly Asp Ile Ser Phe Leu Gln Gly Lys Phe His Leu
    50                  55                  60

Leu Pro Gly Thr Met Val Glu Gly Pro Glu Glu Asp Gly Thr Phe Asp
65                  70                  75                  80

Leu Pro Phe Gln Phe Thr Arg Thr Ala Thr Val Cys Gly Arg Glu Leu
                85                  90                  95

Pro Asp Gly Pro Ser Pro Leu Leu Lys Asn Ala Ile Ser Leu Pro Val
            100                 105                 110

Ile Gly Gly Pro Gln Ala Leu Thr Leu Pro Thr Ala Gln Ala Pro Pro
        115                 120                 125

Lys Pro Pro Arg Leu His Leu Glu Thr Pro Gln Pro Ser Pro Gln Glu
    130                 135                 140

Gly Gly Ser Val Asp Ile Trp Arg Ile Pro Glu Thr Gly Ser Pro Asn
145                 150                 155                 160

Ser Gly Leu Thr Pro Glu Ser Gly Ala Glu Glu Pro Phe Leu Ser Asn
                165                 170                 175

Ala Ser Ser Leu Leu Ser Leu His Val Asp Leu Gly Pro Ser Ile Leu
            180                 185                 190

Asp Asp Val Leu Gln Ile Met Asp Gln Asp Leu Asp Ser Met Gln Ile

        195                 200                 205

Pro Thr
    210

<210> 608
<211> 21
<212> DNA
<213> artificial

<220>
<223> Description of artificial sequence: Oligonucleotide

<400> 608
aacgtcgtac ctgacgctga g 21

<210> 609
<211> 21
<212> DNA
<213> artificial

<220>
<223> Description of artificial sequence: Oligonucleotide

<400> 609
ccaagtgttg ctgtgacatt g 21

<210> 610
<211> 986
<212> DNA
<213> Homo sapiens

<400> 610
attcaagatg atgttagaga gatgacagag tctaggttag ggagggcctg agtccttgta 60

gactctgagt acggtgctga gcagggaaga gacaggctct ggcttagggt ttagaaggaa 120

cacaggctac tgcgatgagg attgtctgaa ggggaacaaa ggccaagatg ttgtttgaag 180

ccgtgagact gagtgagatc atggaggaag tgaatgtaaa tagaaaaggg aagaagtctg 240

aagacggagc cctgagacac tccattgtaa actggaagat gaggaagagc cagcaaagga 300

gactgagaag gggcagccag tgaagatgga gccagggcta gagtaagagc cccttctggg 360

atgctgtgac ccccaagttt gaagactgct gataacccca atctacgaag actagctatg 420

gaacttccta cactgagaca actccagtgg aactctgata attatcctaa aataaggagg 480

cttcttcagt agccctcgaa atatgttcaa atacatgatt acatttatgt ccttaatatt 540

gctattagtt tctgatgtta atgtaaaagt tggggaaaaa gtggaaaagt taaagcagtg 600

caggttaatt caatgccaga gtaacttctc agagggtgta tattcagtgt gaacaatttt 660

caacagagaa atgtcaactt ctggccacaa cggcaaccag taaaatgact atttttactg 720

tcttatctat taatgaagag gagattgcat aatatagatg aaggagcata gtatttgcag 780

gtggaacgcc tagcagggct tgagtctcaa ctctgctgct tttactctaa ttgaccgaga 840

caagtcattt aaactaatag agcttcaatt ttctcatatc taatgtaaca taacaattca 900

cagcctttta ctttgtagtt atcgtgaaga tctaatcgca gtgaaatata tttatatatc 960

tgtctgccga taaaaaaaaa aaaaaa 986

<210> 611
<211> 21
<212> DNA
<213> artificial

<220>
<223> Description of artificial sequence: Oligonucleotide

<400> 611
gactctgagt acggtgctga g 21

<210> 612
<211> 21
<212> DNA
<213> artificial

<220>
<223> Description of artificial sequence: Oligonucleotide

<400> 612
tcttactcta gccctggctc c 21

<210> 613
<211> 899
<212> DNA
<213> Homo sapiens

<400> 613
cccgagcgcc ggccgggcca tgacccccgc tgctctgtct tgcaggctcg tcgccgcggc 60
cccccgagcc cgaccgccgc cgccaccacc accagcgccc gggcgggcct cgcgcgcctc 120
gggcgcggct ccgcagtgag cccaccaaga aggaagcggc ctgcagaggt gccgacatgg 180
ggcttaagat gtcctgcctg aaaggctttc aaatgtgtgt cagcagcagc agcagcagcc 240
acgacgaggc ccccgtcctg aacgacaagc acctggacgt gcccgacatc atcatcacgc 300
cccccacccc cacgggcatg atgctgccga gggacttggg gagcacagtc tggctggatg 360
agacagggtc gtgcccagat gatggagaaa tcgacccaga agcctgagga ggtgtcctgg 420
gtttggctgg ctggctcctg ctccagcggc ccggcttcag gtgtccgggg gcgtggctgc 480
ctggagcagg tgtgctgaat accctggatg ggaactgagc gaacccgggc ctccgctcag 540
agagacgtgg caggaccagc gaggaatcca gcctgtccac ttccagaaca gtgtttccca 600
ggccccgctg agtggaccgg acctctgaca cctccaggtt cttgctgact ccggcctggt 660
gaaagggagc gccatggtcc tggctgttgg ggtcccaggg agaggctctc ttctggacaa 720
acacaccctc ccagccccca gggctgtgca aacacatgcc cctgccataa gcaccaacaa 780
gaacttcttg caggtggagt ggctgttttt tataagttgt tttacagata cggaaacagt 840
ccaaaatggg atttataatt tcttttttgc attataaata aagatcctct gtaacaaaa 899

<210> 614
<211> 76
<212> PRT

<213>  Homo sapiens

<400>  614

Met Gly Leu Lys Met Ser Cys Leu Lys Gly Phe Gln Met Cys Val Ser
1               5                   10                  15

Ser Ser Ser Ser Ser His Asp Glu Ala Pro Val Leu Asn Asp Lys His
            20                  25                  30

Leu Asp Val Pro Asp Ile Ile Ile Thr Pro Pro Thr Pro Thr Gly Met
        35                  40                  45

Met Leu Pro Arg Asp Leu Gly Ser Thr Val Trp Leu Asp Glu Thr Gly
    50                  55                  60

Ser Cys Pro Asp Asp Gly Glu Ile Asp Pro Glu Ala
65                  70                  75

<210>  615
<211>  21
<212>  DNA
<213>  artificial

<220>
<223>  Description of artificial sequence: Oligonucleotide

<400>  615
tgtcctgcct gaaaggcttt c                                                21

<210>  616
<211>  21
<212>  DNA
<213>  artificial

<220>
<223>  Description of artificial sequence: Oligonucleotide

<400>  616
catccagggt attcagcaca c                                                21

<210>  617
<211>  432
<212>  DNA
<213>  Homo sapiens

<400>  617
ttatgtgcct gaagtcgcac agtgaataag ctaaaacacc tgcttttaac aatggtacca      60

tacaaccact actccattaa ctccacccac ctcctgcacc cctccccaca cacacaaaat     120

gaaccacgtt ctttgtatgg gcccaatgag ctgtcaagct gccctgtgtt catttcattt     180

ggaattgccc cctctggttc ctctgtatac tactgcttca tctctaaaga cagctcatcc     240

tcctccttca cccctgaatt tccagagcac ttcatctgct ccttcatcac aagtccagtt     300

ttctgccact agtctgaatt tcatgagaag atgccgattt ggttcctgtg ggtcctcagc     360

actattcagt acagtgcttg actcacagca ggcactcaga aaatactgga ggaaataaaa     420

caccaaagat at 432

<210> 618
<211> 21
<212> DNA
<213> artificial

<220>
<223> Description of artificial sequence: Oligonucleotide

<400> 618
ctgctccttc atcacaagtc c 21

<210> 619
<211> 24
<212> DNA
<213> artificial

<220>
<223> Description of artificial sequence: Oligonucleotide

<400> 619
gagatctcga gatctcgatc gtac 24

<210> 620
<211> 2575
<212> DNA
<213> Homo sapiens

<400> 620
gagaacgggg tagcccggcg cttacacatg tcacatgtgc tttttaagac ggccgggagc 60
gcctgcgagc tggatctggt ggaggatgct gcggcaggtg cttcgcagag ggctccagtc 120
gttctgccac aggctgggtt tgtgcgtgag ccggcacccg gtctttttcc tcaccgtgcc 180
cgcagtcctg acaatcacct tcggcctcag cgcgctcaac cgcttccagc ccgagggcga 240
cctggagcgc ctggtcgctc ccagccacag cctggccaag atcgagcgca gcctggccag 300
cagccttttc cccctggacc agtccaaaag ccagctctat tcggacttac acacccctgg 360
gaggtatggc agggtgatcc tcctctcccc aaccggggac aatattttgc tccaggctga 420
ggggatcctg cagacccacc gagccgtgct ggaaatgaag gatgggagga acagttttat 480
tggacaccaa ctgggcgggg tagtggaagt gccaaacagc aaagatcagc gggtcaagtc 540
agccagagcc attcaaatca cctactacct ccagacctat ggctctgcca cccaagacct 600
cataggggag aagtgggaga atgagttctg taagcttata aggaagctcc aggaggagca 660
tcaagaactc cagctctact ctttagcatc ctttagcctc tggagggact ttcataagac 720
cagcatcctg gccagaagca aggtcctggt gagcctcgtg ctgatcctga ccacagccac 780
cctctccagc tccatgaagg actgcttgcg cagtaagccc ttcctgggcc tcctgggggt 840
gctcacagta tgcatctcca tcatcacagc agcagggatc ttcttcatca ccgatggaaa 900
gtacaactcc accctgctgg gaatcccgtt cttcgccatg ggtcatggaa ctaaaggagt 960
gtttgagctt ctgtccggat ggcggagaac caaagagaac ttgcccttca aagacaggat 1020
agcagatgcc tattctgatg tgatggtcac ctataccatg accagctccc tgtacttcat 1080

cacttttggc atgggtgcca gcccattcac aaacatagag gctgtgaagg tcttctgtca 1140
aaacatgtgt gtctctattc tgttgaacta cttctacatt ttctccttct ttggctcctg 1200
tctggtcttt gctggccaac tagagcaaaa ccgctaccac agcatctttt gctgtaagat 1260
cccttctgca gaatacctgg atcgcaaacc tgtgtggttc cagacagtga tgagtgatgg 1320
gcatcaacag acgtcccatc atgagacgaa cccctaccag caccacttca ttcagcactt 1380
cctccgtgaa cattataatg aatggattac caatatatat gtgaagccat ttgttgtcat 1440
cctctatctc atttatgcct ccttctcctt catggggtgc ttacagatca gtgacggagc 1500
caacatcatc aatctactag ccagtgattc gccaagtgtt tcctatgcca tggttcagca 1560
gaaatatttc agcaactata gccctgtgat aggattctac gtctatgagc ccctagagta 1620
ctggaacagc agcgtccagg atgacctaag aagactctgt agtggattca ctgcagtgtc 1680
ctgggtggag cagtactacc agttcctgaa agtcagcaac gtcagtgcca ataacaaaag 1740
tgacttcatc agtgtcctgc aaagctcatt tttaaaaaag ccagaattcc agcattttcg 1800
aaatgatatc atcttctcca aggcagggga tgaaagcaat atcattgctt ctcgcttgta 1860
tctggtggcc aggactagca gagacaagca gaaagaaatc acagaagtgt tggaaaagct 1920
gaggccccta tccctctcaa agagcatccg attcatcgtg ttcaacccct cctttgtctt 1980
catggaccat tacagcttgt ctgtcacagt gcctgttctg attgcaggct ttggtgttct 2040
cctggtgtta atcctgactt ttttcctagt gatccaccct ctgggaaact tctggctaat 2100
tcttagcgtc acctcaattg agctgggcgt tctgggctta atgacattat ggaacgtcga 2160
catggattgc atttctatct tgtgccttat ctacaccttg aatttcgcca ttgaccactg 2220
tgcaccactg cttttcacat ttgtattagc aactgagcac acccgaacac aatgtataaa 2280
aagctccttg caagaccatg ggacagccat tttgcaaaat gttacttctt ttcttattgg 2340
gttagtcccc cttctatttg tgccttcgaa cctgaccttc acactgttca aatgcttgct 2400
gctcactggg ggttgcacac ttctgcactg ttttgttatt ttacctgtgt tcctaacgtt 2460
tttcccccct tccaaaaagc accacaagaa aaagaaacgt gccaagcgaa aggagagaga 2520
ggaaattgaa tgcatagaaa ttcaagagaa cccggatcac gtcaccacag tatga 2575

<210> 621
<211> 829
<212> PRT
<213> Homo sapiens

<400> 621

Met Leu Arg Gln Val Leu Arg Arg Gly Leu Gln Ser Phe Cys His Arg
1               5                   10                  15

Leu Gly Leu Cys Val Ser Arg His Pro Val Phe Phe Leu Thr Val Pro
            20                  25                  30

Ala Val Leu Thr Ile Thr Phe Gly Leu Ser Ala Leu Asn Arg Phe Gln

35                  40                  45

Pro Glu Gly Asp Leu Glu Arg Leu Val Ala Pro Ser His Ser Leu Ala
    50                  55                  60

Lys Ile Glu Arg Ser Leu Ala Ser Ser Leu Phe Pro Leu Asp Gln Ser
65                  70                  75                  80

Lys Ser Gln Leu Tyr Ser Asp Leu His Thr Pro Gly Arg Tyr Gly Arg
                85                  90                  95

Val Ile Leu Leu Ser Pro Thr Gly Asp Asn Ile Leu Leu Gln Ala Glu
            100                 105                 110

Gly Ile Leu Gln Thr His Arg Ala Val Leu Glu Met Lys Asp Gly Arg
        115                 120                 125

Asn Ser Phe Ile Gly His Gln Leu Gly Gly Val Val Glu Val Pro Asn
    130                 135                 140

Ser Lys Asp Gln Arg Val Lys Ser Ala Arg Ala Ile Gln Ile Thr Tyr
145                 150                 155                 160

Tyr Leu Gln Thr Tyr Gly Ser Ala Thr Gln Asp Leu Ile Gly Glu Lys
                165                 170                 175

Trp Glu Asn Glu Phe Cys Lys Leu Ile Arg Lys Leu Gln Glu Glu His
            180                 185                 190

Gln Glu Leu Gln Leu Tyr Ser Leu Ala Ser Phe Ser Leu Trp Arg Asp
        195                 200                 205

Phe His Lys Thr Ser Ile Leu Ala Arg Ser Lys Val Leu Val Ser Leu
    210                 215                 220

Val Leu Ile Leu Thr Thr Ala Thr Leu Ser Ser Ser Met Lys Asp Cys
225                 230                 235                 240

Leu Arg Ser Lys Pro Phe Leu Gly Leu Leu Gly Val Leu Thr Val Cys
                245                 250                 255

Ile Ser Ile Ile Thr Ala Ala Gly Ile Phe Phe Ile Thr Asp Gly Lys
            260                 265                 270

Tyr Asn Ser Thr Leu Leu Gly Ile Pro Phe Phe Ala Met Gly His Gly
        275                 280                 285

Thr Lys Gly Val Phe Glu Leu Leu Ser Gly Trp Arg Arg Thr Lys Glu
    290                 295                 300

Asn Leu Pro Phe Lys Asp Arg Ile Ala Asp Ala Tyr Ser Asp Val Met

305                 310                 315                 320

Val Thr Tyr Thr Met Thr Ser Ser Leu Tyr Phe Ile Thr Phe Gly Met
                325                 330                 335

Gly Ala Ser Pro Phe Thr Asn Ile Glu Ala Val Lys Val Phe Cys Gln
            340                 345                 350

Asn Met Cys Val Ser Ile Leu Leu Asn Tyr Phe Tyr Ile Phe Ser Phe
        355                 360                 365

Phe Gly Ser Cys Leu Val Phe Ala Gly Gln Leu Glu Gln Asn Arg Tyr
    370                 375                 380

His Ser Ile Phe Cys Cys Lys Ile Pro Ser Ala Glu Tyr Leu Asp Arg
385                 390                 395                 400

Lys Pro Val Trp Phe Gln Thr Val Met Ser Asp Gly His Gln Gln Thr
                405                 410                 415

Ser His His Glu Thr Asn Pro Tyr Gln His His Phe Ile Gln His Phe
            420                 425                 430

Leu Arg Glu His Tyr Asn Glu Trp Ile Thr Asn Ile Tyr Val Lys Pro
        435                 440                 445

Phe Val Val Ile Leu Tyr Leu Ile Tyr Ala Ser Phe Ser Phe Met Gly
    450                 455                 460

Cys Leu Gln Ile Ser Asp Gly Ala Asn Ile Ile Asn Leu Leu Ala Ser
465                 470                 475                 480

Asp Ser Pro Ser Val Ser Tyr Ala Met Val Gln Gln Lys Tyr Phe Ser
                485                 490                 495

Asn Tyr Ser Pro Val Ile Gly Phe Tyr Val Tyr Glu Pro Leu Glu Tyr
            500                 505                 510

Trp Asn Ser Ser Val Gln Asp Asp Leu Arg Arg Leu Cys Ser Gly Phe
        515                 520                 525

Thr Ala Val Ser Trp Val Glu Gln Tyr Tyr Gln Phe Leu Lys Val Ser
    530                 535                 540

Asn Val Ser Ala Asn Asn Lys Ser Asp Phe Ile Ser Val Leu Gln Ser
545                 550                 555                 560

Ser Phe Leu Lys Lys Pro Glu Phe Gln His Phe Arg Asn Asp Ile Ile
                565                 570                 575

Phe Ser Lys Ala Gly Asp Glu Ser Asn Ile Ile Ala Ser Arg Leu Tyr

            580                 585                 590

Leu Val Ala Arg Thr Ser Arg Asp Lys Gln Lys Glu Ile Thr Glu Val
        595                 600                 605

Leu Glu Lys Leu Arg Pro Leu Ser Leu Ser Lys Ser Ile Arg Phe Ile
    610                 615                 620

Val Phe Asn Pro Ser Phe Val Phe Met Asp His Tyr Ser Leu Ser Val
625                 630                 635                 640

Thr Val Pro Val Leu Ile Ala Gly Phe Gly Val Leu Leu Val Leu Ile
                645                 650                 655

Leu Thr Phe Phe Leu Val Ile His Pro Leu Gly Asn Phe Trp Leu Ile
            660                 665                 670

Leu Ser Val Thr Ser Ile Glu Leu Gly Val Leu Gly Leu Met Thr Leu
        675                 680                 685

Trp Asn Val Asp Met Asp Cys Ile Ser Ile Leu Cys Leu Ile Tyr Thr
    690                 695                 700

Leu Asn Phe Ala Ile Asp His Cys Ala Pro Leu Leu Phe Thr Phe Val
705                 710                 715                 720

Leu Ala Thr Glu His Thr Arg Thr Gln Cys Ile Lys Ser Ser Leu Gln
                725                 730                 735

Asp His Gly Thr Ala Ile Leu Gln Asn Val Thr Ser Phe Leu Ile Gly
            740                 745                 750

Leu Val Pro Leu Leu Phe Val Pro Ser Asn Leu Thr Phe Thr Leu Phe
        755                 760                 765

Lys Cys Leu Leu Leu Thr Gly Gly Cys Thr Leu Leu His Cys Phe Val
    770                 775                 780

Ile Leu Pro Val Phe Leu Thr Phe Phe Pro Pro Ser Lys Lys His His
785                 790                 795                 800

Lys Lys Lys Lys Arg Ala Lys Arg Lys Glu Arg Glu Glu Ile Glu Cys
                805                 810                 815

Ile Glu Ile Gln Glu Asn Pro Asp His Val Thr Thr Val
            820                 825

<210> 622
<211> 21
<212> DNA
<213> artificial

<220>
<223>  Description of artificial sequence: Oligonucleotide

<400>  622
cagctctact ctttagcatc c                                                21


<210>  623
<211>  21
<212>  DNA
<213>  artificial

<220>
<223>  Description of artificial sequence: Oligonucleotide

<400>  623
cctttagttc catgacccat g                                                21


<210>  624
<211>  6035
<212>  DNA
<213>  Homo sapiens

<400>  624
ggcggcggcg gcggcggccc cgggcgctga gcgggtgccc ggcgcggaga gcggcgagcg      60

cagccatgcc ccaggccgcc tccggggcag cagcagcggc ggccggggcc gaggcgcggg     120

ccgggggcgc cggggggccg gcggcggccc gggcgggacg atgaagcggc agaacgtgcg     180

cacgctggcg ctcatcgtgt gcaccttcac ctacctgctg gtgggcgccg cggtcttcga     240

cgcgctggag tcggagcccg agctgatcga gcggcagcgg ctggagctgc ggcagcagga     300

gctgcgggcg cgctacaacc tcagccaggg cggctacgag gagctggagc gcgtcgtgct     360

gcgcctcaag ccgcacaagg ccggcgtgca gtggcgcttc gccggctcct tctacttcgc     420

catcaccgtc atcaccacca tcggctacgg gcacgcggcg cccagcacgg atggcggcaa     480

ggtgttctgc atgttctacg cgctgctggg catcccgctc acgctcgtca tgttccagag     540

cctgggcgag cgcatcaaca ccttggtgag gtacctgctg caccgcgcca agaaggggct     600

gggcatctcg tggccttcgc ttcgtctcat ccttacgggc ctcacggtca tcggcgcctt     660

cctcaacctc gtggtgctgc gcttcatgac catgaacgcc gaggacgaga agcgcgacgc     720

cgagcaccgc gcgctgctca cgcgcaacgg gcaggcgggc ggcggcggag ggagtggcag     780

cgcgcacact acggacaccg cctcatccac ggcggcagcg ggcggcggcg gcttccgcaa     840

cgtctacgcg gaggtgctgc acttccagtc catgtgctcg tgcctgtggt acaagagccg     900

cgagaagctg cagtactcca tccccatgat catcccgcgg gacctctcca cgtccgacac     960

gtgcgtggag cagagccact cgtcgccggg agggggcggc cgctacagcg acacgccctc    1020

gcgacgctgc ctgtgcagcg gggcgccacg ctccgccatc agctcggtgt ccacgggtct    1080

gcacagcctg tccaccttcc gcggactcat gaagcgcagg agctccgtgt gactgccccg    1140

aggggcctgg agcacctggg ggcgcgggcg ggggacccct gctgggaggc caggagactg    1200

cccctgctgc cttctgccca gtgggacccc gcacaacatc cctcaccact ctcccccagc    1260

acccccatct ccgactgtgc ctgcttgcac cagccggcag gaggccgggc tctgaggacc    1320

cctggggccc ccatcggagc cctgcaaatt ccgagaaatg tgaaacttgg tggggtcagg 1380
gaggaaaggc agaagctggg agcctccctt ccctttgaaa atctaagaag ctcccagtcc 1440
tcagagaccc tgctggtacc cagaccccca ccttcggagg ggacttcatg ttccgtgtac 1500
gtttgcatct ctatttatac ctctgtcctg ctaggtctcc caccttccct tggttccaaa 1560
agccagggtg tctttgtcca agtcacccct actcagcccc actccctttc ctcatcccca 1620
gctgtgtctc ccaacctccc ttcgtgttgt tttgcatggc tttgcagtta tggagaaagt 1680
ggaaacccag cagtccctaa agctggtccc cagaaagcag gacagaaaga aggagggaca 1740
ggcaggcagc aggaggggcg agctgggagg caggaggcag cggcctgtca gtctgcagaa 1800
tggtcgcact ggaggttcaa gctaactggc ctccagccac attctcatag caggtaggac 1860
ttcagccttc cagacactgc ccttagaatc tggaacagaa gacttcagac tcaccataat 1920
tgctgataat tacctactct taaatttgtc gagtgatttt tagcctctga aaactctatg 1980
ctggccactg attcctttga gtctcacaaa accctactta ggtcatcagg gcaggagttc 2040
tcactcccat tttacagatg agaatactga ggcctggaca ggtgaagtga ccagagagca 2100
aaaggcaaag gggtgggggc tgggtgcagt ggctcacacc tgtattccca acacttttgg 2160
aggctgaggt tagaggattg cttgagccca ggaattcgag accagcctag gcgacatagt 2220
gagaccccat ctctacaaaa aataaaaaat ttaccaggtg tggtggcacg tgcctgggag 2280
tcccagcgac ttgggaggct gaggtgggag gattgtttga gcctgggagg tcaaggctgt 2340
agtgagccct gattacacca ctgtactcca gcctgggtga cagggcaaga ccctgtctca 2400
aaaaaaaaaa aaaaatggca aagggagaca agagcccagc ctacttgttc ctagccaaag 2460
tgttctttcc ttccagcttg gcctgctctt aaaagcaaag ctcctgcagt gtacatcctg 2520
gcattgtgtg gctacctggg ttttaaacca gaatcagaag tcccgggtca gagggcactg 2580
ctgaggctca gcctcttctc ttcttggcca ggaggcagca gctctgaatg ggcccctgag 2640
gctgcacagg ggcctttgtc actggggtgc atgcttacaa acagtgcagt tcttggcacc 2700
gaggtaagca gggctgggtc tcatggcaga aaggccagga tctggggctc taggaatttg 2760
ggaattgggc agagtggcca agaaagctgg caggcatatc ctatgggaca tcacacctgg 2820
caccattgtc attgttggtg cctgtgtccc aagtagctag tgataagctg aggctgcagc 2880
aagaaacacc cttcccaggt gggggagttt ggaccagagg tgccctctgc ccaccacacc 2940
tgcaacccag aagcccagat ggaacgcagc tgatgaaggt gatgcttgag gctcactttt 3000
ggggccccac agctggagcc ggtatagtga ctgggacaac atcaaggggt ggatgagggg 3060
cctctcctcc cgcaacactg ccttcccatg ctgttcccct gccagctcct taacactgcc 3120
gaccaaggcc agacctggca ttcaggaaag ttggagggca gcacccatag ggtggccagc 3180
ctcaggcccc accccagctg tgtcctctag tctctgggga cccctggggg gaagaagtct 3240
accctgcttg tgagtcccgt ctcagtgtgg aggaactggc tgcacgtggg acctgaaggt 3300
gccctctgtg tttatgttgg gggggggggg gcagtgctgg ctgcctctgt cctgtgtgtg 3360

accctgccct cgaagggtcc tgtcctgtca gtcccgaggg agccacaacc aaagctgcgg 3420
agagaaggtg gggaacggtg cggagtggcc gtggggcaca gcgtggcaga ctgttcagtc 3480
tctgctgggt ctttcctagg gacctggaag gccagtgttg cttccccctc actccctttc 3540
actgcaggca gcctctctcc ttccccaatg ccttatgcct gggcacactg ccacagaata 3600
tgcaatatgt gtgggtgacg atgccctcac gaccacaccc ccaccccggg cagcccccgg 3660
actccaaagg tcgtggctgc cacagcctcc ctcagctctt cctgcctatc tgtcttcaca 3720
ctgagaatgg cgcccaataa atgctatcca cggagaccag gctcaggctc cagctgcctc 3780
tgtcatcgta tgcccttgct gctgccaggg aggggccatc tcccaccccc tcccctgccg 3840
gggtctacaa acatacctag ctgctgggtg ccgtggctca cacctataat cacagcacta 3900
ggcgggcaga tcacctgagg tcagaagttc aagaccagcc tggccaacat ggtaaaaccc 3960
cgtctctact aaaaatacaa aaattagctg agcgtggtgg cgcctgtctg tagtcccagc 4020
tactcggcta ctcaggaggc tgacgcacga gaatcgcttg aacccgggag gcggaggttg 4080
cagtgagctg agatcgcgcc actgcactcc agcctgagcg atagagtgag accctgtcta 4140
aaaaaaacaa taataataaa ataaaataac atacctagct gactcgccat gggctcgctg 4200
gcctgtgggc gacactggct tcccttttgg gatttcccag aagatccaga ttttcttaag 4260
tccccttgga acagactaag aaagaaacac cttagaaatc acctggtcct attgtccccc 4320
cgtacatgag taactgaggc ccacagagag caaatcgcct gcctgagtca cacagcagtg 4380
agtggcagac ctaggctagg aactagaact ggggattgct attccagtgc tccccatcct 4440
cacacagcct gtggagtccg cctggacaca ccccagctga cagtggtacc tcccagtcag 4500
ccaggagaat ggattccttc tcctgcagta ggggccccct ggctgagtgg cctgattgac 4560
taaaacatat gtctttgaag gagagtgcat cacaagcacc tttctttggg gtagattttt 4620
ctctgggtct agagggacac ctcaggcttg ggactgggcc tcagaaccta ggacagaccc 4680
tgagagcaga cccaccttat ccatctggtg ccagctcccc aggtcagcta cagcaacccc 4740
cgaacttcat agagtacaat ccacagtaat agcacacagc tctgtaccta tctagctcca 4800
tgcctatcta tctgcctacc tttcacaaaa taattcttag caaccctgct acagccaatg 4860
attctaatac gttctgttct attacatgtt ataaaatgct ggtcacgatc cactaaattg 4920
atgtctctac ctgctaatgg tttaatacct gcagattgaa atatactgga gaaataaaga 4980
gagtaggagt agggacactt tctcccagtg cccacaccgc ccctcgttac ccgcataggt 5040
caactgaaag atacagagag ggaagctttg atggggggtt cagagttcaa aggaagaaat 5100
gatggcacct gcactccctg cccccagagg caggacacag ccagccctcc tgtgacagca 5160
ctcctggcag ctccttgttg gcctgcagcc cttagttgcc attgactcac ccactcctaa 5220
ggccaccaca tcaaaatctg aggcttactg ccctggccca cctgcctctg tctttcttaa 5280
aacagctaaa tgcaacgata gcagaaatta gcttgttttt gaggttggca atgaccagtt 5340
caactcttat tttcttaagc agtgcttgca ggacataaat gtgatgacac ttgccctcct 5400

ttctttatcg cctggggcag actttacaaa cagacctggg aggagtcccc taaggggctg 5460

catttatccc catctcccta ggggtgatca gcattgtgac agctgggcag agcagtggtg 5520

aactgcaccc atgtccctgc tcacatctcc taagatctca gaattgcctg aggttctagc 5580

gtgggctcct tctctccaga tgatgccatc cccacccccc tcatttccac acagcatctg 5640

aggcatcctg cactaaaaga tatatgtaca gcaaaacaaa aatagaaaac cagcacagca 5700

gagtggaggt ggggtataaa tatacccaga tccccgctga tttggttact cggggtgagc 5760

atcagatgga aatagaagtt tccgggggcc aagagagaaa gagggatgta acgacaattc 5820

ttttcaaaac gtgtcccatg gtatgcctcg tggaaaaaat ggttcgttgg tcaaatgaat 5880

ttgggaaaat gctgtcaata tcaccgactc atggagcttc gcaaggcatc ttagcttaat 5940

aaaggttatg aaaagtcttg cagcaaagat gctgtttacc ccacttaatc cagcactgcc 6000

caaactcatt ccaaatacca gagcctctgt ttgca 6035

<210> 625
<211> 323
<212> PRT
<213> Homo sapiens

<400> 625

Met Lys Arg Gln Asn Val Arg Thr Leu Ala Leu Ile Val Cys Thr Phe
1               5                   10                  15

Thr Tyr Leu Leu Val Gly Ala Ala Val Phe Asp Ala Leu Glu Ser Glu
            20                  25                  30

Pro Glu Leu Ile Glu Arg Gln Arg Leu Glu Leu Arg Gln Gln Glu Leu
        35                  40                  45

Arg Ala Arg Tyr Asn Leu Ser Gln Gly Gly Tyr Glu Glu Leu Glu Arg
    50                  55                  60

Val Val Leu Arg Leu Lys Pro His Lys Ala Gly Val Gln Trp Arg Phe
65                  70                  75                  80

Ala Gly Ser Phe Tyr Phe Ala Ile Thr Val Ile Thr Thr Ile Gly Tyr
                85                  90                  95

Gly His Ala Ala Pro Ser Thr Asp Gly Gly Lys Val Phe Cys Met Phe
            100                 105                 110

Tyr Ala Leu Leu Gly Ile Pro Leu Thr Leu Val Met Phe Gln Ser Leu
        115                 120                 125

Gly Glu Arg Ile Asn Thr Leu Val Arg Tyr Leu Leu His Arg Ala Lys
    130                 135                 140

Lys Gly Leu Gly Ile Ser Trp Pro Ser Leu Arg Leu Ile Leu Thr Gly

145                 150                 155                 160

Leu Thr Val Ile Gly Ala Phe Leu Asn Leu Val Val Leu Arg Phe Met
                165                 170                 175

Thr Met Asn Ala Glu Asp Glu Lys Arg Asp Ala Glu His Arg Ala Leu
            180                 185                 190

Leu Thr Arg Asn Gly Gln Ala Gly Gly Gly Gly Gly Ser Gly Ser Ala
        195                 200                 205

His Thr Thr Asp Thr Ala Ser Ser Thr Ala Ala Ala Gly Gly Gly Gly
    210                 215                 220

Phe Arg Asn Val Tyr Ala Glu Val Leu His Phe Gln Ser Met Cys Ser
225                 230                 235                 240

Cys Leu Trp Tyr Lys Ser Arg Glu Lys Leu Gln Tyr Ser Ile Pro Met
                245                 250                 255

Ile Ile Pro Arg Asp Leu Ser Thr Ser Asp Thr Cys Val Glu Gln Ser
            260                 265                 270

His Ser Ser Pro Gly Gly Gly Gly Arg Tyr Ser Asp Thr Pro Ser Arg
        275                 280                 285

Arg Cys Leu Cys Ser Gly Ala Pro Arg Ser Ala Ile Ser Ser Val Ser
    290                 295                 300

Thr Gly Leu His Ser Leu Ser Thr Phe Arg Gly Leu Met Lys Arg Arg
305                 310                 315                 320

Ser Ser Val

<210>  626
<211>  21
<212>  DNA
<213>  artificial

<220>
<223>  Description of artificial sequence: Oligonucleotide

<400>  626
agactttaca aacagacctg g                                              21

<210>  627
<211>  23
<212>  DNA
<213>  artificial

<220>
<223>  Description of artificial sequence: Oligonucleotide

<400>  627

gcttgcagga cataaatgtg atg                                                23

<210>  628
<211>  21
<212>  DNA
<213>  artificial

<220>
<223>  Description of artificial sequence: Oligonucleotide

<400>  628
tgacactggc aaaacaatgc a                                                  21

<210>  629
<211>  21
<212>  DNA
<213>  artificial

<220>
<223>  Description of artificial sequence: Oligonucleotide

<400>  629
ggtccttttc accagcaagc t                                                  21

<210>  630
<211>  21
<212>  DNA
<213>  artificial

<220>
<223>  Description of artificial sequence: siRNA

<400>  630
ccacagaagg uaccaguuau u                                                  21

<210>  631
<211>  21
<212>  DNA
<213>  artificial

<220>
<223>  Description of artificial sequence: siRNA

<400>  631
uaacugguac cuucuguggu u                                                  21

<210>  632
<211>  21
<212>  DNA
<213>  artificial

<220>
<223>  Description of artificial sequence: siRNA

<400>  632
cagcaagacu cccucuaaau u                                                  21

<210>  633
<211>  21
<212>  DNA
<213>  artificial

<220>
<223>  Description of artificial sequence: siRNA

<400>  633
uuuagaggga gucuugcugu u                                                21


<210>  634
<211>  1791
<212>  DNA
<213>  Homo sapiens

<400>  634
atgatgacca tgactacgat ggctgacggc ttggaaggcc aggactcgtc caaatccgcc      60

ttcatggagt tcgggcagca gcagcagcag cagcactccc cggccatggc aggcgcgcac     120

taccctctgc actgcctgca ctcggcggcg gcggcggcag cggccggctc gcaccaccac     180

caccaccacc agcaccacca ccacggctcg ccctacgcgt cgggcggagg gaactcctac     240

aaccaccgct cgctcgccgc ctacccctac atgagccact cgcagcacag cccttacctc     300

cagtcctacc acaacagcag cgcagccgcc cagacgcgag gggacgacac agatcaacaa     360

aaaactacag tgattgaaaa cggggaaatc aggttcaatg gaaaagggaa aaagattcgg     420

aagcctcgga ccatttattc cagcctgcag ctccaggctt taaaccatcg ctttcagcag     480

acacagtatc tggcccttcc agagagagcc gaactggcag cttccttagg actgacacaa     540

acacaggtga agatatggtt tcagaacaaa cgctctaagt ttaagaaact gctgaagcag     600

ggcagtaatc ctcatgagag cgaccccctc cagggctcgg cggccctgtc gccacgctcg     660

ccagcgctgc ctccagtctg ggacgtttct gcctcggcca agggtgtcag tatgcccccc     720

aacagctaca tgcctggcta ttctcactgg tactcctctc cacaccagga cacgatgcag     780

agaccacaga tgatgtgagt tgcccaaggg aacaccctag ggaaacgtct gaacaaggaa     840

aagaggatcc gggacctgct tgtatctgcg aaaaggagcc aaaggagcag gcttaggaga     900

gctcataagt gtggcaagaa gccgactagg ctcattctct ctccctctct ctctctctcc     960

ctctcctttc tttttacttc ttcctttcct ccattccttc tttctttcct tttcctttct    1020

acctttcttt tctttttgcc tttcaccttt tttctcattt accttctctc ttgagcaacg    1080

tcagtaattg atcttgcatc tcagagagag agaaagagca tgtgtgagag agaaactggt    1140

ttctatgcca gcactcctga aaccccttac tgtaaggata ttttctctta ccccttggga    1200

tccaggctct gagtctcttc tctttgggag tatccatcaa aatgactttt tttaaaaaca    1260

gattttcccc caaccagaag aatctgcaca aacttggcag cgtttttact tgtttaatga    1320

gtttaagaca ttacatggtg aaagagaagc attttggact cctgcatttt tatttaccat    1380

tcccagactg acgagaaaaa gaaaattcct cacataacag cccttctcta aagaaaaagg    1440

aaaaagtggc tgtaagatta gaacattgct acaaagggaa tgctgcatgt tttatcaaaa    1500

tgcaatgacc aggaatgatg gttgattaaa aaaaaacaaa acaaaaacca ctctttcccc    1560

accccacccc cccaaaccct gaactggaat caggaaagac ggaggaaaca atcaaaatca    1620

ccattctatt gctttgacac ctttactagg tgaattggtg gcattcacaa agctaatagg    1680

gacgtttata tcaagaaaca tttctgtata tattgttgaa ttttagttgt acatatactt 1740

tgtatgtttt tgtcttcttt catatatgga gtaaaagcca caaaacgctg a 1791

<210> 635
<211> 265
<212> PRT
<213> Homo sapiens

<400> 635

Met Met Thr Met Thr Thr Met Ala Asp Gly Leu Glu Gly Gln Asp Ser
1               5                   10                  15

Ser Lys Ser Ala Phe Met Glu Phe Gly Gln Gln Gln Gln Gln Gln His
            20                  25                  30

Ser Pro Ala Met Ala Gly Ala His Tyr Pro Leu His Cys Leu His Ser
        35                  40                  45

Ala Ala Ala Ala Ala Ala Ala Gly Ser His His His His His His Gln
    50                  55                  60

His His His His Gly Ser Pro Tyr Ala Ser Gly Gly Gly Asn Ser Tyr
65                  70                  75                  80

Asn His Arg Ser Leu Ala Ala Tyr Pro Tyr Met Ser His Ser Gln His
                85                  90                  95

Ser Pro Tyr Leu Gln Ser Tyr His Asn Ser Ser Ala Ala Ala Gln Thr
            100                 105                 110

Arg Gly Asp Asp Thr Asp Gln Gln Lys Thr Thr Val Ile Glu Asn Gly
        115                 120                 125

Glu Ile Arg Phe Asn Gly Lys Gly Lys Lys Ile Arg Lys Pro Arg Thr
    130                 135                 140

Ile Tyr Ser Ser Leu Gln Leu Gln Ala Leu Asn His Arg Phe Gln Gln
145                 150                 155                 160

Thr Gln Tyr Leu Ala Leu Pro Glu Arg Ala Glu Leu Ala Ala Ser Leu
                165                 170                 175

Gly Leu Thr Gln Thr Gln Val Lys Ile Trp Phe Gln Asn Lys Arg Ser
            180                 185                 190

Lys Phe Lys Lys Leu Leu Lys Gln Gly Ser Asn Pro His Glu Ser Asp
        195                 200                 205

Pro Leu Gln Gly Ser Ala Ala Leu Ser Pro Arg Ser Pro Ala Leu Pro
    210                 215                 220

Pro Val Trp Asp Val Ser Ala Ser Ala Lys Gly Val Ser Met Pro Pro
225                 230                 235                 240

Asn Ser Tyr Met Pro Gly Tyr Ser His Trp Tyr Ser Ser Pro His Gln
                245                 250                 255

Asp Thr Met Gln Arg Pro Gln Met Met
            260                 265

<210>  636
<211>  21
<212>  DNA
<213>  artificial

<220>
<223>  Description of artificial sequence: Oligonucleotide

<400>  636
gctgtaagat tagaacattg c                                                21

<210>  637
<211>  22
<212>  DNA
<213>  artificial

<220>
<223>  Description of artificial sequence: Oligonucleotide

<400>  637
caattcacct agtaaaggtg tc                                               22

<210>  638
<211>  1600
<212>  DNA
<213>  Homo sapiens

<400>  638
gaattccggg ctgtagagcc ttgcgcgcgc agtggggatg gaacgttgct aggcttagcg      60

ggtctggctg ctgggggccc gagcagcacg ctcggagccg ccgcgcgcca aagcgggaat     120

ctgggaggcg agcagctctg cagttaatgc acgtatttta aactcccggg cctgcggacg     180

ctatgcacag gaagcacctc caggagattc cagacctgag tagcaacgtt gccaccagct     240

tcacgtgggg atgggattcc agcaagactt ctgaactgct gtcaggcatg ggggtctccg      300

ccctggagaa agaggagccc gacagtgaga acatccccca ggaactgctc tcaaacctgg     360

gccacccgga gagcccccca cggaaacggc tgaagagcaa agggagtgac aaagactttg     420

tgattgtccg caggcctaag ctaaatcgag agaactttcc aggtgtttca tgggactccc     480

ttccggatga gctgctcttg ggaatctttt cctgtctgtg cctccctgag ctgctaaagg     540

tctctggtgt ttgtaagagg tggtatcgcc tagcgtctga tgagtctcta tggcagacct     600

tagacctcac aggtaaaaat ctgcacccgg atgtgactgg tcggttgctg tctcaagggg     660

tgattgcctt ccgctgccca cgatcattta tggaccaacc attggctgaa catttcagcc     720

cttttcgtgt acagcacatg gacctatcga actcagttat agaagtgtcc accctccacg     780

gcatactgtc tcagtgttcc aagttgcaga atctaagcct ggaaggcctg cggctttcgg 840
atcccattgt caatactctc gcaaaaaact caaatttagt gcgacttaac ctttctgggt 900
gttctggatt ctctgaattt gccctgcaga ctttgctaag cagctgttcc agactggatg 960
agctgaacct ctcctggtgt tttgatttca ctgaaaagca tgtacaggtg gctgttgcgc 1020
atgtgtcaga gaccatcacc cagctgaatc ttagcggcta cagaaagaat ctccagaaat 1080
cagatctctc tactttagtt agaagatgcc ccaatcttgt ccatctagac ttaagtgata 1140
gtgtcatgct aaagaatgac tgctttcagg aatttttcca gctcaactac ctccaacacc 1200
tatcactcag tcggtgctat gatataatac ctgaaacttt acttgaactt ggagaaattc 1260
ccacactaaa aacactacaa gtttttggaa tcgtgccaga tggtaccctt caactgttaa 1320
aggaagccct tcctcatcta cagattaatt gctcccattt caccaccatt gccaggccaa 1380
ctattggcaa caaaaagaac caggagatat ggggcatcaa atgccgactg acactgcaaa 1440
agcccagttg tctatgaagt atttattgca ggatggtgtc tcttctttag aacagggaaa 1500
ataggcagga agcccaattg ctggagtact tagctagttt tattcttggt tttccctttg 1560
ccttcattct gcaagtatac tagggagcca tttgagaggg 1600

<210> 639
<211> 424
<212> PRT
<213> Homo sapiens

<400> 639

Met His Arg Lys His Leu Gln Glu Ile Pro Asp Leu Ser Ser Asn Val
1               5                   10                  15

Ala Thr Ser Phe Thr Trp Gly Trp Asp Ser Ser Lys Thr Ser Glu Leu
            20                  25                  30

Leu Ser Gly Met Gly Val Ser Ala Leu Glu Lys Glu Glu Pro Asp Ser
        35                  40                  45

Glu Asn Ile Pro Gln Glu Leu Leu Ser Asn Leu Gly His Pro Glu Ser
    50                  55                  60

Pro Pro Arg Lys Arg Leu Lys Ser Lys Gly Ser Asp Lys Asp Phe Val
65                  70                  75                  80

Ile Val Arg Arg Pro Lys Leu Asn Arg Glu Asn Phe Pro Gly Val Ser
                85                  90                  95

Trp Asp Ser Leu Pro Asp Glu Leu Leu Leu Gly Ile Phe Ser Cys Leu
            100                 105                 110

Cys Leu Pro Glu Leu Leu Lys Val Ser Gly Val Cys Lys Arg Trp Tyr
        115                 120                 125

Arg Leu Ala Ser Asp Glu Ser Leu Trp Gln Thr Leu Asp Leu Thr Gly
    130                 135                 140

Lys Asn Leu His Pro Asp Val Thr Gly Arg Leu Leu Ser Gln Gly Val
145                 150                 155                 160

Ile Ala Phe Arg Cys Pro Arg Ser Phe Met Asp Gln Pro Leu Ala Glu
                165                 170                 175

His Phe Ser Pro Phe Arg Val Gln His Met Asp Leu Ser Asn Ser Val
            180                 185                 190

Ile Glu Val Ser Thr Leu His Gly Ile Leu Ser Gln Cys Ser Lys Leu
        195                 200                 205

Gln Asn Leu Ser Leu Glu Gly Leu Arg Leu Ser Asp Pro Ile Val Asn
    210                 215                 220

Thr Leu Ala Lys Asn Ser Asn Leu Val Arg Leu Asn Leu Ser Gly Cys
225                 230                 235                 240

Ser Gly Phe Ser Glu Phe Ala Leu Gln Thr Leu Leu Ser Ser Cys Ser
                245                 250                 255

Arg Leu Asp Glu Leu Asn Leu Ser Trp Cys Phe Asp Phe Thr Glu Lys
            260                 265                 270

His Val Gln Val Ala Val Ala His Val Ser Glu Thr Ile Thr Gln Leu
        275                 280                 285

Asn Leu Ser Gly Tyr Arg Lys Asn Leu Gln Lys Ser Asp Leu Ser Thr
    290                 295                 300

Leu Val Arg Arg Cys Pro Asn Leu Val His Leu Asp Leu Ser Asp Ser
305                 310                 315                 320

Val Met Leu Lys Asn Asp Cys Phe Gln Glu Phe Phe Gln Leu Asn Tyr
                325                 330                 335

Leu Gln His Leu Ser Leu Ser Arg Cys Tyr Asp Ile Ile Pro Glu Thr
            340                 345                 350

Leu Leu Glu Leu Gly Glu Ile Pro Thr Leu Lys Thr Leu Gln Val Phe
        355                 360                 365

Gly Ile Val Pro Asp Gly Thr Leu Gln Leu Leu Lys Glu Ala Leu Pro
    370                 375                 380

His Leu Gln Ile Asn Cys Ser His Phe Thr Thr Ile Ala Arg Pro Thr
385                 390                 395                 400

Ile Gly Asn Lys Lys Asn Gln Glu Ile Trp Gly Ile Lys Cys Arg Leu
                405                 410                 415

Thr Leu Gln Lys Pro Ser Cys Leu
            420

<210>  640
<211>  22
<212>  DNA
<213>  artificial

<220>
<223>  Description of artificial sequence: Oligonucleotide

<400>  640
gaatctccag aaatcagatc tc                                              22

<210>  641
<211>  22
<212>  DNA
<213>  artificial

<220>
<223>  Description of artificial sequence: Oligonucleotide

<400>  641
gggagcaatt aatctgtaga tg                                              22

<210>  642
<211>  3520
<212>  DNA
<213>  Homo sapiens

<400>  642
attcgctccc tcccacccca ctgctcccgc tccattgtct gggaattgca gccgcggggc      60

gggcggcggc ggcggcggcg gcggccggga cccagcgggc caggtgggga cggcgcggag     120

cgggtgcggg agatgccgtg cgggactggg gccacctgag ccgcccgcct cgtccccgcc     180

ttctgtggga aggatgtgcg cgcggatggc cggtcgcaca acagcggccc ctcgggggcc     240

ctacggcccc tggctctgcc tcctggtggc cctcgccctg gacgtcgtga gagtggactg     300

tggccaggct cccctggacc ctgtctacct gccggcagcc ctggagctcc tagacgcccc     360

tgaacacttc cgtgtgcagc aggtgggcca ctacccacct gccaactcct ctctgagctc     420

ccgatctgag acctttctgc tcctacagcc ctggcccagg gcccagccac ttctccgggc     480

ctcctaccca ccttttgcca ctcagcaggt ggtcccccct cgagtcactg agccccacca     540

acggccagtc ccatgggacg tgcgggccgt ttcagtggaa gcggctgtga ctccagcaga     600

gccctacgcc cgggttctct tccacctcaa agggcaggat tggccaccag ggtctggcag     660

cctgccctgt gcccggctcc atgccacaca ccctgccggc actgctcacc aagcctgccg     720

cttccagcca tccctgggcg cctgcgtggt ggagctggag cttccctcgc actggttctc     780

acaggcctcc accacacggg ccgagctggc ctacacgctt gagcctgcag ctgagggccc     840

tgggggctgt ggctccggcg aggagaacga ccctggggag caggccctcc cagtgggggg     900

tgtggagctg cgcccagcag accccccgca gtaccaggag gtacctctgg acgaggctgt 960
gactctgcgg gtgcctgaca tgccagtgcg gcccggccag ctctttagtg ctaccctcct 1020
gcttcggcac aacttcacag ccagcctcct gaccctgcgg atcaaggtga agaaggggct 1080
gcatgtgaca gccgcccgcc cagcccagcc cacactctgg actgccaagc tggaccgctt 1140
caagggctcc aggcaccaca ccaccctcat cacctgccac cgtgctgggc tcacagagcc 1200
agattccagc agtccccttg aactgtctga gttcctatgg gtggactttg tggtggagaa 1260
tagcactggt gggggcgtag cggtcactcg ccccgtcacg tggcagctgg agtacccagg 1320
ccaggcccct gaagcagaga aggacaaaat ggtgtgggaa atcctggtgt ctgagcggga 1380
catcagagcc cttatcccac tggccaaggc tgaggagctg gtgaatacag caccactgac 1440
tggagtgccc cagcatgtcc ccgtgcgcct tgtcactgtg gacggcgggg gggccttggt 1500
ggaggtgaca gagcatgtcg gctgcgagtc tgccaacaca caggtcctgc aggtgtctga 1560
ggcctgtgat gccgtgttcg tggctggcaa ggagagccgg ggcgcccggg gggtgcgagt 1620
ggacttctgg tggcgccggc tccgcgcctc gctgcggctg accgtgtggg cccccctgct 1680
accgctgcgt atcgagctca ccgacaccac cctcgagcag gtccgcggct ggagggtacc 1740
tggccctgct gaagggcctg cggaacccgc tgcagaggcg tcggatgagg ccgagcggcg 1800
cgcccgtggc tgccacctgc agtaccagcg ggccggtgtg cgcttcctcg cccccttcgc 1860
ggcccacccg ctggacggcg gccgccgcct cacgcacctg cttggccccg actggctgct 1920
agacgtgtcc cacctcgtgg cgccacacgc ccgcgtgctg gactcgcgtg tagcctctct 1980
ggagggtggc cgtgtcgtgg tgggccggga gcccggtgtc acctccattg aggtgcgttc 2040
cccactgtct gactccatcc tgggggagca ggcgctggct gtgacggacg acaaggtctc 2100
agtgctggag ctgagggtgc agccagtgat gggcatctcg ctgaccttga gccggggcac 2160
tgcccacccc ggggaggtca cagctacgtg ctgggcacag tcagcccttc ccgccccaaa 2220
gcaggaggtg gccctctccc tatggctgtc cttctctgat cacactgtgg ccccagctga 2280
gctctacgac cgccgtgacc tgggactgtc cgtctcagcc gaggagcctg gtgccatcct 2340
gccagctgag gagcagggtg cccagctcgg ggtggtggtg agtggggcag gcgccgaggg 2400
gctgccgctg catgtggctc tgcacccgcc cgagccctgc cgccggggcc gccaccgtgt 2460
gcctctggcc tctggcaccg cctggctggg gctgccccct gcctccactc cagcccctgc 2520
tctcccatcc agccctgctt ggagcccacc agccacagaa gccaccatgg gtggtaaacg 2580
gcaggtggca ggcagtgtcg ggggcaacac aggtgtgagg ggcaagtttg agcgggcaga 2640
ggaggaggcc aggaaggagg agaccgaagc cagggaggag gaggaggaag aggaggagga 2700
gatggtccct gcccctcagc atgtcactga gctagagctg ggcatgtacg ccctgctggg 2760
agtcttctgc gtggccatct tcatcttctt ggtcaatggt gtggtcttcg tcctgcgcta 2820
tcagcgcaaa gaacctcccg acagtgccac tgaccccacc tccccccagc cccacaactg 2880
ggtctggctg ggcactgacc aggaggaact gagccgccag ctggaccggc agtcccctgg 2940

cccgcccaag ggggagggga gctgcccctg tgagagtggg ggaggagggg aggcccctac 3000
cctggcccct ggccctcctg ggggcaccac cagctcctca agcaccctgg cccgaaagga 3060
ggctgggggg cggcggaagc gagtagagtt tgtgacattt gcgccagccc ctccagccca 3120
gtcacctgag gagcctgtag gggcccctgc tgtgcagtcc atccttgtgg caggcgagga 3180
ggacatccgc tgggtgtgtg aggacatggg gctgaaggac cctgaggagc ttcgcaacta 3240
catggagagg atccggggca gctcctgacc ctccacagcc acctggtcag ccaccagctg 3300
gggcaacgag ggtggaggtc ccactgagcc tctcgcctgc ccccgccact cgtctggtgc 3360
ttgttgatcc aagtcccctg cctggtcccc cacaaggact cccatccagg ccccctctgc 3420
cctgcccctt gtcatggacc atggtcgtga ggaagggctc atgcccctta tttatgggaa 3480
ccatttcatt ctaacagaat aaaccgagaa ggaaaccaga 3520

<210> 643
<211> 1024
<212> PRT
<213> Homo sapiens

<400> 643

Met Cys Ala Arg Met Ala Gly Arg Thr Thr Ala Ala Pro Arg Gly Pro
1               5                   10                  15

Tyr Gly Pro Trp Leu Cys Leu Leu Val Ala Leu Ala Leu Asp Val Val
            20                  25                  30

Arg Val Asp Cys Gly Gln Ala Pro Leu Asp Pro Val Tyr Leu Pro Ala
        35                  40                  45

Ala Leu Glu Leu Leu Asp Ala Pro Glu His Phe Arg Val Gln Gln Val
    50                  55                  60

Gly His Tyr Pro Pro Ala Asn Ser Ser Leu Ser Ser Arg Ser Glu Thr
65                  70                  75                  80

Phe Leu Leu Leu Gln Pro Trp Pro Arg Ala Gln Pro Leu Leu Arg Ala
                85                  90                  95

Ser Tyr Pro Pro Phe Ala Thr Gln Gln Val Val Pro Pro Arg Val Thr
            100                 105                 110

Glu Pro His Gln Arg Pro Val Pro Trp Asp Val Arg Ala Val Ser Val
        115                 120                 125

Glu Ala Ala Val Thr Pro Ala Glu Pro Tyr Ala Arg Val Leu Phe His
    130                 135                 140

Leu Lys Gly Gln Asp Trp Pro Pro Gly Ser Gly Ser Leu Pro Cys Ala
145                 150                 155                 160

Arg Leu His Ala Thr His Pro Ala Gly Thr Ala His Gln Ala Cys Arg
                165                 170                 175

Phe Gln Pro Ser Leu Gly Ala Cys Val Val Glu Leu Glu Leu Pro Ser
            180                 185                 190

His Trp Phe Ser Gln Ala Ser Thr Thr Arg Ala Glu Leu Ala Tyr Thr
        195                 200                 205

Leu Glu Pro Ala Ala Glu Gly Pro Gly Gly Cys Gly Ser Gly Glu Glu
    210                 215                 220

Asn Asp Pro Gly Glu Gln Ala Leu Pro Val Gly Gly Val Glu Leu Arg
225                 230                 235                 240

Pro Ala Asp Pro Pro Gln Tyr Gln Glu Val Pro Leu Asp Glu Ala Val
                245                 250                 255

Thr Leu Arg Val Pro Asp Met Pro Val Arg Pro Gly Gln Leu Phe Ser
            260                 265                 270

Ala Thr Leu Leu Leu Arg His Asn Phe Thr Ala Ser Leu Leu Thr Leu
        275                 280                 285

Arg Ile Lys Val Lys Lys Gly Leu His Val Thr Ala Ala Arg Pro Ala
    290                 295                 300

Gln Pro Thr Leu Trp Thr Ala Lys Leu Asp Arg Phe Lys Gly Ser Arg
305                 310                 315                 320

His His Thr Thr Leu Ile Thr Cys His Arg Ala Gly Leu Thr Glu Pro
                325                 330                 335

Asp Ser Ser Ser Pro Leu Glu Leu Ser Glu Phe Leu Trp Val Asp Phe
            340                 345                 350

Val Val Glu Asn Ser Thr Gly Gly Gly Val Ala Val Thr Arg Pro Val
        355                 360                 365

Thr Trp Gln Leu Glu Tyr Pro Gly Gln Ala Pro Glu Ala Glu Lys Asp
    370                 375                 380

Lys Met Val Trp Glu Ile Leu Val Ser Glu Arg Asp Ile Arg Ala Leu
385                 390                 395                 400

Ile Pro Leu Ala Lys Ala Glu Glu Leu Val Asn Thr Ala Pro Leu Thr
                405                 410                 415

Gly Val Pro Gln His Val Pro Val Arg Leu Val Thr Val Asp Gly Gly
            420                 425                 430

Gly Ala Leu Val Glu Val Thr Glu His Val Gly Cys Glu Ser Ala Asn
        435                 440                 445

Thr Gln Val Leu Gln Val Ser Glu Ala Cys Asp Ala Val Phe Val Ala
    450                 455                 460

Gly Lys Glu Ser Arg Gly Ala Arg Gly Val Arg Val Asp Phe Trp Trp
465                 470                 475                 480

Arg Arg Leu Arg Ala Ser Leu Arg Leu Thr Val Trp Ala Pro Leu Leu
                485                 490                 495

Pro Leu Arg Ile Glu Leu Thr Asp Thr Thr Leu Glu Gln Val Arg Gly
            500                 505                 510

Trp Arg Val Pro Gly Pro Ala Glu Gly Pro Ala Glu Pro Ala Ala Glu
        515                 520                 525

Ala Ser Asp Glu Ala Glu Arg Arg Ala Arg Gly Cys His Leu Gln Tyr
    530                 535                 540

Gln Arg Ala Gly Val Arg Phe Leu Ala Pro Phe Ala Ala His Pro Leu
545                 550                 555                 560

Asp Gly Gly Arg Arg Leu Thr His Leu Leu Gly Pro Asp Trp Leu Leu
                565                 570                 575

Asp Val Ser His Leu Val Ala Pro His Ala Arg Val Leu Asp Ser Arg
            580                 585                 590

Val Ala Ser Leu Glu Gly Gly Arg Val Val Val Gly Arg Glu Pro Gly
        595                 600                 605

Val Thr Ser Ile Glu Val Arg Ser Pro Leu Ser Asp Ser Ile Leu Gly
    610                 615                 620

Glu Gln Ala Leu Ala Val Thr Asp Asp Lys Val Ser Val Leu Glu Leu
625                 630                 635                 640

Arg Val Gln Pro Val Met Gly Ile Ser Leu Thr Leu Ser Arg Gly Thr
                645                 650                 655

Ala His Pro Gly Glu Val Thr Ala Thr Cys Trp Ala Gln Ser Ala Leu
            660                 665                 670

Pro Ala Pro Lys Gln Glu Val Ala Leu Ser Leu Trp Leu Ser Phe Ser
        675                 680                 685

Asp His Thr Val Ala Pro Ala Glu Leu Tyr Asp Arg Arg Asp Leu Gly
    690                 695                 700

Leu Ser Val Ser Ala Glu Glu Pro Gly Ala Ile Leu Pro Ala Glu Glu
705                 710                 715                 720

Gln Gly Ala Gln Leu Gly Val Val Val Ser Gly Ala Gly Ala Glu Gly
                725                 730                 735

Leu Pro Leu His Val Ala Leu His Pro Pro Glu Pro Cys Arg Arg Gly
            740                 745                 750

Arg His Arg Val Pro Leu Ala Ser Gly Thr Ala Trp Leu Gly Leu Pro
        755                 760                 765

Pro Ala Ser Thr Pro Ala Pro Ala Leu Pro Ser Ser Pro Ala Trp Ser
    770                 775                 780

Pro Pro Ala Thr Glu Ala Thr Met Gly Gly Lys Arg Gln Val Ala Gly
785                 790                 795                 800

Ser Val Gly Gly Asn Thr Gly Val Arg Gly Lys Phe Glu Arg Ala Glu
                805                 810                 815

Glu Glu Ala Arg Lys Glu Glu Thr Glu Ala Arg Glu Glu Glu Glu Glu
            820                 825                 830

Glu Glu Glu Glu Met Val Pro Ala Pro Gln His Val Thr Glu Leu Glu
        835                 840                 845

Leu Gly Met Tyr Ala Leu Leu Gly Val Phe Cys Val Ala Ile Phe Ile
    850                 855                 860

Phe Leu Val Asn Gly Val Val Phe Val Leu Arg Tyr Gln Arg Lys Glu
865                 870                 875                 880

Pro Pro Asp Ser Ala Thr Asp Pro Thr Ser Pro Gln Pro His Asn Trp
                885                 890                 895

Val Trp Leu Gly Thr Asp Gln Glu Glu Leu Ser Arg Gln Leu Asp Arg
            900                 905                 910

Gln Ser Pro Gly Pro Pro Lys Gly Glu Gly Ser Cys Pro Cys Glu Ser
        915                 920                 925

Gly Gly Gly Gly Glu Ala Pro Thr Leu Ala Pro Gly Pro Pro Gly Gly
    930                 935                 940

Thr Thr Ser Ser Ser Ser Thr Leu Ala Arg Lys Glu Ala Gly Gly Arg
945                 950                 955                 960

Arg Lys Arg Val Glu Phe Val Thr Phe Ala Pro Ala Pro Pro Ala Gln
                965                 970                 975

Ser Pro Glu Glu Pro Val Gly Ala Pro Ala Val Gln Ser Ile Leu Val
            980                 985                 990

Ala Gly Glu Glu Asp Ile Arg Trp  Val Cys Glu Asp Met  Gly Leu Lys
        995                 1000                1005

Asp Pro  Glu Glu Leu Arg Asn  Tyr Met Glu Arg Ile  Arg Gly Ser
    1010                 1015                 1020

Ser

<210>  644
<211>  21
<212>  DNA
<213>  artificial

<220>
<223>  Description of artificial sequence: Oligonucleotide

<400>  644
gtgtcacctc cattgaggtg c                                                21

<210>  645
<211>  21
<212>  DNA
<213>  artificial

<220>
<223>  Description of artificial sequence: Oligonucleotide

<400>  645
gtgtgatcag agaaggacag c                                                21

<210>  646
<211>  479
<212>  DNA
<213>  Homo sapiens

<220>
<221>  misc_feature
<222>  (397)..(397)
<223>  n is a, c, g, t or u

<220>
<221>  misc_feature
<222>  (407)..(407)
<223>  n is a, c, g, t or u

<220>
<221>  misc_feature
<222>  (422)..(422)
<223>  n is a, c, g, t or u

<220>
<221>  misc_feature
<222>  (442)..(442)
<223>  n is a, c, g, t or u

<220>

<221>  misc_feature
<222>  (448)..(449)
<223>  n is a, c, g, t or u

<220>
<221>  misc_feature
<222>  (469)..(469)
<223>  n is a, c, g, t or u

<400>  646
gtcttcagtg aagtttactg tatattataa acagtcatag aattcaaaga caatcatata      60

accaactctt ttggatggct taagatgtgc caggtactgt gctaaggaca agagatataa     120

ccagatacaa accagtcccc atcctcaatc attacttatt cactcaacaa atatttttga     180

gtacttaccc tgcaccaggc actagggata taacagataa aaattaagtc tctcgcttca     240

tgaagctttc attctgatag agggagacgg caataagcaa ataaatggtt tattccacca     300

ccccttcaag tcttcactca aatgttcctt tttcaatgag actatataac caacgtattt     360

aaaatttcaa ccaccatcct gcattcactg cttttcntct tgctaanggt gattaatatg     420

tntttatttg actgaacaca angggccnna tacttggtcc aacattatnc tgggtgtgt      479

<210>  647
<211>  21
<212>  DNA
<213>  artificial

<220>
<223>  Description of artificial sequence: Oligonucleotide

<400>  647
aagtctctcg cttcatgaag c                                                21

<210>  648
<211>  22
<212>  DNA
<213>  artificial

<220>
<223>  Description of artificial sequence: Oligonucleotide

<400>  648
aaaagcagtg aatgcaggat gg                                               22

<210>  649
<211>  1232
<212>  DNA
<213>  Homo sapiens

<400>  649
ctagaggggc ggaaagtaac aaggaggtgg gggtacaaat cctcagctcc tgcttccgca      60

agcactaacc tgctctgaag tgagccaggc agctctggcc atcttttccc agccacagaa     120

tcaggtgatg gtccagaatt aagagctgtc acctgtgtca ttcactcaca atggaagaaa     180

tgaagaagac tgccatccgg ctgcccaaag gcaaacagaa gcctataaag acggaatgga     240

attcccggtg tgtccttttc acctacttcc aaggggacat cagcagcgta gtggatgaac     300

acttctccag agctctgagc aatatcaaga gcccccagga attgaccccc tcgagtcaga     360

gtgaaggtgt gatgctgaaa aacgatgata gcatgtctcc aaatcagtgg cgttactcgt 420
ctccatggac aaagccacaa ccagaagtac ctgtcacaaa ccgtgccgcc aactgcaact 480
tgcatgtgcc tggtcccatg gctgtgaatc agttctcacc gtccctggct aggagggcct 540
ctgttcggcc tggggagctg tggcatttct cctccctggc gggcaccagc tccttagagc 600
ctggctactc tcatcccttc cccgctcggc acctggttcc agagccccag cctgatggga 660
aacgtgagcc tctcctaagt ctcctccagc aagacagatg cctagcccgt cctcaggaat 720
ctgccgccag ggagaatggc aaccctggcc agatagctgg aagcacaggg ttgctcttca 780
acctgcctcc cggctcagtt cactataaga aactatatgt atctcgtgga tctgccagta 840
ccagccttcc aaatgaaact ctttcagagt tagagacacc tgggaaatac tcacttacac 900
caccaaacca ctggggccac ccacatcgat acctgcagca tctttagtca agttggagga 960
gaaagacaac acttggtcta agacacggca gcaagacatc cctgcatatt gttccagata 1020
aaaatgaaag ctgctcacac ccacttgcct ccccaatctg ttaaacagct tcgtgtctag 1080
tatgagctca gtacttgccc tgtgaaaatc ccagaagccc ccgctgtcaa tgttccccat 1140
ccacaccctg cttgctcctg tgtaacagct cagatgatga ataataataa aactgtactt 1200
ttttggatgg tgaaaaaaaa aaaaaaaaaa aa 1232

<210> 650
<211> 258
<212> PRT
<213> Homo sapiens

<400> 650

Met Glu Glu Met Lys Lys Thr Ala Ile Arg Leu Pro Lys Gly Lys Gln
1               5                   10                  15

Lys Pro Ile Lys Thr Glu Trp Asn Ser Arg Cys Val Leu Phe Thr Tyr
            20                  25                  30

Phe Gln Gly Asp Ile Ser Ser Val Val Asp Glu His Phe Ser Arg Ala
        35                  40                  45

Leu Ser Asn Ile Lys Ser Pro Gln Glu Leu Thr Pro Ser Ser Gln Ser
    50                  55                  60

Glu Gly Val Met Leu Lys Asn Asp Asp Ser Met Ser Pro Asn Gln Trp
65                  70                  75                  80

Arg Tyr Ser Ser Pro Trp Thr Lys Pro Gln Pro Glu Val Pro Val Thr
                85                  90                  95

Asn Arg Ala Ala Asn Cys Asn Leu His Val Pro Gly Pro Met Ala Val
            100                 105                 110

Asn Gln Phe Ser Pro Ser Leu Ala Arg Arg Ala Ser Val Arg Pro Gly

115                 120                 125

Glu Leu Trp His Phe Ser Ser Leu Ala Gly Thr Ser Ser Leu Glu Pro
    130                 135                 140

Gly Tyr Ser His Pro Phe Pro Ala Arg His Leu Val Pro Glu Pro Gln
145                 150                 155                 160

Pro Asp Gly Lys Arg Glu Pro Leu Leu Ser Leu Leu Gln Gln Asp Arg
                165                 170                 175

Cys Leu Ala Arg Pro Gln Glu Ser Ala Ala Arg Glu Asn Gly Asn Pro
            180                 185                 190

Gly Gln Ile Ala Gly Ser Thr Gly Leu Leu Phe Asn Leu Pro Pro Gly
        195                 200                 205

Ser Val His Tyr Lys Lys Leu Tyr Val Ser Arg Gly Ser Ala Ser Thr
    210                 215                 220

Ser Leu Pro Asn Glu Thr Leu Ser Glu Leu Glu Thr Pro Gly Lys Tyr
225                 230                 235                 240

Ser Leu Thr Pro Pro Asn His Trp Gly His Pro His Arg Tyr Leu Gln
                245                 250                 255

His Leu


<210>  651
<211>  22
<212>  DNA
<213>  artificial

<220>
<223>  Description of artificial sequence: Oligonucleotide

<400>  651
ggtgtgatgc tgaaaaacga tg                                              22


<210>  652
<211>  20
<212>  DNA
<213>  artificial

<220>
<223>  Description of artificial sequence: Oligonucleotide

<400>  652
cctgtgcttc cagctatctg                                                 20


<210>  653
<211>  2960
<212>  DNA
<213>  Homo sapiens

<400> 653
actgcaagct ctgcctcccg ggttcacgcc attctcctgt gtcagcctcc tgagtagctg 60
ggactacagg cgcccgccac catgcccggc taattttttt tgtattttta gtagagacgg 120
ggtttcacca tgttagccag gatagtttca atttccggac ctcgtgatcc gcccgcctcg 180
gcctcccaaa gtgctgggat tataggcgtg agccaccgcg cccagcccct ttcatttttg 240
atatggagtt ttgttcttgt tgcccaggct ggaatgcaat ggcgcgatct cagctcactg 300
caacctctgc ctcctgggtt caagcgattc tcctggttca gcctcctgag tagctgggat 360
tacaggaatg cgccaccaca cctggctact tttgtatttt tagtagagat ggggtttctc 420
cacgtgcatc aggctagtct cacactcccg acttcagatg atccacccgc ctcggcctcc 480
caaagtgctg ggattacagg cgtgagccac cgtgcctggc caaagccttc ttcactttca 540
ttttgtggct gctgttgtga aatctgcaga acctcaggct gtttggactc catcttctag 600
gttgaattag attacaggta atctcatggt ggaggtgtat tctggatcga ctcacttttg 660
aaatgtttat gaaagaaatt ccttcacctc gggacttcca cactggagct ggcaatcagt 720
atcaccttaa tttacctatc acagtgaggc atctctgctc tgaggaaaac aaaacaaaac 780
aaaacaaaac aaaaaagaag agctaatggt tctgttttgt tttgttgttt tagagacagg 840
gtcttgctct gtcgcccggg ctggaatgca gtgacatcat cacagctcac tgtagccttg 900
atctcctggg ctcaagcgat ccttctgcct cagcctgagt agctgggatg ggactacaga 960
tgcacgccac cacacatggc taattttttt attttttaga gacgaggtcc catgtgagcc 1020
acggtgcccc accttttaaa gcaattaccg taaggaagca actgtcttta gcaaatagaa 1080
gatcataaac atccttctgg agatcttgag atgttgactg tggtttatag ggcaatgaaa 1140
atctacatac ctgagtgggg cagtgagaca tgagtgccac ctatgatcct tccagtgagc 1200
aattctttct ggtgccagga gcccaaagga aactcagtca accagctgta gatgggcttg 1260
ttccgagttt gttctagaag tcagaactgc cagctctgaa agtaggtcac aagtgactgg 1320
ccatccctgg gctgctcaca ggaagtagct gcaaaggtca gggaaagttt ttaatgacta 1380
tacaggccag tgaatgacag gctttgagga gcttctcttc ttggcatctg aaacaggcac 1440
atggaggagc tgccttggag acagaatgac agctgtggca atgtgagcct gattcctcag 1500
ccccataaaa tagatgcctt tgcaaaaaac ccatttattt tttttttctt gtttttgaga 1560
gggagtctcg ctctgtcccc ccaggcggag tgcagtggcg tgatctcggc tcactgcaac 1620
ttctgcctcc cgggttcaag cgattctcct gcctcagctc ccaagtagct gagattacag 1680
gcatccgcca ccacacctgg ccaattttgt attttagtag agacggggtt ttgccacatt 1740
ggccaggctg ggaaaaagcc catttctata cagaggaatc atcttgcagg actcaaggaa 1800
tgattaagag gaatctacag cattaacttt agaaactcga tgcaatattc ccaagaggag 1860
ttaagattgg gataagtaat ctctgaatta atttcaacct tgagatgttg tgatcctatg 1920
aagtcaaaga catatttctt ttttctttgt ttgagatgga gtcttgctct gtcacccaaa 1980
ttggagggcg gtgatgtgat ctcggcttac tacgacctct gcctcctggg ttcaagcgat 2040

tctcagcttc agcctcctga gtagctgaga ttacaggtgc ccgccaccat gcccggctaa 2100
tttttgtatt tttagtagag atgaggtttc actgtgttgg ccaggctggt cttgaattct 2160
tggccccagg tgatctgccc gcctgggcct cccaaagtgc tgggattaca ggagtgagca 2220
ccatgtccgg tctagataca tatttctatt aatacacatt tgcagggatt gtttgttact 2280
gaagttgacg ccttgtgctt gagaatgcta tttcactgac acaattgtta tggtataaat 2340
gtgagagtat agttacaatc tggacactat tttttgcttg tcttattggc ccagcaacca 2400
gcttgacact ggggactatc aggctccaaa taataaccaa tgtctcactc caaacagaca 2460
ggatactacg gagccagggt cagcaaacat tttctgtaaa gggccagata gtaaatattt 2520
tgggctttgt gggccctatg gtctctgtca caacgattca actctgctgt tgtaacaaaa 2580
aagcagtcag acaatatgta aatgaatggg catggctgtg ttccagtaaa accacaaaaa 2640
caggcagcta gctttggtgt ggggccctaa gagcttggac tttgaagcca ggttcaaatc 2700
ctggctttct caccttctag ctccgttacc ttaaagaact tcattatctt ccttgtattt 2760
tagtcttttc atctctaaat tagtgatagt acttaacagt gttgttataa agattaaacg 2820
agttaagttt gtaaagtact taaacagtgc ctaccaggta gtaagtgcta tagcagagtg 2880
tgttagataa tgaaacatcc tgtgctgtgg atatattatt cccattacga tttggattca 2940
aataaagaaa tacatgactt 2960

<210> 654
<211> 21
<212> DNA
<213> artificial

<220>
<223> Description of artificial sequence: Oligonucleotide

<400> 654
tcaccttcta gctccgttac c 21

<210> 655
<211> 22
<212> DNA
<213> artificial

<220>
<223> Description of artificial sequence: Oligonucleotide

<400> 655
ctaagagctt ggactttgaa gc 22

<210> 656
<211> 2059
<212> DNA
<213> Homo sapiens

<400> 656
agcacagaag gaggaaggac agcacagctg acagccgtac tcaggaagct tctggatcct 60
aggcttatct ccacagagga gaacacacaa gcagcagaga ccatggggcc cctctcagcc 120

cctccctgca cacagcgcat cacctggaag ggggtcctgc tcacagcatc acttttaaac 180
ttctggaatc cgcccacaac tgcccaagtc acgattgaag cccagccacc caaagtttct 240
gaggggaagg atgttcttct acttgtccac aatttgcccc agaatcttgc tggctacatt 300
tggtacaaag ggcaaatgac atacctctac cattacatta catcatatgt agtagacggt 360
caaagaatta tatatgggcc tgcatacagt ggaagagaaa gagtatattc caatgcatcc 420
ctgctgatcc agaatgtcac gcaggaggat gcaggatcct acaccttaca catcataaag 480
cgacgcgatg ggactggagg agtaactgga catttcacct tcaccttaca cctggagact 540
cccaagccct ccatctccag cagcaactta aatcccaggg aggccatgga ggctgtgatc 600
ttaacctgtg atcctgcgac tccagccgca agctaccagt ggtggatgaa tggtcagagc 660
ctccctatga ctcacaggtt gcagctgtcc aaaaccaaca ggaccctctt tatatttggt 720
gtcacaaagt atattgcagg accctatgaa tgtgaaatac ggaacccagt gagtgccagc 780
cgcagtgacc cagtcaccct gaatctcctc ccaaagctgt ccaagcccta catcacaatc 840
aacaacttaa accccagaga gaataaggat gtcttaacct tcacctgtga acctaagagt 900
aagaactaca cctacatttg gtggctaaat ggtcagagcc tccctgtcag tcccagggta 960
aagcgaccca ttgaaaacag gatcctcatt ctacccaatg tcacgagaaa tgaaacagga 1020
ccttatcaat gtgaaatacg ggaccgatat ggtggcatcc gcagtgaccc agtcaccctg 1080
aatgtcctct atggtccaga cctccccagc atttaccctt cattcaccta ttaccgttca 1140
ggagaaaacc tctacttgtc ctgcttcgcc gagtctaacc cacgggcaca atattcttgg 1200
acaattaatg ggaagtttca gctatcagga caaaagctct ctatccccca aataactaca 1260
aagcatagtg ggctctatgc ttgctctgtt cgtaactcag ccactggcaa ggaaagctcc 1320
aaatccatca cagtcaaagt ctctgactgg atattaccct gaattctact agttcctcca 1380
attccatttt ctcccatgga atcacgaaga gcaagaccca ctctgttcca gaagccctat 1440
aagctggagg tggacaactc gatgtaaatt tcatgggaaa acccttgtac ctgacatgtg 1500
agccactcag aactcaccaa aatgttcgac accataacaa cagctactca aactgtaaac 1560
caggataaca agttgatgac ttcacactgt ggacagtttt tccaaagatg tcagaacaag 1620
actccccatc atgataaggc tcccacccct cttaaccgtc cttgctcatg cctgcctctt 1680
tcacttggca ggataatgca gtcattagaa tttcacatgt agtagcttct gagggtaaca 1740
acagagtgtc agatatgtca tctcaacctc aaacttttac gtaacatctc aggggaaatg 1800
tggctctctc catcttgcat acagggctcc caatagaaat gaacacagag atattgcctg 1860
tgtgtttgca gagaagatgg tttctataaa gagtaggaaa gctgaaatta tagtagagtc 1920
tcctttaaat gcacattgtg tggatggctc tcaccatttc ctaagagata cagtgtaaaa 1980
cgtgacagta atactgattc tagcagaata aaacatgtac cacatttgct aaaaaaaaaa 2040
aaaaaaaaaa aaaaaaaaa 2059

<210> 657

<211>  419
<212>  PRT
<213>  Homo sapiens

<400>  657

Met Gly Pro Leu Ser Ala Pro Pro Cys Thr Gln Arg Ile Thr Trp Lys
1               5                   10                  15

Gly Val Leu Leu Thr Ala Ser Leu Leu Asn Phe Trp Asn Pro Pro Thr
            20                  25                  30

Thr Ala Gln Val Thr Ile Glu Ala Gln Pro Pro Lys Val Ser Glu Gly
        35                  40                  45

Lys Asp Val Leu Leu Leu Val His Asn Leu Pro Gln Asn Leu Ala Gly
    50                  55                  60

Tyr Ile Trp Tyr Lys Gly Gln Met Thr Tyr Leu Tyr His Tyr Ile Thr
65                  70                  75                  80

Ser Tyr Val Val Asp Gly Gln Arg Ile Ile Tyr Gly Pro Ala Tyr Ser
                85                  90                  95

Gly Arg Glu Arg Val Tyr Ser Asn Ala Ser Leu Leu Ile Gln Asn Val
            100                 105                 110

Thr Gln Glu Asp Ala Gly Ser Tyr Thr Leu His Ile Ile Lys Arg Arg
        115                 120                 125

Asp Gly Thr Gly Gly Val Thr Gly His Phe Thr Phe Thr Leu His Leu
    130                 135                 140

Glu Thr Pro Lys Pro Ser Ile Ser Ser Ser Asn Leu Asn Pro Arg Glu
145                 150                 155                 160

Ala Met Glu Ala Val Ile Leu Thr Cys Asp Pro Ala Thr Pro Ala Ala
                165                 170                 175

Ser Tyr Gln Trp Trp Met Asn Gly Gln Ser Leu Pro Met Thr His Arg
            180                 185                 190

Leu Gln Leu Ser Lys Thr Asn Arg Thr Leu Phe Ile Phe Gly Val Thr
        195                 200                 205

Lys Tyr Ile Ala Gly Pro Tyr Glu Cys Glu Ile Arg Asn Pro Val Ser
    210                 215                 220

Ala Ser Arg Ser Asp Pro Val Thr Leu Asn Leu Leu Pro Lys Leu Ser
225                 230                 235                 240

Lys Pro Tyr Ile Thr Ile Asn Asn Leu Asn Pro Arg Glu Asn Lys Asp
                245                 250                 255

Val Leu Thr Phe Thr Cys Glu Pro Lys Ser Lys Asn Tyr Thr Tyr Ile
            260                 265                 270

Trp Trp Leu Asn Gly Gln Ser Leu Pro Val Ser Pro Arg Val Lys Arg
        275                 280                 285

Pro Ile Glu Asn Arg Ile Leu Ile Leu Pro Asn Val Thr Arg Asn Glu
    290                 295                 300

Thr Gly Pro Tyr Gln Cys Glu Ile Arg Asp Arg Tyr Gly Gly Ile Arg
305                 310                 315                 320

Ser Asp Pro Val Thr Leu Asn Val Leu Tyr Gly Pro Asp Leu Pro Ser
                325                 330                 335

Ile Tyr Pro Ser Phe Thr Tyr Tyr Arg Ser Gly Glu Asn Leu Tyr Leu
            340                 345                 350

Ser Cys Phe Ala Glu Ser Asn Pro Arg Ala Gln Tyr Ser Trp Thr Ile
        355                 360                 365

Asn Gly Lys Phe Gln Leu Ser Gly Gln Lys Leu Ser Ile Pro Gln Ile
    370                 375                 380

Thr Thr Lys His Ser Gly Leu Tyr Ala Cys Ser Val Arg Asn Ser Ala
385                 390                 395                 400

Thr Gly Lys Glu Ser Ser Lys Ser Ile Thr Val Lys Val Ser Asp Trp
                405                 410                 415

Ile Leu Pro

<210>  658
<211>  21
<212>  DNA
<213>  artificial

<220>
<223>  Description of artificial sequence: Oligonucleotide

<400>  658
gtctctgact ggatattacc c                                                21

<210>  659
<211>  21
<212>  DNA
<213>  artificial

<220>
<223>  Description of artificial sequence: Oligonucleotide

<400>  659
cctgccaagt gaaagaggca g                                                21

<210> 660
<211> 7289
<212> DNA
<213> Homo sapiens

<400> 660
   gctgcaattc aagttggctg ggggccggta gtgagatcta gggctacttc aacaaaactt 60
   tgctgccctt cctgctcctc ttgtcttctt ttctcctgat accttttgat gctctgcaca 120
   tgttatttgc atagcaaagg cactaagctt tccaggaaga gagggcacca cttccacccc 180
   caataagtct tttttcccgt cttttctttc ttttcctttc cttttttggg gggtggggag 240
   ggagagaaag ggggtttgca aaggcagcat ctcagagtga tagcctagat gtattgaagg 300
   catctcttat tctgccaaat cggaaagtca gttctctaaa gcccgggtta gccagaccat 360
   agggttttat tctggctgca gaataactgg ctggtgtggc tttgcaaagg ggggcaaaaa 420
   ataaaaaaat aaaaaaaatt aaaaaaagtt agagaggaga gggagtacgt gagtcgtcca 480
   gtgcaatctc tattgtctga aacttacttt ttatcagaat ttgaagatga aaacggttaa 540
   aaaatggcca tactttagta actaatcagc ctaatgcttt gcttatgaaa acttttacca 600
   tcattatttt tttcattttg gattgagaaa atgaatccag atatagaaga aagtgcagga 660
   tgttggataa aagtggctct taaacagtgg aacatccaat aactaatttg caagaagttt 720
   taaagaaata aaattgttat gcttcgattt tggtatggta ttgactcttt agcacatagg 780
   tagccctcaa aaaaatcatc cagttttcta aattatggaa attttgtgga agacgttgac 840
   ctggattttg agcctcatca tggcttcatc ggaatttcat agtgaccaca ggctttcata 900
   cagttctcaa gaggaattcc tgacttatct tgaacactac cagctaacta ttccaataag 960
   ggttgatcaa aatggagcat ttctcagctt tactgtgaaa aatgataaac actcaaggag 1020
   aagacggagt atggacccta ttgatccaca gcaggcagta tctaagttat tttttaaact 1080
   ttcagcctat ggcaagcact ttcatctaaa cttgactctc aacacagatt ttgtgtccaa 1140
   acattttaca gtagaatatt gggggaaaga tggaccccag tggaaacatg attttttaga 1200
   caactgtcat tacacaggat atttgcaaga tcaacgtagt acaactaaag tggctttaag 1260
   caactgtgtt gggttgcatg gtgttattgc tacagaagat gaagagtatt ttatcgaacc 1320
   tttaaagaat accacagagg attccaagca ttttagttat gaaaatggcc accctcatgt 1380
   tatttacaaa aagtctgccc ttcaacaacg acatctgtat gatcactctc attgtggggt 1440
   ttcggatttc acaagaagtg gcaaaccttg gtggctgaat gacacatcca ctgtttctta 1500
   ttcactacca attaacaaca cacatatcca ccacagacag aagagatcag tgagcattga 1560
   acggtttgtg gagacattgg tagtggcaga caaaatgatg gtgggctacc atggccgcaa 1620
   agacattgaa cattacattt tgagtgtgat gaatattgtt gccaaacttt accgtgattc 1680
   cagcctagga aacgttgtga atattatagt ggcccgctta attgttctca cagaagatca 1740
   gccaaacttg gagataaacc accatgcaga caagtccctc gatagcttct gtaaatggca 1800
   gaaatccatt ctctcccacc aaagtgatgg aaacaccatt ccagaaaatg ggattgccca 1860
   ccacgataat gcagttctta ttactagata tgatatctgc acttataaaa ataagccctg 1920
   tggaacactg ggcttggcct ctgtggctgg aatgtgtgag cctgaaagga gctgcagcat 1980
   taatgaagac attggcctgg gttcagcttt taccattgca catgagattg gtcacaattt 2040
   tggtatgaac catgatggaa ttggaaattc ttgtgggacg aaaggtcatg aagcagcaaa 2100
   acttatggca gctcacatta ctgcgaatac caatcctttt tcctggtctg cttgcagtcg 2160
   agactacatc accagctttc tagattcagg ccgtggtact tgccttgata atgagcctcc 2220
   caagcgtgac tttctttatc cagctgtggc cccaggtcag gtgtatgatg ctgatgagca 2280
   atgtcgtttc cagtatggag caacctcccg ccaatgtaaa tatggggaag tgtgtagaga 2340
   gctctggtgt ctcagcaaaa gcaaccgctg tgtcaccaac agtattccag cagctgaggg 2400
   gacactgtgt caaactggga atattgaaaa agggtggtgt tatcagggag attgtgttcc 2460
   ttttggcact tggccccaga gcatagatgg gggctggggt ccctggtcac tatggggaga 2520
   gtgcagcagg acctgcgggg gaggcgtctc ctcatcccta agacactgtg acagtccagc 2580
   accttcagga ggtggaaaat attgccttgg ggaaaggaaa cggtatcgct cctgtaacac 2640
   agatccatgc cctttgggtt cccgagattt tcgagagaaa cagtgtgcag actttgacaa 2700
   tatgcctttc cgaggaaagt attataactg gaaaccctat actggaggtg gggtaaaacc 2760
   ttgtgcatta aactgcttgg ctgaaggtta taatttctac actgaacgtg ctcctgcggt 2820
   gatcgatggg acccagtgca atgcggattc actggatatc tgcatcaatg gagaatgcaa 2880
   gcacgtaggc tgtgataata ttttgggatc tgatgctagg gaagatagat gtcgagtctg 2940
   tggaggggac ggaagcacat gtgatgccat tgaagggttc ttcaatgatt cactgcccag 3000
   gggaggctac atggaagtgg tgcagatacc aagaggctct gttcacattg aagttagaga 3060
   agttgccatg tcaaagaact atattgcttt aaaatctgaa ggagatgatt actatattaa 3120
   tggtgcctgg actattgact ggcctaggaa atttgatgtt gctgggacag cttttcatta 3180
   caagagacca actgatgaac cagaatcctt ggaagctcta ggtcctacct cagaaaatct 3240
   catcgtcatg gttctgcttc aagaacagaa tttgggaatt aggtataagt tcaatgttcc 3300
   catcactcga actggcagtg gagataatga agttggcttt acatggaatc atcagccttg 3360
   gtcagaatgc tcagctactt gtgctggagg tgtccaaaga caggaggtgg tctgtaaaag 3420
   gttggatgac aactccattg tccagaacaa ttactgtgat cctgacagta agccacctga 3480
   aaatcaaaga gcctgcaaca ctgagccctg cccacctgag tggttcattg gggattggtt 3540
   ggaatgcagc aagacttgtg atggtgggat gcgcacaagg gcagtgctct gcatcaggaa 3600
   gatcggacct tctgaggagg agacgctgga ctacagtggt tgtttaacac accggcctgt 3660
   cgaaaaagag ccctgcaaca accagtcatg tccaccacag tgggtggctt tggactggtc 3720
   tgagtgtact ccaaaatgtg gtccaggatt caagcatcgg attgttctgt gcaagagcag 3780
   tgacctttct aagacattcc cagctgcaca atgtccagag gaaagcaaac ctcctgtccg 3840
   catccgctgc agtttgggcc gctgccctcc tcctcgctgg gtcacaggag actggggcca 3900
   gtgttctgct cagtgtggcc ttggacagca gatgagaact gtgcagtgtc tctcctacac 3960
   cggacaggca tctagtgact gtctagaaac tgttcggcct ccatcaatgc agcagtgtga 4020
   aagcaaatgt gacagtaccc ccatttctaa tactgaagag tgcaaagatg tgaataaagt 4080
   ggcttattgc ccactggtgc tgaagttcaa gttctgcagt cgagcatact tcagacagat 4140
   gtgttgtaag acctgccaag gacactgacc cacagaaagc cagagagagt gccttgtcat 4200
   ttcatcatgg aaatgcatcc atcaaagaga gccacccaga ggaagaggat tgatgtcctt 4260
   gcaaatgcat taccctgtgg aaaacgtaac cactggtcag ccctagctga caaaatttca 4320
   atattatttt agcttctgtg aagtgggatt tattgatcca aagtgctgga cacggtatta 4380
   ggagggaatg ccagattgga gagatccaaa caacacaggg agacttgctt actgtggagc 4440
   gtttgtgttc tttcgagtaa atccaatagc ctgtttacct ccttggacca ttaagataat 4500
   ttttattatg gacttagcaa tgacactgaa tccatttgta tttaaaactg tttaaaatgt 4560
   agctgttatg acttggtcaa ctatggaagt gaagaaggtt cagaattctt aagtcatagc 4620
   ttaaaaatat ttactgtact ttatctcact acaacagcac cacaatttaa attataaaac 4680
   gggctttgaa ctataattta aggagcaatt ataaatcaaa agtaatgaaa gtttgtatta 4740
   tttttcttca ttccacttaa tttccttagg aataatcccc tggttctgaa cactgctgtg 4800
   agccatatat aaaactatat taaactgaac aataatgagg ggcatagttt aaagcagtgc 4860
   atcagttact gcagctgtgc aagtctataa actcagtgct gaaagactgt ggccaacttg 4920
   ccattgtgca agtaaagctg agatttccat taaaacttta agagaaaaac atttcaattt 4980
   catgcagaaa ccagacctgg ggtatggtac agaccaaagg accaggccct ttgctgccac 5040
   cacacaggat gccttagttc ttatttgagt ccctccaact cacttgtgtt tacatcctcc 5100
   ccagccacag cacggcttct gccctttgga ttgctgcacg tgtgttgagc ttactgagat 5160
   gataccatgc aaaagataga ctggctcggt aaccaggcag acccttttgc agtttgttga 5220
   caattacgat gagttccaga tgtcccttct ttgatatggt agaagggcat ttatttatat 5280
   gagagcaaat gtgtgtgtgt gtttgcgggc gcttttaagt gtgtggatag atgagtgtgc 5340
   ttgcacataa tgtgctattt ctgtgagttt taaagtaggc aagggataat aaccaaagaa 5400
   gaaaatttca tgaagactag acatcataaa gcataatttt aatagtcact caaccaagta 5460
   tttttatttt tttatggata ctctgaatgg caattaaatg tgaaacccag tttcttgggc 5520
   aagtcaaatt ctggaatcac atccacctaa attaaaatga ctagctcgta ttttccccat 5580
   cttcaagttt cacatcctgg tcatcaaaag actcgacagc aagacttaga atgaaaaagg 5640
   gtacttgttt atattaatat tttttacttg aacacgtgta gcttgcagca ggttcttgat 5700
   gaatgtgctt tgtgtccaaa atgcctcccc attgtacaca ggtgtacacc atgcatgcac 5760
   caacacctaa aactcaaaac taaatggcta ttttgtaagg ttaatacttt cagttaaaca 5820
   gcatgtttga cttgattcca tcatggtgct cttaaattac atgtcagtgc atcacatata 5880
   tcatgatcta atgcagatga ctaggctttt ttccaaagga agacagaccc tcagacacca 5940
   aaagccaatc taaacaactc ccaggtttgc tgtggacaat cagcatggaa tgttttctgc 6000
   actctcagtc atgaccatct gtatcttgtt acctgctttc tctctcaaca ccacagttct 6060
   caaccctgag ccttccagag agagctattg atgatacaag aggaatcacc agggcccgga 6120
   tctaagatgc ccttagaaga ccagcccaag tgccgtctta gccattcagt gaagggcaaa 6180
   cagcccatgg gtagtatggc ccgagcactg aattcccttg cgccttttca aagaacagtt 6240
   aacttggtgc taatgtgccc tggtgaaata aataaaagat gggcagtttc tgtggcattt 6300
   taggcatagg tttgcaatcc agatctgatt ttctccaaca taaatatcag ctcatgttct 6360
   tatttcaaaa agatttctta ttaccgacta aaagctattt tttacctcac ctggaaacta 6420
   ccattgtgag ggccatcccc caggcactgc acagcacctt ggctgatgct ggaagaggag 6480
   ggcagtcagt gtcacttctg ggatgtgccc cagcactgag aacaaaatgc aggcatcccc 6540
   cggggcagca tcagagtgcc tttctagagg gagccacgca cagaatgtaa caggatgaaa 6600
   cagtttcaag taagccttga attgaaacct gagtaggtta aaacaattct atttcatagc 6660
   acatcacaat actgctgcta ctctgtagcc acccccatgg ctacatgatg ccctattcct 6720
   aaataataac aatagcattg tcagtggagg ctgggccacc atggcagacc ttccaaaagt 6780
   agtgagctac atagactact tagggaaccc cagggaaact ggtaccctac acctgggagc 6840
   agtatctgcc actgggataa agtcctacta aaaaaggaac ggtaaatgta ccctaatgat 6900
   taaaccccgt gagatacata tgatttccaa atagtccatt tcattaggaa cttttttgtt 6960
   tgaatgaatg tcacataggt atcctcagta acacagaacg aaattacctt tgtattattg 7020
   tgattagttg ttgcttatta ttttatactc agtaataatg tggtacactg ttaatttttt 7080
   tgcttttgta aattatattc taatttattg ccatgtttcc taacacttgt cctacattca 7140
   ttctcctgct tgtaatgaaa atgaaaaaat cattgtaaca cttgatggag tgaaattcca 7200
   cgccaggcac agaatttttt tgacatagat aatttagtaa aataaaaatt cagcttataa 7260
   taatgataaa aaaaaaaaaa aaaaaaaaa 7289

<210> 661
<211> 1117
<212> PRT
<213> Homo sapiens

<400> 661

<210> 662
<211> 21
<212> DNA
<213> artificial

<220>
<223> Description of artificial sequence: Oligonucleotide

<400> 662
atactgaaga gtgcaaagat g 21


<210> 663
<211> 21
<212> DNA
<213> artificial

<220>
<223> Description of artificial sequence: Oligonucleotide

<400> 663
tccacagggt aatgcatttg c 21


<210> 664
<211> 1541
<212> DNA
<213> Homo sapiens

<400> 664
gtctcagcgc agaaggagga aggacagcac agctgacagc cgtgctctgg aagcttctgg 60

atcctaggct catctccaca gaggagaaca tgcacgcagc agagatcatg gggcccctct 120

cagcccctcc ctgcacagag cacatcaaat ggaaggggct cctgctcaca gcattacttt 180

taaacttctg gaacttgcct accactgccc aagtcatgat tgaagcccag ccacccaaag 240

tgtccgaggg gaaggatgtt cttctacttg tccacaattt gccccagaat cttactggct 300

acatctggta caaagggcaa atcagggacc tctaccatta cattacatca tatgtagtag 360

acggtcaaat aattatatat ggaccggcat acagtggacg agaaacagta tattccaatg 420

catccctgct gatccagaat gtcacccggg aggacgcagg atcctacacc ttacacatca 480

taaagcgagg tgatgggact agaggagtaa ctggatattt caccttcacc ttatacctgg 540

agactcccaa gccctccatc tccagcagca acttaaaccc cagggaggcc atggagactg 600

tgatcttaac ctgtaatcct gagactccgg acgcaagcta cctgtggtgg atgaatggtc 660

agagcctccc tatgactcat aggatgcagc tgtctgaaac caacaggacc ctctttctat 720

ttggtgtcac aaagtatact gcaggaccct atgaatgtga aatatggaac tcagggagtg 780

ccagccgcag tgacccagtc accctgaatc tcctccatgg tccagacctc cccagaattt 840

tcccttcagt cacctcttac tattcaggag agaacctcga cttgtcctgc ttcgcaaact 900

ctaacccacc agcacagtat tcttggacaa ttaatgggaa gtttcagcta tcaggacaaa 960

agctctttat ccctcagatt actccaaagc ataatgggct ctatgcttgc tctgctcgta 1020

actcagccac tggcgaggaa agctccacat ccttgacaat cagagtcatt gctcctccag 1080

gattaggaac ttttgctttc aataatccaa cgtagcagcc gtgatgtcat ttttgtattt 1140

caggaagact ggcaggagat ttatggaaaa gactatgaaa aggactcttg aatacaagtt    1200

cctgataact tcaagatcat accactggac taagaacttt caaaattttg atgaacaggc    1260

tgataccttc atgaaattca agacaaagaa gaaaagaact ccatttcatt ggactaaata    1320

acaaaaggat aatgttttca taatttttta ttggaaaatg tgctgatttt ttgaatgttt    1380

tatcctccag atttatgaat ttttttcttc agcaattggt aaagtatact tttgtaaaca    1440

aaaattgaaa catttgcttt tgctctctga gtgccccaga attgggaatc tattcatgaa    1500

tattcatatg tttatggtaa taaagttatt tgcacaagtt t                        1541

<210>  665
<211>  335
<212>  PRT
<213>  Homo sapiens

<400>  665

Met Gly Pro Leu Ser Ala Pro Pro Cys Thr Glu His Ile Lys Trp Lys
1               5                   10                  15

Gly Leu Leu Leu Thr Ala Leu Leu Leu Asn Phe Trp Asn Leu Pro Thr
            20                  25                  30

Thr Ala Gln Val Met Ile Glu Ala Gln Pro Pro Lys Val Ser Glu Gly
        35                  40                  45

Lys Asp Val Leu Leu Leu Val His Asn Leu Pro Gln Asn Leu Thr Gly
    50                  55                  60

Tyr Ile Trp Tyr Lys Gly Gln Ile Arg Asp Leu Tyr His Tyr Ile Thr
65                  70                  75                  80

Ser Tyr Val Val Asp Gly Gln Ile Ile Ile Tyr Gly Pro Ala Tyr Ser
                85                  90                  95

Gly Arg Glu Thr Val Tyr Ser Asn Ala Ser Leu Leu Ile Gln Asn Val
            100                 105                 110

Thr Arg Glu Asp Ala Gly Ser Tyr Thr Leu His Ile Ile Lys Arg Gly
        115                 120                 125

Asp Gly Thr Arg Gly Val Thr Gly Tyr Phe Thr Phe Thr Leu Tyr Leu
    130                 135                 140

Glu Thr Pro Lys Pro Ser Ile Ser Ser Ser Asn Leu Asn Pro Arg Glu
145                 150                 155                 160

Ala Met Glu Thr Val Ile Leu Thr Cys Asn Pro Glu Thr Pro Asp Ala
                165                 170                 175

Ser Tyr Leu Trp Trp Met Asn Gly Gln Ser Leu Pro Met Thr His Arg
            180                 185                 190

Met Gln Leu Ser Glu Thr Asn Arg Thr Leu Phe Leu Phe Gly Val Thr
        195                 200                 205

Lys Tyr Thr Ala Gly Pro Tyr Glu Cys Glu Ile Trp Asn Ser Gly Ser
    210                 215                 220

Ala Ser Arg Ser Asp Pro Val Thr Leu Asn Leu Leu His Gly Pro Asp
225                 230                 235                 240

Leu Pro Arg Ile Phe Pro Ser Val Thr Ser Tyr Tyr Ser Gly Glu Asn
                245                 250                 255

Leu Asp Leu Ser Cys Phe Ala Asn Ser Asn Pro Pro Ala Gln Tyr Ser
            260                 265                 270

Trp Thr Ile Asn Gly Lys Phe Gln Leu Ser Gly Gln Lys Leu Phe Ile
        275                 280                 285

Pro Gln Ile Thr Pro Lys His Asn Gly Leu Tyr Ala Cys Ser Ala Arg
    290                 295                 300

Asn Ser Ala Thr Gly Glu Glu Ser Ser Thr Ser Leu Thr Ile Arg Val
305                 310                 315                 320

Ile Ala Pro Pro Gly Leu Gly Thr Phe Ala Phe Asn Asn Pro Thr
                325                 330                 335

<210>  666
<211>  21
<212>  DNA
<213>  artificial

<220>
<223>  Description of artificial sequence: Oligonucleotide

<400>  666
gctctatgct tgctctgctc g                                                21

<210>  667
<211>  21
<212>  DNA
<213>  artificial

<220>
<223>  Description of artificial sequence: Oligonucleotide

<400>  667
taaatctcct gccagtcttc c                                                21

<210>  668
<211>  2243
<212>  DNA
<213>  Homo sapiens

<400>  668

cggcaatgtc cgccacctgg tgcagcccag agggtcaagg aatgggccag ggccctggca 60
gggaggtcgg aggaaattcc gccgccagcg gccccgcctc tcccataagg aacccatgcc 120
tttctgaagc aggactgaag gggcccccaa gtgcccaccc cctgcggtta tgtctcctcc 180
atagattggt ctgcttctct ggaggcctca cgtccattca gctctcacct cgcacctgct 240
gtagccacca gtgggcccag ctcttctcac ctgcctgctt cccccagtgg cgtgctcctg 300
gctgtagttt ggatgattcc cgttctctca caagaatccg tccagtccat cttcctggcc 360
cctccctgga ctgactttgg agacctagcc ccagaaagcc tcccttcttc tccaggtccc 420
ctccgcccta gtccctgcct gtctcatcta acgccccaaa ccttcatttg ggccttcctt 480
cctcatgtct gccctgagcg cggggtggaa atgctccctt ctgtgggctc cagcagatcc 540
cttgttttcc tgtcagttgg acccctcacc tggcctccag ggaagaatgc agagaaaagc 600
aaggagagac tctagttaag aggtgctggc tgcggggatc cagacagggc acattggggg 660
catggaagtg ccagggtggt tttcaggagc tctggtgaag tgggtggagc atcagcgttt 720
gctcagttaa gggagaggta gagaggggcc cgtgaagtcc tttgtcactt ctcttgcctt 780
agtgtgcctc ccaatactcc cttcttcctg cccccacacc ccatccccag ctagcccagg 840
ctccaggtca ggaggggagg gtgctgggcc tgacatggct atataccctc ccaggagtaa 900
aagccaagca agaggttgtt tttgccaaga atcacagaat gttagagctg acaggaccct 960
tgaaggtcac ttagccttct taggcaaacg cctgcaaaac agaagcctgg agaggggagt 1020
gacctgctca gagtcattgc agagccggga tggggaccag gtctcccatc tcctacttta 1080
tgacgccctc ttccctcttg atgatgtctt ttcaaagcaa atgaagtgcc ttttcccgag 1140
gctggggctg ggggtggctg ggaggggaag ggaagggaga ggcaagctgg ctgtgaactg 1200
tcctgttgtg gggctggagc tgctcccacc tccctgacct acccctgctg caccattccc 1260
ccagctgggc tggaaggttc cataactggc cagctgcccc cataactggc agcattccca 1320
gacccagggt actctaatag gggcggctca ggcactgaga ctaccgctca accccagggt 1380
ggttttcagg agtccgaggt agccttcaat cactggactc catggccttc ccttcgtgtt 1440
gaccggacct tccttccagg gcttttcctt tgggggaggc ggagagggga gaagaaggaa 1500
gggaagggca gaaggaagga gggaagaaaa gaaagcaaag gaacagaagg aaggaaagaa 1560
agatgggagg aagtgcagca ggaatagcac cctctccccg ggaggcccta gcttccgtga 1620
ggggccatca ccagccattc cttggagggg gctttctccc cttttgcttg agcagggttc 1680
ccaggaggga gaaagagaag acaagagcct gatgcccaac tttgtgtgtg tggggacggg 1740
ggagtcaggg ccccccaagt cccacaatag ccccaatgtt tgcctatcca cctcccccaa 1800
gcccctttac ctatgctgct gctaacgcta ctgctgctgc tgctgctgct gcttaaaggc 1860
tcatgcttgg agtggggact ggtcggtgcc cagaaagtct cttctgccac tgacgccccc 1920
atcagggatt gggccttctt tcccccttcc tttctgtgtc tcctgcctca tcggcctgcc 1980
atgacctgca gccaagccca gccccgtggg gaaggggaga aagtggggga tggctaagaa 2040

agctgggaga tagggaacag aagagggtag tgggtgggct aggggggctg ccttatttaa 2100

agtggttgtt tatgattctt atactaattt atacaaagat attaaggccc tgttcattaa 2160

gaaattgttc ccttcccctg tgttcaatgt ttgtaaagat tgttctgtgt aaatatgtct 2220

ttataataaa cagttaaaag ctg 2243

<210> 669
<211> 21
<212> DNA
<213> artificial

<220>
<223> Description of artificial sequence: Oligonucleotide

<400> 669
ttctgaagca ggactgaagg g 21

<210> 670
<211> 21
<212> DNA
<213> artificial

<220>
<223> Description of artificial sequence: Oligonucleotide

<400> 670
ggtctccaaa gtcagtccag g 21

<210> 671
<211> 1699
<212> DNA
<213> Homo sapiens

<400> 671
ctcagagagt ttctggatcc taggcttatc tccacagagg agaacacaca agcagcagag 60

accatgggaa ccctctcagc ccctccctgc acacagcgca tcaaatggaa ggggctcctg 120

ctcacagcat cacttttaaa cttctggaac ctgcccacca ctgcccaagt cacgattgaa 180

gcccagccac ccaaagtttc cgaggggaag gatgttcttc tacttgtcca caatttgccc 240

cagaatctta ccggctacat ctggtacaaa gggcaaatga gggacctcta ccattacatt 300

acatcatatg tagtagacgg tgaaataatt atatatgggc ctgcatatag tggacgagaa 360

acagcatatt ccaatgcatc cctgctgatc cagaatgtca cccgggagga cgcaggatcc 420

tacaccttac acatcataaa gggagatgat gggactagag gagtaactgg acgtttcacc 480

ttcaccttac acctggagac tcctaagccc tccatctcca gcagcaactt aaatcccagg 540

gagaccatgg aggctgtgag cttaacctgt gaccctgaga ctccagacgc aagctacctg 600

tggtggatga atggtcagag cctccctatg actcacagct tgaagctgtc cgaaaccaac 660

aggaccctct ttctattggg tgtcacaaag tatactgcag gaccctatga atgtgaaata 720

cggaacccag tgagtgccag ccgcagtgac ccagtcaccc tgaatctcct cccgaagctg 780

cccaagccct acatcaccat caacaactta aaccccaggg agaataagga tgtcttaaac 840

ttcacctgtg aacctaagag tgagaactac acctacattt ggtggctaaa tggtcagagc 900

ctcccggtca gtcccagggt aaagcgaccc attgaaaaca ggatcctcat tctacccagt 960

gtcacgagaa atgaaacagg accctatcaa tgtgaaatac gggaccgata tggtggcatc 1020

cgcagtgacc cagtcaccct gaatgtcctc tatggtccag acctccccag aatttaccct 1080

tcattcacct attaccgttc aggagaagtc ctctacttgt cctgttctgc ggactctaac 1140

ccaccggcac agtattcttg gacaattaat gaaaagtttc agctaccagg acaaaagctc 1200

tttatccgcc atattactac aaagcatagc gggctctatg tttgctctgt tcgtaactca 1260

gccactggca aggaaagctc caaatccatg acagtcgaag tctctggtaa gtggatccca 1320

gcatcgttgg caatagggtt ttaggtggag tctatctggc attcagagaa gagtcaggaa 1380

aacaattgta ttcccagcct gtgtccctag ggcacaagca aatcccaaat tctcctcctg 1440

aaccctccaa atttgtctaa gaacttcgaa aactttaaca aacaggctga tatcttcata 1500

atattcccag cctagaccaa gcaggaagaa cattgatttc attgaaataa ttgataataa 1560

tgaagataat gtttttatga tttttatttg aaaatttgct aattctttaa atggtttgtt 1620

ttctacattg atggaatttt tctcttttaa tctatctaca gcttatagca gttcaataaa 1680

ctatacttct gggaaccgt 1699

<210> 672
<211> 426
<212> PRT
<213> Homo sapiens

<400> 672

<210>  673
<211>  21
<212>  DNA
<213>  artificial

<220>
<223>  Description of artificial sequence: Oligonucleotide

<400>  673
cctgaatgtc ctctatggtc c                                                21

<210>  674
<211>  21
<212>  DNA
<213>  artificial

<220>
<223>  Description of artificial sequence: Oligonucleotide

<400>  674
tggctgagtt acgaacagag c                                                21

<210>  675
<211>  10724
<212>  DNA
<213>  Homo sapiens

<400>  675
ctttccaata tccgtcttcg gagcgcacgg gaatccgccg agctctgcgt gcaggccctt      60

ttttcttttg aggttcacat tttttgaaat tttacgccag ggcttttgta atttcctccc     120

ccgcccgctg acggtcctgg agtcgctcgg ggctttaggc cggttatgca acgtgtaccg     180

ctcggggctg ccggctgcac ctccgccgcg cctcgccgct cactgcgcta gacccggcgc     240

cccgcgtctc gcttcgcggg cagtcagggg gccggcgctc tgtcgaggtc tccagctaga     300

gcagggagcc cgagcccgag ggagtccccg gagccgacga agggcttatt agaccctgac     360

tcttttctga ggcgcgcaga ttttgtcttt gatcactccc tctccgcggg tctacggccg     420

cgcgctttcg gcgccggcga tggggagaag acggaggctg tgtctccagc tctacttcct     480

gtggctgggc tgtgtggtgc tctgggcgca gggcacggcc ggccagcctc agcctcctcc     540

gcccaagccg ccccggcccc agccgccgcc gcaacaggtt cggtccgcta cagcaggctc     600

tgaaggcggg tttctagcgc ccgagtatcg cgaggagggt gccgcagtgg ccagccgcgt     660

ccgccggcga ggacagcagg acgtgctccg agggcccaac gtgtgcggct ccagattcca     720

ctcctactgc tgccctggat ggaagacgct ccctggagga aaccagtgca ttgtcccgat     780

ttgtagaaat agttgtggag atggattttg ttcccgtcct aacatgtgta cttgttccag     840

tgggcaaata tcatcaacct gtggatcaaa atcaattcag cagtgcagtg tgagatgcat     900

gaatggtggg acctgtgcag atgaccactg ccagtgccag aaaggatata ttggaactta 960
ttgtggacaa cctgtctgtg aaaatggatg tcagaatggt ggacgttgca tcggacccaa 1020
ccgctgtgct tgtgtttatg ggttcactgg tccacagtgt gaaagagatt acaggacagg 1080
cccgtgtttc actcaggtca acaaccagat gtgccaaggg cagctgacag gcattgtctg 1140
cacgaagact ctgtgctgtg ccaccattgg acgggcgtgg ggccatccct gtgagatgtg 1200
tccagcccag cctcagccct gccgacgggg tttcatcccc aacatccgca ctggagcttg 1260
ccaagatgtt gatgaatgcc aggctatccc agggatatgc caaggaggaa actgtatcaa 1320
tacagtgggc tcttttgaat gcagatgccc tgctggtcac aaacagagtg aaactactca 1380
gaaatgtgaa gacattgatg agtgcagcat cattcctggg atatgtgaaa ctggtgaatg 1440
ttccaacacc gtgggaagct atttttgtgt ttgtccacgt ggatatgtaa cctcaacaga 1500
tggctctcga tgcatcgatc agagaacagg catgtgtttc tcgggcctgg tgaatggccg 1560
ctgtgcacaa gagctcccgg ggagaatgac gaaaatgcag tgctgctgtg agcctggccg 1620
ctgctggggc atcggaacca ttcctgaagc ctgtcctgtc agaggttctg aggaatatcg 1680
cagactttgc atggatggac ttccaatggg aggaattcca gggagtgctg gttccagacc 1740
tggaggcact gggggaaatg gctttgcccc aagtggcaat ggcaatggct atggcccagg 1800
agggacaggc ttcatcccca tccctggagg caatggcttt tctcctggcg ttgggggagc 1860
cggtgtgggg gccgggggac agggacctat catcactgga ctaacaattc tgaaccagac 1920
aatagatatc tgtaagcatc atgctaacct ttgtttaaat ggacgctgta taccaactgt 1980
ctcaagctac cgatgtgaat gcaacatggg ttataagcag gatgcaaatg gagattgtat 2040
agatgttgat gaatgcacat caaatccctg cactaatgga gattgtgtta acacacctgg 2100
ttcctattat tgtaaatgtc atgctggatt ccagaggact cctaccaagc aagcatgcat 2160
tgatattgat gagtgcatcc agaatggggt tctttgtaaa aacggtcgat gcgtgaacac 2220
agatggaagt ttccagtgca tttgcaatgc cggctttgaa ttaactacag atggaaaaaa 2280
ctgtgttgat catgatgaat gtacaactac caacatgtgt ttgaatggaa tgtgcatcaa 2340
tgaagatggc agcttcaagt gcatctgcaa accaggattt gtcttggctc caaatgggcg 2400
ttactgtact gatgttgatg aatgccagac cccaggaatc tgcatgaatg ggcactgcat 2460
caacagtgaa gggtccttcc gctgtgactg tcccccaggc ctggctgtgg gcatggatgg 2520
acgtgtgtgt gttgatactc acatgcgcag tacctgctat ggaggaatca agaaaggagt 2580
gtgtgtgcgt cctttccccg gtgcagtgac caagtccgaa tgctgctgtg ccaatccaga 2640
ctatggtttt ggagaaccct gccagccatg ccctgcaaaa aattcagctg aattccacgg 2700
cctttgtagt agtggagtag gtatcactgt ggatggaaga gatatcaatg aatgtgcttt 2760
ggatcctgat atatgtgcca atgggatttg tgaaaactta cgtggtagtt accgttgtaa 2820
ttgcaacagt ggctatgaac cagatgcctc tggaagaaac tgtattgaca ttgatgaatg 2880
tttagtaaac agactgcttt gtgataacgg attgtgccga aacacgccag gaagttacag 2940

ctgtacgtgc ccaccagggt atgtgttcag gactgagaca gagacctgtg aagatataaa 3000
tgaatgtgaa agcaacccat gtgtcaatgg ggcctgcaga aacaaccttg gatctttcaa 3060
ttgtgaatgt tcgcccggca gcaaactcag ctccacagga ttgatctgta ttgacagcct 3120
gaaggggacc tgttggctca acatccagga cagccgctgt gaggtgaata ttaatggagc 3180
cactctgaaa tctgaatgct gtgccaccct cggagccgcc tgggggagcc cctgtgagcg 3240
gtgtgaacta gatacagctt gcccaagagg gcttgccagg attaaaggtg ttacgtgtga 3300
agatgttaat gagtgtgagg tgttccctgg cgtttgtcca aatggacgct gtgtcaacag 3360
taagggatct tttcattgcg agtgccctga aggccttacg ttggatggga ctggccgtgt 3420
atgtttggat attcgcatgg agcagtgtta cttgaagtgg gatgaagatg aatgcatcca 3480
ccccgttcct ggaaagttcc gcatggatgc ctgctgctgt gctgtcgggg cggcttgggg 3540
caccgagtgt gaggagtgcc ccaaacctgg caccaaggaa tacgagacgc tgtgcccccg 3600
cggggctggc tttgctaacc gaggggatgt tcttactggg cggccatttt acaaagacat 3660
caatgaatgc aaagcatttc ctgggatgtg cacttatggg aagtgcagaa atacaatcgg 3720
aagcttcaaa tgccgttgca atagtggctt tgctctagac atggaggaaa gaaactgcac 3780
ggacatcgac gagtgcagga tttctcctga cctctgtggc agtggaatct gcgtcaatac 3840
accgggcagc tttgagtgcg agtgcttcga aggctatgaa agtggcttca tgatgatgaa 3900
gaactgcatg gacattgacg aatgtgaacg taaccctctc ctttgtaggg gtggcacctg 3960
tgtgaacact gagggcagct ttcagtgtga ctgcccactg ggacacgagc tgtcaccatc 4020
ccgtgaggac tgtgtggata ttaatgaatg ctccctgagt gacaatctct gcagaaatgg 4080
aaaatgtgtg aacatgattg gaacctatca gtgctcttgc aatcctggat atcaggctac 4140
gccagaccgc cagggctgta cagatattga tgaatgtatg ataatgaacg gaggctgtga 4200
cacccagtgc acaaattcag agggaagcta cgaatgcagc tgcagtgagg gttatgccct 4260
gatgccagat gggagatcgt gtgcagacat tgatgaatgt gaaaacaatc ctgatatctg 4320
tgatggcggc cagtgtacca acattcctgg agagtatcgc tgcctctgct atgatggctt 4380
catggcttcc atggacatga aaacatgcat tgatgtcaat gaatgtgacc taaattcaaa 4440
tatctgcatg tttggggaat gtgagaacac aaagggatcc ttcatttgcc actgtcagct 4500
gggttactca gtgaagaagg ggaccacagg atgtacagat gtggatgagt gtgaaattgg 4560
tgctcataac tgcgacatgc atgcctcatg tctgaatatc ccaggaagct tcaagtgtag 4620
ctgcagagaa ggctggattg gaaacggcat caagtgtatt gatctggacg aatgttctaa 4680
tggaacccac cagtgtagca tcaatgctca gtgtgtaaat accccgggct cataccgctg 4740
tgcctgctcc gaaggtttca ctggtgatgg ctttacctgc tcagatgttg atgagtgtgc 4800
agaaaacata aacctctgtg agaacggaca gtgccttaat gtcccgggtg catatcgctg 4860
cgagtgtgag atgggcttca ctccagcctc agacagcaga tcctgccaag atattgatga 4920
atgctccttc caaaacattt gtgtctttgg aacatgtaat aacctgcctg gaatgtttca 4980

ttgcatctgc gatgatggtt atgaattgga cagaacagga gggaactgta cagatattga 5040
tgagtgtgca gatcctataa actgtgtcaa tggcctatgt gtcaacacgc ctggtcgcta 5100
tgagtgtaac tgcccacccg attttcagtt gaacccaact ggtgtgggtt gtgttgacaa 5160
ccgtgtgggc aactgctacc tgaagtttgg acctcgagga gatgggagtc tgtcttgcaa 5220
caccgagatc ggggtgggcg tcagtcgctc ttcatgctgc tgctctctgg gaaaggcctg 5280
gggaaacccc tgtgagacat gcccccctgt caatagcact gaatattaca ccctgtgtcc 5340
cggaggtgaa ggcttcagac ctaaccccat cacaatcatt ttagaagaca ttgacgaatg 5400
ccaggagtta ccaggtctct gccagggtgg aaactgcatc aacacttttg ggagcttcca 5460
gtgtgagtgc ccacaaggct actacctcag cgaggatacc cgcatctgtg aagatattga 5520
tgagtgtttt gcacatcctg gtgtgtgtgg gcctgggacc tgctataaca ccctgggaaa 5580
ttacacctgc atttgcccac ctgagtacat gcaggtcaat ggaggccaca actgcatgga 5640
catgagaaaa agcttttgct accgaagcta taatggaacc acttgtgaga atgagttgcc 5700
tttcaatgtg acaaaaagga tgtgctgctg cacatataat gtgggcaaag cctggaacaa 5760
accttgtgaa ccatgcccaa ctccaggaac agctgacttt aaaaccatat gtggaaatat 5820
tcctggattc acctttgaca ttcacacagg aaaagctgtt gacattgatg aatgtaaaga 5880
gattccaggc atttgtgcaa atggtgtgtg cattaaccag attggcagtt tccgctgtga 5940
atgccctaca ggattcagtt acaatgacct gctgttggtt tgtgaagata tagatgagtg 6000
cagcaatggt gataatctct gccagcggaa tgcagactgc atcaatagtc ctggtagtta 6060
ccgctgtgaa tgtgccgcgg gtttcaaact ttcacccaat ggggcctgtg tagatcgcaa 6120
tgaatgttta gaaattccta acgtttgcag tcatggcttg tgtgttgatc tgcaaggaag 6180
ttaccagtgc atctgccaca atggctttaa ggcttctcag gaccagacca tgtgcatgga 6240
tgttgatgag tgcgagcggc atccatgtgg aaatggaact tgtaaaaaca ccgttggatc 6300
ctataactgt ctgtgctacc cagggtttga actcactcat aataatgatt gcctggacat 6360
agatgagtgc agttcctttt ttggtcaggt gtgcagaaat ggacgttgtt ttaatgaaat 6420
tggttctttc aagtgtctat gtaacgaagg ttatgaactt accccagatg gcaaaaactg 6480
tatagacact aatgagtgtg tcgcccttcc cggctcttgc tctcctggta cctgtcagaa 6540
tttggaggga tccttcagat gcatctgtcc cccagggtat gaagtaaaaa gcgagaactg 6600
cattgatata aatgaatgtg atgaagatcc caacatttgt ctttttggtt cctgtactaa 6660
tactccaggg ggcttccagt gcctctgccc ccctggcttt gtactatctg ataatggacg 6720
gagatgcttt gatactcgcc agagcttctg cttcacaaat tttgaaaatg gaaagtgttc 6780
tgtacccaaa gctttcaaca ccacaaaagc aaaatgctgc tgtagtaaga tgccaggaga 6840
gggctggggg gacccctgtg agctgtgccc caaagacgat gaagttgcat ttcaggattt 6900
gtgtccatat ggccatggaa ctgtccctag tcttcatgat acacgtgaag atgtcaatga 6960
gtgtcttgag agcccaggca tttgttcaaa tggtcaatgt atcaacaccg acggatcttt 7020

tcgctgtgaa tgtccaatgg gctacaacct tgactacact ggagtacgct gtgtggatac 7080
tgatgagtgt tcaatcggca atccgtgtgg aaatggtaca tgcaccaatg ttattgggag 7140
ttttgaatgc aattgcaatg aaggctttga gccagggccc atgatgaatt gtgaagatat 7200
caacgaatgt gcccagaacc cactgctgtg tgctttccgc tgcatgaaca cttttgggtc 7260
ctatgaatgc acgtgcccga ttggctatgc cctcagggaa gatcaaaaga tgtgcaaaga 7320
tctggatgaa tgtgctgaag ggttacacga ctgtgaatct aggggcatga tgtgtaagaa 7380
tctaatcggc accttcatgt gcatctgccc tcctggaatg gcccgaaggc ccgatggaga 7440
aggctgtgta gatgaaaatg aatgcaggac caagccagga atctgtgaaa atggacgttg 7500
tgttaacatt attggaagct atagatgtga gtgtaatgaa ggattccagt caagttcttc 7560
aggcactgaa tgccttgaca atcgacaggg tctctgcttt gcagaggtac tgcagacaat 7620
atgtcaaatg gcatccagta gtcgcaatct cgtcactaag tcagaatgct gctgtgatgg 7680
tgggcgaggc tggggccacc agtgcgagct ttgcccactt cctggaactg cccagtacaa 7740
aaagatatgt cctcatggcc caggatatac aactgatgga agagatattg atgaatgtaa 7800
ggtaatgcca aacctctgca ccaatggtca gtgcatcaat accatgggct cattccgatg 7860
cttctgcaag gttggctaca ccacagacat cagtggaacc tcttgtatag accttgatga 7920
atgctcccag tccccgaaac catgcaacta catctgcaag aacactgagg ggagttatca 7980
gtgttcatgt ccgagggggt atgtcctgca agaggatgga aagacatgca aagaccttga 8040
tgaatgtcaa acaaagcagc ataactgcca gttcctctgt gtcaacaccc tgggggggtt 8100
tacctgtaaa tgtccacctg gtttcacaca gcatcacact gcttgtatcg acaacaacga 8160
atgtgggtct caaccttcgc tttgtggagc aaagggaatc tgtcaaaaca ctccaggcag 8220
tttcagctgt gaatgccaaa gagggttctc tcttgatgcc accggactga actgtgaaga 8280
tgttgatgaa tgtgatggga accacaggtg ccaacacggc tgccagaaca tcctgggtgg 8340
ctacagatgt ggctgccccc aaggctacat ccagcactac cagtggaatc agtgtgtcga 8400
tgagaatgaa tgctccaatc ccaatgcctg tggctctgct tcctgctaca acaccctggg 8460
gagttacaag tgcgcctgcc cctcggggtt ctccttcgac cagttctcca gtgcctgcca 8520
cgacgtgaat gagtgctcgt cctccaagaa cccctgcaat tacggctgct ctaacacgga 8580
ggggggctac ctctgtggct gcccccctgg gtattacaga gtgggacaag gccactgtgt 8640
ctcaggaatg ggatttaaca aggggcagta cctgtcactg gatacagagg tcgatgagga 8700
aaatgctctg tccccagaag catgctacga gtgcaaaatc aacggctatt ctaagaaaga 8760
cagcaggcag aagagaagta ttcatgaacc tgatcccact gctgttgaac agatcagcct 8820
agagagtgtc gacatggaca gccccgtcaa catgaagttc aacctctccc acctcggctc 8880
taaggagcac atcctggaac taaggcccgc catccagccc ctcaacaacc acatccgtta 8940
tgtcatctct caagggaacg atgacagcgt cttccgcatc caccaaagga atgggctcag 9000
ctacttgcac acggccaaga agaagctcat gcccggcaca tacacactgg aaatcactag 9060

catccctctc tacaagaaga aggagcttaa gaaactggaa gagagcaatg aggatgacta 9120
cctcctaggg gagcttgggg aggctctcag aatgaggctg cagattcagc tctattaacc 9180
cttcacagac ttgggcccag gctcaaatcc tagcacagcc agtctgcaga agcatttgaa 9240
aagtcaagga ctaattttaa agaggaaaaa taataataac tcttgtttct ttcctccctg 9300
tcttagactt tgaatgttga ccctcacagg gagggataat ttagactctg gtatggccaa 9360
agatttgagc acaaaggcaa ccgtggttac tgtatttttt atataacttc attttaaaat 9420
atattaaaag aaacctaaat gttcaagata tcagcatatg gcactaaatg cacaaaaata 9480
atgtgagctt tttttttttt tcctgttagc agtctgtaac actttgggta ttttgctata 9540
gttgctaatt aaaaaaatat agatgtttat ttatttttaa tgcagtaata tatggagaaa 9600
tgaacaaact atgtaaacaa aaagggaaac tcacttgttt ttctttagat ttataaattt 9660
gagctatttt ttttagaggt gctttttaaa aatccaatag atacaagaga tgtttccttt 9720
ggttttctgc cagtcatcca gctgatacac acctgatgat tttaaagaaa gccacacaga 9780
gctgaatggg cagtgtaatc aataatttaa aaaacatgaa tgtcattaga tcctttataa 9840
cgtagatcga agccaaagca gctcatttgt gacaacattt catatcacca gacacaccag 9900
gcaacagaag ttgaagcaca accactgtag caaaatacct tgactgcttg tgagaccatt 9960
agcattgcag gccaaaccgt actgtatttc cttctcataa cctcaaggaa ccatatgtgc 10020
tacccacaac acctcattct tacccagggt gcgctgcgtc ctcatggtac tgtaggcagc 10080
tgaagaaccg ccgttccctt gaaagggaac acctggcatt ctgtggtgtt tcgtgctgtc 10140
ttaaataatg gtgcatttat tatgttcaag ttatttcagg attgccatat gtgcaaacaa 10200
atcatgcaat gcagccaagg aatatatgtt gttgttgttg ttttaaaccc attttttttt 10260
tagaattttc attaatactg tagttataca ccatatgcct cattttatca tagcctattg 10320
tgtatgaaag atgtttgtac aatgaattga tgtttagttt gctttagtca tttaaaaaga 10380
tattgtacca ggatgtgcta ttaagagcac gtatccatta ttcttctcaa cccaagaacc 10440
tgtttcctgg accagtgacc aaacctcata tgtgaaatgg ccaaagcaca tgcaggctcc 10500
tggttgttcc tctcaaacct gtgctgacca aagattagta accagttata cccagtattt 10560
tgaggtttta ttgttttttt aataactaaa aagaaaaaag gaacagtgta aatttgtaat 10620
caagagtttg tgaggaaaaa cttatggttt ttggttttgt ttgtttttgt aggtctgtgt 10680
atcaaatatg acacttttct tgcaataaag cttattttgg ggta 10724

<210> 676
<211> 2912
<212> PRT
<213> Homo sapiens

<400> 676
   Met Gly Arg Arg Arg Arg Leu Cys Leu Gln Leu Tyr Phe Leu Trp Leu
   1 5 10 15
   Gly Cys Val Val Leu Trp Ala Gln Gly Thr Ala Gly Gln Pro Gln Pro
               20                  25                  30
   Pro Pro Pro Lys Pro Pro Arg Pro Gln Pro Pro Pro Gln Gln Val Arg
           35                  40                  45
   Ser Ala Thr Ala Gly Ser Glu Gly Gly Phe Leu Ala Pro Glu Tyr Arg
       50                  55                  60
   Glu Glu Gly Ala Ala Val Ala Ser Arg Val Arg Arg Arg Gly Gln Gln
   65                  70                  75                  80
   Asp Val Leu Arg Gly Pro Asn Val Cys Gly Ser Arg Phe His Ser Tyr
                   85                  90                  95
   Cys Cys Pro Gly Trp Lys Thr Leu Pro Gly Gly Asn Gln Cys Ile Val
               100                 105                 110
   Pro Ile Cys Arg Asn Ser Cys Gly Asp Gly Phe Cys Ser Arg Pro Asn
           115                 120                 125
   Met Cys Thr Cys Ser Ser Gly Gln Ile Ser Ser Thr Cys Gly Ser Lys
       130                 135                 140
   Ser Ile Gln Gln Cys Ser Val Arg Cys Met Asn Gly Gly Thr Cys Ala
   145                 150                 155                 160
   Asp Asp His Cys Gln Cys Gln Lys Gly Tyr Ile Gly Thr Tyr Cys Gly
                   165                 170                 175
   Gln Pro Val Cys Glu Asn Gly Cys Gln Asn Gly Gly Arg Cys Ile Gly
               180                 185                 190
   Pro Asn Arg Cys Ala Cys Val Tyr Gly Phe Thr Gly Pro Gln Cys Glu
           195                 200                 205
   Arg Asp Tyr Arg Thr Gly Pro Cys Phe Thr Gln Val Asn Asn Gln Met
       210                 215                 220
   Cys Gln Gly Gln Leu Thr Gly Ile Val Cys Thr Lys Thr Leu Cys Cys
   225                 230                 235                 240
   Ala Thr Ile Gly Arg Ala Trp Gly His Pro Cys Glu Met Cys Pro Ala
                   245                 250                 255
   Gln Pro Gln Pro Cys Arg Arg Gly Phe Ile Pro Asn Ile Arg Thr Gly
               260                 265                 270
   Ala Cys Gln Asp Val Asp Glu Cys Gln Ala Ile Pro Gly Ile Cys Gln
           275                 280                 285
   Gly Gly Asn Cys Ile Asn Thr Val Gly Ser Phe Glu Cys Arg Cys Pro
       290                 295                 300
   Ala Gly His Lys Gln Ser Glu Thr Thr Gln Lys Cys Glu Asp Ile Asp
   305                 310                 315                 320
   Glu Cys Ser Ile Ile Pro Gly Ile Cys Glu Thr Gly Glu Cys Ser Asn
                   325                 330                 335
   Thr Val Gly Ser Tyr Phe Cys Val Cys Pro Arg Gly Tyr Val Thr Ser
               340                 345                 350
   Thr Asp Gly Ser Arg Cys Ile Asp Gln Arg Thr Gly Met Cys Phe Ser
           355                 360                 365
   Gly Leu Val Asn Gly Arg Cys Ala Gln Glu Leu Pro Gly Arg Met Thr
       370                 375                 380
   Lys Met Gln Cys Cys Cys Glu Pro Gly Arg Cys Trp Gly Ile Gly Thr
   385                 390                 395                 400
   Ile Pro Glu Ala Cys Pro Val Arg Gly Ser Glu Glu Tyr Arg Arg Leu
                   405                 410                 415
   Cys Met Asp Gly Leu Pro Met Gly Gly Ile Pro Gly Ser Ala Gly Ser
               420                 425                 430
   Arg Pro Gly Gly Thr Gly Gly Asn Gly Phe Ala Pro Ser Gly Asn Gly
           435                 440                 445
   Asn Gly Tyr Gly Pro Gly Gly Thr Gly Phe Ile Pro Ile Pro Gly Gly
       450                 455                 460
   Asn Gly Phe Ser Pro Gly Val Gly Gly Ala Gly Val Gly Ala Gly Gly
   465                 470                 475                 480
   Gln Gly Pro Ile Ile Thr Gly Leu Thr Ile Leu Asn Gln Thr Ile Asp
                   485                 490                 495
   Ile Cys Lys His His Ala Asn Leu Cys Leu Asn Gly Arg Cys Ile Pro
               500                 505                 510
   Thr Val Ser Ser Tyr Arg Cys Glu Cys Asn Met Gly Tyr Lys Gln Asp
           515                 520                 525
   Ala Asn Gly Asp Cys Ile Asp Val Asp Glu Cys Thr Ser Asn Pro Cys
       530                 535                 540
   Thr Asn Gly Asp Cys Val Asn Thr Pro Gly Ser Tyr Tyr Cys Lys Cys
   545                 550                 555                 560
   His Ala Gly Phe Gln Arg Thr Pro Thr Lys Gln Ala Cys Ile Asp Ile
                   565                 570                 575
   Asp Glu Cys Ile Gln Asn Gly Val Leu Cys Lys Asn Gly Arg Cys Val
               580                 585                 590
   Asn Thr Asp Gly Ser Phe Gln Cys Ile Cys Asn Ala Gly Phe Glu Leu
           595                 600                 605
   Thr Thr Asp Gly Lys Asn Cys Val Asp His Asp Glu Cys Thr Thr Thr
       610                 615                 620
   Asn Met Cys Leu Asn Gly Met Cys Ile Asn Glu Asp Gly Ser Phe Lys
   625                 630                 635                 640
   Cys Ile Cys Lys Pro Gly Phe Val Leu Ala Pro Asn Gly Arg Tyr Cys
                   645                 650                 655
   Thr Asp Val Asp Glu Cys Gln Thr Pro Gly Ile Cys Met Asn Gly His
               660                 665                 670
   Cys Ile Asn Ser Glu Gly Ser Phe Arg Cys Asp Cys Pro Pro Gly Leu
           675                 680                 685
   Ala Val Gly Met Asp Gly Arg Val Cys Val Asp Thr His Met Arg Ser
       690                 695                 700
   Thr Cys Tyr Gly Gly Ile Lys Lys Gly Val Cys Val Arg Pro Phe Pro
   705                 710                 715                 720
   Gly Ala Val Thr Lys Ser Glu Cys Cys Cys Ala Asn Pro Asp Tyr Gly
                   725                 730                 735
   Phe Gly Glu Pro Cys Gln Pro Cys Pro Ala Lys Asn Ser Ala Glu Phe
               740                 745                 750
   His Gly Leu Cys Ser Ser Gly Val Gly Ile Thr Val Asp Gly Arg Asp
           755                 760                 765
   Ile Asn Glu Cys Ala Leu Asp Pro Asp Ile Cys Ala Asn Gly Ile Cys
       770                 775                 780
   Glu Asn Leu Arg Gly Ser Tyr Arg Cys Asn Cys Asn Ser Gly Tyr Glu
   785                 790                 795                 800
   Pro Asp Ala Ser Gly Arg Asn Cys Ile Asp Ile Asp Glu Cys Leu Val
                   805                 810                 815
   Asn Arg Leu Leu Cys Asp Asn Gly Leu Cys Arg Asn Thr Pro Gly Ser
               820                 825                 830
   Tyr Ser Cys Thr Cys Pro Pro Gly Tyr Val Phe Arg Thr Glu Thr Glu
   835 840 845
   Thr Cys Glu Asp Ile Asn Glu Cys Glu Ser Asn Pro Cys Val Asn Gly
   850 855 860
   Ala Cys Arg Asn Asn Leu Gly Ser Phe Asn Cys Glu Cys Ser Pro Gly
   865 870 875 880
   Ser Lys Leu Ser Ser Thr Gly Leu Ile Cys Ile Asp Ser Leu Lys Gly
   885 890 895
   Thr Cys Trp Leu Asn Ile Gln Asp Ser Arg Cys Glu Val Asn Ile Asn
   900 905 910
   Gly Ala Thr Leu Lys Ser Glu Cys Cys Ala Thr Leu Gly Ala Ala Trp
   915 920 925
   Gly Ser Pro Cys Glu Arg Cys Glu Leu Asp Thr Ala Cys Pro Arg Gly
   930 935 940
   Leu Ala Arg Ile Lys Gly Val Thr Cys Glu Asp Val Asn Glu Cys Glu
   945 950 955 960
   Val Phe Pro Gly Val Cys Pro Asn Gly Arg Cys Val Asn Ser Lys Gly
   965 970 975
   Ser Phe His Cys Glu Cys Pro Glu Gly Leu Thr Leu Asp Gly Thr Gly
   980 985 990
   Arg Val Cys Leu Asp Ile Arg Met Glu Gln Cys Tyr Leu Lys Trp Asp
   995 1000 1005
   Glu Asp Glu Cys Ile His Pro Val Pro Gly Lys Phe Arg Met Asp
   1010 1015 1020
   Ala Cys Cys Cys Ala Val Gly Ala Ala Trp Gly Thr Glu Cys Glu
   1025 1030 1035
   Glu Cys Pro Lys Pro Gly Thr Lys Glu Tyr Glu Thr Leu Cys Pro
   1040 1045 1050
   Arg Gly Ala Gly Phe Ala Asn Arg Gly Asp Val Leu Thr Gly Arg
   1055 1060 1065
   Pro Phe Tyr Lys Asp Ile Asn Glu Cys Lys Ala Phe Pro Gly Met
   1070 1075 1080
   Cys Thr Tyr Gly Lys Cys Arg Asn Thr Ile Gly Ser Phe Lys Cys
   1085 1090 1095
   Arg Cys Asn Ser Gly Phe Ala Leu Asp Met Glu Glu Arg Asn Cys
       1100                1105                1110
   Thr Asp Ile Asp Glu Cys Arg Ile Ser Pro Asp Leu Cys Gly Ser
       1115                1120                1125
   Gly Ile Cys Val Asn Thr Pro Gly Ser Phe Glu Cys Glu Cys Phe
       1130                1135                1140
   Glu Gly Tyr Glu Ser Gly Phe Met Met Met Lys Asn Cys Met Asp
       1145                1150                1155
   Ile Asp Glu Cys Glu Arg Asn Pro Leu Leu Cys Arg Gly Gly Thr
       1160                1165                1170
   Cys Val Asn Thr Glu Gly Ser Phe Gln Cys Asp Cys Pro Leu Gly
       1175                1180                1185
   His Glu Leu Ser Pro Ser Arg Glu Asp Cys Val Asp Ile Asn Glu
       1190                1195                1200
   Cys Ser Leu Ser Asp Asn Leu Cys Arg Asn Gly Lys Cys Val Asn
       1205                1210                1215
   Met Ile Gly Thr Tyr Gln Cys Ser Cys Asn Pro Gly Tyr Gln Ala
       1220                1225                1230
   Thr Pro Asp Arg Gln Gly Cys Thr Asp Ile Asp Glu Cys Met Ile
       1235                1240                1245
   Met Asn Gly Gly Cys Asp Thr Gln Cys Thr Asn Ser Glu Gly Ser
       1250                1255                1260
   Tyr Glu Cys Ser Cys Ser Glu Gly Tyr Ala Leu Met Pro Asp Gly
       1265                1270                1275
   Arg Ser Cys Ala Asp Ile Asp Glu Cys Glu Asn Asn Pro Asp Ile
       1280                1285                1290
   Cys Asp Gly Gly Gln Cys Thr Asn Ile Pro Gly Glu Tyr Arg Cys
       1295                1300                1305
   Leu Cys Tyr Asp Gly Phe Met Ala Ser Met Asp Met Lys Thr Cys
       1310                1315                1320
   Ile Asp Val Asn Glu Cys Asp Leu Asn Ser Asn Ile Cys Met Phe
       1325                1330                1335
   Gly Glu Cys Glu Asn Thr Lys Gly Ser Phe Ile Cys His Cys Gln
       1340                1345                1350
   Leu Gly Tyr Ser Val Lys Lys Gly Thr Thr Gly Cys Thr Asp Val
       1355                1360                1365
   Asp Glu Cys Glu Ile Gly Ala His Asn Cys Asp Met His Ala Ser
       1370                1375                1380
   Cys Leu Asn Ile Pro Gly Ser Phe Lys Cys Ser Cys Arg Glu Gly
       1385                1390                1395
   Trp Ile Gly Asn Gly Ile Lys Cys Ile Asp Leu Asp Glu Cys Ser
       1400                1405                1410
   Asn Gly Thr His Gln Cys Ser Ile Asn Ala Gln Cys Val Asn Thr
       1415                1420                1425
   Pro Gly Ser Tyr Arg Cys Ala Cys Ser Glu Gly Phe Thr Gly Asp
       1430                1435                1440
   Gly Phe Thr Cys Ser Asp Val Asp Glu Cys Ala Glu Asn Ile Asn
       1445                1450                1455
   Leu Cys Glu Asn Gly Gln Cys Leu Asn Val Pro Gly Ala Tyr Arg
       1460                1465                1470
   Cys Glu Cys Glu Met Gly Phe Thr Pro Ala Ser Asp Ser Arg Ser
       1475                1480                1485
   Cys Gln Asp Ile Asp Glu Cys Ser Phe Gln Asn Ile Cys Val Phe
       1490                1495                1500
   Gly Thr Cys Asn Asn Leu Pro Gly Met Phe His Cys Ile Cys Asp
       1505                1510                1515
   Asp Gly Tyr Glu Leu Asp Arg Thr Gly Gly Asn Cys Thr Asp Ile
       1520                1525                1530
   Asp Glu Cys Ala Asp Pro Ile Asn Cys Val Asn Gly Leu Cys Val
       1535                1540                1545
   Asn Thr Pro Gly Arg Tyr Glu Cys Asn Cys Pro Pro Asp Phe Gln
       1550                1555                1560
   Leu Asn Pro Thr Gly Val Gly Cys Val Asp Asn Arg Val Gly Asn
       1565                1570                1575
   Cys Tyr Leu Lys Phe Gly Pro Arg Gly Asp Gly Ser Leu Ser Cys
       1580                1585                1590
   Asn Thr Glu Ile Gly Val Gly Val Ser Arg Ser Ser Cys Cys Cys
       1595                1600                1605
   Ser Leu Gly Lys Ala Trp Gly Asn Pro Cys Glu Thr Cys Pro Pro
       1610                1615                1620
   Val Asn Ser Thr Glu Tyr Tyr Thr Leu Cys Pro Gly Gly Glu Gly
       1625                1630                1635
   Phe Arg Pro Asn Pro Ile Thr Ile Ile Leu Glu Asp Ile Asp Glu
       1640                1645                1650
   Cys Gln Glu Leu Pro Gly Leu Cys Gln Gly Gly Asn Cys Ile Asn
       1655                1660                1665
   Thr Phe Gly Ser Phe Gln Cys Glu Cys Pro Gln Gly Tyr Tyr Leu
       1670                1675                1680
   Ser Glu Asp Thr Arg Ile Cys Glu Asp Ile Asp Glu Cys Phe Ala
       1685                1690                1695
   His Pro Gly Val Cys Gly Pro Gly Thr Cys Tyr Asn Thr Leu Gly
       1700                1705                1710
   Asn Tyr Thr Cys Ile Cys Pro Pro Glu Tyr Met Gln Val Asn Gly
       1715                1720                1725
   Gly His Asn Cys Met Asp Met Arg Lys Ser Phe Cys Tyr Arg Ser
       1730                1735                1740
   Tyr Asn Gly Thr Thr Cys Glu Asn Glu Leu Pro Phe Asn Val Thr
       1745                1750                1755
   Lys Arg Met Cys Cys Cys Thr Tyr Asn Val Gly Lys Ala Trp Asn
       1760                1765                1770
   Lys Pro Cys Glu Pro Cys Pro Thr Pro Gly Thr Ala Asp Phe Lys
       1775                1780                1785
   Thr Ile Cys Gly Asn Ile Pro Gly Phe Thr Phe Asp Ile His Thr
       1790                1795                1800
   Gly Lys Ala Val Asp Ile Asp Glu Cys Lys Glu Ile Pro Gly Ile
       1805                1810                1815
   Cys Ala Asn Gly Val Cys Ile Asn Gln Ile Gly Ser Phe Arg Cys
       1820                1825                1830
   Glu Cys Pro Thr Gly Phe Ser Tyr Asn Asp Leu Leu Leu Val Cys
       1835                1840                1845
   Glu Asp Ile Asp Glu Cys Ser Asn Gly Asp Asn Leu Cys Gln Arg
       1850                1855                1860
   Asn Ala Asp Cys Ile Asn Ser Pro Gly Ser Tyr Arg Cys Glu Cys
       1865                1870                1875
   Ala Ala Gly Phe Lys Leu Ser Pro Asn Gly Ala Cys Val Asp Arg
       1880                1885                1890
   Asn Glu Cys Leu Glu Ile Pro Asn Val Cys Ser His Gly Leu Cys
       1895                1900                1905
   Val Asp Leu Gln Gly Ser Tyr Gln Cys Ile Cys His Asn Gly Phe
       1910                1915                1920
   Lys Ala Ser Gln Asp Gln Thr Met Cys Met Asp Val Asp Glu Cys
       1925                1930                1935
   Glu Arg His Pro Cys Gly Asn Gly Thr Cys Lys Asn Thr Val Gly
       1940                1945                1950
   Ser Tyr Asn Cys Leu Cys Tyr Pro Gly Phe Glu Leu Thr His Asn
       1955                1960                1965
   Asn Asp Cys Leu Asp Ile Asp Glu Cys Ser Ser Phe Phe Gly Gln
       1970                1975                1980
   Val Cys Arg Asn Gly Arg Cys Phe Asn Glu Ile Gly Ser Phe Lys
       1985                1990                1995
   Cys Leu Cys Asn Glu Gly Tyr Glu Leu Thr Pro Asp Gly Lys Asn
       2000                2005                2010
   Cys Ile Asp Thr Asn Glu Cys Val Ala Leu Pro Gly Ser Cys Ser
       2015                2020                2025
   Pro Gly Thr Cys Gln Asn Leu Glu Gly Ser Phe Arg Cys Ile Cys
       2030                2035                2040
   Pro Pro Gly Tyr Glu Val Lys Ser Glu Asn Cys Ile Asp Ile Asn
       2045                2050                2055
   Glu Cys Asp Glu Asp Pro Asn Ile Cys Leu Phe Gly Ser Cys Thr
       2060                2065                2070
   Asn Thr Pro Gly Gly Phe Gln Cys Leu Cys Pro Pro Gly Phe Val
       2075                2080                2085
   Leu Ser Asp Asn Gly Arg Arg Cys Phe Asp Thr Arg Gln Ser Phe
       2090                2095                2100
   Cys Phe Thr Asn Phe Glu Asn Gly Lys Cys Ser Val Pro Lys Ala
       2105                2110                2115
   Phe Asn Thr Thr Lys Ala Lys Cys Cys Cys Ser Lys Met Pro Gly
   2120 2125 2130
   Glu Gly Trp Gly Asp Pro Cys Glu Leu Cys Pro Lys Asp Asp Glu
   2135 2140 2145
   Val Ala Phe Gln Asp Leu Cys Pro Tyr Gly His Gly Thr Val Pro
   2150 2155 2160
   Ser Leu His Asp Thr Arg Glu Asp Val Asn Glu Cys Leu Glu Ser
   2165 2170 2175
   Pro Gly Ile Cys Ser Asn Gly Gln Cys Ile Asn Thr Asp Gly Ser
   2180 2185 2190
   Phe Arg Cys Glu Cys Pro Met Gly Tyr Asn Leu Asp Tyr Thr Gly
   2195 2200 2205
   Val Arg Cys Val Asp Thr Asp Glu Cys Ser Ile Gly Asn Pro Cys
   2210 2215 2220
   Gly Asn Gly Thr Cys Thr Asn Val Ile Gly Ser Phe Glu Cys Asn
   2225 2230 2235
   Cys Asn Glu Gly Phe Glu Pro Gly Pro Met Met Asn Cys Glu Asp
   2240 2245 2250
   Ile Asn Glu Cys Ala Gln Asn Pro Leu Leu Cys Ala Phe Arg Cys
   2255 2260 2265
   Met Asn Thr Phe Gly Ser Tyr Glu Cys Thr Cys Pro Ile Gly Tyr
   2270 2275 2280
   Ala Leu Arg Glu Asp Gln Lys Met Cys Lys Asp Leu Asp Glu Cys
   2285 2290 2295
   Ala Glu Gly Leu His Asp Cys Glu Ser Arg Gly Met Met Cys Lys
   2300 2305 2310
   Asn Leu Ile Gly Thr Phe Met Cys Ile Cys Pro Pro Gly Met Ala
   2315 2320 2325
   Arg Arg Pro Asp Gly Glu Gly Cys Val Asp Glu Asn Glu Cys Arg
   2330 2335 2340
   Thr Lys Pro Gly Ile Cys Glu Asn Gly Arg Cys Val Asn Ile Ile
   2345 2350 2355
   Gly Ser Tyr Arg Cys Glu Cys Asn Glu Gly Phe Gln Ser Ser Ser
   2360 2365 2370
   Ser Gly Thr Glu Cys Leu Asp Asn Arg Gln Gly Leu Cys Phe Ala
       2375                2380                2385
   Glu Val Leu Gln Thr Ile Cys Gln Met Ala Ser Ser Ser Arg Asn
       2390                2395                2400
   Leu Val Thr Lys Ser Glu Cys Cys Cys Asp Gly Gly Arg Gly Trp
       2405                2410                2415
   Gly His Gln Cys Glu Leu Cys Pro Leu Pro Gly Thr Ala Gln Tyr
       2420                2425                2430
   Lys Lys Ile Cys Pro His Gly Pro Gly Tyr Thr Thr Asp Gly Arg
       2435                2440                2445
   Asp Ile Asp Glu Cys Lys Val Met Pro Asn Leu Cys Thr Asn Gly
       2450                2455                2460
   Gln Cys Ile Asn Thr Met Gly Ser Phe Arg Cys Phe Cys Lys Val
       2465                2470                2475
   Gly Tyr Thr Thr Asp Ile Ser Gly Thr Ser Cys Ile Asp Leu Asp
       2480                2485                2490
   Glu Cys Ser Gln Ser Pro Lys Pro Cys Asn Tyr Ile Cys Lys Asn
       2495                2500                2505
   Thr Glu Gly Ser Tyr Gln Cys Ser Cys Pro Arg Gly Tyr Val Leu
       2510                2515                2520
   Gln Glu Asp Gly Lys Thr Cys Lys Asp Leu Asp Glu Cys Gln Thr
       2525                2530                2535
   Lys Gln His Asn Cys Gln Phe Leu Cys Val Asn Thr Leu Gly Gly
       2540                2545                2550
   Phe Thr Cys Lys Cys Pro Pro Gly Phe Thr Gln His His Thr Ala
       2555                2560                2565
   Cys Ile Asp Asn Asn Glu Cys Gly Ser Gln Pro Ser Leu Cys Gly
       2570                2575                2580
   Ala Lys Gly Ile Cys Gln Asn Thr Pro Gly Ser Phe Ser Cys Glu
       2585                2590                2595
   Cys Gln Arg Gly Phe Ser Leu Asp Ala Thr Gly Leu Asn Cys Glu
       2600                2605                2610
   Asp Val Asp Glu Cys Asp Gly Asn His Arg Cys Gln His Gly Cys
       2615                2620                2625
   Gln Asn Ile Leu Gly Gly Tyr Arg Cys Gly Cys Pro Gln Gly Tyr
       2630                2635                2640
   Ile Gln His Tyr Gln Trp Asn Gln Cys Val Asp Glu Asn Glu Cys
       2645                2650                2655
   Ser Asn Pro Asn Ala Cys Gly Ser Ala Ser Cys Tyr Asn Thr Leu
       2660                2665                2670
   Gly Ser Tyr Lys Cys Ala Cys Pro Ser Gly Phe Ser Phe Asp Gln
       2675                2680                2685
   Phe Ser Ser Ala Cys His Asp Val Asn Glu Cys Ser Ser Ser Lys
       2690                2695                2700
   Asn Pro Cys Asn Tyr Gly Cys Ser Asn Thr Glu Gly Gly Tyr Leu
       2705                2710                2715
   Cys Gly Cys Pro Pro Gly Tyr Tyr Arg Val Gly Gln Gly His Cys
       2720                2725                2730
   Val Ser Gly Met Gly Phe Asn Lys Gly Gln Tyr Leu Ser Leu Asp
       2735                2740                2745
   Thr Glu Val Asp Glu Glu Asn Ala Leu Ser Pro Glu Ala Cys Tyr
       2750                2755                2760
   Glu Cys Lys Ile Asn Gly Tyr Ser Lys Lys Asp Ser Arg Gln Lys
       2765                2770                2775
   Arg Ser Ile His Glu Pro Asp Pro Thr Ala Val Glu Gln Ile Ser
       2780                2785                2790
   Leu Glu Ser Val Asp Met Asp Ser Pro Val Asn Met Lys Phe Asn
       2795                2800                2805
   Leu Ser His Leu Gly Ser Lys Glu His Ile Leu Glu Leu Arg Pro
       2810                2815                2820
   Ala Ile Gln Pro Leu Asn Asn His Ile Arg Tyr Val Ile Ser Gln
       2825                2830                2835
   Gly Asn Asp Asp Ser Val Phe Arg Ile His Gln Arg Asn Gly Leu
       2840                2845                2850
   Ser Tyr Leu His Thr Ala Lys Lys Lys Leu Met Pro Gly Thr Tyr
       2855                2860                2865
   Thr Leu Glu Ile Thr Ser Ile Pro Leu Tyr Lys Lys Lys Glu Leu
       2870                2875                2880
   Lys Lys Leu Glu Glu Ser Asn Glu Asp Asp Tyr Leu Leu Gly Glu
       2885                2890                2895
   Leu Gly Glu Ala Leu Arg Met Arg Leu Gln Ile Gln Leu Tyr
       2900                2905                2910

<210> 677
<211> 21
<212> DNA
<213> artificial

<220>
<223> Description of artificial sequence: Oligonucleotide

<400> 677
ggctggcttt gctaaccgag g 21

<210> 678
<211> 21
<212> DNA
<213> artificial

<220>
<223> Description of artificial sequence: Oligonucleotide

<400> 678
ccgtcaatgt ccatgcagtt c 21

<210> 679
<211> 593
<212> DNA
<213> Homo sapiens

<220>
<221> misc_feature
<222> (469)..(469)
<223> n is a, c, g, t or u

<220>
<221> misc_feature
<222> (480)..(480)
<223> n is a, c, g, t or u

<220>
<221> misc_feature
<222> (507)..(507)
<223> n is a, c, g, t or u

<220>
<221> misc_feature
<222> (521)..(522)
<223> n is a, c, g, t or u

<220>
<221> misc_feature
<222> (546)..(547)
<223> n is a, c, g, t or u

<220>
<221> misc_feature
<222> (553)..(553)
<223> n is a, c, g, t or u

<220>
<221>  misc_feature
<222>  (561)..(561)
<223>  n is a, c, g, t or u

<220>
<221>  misc_feature
<222>  (581)..(582)
<223>  n is a, c, g, t or u

<220>
<221>  misc_feature
<222>  (589)..(589)
<223>  n is a, c, g, t or u

<400>  679
ataatgcgga cgctaccgac tgccagatct ttacactcac ccctccacct gccccgagga      60

gtccggtcac aagggcccag cccatcacaa agacacccag gtgtcccttc catttttttc     120

cacgaaggcc cagaatccat tttaggtttc caaacagacc tttcgtccct tcaaggtgta     180

accaccgttt tccattccag ccattttatt ggccacaccg ttaccttact tataggtatt     240

tccccagaag aagactccag agagcaagct catctgagga aagctgagag ggaagagaaa     300

cccaaacata ctgaagcaaa aaaaagccta tccttcagaa aaaagcaaca aaaagatttc     360

tgttttatct ttcgaaacta aaactattgg atttgaagat taagtatcct aaacatcact     420

gactagaaac tgttctcttt gtcagcagtg aagatattgg atcataggnt tattgatggn     480

ttgcaaaatt ggaccaaata accacgntta tttttatcct nncaaccttc ttatggctca     540

cagganntat ttnatgcaaa ntaaaatcta ttaaatggga nnaaaaaana aaa           593

<210>  680
<211>  22
<212>  DNA
<213>  artificial

<220>
<223>  Description of artificial sequence: Oligonucleotide

<400>  680
ctgccagatc tttacactca cc                                               22

<210>  681
<211>  25
<212>  DNA
<213>  artificial

<220>
<223>  Description of artificial sequence: Oligonucleotide

<400>  681
gctttcctca gatgagcttg ctctc                                            25

<210>  682
<211>  1587
<212>  DNA
<213>  Homo sapiens

<400>  682

aaggagccag aggccatgca gtggctcagg gtccgtgagt cgcctgggga ggccacagga 60
cacagggtca ccatggggac agccgccctg ggtcccgtct gggcagcgct cctgctcttt 120
ctcctgatgt gtgagatccc tatggtggag ctcacctttg acagagctgt ggccagcggc 180
tgccaacggt gctgtgactc tgaggacccc ctggatcctg cccatgtatc ctcagcctct 240
tcctccggcc gcccccacgc cctgcctgag atcagaccct acattaatat caccatcctg 300
aagggtgaca aaggggaccc aggcccaatg ggcctgccag ggtacatggg cagggagggt 360
ccccaagggg agcctggccc tcagggcagc aagggtgaca agggggagat gggcagcccc 420
ggcgccccgt gccagaagcg cttcttcgcc ttctcagtgg gccgcaagac ggccctgcac 480
agcggcgagg acttccagac gctgctcttc gaaagggtct ttgtgaacct tgatgggtgc 540
tttgacatgg cgaccggcca gtttgctgct cccctgcgtg gcatctactt cttcagcctc 600
aatgtgcaca gctggaatta caaggagacg tacgtgcaca ttatgcataa ccagaaagag 660
gctgtcatcc tgtacgcgca gcccagcgag cgcagcatca tgcagagcca gagtgtgatg 720
ctggacctgg cctacgggga ccgcgtctgg gtgcggctct tcaagcgcca gcgcgagaac 780
gccatctaca gcaacgactt cgacacctac atcaccttca gcggccacct catcaaggcc 840
gaggacgact gagggcctct gggccaccct cccggctgga gagctcagct gatacggcat 900
cctgcgagaa gacctgccct cctcactggg atccccttcc tgcctcctcc cagggctctg 960
ccagggcctt gctcagtccc ttccaccaaa gtcatctgaa cttccgtttc cccagggcct 1020
ccagctgccc tcagacactg atgtctgtcc ccaggtgctc tctgcccctc atgcccctct 1080
caccggccca gtgccccgac tctccaggct ttatcaaggt gctaaggccc gggtgggcag 1140
ctcctcgtct cagagccctc ctccggcctg gtgctgcctt tacaaacacc tgcaggagaa 1200
gggccacgga agccccaggc tttagagccc tcagcaggtc tggggagcta gagcaaagga 1260
gggacctcag gccttccgtt tcttcttcca gggtggggtg gcctggtgtt cccctagcct 1320
tccaaaccca ggtggcctgc ccttctcccc agagggaggc ggcctccgcc cattggtgct 1380
catgcagact ctggggctga ggtgccccgg ggggtgatct ctggtgctca cagccgaggg 1440
agccgtggct ccatggccag atgacggaaa cagggtctga ccaagtgcca ggaagacctg 1500
tgctataaac caccctgcct gatcctgccc ctgcctgacc ccgccacgcc ctgccgtcca 1560
gcatgattaa agaatgctgt ctcctca 1587

<210> 683
<211> 278
<212> PRT
<213> Homo sapiens

<400> 683

Met Gln Trp Leu Arg Val Arg Glu Ser Pro Gly Glu Ala Thr Gly His
1               5                   10                  15

Arg Val Thr Met Gly Thr Ala Ala Leu Gly Pro Val Trp Ala Ala Leu
            20                  25                  30

Leu Leu Phe Leu Leu Met Cys Glu Ile Pro Met Val Glu Leu Thr Phe
        35                  40                  45

Asp Arg Ala Val Ala Ser Gly Cys Gln Arg Cys Cys Asp Ser Glu Asp
    50                  55                  60

Pro Leu Asp Pro Ala His Val Ser Ser Ala Ser Ser Ser Gly Arg Pro
65                  70                  75                  80

His Ala Leu Pro Glu Ile Arg Pro Tyr Ile Asn Ile Thr Ile Leu Lys
                85                  90                  95

Gly Asp Lys Gly Asp Pro Gly Pro Met Gly Leu Pro Gly Tyr Met Gly
            100                 105                 110

Arg Glu Gly Pro Gln Gly Glu Pro Gly Pro Gln Gly Ser Lys Gly Asp
        115                 120                 125

Lys Gly Glu Met Gly Ser Pro Gly Ala Pro Cys Gln Lys Arg Phe Phe
    130                 135                 140

Ala Phe Ser Val Gly Arg Lys Thr Ala Leu His Ser Gly Glu Asp Phe
145                 150                 155                 160

Gln Thr Leu Leu Phe Glu Arg Val Phe Val Asn Leu Asp Gly Cys Phe
                165                 170                 175

Asp Met Ala Thr Gly Gln Phe Ala Ala Pro Leu Arg Gly Ile Tyr Phe
            180                 185                 190

Phe Ser Leu Asn Val His Ser Trp Asn Tyr Lys Glu Thr Tyr Val His
        195                 200                 205

Ile Met His Asn Gln Lys Glu Ala Val Ile Leu Tyr Ala Gln Pro Ser
    210                 215                 220

Glu Arg Ser Ile Met Gln Ser Gln Ser Val Met Leu Asp Leu Ala Tyr
225                 230                 235                 240

Gly Asp Arg Val Trp Val Arg Leu Phe Lys Arg Gln Arg Glu Asn Ala
                245                 250                 255

Ile Tyr Ser Asn Asp Phe Asp Thr Tyr Ile Thr Phe Ser Gly His Leu
            260                 265                 270

Ile Lys Ala Glu Asp Asp
        275

<210>  684
<211>  21

<212>  DNA
<213>  artificial

<220>
<223>  Description of artificial sequence: Oligonucleotide

<400>  684
catcctgaag ggtgacaaag g                                                21


<210>  685
<211>  21
<212>  DNA
<213>  artificial

<220>
<223>  Description of artificial sequence: Oligonucleotide

<400>  685
gaggctgaag aagtagatgc c                                                21


<210>  686
<211>  551
<212>  DNA
<213>  Homo sapiens


<220>
<221>  misc_feature
<222>  (43)..(43)
<223>  n is a, c, g, t or u

<220>
<221>  misc_feature
<222>  (80)..(80)
<223>  n is a, c, g, t or u

<220>
<221>  misc_feature
<222>  (84)..(84)
<223>  n is a, c, g, t or u

<220>
<221>  misc_feature
<222>  (89)..(89)
<223>  n is a, c, g, t or u

<220>
<221>  misc_feature
<222>  (149)..(149)
<223>  n is a, c, g, t or u

<220>
<221>  misc_feature
<222>  (158)..(158)
<223>  n is a, c, g, t or u

<400>  686
aggtccatta aggttttggc tacaatggct ctttgggggt tgngaataga attatccaag      60

tattgtaaaa gtcaatattn accnaatgna ttatttccta aaaggcttaa acttgaatta     120

tatcatgtaa agatggcatc tgtcttagnc aacttggnct ttatgcagtt tgtcctttca     180

gttttcagga atgagacctc ttgacccctc ccctccaatg cagcccctac taagggggag     240

tttaaggagc catacatagt tctataattc aaatcaagta aacatgcttc ttgtcccagg     300

ttaacttgtg ctgcctcagt cgctgtttaa acatttttat acgcactgtt aacctgcctg 360

cccattaccc tattactttt aatgggtaaa ctactgttcc ctgggcagtt gtctctttta 420

acgtcccacc ctaaacttgc caaccctcat atgaaggcct caggcttgtt attggcaaag 480

gtcagaagtc ttaagctagt gaccttgcag gctaaagtag gccttgaatt tgtttgacca 540

ggaactaaat t 551

<210> 687
<211> 20
<212> DNA
<213> artificial

<220>
<223> Description of artificial sequence: Oligonucleotide

<400> 687
taacttgtgc tgcctcagtc 20

<210> 688
<211> 22
<212> DNA
<213> artificial

<220>
<223> Description of artificial sequence: Oligonucleotide

<400> 688
ctttgccaat aacaagcctg ag 22

## Description

The invention relates to nucleic acids and encoded polypeptides which are expressed in cancers. The invention also relates to agents which bind the polypeptides. The nucleic acids, polypeptides coded for by such nucleic acids and peptides derived therefrom, as well as related antibodies and cytolytic T lymphocytes, are useful, inter alia, in diagnostic and therapeutic contexts.

Despite interdisciplinary approaches and exhaustive use of classical therapeutic procedures, cancers are still among the leading causes of death.

More recent therapeutic concepts in cancer therapy aim at incorporating the patient's immune system into the overall therapeutic concept by using recombinant tumor vaccines and other specific measures such as antibody therapy. A prerequisite for the success of such a strategy is the recognition of tumor-specific or tumor-associated antigens or epitopes by the patient's immune system whose effector functions are to be interventionally enhanced.

Tumor cells biologically differ substantially from their nonmalignant cells of origin. These differences are due to genetic alterations acquired during tumor development and result, inter alia, also in the formation of qualitatively or quantitatively altered molecular structures in the cancer cells. Tumor-associated structures of this kind which are recognized by the specific immune system of the tumor-harboring host are referred to as tumor-associated antigens.

The specific recognition of tumor-associated antigens involves cellular and humoral mechanisms which are two functionally interconnected units: CD4⁺ and CD8⁺ T lymphocytes recognize the processed antigens presented on the molecules of the MHC (major histocompatibility complex) classes II and I, respectively, while B lymphocytes produce circulating antibody molecules which bind directly to unprocessed antigens. The potential clinical-therapeutical importance of tumor-associated antigens results from the fact that the recognition of antigens on neoplastic cells by the immune system leads to the initiation of cytotoxic effector mechanisms and, in the presence of T helper cells, can cause elimination of the cancer cells (Pardoll, Nat. Med. 4:525-31, 1998).

Antibody based cancer therapies have been successfully introduced into the clinic and have emerged as the most promising therapeutics in oncology over the last decade. Eight antibodies have been approved for treatment of neoplastic diseases, most of them, however in lymphoma and leukemia (Adams GP, Weiner LM, Nat Biotechnol 23:1147-57, 2005).

One of the challenges to be mastered for the advent of the next generation of upgraded antibody-based cancer therapeutics is the selection of appropriate target molecules, which is the key for a favorable toxicity/efficacy profile.

The search for genes tightly silenced in the vast majority of healthy tissues moves into the focus of attention the intriguing observation that genes of the gametogenic and/or trophoblastic lineage are frequently ectopically activated and robustly expressed in human cancer. Based on phenotypical similarities between germ cells, pregnancy trophoblast and cancer cells, John Beard proposed as much as 100 years ago a "trophoblastic theory of cancer" (Beard J, Lancet 1:1758-63, 1902; Gurchot C, Oncology 31:310-3, 1975). The discovery of the sporadic production of chorionic gonadotropin, alpha-fetoprotein, CEA and other trophoblastic hormones by cancer cells provided the first molecules shared between neoplastic and trophoblastic cells (Acevedo HF et al., Cancer 76:1467-75, 1995; Dirnhofer S et al., Hum Pathol 29:377-82, 1998; Gurchot C, Oncology 31:310-3, 1975; Iles RK, Chard T, J Urol 145:453-8, 1991; Laurence DJ, Neville AM, Br J Cancer 26:335-55, 1972). The concept was reignited by the inauguration of the steadily growing so-called cancer/germline (CG) class of genes, which represents more than 100 members, each expressed in a variety of tumor types. The observation that entire trophoblastic and gametogenic programs escape transcriptional silencing and are ectopically activated in cancer cells (Koslowski M et al., Cancer Res 64:5988-93, 2004; Simpson AJ et al., Nat Rev Cancer 5:615-25, 2005) indicates that within this class of genes with exquisitely selective tissue distribution, appropriate targets for mAB therapy may be found.

It was the object of the present invention to provide target structures for a diagnosis and therapy of cancers.

This object is achieved by the subject matter of the claims.

According to the invention, placenta-specific genes are identified which are selectively or aberrantly expressed in tumor cells and thus, provide target structures for therapeutic and diagnostic approaches.

The nucleic acids identified according to the invention to be selectively or aberrantly expressed in tumor cells are selected from the group consisting of (a) a nucleic acid which comprises a nucleic acid sequence selected from the group consisting of SEQ ID NOs: 1-540, 541, 545, 549, 553, 557, 560, 563, 566, 570, 574, 577, 580, 583, 587, 591, 595, 599, 602, 606, 610, 613, 617, 620, 624, 634, 638, 642, 646, 649, 653, 656, 660, 664, 668, 671, 675, 679, 682, and 686 of the sequence listing, a part or derivative thereof, (b) a nucleic acid which hybridizes with the nucleic acid of (a) under stringent conditions, (c) a nucleic acid which is degenerate with respect to the nucleic acid of (a) or (b), and (d) a nucleic acid which is complementary to the nucleic acid of (a), (b) or (c). These nucleic acids are also termed "tumor-associated nucleic acids" herein.

In another aspect, the invention relates to antigens encoded by the tumor-associated nucleic acids identified according to the invention. Accordingly, the tumor-associated antigens identified according to the invention have an amino acid sequence encoded by a nucleic acid which is selected from the group consisting of (a) a nucleic acid which comprises a nucleic acid sequence selected from the group consisting of SEQ ID NOs: 1-540, 541, 545, 549, 553, 557, 560, 563, 566, 570, 574, 577, 580, 583, 587, 591, 595, 599, 602, 606, 610, 613, 617, 620, 624, 634, 638, 642, 646, 649, 653, 656, 660, 664, 668, 671, 675, 679, 682, and 686 of the sequence listing, a part or derivative thereof, (b) a nucleic acid which hybridizes with the nucleic acid of (a) under stringent conditions, (c) a nucleic acid which is degenerate with respect to the nucleic acid of (a) or (b), and (d) a nucleic acid which is complementary to the nucleic acid of (a), (b) or (c). In a preferred embodiment, the tumor-associated antigens identified according to the invention comprises an amino acid sequence selected from the group consisting of SEQ ID NOs: 542, 546, 550, 554, 567, 571, 584, 588, 592, 596, 603, 607, 614, 621, 625, 635, 639, 643, 650, 657, 661, 665, 672, 676, and 683 of the sequence listing, a part or derivative thereof.

If according to the invention reference is made to nucleic acids comprising certain nucleic acid sequences or tumor-associated antigens comprising certain amino acid sequences this also includes embodiments wherein the nucleic acids or tumor-associated antigens consist of these certain nucleic acid sequences or amino acid sequences, respectively.

The present invention generally embraces the treatment and/or diagnosis by targeting tumor-associated nucleic acids or tumor-associated antigens identified according to the invention. This provides for the selective detection of cells and/or eradication of cells that express such tumor-associated nucleic acids and/or tumor-associated antigens thereby minimizing adverse effects to normal cells not expressing such tumor-associated nucleic acids and/or tumor-associated antigens. Thus, preferred diseases for a therapy or diagnosis are those in which one or more of the tumor-associated nucleic acids and/or tumor-associated antigens identified according to the invention are expressed such as tumor diseases, in particular cancer diseases such as those described herein.

The present invention generally involves the use of tumor-associated nucleic acids and tumor-associated antigens identified according to the invention or of parts or derivatives thereof, of nucleic acids directed against said tumor-associated nucleic acids, of antibodies or T cells directed against the tumor-associated antigens identified according to the invention or parts or derivatives thereof and/or of host cells expressing the tumor-associated antigens identified according to the invention or parts or derivatives thereof for therapy, prophylaxis, diagnosis and/or monitoring of neoplastic diseases such as tumor diseases, in particular cancer diseases such as those described herein.

This may also involve the use of a combination of two or more of these nucleic acids, antigens, antibodies, T cells and/or host cells.

In those embodiments of the invention relating to the use of antibodies directed against the tumor-associated antigens identified according to the invention or parts or derivatives thereof also T cell receptors directed against the tumor-associated antigens identified according to the invention or parts or derivatives thereof, optionally in a complex with MHC molecules, may be used.

Especially suitable for therapy, prophylaxis, diagnosis and/or monitoring is a part of the tumor-associated antigens identified according to the invention which corresponds to the non-transmembrane portion, in particular the extracellular portion of the tumor-associated antigens or is comprised thereof. Therefore, according to the invention, a part of the tumor-associated antigens identified according to the invention which corresponds to the non-transmembrane portion, in particular the extracellular portion of the tumor-associated antigens or is comprised thereof, or a corresponding part of the nucleic acids coding for the tumor-associated antigens identified according to the invention is preferred for therapy, prophylaxis, diagnosis and/or monitoring. Similarly the use of antibodies is preferred which are directed against a part of the tumor-associated antigens identified according to the invention which corresponds to the non-transmembrane portion, in particular the extracellular portion of the tumor-associated antigens or is comprised thereof.

Preferred diseases for a therapy, prophylaxis, diagnosis and/or monitoring are those in which one or more of the tumor-associated nucleic acids identified according to the invention are selectively expressed or abnormally expressed. Particularly preferred diseases for a therapy, prophylaxis, diagnosis and/or monitoring are those in which one or more of the tumor-associated nucleic acids identified according to the invention and/or one or more of the tumor-associated antigens encoded thereby are selectively expressed or abnormally expressed.

In one aspect, the invention relates to a pharmaceutical composition comprising an agent which recognizes a tumor-associated antigen identified according to the invention or a nucleic acid coding for the tumor-associated antigen and which is preferably selective for cells which have expression or abnormal expression of a tumor-associated antigen identified according to the invention or a nucleic acid coding for the tumor-associated antigen.

In a further aspect, the invention relates to a pharmaceutical composition comprising an agent which (I) inhibits expression or activity of a tumor-associated antigen identified according to the invention, and/or (II) has tumor-inhibiting or tumor-destroying activity and is selective for cells expressing or abnormally expressing a tumor-associated nucleic acid and/or a tumor-associated antigen identified according to the invention, and/or (III) when administered, selectively increases the amount of complexes between an MHC molecule and a tumor-associated antigen identified according to the invention or a part thereof, such as a peptide epitope. In particular embodiments, said agent may cause induction of cell death, reduction in cell growth, damage to the cell membrane or secretion of cytokines and preferably have a tumor-inhibiting activity.

In one embodiment, an agent which inhibits expression or activity of a tumor-associated antigen is specific for a tumor-associated nucleic acid identified according to the invention. In another embodiment, an agent which inhibits expression or activity of a tumor-associated antigen is specific for a tumor-associated antigen identified according to the invention. According to the invention the phrase "inhibit expression and/or activity" includes a complete or essentially complete inhibition of expression and/or activity and a reduction in expression and/or activity. Preferably, said inhibition of expression of a tumor-associated antigen identified according to the invention may take place by inhibiting the production of or reducing the level of transcript, i.e. mRNA, coding for a tumor-associated antigen identified according to the invention, e.g. by inhibiting transcription or inducing degradation of transcript, and/or by inhibiting the production of tumor-associated antigen identified according to the invention, e.g. by inhibiting translation of transcript coding for a tumor-associated antigen identified according to the invention. Preferably, said inhibition of expression and/or activity of a tumor-associated antigen identified according to the present invention reduces tumor cell growth and/or induces tumor cell death and thus, has a tumor-inhibiting or tumor-destroying effect.

In a particular embodiment, the agent which inhibits expression of a tumor-associated antigen identified according to the invention is an inhibitory nucleic acid (e.g., anti-sense oligonucleotide, ribozyme, iRNA, siRNA or a DNA encoding the same) selectively hybridizing to and being specific for a tumor-associated nucleic acid identified according to the invention, thereby inhibiting (e.g., reducing) transcription and/or translation thereof.

Inhibitory nucleic acids include oligonucleotides having sequences in the antisense orientation relative to the target nucleic acids. Suitable inhibitory oligonucleotides typically vary in length from five to several hundred nucleotides, more typically about 20-70 nucleotides in length or shorter, even more typically about 10-30 nucleotides in length. These inhibitory oligonucleotides may be administered as free (naked) nucleic acids or in protected forms, e.g., encapsulated in liposomes. The use of liposomal or other protected forms may be advantageous as it may enhance in vivo stability and thus facilitate delivery to target sites.

Also, the target tumor-associated nucleic acid may be used to design ribozymes that target the cleavage of the corresponding mRNAs in tumor cells. Similarly, these ribozymes may be administered in free (naked) form or by the use of delivery systems that enhance stability and/or targeting, e.g., liposomes.

Also, the target tumor-associated nucleic acid may be used to design siRNAs that can inhibit (e.g., reduce) expression of the tumor-associated nucleic acid. The siRNAs may be administered in free (naked) form or by the use of delivery systems that enhance stability and/or targeting, e.g., liposomes. They may also be administered in the form of their precursors or encoding DNAs.

In a further embodiment, the agent which inhibits activity of a tumor-associated antigen identified according to the invention is an antibody that specifically binds to said tumor-associated antigen. Binding of the antibody to the tumor-associated antigen can interfere with the function of the the tumor-associated antigen, e.g. by inhibiting binding activity or catalytic activity.

Also, the present invention in another aspect relates to therapies for treatment of tumor diseases involving the administration of a ligand of a target molecule, i.e. a tumor-associated nucleic acid or tumor-associated antigen identified according to the invention. In this respect, a nucleic acid may be administered that selectively hybridizes to the target nucleic acid or an antibody may be administered that specifically binds to a target antigen, attached to therapeutic effector moieties, e.g., radiolabels, cytotoxins, cytotoxic enzymes, and the like in order to selectively target and kill cells that express these targets, e.g. tumor cells.

In one embodiment of the above aspects, the agent is an antisense nucleic acid which hybridizes selectively with the nucleic acid coding for the tumor-associated antigen. In a further embodiment, the agent is a siRNA preferably comprising a sense RNA strand and an antisense RNA strand, wherein the sense and antisense RNA strands form an RNA duplex, and wherein the sense RNA strand comprises a nucleotide sequence substantially identical to a target sequence of about 19 to about 25 contiguous nucleotides in a nucleic acid coding for the tumor-associated antigen, preferably mRNA coding for the tumor-associated antigen. In a further embodiment, the agent is an antibody which binds selectively to the tumor-associated antigen, in particular a complement-activated or toxin conjugated antibody which binds selectively to the tumor-associated antigen. In a preferred embodiment, the antibody which binds selectively to the tumor-associated antigen is coupled to a therapeutically useful substance and/or recruits natural or artificial effector mechanisms to said cell expressing or abnormally expressing said tumor-associated antigen. In a further embodiment, the agent is a cytotoxic T lymphocyte which recognizes the tumor-associated antigen or a part thereof bound by an MHC molecule on a cell and lyses the cells labeled in this way. In a further embodiment, the agent is a T helper lymphocyte which recognizes the tumor-associated antigen or a part thereof bound by an MHC molecule on a cell and enhances effector functions of other cells specifically recognizing said tumor-associated antigen or a part thereof.

In a further embodiment, the agent comprises two or more agents which each recognize different tumor-associated antigens or different nucleic acids coding for tumor-associated antigens and/or inhibit expression or activity of different tumor-associated antigens, and/or have tumor-inhibiting or tumor-destroying activity and are selective for cells expressing or abnormally expressing different tumor-associated antigens, and/or when administered, selectively increase the amount of complexes between MHC molecules and different tumor-associated antigens or parts thereof, wherein at least one of said different tumor-associated antigens is a tumor-associated antigen identified according to the invention.

Preferably, a tumor-associated antigen selectively limited to tumors serves as a label for recruiting effector mechanisms to this specific location. In this aspect, the invention includes embodiments wherein the agent itself does not have an ability to inhibit activity of a tumor-associated antigen or a tumor-inhibiting or tumor-destroying activity but mediates such effect, in particular by recruiting effector mechanisms, in particular those having cell damaging potential, to a specific location, in particular a tumor or tumor cells.

Preferably, said cells expressing or abnormally expressing a tumor-associated nucleic acid and/or a tumor-associated antigen identified according to the invention are non-placenta cells.

The activity of a tumor-associated antigen identified according to the invention can be any activity of a protein or a peptide. In one embodiment this activity is an enzymatic activity.

According to the invention the phrase "inhibit expression or activity" includes a complete or essentially complete inhibition of expression or activity and a reduction in expression or activity.

The agent which, when administered, selectively increases the amount of complexes between an MHC molecule and a tumor-associated antigen identified according to the invention or a part thereof comprises one or more components selected from the group consisting of (i) the tumor-associated antigen or a part thereof, (ii) a nucleic acid which codes for said tumor-associated antigen or a part thereof, (iii) a host cell which expresses said tumor-associated antigen or a part thereof, and (iv) isolated complexes between peptide epitopes from said tumor-associated antigen and an MHC molecule.

The invention furthermore relates to a pharmaceutical composition which comprises one or more components selected from the group consisting of (i) a tumor-associated antigen identified according to the invention or a part thereof, (ii) a nucleic acid which codes for a tumor-associated antigen identified according to the invention or a part thereof, (iii) an antibody which binds to a tumor-associated antigen identified according to the invention or to a part thereof, (iv) an antisense nucleic acid which hybridizes specifically with a tumor-associated nucleic acid identified according to the invention / a nucleic acid coding for a tumor-associated antigen identified according to the invention, (v) an siRNA directed against a tumor-associated nucleic acid identified according to the invention / a nucleic acid coding for a tumor-associated antigen identified according to the invention, (vi) a host cell which expresses a tumor-associated antigen identified according to the invention or a part thereof, and (vii) isolated complexes between a tumor-associated antigen identified according to the invention or a part thereof and an MHC molecule.

In one embodiment, a nucleic acid coding for a tumor-associated antigen identified according to the invention or a part thereof is present in the pharmaceutical composition in an expression vector and functionally linked to a promoter. In a further embodiment, a nucleic acid coding for a tumor-associated antigen identified according to the invention or a part thereof is present in the pharmaceutical composition in a virus as further described below.

A host cell present in a pharmaceutical composition of the invention may secrete the tumor-associated antigen or the part thereof, may express it on the surface and preferably may additionally express an MHC molecule which binds to said tumor-associated antigen or said part thereof. In one embodiment, the host cell expresses the MHC molecule endogenously. In a further embodiment, the host cell expresses the MHC molecule and/or the tumor-associated antigen or the part thereof in a recombinant manner. The host cell is preferably nonproliferative. In a preferred embodiment, the host cell is an antigen-presenting cell, in particular a dendritic cell, a monocyte or a macrophage.

In a further embodiment, an antibody present in a pharmaceutical composition of the invention is a monoclonal antibody. In further embodiments, the antibody is a chimeric, human or humanized antibody, a fragment of an antibody or a synthetic antibody. The antibody may be coupled to a therapeutically or diagnostically useful agent also termed therapeutic or diagnostic agent or substance herein.

An antisense nucleic acid present in a pharmaceutical composition of the invention may comprise a sequence of 6-50, in particular 10-30, 15-30 and 20-30, contiguous nucleotides of the nucleic acid coding for the tumor-associated antigen identified according to the invention.

In further embodiments, a tumor-associated antigen or a part thereof, provided by a pharmaceutical composition of the invention either directly or via expression of a nucleic acid, binds to MHC molecules on the surface of cells, said binding preferably causing a cytolytic response and/or inducing cytokine release.

A pharmaceutical composition of the invention may comprise a pharmaceutically compatible carrier and/or an adjuvant.

A pharmaceutical composition of the invention is preferably used for the treatment or prevention of a disease characterized by selective expression or abnormal expression of a tumor-associated nucleic acid and/or tumor-associated antigen. In a preferred embodiment, the disease is a neoplastic disease, preferably cancer.

In a preferred embodiment, the pharmaceutical composition of the invention is in the form of a vaccine which may be used therapeutically or prophylactically. Such vaccine preferably comprises a tumor-associated antigen identified according to the invention or a part thereof, and/or a nucleic acid which codes for a tumor-associated antigen identified according to the invention or a part thereof. In particular embodiments, the nucleic acid is present in a virus or host cell.

The invention furthermore relates to methods of treating, preventing, diagnosing or monitoring, i.e. determining the regression, progression, course and/or onset of, a disease characterized by expression or abnormal expression of one of more tumor-associated nucleic acids identified according to the invention, preferably also resulting in expression or abnormal expression of one of more tumor-associated antigens identified according to the invention, preferably a neoplastic disease, in particular cancer. In one embodiment, the treatment or prevention comprises administering a pharmaceutical composition of the invention.

The methods of diagnosing and/or methods of monitoring according to the invention generally concern the detection of and/or determination of the quantity of one or more parameters selected from the group consisting of (i) a tumor-associated nucleic acid identified according to the invention, or a part thereof, (ii) a tumor-associated antigen identified according to the invention, or a part thereof, (iii) an antibody against a tumor-associated antigen identified according to the invention or a part thereof, and (iv) T lymphocytes, preferably cytotoxic or T helper lymphocytes, which are specific for a tumor-associated antigen identified according to the invention or a part thereof and/or a complex between the tumor-associated antigen or a part thereof and an MHC molecule, in a biological sample isolated from a patient, preferably from a patient having said disease, being suspected of having or falling ill with said disease or having a potential for said disease. Means for accomplishing said detection and/or determination of the quantity are described herein and will be apparent to the skilled person.

Preferably, the presence of said nucleic acid or said part thereof, said tumor-associated antigen or said part thereof, said antibody and/or said T lymphocytes and/or a quantity of said nucleic acid or said part thereof, said tumor-associated antigen or said part thereof, said antibody and/or said T lymphocytes which is increased compared to a patient and/or a tissue without said disease is indicative for the presence of said disease or a potential for a development of said disease.

The methods of diagnosing and/or monitoring of the invention also include embodiments wherein by detection or determination of the quantity of said nucleic acid or said part thereof, said tumor-associated antigen or said part thereof, said antibody and/or said T lymphocytes it is possible to assess and/or prognose the metastatic behavior of said disease, wherein, preferably, the presence of said nucleic acid or said part thereof, said tumor-associated antigen or said part thereof, said antibody and/or said T lymphocytes and/or a quantity of said nucleic acid or said part thereof, said tumor-associated antigen or said part thereof, said antibody and/or said T lymphocytes which is increased compared to a patient without said disease or without a metastasis of said disease is indicative for a metastatic behavior of said disease or a potential for a metastatic behavior of said disease.

In particular embodiments, said detection or determination of the quantity comprises (i) contacting a biological sample with an agent which binds specifically to said tumor-associated nucleic acid or said part thereof, to said tumor-associated antigen or said part thereof, to said antibody or to said T lymphocytes, and (ii) detecting the formation of or determining the amount of a complex between the agent and the nucleic acid or the part thereof, the tumor-associated antigen or the part thereof, the antibody, or the T lymphocytes.

In one embodiment, the disease is characterized by expression or abnormal expression of two or more different tumor-associated nucleic acids preferably also resulting in expression or abnormal expression of two or more different tumor-associated antigens and a detection or determination of the quantity comprises a detection or determination of the quantity of two or more different tumor-associated nucleic acids or of parts thereof, of two or more different tumor-associated antigens or of parts thereof, of two or more antibodies binding to said two or more different tumor-associated antigens or to parts thereof and/or of two or more T lymphocytes specific for said two or more different tumor-associated antigens or parts thereof, or complexes thereof with MHC molecules. In a further embodiment, the biological sample isolated from the patient is compared to a comparable normal biological sample.

The methods of monitoring according to the invention preferably comprise a detection of and/or determination of the quantity of one or more of the parameters mentioned above in a first sample at a first point in time and in a further sample at a second point in time, wherein the course of the disease is determined by comparing the two samples.

Preferably, a level of said nucleic acid or said part thereof, said tumor-associated antigen or said part thereof, said antibody and/or said T lymphocytes which is increased in a sample compared to a sample taken earlier from a patient indicates that the patient has developed or is about to develop cancer and/or a metastasis of cancer and/or a relapse of cancer. Preferably, a level of said nucleic acid or said part thereof, said tumor-associated antigen or said part thereof, said antibody and/or said T lymphocytes which is decreased in a sample compared to a sample taken earlier from a patient indicates regression of cancer and/or a metastasis of cancer in said patient and thus, preferably indicates a successful cancer therapy.

According to the invention, detection of a nucleic acid or of a part thereof or determining the quantity of a nucleic acid or of a part thereof may be carried out using a oligo- or polynucleotide probe which hybridizes specifically to said nucleic acid or said part thereof or may be carried out by selective amplification of said nucleic acid or said part thereof, e.g. by means of PCR amplification. In one embodiment, the oligo- or polynucleotide probe comprises a sequence of 6-50, in particular 10-30, 15-30 and 20-30, contiguous nucleotides of said nucleic acid.

In particular embodiments, the tumor-associated antigen or the part thereof which is to be detected or the quantity of which is to be determined in the methods of the present invention is present intracellularly, on the cell surface or in a complex with an MHC molecule.

According to the invention, detection of a tumor-associated antigen or of a part thereof or determining the quantity of a tumor-associated antigen or of a part thereof may be carried out using an antibody binding specifically to said tumor-associated antigen or said part thereof.

According to the invention, detection of an antibody or determining the quantity of an antibody may be carried out using a protein or peptide binding specifically to said antibody.

According to the invention, detection of or determining the quantity of T lymphocytes which are specific for a tumor-associated antigen or a part thereof and/or a complex thereof with an MHC molecule may be carried out using a cell presenting the complex between said tumor-associated antigen or said part thereof and an MHC molecule. T lymphocytes may additionally be detected by detecting their proliferation, their cytokine production, and their cytotoxic activity triggered by specific stimulation with a complex of an MHC molecule and a tumor-associated antigen or a part thereof. T lymphocytes may also be detected with aid of a recombinant MHC molecule or a complex of two or more MHC molecules loaded with immunogenic fragments of one or more tumor-associated antigens.

An agent which is used for detection or determining the quantity in the methods of the invention such as a oligo- or polynucleotide probe, an antibody, a protein or peptide or a cell is preferably labeled in a detectable manner, in particular by a detectable marker such as a radioactive marker or an enzymic marker.

In a particular aspect, the invention relates to a method of treating, preventing, diagnosing or monitoring a disease characterized by expression or abnormal expression of a tumor-associated antigen identified according to the invention, which method comprises administering an antibody which binds to said tumor-associated antigen or to a part thereof and which is coupled to a therapeutic or diagnostic agent. The antibody may be a monoclonal antibody. In further embodiments, the antibody is a chimeric or humanized antibody or a fragment of an antibody.

In certain embodiments, the methods of the invention of diagnosing or monitoring a disease are performed with a biological sample containing or suspected of containing tumor cells such as disseminating tumor cells or metastatic tumor cells. Such biological samples include, for example, tissue, blood, serum, bone marrow, sputum, bronchial aspirate, and/or bronchial lavage. Preferably, the methods of the invention of diagnosing or monitoring a disease are performed with a biological sample not containing placental cells and, in particular, being a non-placenta biological sample isolated from a subject. In one embodiment, the biological sample is from a tissue or organ wherein the cells when the tissue or organ is free of tumors do not substantially express a tumor-associated antigen identified according to the invention and/or a tumor-associated nucleic acid identified according to the invention.

In one particular aspect, the invention relates to a method of treating a patient having a disease characterized by expression or abnormal expression of a tumor-associated antigen identified according to the invention, which method comprises (i) providing a sample containing immunoreactive cells, either obtained from said patient or from another individual of the same species, in particular a healthy individual, or an individual of a different species, (ii) contacting said sample with a host cell expressing said tumor-associated antigen or a part thereof, under conditions which favor production of cytolytic T cells against said tumor-associated antigen or a part thereof, and (iii) introducing the cytolytic T cells into the patient in an amount suitable for lysing cells expressing the tumor-associated antigen or a part thereof. In one embodiment, the method includes cloning of the T cell receptor of cytolytic T cells obtained and transferring the nucleic acid coding for the T cell receptor to T cells, either obtained from said patient or from another individual of the same species, in particular a healthy individual, or an individual of a different species, which T cells thus receive the desired specificity and, as under (iii), may be introduced into the patient.

In one embodiment, the host cell endogenously expresses an MHC molecule. In a further embodiment, the host cell recombinantly expresses an MHC molecule and/or the tumor-associated antigen or the part thereof. Preferably, the host cell presents the tumor-associated antigen or the part thereof by MHC molecules on its surface. The host cell is preferably nonproliferative. In a preferred embodiment, the host cell is an antigen-presenting cell, in particular a dendritic cell, a monocyte or a macrophage.

The invention also relates to a method of treating a disease characterized by expression or abnormal expression of a tumor-associated antigen identified according to the invention, which method comprises (i) identifying cells from the patient which express abnormal amounts of the tumor-associated antigen, (ii) isolating a sample of said cells, (iii) culturing said cells, and (iv) introducing said cells into the patient in an amount suitable for triggering an immune response to the cells.

The present invention furthermore relates to a nucleic acid selected from the group consisting of (a) a nucleic acid which comprises a nucleic acid sequence selected from the group consisting of SEQ ID NOs: 1-540, 541, 545, 549, 553, 557, 560, 563, 566, 570, 574, 577, 580, 583, 587, 591, 595, 599, 602, 606, 610, 613, 617, 620, 624, 634, 638, 642, 646, 649, 653, 656, 660, 664, 668, 671, 675, 679, 682, and 686, a part or derivative thereof, (b) a nucleic acid which hybridizes with the nucleic acid of (a) under stringent conditions, (c) a nucleic acid which is degenerate with respect to the nucleic acid of (a) or (b), and (d) a nucleic acid which is complementary to the nucleic acid of (a), (b) or (c).

In a further aspect, the invention relates to a recombinant nucleic acid molecule, in particular DNA or RNA molecule, which comprises a nucleic acid of the invention.

The invention also relates to host cells which contain a nucleic acid or recombinant nucleic acid molecule of the invention.

The host cell may also comprise a nucleic acid coding for a MHC molecule. In one embodiment, the host cell endogenously expresses the MHC molecule. In a further embodiment, the host cell recombinantly expresses the MHC molecule and/or the nucleic acid or recombinant nucleic acid molecule of the invention or a part thereof. Preferably, the host cell is nonproliferative. In a preferred embodiment, the host cell is an antigen-presenting cell, in particular a dendritic cell, a monocyte or a macrophage.

In a further embodiment, the invention relates to oligonucleotides which hybridize with a nucleic acid identified according to the invention and which may be used as genetic probes or as "antisense" molecules. Nucleic acid molecules in the form of oligonucleotide primers or competent probes, which hybridize with a nucleic acid identified according to the invention or parts thereof, may be used for detecting said nucleic acid and/or finding nucleic acids which are homologous to said nucleic acid identified according to the invention, e.g. by PCR amplification, Southern and Northern hybridization. Hybridization may be carried out under low stringency, more preferably under medium stringency and most preferably under high stringency conditions.

In a further aspect, the invention relates to a protein or peptide which is encoded by a nucleic acid selected from the group consisting of (a) a nucleic acid which comprises a nucleic acid sequence selected from the group consisting of SEQ ID NOs: 1-540, 541, 545, 549, 553, 557, 560, 563, 566, 570, 574, 577, 580, 583, 587, 591, 595, 599, 602, 606, 610, 613, 617, 620, 624, 634, 638, 642, 646, 649, 653, 656, 660, 664, 668, 671, 675, 679, 682, and 686, a part or derivative thereof, (b) a nucleic acid which hybridizes with the nucleic acid of (a) under stringent conditions, (c) a nucleic acid which is degenerate with respect to the nucleic acid of (a) or (b), and (d) a nucleic acid which is complementary to the nucleic acid of (a), (b) or (c). In a preferred embodiment, the protein or peptide comprises an amino acid sequence selected from the group consisting of SEQ ID NOs: 542, 546, 550, 554, 567, 571, 584, 588, 592, 596, 603, 607, 614, 621, 625, 635, 639, 643, 650, 657, 661, 665, 672, 676, and 683 of the sequence listing, a part or derivative thereof.

In a further aspect, the invention relates to an immunogenic fragment of a tumor-associated antigen identified according to the invention. Said fragment preferably binds to a MHC molecule or an antibody, preferably to a human HLA receptor or a human antibody. According to the invention, a part or fragment preferably comprises a sequence of at least 5, at least 6, in particular at least 8, at least 10, at least 12, at least 15, at least 20, at least 30 or at least 50, amino acids.

In a further aspect, the invention relates to an agent which binds to a tumor-associated antigen identified according to the invention or to a part thereof. In a preferred embodiment, the agent is a protein or peptide, in particular an antibody, a T cell receptor or an MHC molecule. In further embodiments, the antibody is a monoclonal, chimeric, human or humanized antibody, an antibody produced by combinatory techniques, or a fragment of an antibody. In one preferred embodiment, the invention relates to an antibody which binds selectively to a complex of (i) a tumor-associated antigen identified according to the invention or a part thereof and (ii) an MHC molecule to which said tumor-associated antigen identified according to the invention or said part thereof binds, with said antibody not binding to (i) or (ii) alone.

According to the invention, the term "binding" preferably relates to a specific binding. "Specific binding" means that an agent such as an antibody binds stronger to a target such as an epitope for which it is specific compared to the binding to another target. An agent binds stronger to a first target compared to a second target if it binds to the first target with a dissociation constant (K_{D}) which is lower than the dissociation constant for the second target. Preferably the dissociation constant (K_{D}) for the target to which the agent binds specifically is more than 10-fold, preferably more than 20-fold, more preferably more than 50-fold, even more preferably more than 100-fold, 200-fold, 500-fold or 1000-fold lower than the dissociation constant (K_{D}) for the target to which the agent does not bind specifically.

Such specific antibodies may, for example, be obtained by immunization using the aforementioned peptides.

The invention furthermore relates to a conjugate between an agent of the invention which binds to a tumor-associated antigen identified according to the invention or to a part thereof or an antibody of the invention and a therapeutic or diagnostic agent. In one embodiment, the therapeutic or diagnostic agent is a toxin.

In a further aspect, the invention relates to a kit for detecting a disease characterized by expression or abnormal expression of one of more tumor-associated nucleic acids identified according to the invention, preferably also resulting in expression or abnormal expression of one of more tumor-associated antigens identified according to the invention, preferably a neoplastic disease, in particular cancer, which kit comprises agents for detection or determining the quantity (i) of the tumor-associated nucleic acid or of a part thereof, (ii) of the tumor-associated antigen or of a part thereof, (iii) of antibodies which bind to the tumor-associated antigen or to a part thereof, and/or (iv) of T cells which are specific for the tumor-associated antigen or a part thereof or a complex thereof with an MHC molecule. Such agents are described herein above.

### Detailed description of the invention

A reference herein to a range of numerical values is to be understood so as to specify and mention each of the individual numerical values comprised by said range. For example, a reference to SEQ ID NOs: 1-540 is to be understood so as to refer to each and every of the following individual SEQ ID NOs: 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330, 331, 332, 333, 334, 335, 336, 337, 338, 339, 340, 341, 342, 343, 344, 345, 346, 347, 348, 349, 350, 351, 352, 353, 354, 355, 356, 357, 358, 359, 360, 361, 362, 363, 364, 365, 366, 367, 368, 369, 370, 371, 372, 373, 374, 375, 376, 377, 378, 379, 380, 381, 382, 383, 384, 385, 386, 387, 388, 389, 390, 391, 392, 393, 394, 395, 396, 397, 398, 399, 400, 401, 402, 403, 404, 405, 406, 407, 408, 409, 410, 411, 412, 413, 414, 415, 416, 417, 418, 419, 420, 421, 422, 423, 424, 425, 426, 427, 428, 429, 430, 431, 432, 433, 434, 435, 436, 437, 438, 439, 440, 441, 442, 443, 444, 445, 446, 447, 448, 449, 450, 451, 452, 453, 454, 455, 456, 457, 458, 459, 460, 461, 462, 463, 464, 465, 466, 467, 468, 469, 470, 471, 472, 473, 474, 475, 476, 477, 478, 479, 480, 481, 482, 483, 484, 485, 486, 487, 488, 489, 490, 491, 492, 493, 494, 495, 496, 497, 498, 499, 500, 501, 502, 503, 504, 505, 506, 507, 508, 509, 510, 511, 512, 513, 514, 515, 516, 517, 518, 519, 520, 521, 522, 523, 524, 525, 526, 527, 528, 529, 530, 531, 532, 533, 534, 535, 536, 537, 538, 539, and 540.

According to the invention, a "reference" such as a reference sample or reference organism may be used to correlate and compare the results obtained in the methods of the invention from a test sample or test organism, i.e. a patient. Typically the reference sample is a sample from a healthy tissue, in particular a corresponding tissue which is not affected by cancer, or the reference organism is a healthy organism, in particular an organism which does not suffer from cancer.

A "reference value" can be determined from a reference empirically by measuring a sufficiently large number of references. Preferably the reference value is determined by measuring at least 2, preferably at least 3, preferably at least 5, preferably at least 8, preferably at least 12, preferably at least 20, preferably at least 30, preferably at least 50, or preferably at least 100 references.

According to the invention, a nucleic acid is preferably deoxyribonucleic acid (DNA) or ribonucleic acid (RNA). Nucleic acids comprise according to the invention genomic DNA, cDNA, mRNA, recombinantly produced and chemically synthesized molecules. According to the invention, a nucleic acid may be present as a single-stranded or double-stranded and linear or covalently circularly closed molecule.

The terms "tumor-associated nucleic acid identified according to the invention" and "nucleic acid encoding a tumor-associated antigen identified according to the invention" have similar meanings. However, the different terms are used herein to account for the fact that in some embodiments only the expression of nucleic acid, in particular mRNA, is of relevance while the expression of protein is not a critical factor.

As used herein, the term "RNA" means a molecule comprising at least one ribonucleotide residue. By "ribonucleotide" is meant a nucleotide with a hydroxyl group at the 2'-position of a beta-D-ribo-furanose moiety. The term includes double stranded RNA, single stranded RNA, isolated RNA such as partially purified RNA, essentially pure RNA, synthetic RNA, recombinantly produced RNA, as well as altered RNA that differs from naturally occurring RNA by the addition, deletion, substitution and/or alteration of one or more nucleotides. Such alterations can include addition of non-nucleotide material, such as to the end(s) of a RNA or internally, for example at one or more nucleotides of the RNA. Nucleotides in RNA molecules can also comprise non-standard nucleotides, such as non-naturally occurring nucleotides or chemically synthesized nucleotides or deoxynucleotides. These altered RNAs can be referred to as analogs or analogs of naturally-occurring RNA.

If reference is made herein to the detection of or the determination of the quantity of a nucleic acid, the nucleic acid which is actually to be detected or the quantity of which is actually to be determined is preferably mRNA. However, it should be understood that this may also include embodiments wherein mRNA is detected or the quantity of mRNA is determined indirectly. For example, mRNA may be transformed into cDNA and the cDNA detected or its quantity determined. mRNA is given herein as the cDNA equivalent. One skilled in the art would understand that the cDNA sequence is equivalent to the mRNA sequence, and can be used for the same purpose herein, e.g., the generation of probes hybridizing to the nucleic acid to be detected. Thus, if reference is made herein to the sequences shown in the sequence listing this is also to include the RNA equivalents of said sequences.

The nucleic acids described according to the invention have preferably been isolated. The term "isolated nucleic acid" means according to the invention that the nucleic acid was (i) amplified *in vitro*, for example by polymerase chain reaction (PCR), (ii) recombinantly produced by cloning, (iii) purified, for example by cleavage and gel-electrophoretic fractionation, or (iv) synthesized, for example by chemical synthesis. An isolated nucleic acid is a nucleic acid which is available for manipulation by recombinant DNA techniques.

A degenerate nucleic acid according to the invention is a nucleic acid that differs from a reference nucleic acid in codon sequence due to the degeneracy of the genetic code.

The term "derivative" with respect to, for example, nucleic acid and amino acid sequences, according to the invention includes any variants, in particular mutants, splice variants, conformations, isoforms, allelic variants, species variants and species homologs, in particular those which are naturally present. An allelic variant relates to an alteration in the normal sequence of a gene, the significance of which is often unclear. Complete gene sequencing often identifies numerous allelic variants for a given gene. A species homolog is a nucleic acid or amino acid sequence with a different species of origin from that of a given nucleic acid or amino acid sequence.

A "derivative" of a nucleic acid according to the invention also includes nucleic acids comprising single or multiple such as at least 2, at least 4, or at least 6 and preferably up to 3, up to 4, up to 5, up to 6, up to 10, up to 15, or up to 20 nucleotide substitutions, deletions and/or additions. Furthermore, the term "derivative" also comprises chemical derivatization of a nucleic acid on a nucleotide base, on the sugar or on the phosphate. The term "derivative" also comprises nucleic acids which contain nucleotides and nucleotide analogs not occurring naturally. Preferably, a derivatization of a nucleic acid increases its stability.

Preferably the degree of identity between a specific nucleic acid sequence described herein and a nucleic acid sequence which is a derivative of said specific nucleic acid sequence, which hybridizes with said specific nucleic acid sequence and/or which is degenerate with respect to said specific nucleic acid sequence will be at least 70%, preferably at least 75%, preferably at least 80%, more preferably at least 85%, even more preferably at least 90% or most preferably at least 95%, 96%, 97%, 98% or 99%. The degree of identity is preferably given for a region of at least about 30, at least about 50, at least about 70, at least about 90, at least about 100, at least about 150, at least about 200, at least about 250, at least about 300, or at least about 400 nucleotides. In preferred embodiments, the degree of identity is given for the entire length of the reference nucleic acid sequence, such as the nucleic acid sequences given in the sequence listing.

A nucleic acid is "complementary" to another nucleic acid if the two sequences are capable of hybridizing and forming a stable duplex with one another, with hybridization preferably being carried out under conditions which allow specific hybridization between polynucleotides (stringent conditions). Stringent conditions are described, for example, in Molecular Cloning: A Laboratory Manual, J. Sambrook et al., Editors, 2nd Edition, Cold Spring Harbor Laboratory press, Cold Spring Harbor, New York, 1989 or Current Protocols in Molecular Biology, F.M. Ausubel et al., Editors, John Wiley & Sons, Inc., New York and refer, for example, to hybridization at 65°C in hybridization buffer (3.5 x SSC, 0.02% Ficoll, 0.02% polyvinylpyrrolidone, 0.02% bovine serum albumin, 2.5 mM NaH₂PO₄ (pH 7), 0.5% SDS, 2 mM EDTA). SSC is 0.15 M sodium chloride/0.15 M sodium citrate, pH 7. After hybridization, the membrane to which the DNA has been transferred is washed, for example, in 2 × SSC at room temperature and then in 0.1-0.5 × SSC/0.1 × SDS at temperatures of up to 68°C.

A percent complementarity indicates the percentage of contiguous residues in a nucleic acid molecule that can form hydrogen bonds (e.g., Watson-Crick base pairing) with a second nucleic acid sequence (e.g., 5, 6, 7, 8, 9, 10 out of 10 being 50%, 60%, 70%, 80%, 90%, and 100% complementary). "Perfectly complementary" or "fully complementary" means that all the contiguous residues of a nucleic acid sequence will hydrogen bond with the same number of contiguous residues in a second nucleic acid sequence. Preferably, the degree of complementarity according to the invention is at least 70%, preferably at least 75%, preferably at least 80%, more preferably at least 85%, even more preferably at least 90% or most preferably at least 95%, 96%, 97%, 98% or 99%. Most preferably, the degree of complementarity according to the invention is 100%.

"Sequence similarity" indicates the percentage of amino acids that either are identical or that represent conservative amino acid substitutions. "Sequence identity" between two polypeptide or nucleic acid sequences indicates the percentage of amino acids or nucleotides that are identical between the sequences.

The term "percentage identity" is intended to denote a percentage of nucleotides or of amino acid residues which are identical between the two sequences to be compared, obtained after the best alignment, this percentage being purely statistical and the differences between the two sequences being distributed randomly and over their entire length. Sequence comparisons between two nucleotide or amino acid sequences are conventionally carried out by comparing these sequences after having aligned them optimally, said comparison being carried out by segment or by "window of comparison" in order to identify and compare local regions of sequence similarity. The optimal alignment of the sequences for comparison may be produced, besides manually, by means of the local homology algorithm of Smith and Waterman, 1981, Ads App. Math. 2, 482, by means of the local homology algorithm of Neddleman and Wunsch, 1970, J. Mol. Biol. 48, 443, by means of the similarity search method of Pearson and Lipman, 1988, Proc. Natl Acad. Sci. USA 85, 2444, or by means of computer programs which use these algorithms (GAP, BESTFIT, FASTA, BLAST P, BLAST N and TFASTA in Wisconsin Genetics Software Package, Genetics Computer Group, 575 Science Drive, Madison, Wis.).

The percentage identity is calculated by determining the number of identical positions between the two sequences being compared, dividing this number by the number of positions compared and multiplying the result obtained by 100 so as to obtain the percentage identity between these two sequences.

In one embodiment, a nucleic acid sequence which is a derivative of a specific nucleic acid sequence, which is degenerate with respect to a specific nucleic acid sequence or which is a part of a specific nucleic acid sequence has a relevant function and/or activity of the specific nucleic acid sequence, i.e. it may encode a protein or peptide having the same activity or immunological properties as the protein or peptide encoded by the specific nucleic acid sequence and, in one embodiment, encodes the same protein or peptide.

Nucleic acids coding for tumor-associated antigens may, according to the invention, be present alone or in combination with other nucleic acids, in particular heterologous nucleic acids. Preferably, a nucleic acid coding for a tumor-associated antigen or a part thereof expresses said tumor-associated antigen or part thereof. In preferred embodiments, a nucleic acid is functionally linked to expression control sequences or regulatory sequences which may be homologous or heterologous with respect to said nucleic acid. A coding sequence and a regulatory sequence are "functionally" linked to one another, if they are covalently linked to one another in such a way that expression or transcription of said coding sequence is under the control or under the influence of said regulatory sequence. If the coding sequence is to be translated into a functional protein, then, with a regulatory sequence functionally linked to said coding sequence, induction of said regulatory sequence results in transcription of said coding sequence, without causing a frame shift in the coding sequence or said coding sequence not being capable of being translated into the desired protein or peptide.

The term "expression control sequence" or "regulatory sequence" comprises according to the invention promoters, enhancers and other control elements which regulate expression of a gene. In particular embodiments of the invention, the expression control sequences can be regulated. The exact structure of regulatory sequences may vary as a function of the species or cell type, but generally comprises 5'untranscribed and 5'untranslated sequences which are involved in initiation of transcription and translation, respectively, such as TATA box, capping sequence, CAAT sequence, and the like. More specifically, 5'untranscribed regulatory sequences comprise a promoter region which includes a promoter sequence for transcriptional control of the functionally linked gene. Regulatory sequences may also comprise enhancer sequences or upstream activator sequences.

According to the invention, a nucleic acid may furthermore be present in combination with another nucleic acid which codes for a peptide controlling secretion of the protein or peptide encoded by said nucleic acid from a host cell. According to the invention, a nucleic acid may also be present in combination with another nucleic acid which codes for a peptide causing the encoded protein or peptide to be anchored on the cell membrane of the host cell or compartmentalized into particular organelles of said cell. Similarly, a combination with a nucleic acid is possible which represents a reporter gene or any "tag".

In a preferred embodiment, a recombinant nucleic acid molecule is according to the invention a vector, where appropriate with a promoter, which controls expression of a nucleic acid, for example a nucleic acid coding for a tumor-associated antigen identified according to the invention. The term "vector" is used here in its most general meaning and comprises any intermediary vehicle for a nucleic acid which enables said nucleic acid, for example, to be introduced into prokaryotic and/or eukaryotic cells and, where appropriate, to be integrated into a genome. Vectors of this kind are preferably replicated and/or expressed in the cells. An intermediary vehicle may be adapted, for example, to the use in electroporation, in bombardment with microprojectiles, in liposomal administration, in the transfer with the aid of agrobacteria or in insertion via DNA or RNA viruses. Vectors comprise plasmids, phagemids, bacteriophages or viral genomes.

The nucleic acids coding for a tumor-associated antigen identified according to the invention may be used for transfection of host cells. Nucleic acids here mean both recombinant DNA and RNA. Recombinant RNA may be prepared by in-vitro transcription of a DNA template. Furthermore, it may be modified by stabilizing sequences, capping and polyadenylation prior to application.

According to the invention, the term "host cell" relates to any cell which can be transformed or transfected with an exogenous nucleic acid. The term "host cells" comprises according to the invention prokaryotic (e.g. *E. coli*) or eukaryotic cells (e.g. dendritic cells, B cells, CHO cells, COS cells, K562 cells, yeast cells and insect cells). Particular preference is given to mammalian cells such as cells from humans, mice, hamsters, pigs, goats, primates. The cells may be derived from a multiplicity of tissue types and comprise primary cells and cell lines. Specific examples comprise keratinocytes, peripheral blood leukocytes, stem cells of the bone marrow and embryonic stem cells. In further embodiments, the host cell is an antigen-presenting cell, in particular a dendritic cell, monocyte or a macrophage. A nucleic acid may be present in the host cell in the form of a single copy or of two or more copies and, in one embodiment, is expressed in the host cell.

According to the invention, the term "expression" is used in its most general meaning and comprises the production of RNA or of RNA and protein. It also comprises partial expression of nucleic acids. Furthermore, expression may be carried out transiently or stably. Preferred expression systems in mammalian cells comprise pcDNA3.1 and pRc/CMV (Invitrogen, Carlsbad, CA), which contain a selectable marker such as a gene imparting resistance to G418 (and thus enabling stably transfected cell lines to be selected) and the enhancer-promoter sequences of cytomegalovirus (CMV).

In those cases of the invention in which a MHC molecule presents a tumor-associated antigen or a part thereof, an expression vector may also comprise a nucleic acid sequence coding for said MHC molecule. The nucleic acid sequence coding for the MHC molecule may be present on the same expression vector as the nucleic acid coding for the tumor-associated antigen or the part thereof, or both nucleic acids may be present on different expression vectors. In the latter case, the two expression vectors may be cotransfected into a cell. If a host cell expresses neither the tumor-associated antigen or the part thereof nor the MHC molecule, both nucleic acids coding therefor may be transfected into the cell either on the same expression vector or on different expression vectors. If the cell already expresses the MHC molecule, only the nucleic acid sequence coding for the tumor-associated antigen or the part thereof can be transfected into the cell.

The invention also comprises kits for detection and/or determination of the quantity of nucleic acids. Such kits comprise, for example, a pair of amplification primers which hybridize to the nucleic acid which is to be detected or the amount of which is to be determined. The primers preferably comprise a sequence of 6-50, in particular 10-30, 15-30 and 20-30 contiguous nucleotides of the nucleic acid and are nonoverlapping, in order to avoid the formation of primer dimers. One of the primers will hybridize to one strand of the nucleic acid, and the other primer will hybridize to the complementary strand in an arrangement which allows amplification of the nucleic acid.

"Antisense molecules" or "antisense nucleic acids" may be used for regulating, in particular reducing, expression of a nucleic acid. The term "antisense molecule" or "antisense nucleic acid" refers according to the invention to an oligonucleotide which is an oligoribonucleotide, oligodeoxyribonucleotide, modified oligoribonucleotide or modified oligodeoxyribonucleotide and which hybridizes under physiological conditions to DNA comprising a particular gene or to mRNA of said gene, thereby inhibiting transcription of said gene and/or translation of said mRNA. According to the invention, an "antisense molecule" also comprises a construct which contains a nucleic acid or a part thereof in reverse orientation with respect to its natural promoter. An antisense transcript of a nucleic acid or of a part thereof may form a duplex with naturally occurring mRNA and thus prevent accumulation of or translation of the mRNA. Another possibility is the use of ribozymes for inactivating a nucleic acid.

Antisense oligonucleotides preferred according to the invention have a sequence of 6-50, in particular 10-30, 15-30 and 20-30, contiguous nucleotides of the target nucleic acid and preferably are fully complementary to the target nucleic acid or to a part thereof.

In preferred embodiments, the antisense oligonucleotide hybridizes with an N-terminal or 5' upstream site such as a translation initiation site, transcription initiation site or promoter site. In further embodiments, the antisense oligonucleotide hybridizes with a 3'untranslated region or mRNA splicing site.

In one embodiment, an oligonucleotide of the invention consists of ribonucleotides, deoxyribonucleotides or a combination thereof, with the 5' end of one nucleotide and the 3' end of another nucleotide being linked to one another by a phosphodiester bond. These oligonucleotides may be synthesized in the conventional manner or produced recombinantly.

In preferred embodiments, an oligonucleotide of the invention is a "modified" oligonucleotide. Here, the oligonucleotide may be modified in very different ways, without impairing its ability to bind its target, in order to increase, for example, its stability or therapeutic efficacy. According to the invention, the term "modified oligonucleotide" means an oligonucleotide in which (i) at least two of its nucleotides are linked to one another by a synthetic internucleoside bond (i.e. an internucleoside bond which is not a phosphodiester bond) and/or (ii) a chemical group which is usually not found in nucleic acids is covalently linked to the oligonucleotide. Preferred synthetic internucleoside bonds are phosphorothioates, alkyl phosphonates, phosphorodithioates, phosphate esters, alkyl phosphonothioates, phosphoramidates, carbamates, carbonates, phosphate triesters, acetamidates, carboxymethyl esters and peptides.

The term "modified oligonucleotide" also comprises oligonucleotides having a covalently modified base and/or sugar. "Modified oligonucleotides" comprise, for example, oligonucleotides with sugar residues which are covalently bound to low molecular weight organic groups other than a hydroxyl group at the 3' position and a phosphate group at the 5' position. Modified oligonucleotides may comprise, for example, a 2'-O-alkylated ribose residue or another sugar instead of ribose, such as arabinose.

It is to be understood that all embodiments described above with respect to oligonucleotides may also apply to polynucleotides.

By "small interfering RNA" or "siRNA" as used herein is meant an isolated RNA molecule, preferably greater than 10 nucleotides in length, more preferably greater than 15 nucleotides in length, and most preferably 18, 19, 20, 21, 22, 23, 24, 25, 26, 27, 28, 29, or 30 nucleotides in length that is used to identify a target gene or mRNA to be degraded. A range of 19-25 nucleotides is the most preferred size for siRNAs.

siRNA according to the invention can comprise partially purified RNA, substantially pure RNA, synthetic RNA, or recombinantly produced RNA, as well as altered RNA that differs from naturally-occurring RNA by the addition, deletion, substitution and/or alteration of one or more nucleotides. Such alterations can include addition of non-nucleotide material, such as to the end(s) of the siRNA or to one or more internal nucleotides of the siRNA; modifications that make the siRNA resistant to nuclease digestion (e. g., the use of 2'-substituted ribonucleotides or modifications to the sugar-phosphate backbone); or the substitution of one or more nucleotides in the siRNA with deoxyribonucleotides. Furthermore, siRNA may be modified to increase the stability thereof as described above for modified oligonucleotides, in particular by introducing one or more phosphorothioate linkages.

One or both strands of the siRNA can also comprise a 3'-overhang. As used herein, a "3'-overhang" refers to at least one unpaired nucleotide extending from the 3'-end of an RNA strand. Thus in one embodiment, the siRNA comprises at least one 3'-overhang of from 1 to about 6 nucleotides (which includes ribonucleotides or deoxynucleotides) in length, preferably from 1 to about 5 nucleotides in length, more preferably from 1 to about 4 nucleotides in length, and particularly preferably from about 2 to about 4 nucleotides in length. In the embodiment in which both strands of the siRNA molecule comprise a 3'-overhang, the length of the overhangs can be the same or different for each strand. In a most preferred embodiment, the 3'-overhang is present on both strands of the siRNA, and is 2 nucleotides in length. For example, each strand of the siRNA of the invention can comprise 3'-overhangs of dideoxythymidylic acid ("TT") or diuridylic acid ("uu").

In order to enhance the stability of the siRNA, the 3'-overhangs can be also stabilized against degradation. In one embodiment, the overhangs are stabilized by including purine nucleotides, such as adenosine or guanosine nucleotides. Alternatively, substitution of pyrimidine nucleotides by modified analogues, e.g., substitution of uridine nucleotides in the 3'-overhangs with 2'-deoxythymidine, is tolerated and does not affect the efficiency of RNAi degradation. In particular, the absence of a 2'-hydroxyl in the 2'-deoxythymidine significantly enhances the nuclease resistance of the 3'-overhang in tissue culture medium.

The sense and antisense strands of the siRNA can comprise two complementary, single-stranded RNA molecules or can comprise a single molecule in which two complementary portions are base-paired and are covalently linked by a single-stranded "hairpin" area. That is, the sense region and antisense region can be covalently connected via a linker molecule. The linker molecule can be a polynucleotide or non-nucleotide linker. Without wishing to be bound by any theory, it is believed that the hairpin area of the latter type of siRNA molecule is cleaved intracellularly by the "Dicer" protein (or its equivalent) to form a siRNA of two individual base-paired RNA molecules.

As used herein, "target mRNA" refers to an RNA molecule that is a target for downregulation.

siRNA can be expressed from pol III expression vectors without a change in targeting site, as expression of RNAs from pol III promoters is only believed to be efficient when the first transcribed nucleotide is a purine.

siRNA according to the invention can be targeted to any stretch of approximately 19-25 contiguous nucleotides in any of the target mRNA sequences (the "target sequence"). Techniques for selecting target sequences for siRNA are given, for example, in Tuschl T. et al., "The siRNA User Guide", revised Oct. 11, 2002, the entire disclosure of which is herein incorporated by reference. "The siRNA User Guide" is available on the world wide web at a website maintained by Dr. Thomas Tuschl, Laboratory of RNA Molecular Biology, Rockefeller University, New York, USA, and can be found by accessing the website of the Rockefeller University and searching with the keyword "siRNA". Thus, the sense strand of the present siRNA comprises a nucleotide sequence substantially identical to any contiguous stretch of about 19 to about 25 nucleotides in the target mRNA.

Generally, a target sequence on the target mRNA can be selected from a given cDNA sequence corresponding to the target mRNA, preferably beginning 50 to 100 nt downstream (i.e., in the 3'-direction) from the start codon. The target sequence can, however, be located in the 5'- or 3'-untranslated regions, or in the region nearby the start codon.

siRNA can be obtained using a number of techniques known to those of skill in the art. For example, siRNA can be chemically synthesized or recombinantly produced using methods known in the art, such as the Drosophila in vitro system described in U.S. published application 2002/0086356 of Tuschl et al., the entire disclosure of which is herein incorporated by reference.

Preferably, siRNA is chemically synthesized using appropriately protected ribonucleoside phosphoramidites and a conventional DNA/RNA synthesizer. siRNA can be synthesized as two separate, complementary RNA molecules, or as a single RNA molecule with two complementary regions.

Alternatively, siRNA can also be expressed from recombinant circular or linear DNA plasmids using any suitable promoter. Such embodiments are included according to the present invention when reference is made herein to the administration of siRNA or the incorporation of siRNA into pharmaceutical compositions. Suitable promoters for expressing siRNA of the invention from a plasmid include, for example, the U6 or H1 RNA pol III promoter sequences and the cytomegalovirus promoter.

Selection of other suitable promoters is within the skill in the art. The recombinant plasmids of the invention can also comprise inducible or regulatable promoters for expression of the siRNA in a particular tissue or in a particular intracellular environment.

The siRNA expressed from recombinant plasmids can either be isolated from cultured cell expression systems by standard techniques, or can be expressed intracellularly. The use of recombinant plasmids to deliver siRNA to cells in vivo is discussed in more detail below. siRNA can be expressed from a recombinant plasmid either as two separate, complementary RNA molecules, or as a single RNA molecule with two complementary regions.

Selection of plasmids suitable for expressing siRNA, methods for inserting nucleic acid sequences for expressing the siRNA into the plasmid, and methods of delivering the recombinant plasmid to the cells of interest are within the skill in the art.

siRNA can also be expressed from recombinant viral vectors intracellularly in vivo. The recombinant viral vectors comprise sequences encoding the siRNA and any suitable promoter for expressing the siRNA sequences. The recombinant viral vectors can also comprise inducible or regulatable promoters for expression of the siRNA in a particular tissue or in a particular intracellular environment. siRNA can be expressed from a recombinant viral vector either as two separate, complementary RNA molecules, or as a single RNA molecule with two complementary regions.

The term "peptide" comprises oligo- and polypeptides and refers to substances comprising two or more, preferably 3 or more, preferably 4 or more, preferably 6 or more, preferably 8 or more, preferably 10 or more, preferably 13 or more, preferably 16 more, preferably 21 or more and up to preferably 8, 10, 20, 30, 40 or 50, in particular 100 amino acids joined covalently by peptide bonds. The term "protein" refers to large peptides, preferably to peptides with more than 100 amino acid residues, but in general the terms "peptides" and "proteins" are synonyms and are used interchangeably herein.

Preferably, the proteins and peptides described according to the invention have been isolated. The terms "isolated protein" or "isolated peptide" mean that the protein or peptide has been separated from its natural environment. An isolated protein or peptide may be in an essentially purified state. The term "essentially purified" means that the protein or peptide is essentially free of other substances with which it is associated in nature or *in vivo.*

Such proteins and peptides may be used, for example, in producing antibodies and in an immunological or diagnostic assay or as therapeutics. Proteins and peptides described according to the invention may be isolated from biological samples such as tissue or cell homogenates and may also be expressed recombinantly in a multiplicity of pro- or eukaryotic expression systems.

For the purposes of the present invention, "derivatives" of a protein or peptide or of an amino acid sequence comprise amino acid insertion variants, amino acid deletion variants and/or amino acid substitution variants.

Amino acid insertion variants comprise amino- and/or carboxy-terminal fusions and also insertions of single or two or more amino acids in a particular amino acid sequence. In the case of amino acid sequence variants having an insertion, one or more amino acid residues are inserted into a particular site in an amino acid sequence, although random insertion with appropriate screening of the resulting product is also possible.

Amino acid deletion variants are characterized by the removal of one or more amino acids from the sequence.

Amino acid substitution variants are characterized by at least one residue in the sequence being removed and another residue being inserted in its place. Preference is given to the modifications being in positions in the amino acid sequence which are not conserved between homologous proteins or peptides and/or to replacing amino acids with other ones having similar properties.

"Conservative substitutions" may be made, for instance, on the basis of similarity in polarity, charge, solubility, hydrophobicity, hydrophilicity, and/or the amphipathic nature of the residues involved. For example: (a) nonpolar (hydrophobic) amino acids include alanine, leucine, isoleucine, valine, proline, phenylalanine, tryptophan, and methionine; (b) polar neutral amino acids include glycine, serine, threonine, cysteine, tyrosine, asparagine, and glutamine; (c) positively charged (basic) amino acids include arginine, lysine, and histidine; and (d) negatively charged (acidic) amino acids include aspartic acid and glutamic acid. Substitutions typically may be made within groups (a)-(d). In addition, glycine and proline may be substituted for one another based on their ability to disrupt α-helices. Some preferred substitutions may be made among the following groups: (i) S and T; (ii) P and G; and (iii) A, V, L and I. Given the known genetic code, and recombinant and synthetic DNA techniques, the skilled scientist readily can construct DNAs encoding the conservative amino acid variants.

Preferably the degree of similarity, preferably identity between a specific amino acid sequence described herein and an amino acid sequence which is a derivative of said specific amino acid sequence will be at least 70%, preferably at least 80%, preferably at least 85%, even more preferably at least 90% or most preferably at least 95%, 96%, 97%, 98% or 99%. The degree of similarity or identity is given preferably for a region of at least about 20, at least about 40, at least about 60, at least about 80, at least about 100, at least about 120, at least about 140, at least about 160, at least about 200 or 250 amino acids. In preferred embodiments, the degree of similarity or identity is given for the entire length of the reference amino acid sequence.

The amino acid variants described above may be readily prepared with the aid of known peptide synthesis techniques such as, for example, by solid phase synthesis (Merrifield, 1964) and similar methods or by recombinant DNA manipulation. The manipulation of DNA sequences for preparing proteins and peptides having substitutions, insertions or deletions, is described in detail in Sambrook et al. (1989), for example.

According to the invention, "derivatives" of proteins and peptides include modified forms of proteins and peptides. Such modifications include any chemical modification and comprise single or multiple substitutions, deletions and/or additions of any molecules associated with the protein or peptide, such as carbohydrates, lipids and/or proteins or peptides. The term "derivative" also extends to all functional chemical equivalents of said proteins and peptides.

According to the invention, a part or fragment or derivative of a protein, peptide or nucleic acid, e.g. of a tumor-associated antigen or tumor-associated nucleic acid, preferably has a functional property of the protein, peptide or nucleic acid from which it has been derived. Such functional properties comprise the interaction with antibodies, the interaction with other peptides or proteins, the selective binding of nucleic acids and an enzymatic activity. In one embodiment, a part or fragment or derivative of a protein, peptide or nucleic acid is immunologically equivalent to the protein, peptide or nucleic acid from which it has been derived. In one embodiment, the functional property is an immunological property. A particular property is the ability to form a complex with MHC molecules and, where appropriate, generate an immune response, preferably by stimulating cytotoxic or T helper cells.

A part or fragment of a tumor-associated antigen or of an amino acid sequence described herein preferably comprises a sequence of at least 6, in particular at least 8, at least 10, at least 12, at least 15, at least 20, at least 30 or at least 50, consecutive amino acids of the tumor-associated antigen or of the amino acid sequence. A part or fragment of a tumor-associated antigen or of an amino acid sequence described herein preferably comprises a sequence of up to 8, in particular up to 10, up to 12, up to 15, up to 20, up to 30 or up to 55, consecutive amino acids of the tumor-associated antigen or of the amino acid sequence. A part or fragment of a tumor-associated antigen or of an amino acid sequence described herein is preferably a part of the tumor-associated antigen or of the amino acid sequence which corresponds to the non-transmembrane portion, in particular the extracellular portion of the antigen or amino acid sequence, or is comprised thereof. A part or fragment of a tumor-associated antigen or of an amino acid sequence described herein is preferably a part of the tumor-associated antigen or of the amino acid sequence which may be presented with MHC molecules and when so presented is capable of stimulating a cellular response.

A "cellular response" is meant to include a cellular response directed to cells characterized by presentation of a tumor-associated antigen with class I or class II MHC. The cellular response relates to cells called T cells or T lymphocytes which act as either 'helpers' or 'killers'. The helper T cells (also termed CD4+ T cells) play a central role by regulating the immune response and the killer cells (also termed cytotoxic T cells, cytolytic T cells, CD8+ T cells or CTLs) kill tumor cells, preventing the production of more tumor cells. Although both arms of the immune response are thought to be necessary, the CTL response may be more important for controlling cancer.

By "cell characterized by presentation of a tumor-associated antigen with class I MHC" or "cell presenting a tumor-associated antigen with class I MHC" or similar expressions is meant a cell such as a tumor cell or an antigen presenting cell presenting the tumor-associated antigen it expresses or a fragment derived from said tumor-associated antigen, e.g. by processing of the tumor-associated antigen, in the context of MHC Class I molecules. Similarly, the term "tumor characterized by presentation of a tumor-associated antigen with class I MHC" denotes a tumor comprising cells characterized by presentation of a tumor-associated antigen with class I MHC.

Preferred parts or fragments of a tumor-associated antigen or of an amino acid sequence described herein are in particular suitable for the stimulation of cytotoxic T-lymphocytes in vivo but also for the production of expanded and stimulated T-lymphocytes for the therapeutic adoptive transfer ex vivo.

In one aspect, a part or fragment of a tumor-associated antigen or of an amino acid sequence described herein comprises an amino acid sequence derived from the sequence of the tumor-associated antigen or of the amino acid sequence. Preferably, a part or fragment of a tumor-associated antigen or of an amino acid sequence described herein is capable of stimulating a cellular response against cells characterized by presentation of a tumor-associated antigen identified according to the invention with class I MHC and/or of elicting antibodies that specifically bind to a tumor-associated antigen identified according to the invention when used itself or attached to an immunogenic carrier. Preferably, a part or fragment of a tumor-associated antigen or of an amino acid sequence described herein may be presented, directly or following processing, with class I MHC molecules. Preferably, a part or fragment of a tumor-associated antigen or of an amino acid sequence described herein is a MHC class I and/or class II presented peptide or can be processed to produce a MHC class I and/or class II presented peptide. Preferably, a part or fragment of a tumor-associated antigen or of an amino acid sequence described herein comprises an amino acid sequence substantially corresponding to the amino acid sequence of a fragment of a tumor-associated antigen or of an amino acid sequence described herein.

A part or fragment of a tumor-associated antigen or of an amino acid sequence described herein may be present according to the invention as a peptide or peptide antigen as such or in combination with additional amino acid sequences, i.e. it may be comprised in a peptide or peptide antigen. Such peptides or peptide antigens are useful in the compositions and methods of the present invention and generally are included herein by the term "tumor-associated antigen". In general, such peptides or peptide antigens relate to oligopeptides or polypeptides comprising an amino acid sequence substantially corresponding to the amino acid sequence of a part or fragment of a tumor-associated antigen or of an amino acid sequence described herein. Preferably, such peptides or peptide antigens are capable of stimulating a cellular response against a tumor characterized by presentation of a tumor-associated antigen identified herein with class I MHC and/or of eliciting antibodies that specifically bind to a tumor-associated antigen identified according to the present invention.

If a peptide or peptide antigen is to be presented directly, i.e. without processing, in particular without cleavage, it has a length which is suitable for binding to an MHC molecule, in particular a class I MHC molecule, and preferably is 7-20 amino acids in length, more preferably 7-12 amino acids in length, more preferably 8-11 amino acids in length, in particular 9 or 10 amino acids in length. Preferably the sequence of a peptide or peptide antigen which is to be presented directly is derived from the amino acid sequence of a tumor-associated antigen or of an amino acid sequence described herein, i.e. its sequence substantially corresponds and is preferably completely identical to a fragment of a tumor-associated antigen or of an amino acid sequence described herein. If a peptide or peptide antigen is to be presented following processing, in particular following cleavage, the peptide produced by processing has a length which is suitable for binding to an MHC molecule, in particular a class I MHC molecule, and preferably is 7-20 amino acids in length, more preferably 7-12 amino acids in length, more preferably 8-11 amino acids in length, in particular 9 or 10 amino acids in length. Preferably the sequence of the peptide which is to be presented following processing is derived from the amino acid sequence of a tumor-associated antigen or of an amino acid sequence described herein, i.e. its sequence substantially corresponds and is preferably completely identical to a fragment of a tumor-associated antigen or of an amino acid sequence described herein. Thus, a peptide or peptide antigen according to the invention in one embodiment comprises a sequence of 7-20 amino acids in length, more preferably 7-12 amino acids in length, more preferably 8-11 amino acids in length, in particular 9 or 10 amino acids in length which substantially corresponds and is preferably completely identical to a fragment of a tumor-associated antigen or of an amino acid sequence described herein and following processing of the peptide or peptide antigen makes up the presented peptide. However, the peptide or peptide antigen may also comprise a sequence which substantially corresponds and preferably is completely identical to a fragment of a tumor-associated antigen or of an amino acid sequence described herein which is even longer than the above stated sequence. In one embodiment, a peptide or peptide antigen may comprise the entire sequence of a tumor-associated antigen or of an amino acid sequence described herein.

Peptides having amino acid sequences differing at residues that do not affect TCR recognition but improve the stability of binding to MHC may improve the immunogenicity of the peptide or peptide antigen, and may be referred to herein as "optimized peptides". Using existing knowledge about which of these residues may be more likely to affect binding either to the MHC or to the TCR, a rational approach to the design of substantially corresponding peptides may be employed. Resulting peptides that are functional are contemplated herein.

In one embodiment, a part or a fragment of a nucleic acid coding for a tumor-associated antigen relates according to the invention to the part of the nucleic acid, which codes at least for the tumor-associated antigen and/or for a part or a fragment of said tumor-associated antigen, as defined above. A part or fragment of a nucleic acid coding for a tumor-associated antigen is preferably that part of the nucleic acid corresponding to the open reading frame.

According to the invention, particular embodiments ought to involve providing "dominant negative" proteins or peptides derived from tumor-associated antigens. A dominant negative protein or peptide is an inactive protein or peptide variant which, by way of interacting with the cellular machinery, displaces an active protein or peptide from its interaction with the cellular machinery or which competes with the active protein or peptide, thereby reducing the effect of said active protein.

Antisera which contain specific antibodies specifically binding to the target protein can be prepared by various standard processes; see, for example, "Monoclonal Antibodies: A Practical Approach" by Philip Shepherd, Christopher Dean ISBN 0-19-963722-9; "Antibodies: A Laboratory Manual" by Ed Harlow, David Lane, ISBN: 0879693142 and "Using Antibodies: A Laboratory Manual: Portable Protocol NO" by Edward Harlow, David Lane, Ed Harlow ISBN 0879695447. Thereby it is also possible to generate affine and specific antibodies which recognize complex membrane proteins in their native form (Azorsa et al., J. Immunol. Methods 229: 35-48, 1999; Anderson et al., J. Immunol. 143: 1899-1904, 1989; Gardsvoll, J. Immunol. Methods 234: 107-116, 2000). This is in particular relevant for the preparation of antibodies which are to be used therapeutically, but also for many diagnostic applications. In this respect, it is possible to immunize with the whole protein, with extracellular partial sequences as well as with cells which express the target molecule in physiologically folded form.

Monoclonal antibodies are traditionally prepared using the hybridoma technology. (for technical details see: "Monoclonal Antibodies: A Practical Approach" by Philip Shepherd, Christopher Dean ISBN 0-19-963722-9; "Antibodies: A Laboratory Manual" by Ed Harlow, David Lane ISBN: 0879693142; "Using Antibodies: A Laboratory Manual: Portable Protocol NO" by Edward Harlow, David Lane, Ed Harlow ISBN: 0879695447).

It is known that only a small part of an antibody molecule, the paratope, is involved in binding of the antibody to its epitope (cf. Clark, W.R. (1986), The Experimental Foundations of Modern Immunology, Wiley & Sons, Inc., New York; Roitt, I. (1991), Essential Immunology, 7th Edition, Blackwell Scientific Publications, Oxford). The pFc' and Fc regions are, for example, effectors of the complement cascade but are not involved in antigen binding. An antibody from which the pFc' region has been enzymatically removed or which has been produced without the pFc' region, referred to as F(ab')₂ fragment, carries both antigen binding sites of a complete antibody. Similarly, an antibody from which the Fc region has been enzymatically removed or which has been produced without said Fc region, referred to as Fab fragment, carries one antigen binding site of an intact antibody molecule. Furthermore, Fab fragments consist of a covalently bound light chain of an antibody and part of the heavy chain of said antibody, referred to as Fd. The Fd fragments are the main determinants of antibody specificity (a single Fd fragment can be associated with up to ten different light chains, without altering the specificity of the antibody) and Fd fragments, when isolated, retain the ability to bind to an epitope.

Located within the antigen-binding part of an antibody are complementary-determining regions (CDRs) which interact directly with the antigen epitope and framework regions (FRs) which maintain the tertiary structure of the paratope. Both the Fd fragment of the heavy chain and the light chain of IgG immunoglobulins contain four framework regions (FR1 to FR4) which are separated in each case by three complementary-determining regions (CDR1 to CDR3). The CDRs and, in particular, the CDR3 regions and, still more particularly, the CDR3 region of the heavy chain are responsible to a large extent for antibody specificity.

Non-CDR regions of a mammalian antibody are known to be able to be replaced by similar regions of antibodies with the same or a different specificity, with the specificity for the epitope of the original antibody being retained. This made possible the development of "humanized" antibodies in which nonhuman CDRs are covalently linked to human FR and/or Fc/pFc' regions to produce a functional antibody.

As another example, WO 92/04381 describes the production and use of humanized murine RSV antibodies in which at least part of the murine FR regions have been replaced with FR regions of a human origin. Antibodies of this kind, including fragments of intact antibodies with antigen-binding capability, are often referred to as "chimeric" antibodies.

According to the invention, the term "antibody" also includes F(ab')₂, Fab, Fv, and Fd fragments of antibodies, chimeric antibodies, in which the Fc and/or FR and/or CDR1 and/or CDR2 and/or light chain-CDR3 regions have been replaced with homologous human or nonhuman sequences, chimeric F(ab')₂-fragment antibodies in which the FR and/or CDR1 and/or CDR2 and/or light chain-CDR3 regions have been replaced with homologous human or nonhuman sequences, chimeric Fab-fragment antibodies in which the FR and/or CDR1 and/or CDR2 and/or light chain-CDR3 regions have been replaced with homologous human or nonhuman sequences, and chimeric Fd-fragment antibodies in which the FR and/or CDR1 and/or CDR2 regions have been replaced with homologous human or nonhuman sequences. The term "antibody" also comprises "single-chain" antibodies.

The invention also comprises proteins and peptides which bind specifically to tumor-associated antigens. Binding substances of this kind may be provided, for example, by degenerate peptide libraries which may be prepared simply in solution in an immobilized form or as phage-display libraries. It is likewise possible to prepare combinatorial libraries of peptides with one or more amino acids. Libraries of peptoids and nonpeptidic synthetic residues may also be prepared.

Antibodies may also be coupled to specific diagnostic substances for displaying cells and tissues expressing tumor-associated antigens. They may also be coupled to therapeutically useful substances.

Diagnostic substances or agents include any label that functions to: (i) provide a detectable signal; (ii) interact with a second label to modify the detectable signal provided by the first or second label, e.g. FRET (Fluorescence Resonance Energy Transfer); (iii) affect mobility, e.g. electrophoretic mobility, by charge, hydrophobicity, shape, or other physical parameters, or (iv) provide a capture moiety, e.g., affinity, antibody/antigen, or ionic complexation. Suitable as label are structures, such as fluorescent labels, luminescent labels, chromophore labels, radioisotopic labels, isotopic labels, preferably stable isotopic labels, isobaric labels, enzyme labels, particle labels, in particular metal particle labels, magnetic particle labels, polymer particle labels, small organic molecules such as biotin, ligands of receptors or binding molecules such as cell adhesion proteins or lectins, label-sequences comprising nucleic acids and/or amino acid residues which can be detected by use of binding agents, etc. Diagnostic substances comprise, in a nonlimiting manner, barium sulfate, iocetamic acid, iopanoic acid, calcium ipodate, sodium diatrizoate, meglumine diatrizoate, metrizamide, sodium tyropanoate and radio diagnostic, including positron emitters such as fluorine-18 and carbon-11, gamma emitters such as iodine-123, technetium-99m, iodine-131 and indium-111, nuclides for nuclear magnetic resonance, such as fluorine and gadolinium.

According to the invention, the terms "therapeutically useful substance", "therapeutic substance" or "therapeutic agent" means any molecule which may exert a therapeutic effect. According to the invention, a therapeutically useful substance is preferably selectively guided to a cell which expresses one or more tumor-associated antigens and includes anticancer agents, radioactive compounds such as radioactive iodine-labeled compounds, toxins, cytostatic or cytolytic drugs, etc. Anticancer agents comprise, for example, aminoglutethimide, azathioprine, bleomycin sulfate, busulfan, carmustine, chlorambucil, cisplatin, cyclophosphamide, cyclosporine, cytarabidine, dacarbazine, dactinomycin, daunorubin, doxorubicin, taxol, etoposide, fluorouracil, interferon-α, lomustine, mercaptopurine, methotrexate, mitotane, procarbazine HCl, thioguanine, vinblastine sulfate and vincristine sulfate. Other anticancer agents are described, for example, in Goodman and Gilman, "The Pharmacological Basis of Therapeutics", 8th Edition, 1990, McGraw-Hill, Inc., in particular Chapter 52 (Antineoplastic Agents (Paul Calabresi and Bruce A. Chabner). Toxins may be proteins such as pokeweed antiviral protein, cholera toxin, pertussis toxin, ricin, gelonin, abrin, diphtheria exotoxin or *Pseudomonas* exotoxin. Toxin residues may also be high energy-emitting radionuclides such as cobalt-60.

The term "major histocompatibility complex" or "MHC" relates to a complex of genes present in all vertebrates. MHC proteins or molecules are involved in signaling between lymphocytes and antigen presenting cells in normal immune reactions by binding peptides and presenting them for recognition by T cell receptors (TCR). MHC molecules bind peptides within an intracellular processing compartment and present these peptides on the surface of antigen presenting cells for recognition by T cells. The human MHC region also termed HLA is located on chromosome 6 and includes the class I and class II region. In one preferred embodiment of all aspects of the invention an MHC molecule is an HLA molecule.

"Reduce" or "inhibit" as used herein means the ability to cause an overall decrease, preferably of 20% or greater, more preferably of 50% or greater, and most preferably of 75% or greater, in the level, e.g. in the level of protein or mRNA as compared to a reference sample (e.g., a sample not treated with siRNA). This reduction or inhibition of RNA or protein expression can occur through targeted mRNA cleavage or degradation. Assays for protein expression or nucleic acid expression are known in the art and include, for example, ELISA, western blot analysis for protein expression, and northern blotting or RNase protection assays for RNA.

The term "patient" means according to the invention a human being, a nonhuman primate or another animal, in particular a mammal such as a cow, horse, pig, sheep, goat, dog, cat or a rodent such as a mouse and rat. In a particularly preferred embodiment, the patient is a human being.

According to the invention the term "increased" or "increased amount" preferably refers to an increase by at least 10%, in particular at least 20%, at least 50% or at least 100%. The amount of a substance is also increased in a test sample such as a biological sample compared to a reference sample if it is detectable in the test sample but absent or not detectable in the reference sample.

According to the invention, the term "disease" refers to any pathological state in which tumor-associated nucleic acids and/or tumor-associated antigens are expressed or abnormally expressed. According to the invention, the term "tumor" or "tumor disease" refers to a swelling or lesion formed by an abnormal growth of cells (called neoplastic cells or tumor cells). By "tumor cell" is meant an abnormal cell that grows by a rapid, uncontrolled cellular proliferation and continues to grow after the stimuli that initiated the new growth cease. Tumors show partial or complete lack of structural organization and functional coordination with the normal tissue, and usually form a distinct mass of tissue, which may be either benign, pre-malignant or malignant. Preferably, a tumor disease according to the invention is a cancer disease, i.e. a malignant disease and a tumor cell is a cancer cell. Preferably, a tumor disease is characterized by cells in which a tumor-associated nucleic acid and/or tumor-associated antigen identified according to the invention is expressed or abnormally expressed and a tumor cell or a circulating or metastatic tumor cell is characterized by expression or abnormal expression of a tumor-associated nucleic acid and/or tumor-associated antigen identified according to the invention. Preferably, a tumor disease, a tumor cell or a circulating or metastatic tumor cell is characterized by presentation of a tumor-associated antigen identified according to the invention with class I MHC.

"Abnormal expression" means according to the invention that expression is altered, preferably increased, compared to the state in a healthy individual. An increase in expression refers to an increase by at least 10%, in particular at least 20%, at least 50% or at least 100%. In one embodiment, expression is only found in a diseased tissue, while expression in a healthy tissue is repressed or essentially repressed. One example of such a disease is cancer, wherein the term "cancer" according to the invention comprises leukemias, seminomas, melanomas, teratomas, lymphomas, neuroblastomas, gliomas, rectal cancer, endometrial cancer, kidney cancer, adrenal cancer, thyroid cancer, blood cancer, skin cancer, cancer of the brain, cervical cancer, intestinal cancer, liver cancer, colon cancer, stomach cancer, intestine cancer, head and neck cancer, gastrointestinal cancer, lymph node cancer, esophagus cancer, colorectal cancer, pancreas cancer, ear, nose and throat (ENT) cancer, breast cancer, prostate cancer, cancer of the uterus, ovarian cancer and lung cancer and the matastases thereof. Examples thereof are lung carcinomas, mamma carcinomas, prostate carcinomas, colon carcinomas, renal cell carcinomas, cervical carcinomas, or metastases of the cancer types or tumors described above. The term cancer according to the invention also comprises cancer metastases.

By "metastasis" is meant the spread of cancer cells from its original site to another part of the body. The formation of metastasis is a very complex process and depends on detachment of malignant cells from the primary tumor, invasion of the extracellular matrix, penetration of the endothelial basement membranes to enter the body cavity and vessels, and then, after being transported by the blood, infiltration of target organs. Finally, the growth of a new tumor at the target site depends on angiogenesis. Tumor metastasis often occurs even after the removal of the primary tumor because tumor cells or components may remain and develop metastatic potential. In one embodiment, the term "metastasis" according to the invention relates to "distant metastasis" which relates to a metastasis which is remote from the primary tumor and the regional lymph node system.

According to the invention, a biological sample may be a tissue sample, including bodily fluids, and/or a cellular sample and may be obtained in the conventional manner such as by tissue biopsy, including punch biopsy, and by taking blood, bronchial aspirate, sputum, urine, feces or other body fluids. In one embodiment, a biological sample is obtained from a tissue for which a connection between expression of the target nucleic acids or target antigens identified herein and a cancer disease is demonstrated herein. In one embodiment, a biological sample is obtained from a tissue for which overexpression of the target nucleic acids or target antigens identified herein in a cancer tissue compared to normal tissues is demonstrated herein. In this embodiment, the diagnostic methods disclosed herein aim at diagnosing cancer of said tissue. For example, in the case of lung cancer, the sample may be a sample comprising lung tissue. According to the invention, the term "biological sample" also includes processed biological samples such as fractions or isolates of biological samples, e.g. nucleic acid and peptide/protein isolates.. Preferably, the term "biological sample" according to the invention does not include samples derived from placental tissue or samples derived from tissues which demonstrate expression of the target nucleic acids or target antigens identified herein even if not affected by cancer.

Some aspects of the present invention envision the immunotherapy of tumor diseases, in particular cancer diseases, utilizing the tumor-associated nucleic acids and tumor-associated antigens identified according to the invention by means of active or passive immunotherapeutioc approaches which can be summarized as follows:

### Immunotherapy

### I. Active immunotherapy ("Cancer vaccines")

Immunisation with:
i) antigen or peptide (native or modified)
ii) nucleic acid encoding the antigen or peptide
iii) recombinant cells encoding the antigen or peptide
iv) recombinant viruses encoding the antigen or peptide
v) antigen presenting cells pulsed with antigen or peptide (native or modified) or transfected with nucleic acids encoding the antigen or peptide

### II. Passive immunotherapy ("Adoptive immunotherapy")

vi) Transfer of antibodies or T cell receptors that recognise antigen
vii) Transfer of cells sensitized in vitro to antigen (bulk or cloned populations)
viii) Transfer of effector cells (or stem cells) transduced with nucleic acids encoding T cell receptors that recognise antigen and preferably are responsive to tumor-specific class I MHC presented peptides

Antigen presenting cells (APC) such as dendritic cells (DCs) can be loaded with either MHC class I-presented peptide antigens or tumor lysate, or transduced with nucleic acid such as by transduction using adenovirus encoding a tumor-associated antigen, in particular a peptide antigen.

In a preferred embodiment, an anti-tumor vaccine of the invention comprises an APC loaded with peptide antigen. In this respect, protocols may rely on *in vitro* culture/differentiation of DCs manipulated in such a way that they artificially present peptide antigen. Production of genetically engineered DCs may involve introduction of nucleic acids encoding tumor-associated antigens or peptide antigens into DCs. Transfection of DCs with mRNA is a promising antigen-loading technique of stimulating strong antitumor immunity.

If used for passive anti-tumor immunotherapy, antibodies may or may not be attached to therapeutic effector moieties, e.g., radiolabels, cytotoxins, therapeutic enzymes, agents that induce apoptosis, and the like in order to provide for targeted cytotoxicity, i.e., killing of tumor cells. In one embodiment of the present invention, such antibodies or fragments are administered in labeled or unlabeled form, alone or in conjunction with other therapeutics, e.g., chemotherapeutics such as cisplatin, methotrexate, adriamycin, and the like suitable for cancer therapy.

Preferably the antibodies described herein mediate killing of cells by inducing complement dependent cytotoxicity (CDC) mediated lysis, antibody dependent cellular cytotoxicity (ADCC) mediated lysis, apoptosis, homotypic adhesion, and/or phagocytosis, preferably by inducing CDC mediated lysis and/or ADCC mediated lysis. The antibodies described herein preferably interact with components of the immune system, preferably through ADCC or CDC. However, antibodies of the invention may also exert an effect simply by binding to tumor-associated antigens on the cell surface, thus, e.g. blocking proliferation of the cells.

ADCC describes the cell-killing ability of effector cells as described herein, in particular lymphocytes, which preferably requires the target cell being marked by an antibody.

ADCC preferably occurs when antibodies bind to antigens on tumor cells and the antibody Fc domains engage Fc receptors (FcR) on the surface of immune effector cells. Several families of Fc receptors have been identified, and specific cell populations characteristically express defined Fc receptors. ADCC can be viewed as a mechanism to directly induce a variable degree of immediate tumor destruction that also leads to antigen presentation and the induction of tumor-directed T-cell responses. Preferably, *in vivo* induction of ADCC will lead to tumor-directed T-cell responses and host-derived antibody responses.

CDC is another cell-killing method that can be directed by antibodies. IgM is the most effective isotype for complement activation. IgG1 and IgG3 are also both very effective at directing CDC via the classical complement-activation pathway. Preferably, in this cascade, the formation of antigen-antibody complexes results in the uncloaking of multiple C1q binding sites in close proximity on the C_{H}2 domains of participating antibody molecules such as IgG molecules (C1q is one of three subcomponents of complement C1). Preferably these uncloaked C1q binding sites convert the previously low-affinity C1q-IgG interaction to one of high avidity, which triggers a cascade of events involving a series of other complement proteins and leads to the proteolytic release of the effector-cell chemotactic/activating agents C3a and C5a. Preferably, the complement cascade ends in the formation of a membrane attack complex, which creates pores in the cell membrane that facilitate free passage of water and solutes into and out of the cell and may lead to apoptosis.

Passive immunotherapy with immune cells (optionally genetically modified) capable of recognizing tumor-associated antigens is effective in mediating the regression of cancer in selected patients. These techniques may be based on ex-vivo reactivation and expansion of cloned or polyclonal cultures of tumor reactive T cells. After culture, T cells may be reinfused into the patient along with IL-2. In vitro techniques have been developed in which human lymphocytes are sensitized in vitro to tumor peptide antigens presented on antigen presenting cells. By repetitive in vitro stimulation cells can be derived with a great capacity to recognize human tumor-associated antigens. The adoptive transfer of these cells may be more effective in mediating tumor regression in vivo than are conventionally grown cells.

According to the invention, the term "immunoreactive cell" means a cell which can mature into an immune cell (such as B cell, T helper cell, or cytolytic T cell) with suitable stimulation. Immunoreactive cells comprise CD34⁺ hematopoietic stem cells, immature and mature T cells and immature and mature B cells. If production of cytolytic or T helper cells recognizing a tumor-associated antigen is desired, the immunoreactive cell is contacted with a cell expressing a tumor-associated antigen under conditions which favor production, differentiation and/or selection of cytolytic T cells and of T helper cells. The differentiation of T cell precursors into a cytolytic T cell, when exposed to an antigen, is similar to clonal selection of the immune system.

The terms "T cell" and "T lymphocyte" are used interchangeably herein and include T helper cells and cytotoxic T cells which comprise cytolytic T cells.

Some therapeutic methods are based on a reaction of the immune system of a patient, which results in a lysis of antigen-presenting cells such as cancer cells which present one or more tumor-associated antigens. In this connection, for example autologous cytotoxic T lymphocytes specific for a complex of a tumor-associated antigen and an MHC molecule are administered to a patient having a cellular abnormality. The production of such cytotoxic T lymphocytes *in vitro* is known. An example of a method of differentiating T cells can be found in WO-A-9633265. Generally, a sample containing cells such as blood cells is taken from the patient and the cells are contacted with a cell which presents the complex and which can cause propagation of cytotoxic T lymphocytes (e.g. dendritic cells). The target cell may be a transfected cell such as a COS cell. These transfected cells present the desired complex on their surface and, when contacted with cytotoxic T lymphocytes, stimulate propagation of the latter. The clonally expanded autologous cytotoxic T lymphocytes are then administered to the patient.

In another method of selecting antigen-specific cytotoxic T lymphocytes, fluorogenic tetramers of MHC class I molecule/peptide complexes are used for obtaining specific clones of cytotoxic T lymphocytes (Altman et al., Science 274:94-96, 1996; Dunbar et al., Curr. Biol. 8:413-416, 1998).

The present invention also includes therapeutic methods referred to as adoptive transfer (Greenberg, J. Immunol. 136(5):1917, 1986; Riddel et al., Science 257:238, 1992; Lynch et al., Eur. J. Immunol. 21:1403-1410, 1991; Kast et al., Cell 59:603-614, 1989), wherein cells presenting the desired complex (e.g. dendritic cells) are combined with cytotoxic T lymphocytes of the patient to be treated, resulting in a propagation of specific cytotoxic T lymphocytes. The propagated cytotoxic T lymphocytes are then administered to a patient having a cellular anomaly characterized by particular abnormal cells presenting the specific complex. The cytotoxic T lymphocytes then lyse the abnormal cells, thereby achieving a desired therapeutic effect.

Furthermore, cells presenting the desired complex (e.g. dendritic cells) may be combined with cytotoxic T lymphocytes of healthy individuals or another species (e.g. mouse) which may result in propagation of specific cytotoxic T lymphocytes with high affinity. The high affinity T cell receptor of these propagated specific T lymphocytes may be cloned and optionally humanized to a different extent, and the T cell receptors thus obtained then transduced via gene transfer, for example using retroviral vectors, into T cells of patients. Adoptive transfer may then be carried out using these genetically altered T lymphocytes (Stanislawski et al., Nat Immunol. 2:962-70, 2001; Kessels et al., Nat Immunol. 2:957-61, 2001).

Adoptive transfer is not the only form of therapy which can be applied according to the invention. Cytotoxic T lymphocytes may also be generated *in vivo* in a manner known per se. One method uses nonproliferative cells expressing the complex. The cells used here will be those which usually express the complex, such as irradiated tumor cells or cells transfected with one or both genes necessary for presentation of the complex (i.e. the antigenic peptide and the presenting MHC molecule). Another preferred form is the introduction of the tumor-associated antigen in the form of recombinant RNA which may be introduced into cells by liposomal transfer or by electroporation, for example. The resulting cells present the complex of interest and are recognized by autologous cytotoxic T lymphocytes which then propagate.

A similar effect can be achieved by combining the tumor-associated antigen or a fragment thereof with an adjuvant in order to make incorporation into antigen-presenting cells *in vivo* possible. The tumor-associated antigen or a fragment thereof may be represented as protein, as DNA (e.g. within a vector) or as RNA. The tumor-associated antigen is processed to produce a peptide partner for the MHC molecule, while a fragment thereof may be presented without the need for further processing. The latter is the case in particular, if these can bind to MHC molecules. Preference is given to administration forms in which the complete antigen is processed *in vivo* by a dendritic cell, since this may also produce T helper cell responses which are needed for an effective immune response (Ossendorp et al., Immunol Lett. 74:75-9, 2000; Ossendorp et al., J. Exp. Med. 187:693-702, 1998). In general, it is possible to administer an effective amount of the tumor-associated antigen to a patient by intradermal injection, for example. However, injection may also be carried out intranodally into a lymph node (Maloy et al., Proc Natl Acad Sci USA 98:3299-303, 2001).

The pharmaceutical compositions and methods of treatment described according to the invention may also be used for immunization or vaccination to therapeutically treat or prevent a disease described herein. According to the invention, the terms "immunization" or "vaccination" preferably relate to an increase in or activation of an immune response to an antigen. It is possible to use animal models for testing an immunizing effect on cancer by using a tumor-associated antigen or a nucleic acid coding therefor. For example, human cancer cells may be introduced into a mouse to generate a tumor, and one or more nucleic acids coding for tumor-associated antigens may be administered. The effect on the cancer cells (for example reduction in tumor size) may be measured as a measure for the effectiveness of an immunization by the nucleic acid.

As part of the composition for an immunization or a vaccination, preferably one or more tumor-associated antigens or stimulating fragments thereof are administered together with one or more adjuvants for inducing an immune response or for increasing an immune response. An adjuvant is a substance which is incorporated into the antigen or administered together with the latter and which enhances the immune response. Adjuvants may enhance the immune response by providing an antigen reservoir (extracellularly or in macrophages), activating macrophages and/or stimulating particular lymphocytes. Adjuvants are known and comprise in a nonlimiting way monophosphoryl lipid A (MPL, SmithKline Beecham), saponins such as QS21 (SmithKline Beecham), DQS21 (SmithKline Beecham; WO 96/33739), QS7, QS17, QS18 and QS-L1 (So et al., Mol. Cells 7:178-186, 1997), incomplete Freund's adjuvant, complete Freund's adjuvant, vitamin E, montanide, alum, CpG oligonucleotides (cf. Kreig et al., Nature 374:546-9, 1995) and various water-in-oil emulsions prepared from biologically degradable oils such as squalene and/or tocopherol. Preferably, the peptides are administered in a mixture with DQS21/MPL. The ratio of DQS21 to MPL is typically about 1:10 to 10:1, preferably about 1:5 to 5:1 and in particular about 1:1. For administration to humans, a vaccine formulation typically contains DQS21 and MPL in a range from about 1 µg to about 100 µg.

Other substances which stimulate an immune response of the patient may also be administered. It is possible, for example, to use cytokines in a vaccination, owing to their regulatory properties on lymphocytes. Such cytokines comprise, for example, interleukin-12 (IL-12) which was shown to increase the protective actions of vaccines (cf. Science 268:1432-1434, 1995), GM-CSF and IL-18.

There are a number of compounds which enhance an immune response and which therefore may be used in a vaccination. Said compounds comprise costimulating molecules provided in the form of proteins or nucleic acids such as B7-1 and B7-2 (CD80 and CD86, respectively).

The invention also provides for administration of nucleic acids, proteins or peptides. Proteins and peptides may be administered in a manner known per se. In one embodiment, nucleic acids are administered by *ex vivo* methods, i.e. by removing cells from a patient, genetic modification of said cells in order to incorporate a tumor-associated antigen and reintroduction of the altered cells into the patient. This generally comprises introducing a functional copy of a gene into the cells of a patient *in vitro* and reintroducing the genetically altered cells into the patient. The functional copy of the gene is under the functional control of regulatory elements which allow the gene to be expressed in the genetically altered cells. Transfection and transduction methods are known to the skilled worker. The invention also provides for administering nucleic acids *in vivo* by using vectors such as viruses and target-controlled liposomes. If according to the invention reference is made to the administration or incorporation into pharmaceutical compositions of nucleic acids this includes embodiments wherein the nucleic acid is present in such vectors.

In a preferred embodiment, a virus or viral vector for administering a nucleic acid coding for a tumor-associated antigen is selected from the group consisting of adenoviruses, adeno-associated viruses, pox viruses, including vaccinia virus and attenuated pox viruses, Semliki Forest virus, retroviruses, Sindbis virus and Ty virus-like particles. Particular preference is given to adenoviruses and retroviruses. The retroviruses are typically replication-deficient (i.e. they are incapable of generating infectious particles).

Methods of introducing nucleic acids into cells *in vitro* or *in vivo* comprise transfection of nucleic acid calcium phosphate precipitates, transfection of nucleic acids associated with DEAE, transfection or infection with the above viruses carrying the nucleic acids of interest, liposome-mediated transfection, and the like. In particular embodiments, preference is given to directing the nucleic acid to particular cells. In such embodiments, a carrier used for administering a nucleic acid to a cell (e.g. a retrovirus or a liposome) may have a bound target control molecule. For example, a molecule such as an antibody specific for a surface membrane protein on the target cell or a ligand for a receptor on the target cell may be incorporated into or attached to the nucleic acid carrier. Preferred antibodies comprise antibodies which bind selectively a tumor-associated antigen. If administration of a nucleic acid via liposomes is desired, proteins binding to a surface membrane protein associated with endocytosis may be incorporated into the liposome formulation in order to make target control and/or uptake possible. Such proteins comprise capsid proteins or fragments thereof which are specific for a particular cell type, antibodies to proteins which are internalized, proteins addressing an intracellular site, and the like.

The therapeutic compositions of the invention may be administered in pharmaceutically compatible preparations. Such preparations may usually contain pharmaceutically compatible concentrations of salts, buffer substances, preservatives, carriers, supplementing immunity-enhancing substances such as adjuvants, e.g. CpG oligonucleotides, cytokines, chemokines, saponin, GM-CSF and/or RNA and, where appropriate, other therapeutically active compounds.

The therapeutically active compounds of the invention may be administered via any conventional route, including by injection or infusion. The administration may be carried out, for example, orally, intravenously, intraperitonealy, intramuscularly, subcutaneously or transdermally. Preferably, antibodies are therapeutically administered by way of a lung aerosol. Antisense nucleic acids are preferably administered by slow intravenous administration.

The compositions of the invention are administered in effective amounts. An "effective amount" refers to the amount which achieves a desired reaction or a desired effect alone or together with further doses. In the case of treatment of a particular disease or of a particular condition characterized by expression of one or more tumor-associated antigens, the desired reaction preferably relates to inhibition of the course of the disease. This comprises slowing down the progress of the disease and, in particular, interrupting or reversing the progress of the disease. The desired reaction in a treatment of a disease or of a condition may also be delay of the onset or a prevention of the onset of said disease or said condition. According to the invention, a diagnosis or treatment of cancer may also include the diagnosis or treatment of cancer metastases which have already formed or will form. According to the invention, the term "treatment" comprises therapeutic and prophylactic treatment, i.e. prevention.

An effective amount of a composition of the invention will depend on the condition to be treated, the severeness of the disease, the individual parameters of the patient, including age, physiological condition, size and weight, the duration of treatment, the type of an accompanying therapy (if present), the specific route of administration and similar factors.

The pharmaceutical compositions of the invention are preferably sterile and contain an effective amount of the therapeutically active substance to generate the desired reaction or the desired effect.

The doses administered of the compositions of the invention may depend on various parameters such as the type of administration, the condition of the patient, the desired period of administration, etc. In the case that a reaction in a patient is insufficient with an initial dose, higher doses (or effectively higher doses achieved by a different, more localized route of administration) may be used.

Generally, doses of the tumor-associated antigen of from 1 ng to 1 mg, preferably from 10 ng to 100 µg, are formulated and administered for a treatment or for generating or increasing an immune response. If the administration of nucleic acids (DNA and RNA) coding for tumor-associated antigens is desired, doses of from 1 ng to 0.1 mg are formulated and administered.

The pharmaceutical compositions of the invention are generally administered in pharmaceutically compatible amounts and in pharmaceutically compatible compositions. The term "pharmaceutically compatible" refers to a nontoxic material which does not interact with the action of the active component of the pharmaceutical composition. Preparations of this kind may usually contain salts, buffer substances, preservatives, carriers and, where appropriate, other therapeutically active compounds. When used in medicine, the salts should be pharmaceutically compatible. However, salts which are not pharmaceutically compatible may used for preparing pharmaceutically compatible salts and are included in the invention. Pharmacologically and pharmaceutically compatible salts of this kind comprise in a nonlimiting way those prepared from the following acids: hydrochloric, hydrobromic, sulfuric, nitric, phosphoric, maleic, acetic, salicylic, citric, formic, malonic, succinic acids, and the like. Pharmaceutically compatible salts may also be prepared as alkali metal salts or alkaline earth metal salts, such as sodium salts, potassium salts or calcium salts.

A pharmaceutical composition of the invention may comprise a pharmaceutically compatible carrier. According to the invention, the term "pharmaceutically compatible carrier" refers to one or more compatible solid or liquid fillers, diluents or encapsulating substances, which are suitable for administration to humans. The term "carrier" refers to an organic or inorganic component, of a natural or synthetic nature, in which the active component is combined in order to facilitate application. The components of the pharmaceutical composition of the invention are usually such that no interaction occurs which substantially impairs the desired pharmaceutical efficacy.

The pharmaceutical compositions of the invention may contain suitable buffer substances such as acetic acid in a salt, citric acid in a salt, boric acid in a salt and phosphoric acid in a salt.

The pharmaceutical compositions may, where appropriate, also contain suitable preservatives such as benzalkonium chloride, chlorobutanol, paraben and thimerosal.

The pharmaceutical compositions are usually provided in a uniform dosage form and may be prepared in a manner known per se. Pharmaceutical compositions of the invention may be in the form of capsules, tablets, lozenges, solutions, suspensions, syrups, elixirs or in the form of an emulsion, for example.

Compositions suitable for parenteral administration usually comprise a sterile aqueous or nonaqueous preparation of the active compound, which is preferably isotonic to the blood of the recipient. Examples of compatible carriers and solvents are Ringer solution and isotonic sodium chloride solution. In addition, usually sterile, fixed oils are used as solution or suspension medium.

The present invention is described in detail by the figures and examples below, which are used only for illustration purposes and are not meant to be limiting. Owing to the description and the examples, further embodiments which are likewise included in the invention are accessible to the skilled worker.

### Figures:

### Fig. 1. Expression of a tumor-associated nucleic acid identified according to the present invention in normal tissues and cancer tissue

Significant expression of the nucleic acid sequence according to SEQ ID NO:540 was found only in placenta tissue and mamma carcinomas.

### Fig. 2. Quantitative expression of a tumor-associated nucleic acid identified according to the present invention in normal tissues and cancer tissue

Quantitative RT-PCR showed selective expression of the nucleic acid sequence according to SEQ ID NO:540 in placenta tissue and mamma carcinomas.

### Fig. 3. Quantitative expression of SEQ ID NO:540 mRNA in MCF-7 breast cancer cells

Real-time RT-PCR 24h after transfection with siRNA oligos showed that both SEQ ID NO:540-specific siRNAs (siRNA#1 (SEQ ID NO:630, 631), siRNA#2 (SEQ ID NO:632, 633)) induce robust silencing of SEQ ID NO:540 expression.

### Fig. 4. Silencing of SEQ ID NO:540 expression by transfection with siRNA oligos results in impaired proliferation of MCF-7 breast cancer cells

Proliferation was quantified 96h after transfection with siRNAs by measuring incorporation of BrdU in newly synthesized DNA strands. These results show that SEQ ID NO:540 is a positive factor for the proliferation of breast cancer cells.

### Fig. 5. Quantitative expression of SEQ ID NO:541 in normal tissues and cancer tissue

Real-time RT-PCR showed overexpression of the nucleic acid sequence according to SEQ ID NO:541 in lung cancer.

### Fig. 6. Quantitative expression of SEQ ID NO:545 in normal tissues and cancer tissues

Real-time RT-PCR showed overexpression of the nucleic acid sequence according to SEQ ID NO:545 in malignant melanomas.

### Fig. 7. Quantitative expression of SEQ ID NO:549 in normal tissues and cancer tissues

Real-time RT-PCR showed overexpression of the nucleic acid sequence according to SEQ ID NO: 549 in ovarian cancer.

### Fig. 8. Quantitative expression of SEQ ID NO:553 in normal tissues and cancer tissues

Real-time RT-PCR showed overexpression of the nucleic acid sequence according to SEQ ID NO:553 in colon cancer and ovarian cancer.

### Fig. 9. Quantitative expression of SEQ ID NO:557 in normal tissues and cancer tissues

Real-time RT-PCR showed overexpression of the nucleic acid sequence according to SEQ ID NO:557 in breast cancer.

### Fig. 10. Quantitative expression of SEQ ID NO:560 in normal tissues and cancer tissues

Real-time RT-PCR showed overexpression of the nucleic acid sequence according to SEQ ID NO:560 in colon cancer and ovarian cancer.

### Fig. 11. Quantitative expression of SEQ ID NO:563 in normal tissues and cancer tissues

Real-time RT-PCR showed overexpression of the nucleic acid sequence according to SEQ ID NO:563 in breast cancer, colon cancer, ovarian cancer, lung cancer and melanoma.

### Fig. 12. Quantitative expression of SEQ ID NO:566 in normal tissues and cancer tissues

Real-time RT-PCR showed overexpression of the nucleic acid sequence according to SEQ ID NO:566 in gastric cancer, breast cancer, colon cancer, ovarian cancer, lung cancer and melanoma.

### Fig. 13. Quantitative expression of SEQ ID NO:570 in normal tissues and cancer tissues

Real-time RT-PCR showed overexpression of the nucleic acid sequence according to SEQ ID NO: 570 in ovarian cancer, lung cancer and melanoma.

### Fig. 14. Quantitative expression of SEQ ID NO:574 in normal tissues and cancer tissues

Real-time RT-PCR showed overexpression of the nucleic acid sequence according to SEQ ID NO:574 in lung cancer and melanoma.

### Fig. 15. Quantitative expression of SEQ ID NO:577 in normal tissues and cancer tissues

Real-time RT-PCR showed overexpression of the nucleic acid sequence according to SEQ ID NO:577 in gastric cancer, breast cancer and lung cancer.

### Fig. 16. Quantitative expression of SEQ ID NO:580 in normal tissues and cancer tissues

Real-time RT-PCR showed overexpression of the nucleic acid sequence according to SEQ ID NO:580 in ovarian cancer and lung cancer.

### Fig. 17. Quantitative expression of SEQ ID NO:583 in normal tissues and cancer tissues

Real-time RT-PCR showed overexpression of the nucleic acid sequence according to SEQ ID NO:583 in colon cancer, ovarian cancer and lung cancer.

### Fig. 18. Quantitative expression of SEQ ID NO:587 in normal tissues and cancer tissues

Real-time RT-PCR showed overexpression of the nucleic acid sequence according to SEQ ID NO: 587 in lung cancer.

### Fig. 19. Quantitative expression of SEQ ID NO:591 in normal tissues and cancer tissues

Real-time RT-PCR showed overexpression of the nucleic acid sequence according to SEQ ID NO: 591 in breast cancer, colon cancer, ovarian cancer, lung cancer and melanoma.

### Fig. 20. Quantitative expression of SEQ ID NO:595 in normal tissues and cancer tissues

Real-time RT-PCR showed overexpression of the nucleic acid sequence according to SEQ ID NO:595 in gastric cancer, colon cancer, ovarian cancer, lung cancer and melanoma.

### Fig. 21. Quantitative expression of SEQ ID NO:599 in normal tissues and cancer tissues

Real-time RT-PCR showed overexpression of the nucleic acid sequence according to SEQ ID NO:599 in gastric cancer, breast cancer, lung cancer and melanoma.

### Fig. 22. Quantitative expression of SEQ ID NO:602 in normal tissues and cancer tissues

Real-time RT-PCR showed overexpression of the nucleic acid sequence according to SEQ ID NO:602 in ovarian cancer and lung cancer.

### Fig. 23. Quantitative expression of SEQ ID NO:606 in normal tissues and cancer tissues

Real-time RT-PCR showed overexpression of the nucleic acid sequence according to SEQ ID NO:606 in gastric cancer, colon cancer and lung cancer.

### Fig. 24. Quantitative expression of SEQ ID NO:610 in normal tissues and cancer tissues

Real-time RT-PCR showed overexpression of the nucleic acid sequence according to SEQ ID NO:610 in gastric cancer, breast cancer and lung cancer.

### Fig. 25. Quantitative expression of SEQ ID NO:613 in normal tissues and cancer tissues

Real-time RT-PCR showed overexpression of the nucleic acid sequence according to SEQ ID NO:613 in breast cancer, lung cancer and melanoma.

### Fig. 26. Quantitative expression of SEQ ID NO:617 in normal tissues and cancer tissues

Real-time RT-PCR showed overexpression of the nucleic acid sequence according to SEQ ID NO:617 in lung cancer and melanoma.

### Fig. 27. Quantitative expression of SEQ ID NO:620 in normal tissues and cancer tissues

Real-time RT-PCR showed overexpression of the nucleic acid sequence according to SEQ ID NO:620 in ovarian cancer and melanoma.

### Fig. 28. Quantitative expression of SEQ ID NO:624 in normal tissues and cancer tissues

Real-time RT-PCR showed overexpression of the nucleic acid sequence according to SEQ ID NO:624 in gastric cancer and lung cancer.

### Fig. 29. Quantitative expression of SEQ ID NO:634 in normal tissues and cancer tissues

Real-time RT-PCR showed overexpression of the nucleic acid sequence according to SEQ ID NO:634 in ovarian cancer and lung cancer.

### Fig. 30. Quantitative expression of SEQ ID NO:638 in normal tissues and cancer tissues

Real-time RT-PCR showed overexpression of the nucleic acid sequence according to SEQ ID NO:638 in colon cancer, ovarian cancer, lung cancer, and malignant melanomas.

### Fig. 31. Quantitative expression of SEQ ID NO:642 in normal tissues and cancer tissues

Real-time RT-PCR showed overexpression of the nucleic acid sequence according to SEQ ID NO:642 in breast cancer, ovarian cancer, lung cancer, and malignant melanomas.

### Fig. 32. Quantitative expression of SEQ ID NO:646 in normal tissues and cancer tissues

Real-time RT-PCR showed overexpression of the nucleic acid sequence according to SEQ ID NO:646 in gastric cancer, ovarian cancer, and lung cancer.

### Fig. 33. Quantitative expression of SEQ ID NO:649 in normal tissues and cancer tissues

Real-time RT-PCR showed overexpression of the nucleic acid sequence according to SEQ ID NO:649 in ovarian cancer, lung cancer, and malignant melanomas.

### Fig. 34. Quantitative expression of SEQ ID NO:653 in normal tissues and cancer tissues

Real-time RT-PCR showed overexpression of the nucleic acid sequence according to SEQ ID NO:653 in colon cancer, and lung cancer.

### Fig. 35. Quantitative expression of SEQ ID NO:656 in normal tissues and cancer tissues

Real-time RT-PCR showed overexpression of the nucleic acid sequence according to SEQ ID NO:656 in lung cancer, and malignant melanomas.

### Fig. 36. Quantitative expression of SEQ ID NO:660 in normal tissues and cancer tissues

Real-time RT-PCR showed overexpression of the nucleic acid sequence according to SEQ ID NO:660 in gastric cancer, ovarian cancer, and malignant melanomas.

### Fig. 37. Quantitative expression of SEQ ID NO:664 in normal tissues and cancer tissues

Real-time RT-PCR showed overexpression of the nucleic acid sequence according to SEQ ID NO:664 in gastric cancer, colon cancer, lung cancer, and malignant melanomas.

### Fig. 38. Quantitative expression of SEQ ID NO:668 in normal tissues and cancer tissues

Real-time RT-PCR showed overexpression of the nucleic acid sequence according to SEQ ID NO:668 in lung cancer.

### Fig. 39. Quantitative expression of SEQ ID NO:671 in normal tissues and cancer tissues

Real-time RT-PCR showed overexpression of the nucleic acid sequence according to SEQ ID NO:671 in lung cancer, and malignant melanomas.

### Fig. 40. Quantitative expression of SEQ ID NO:675 in normal tissues and cancer tissues

Real-time RT-PCR showed overexpression of the nucleic acid sequence according to SEQ ID NO:675 in lung cancer, and malignant melanomas.

### Fig. 41. Quantitative expression of SEQ ID NO:679 in normal tissues and cancer tissues

Real-time RT-PCR showed overexpression of the nucleic acid sequence according to SEQ ID NO:679 in lung cancer.

### Fig. 42. Quantitative expression of SEQ ID NO:682 in normal tissues and cancer tissues

Real-time RT-PCR showed overexpression of the nucleic acid sequence according to SEQ ID N0:682 in gastric cancer, breast cancer, and malignant melanomas.

### Fig. 43. Quantitative expression of SEQ ID NO:686 in normal tissues and cancer tissues

Real-time RT-PCR showed overexpression of the nucleic acid sequence according to SEQ ID NO:686 in breast cancer, ovarian cancer, and lung cancer.

### Examples:

The techniques and methods mentioned herein are carried out in a manner known per se and are described, for example, in Sambrook et al., Molecular Cloning: A Laboratory Manual, 2nd Edition (1989) Cold Spring Harbor Laboratory Press, Cold Spring Harbor, N.Y. All methods including the use of kits and reagents are carried out according to the manufacturers' information unless specifically indicated.

### Example 1: Screening for placenta-specific genes aberrantly activated in tumors

### Tissues and cell lines

Tissues were obtained as human surplus materials during routine diagnostic or therapeutic procedures and were stored at -80°C until use. Cell lines were purchased from the *American Type Culture Collection* (ATCC) and the *German Resource Collection of Microorganisms and Cell Culture* (DSMZ).

### RNA isolation and microarray hybridization

Total RNA was isolated using the RNeasy Mini Kit protocol (Qiagen). Quantification of isolated RNA was performed using UV-spectroscopy and the quality was determined both by A₂₆₀/A₂₈₀ ratio and Agilent bioanalyzer (Agilent Technologies). Five micrograms total RNA were used for cDNA synthesis with 5 pmol µl⁻¹ T7-oligo(dT)₂₄ primer and was performed at 43°C for 90 minutes with the "Superscript First-Strand Synthesis-System" for RT-PCR (Invitrogen). Second-strand synthesis was performed with complete cDNA. The cDNA solution was incubated at 16°C for 2 hours followed by an incubation step for 20 min with 6 U T4-DNA polymerase at 16°C and the reaction was stopped using 10 µl of 0.5 M EDTA. After purification of the double stranded cDNA using the GeneChip Sample Cleanup Module (Affymetrix) labeled cRNA was generated from the cDNA sample by an *in vitro* transcription reaction that was supplemented with biotin-11-CTP and biotin-16-UTP (Enzo Diagnostics) according to the manufacturer's instructions. The cRNA was quantified by A₂₆₀, and the quality was determined using the labchip bioanalyzer (Agilent). Only cRNA specimens with a high quality were selected for further analyses. Fragmented cRNA (15 µg) was used to prepare 300 µl hybridization cocktail (100 mM MES, 1 M NaCl, 20 mM EDTA, 0.01% Tween-20) containing 0.1 mg ml⁻¹ of herring sperm DNA, and 0.5 mg ml⁻¹ acetylated bovine serum albumine. Control cRNA was used in order to compare hybridization efficiencies between arrays and to standardize the quantification of measured transcript levels and was included as component of the 'Eukaryotic Hybridization Control kit' (Affymetrix, Santa Clara, CA, USA). The cocktails were heated to 95°C for 5 minutes, equilibrated at 45°C for 5 minutes, and clarified by centrifugation. The cocktail was hybridized to HG U133 Plus 2.0 arrays (Affymetrix) at 45°C for 16 hours. The arrays were washed and stained with a streptavidin-conjugated fluor using the GeneChip fluidics station protocol EukGE-WS2 (Affymetrix) according to the manufacturer's instructions. Arrays were scanned with an argon-ion laser confocal scanner (Hewlett-Packard, Santa Clara, CA) with detection at 570 nm. Data were extracted using Microarray Suite version 5.0 (Affymetrix) and linearly scaled to achieve an average intensity of 2,500 per gene. Text files were exported to determine the intensity of each interrogating oligonucleotide perfect match probe cells or mismatch probe cells. In addition, the ratios of 5'- and 3'-ends of mRNA were analyzed of six randomly selected specimens (two of each group) using microarray test-chips (Test3 Array) containing 24 human housekeeping/maintenance genes (Affymetrix) and RNA degradation was not observed.

### Bioinformatic analysis

The GeneChip^{®} Operating Software 1.4 (Affymetrix) and ArrayAssist software package 5.2 (Stratagene) were used for statistical analyses.

### Results

Screening of samples from the 18 normal tissues shown below in table 1 and 30 tumor cell lines of different entities shown below in table 2 resulted in the sequences decribed herein which are expressed in placenta among the normal tissues investigated and in tumor cell lines.

**Tab. 1 Tissues used for microarray expression analysis**

| **Tissue** | **Number** |
|---|---|
| Placenta | 2 |
| Testis | 2 |
| Mammary gland | 2 |
| Thymus | 2 |
| Skin | 2 |
| Liver | 2 |
| Colon | 2 |
| Esophagus | 2 |
| Stomach | 2 |
| Lung | 2 |
| Kidney | 2 |
| Lymph node | 2 |
| Skeletal muscle | 2 |
| Myocard | 1 |
| Brain | 1 |
| Cerebellum | 1 |
| resting PBMCs | 2 |
| activ. PBMCs | 2 |

**Tab. 2 Cell lines used for microarray expression analysis**

| **Cell line** | **Tissue** |
|---|---|
| BT-549 | Breast cancer |
| MDA-MB-231 metastasizing | Breast cancer |
| MDA-MB-231 non-metastatsizing | Breast cancer |
| MDA-MB-435S | Breast cancer |
| MDA-MB-468 | Breast cancer |
| SK-BR-3 | Breast cancer |
| Caov-3 | Ovarian cancer |
| FU-OV | Ovarian cancer |
| NIH-OVCAR-3 | Ovarian cancer |
| COLO-205 | Colorectal cancer |
| HCT-116 | Colorectal cancer |
| HCT-116 DKO | Colorectal cancer |
| HCT-15 | Colorectal cancer |
| HT-29 | Colorectal cancer |
| LOVO | Colorectal cancer |
| SW-480 | Colorectal cancer |
| CPC-N | Lung cancer |
| LOU-NH-91 | Lung cancer |
| SHP-77 | Lung cancer |
| SK-MES-1 | Lung cancer |
| NCI-H-187 | Lung cancer |
| NCI-H-209 | Lung cancer |
| NCI-H-522 | Lung cancer |
| DU-145 | Prostate cancer |
| LnCaP | Prostate cancer |
| PC-3 | Prostate cancer |
| MEL-JUSO | Melanoma |
| Murowsky | Melanoma |
| SK-MEL-37 | Melanoma |
| HELA | Cervical cancer |

### Example 2: Validation of the identified tumor-associated markers

### 1. Examination of RNA expression

The identified tumor-associated markers are first validated with the aid of RNA which is obtained from various tissues or from tissue-specific cell lines. Since the differential expression pattern of healthy tissue in comparison with tumor tissue is of decisive importance for the subsequent therapeutic application, the target genes are preferably characterized with the aid of these tissue samples.

Total RNA is isolated from native tissue samples or from tumor cell lines by standard methods of molecular biology. Said isolation may be carried out, for example, with the aid of the RNeasy Maxi kit (Qiagen, Cat. No. 75162) according to the manufacturer's instructions. This isolation method is based on the use of chaotropic reagent guanidinium isothiocyanate. Alternatively, acidic phenol can be used for isolation (Chomczynski & Sacchi, Anal. Biochem. 162: 156-159, 1987). After the tissue has been worked up by means of guanidinium isothiocyanate, RNA is extracted with acidic phenol, subsequently precipitated with isopropanol and taken up in DEPC-treated water.

2-4 µg of the RNA isolated in this way are subsequently transcribed into cDNA, for example by means of Superscript II (Invitrogen) according to the manufacturer's protocol. cDNA synthesis is primed with the aid of random hexamers (e.g. Roche Diagnostics) according to standard protocols of the relevant manufacturer. For quality control, the cDNAs are amplified over 30 cycles, using primers specific for the p53 gene which is expressed only lowly. Only p53-positive cDNA samples will be used for the subsequent reaction steps.

The targets are analyzed in detail by carrying out an expression analysis by means of PCR or quantitative PCR (qPCR) on the basis of a cDNA archive which has been isolated from various normal and tumor tissues and from tumor cell lines. For this purpose, 0.5 µl of cDNA of the above reaction mixture is amplified by a DNA polymerase (e.g. 1 U of HotStarTaq DNA polymerase, Qiagen) according to the protocols of the particular manufacturer (total volume of the reaction mixture: 25-50 µl). Aside from said polymerase, the amplification mixture comprises 0.3 mM dNTPs, reaction buffer (final concentration 1 x, depending on the manufacturer of the DNA polymerase) and in each case 0.3 mM gene-specific "sense" and "antisense" primers.

The specific primers of the target gene are, as far as possible, selected in such a way that they are located in two different exons so that genomic contaminations do not lead to false-positive results. In a non-quantitative end point PCR, the cDNA is typically incubated at 95°C for 15 minutes in order to denature the DNA and to activate the Hot-Start enzyme. Subsequently the DNA is amplified over 35 cycles (1 min at 95°C, 1 min at the primer-specific hybridization temperature (approx. 55-65°C), 1 min at 72°C to elongate the amplicons). Subsequently, 10 µl of the PCR mixture are applied to agarose gels and fractionated in the electric field. The DNA is made visible in the gels by staining with ethidium bromide and the PCR result is documented by way of a photograph.

As an alternative to conventional PCR, expression of a target gene may also be analyzed by quantitative real time PCR. Meanwhile various analytical systems are available for this analysis, of which the best known ones are the ABI PRISM sequence detection system (TaqMan, Applied Biosystems), the iCycler (Biorad) and the Light cycler (Roche Diagnostics). As described above, a specific PCR mixture is subjected to a run in the real time instruments. By adding a DNA-intercalating dye (e.g. ethidium bromide, CybrGreen), the newly synthesized DNA is made visible by specific light excitation (according to the dye manufacturers' information). A multiplicity of points measured during amplification enables the entire process to be monitored and the nucleic acid concentration of the target gene to be determined quantitatively. The PCR mixture is normalized by measuring a housekeeping gene (e.g. 18S RNA, β-actin). Alternative strategies via fluorescently labeled DNA probes likewise allow quantitative determination of the target gene of a specific tissue sample (see TaqMan applications from Applied Biosystems).

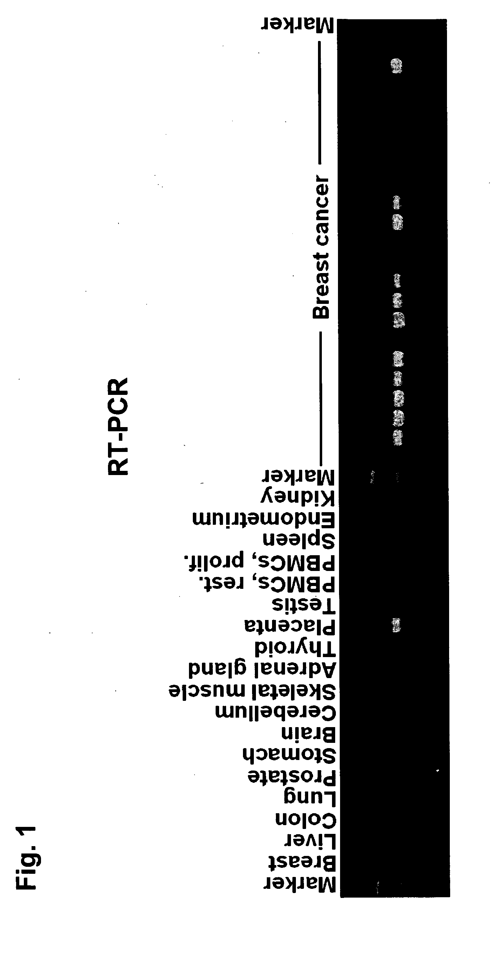

As shown in Fig. 1, placenta was confirmed in RT-PCR analyses as the only healthy tissue expressing the nucleic acid sequence according to SEQ ID NO:540. No significant expression was found in any other normal tissue. However, high and significant levels of expression were found in breast cancer.

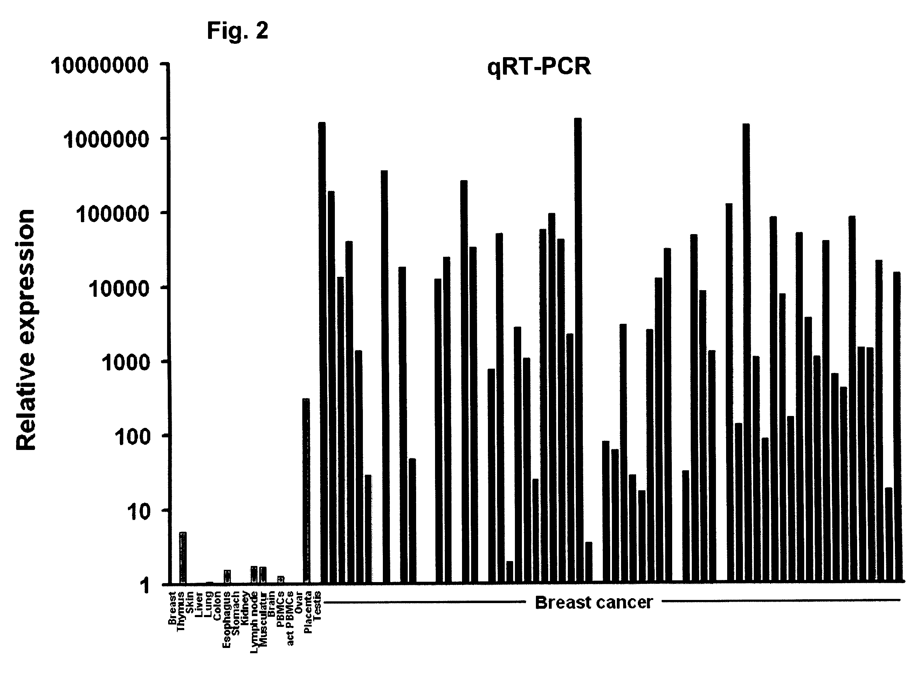

Quantitative real-time RT-PCR analyses revealed that the nucleic acid sequence according to SEQ ID NO:540 was expressed in significant levels in the majority of breast cancer samples analyzed; cf. Fig. 2.

### 2. Cloning

The complete target gene which is required for further characterization of the tumor-associated marker is cloned according to common molecular-biological methods (e.g. in "Current Protocols in Molecular Biology", John Wiley & Sons Ltd., Wiley InterScience). In order to clone the target gene or to analyze its sequence, said gene is first amplified by a DNA polymerase having a proof reading function (e.g. pfu, Roche Diagnostics). The amplicon is then ligated by standard methods into a cloning vector. Positive clones are identified by sequence analysis and subsequently characterized with the aid of prediction programs and known algorithms.

### 3. Prediction of the protein

Genes found according to the invention (in particular those from the RefSeq XM domain) may require cloning of the full-length gene, determination of the open reading frame and deduction and analysis of the protein sequence.

In order to clone the full-length sequence, common protocols for the rapid amplification of cDNA ends and the screening of cDNA expression libraries with gene-specific probes may be used (Sambrook et al., Molecular Cloning: A Laboratory Manual, 2nd edition (1989), Cold Spring Harbor Laboratory Press, Cold Spring Harbor, N. Y*.*).
After assembling the fragments found in this way, potential open reading frames (ORF) can be predicted using common prediction programs. Since the position of the PolyA tail and of polyadenylation motifs predetermines the orientation of the potential gene product, only the 3 reading frames of that particular orientation remain out of a possible 6 reading frames. The former often yield only one sufficiently large open reading frame which may code for a protein, while the other reading frames have too many stop codons and would not code for any realistic protein. In the case of alternative open reading frames, identification of the authentic ORF is assisted by taking into account the Kozak criteria for optimal transcription initiation and by analyzing the deduced protein sequences which may arise. Said ORF is further verified by generating immune sera against proteins deduced from the potential ORFs and analyzing said immune sera for recognition of a real protein in tissues and cell lines.

### 4. Production of antibodies

The tumor-associated antigens identified according to the invention are characterized, for example, by using antibodies. The invention further comprises the diagnostic or therapeutic use of antibodies. Antibodies may recognize proteins in the native and/or denatured state (Anderson et al., J. Immunol, 143: 1899-1904, 1989; Gardsvoll, J. Immunol. Methods 234: 107-116, 2000; Kayyem et al., Eur. J. Biochem. 208: 1-8, 1992; Spiller et al., J. Immunol. Methods 224: 51-60, 1999).

Antisera comprising specific antibodies which specifically bind to the target protein may be prepared by various standard methods; cf., for example, "Monoclonal Antibodies: A Practical Approach" by Phillip Shepherd, Christopher Dean ISBN 0-19-963722-9, "Antibodies: A Laboratory Manual" by Ed Harlow, David Lane ISBN: 0879693142 and "Using Antibodies: A Laboratory Manual: Portable Protocol NO" by Edward Harlow, David Lane, Ed Harlow ISBN: 0879695447. It is also possible here to generate affine and specific antibodies which recognize complex membrane proteins in their native form (Azorsa et al., J. Immunol. Methods 229: 35-48, 1999; Anderson et al., J. Immunol. 143: 1899-1904, 1989; Gardsvoll, J. Immunol. Methods. 234: 107-116, 2000). This is especially important in the preparation of antibodies which are intended to be used therapeutically but also for many diagnostic applications. For this purpose, both the complete protein and extracellular partial sequences may be used for immunization.

### Immunization and production of polyclonal antibodies

Various immunization protocols are published. A species (e.g. rabbits, mice) is immunized by a first injection of the desired target protein. The immune response of the animal to the immunogen can be enhanced by a second or third immunization within a defined period of time (approx. 2-4 weeks after the previous immunization). Blood is taken from said animals and immune sera obtained, again after various defined time intervals (1st bleeding after 4 weeks, then every 2-3 weeks, up to 5 takings). The immune sera taken in this way comprise polyclonal antibodies which may be used to detect and characterize the target protein in Western blotting, by flow cytometry, immunofluorescence or immunohistochemistry.

The animals are usually immunized by any of four well-established methods, with other methods also in existence. The immunization may be carried out using peptides specific for the target protein, using the complete protein, or using extracellular partial sequences of a protein which can be identified experimentally or via prediction programs. Since the prediction programs do not always work perfectly, it is also possible to employ two domains separated from one another by a transmembrane domain. In this case, one of the two domains has to be extracellular, which may then be proved experimentally (see below). Immunization is offered commercially by different service providers.
(1) In the first case, peptides (length: 8-12 amino acids) are synthesized by *in vitro* methods (possibly carried out by a commercial service), and said peptides are used for immunization. Normally 3 immunizations are carried out (e.g. with a concentration of 5-100 µg/immunization).
(2) Alternatively, immunization may be carried out using recombinant proteins. For this purpose, the cloned DNA of the target gene is cloned into an expression vector and the target protein is synthesized, for example, cell-free *in vitro,* in bacteria (e.g. *E. coli*), in yeast (e.g. *S. pombe*), in insect cells or in mammalian cells, according to the conditions of the particular manufacturer (e.g. Roche Diagnostics, Invitrogen, Clontech, Qiagen). It is also possible to synthesize the target protein with the aid of viral expression systems (e.g. baculovirus, vacciniavirus, adenovirus). After it has been synthesized in one of said systems, the target protein is purified, normally by employing chromatographic methods. In this context, it is also possible to use for immunization proteins which have a molecular anchor as an aid for purification (e.g. His tag, Qiagen; FLAG tag, Roche Diagnostics; GST fusion proteins). A multiplicity of protocols can be found, for example, in "Current Protocols in Molecular Biology", John Wiley & Sons Ltd., Wiley InterScience. After the target protein has been purified, an immunization is carried out as described above.
(3) If a cell line is available which synthesizes the desired protein endogenously, it is also possible to use this cell line directly for preparing the specific antiserum. In this case, immunization is carried out by 1-3 injections with in each case approx. 1-5 x 10⁷ cells.
(4) The immunization may also be carried out by injecting DNA (DNA immunization). For this purpose, the target gene is first cloned into an expression vector so that the target sequence is under the control of a strong eukaryotic promoter (e.g. CMV promoter). Subsequently, DNA (e.g. 1-10 µg per injection) is transferred as immunogen using a gene gun into capillary regions with a strong blood flow in an organism (e.g. mouse, rabbit). The transferred DNA is taken up by the animal's cells, the target gene is expressed, and the animal finally develops an immune response to the target protein (Jung et al., Mol. Cells 12: 41-49, 2001; Kasinrerk et al., Hybrid Hybridomics 21: 287-293,2002).

### Production of monoclonal antibodies

Monoclonal antibodies are traditionally produced with the aid of the hybridoma technology (technical details: see "Monoclonal Antibodies: A Practical Approach" by Philip Shepherd, Christopher Dean ISBN 0-19-963722-9; "Antibodies: A Laboratory Manual" by Ed Harlow, David Lane ISBN: 0879693142, "Using Antibodies: A Laboratory Manual: Portable Protocol NO" by Edward Harlow, David Lane, Ed Harlow ISBN: 0879695447). A new method which is also used is the "SLAM" technology. Here, B cells are isolated from whole blood and the cells are made monoclonal. Subsequently the supernatant of the isolated B cell is analyzed for its antibody specificity. In contrast to the hybridoma technology, the variable region of the antibody gene is then amplified by single-cell PCR and cloned into a suitable vector. In this manner production of monoclonal antibodies is accelerated (de Wildt et al., J. Immunol. Methods 207:61-67, 1997).

### 5. Validation of the targets by protein-chemical methods using antibodies

The antibodies which can be produced as described above can be used to further analyse the target protein as follows:

### Specificity of the antibody

Assays based on cell culture with subsequent Western blotting are most suitable for demonstrating the fact that an antibody binds specifically only to the desired target protein (various variations are described, for example, in "Current Protocols in Proteinchemistry", John Wiley & Sons Ltd., Wiley InterScience). For the demonstration, cells are transfected with a cDNA for the target protein, which is under the control of a strong eukaryotic promoter (e.g. cytomegalovirus promoter; CMV). A wide variety of methods (e.g. electroporation, liposome-based transfection, calcium phosphate precipitation) are well established for transfecting cell lines with DNA (e.g. Lemoine et al., Methods Mol. Biol. 75: 441-7, 1997). As an alternative, it is also possible to use cell lines which express the target gene endogenously (detection via target gene-specific RT-PCR). As a control, in the ideal case, homologous genes are cotransfected in the experiment, in order to be able to demonstrate in the following Western blot the specificity of the analyzed antibody.

In the subsequent Western blotting, cells from cell culture or tissue samples which might contain the target protein are lysed in a 1% strength SDS solution, and the proteins are denatured in the process. The lysates are fractionated according to size by electrophoresis on 8-15% strength denaturing polyacrylamide gels (contain 1% SDS) (SDS polyacrylamide gel electrophoresis, SDS-PAGE). The proteins are then transferred by one of a plurality of blotting methods (e.g. semi-dry electroblot; Biorad) to a specific membrane (e.g. nitrocellulose, Schleicher & Schüll). The desired protein can be visualized on this membrane. For this purpose, the membrane is first incubated with the antibody which recognizes the target protein (dilution approx. 1:20-1:200, depending on the specificity of said antibody), for 60 minutes. After a washing step, the membrane is incubated with a second antibody which is coupled to a marker (e.g. enzymes such as peroxidase or alkaline phosphatase) and which recognizes the first antibody. It is then possible to make the target protein visible on the membrane in a color or chemiluminescent reaction (e.g. ECL, Amersham Bioscience). An antibody with a high specificity for the target protein should in the ideal case only recognise the desired protein itself.

### Localization of the target protein

Various methods are used to confirm the membrane localization, identified in the in silico approach, of the target protein. An important and well-established method using the antibodies described above is immunofluorescence (IF). For this purpose, cells of established cell lines which either synthesize the target protein (detection of the RNA by RT-PCR or of the protein by Western blotting) or else have been transfected with plasmid DNA are utilized. A wide variety of methods (e.g. electroporation, liposome-based transfection, calcium phosphate precipitation) are well established for transfection of cell lines with DNA (e.g. Lemoine et al., Methods Mol. Biol. 75: 441-7, 1997). The plasmid transfected into the cells, in immunofluorescence, may encode the unmodified protein or else couple different amino acid markers to the target protein. The principle markers are, for example, the fluorescent green fluorescent protein (GFP) in various differentially fluorescent forms, short peptide sequences of 6-12 amino acids for which high-affinity and specific antibodies are available, or the short amino acid sequence Cys-Cys-X-X-Cys-Cys which can bind via its cysteines specific fluorescent substances (Invitrogen). Cells which synthesize the target protein are fixed, for example, with paraformaldehyde or methanol. The cells may then, if required, be permeabilized by incubation with detergents (e.g. 0.2% Triton X-100). The cells are then incubated with a primary antibody which is directed against the target protein or against one of the coupled markers. After a washing step, the mixture is incubated with a second antibody coupled to a fluorescent marker (e.g. fluorescein, Texas Red, Dako), which binds to the first antibody. The cells labeled in this way are then overlaid with glycerol and analyzed with the aid of a fluorescence microscope according to the manufacturer's information. Specific fluorescence emissions are achieved in this case by specific excitation depending on the substances employed. The analysis usually permits reliable localization of the target protein, the antibody quality and the target protein being confirmed in double stainings with, in addition to the target protein, also the coupled amino acid markers or other marker proteins whose localization has already been described in the literature being stained. GFP and its derivatives represent a special case, being excitable directly and themselves fluorescing. The membrane permeability which may be controlled through the use of detergents, in immunofluorescence, allows demonstration of whether an immunogenic epitope is located inside or outside the cell. The prediction of the selected proteins can thus be supported experimentally. An alternative possibility is to detect extracellular domains by means of flow cytometry. For this purpose, cells are fixed under non-permeabilizing conditions (e.g. with PBS/Na azide/2% FCS/5 mM EDTA) and analyzed in a flow cytometer in accordance with the manufacturer's instructions. Only extracellular epitopes can be recognized by the antibody to be analyzed in this method. A difference from immunofluorescence is that it is possible to distinguish between dead and living cells by using, for example, propidium iodide or trypan blue, and thus avoid false-positive results.

Another important detection is by immunohistochemistry (IHC) on specific tissue samples. The aim of this method is to identify the localization of a protein in a functionally intact tissue aggregate. IHC serves specifically for (1) being able to estimate the amount of target protein in tumor and normal tissues, (2) analyzing how many cells in tumor and healthy tissues synthesize the target gene, and (3) defining the cell type in a tissue (tumor, healthy cells) in which the target protein is detectable. Alternatively, the amounts of protein of a target gene may be quantified by tissue immunofluorescence using a digital camera and suitable software (e.g. Tillvision, Till-photonics, Germany). The technology has frequently been published, and details of staining and microscopy can therefore be found, for example, in "Diagnostic Immunohistochemistry" by David J., MD Dabbs ISBN: 0443065667 or in "Microscopy, Immunohistochemistry, and Antigen Retrieval Methods: For Light and Electron Microscopy" ISBN: 0306467704. It should be noted that, owing to the properties of antibodies, different protocols have to be used (an example is described below) in order to obtain a meaningful result:

Normally, histologically defined tumor tissues and, as reference, comparable healthy tissues are employed in IHC. It is also possible to use as positive and negative controls cell lines in which the presence of the target gene is known through RT-PCR analyses. A background control must always be included.

Formalin-fixed (another fixation method, for example with methanol, is also possible) and paraffin-embedded tissue pieces with a thickness of 4 µm are applied to a glass support and deparaffinated with xylene, for example. The samples are washed with TBS-T and blocked in serum. This is followed by incubation with the first antibody (dilution: 1:2 to 1:2000) for 1-18 hours, with affinity-purified antibodies normally being used. A washing step is followed by incubation with a second antibody which is coupled to an alkaline phosphatase (alternative: for example peroxidase) and directed against the first antibody, for approx. 30-60 minutes. This is followed by a color reaction using alkaline phosphatase (cf., for example, Shi et al., J. Histochem. Cytochem. 39: 741-748, 1991; Shin et al., Lab. Invest. 64: 693-702, 1991). To demonstrate antibody specificity, the reaction can be blocked by previous addition of the immunogen.

### Analysis of protein modifications

Secondary protein modifications such as, for example, N- or O-glycosylations or myristilations may impair or even completely prevent the accessibility of immunogenic epitopes and thus call into question the efficacy of antibody therapies. Moreover, it has frequently been demonstrated that the type and amount of secondary modifications differ in normal and tumor tissues (e.g. Durand & Seta, 2000; Clin. Chem. 46: 795-805; Hakomori, 1996; Cancer Res. 56: 5309-18). The analysis of these modifications is therefore essential to the therapeutic success of an antibody. Potential binding sites can be predicted by specific algorithms.

Analysis of protein modifications usually takes place by Western blotting (see above). Glycosylations which usually have a size of several kDa, especially lead to a larger total mass of the target protein, which can be fractionated in SDS-PAGE. To detect specific O- and N-glycosidic bonds, protein lysates are incubated prior to denaturation by SDS with O- or N-glycosylases (in accordance with their respective manufacturer's instructions, e.g. PNgase, endoglycosidase F, endoglycosidase H, Roche Diagnostics). This is followed by Western blotting as described above. Thus, if there is a reduction in the size of a target protein after incubation with a glycosidase, it is possible to detect a specific glycosylation and, in this way, also analyze the tumor specificity of a modification.

### Functional analysis of the target gene

The function of the target molecule may be crucial for its therapeutic usefulness, so that functional analyses are an important component in the characterization of therapeutically utilizable molecules. The functional analysis may take place either in cells in cell culture experiments or else in vivo with the aid of animal models. This involves either switching off the gene of the target molecule by mutation (knockout) or inserting the target sequence into the cell or the organism (knockin). Thus it is possible to analyze functional modifications in a cellular context firstly by way of the loss of function of the gene to be analyzed (loss of function). In the second case, modifications caused by addition of the analyzed gene can be analyzed (gain of function).

### a. Functional analysis in cells

Transfection. In order to analyze the gain of function, the gene of the target molecule must be transferred into the cell. For this purpose, cells which allow synthesis of the target molecule are transfected with a DNA. Normally, the gene of the target molecule here is under the control of a strong eukaryotic promoter (e.g. cytomegalovirus promoter; CMV). A wide variety of methods (e.g. electroporation, liposome-based transfection, calcium phosphate precipitation) are well established for transfecting cell lines with DNA (e.g. Lemoine et, al., Methods Mol. Biol. 75: 441-7, 1997). The gene may be synthesized either transiently, without genomic integration, or else stably, with genomic integration after selection with neomycin, for example.

RNA interference (siRNA). An inhibition of expression of the target gene, which may induce a complete loss of function of the target molecule in cells, may be generated by the RNA interference (siRNA) technology in cells (Hannon, GJ. 2002. RNA interference. Nature 418: 244-51; Czauderna et al. 2003. Nucl. Acid Res. 31: 670-82). For this purpose, cells are transfected with short, double-stranded RNA molecules of approx. 20-25 nucleotides in length, which are specific for the target molecule. An enzymic process then results in degradation of the specific RNA of the target gene and thus in reduced expression of the target protein and consequently enables the target gene to be functionally analyzed.

Cell lines which have been modified by means of transfection or siRNA may subsequently be analyzed in different ways. The most common examples are listed below.

### 1. Proliferation and cell cycle behavior

A multiplicity of methods for analyzing cell proliferation are established and are commercially supplied by various companies (e.g. Roche Diagnostics, Invitrogen; details of the assay methods are described in the numerous application protocols). The number of cells in cell culture experiments can be determined by simple counting or by colorimetric assays which measure the metabolic activity of the cells (e.g. wst-1, Roche Diagnostics). Metabolic assay methods measure the number of cells in an experiment indirectly via enzymic markers. Cell proliferation may be measured directly by analyzing the rate of DNA synthesis, for example by adding bromodeoxyuridine (BrdU), with the integrated BrdU being detected colorimetrically via specific antibodies.

### 2. Apoptosis and cytotoxicity

A large number of assay systems for detecting cellular apoptosis and cytotoxicity are available. A decisive characteristic is the specific, enzyme-dependent fragmentation of genomic DNA, which is irreversible and in any case results in death of the cell. Methods for detecting these specific DNA fragments are commercially obtainable. An additional method available is the TUNEL assay which can detect DNA single-strand breaks also in tissue sections. Cytotoxicity is mainly detected via an altered cell permeability which serves as marker of the vitality state of cells. This involves on the one hand the analysis of markers which can typically be found intracellularly in the cell culture supernatant. On the other hand, it is also possible to analyze the absorbability of dye markers which are not absorbed by intact cells. The best-known examples of dye markers are Trypan blue and propidium iodide, a common intracellular marker is lactate dehydrogenase which can be detected enzymatically in the supernatant. Different assay systems of various commercial suppliers (e.g. Roche Diagnostics, Invitrogen) are available.

### 3. Migration assay

The ability of cells to migrate is analyzed in a specific migration assay, preferably with the aid of a Boyden chamber (Corning Costar) (Cinamon G., Alon R. J. Immunol. Methods. 2003 Feb; 273(1-2):53-62; Stockton et al. 2001. Mol. Biol. Cell. 12: 1937-56). For this purpose, cells are cultured on a filter with a specific pore size. Cells which can migrate are capable of migrating through this filter into another culture vessel below. Subsequent microscopic analysis then permits determination of a possibly altered migration behavior induced by the gain of function or loss of function of the target molecule.

### b. Functional analysis in animal models

A possible alternative of cell culture experiments for the analysis of target gene function are complicated in vivo experiments in animal models. Compared to the cell-based methods, these models have the advantage of being able to detect faulty developments or diseases which are detectable only in the context of the whole organism. A multiplicity of models for human disorders are available by now (Abate-Shen & Shen. 2002. Trends in Genetics S1-5; Matsusue et al. 2003. J. Clin. Invest. 111:737-47). Various animal models such as, for example, yeast, nematodes or zebra fish have since been characterized intensively. However, models which are preferred over other species are mammalian animal models such as, for example, mice (Mus musculus) because they offer the best possibility of reproducing the biological processes in a human context. For mice, on the one hand transgenic methods which integrate new genes into the mouse genome have been established in recent years (gain of function; Jegstrup I. et al. 2003. Lab Anim. 2003 Jan.; 37(1):1-9). On the other hand, other methodical approaches switch off genes in the mouse genome and thus induce a loss of function of a desired gene (knockout models, loss of function; Zambrowicz BP & Sands AT. 2003. Nat. Rev. Drug Discov. 2003 Jan; 2(1):38-51; Niwa H. 2001. Cell Struct. Funct. 2001 Jun; 26(3):137-48); technical details have been published in large numbers.

After the mouse models have been generated, alterations induced by the transgene or by the loss of function of a gene can be analyzed in the context of the whole organism (Balling R, 2001. Ann. Rev. Genomics Hum. Genet. 2:463-92). Thus it is possible to carry out, for example, behavior tests as well as to biochemically study established blood parameters. Histological analyses, immunohistochemistry or electron microscopy enable alterations to be characterized at the cellular level. The specific expression pattern of a gene can be detected by in-situ hybridization (Peters T. et al. 2003. Hum. Mol. Genet 12:2109-20).

### Example 3: Detailed analysis of the identified tumor-associated markers

### RNA-Isolation, RT-PCR and real-Time RT-PCR

RNA extraction, first-strand cDNA synthesis, RT-PCR and real-time RT-PCR was performed as previously described (Koslowski, M. et al., Cancer Res. 62, 6750-6755 (2002), Koslowski, M. et al., Cancer Res. 64, 5988-5993 (2004)). Real-time quantitative expression analysis was performed in a 40 cycle RT-PCR. After normalization to HPRT (sense 5'-TGA CAC TGG CAA AAC AAT GCA-3'; antisense 5'-GGT CCT TTT CAC CAG CAA GCT-3', 62°C annealing) gene-specific transcripts in tumor samples were quantified relative to normal tissues using ΔΔCT calculation.

### siRNA Duplexes

The SEQ ID NO:540 siRNA duplexes (Qiagen, Hilden, Germany) were directed against target sequences 5'-NNC CAC AGA AGG UAC CAG UUA-3' (siRNA#1; sense (5'-CCA CAG AAG GUA CCA GUU AUU-3'), antisense (5'-UAA CUG GUA CCU UCU GUG GUU-3') and 5'-NNC AGC AAG ACU CCC UCU AAA-3' (siRNA#2; sense (5'-CAG CAA GAC UCC CUC UAA AUU-3'), antisense (5'-UUU AGA GGG AGU CUU GCU GUU-3') of the SEQ ID NO:540 mRNA sequence.

### Cell proliferation analysis

24 h after transfection with siRNA duplexes 1x10⁴ cells were cultured for 48 h in medium supplemented with 10% FCS. Proliferation was analyzed by measuring the incorporation of BrdU into newly synthesized DNA strands using the DELFIA cell proliferation Kit (Perkin Elmer, Boston, MA) according to the manufacturer's instructions on a Wallac Victor² multi-label counter (Perkin Elmer, Boston, MA).

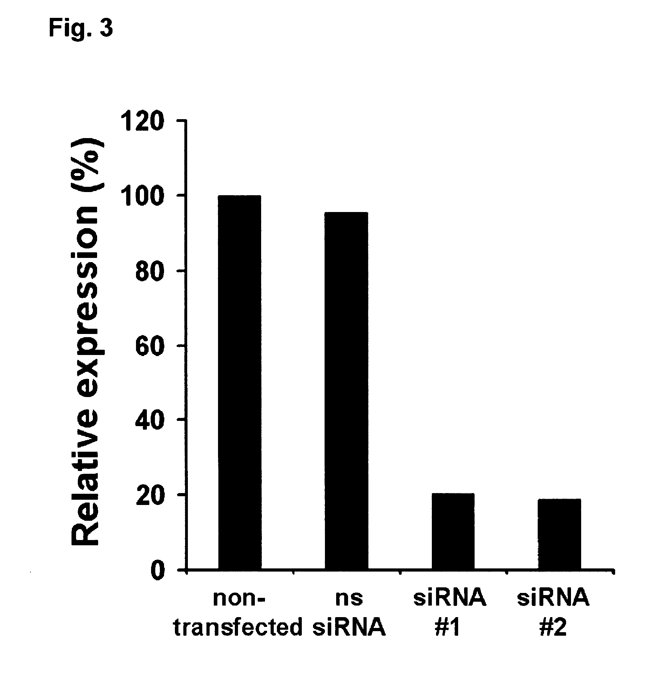

Fig. 3 shows the quantification of SEQ ID NO:540 mRNA expression in MCF-7 breast cancer cells by real-time RT-PCR 24h after transfection with siRNA oligos. Compared to non-transfected cells and cells transfected with non-silencing (ns) siRNA both SEQ ID NO:540-specific siRNAs (siRNA#1 (SEQ ID NO:630, 631), siRNA#2 (SEQ ID NO:632, 633)) induce robust silencing of SEQ ID NO:540 expression.

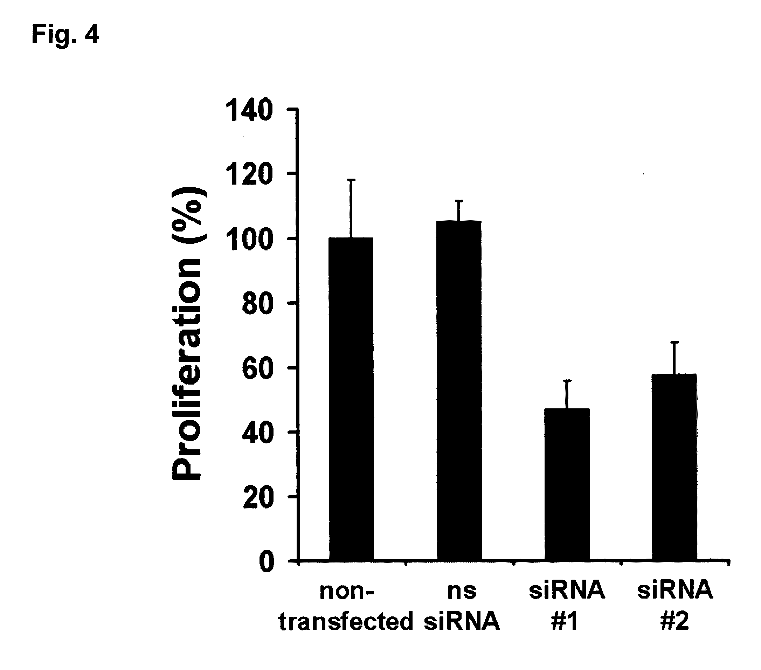

Fig. 4 shows that silencing of SEQ ID NO:540 expression by transfection with siRNA oligos results in impaired proliferation of MCF-7 breast cancer cells. Proliferation was quantified 96h after transfection with siRNAs by measuring incorporation of BrdU in newly synthesized DNA strands. These results show that SEQ ID NO:540 is a positive factor for the proliferation of breast cancer cells.

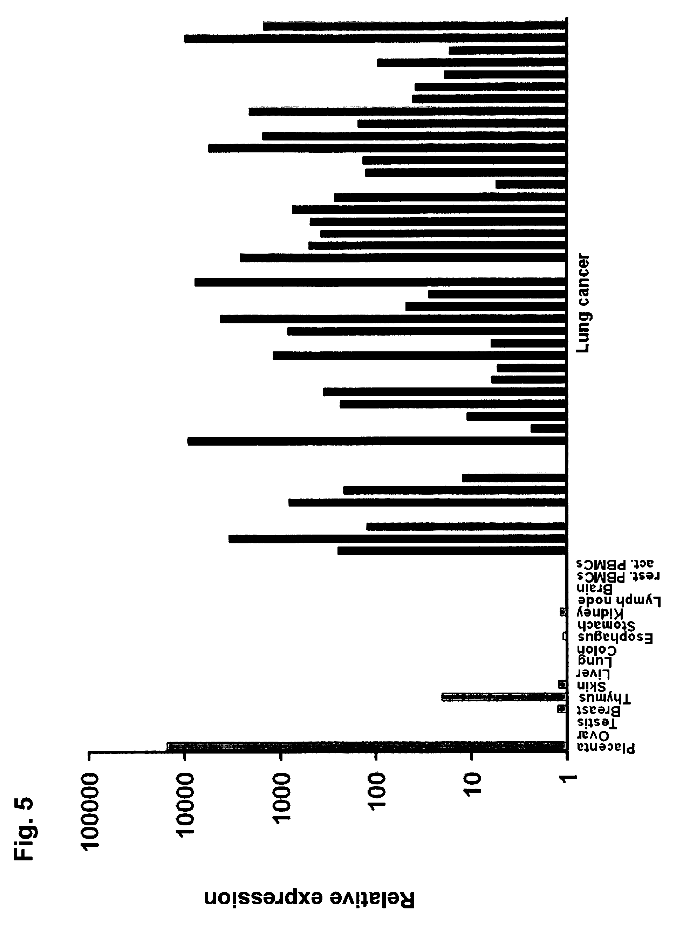

The nucleotide sequence according to SEQ ID NO:541 was deduced from SEQ ID NO:65 and codes for a 177 aa protein (SEQ ID NO:542) of unknown function. Expression of SEQ ID NO:541 in normal and cancerous tissues was quantified by real-time RT-PCR using sequence-specific oligos (SEQ ID NO:543, 544); see Fig. 5. In normal tissues SEQ ID NO:541 is highly expressed in placenta and shows only weak expression in thymus. SEQ ID NO:541 is overexpressed in lung cancer. Based on these expression results, SEQ ID NO:541 and its expression products qualify as molecular markers and/or target candidates for targeted therapies, in particular for this particular tumor type.

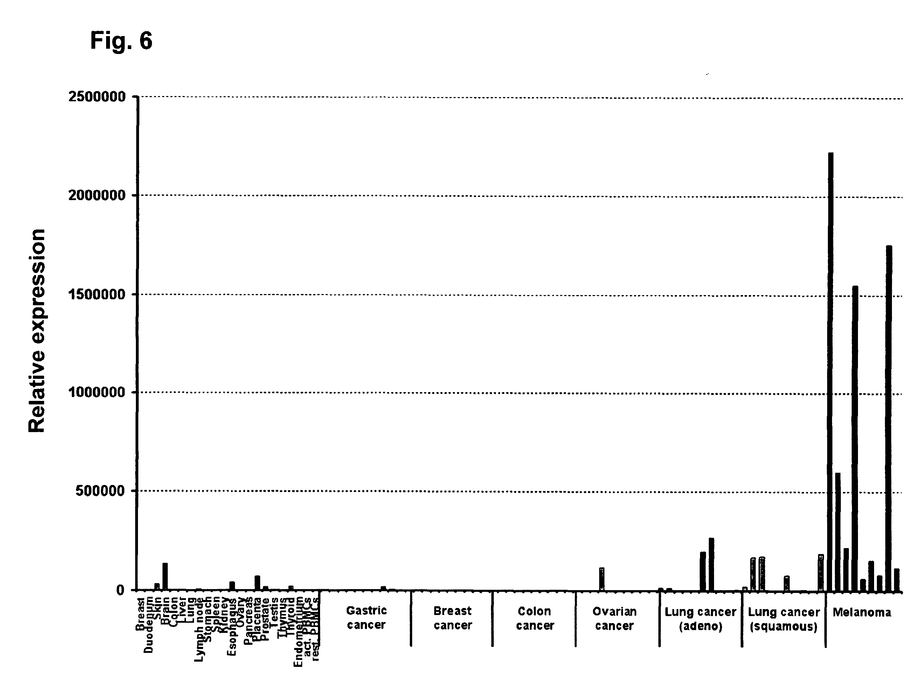

The nucleotide sequence according to SEQ ID NO:545 was deduced from SEQ ID NO:249 and codes for a member of the solute carrier (SLC) group of membrane proteins (SEQ ID NO:546). As is typical of integral membrane proteins, SLCs contain a number of hydrophobic transmembrane alpha helices connected to each other by hydrophilic intra- or extra-cellular loops. Depending on the SLC, these transporters are functional as either monomers or obligate homo- or hetero-oligomers. The protein encoded by SEQ ID NO:545 is a cell surface protein. Expression of SEQ ID NO:545 in normal and cancerous tissues was quantified by real-time RT-PCR using sequence-specific oligos (SEQ ID NO:547, 548); see Fig. 6. Compared to normal tissues, SEQ ID NO:545 is overexpressed in malignant melanomas. Based on these expression results, SEQ ID NO:545 and its expression products qualify as molecular markers and/or target candidates for targeted therapies, in particular for this particular tumor type.

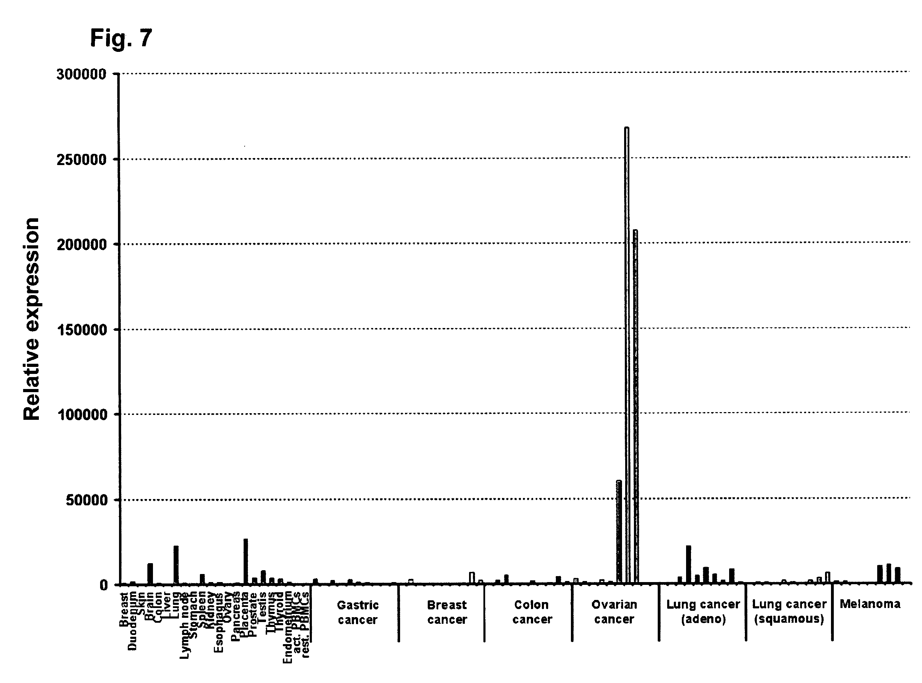

The nucleotide sequence according to SEQ ID NO:549 was deduced from SEQ ID NO:4 and codes for a 763 aa protein (SEQ ID NO:550) of unknown function. The protein harbors two potential transmembrane domains and a typical fibronectin type III domain. Fibronectin is a high-molecular-weight extracellular matrix glycoprotein that binds to membrane spanning receptor proteins (integrins). In addition to integrins, they also bind extracellular matrix components such as collagen, fibrin and heparan sulfate. The protein encoded by SEQ ID NO:549 might represent a hitherto unknown new fibronection-like protein. Expression of SEQ ID NO: 549 in normal and cancerous tissues was quantified by real-time RT-PCR using sequence-specific oligos (SEQ ID NO:551, 552); see Fig. 7. Compared to normal tissues, SEQ ID NO:549 is overexpressed in ovarian cancer. Based on these expression results, SEQ ID NO:549 and its expression products qualify as molecular markers and/or target candidates for targeted therapies, in particular of this particular tumor type.

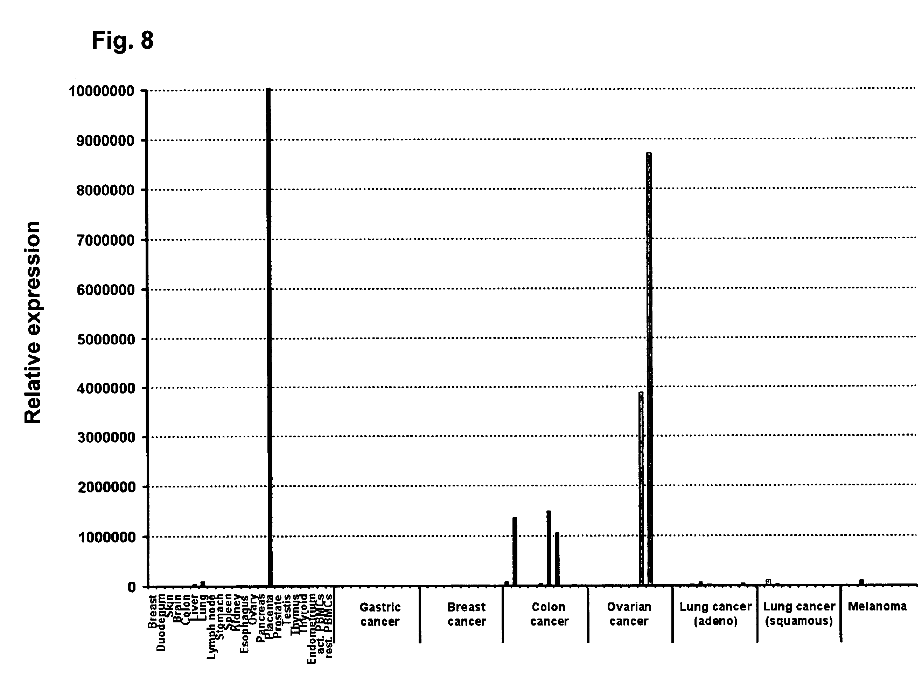

The nucleotide sequence according to SEQ ID NO:553 was deduced from SEQ ID NO: 156 and codes for a 496 aa protein (SEQ ID NO:554) of unknown function. The protein harbors a potential transmembrane protein. Expression of SEQ ID NO:553 in normal and cancerous tissues was quantified by real-time RT-PCR using sequence-specific oligos (SEQ ID NO:555, 556); see Fig. 8. In normal tissues SEQ ID NO:553 is highly expressed in placenta. Compared to other normal tissues, SEQ ID NO:553 is overexpressed in, colon cancer and ovarian cancer. Based on these expression results, SEQ ID NO:553 and its expression products qualify as molecular markers and/or target candidates for targeted therapies, in particular for these particular tumor types.

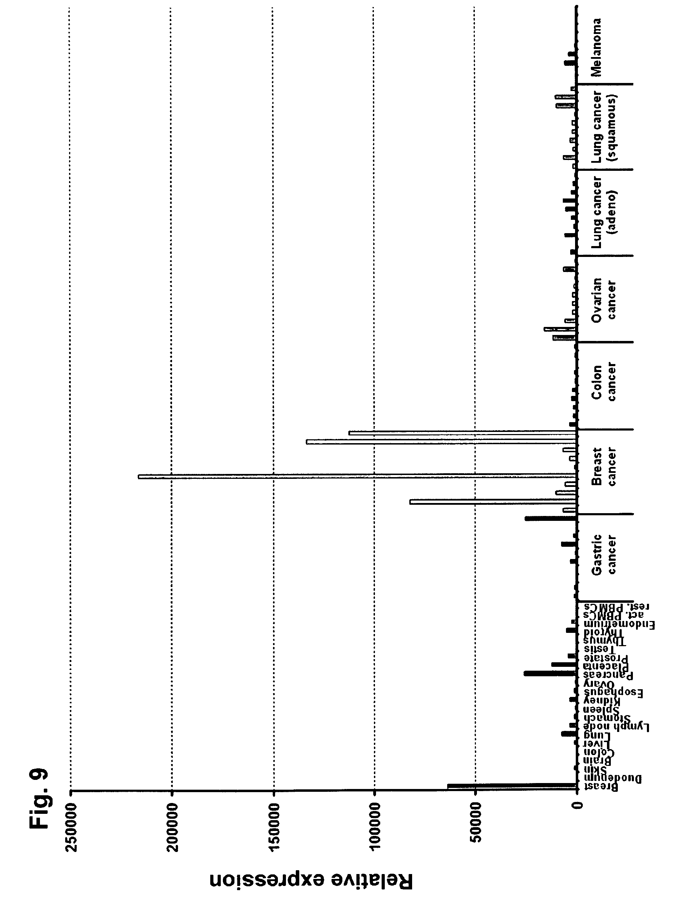

The nucleotide sequence according to SEQ ID NO:557 was deduced from SEQ ID NO:273. SEQ ID NO:557 represents a partial cDNA with no apparent open reading frame. Expression of SEQ ID NO:557 in normal and cancerous tissues was quantified by real-time RT-PCR using sequence-specific oligos (SEQ ID NO:558, 559); see Fig. 9. In normal tissues high expression of SEQ ID NO:557 is detectable in breast. Compared to normal tissues, SEQ ID NO:557 is overexpressed in breast cancer. Based on these expression results, SEQ ID NO:557 and its expression products qualify as molecular markers and/or target candidates for targeted therapies, in particular for this particular tumor type.

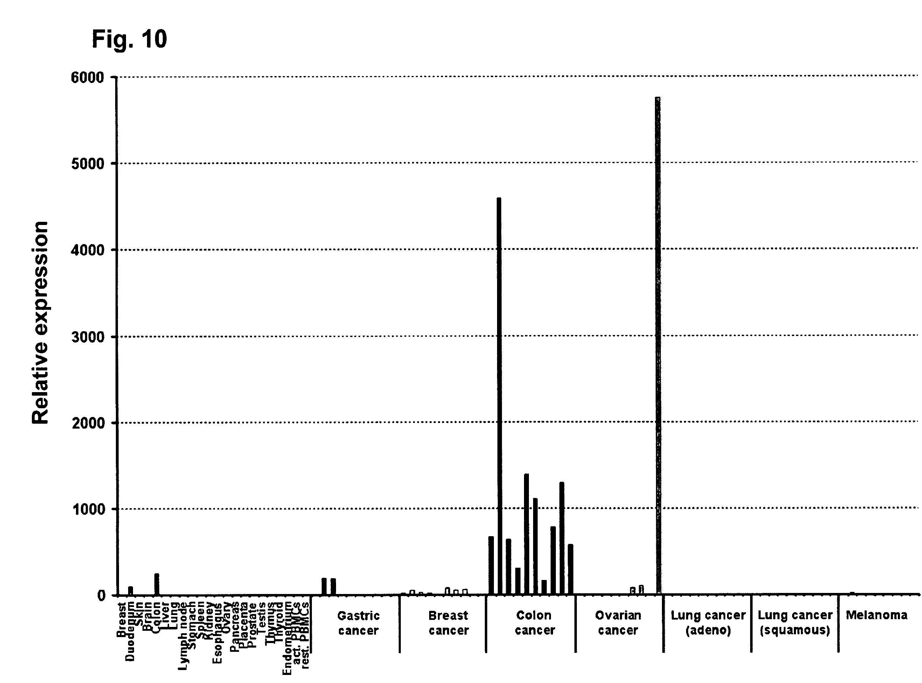

The nucleotide sequence according to SEQ ID NO:560 was deduced from SEQ ID NO: 135. SEQ ID NO:560 has no apparent open reading frame. Expression of SEQ ID NO:560 in normal and cancerous tissues was quantified by real-time RT-PCR using sequence-specific oligos (SEQ ID NO:561, 562); see Fig. 10. In normal tissues expression of SEQ ID NO:560 is detectable in duodenum and colon. Compared to normal tissues, SEQ ID NO:560 is overexpressed in colon cancer and ovarian cancer. Based on these expression results, SEQ ID NO:560 and its expression products qualify as molecular markers and/or target candidates for targeted therapies, in particular for these particular tumor types.

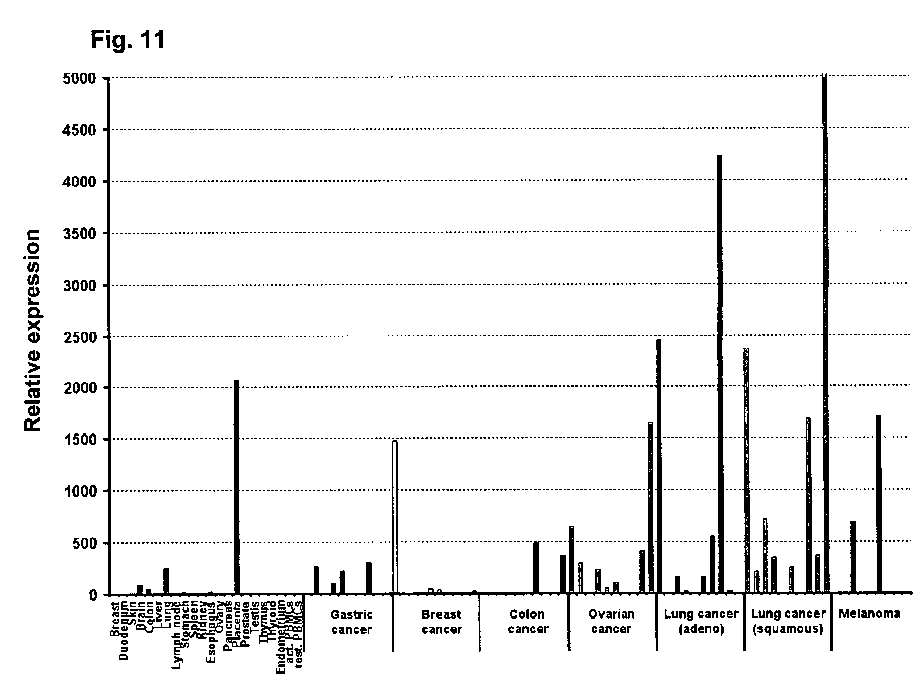

The nucleotide sequence according to SEQ ID NO:563 was deduced from SEQ ID NO:177. SEQ ID NO:563 has no apparent open reading frame. Expression of SEQ ID NO:563 in normal and cancerous tissues was quantified by real-time RT-PCR using sequence-specific oligos (SEQ ID NO:564, 565); see Fig. 11. SEQ ID NO:563 is highly expressed in placenta. Compared to normal tissues, SEQ ID NO:563 is overexpressed in breast cancer, colon cancer, ovarian cancer, lung cancer and melanoma. Based on these expression results, SEQ ID NO:563 and its expression products qualify as molecular markers and/or target candidates for targeted therapies, in particular for these particular tumor types.

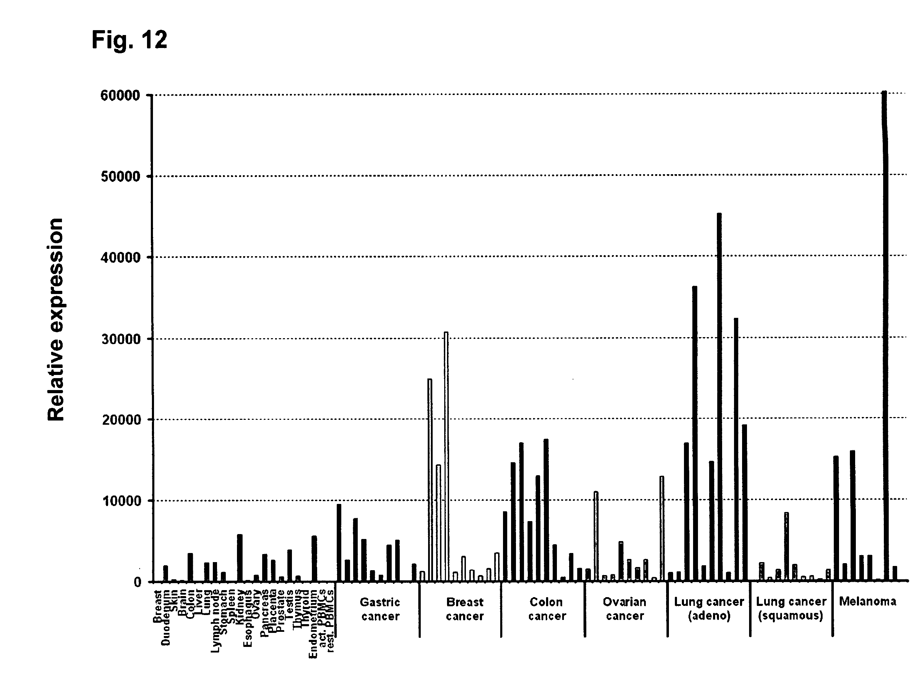

The nucleotide sequence according to SEQ ID NO:566 was deduced from SEQ ID NO:149 and codes for a 155 aa protein (SEQ ID NO:567) of unknown function. The protein sequence is partially homologous to members of the tumor necrosis factor receptor superfamily and harbors a potential transmembrane domain. The protein encoded by SEQ ID NO:566 might represent a new member of the tumor necrosis factor receptor superfamily. Expression of SEQ ID NO:566 in normal and cancerous tissues was quantified by real-time RT-PCR using sequence-specific oligos (SEQ ID NO:568, 569); see Fig. 12. Compared to normal tissues, SEQ ID NO:566 is overexpressed in gastric cancer, breast cancer, colon cancer, ovarian cancer, lung cancer and melanoma. Based on these expression results, SEQ ID NO:566 and its expression products qualify as molecular markers and/or target candidates for targeted therapies, in particular for these particular tumor types.

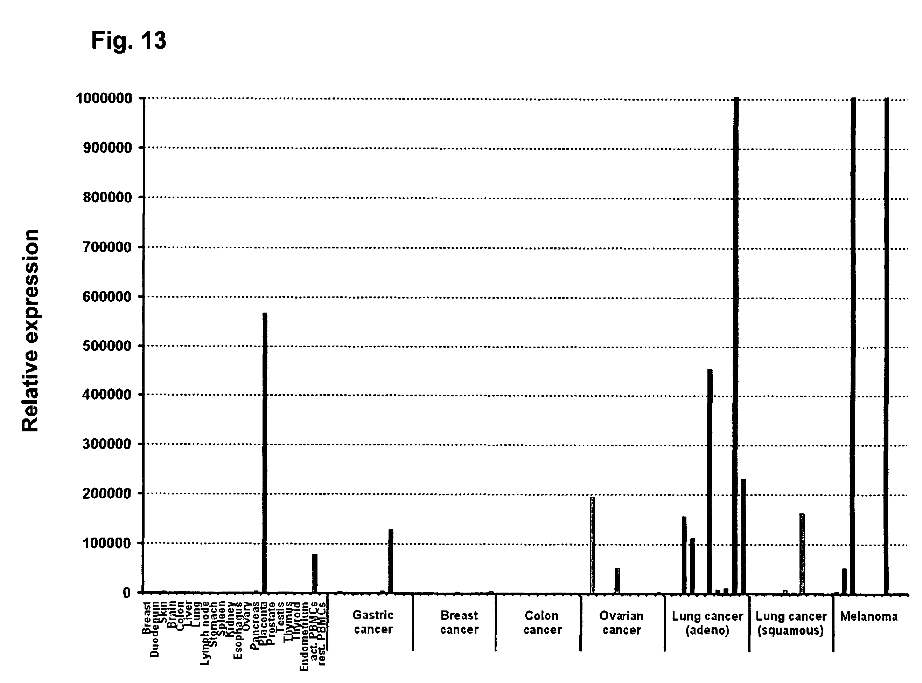

The nucleotide sequence according to SEQ ID NO:570 was deduced from SEQ ID NO:53 and codes for a member of the kernel lipocain superfamily (SEQ ID NO:571). These secreted glycoproteins have distinct and essential roles in regulating an uterine environment suitable for pregnancy and in the timing and occurrence of the appropriate sequence of events in the fertilization process. Expression of SEQ ID NO:570 in normal and cancerous tissues was quantified by real-time RT-PCR using sequence-specific oligos (SEQ ID NO:572, 573); see Fig. 13. SEQ ID NO:570 is highly expressed in placenta. Compared to other normal tissues, SEQ ID NO:570 is overexpressed in ovarian cancer, lung cancer and melanoma. Based on these expression results, SEQ ID NO:570 and its expression products qualify as molecular markers and/or target candidates for targeted therapies, in particular for these particular tumor types.

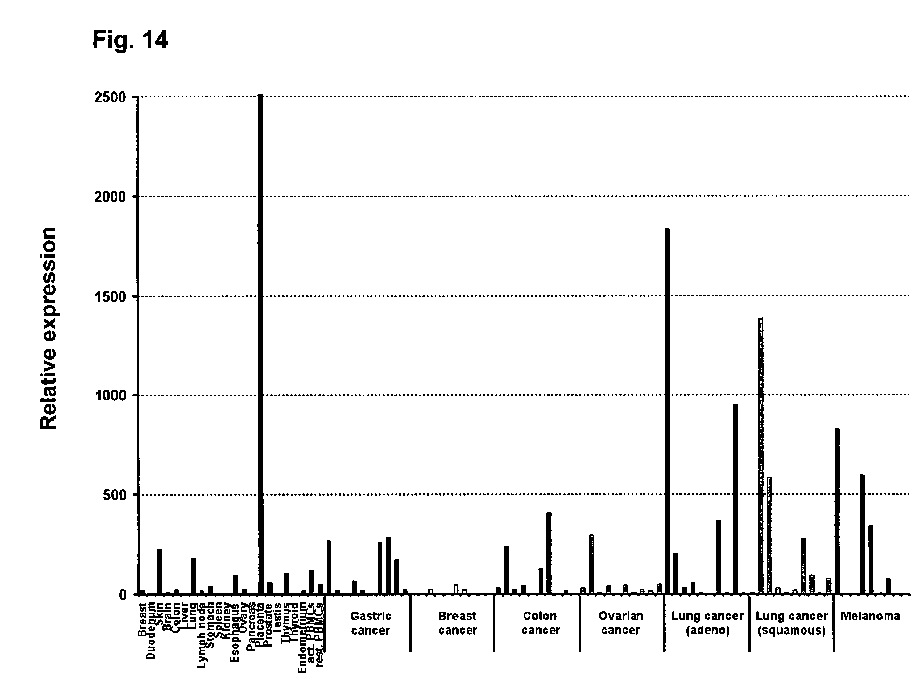

The nucleotide sequence according to SEQ ID NO:574 has no apparent open reading frame. Expression of SEQ ID NO:574 in normal and cancerous tissues was quantified by real-time RT-PCR using sequence-specific oligos (SEQ ID NO:575, 576); see Fig. 14. SEQ ID NO:574 is highly expressed in placenta. Compared to other normal tissues, SEQ ID NO:574 is overexpressed in lung cancer and melanoma. Based on these expression results, SEQ ID NO:574 and its expression products qualify as molecular markers and/or target candidates for targeted therapies, in particular for these particular tumor types.

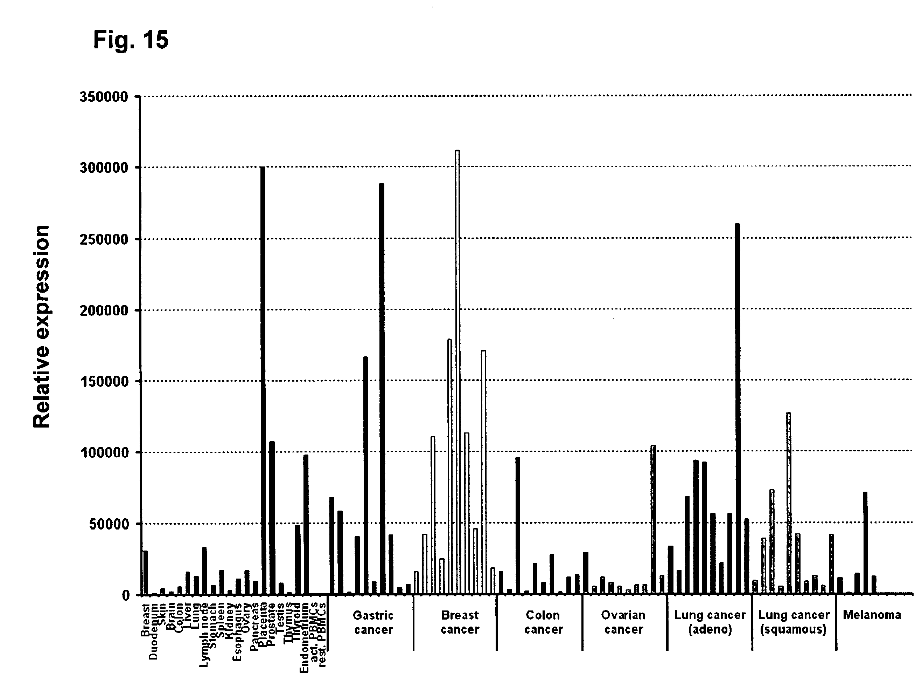

The nucleotide sequence according to SEQ ID NO:577 was deduced from SEQ ID NO:20. SEQ ID NO:577 represents a partial cDNA with no apparent open reading frame. Expression of SEQ ID NO:577 in normal and cancerous tissues was quantified by real-time RT-PCR using sequence-specific oligos (SEQ ID NO:578, 579); see Fig. 15. SEQ ID NO:577 is highly expressed in placenta. Compared to other normal tissues, SEQ ID NO:577 is overexpressed in gastric cancer, breast cancer and lung cancer. Based on these expression results, SEQ ID NO:577 and its expression products qualify as molecular markers and/or target candidates for targeted therapies, in particular for these particular tumor types.

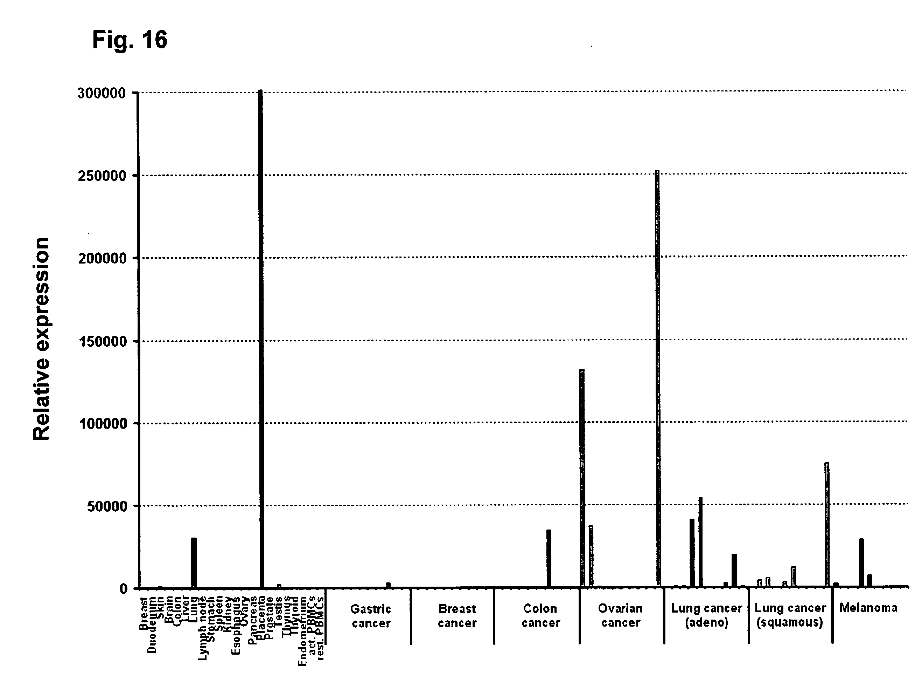

The nucleotide sequence according to SEQ ID NO:580 was deduced from SEQ ID NO:32. SEQ ID NO:580 represents a partial cDNA with no apparent open reading frame. Expression of SEQ ID NO:580 in normal and cancerous tissues was quantified by real-time RT-PCR using sequence-specific oligos (SEQ ID NO:581, 582); see Fig. 16. SEQ ID NO:580 is highly expressed in placenta. Compared to other normal tissues, SEQ ID NO:580 is overexpressed in ovarian cancer and lung cancer. Based on these expression results, SEQ ID NO:580 and its expression products qualify as molecular markers and/or target candidates for targeted therapies, in particular for these particular tumor types.

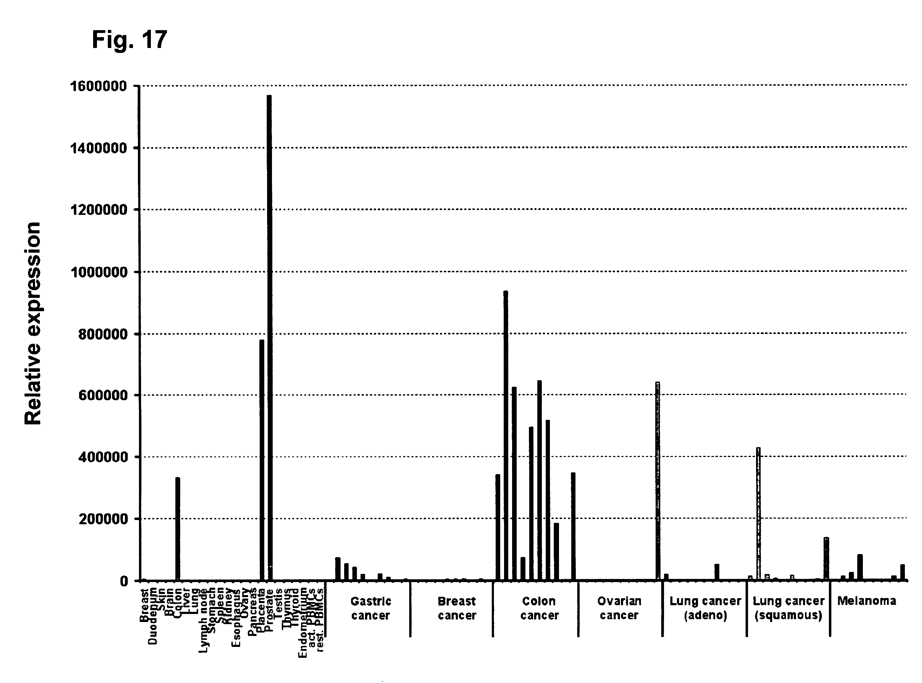

The nucleotide sequence according to SEQ ID NO:583 was deduced from SEQ ID NO:257 and codes for a member of the homeobox class of transcription factors (SEQ ID NO:584). Expression of these proteins is spatially and temporally regulated during embryonic development. Expression of SEQ ID NO:583 in normal and cancerous tissues was quantified by real-time RT-PCR using sequence-specific oligos (SEQ ID NO:585, 586); see Fig. 17. SEQ ID NO:583 is highly expressed in placenta and prostate. Compared to other normal tissues, SEQ ID NO:583 is overexpressed in colon cancer, ovarian cancer and lung cancer. Based on these expression results, SEQ ID NO:583 and its expression products qualify as molecular markers and/or target candidates for targeted therapies, in particular for these particular tumor types.

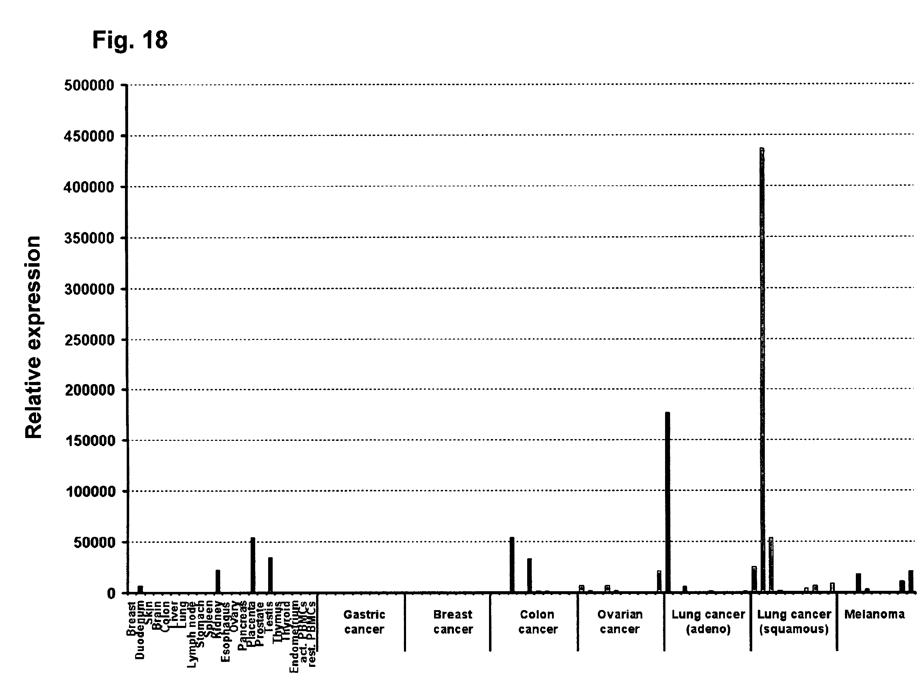

The nucleotide sequence according to SEQ ID NO:587 was deduced from SEQ ID NO:148 and codes for a member of the IGF-II mRNA-binding protein (IMP) family (SEQ ID NO:588). It functions by binding to the 5' UTR of the insulin-like growth factor 2 (IGF2) mRNA and regulating IGF2 translation. Expression of SEQ ID NO:587 in normal and cancerous tissues was quantified by real-time RT-PCR using sequence-specific oligos (SEQ ID NO:589, 590); see Fig. 18. Compared to normal tissues, SEQ ID NO:587 is overexpressed in lung cancer. Based on these expression results, SEQ ID NO:587 and its expression products qualify as molecular markers and/or target candidates for targeted therapies, in particular for this particular tumor type.

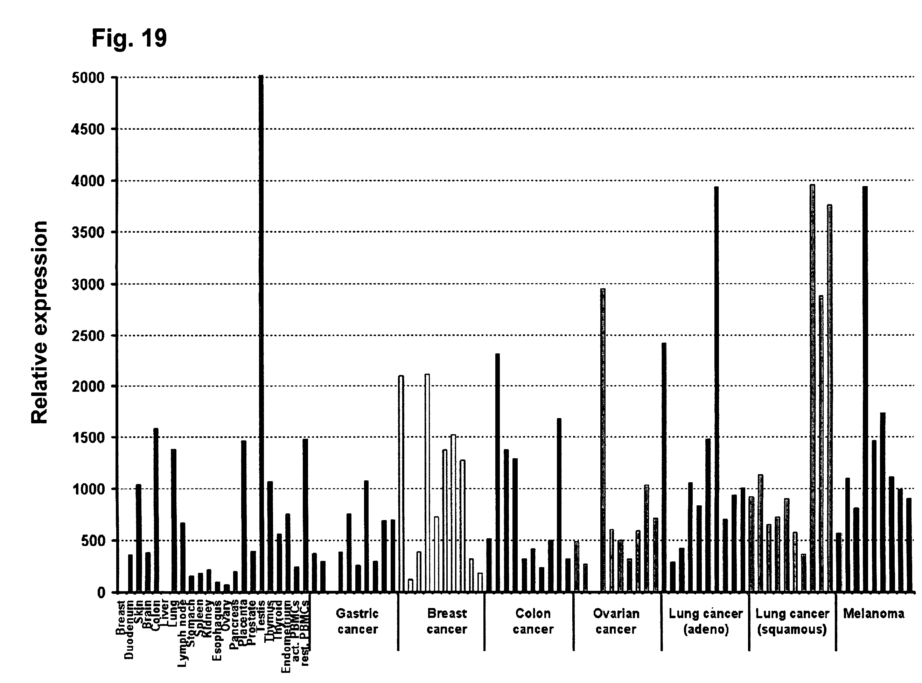

The nucleotide sequence according to SEQ ID NO:59 was deduced from SEQ ID NO:194 and codes for a 372 aa protein (SEQ ID NO:592) of unknown function. Expression of SEQ ID NO:591 in normal and cancerous tissues was quantified by real-time RT-PCR using sequence-specific oligos (SEQ ID NO:593, 594); see Fig. 19. SEQ ID NO:591 is highly expressed in testis. Compared to other normal tissues, SEQ ID NO:591 is overexpressed in breast cancer, colon cancer, ovarian cancer, lung cancer and melanoma. Based on these expression results, SEQ ID NO:591 and its expression products qualify as molecular markers and/or target candidates for targeted therapies, in particular for these particular tumor types.

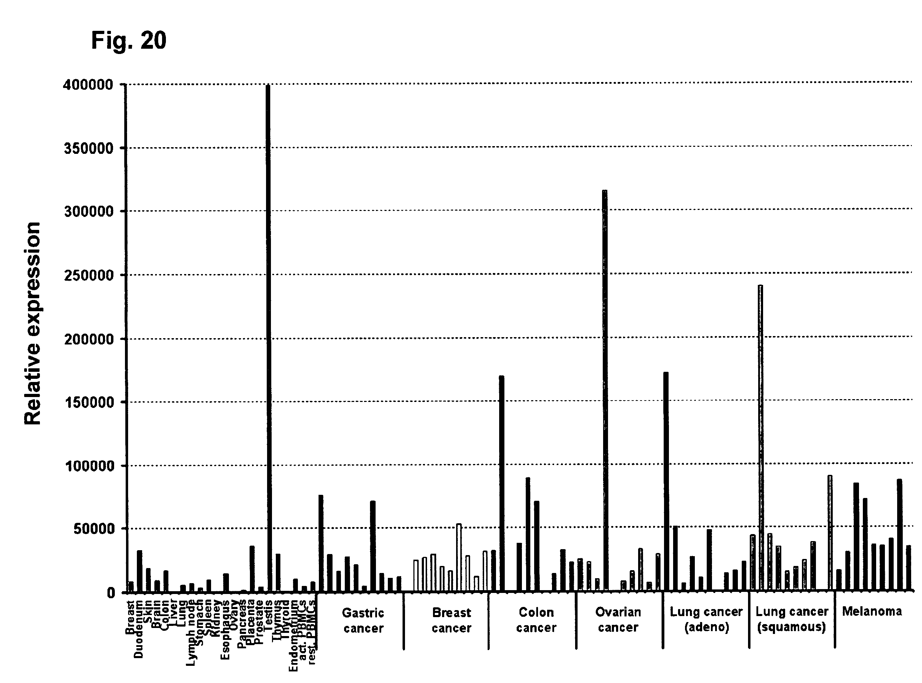

The nucleotide sequence according to SEQ ID NO:595 was deduced from SEQ ID NO:191 and codes for a 357 aa protein (SEQ ID NO:596) of unknown function. Expression of SEQ ID NO:595 in normal and cancerous tissues was quantified by real-time RT-PCR using sequence-specific oligos (SEQ ID NO:597, 598); see Fig. 20. SEQ ID NO:595 is highly expressed in testis. Compared to other normal tissues, SEQ ID NO:595 is overexpressed in gastric cancer, colon cancer, ovarian cancer, lung cancer and melanoma. Based on these expression results, SEQ ID NO:595 and its expression products qualify as molecular markers and/or target candidates for targeted therapies, in particular for these particular tumor types.

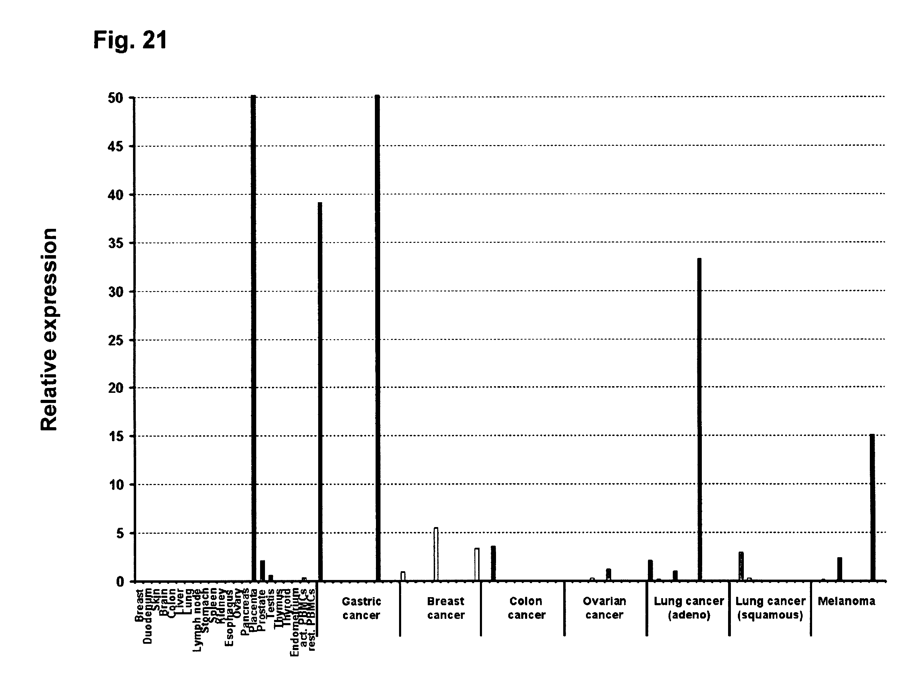

The nucleotide sequence according to SEQ ID NO:599 was deduced from SEQ ID NO: 18 and has no apparent open reading frame. Expression of SEQ ID NO:599 in normal and cancerous tissues was quantified by real-time RT-PCR using sequence-specific oligos (SEQ ID NO:600, 601); see Fig. 21. SEQ ID NO:599 is highly expressed in placenta. Compared to other normal tissues, SEQ ID NO:599 is overexpressed in gastric cancer, breast cancer, lung cancer and melanoma. Based on these expression results, SEQ ID NO:599 and its expression products qualify as molecular markers and/or target candidates for targeted therapies, in particular for these particular tumor types.

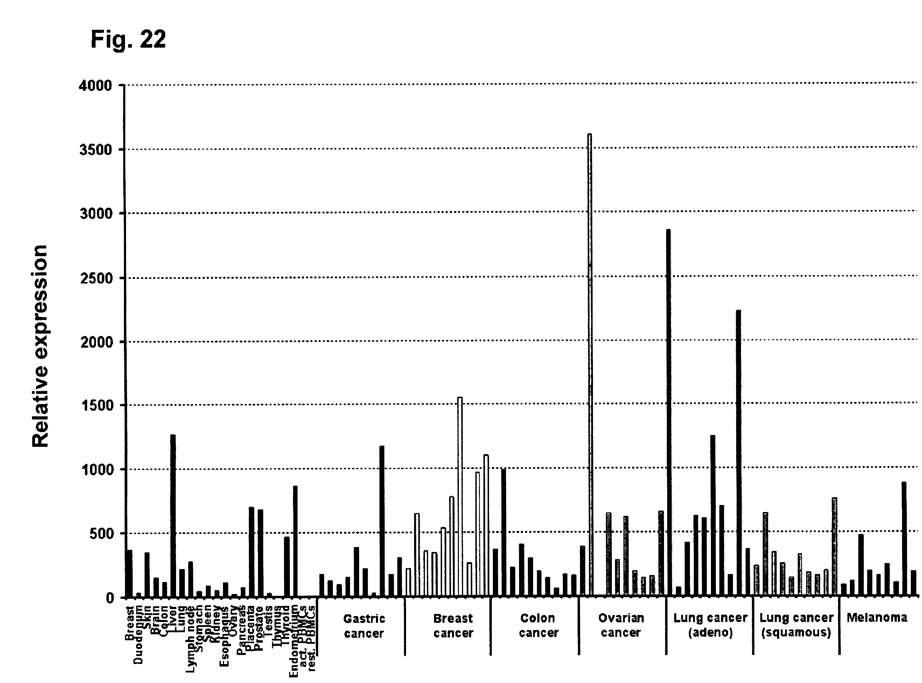

The nucleotide sequence according to SEQ ID NO:602 was deduced from SEQ ID NO:133 and codes for a member of the von Willebrand factor domain superfamily of extracellular matrix proteins (SEQ ID NO:603). Expression of SEQ ID NO:602 in normal and cancerous tissues was quantified by real-time RT-PCR using sequence-specific oligos (SEQ ID NO:604, 605); see Fig. 22. Compared to normal tissues, SEQ ID NO:602 is overexpressed in ovarian cancer and lung cancer. Based on these expression results, SEQ ID NO:602 and its expression products qualify as molecular markers and/or target candidates for targeted therapies, in particular for these particular tumor types.

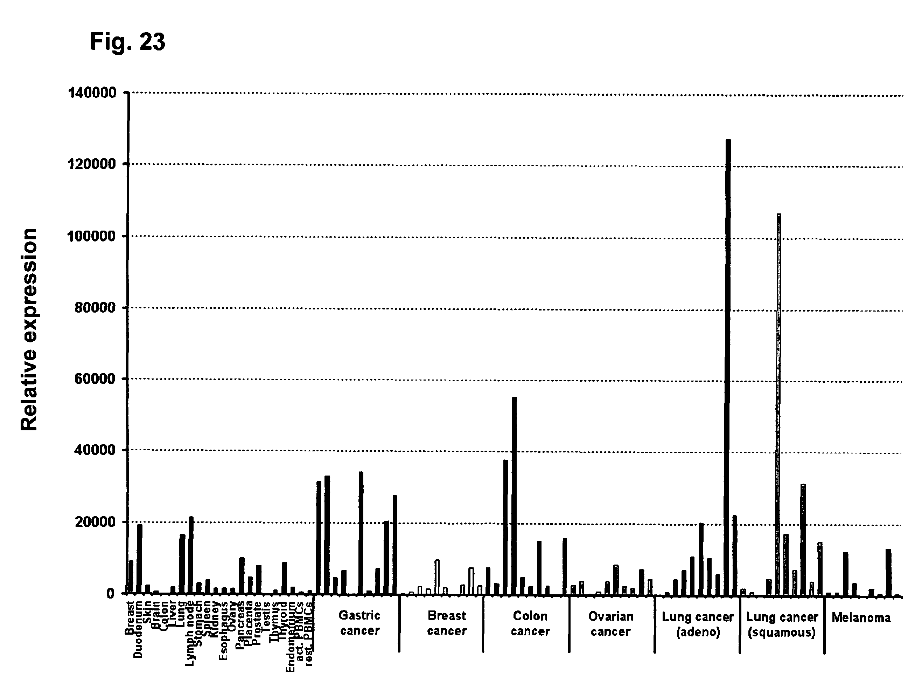

The nucleotide sequence according to SEQ ID NO:606 was deduced from SEQ ID NO:128 and codes for a member of the Borg family of CDC42 effector proteins (SEQ ID NO:607). Borg family proteins contain a CRIB (Cdc42/Rac interactive-binding) domain. They bind to, and negatively regulate the function of CDC42. CDC42, a small Rho GTPase, regulates the formation of F-actin-containing structures through its interaction with the downstream effector proteins. Expression of SEQ ID NO:606 in normal and cancerous tissues was quantified by real-time RT-PCR using sequence-specific oligos (SEQ ID NO:608, 609); see Fig. 23. Compared to normal tissues, SEQ ID NO:606 is overexpressed in gastric cancer, colon cancer and lung cancer. Based on these expression results, SEQ ID NO:606 and its expression products qualify as molecular markers and/or target candidates for targeted therapies, in particular for these particular tumor types.

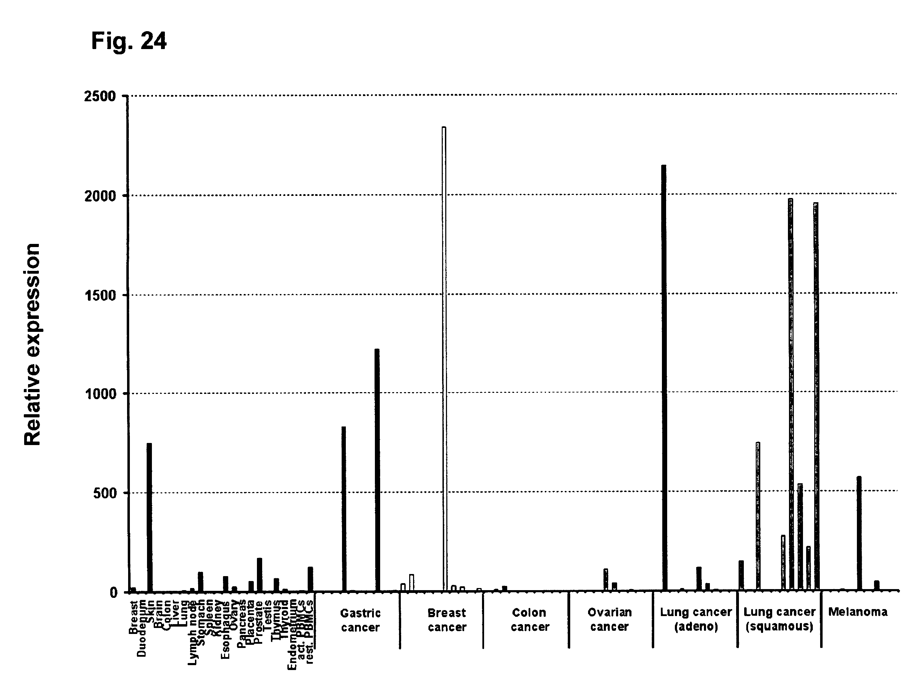

The nucleotide sequence according to SEQ ID NO:610 was deduced from SEQ ID NO:118 and has no apparent open reading frame. Expression of SEQ ID NO:61 0 in normal and cancerous tissues was quantified by real-time RT-PCR using sequence-specific oligos (SEQ ID NO:611, 612); see Fig. 24. Compared to normal tissues, SEQ ID NO:610 is overexpressed in gastric cancer, breast cancer and lung cancer. Based on these expression results, SEQ ID NO:610 and its expression products qualify as molecular markers and/or target candidates for targeted therapies, in particular for these particular tumor types.

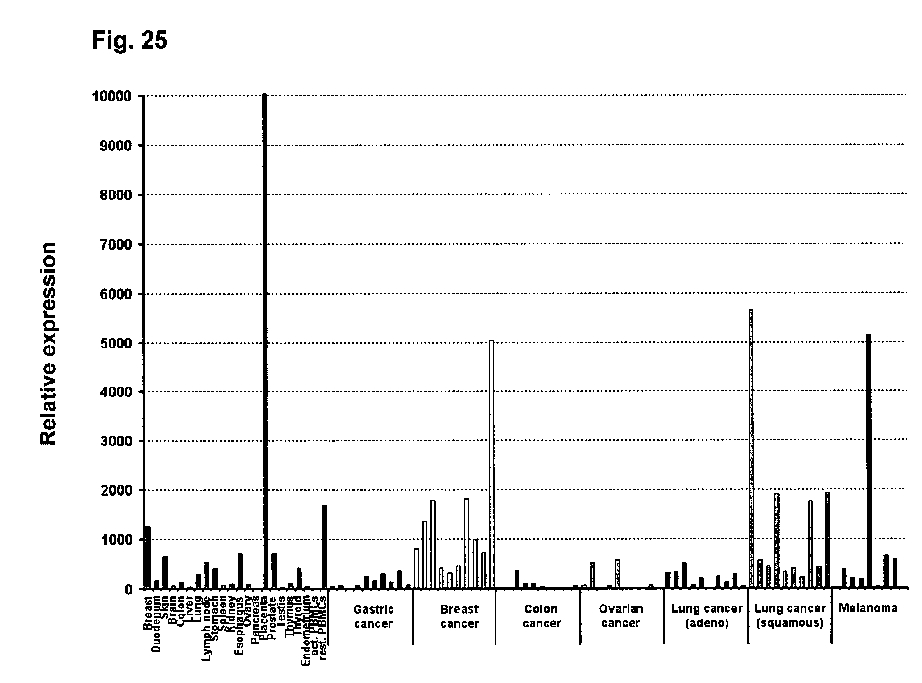

The nucleotide sequence according to SEQ ID NO:613 was deduced from SEQ ID NO:116 and codes for a 76 aa protein (SEQ ID NO:614) of unknown function. Expression of SEQ ID NO:613 in normal and cancerous tissues was quantified by real-time RT-PCR using sequence-specific oligos (SEQ ID NO:615, 616); see Fig. 25. SEQ ID NO:613 is highly expressed in placenta. Compared to other normal tissues, SEQ ID NO:613 is overexpressed in breast cancer, lung cancer and melanoma. Based on these expression results, SEQ ID NO:613 and its expression products qualify as molecular markers and/or target candidates for targeted therapies, in particular for these particular tumor types.

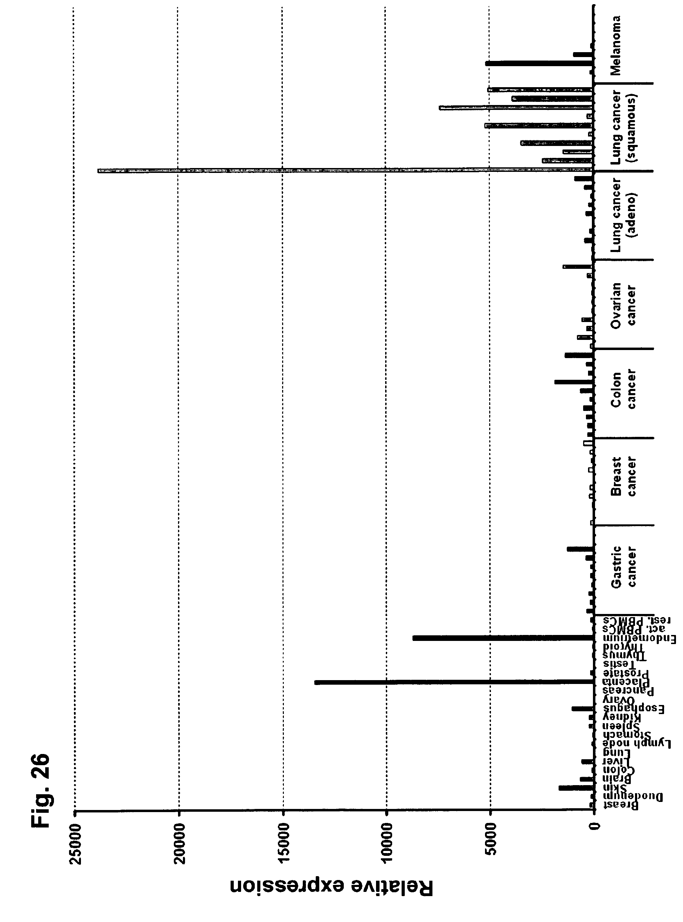

The nucleotide sequence according to SEQ ID NO:617 was deduced from SEQ ID NO:267. SEQ ID NO:617 represents a partial cDNA with no apparent open reading frame. Expression of SEQ ID NO:617 in normal and cancerous tissues was quantified by real-time RT-PCR using sequence-specific oligos (SEQ ID NO:618, 619); see Fig. 26. SEQ ID NO:617 is highy expressed in placenta and endometrium. Compared to other normal tissues, SEQ ID NO:617 is overexpressed in lung cancer and melanoma. Based on these expression results, SEQ ID NO:617 and its expression products qualify as molecular markers and/or target candidates for targeted therapies, in particular for these particular tumor types.

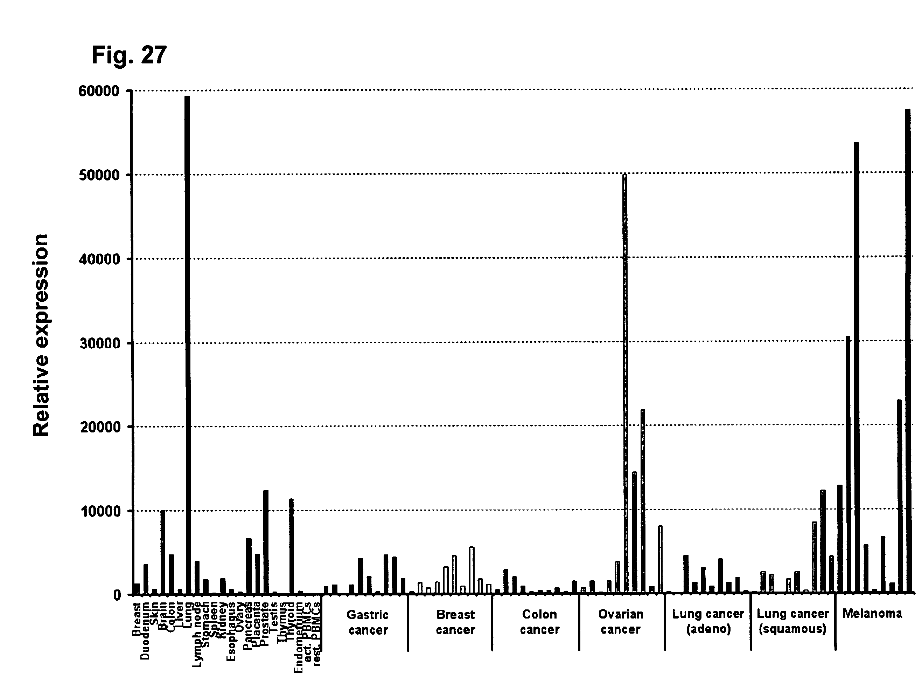

The nucleotide sequence according to SEQ ID NO:620 was deduced from SEQ ID NO:182 and codes for a 829 aa protein (SEQ ID NO:621) harboring multiple putative transmembrane domains and a patched family domain. The transmembrane protein Patched is a receptor for the morphogene Sonic Hedgehog. This protein associates with the smoothened protein to transduce hedgehog signals. SEQ ID NO:620 might represent a novel member of the Patched family. Expression of SEQ ID NO:620 in normal and cancerous tissues was quantified by real-time RT-PCR using sequence-specific oligos (SEQ ID NO:622, 623); see Fig. 27. SEQ ID NO:620 is highly expressed in lung. Compared to other normal tissues, SEQ ID NO:620 is overexpressed in ovarian cancer and melanoma. Based on these expression results, SEQ ID NO:620 and its expression products qualify as molecular markers and/or target candidates for targeted therapies, in particular for these particular tumor types.

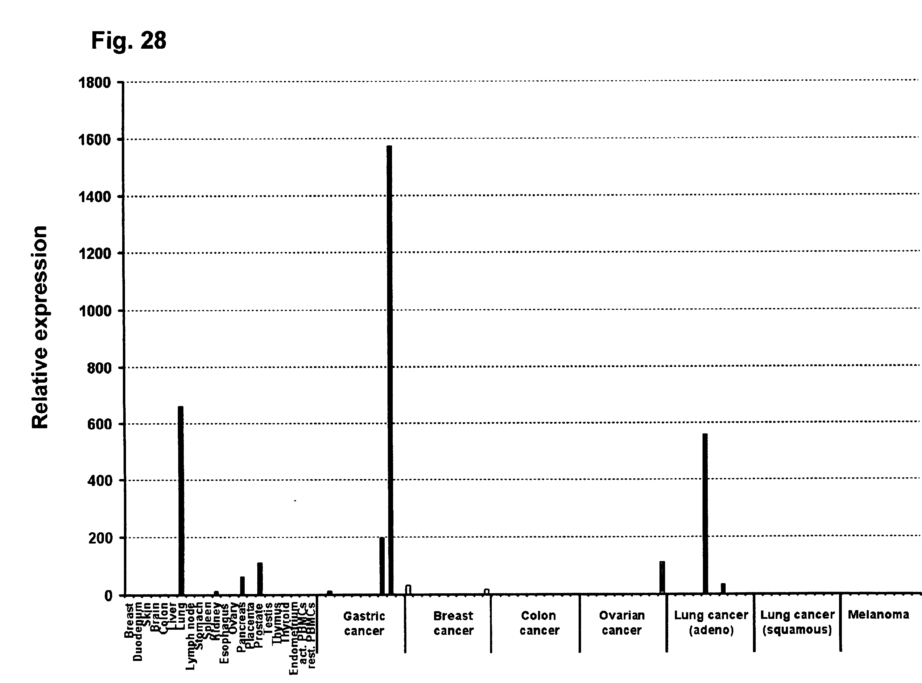

The nucleotide sequence according to SEQ ID NO:624 was deduced from SEQ ID NO:184 and codes for a 323 aa protein (SEQ ID NO:625) similar to TWIK-related acid-sensitive K⁺ channel, a member of the superfamily of potassium channel proteins that contain two pore-forming P domains. Expression of SEQ ID NO:624 in normal and cancerous tissues was quantified by real-time RT-PCR using sequence-specific oligos (SEQ ID NO:626, 627); see Fig. 28. SEQ ID NO:624 is highly expressed in lung. Compared to other normal tissues, SEQ ID NO:624 is overexpressed in gastric cancer and lung cancer. Based on these expression results, SEQ ID NO:624 and its expression products qualify as molecular markers and/or target candidates for targeted therapies, in particular for these particular tumor types.

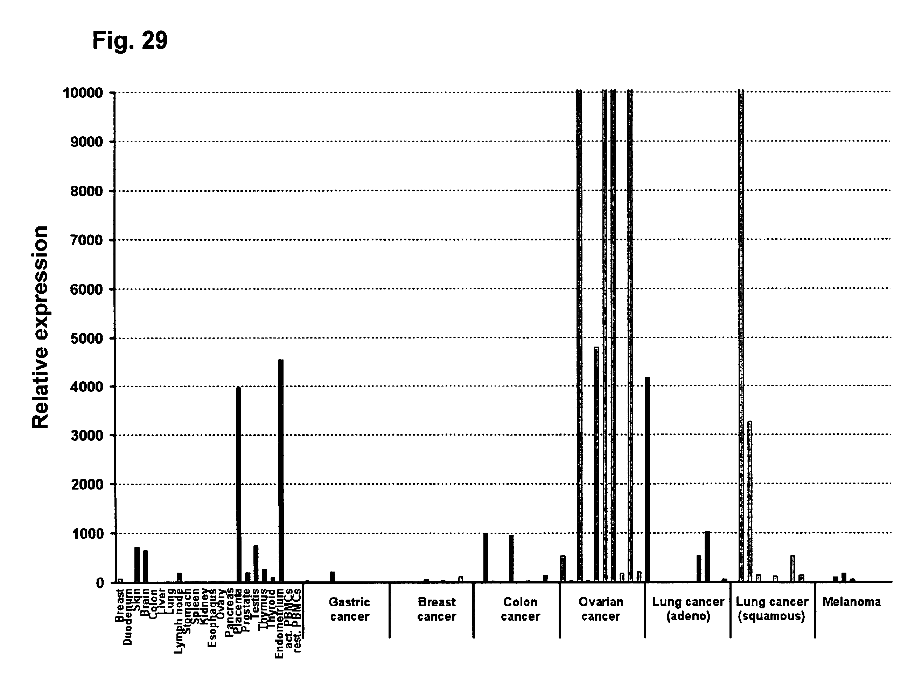

The nucleotide sequence according to SEQ ID NO:634 was deduced from SEQ ID NO:346 and codes for a member of the homeobox class of transcription factors (SEQ ID NO:635). Expression of these proteins is spatially and temporally regulated during embryonic development. Expression of SEQ ID NO:634 in normal and cancerous tissues was quantified by real-time RT-PCR using sequence-specific oligos (SEQ ID NO:636, 637); see Fig. 29. SEQ ID NO:634 is highly expressed in placenta and endometrium. Compared to other normal tissues, SEQ ID NO:634 is overexpressed in ovarian cancer and lung cancer. Based on these expression results SEQ ID NO:634 and its expression products qualify as molecular markers and/or target candidates for targeted therapies, in particular for these particular tumor types.

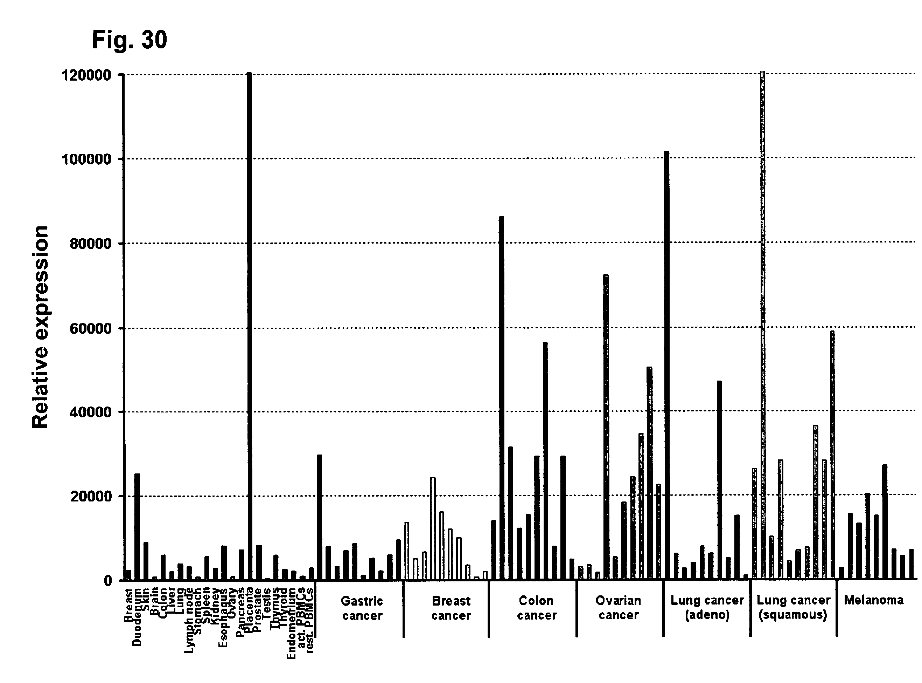

The nucleotide sequence according to SEQ ID NO:638 was deduced from SEQ ID NO:252 and codes for a member of the F-box protein family (SEQ ID NO:639) which is characterized by an approximately 40 amino acid motif, the F-box. The F-box proteins constitute one of the four subunits of ubiquitin protein ligase complex called SCFs (SKP1-cullin-F-box), which function in phosphorylation-dependent ubiquitination. The F-box proteins are divided into 3 classes: Fbws containing WD-40 domains, Fbls containing leucine-rich repeats, and Fbxs containing either different protein-protein interaction modules or no recognizable motifs. The protein encoded by this gene belongs to the Fbls class; in addition to an F-box, this protein contains 10 tandem leucine-rich repeats. This protein is an essential element of the cyclin A-CDK2 S-phase kinase. It specifically recognizes phosphorylated cyclin-dependent kinase inhibitor 1B (CDKN1B, also referred to as p27 or KIP1) predominantly in S phase and interacts with S-phase kinase-associated protein 1 (SKP1 or p19). Expression of SEQ ID NO:638 in normal and cancerous tissues was quantified by real-time RT-PCR using sequence-specific oligos (SEQ ID NO:640, 641); see Fig. 30. SEQ ID NO:638 is highly expressed in placenta. Compared to normal tissues, SEQ ID NO:638 is overexpressed in colon cancer, ovarian cancer, lung cancer, and malignant melanomas. Based on these expression results SEQ ID NO:638 and its expression products qualify as molecular markers and/or target candidates for targeted therapies, in particular for these particular tumor types.

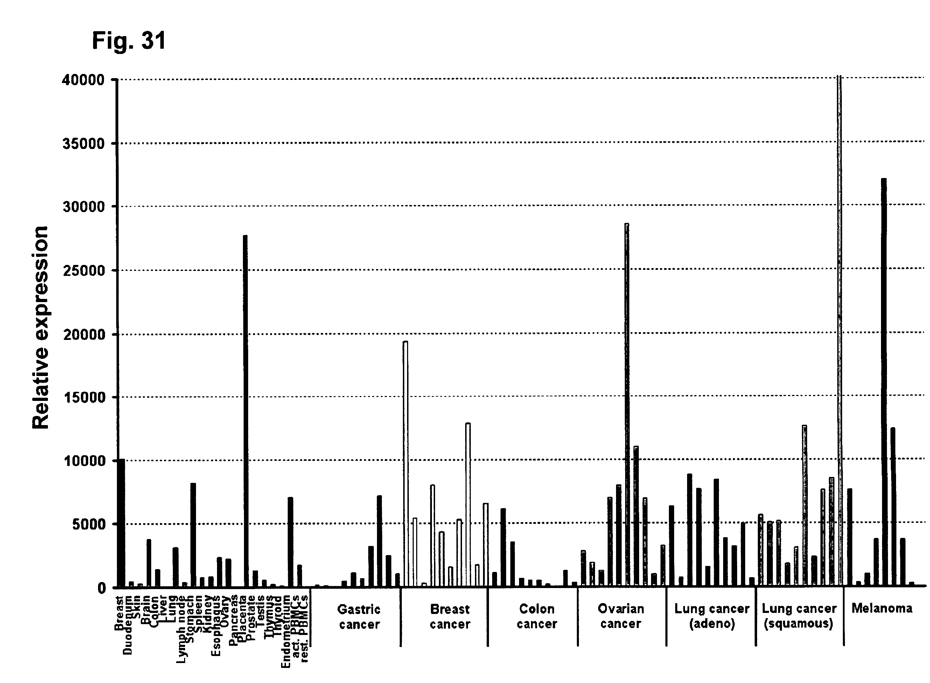

The nucleotide sequence according to SEQ ID NO:642 was deduced from SEQ ID NO:248 and codes for a 1.024 aa protein (SEQ ID NO:643) of unknown function. Expression of SEQ ID NO:642 in normal and cancerous tissues was quantified by real-time RT-PCR using sequence-specific oligos (SEQ ID NO:644, 645); see Fig. 31. SEQ ID NO:642 is highly expressed in placenta. Compared to normal tissues, SEQ ID NO:642 is overexpressed in breast cancer, ovarian cancer, lung cancer, and malignant melanomas. Based on these expression results SEQ ID NO:642 and its expression products qualify as molecular markers and/or target candidates for targeted therapies, in particular of these particular tumor types.

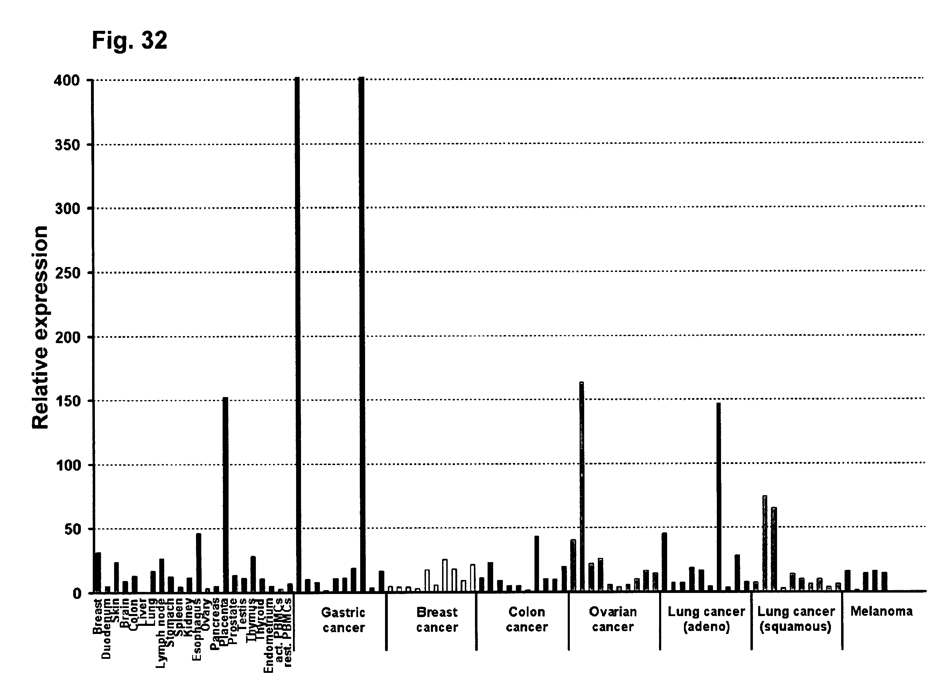

The nucleotide sequence according to SEQ ID NO:646 was deduced from SEQ ID NO:269. SEQ ID NO:646 represents a partial cDNA with no apparent open reading frame. Expression of SEQ ID NO:646 in normal and cancerous tissues was quantified by real-time RT-PCR using sequence-specific oligos (SEQ ID NO:647, 648); see Fig. 32. SEQ ID NO:646 is expressed in placenta. Compared to normal tissues, SEQ ID NO:646 is overexpressed in gastric cancer, ovarian cancer, and lung cancer. Based on these expression results SEQ ID NO:646 and its expression products qualify as molecular markers and/or target candidates for targeted therapies, in particular for these particular tumor types.

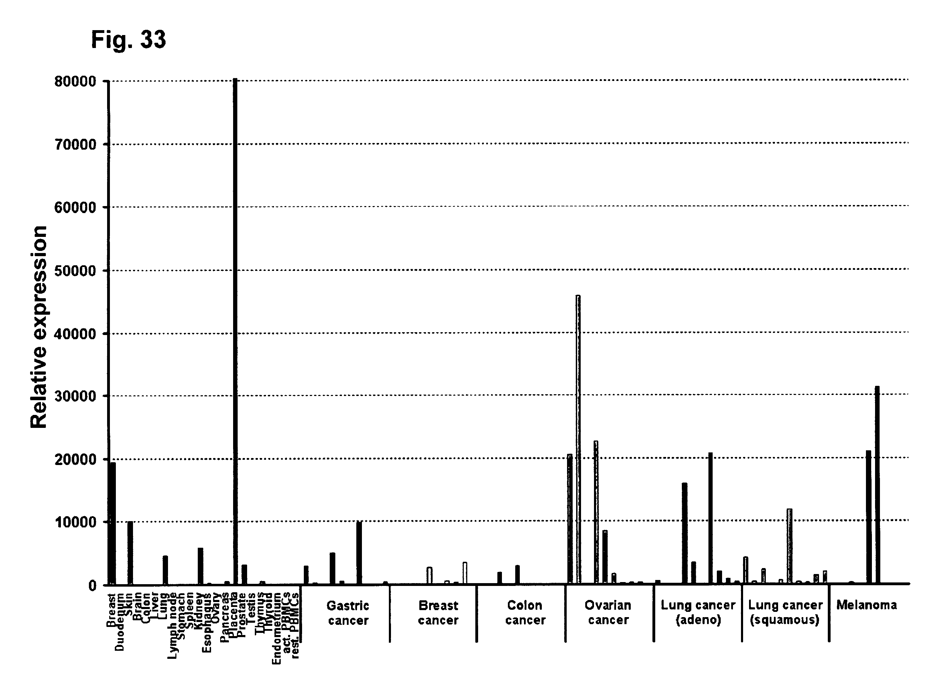

The nucleotide sequence according to SEQ ID NO:649 was deduced from SEQ ID NO:27 and codes for a 258 aa protein (SEQ ID NO:650) of unknown function. Expression of SEQ ID NO:649 in normal and cancerous tissues was quantified by real-time RT-PCR using sequence-specific oligos (SEQ ID NO:651, 652); cf. Fig. 33. SEQ ID NO:649 is highly expressed in placenta. Compared to normal tissues, SEQ ID NO:649 is overexpressed in ovarian cancer, lung cancer, and malignant melanomas. Based on these expression results SEQ ID NO:649 and its expression products qualify as molecular markers and/or target candidates for targeted therapies, in particular for these particular tumor types.

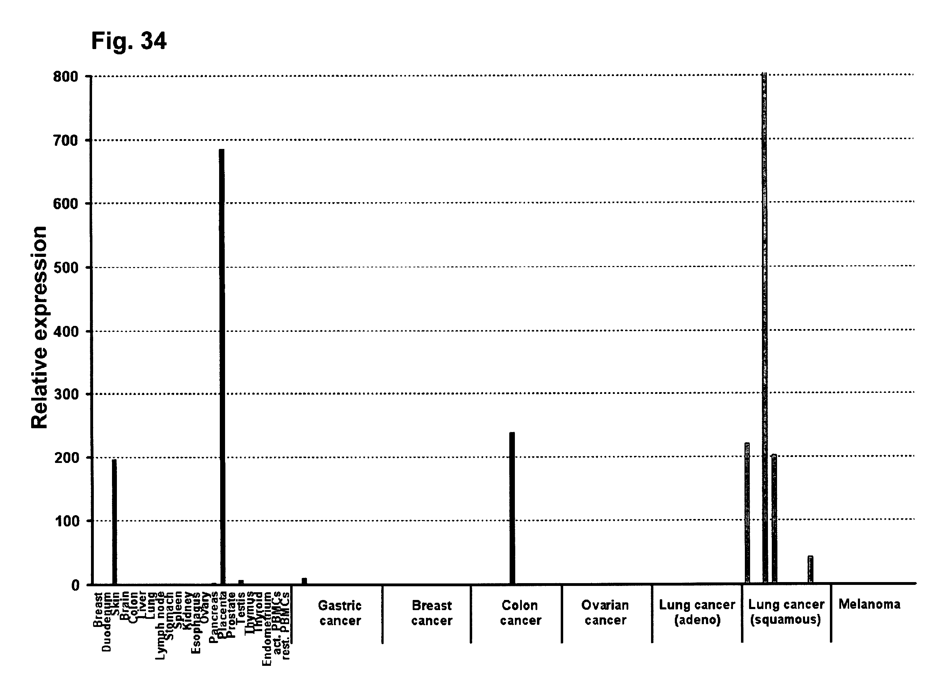

The nucleotide sequence according to SEQ ID NO:653 was deduced from SEQ ID NO:139. SEQ ID NO:653 represents a partial cDNA with no apparent open reading frame. Expression of SEQ ID NO:653 in normal and cancerous tissues was quantified by real-time RT-PCR using sequence-specific oligos (SEQ ID NO:654, 655); see Fig. 34. SEQ ID NO:653 is expressed in placenta and skin. Compared to other normal tissues, SEQ ID NO:653 is overexpressed in colon cancer, and lung cancer. Based on these expression results SEQ ID NO:653 and its expression products qualify as molecular markers and/or target candidates for targeted therapies, in particular for these particular tumor types.

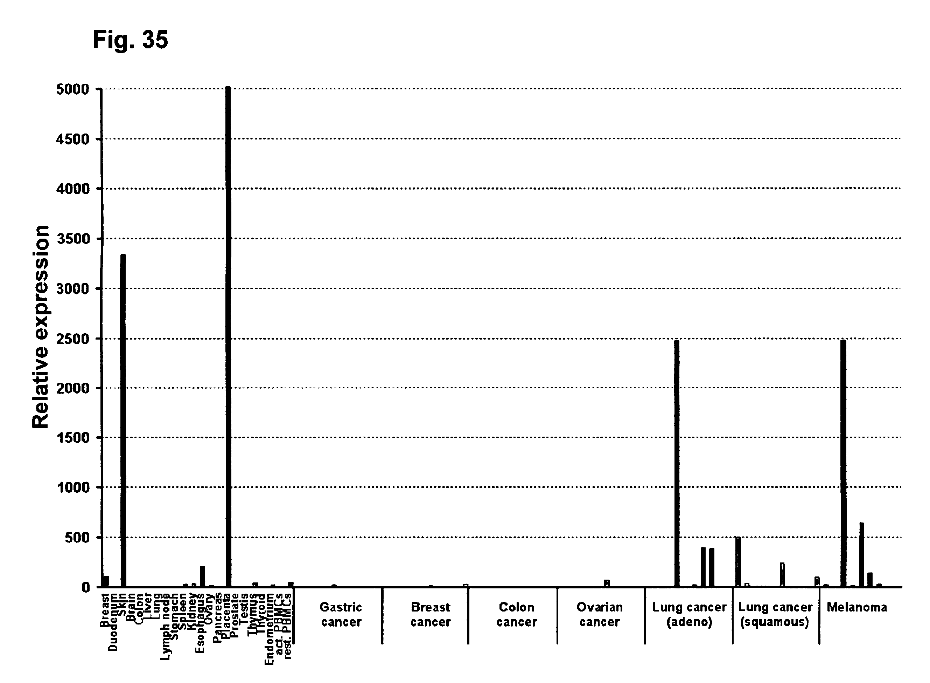

The nucleotide sequence according to SEQ ID NO:656 was deduced from SEQ ID NO:305 and codes for a 419 aa protein (SEQ ID NO:657) of unknown function. Expression of SEQ ID NO:656 in normal and cancerous tissues was quantified by real-time RT-PCR using sequence-specific oligos (SEQ ID NO:658, 659); see Fig. 35. SEQ ID NO:656 is expressed in placenta and skin. Compared to other normal tissues, SEQ ID NO:656 is overexpressed in lung cancer, and malignant melanomas. Based on these expression results SEQ ID NO:656 and its expression products qualify as molecular markers and/or target candidates for targeted therapies, in particular for these particular tumor types.

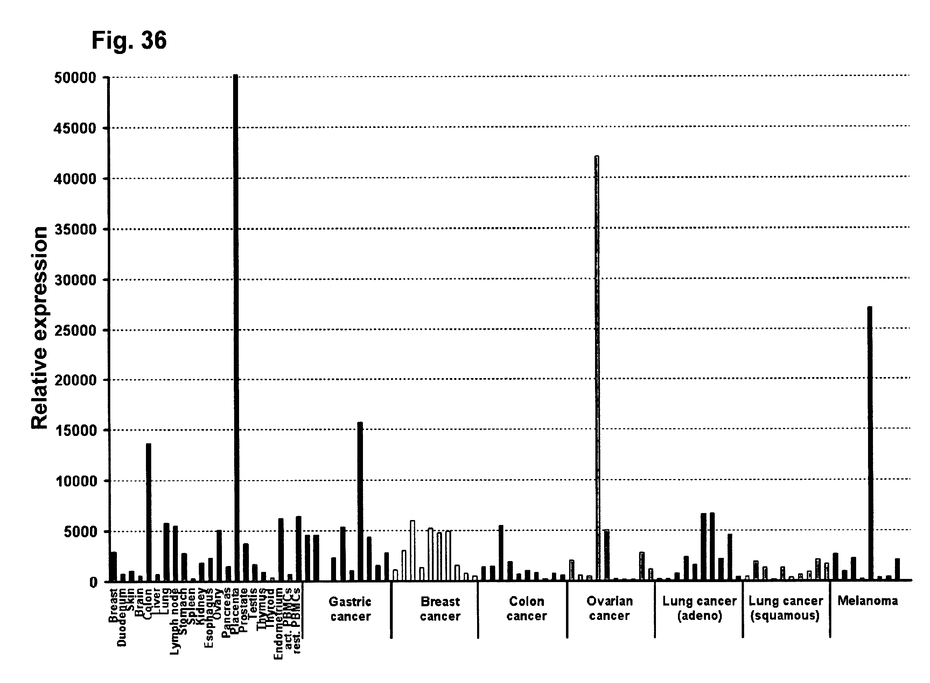

The nucleotide sequence according to SEQ ID NO:660 was deduced from SEQ ID NO: 125 and codes for a a disintegrin and metalloproteinase with thrombospondin motif (SEQ ID NO:661). Expression of SEQ ID NO:660 in normal and cancerous tissues was quantified by real-time RT-PCR using sequence-specific oligos (SEQ ID NO:662, 663); see Fig. 36. SEQ ID NO:660 is highly expressed in placenta. Compared to normal tissues, SEQ ID NO:660 is overexpressed in gastric cancer, ovarian cancer, and malignant melanomas. Based on these expression results. SEQ ID NO:660 and its expression products qualify as molecular markers and/or target candidates for targeted therapies, in particular for these particular tumor types.

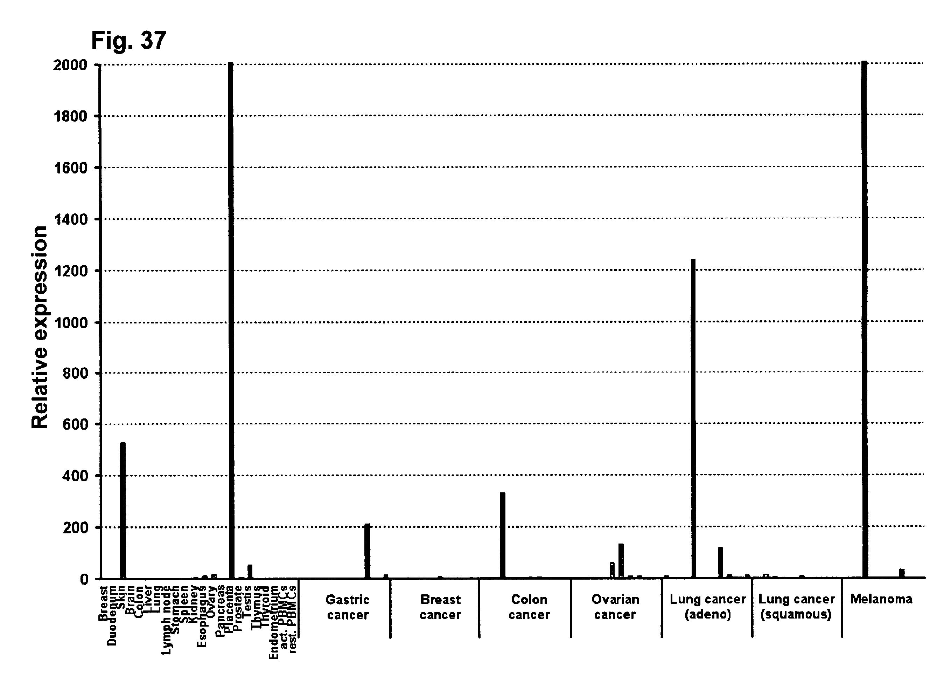

The nucleotide sequence according to SEQ ID NO:664 was deduced from SEQ ID NO:61 and codes for a 334 aa protein (SEQ ID NO:665) of unknown function. Expression of SEQ ID NO:664 in normal and cancerous tissues was quantified by real-time RT-PCR using sequence-specific oligos (SEQ ID NO:666, 667); see Fig. 37. SEQ ID NO:664 is expressed in placenta and skin. Compared to other normal tissues, SEQ ID NO:664 is overexpressed in gastric cancer, colon cancer, lung cancer, and malignant melanomas. Based on these expression results SEQ ID NO:664 and its expression products qualify as molecular markers and/or target candidates for targeted therapies, in particular for these particular tumor types.

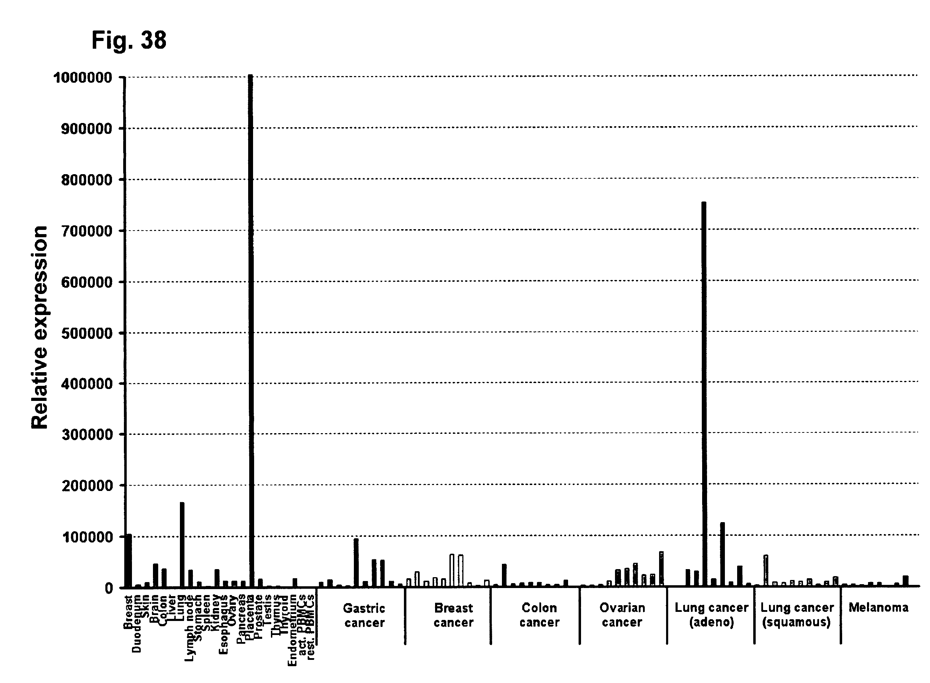

The nucleotide sequence according to SEQ ID NO:668 was deduced from SEQ ID NO: 13. SEQ ID NO:668 represents a partial cDNA with no apparent open reading frame. Expression of SEQ ID NO:668 in normal and cancerous tissues was quantified by real-time RT-PCR using sequence-specific oligos (SEQ ID NO:669, 670); see Fig. 38. SEQ ID NO:668 is highly expressed in placenta. Compared to other normal tissues, SEQ ID NO:668 is overexpressed in lung cancer. Based on these expression results SEQ ID NO:668 and its expression products qualify as molecular markers and/or target candidates for targeted therapies, in particular for this particular tumor type.

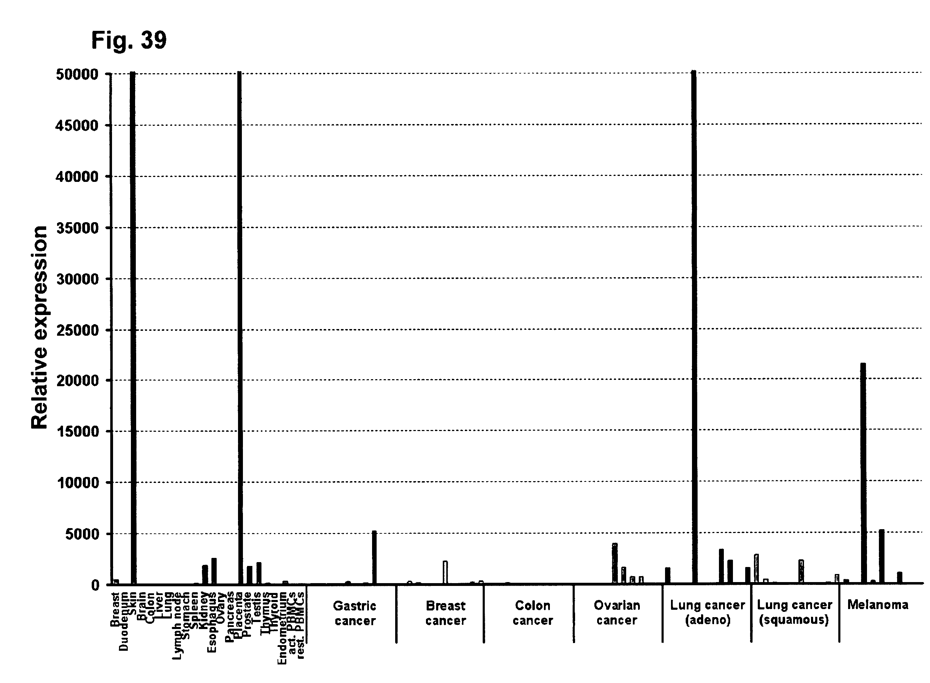

The nucleotide sequence according to SEQ ID NO:671 was deduced from SEQ ID NO:42 and codes for a 426 aa protein (SEQ ID NO:672) of unknown function. Expression of SEQ ID NO:671 in normal and cancerous tissues was quantified by real-time RT-PCR using sequence-specific oligos (SEQ ID NO:673, 674); see Fig. 39. SEQ ID NO:671 is expressed in placenta and skin. Compared to other normal tissues, SEQ ID NO:671 is overexpressed in lung cancer, and malignant melanomas. Based on these expression results SEQ ID NO:671 and its expression products qualify as molecular markers and/or target candidates for targeted therapies, in particular for these particular tumor types.

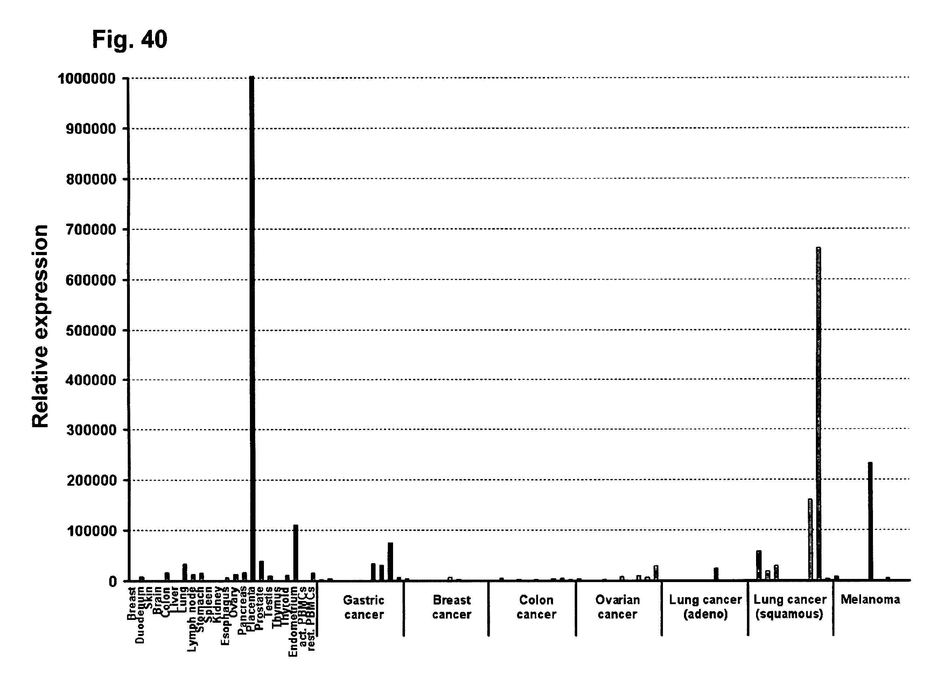

The nucleotide sequence according to SEQ ID NO:675 was deduced from SEQ ID NO:314 and codes for a 2.912 aa protein (SEQ ID NO:676). Expression of SEQ ID NO:675 in normal and cancerous tissues was quantified by real-time RT-PCR using sequence-specific oligos (SEQ ID NO:677, 678); see Fig. 40. SEQ ID NO:675 is highly expressed in placenta. Compared to other normal tissues, SEQ ID NO:675 is overexpressed in lung cancer, and malignant melanomas. Based on these expression results SEQ ID NO:675 and its expression products qualify as molecular markers and/or target candidates for targeted therapies, in particular for these particular tumor types.

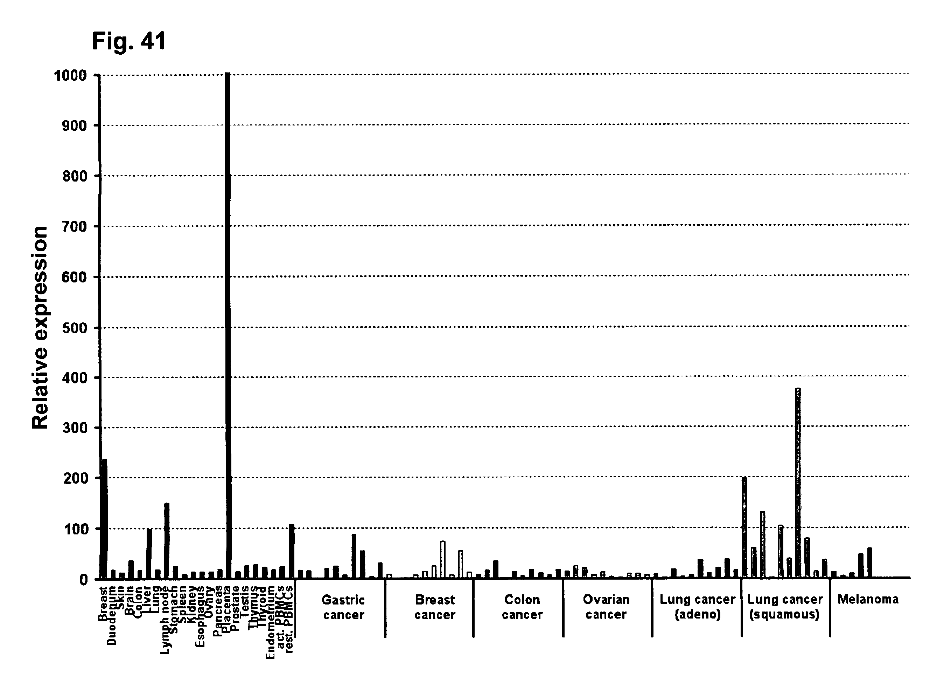

The nucleotide sequence according to SEQ ID NO:679 was deduced from SEQ ID NO:31. SEQ ID NO:679 represents a partial cDNA with no apparent open reading frame. Expression of SEQ ID NO:679 in normal and cancerous tissues was quantified by real-time RT-PCR using sequence-specific oligos (SEQ ID NO:680, 681); see Fig. 41. SEQ ID NO:679 is highly expressed in placenta. Compared to other normal tissues, SEQ ID NO:679 is overexpressed in lung cancer. Based on these expression results SEQ ID NO:679 and its expression products qualify as molecular markers and/or target candidates for targeted therapies, in particular for this particular tumor type.

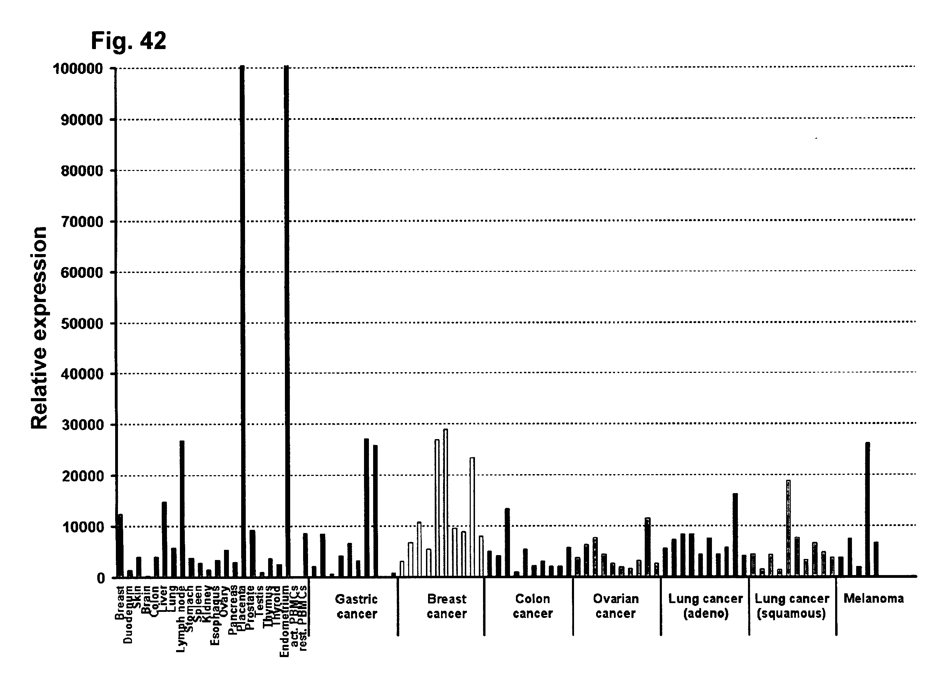

The nucleotide sequence according to SEQ ID NO:682 was deduced from SEQ ID NO:21. and codes for a 278 aa protein (SEQ ID NO:683). Expression of SEQ ID NO:682 in normal and cancerous tissues was quantified by real-time RT-PCR using sequence-specific oligos (SEQ ID N0:684, , 685); see Fig. 42. SEQ ID NO:682 is highly expressed in placenta, endometrium, and at lower levels in lymph node. Compared to other normal tissues SEQ ID NO:682 is overexpressed in gastric cancer, breast cancer, and malignant melanomas. Based on these expression results SEQ ID NO:682 and its expression products qualify as molecular markers and/or target candidates for targeted therapies, in particular for these particular tumor types.

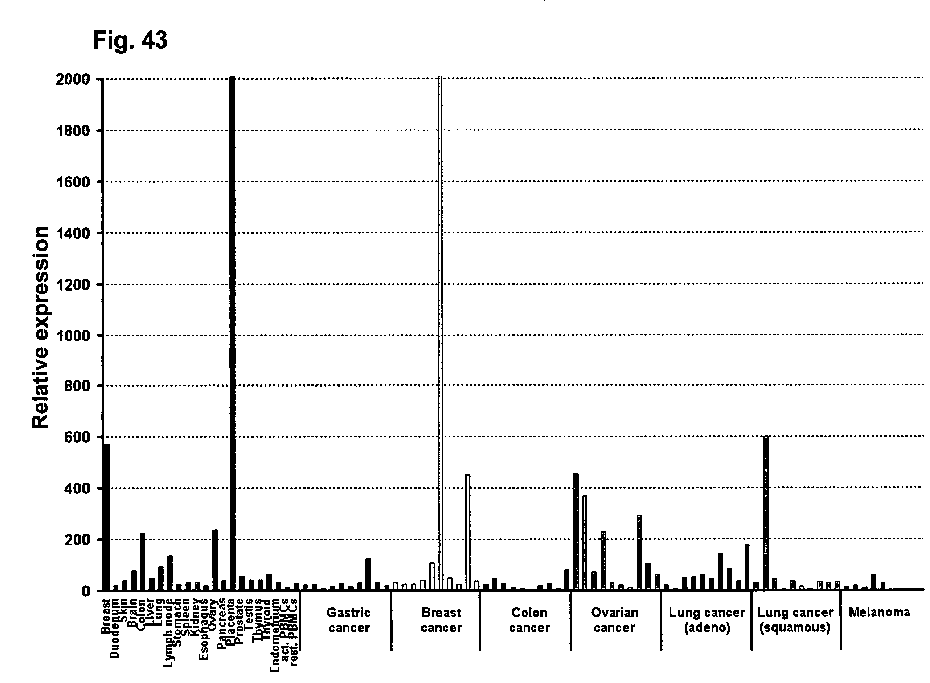

The nucleotide sequence according to SEQ ID NO:686 was deduced from SEQ ID NO:58. SEQ ID NO:686 represents a partial cDNA with no apparent open reading frame. Expression of SEQ ID NO:686 in normal and cancerous tissues was quantified by real-time RT-PCR using sequence-specific oligos (SEQ ID NO:687, 688); see Fig. 43. SEQ ID NO:686 is highly expressed in placenta and at lower levels in breast. Compared to other normal tissues, SEQ ID NO:686 is overexpressed in breast cancer, ovarian cancer, and lung cancer. Based on these expression results SEQ ID NO:686 and its expression products qualify as molecular markers and/or target candidates for targeted therapies, in particular for these particular tumor types.

## Claims

1. A pharmaceutical composition, comprising an agent which
(I) inhibits expression or activity of a tumor-associated antigen and/or
(II) has tumor-inhibiting activity, and is selective for cells expressing or abnormally expressing a nucleic acid coding for a tumor-associated antigen or a tumor-associated antigen and/or
(III) when administered, selectively increases the amount of complexes between an MHC molecule and a tumor-associated antigen or a part thereof,
said tumor-associated antigen having a sequence encoded by a nucleic acid which is selected from the group consisting of:
(a) a nucleic acid which comprises a nucleic acid sequence selected from the group consisting of SEQ ID NOs: 634, 1-540, 541, 545, 549, 553, 557, 560, 563, 566, 570, 574, 577, 580, 583, 587, 591, 595, 599, 602, 606, 610, 613, 617, 620, 624, 638, 642, 646, 649, 653, 656, 660, 664, 668, 671, 675, 679, 682, and 686, a part or derivative thereof,
(b) a nucleic acid which hybridizes with the nucleic acid of (a) under stringent conditions,
(c) a nucleic acid which is degenerate with respect to the nucleic acid of (a) or (b), and
(d) a nucleic acid which is complementary to the nucleic acid of (a), (b) or (c).

2. The pharmaceutical composition as claimed in claim 1, in which the agent under (I) or (II) is an antisense nucleic acid which hybridizes selectively with the nucleic acid coding for the tumor-associated antigen or is an antibody which binds selectively to the tumor-associated antigen.

3. The pharmaceutical composition as claimed in claim 1, in which the agent comprises one or more components selected from the group consisting of:
(i) the tumor-associated antigen or a part thereof,
(ii) a nucleic acid which codes for the tumor-associated antigen or a part thereof,
(iii) an antibody which binds to the tumor-associated antigen or a part thereof,
(iv) an antisense nucleic acid which hybridizes specifically with a nucleic acid coding for the tumor-associated antigen,
(v) an siRNA directed against a nucleic acid coding for the tumor-associated antigen,
(vi) a host cell which expresses the tumor-associated antigen or a part thereof, and
(vii) isolated complexes between the tumor-associated antigen or a part thereof and an MHC molecule.

4. A pharmaceutical composition, comprising one or more components selected from the group consisting of:
(i) a tumor-associated antigen or a part thereof,
(ii) a nucleic acid which codes for a tumor-associated antigen or a part thereof,
(iii) an antibody which binds to a tumor-associated antigen or a part thereof,
(iv) an antisense nucleic acid which hybridizes specifically with a nucleic acid coding for a tumor-associated antigen,
(v) an siRNA directed against a nucleic acid coding for a tumor-associated antigen,
(vi) a host cell which expresses a tumor-associated antigen or a part thereof, and
(vii)isolated complexes between a tumor-associated antigen or a part thereof and an MHC molecule,
said tumor-associated antigen having a sequence encoded by a nucleic acid which is selected from the group consisting of:
(a) a nucleic acid which comprises a nucleic acid sequence selected from the group consisting of SEQ ID NOs: 634, 1-540, 541, 545, 549, 553, 557, 560, 563, 566, 570, 574, 577, 580, 583, 587, 591, 595, 599, 602, 606, 610, 613, 617, 620, 624, 638, 642, 646, 649, 653, 656, 660, 664, 668, 671, 675, 679, 682, and 686, a part or derivative thereof,
(b) a nucleic acid which hybridizes with the nucleic acid of (a) under stringent conditions,
(c) a nucleic acid which is degenerate with respect to the nucleic acid of (a) or (b), and
(d) a nucleic acid which is complementary to the nucleic acid of (a), (b) or (c).

5. The pharmaceutical composition as claimed in claim 3 or 4, in which the host cell additionally expresses an MHC molecule which binds to the tumor-associated antigen or the part thereof, wherein the host cell preferably is an antigen-presenting cell.

6. The pharmaceutical composition as claimed in any of claims 2 to 4, in which the antibody is a monoclonal, chimeric, human or humanized antibody, or is a fragment of an antibody.

7. The pharmaceutical composition as claimed in any of claims 2 to 4 and 6 in which the antibody is coupled to a therapeutic or diagnostic agent.

8. The pharmaceutical composition as claimed in any of claims 1-7, which may be used for the treatment or prevention of cancer.

9. The pharmaceutical composition as claimed in claim 8, in which the cancer is a lung tumor, a breast tumor, a prostate tumor, a melanoma, a colon tumor, a gastric tumor, a pancreatic tumor, an ENT tumor, an ovarian tumor, a colorectal tumor, a cervical carcinoma, a colon carcinoma or a mammary carcinoma.

10. The pharmaceutical composition as claimed in any of claims 1, 3-5, 8 and 9, which is in the form of a vaccine and preferably is for therapeutic and/or prophylactic use.

11. A method of diagnosing or monitoring a cancer disease, which method comprises detecting or determining the quantity
(i) of a tumor-associated nucleic acid or of a part thereof, and/or
(ii) of a tumor-associated antigen or of a part thereof, and/or
(iii) of an antibody to the tumor-associated antigen or a part thereof and/or
(iv) of T lymphocytes which are specific to the tumor-associated antigen or to a part thereof
in a biological sample isolated from a patient,
said tumor-associated nucleic acid being selected from the group consisting of:
(a) a nucleic acid which comprises a nucleic acid sequence selected from the group consisting of SEQ ID NOs: 634, 1-540, 541, 545, 549, 553, 557, 560, 563, 566, 570, 574, 577, 580, 583, 587, 591, 595, 599, 602, 606, 610, 613, 617, 620, 624, 638, 642, 646, 649, 653, 656, 660, 664, 668, 671, 675, 679, 682, and 686, a part or derivative thereof,
(b) a nucleic acid which hybridizes with the nucleic acid of (a) under stringent conditions,
(c) a nucleic acid which is degenerate with respect to the nucleic acid of (a) or (b), and
(d) a nucleic acid which is complementary to the nucleic acid of (a), (b) or (c), and
said tumor-associated antigen having a sequence encoded by a nucleic acid which is selected from said group of nucleic acids.

12. The method as claimed in claim 11, in which the detection or determination of the quantity comprises
(i) contacting the biological sample with an agent which binds specifically to the tumor-associated nucleic acid or to the part thereof, to the tumor-associated antigen or the part thereof, to the antibody or to the T lymphocytes, and
(ii) detecting the formation of or determining the quantity of a complex between the agent and the nucleic acid or the part thereof, the tumor-associated antigen or the part thereof, the antibody or the T lymphocytes.

13. The method as claimed in claim 12, in which the agent which binds specifically to the tumor-associated nucleic acid or to the part thereof is an oligonucleotide or polynucleotide, which hybridizes specifically to said nucleic acid or to said part thereof.

14. The method as claimed in claim 12, in which the agent which binds specifically to the tumor-associated antigen or the part thereof is an antibody binding specifically to said tumor-associated antigen or to said part thereof.

15. The method as claimed in claim 12, in which the agent which binds specifically to the antibody is a protein or peptide binding specifically to said antibody.

16. The method as claimed in claim 12, in which the agent which binds specifically to the T lymphocytes is a cell presenting the complex between the tumor-associated antigen or the part thereof and an MHC molecule.

17. The method as claimed in any of claims 11 to 16 wherein said monitoring of said disease comprises determining regression, course or onset of said disease in a sample from a patient who has said disease or is suspected of falling ill with said disease.

18. The method as claimed in any of claims 12-17, in which the agent is labeled in a detectable manner.

19. The method as claimed in any of claims 11-18, in which the sample comprises body fluid and/or body tissue.

20. The method as claimed in any of claims 11-19, in which said cancer disease is **characterized by** expression or abnormal expression of said tumor-associated nucleic acid and preferably is further **characterized by** expression or abnormal expression of a tumor-associated antigen encoded by said tumor-associated nucleic acid.

21. A method of treating or preventing a disease **characterized by** expression or abnormal expression of a tumor-associated antigen, which method comprises administration of a pharmaceutical composition as claimed in any of claims 1-10,
said tumor-associated antigen having a sequence encoded by a nucleic acid which is selected from the group consisting of:
(a) a nucleic acid which comprises a nucleic acid sequence selected from the group consisting of SEQ ID NOs: 634, 1-540, 541, 545, 549, 553, 557, 560,563,566,570,574,577,580,583,587,591,595,599,602,606,610, 613,617,620,624,638,642,646,649,653,656,660,664,668,671,675, 679, 682, and 686, a part or derivative thereof,
(b) a nucleic acid which hybridizes with the nucleic acid of (a) under stringent conditions,
(c) a nucleic acid which is degenerate with respect to the nucleic acid of (a) or (b), and
(d) a nucleic acid which is complementary to the nucleic acid of (a), (b) or (c).

22. A method of treating, preventing, diagnosing or monitoring a disease **characterized by** expression or abnormal expression of a tumor-associated antigen, which method comprises administering an antibody binding to said tumor-associated antigen or to a part thereof and coupled to a therapeutic or diagnostic agent,
said tumor-associated antigen having a sequence encoded by a nucleic acid which is selected from the group consisting of:
(a) a nucleic acid which comprises a nucleic acid sequence selected from the group consisting of SEQ ID NOs: 634, 1-540, 541, 545, 549, 553, 557, 560, 563, 566, 570, 574, 577, 580, 583, 587, 591, 595, 599, 602, 606, 610, 613,617,620,624,638,642,646,649,653,656,660,664,668,671,675, 679, 682, and 686, a part or derivative thereof,
(b) a nucleic acid which hybridizes with the nucleic acid of (a) under stringent conditions,
(c) a nucleic acid which is degenerate with respect to the nucleic acid of (a) or (b), and
(d) a nucleic acid which is complementary to the nucleic acid of (a), (b) or (c).

23. The method as claimed in claim 14 or 22, in which the antibody is a monoclonal, chimeric, human or humanized antibody, or is a fragment of an antibody.

24. A method of treating a patient having a disease **characterized by** expression or abnormal expression of a tumor-associated antigen, which method comprises:
(i) providing a sample containing immunoreactive cells,
(ii) contacting said sample with a host cell expressing said tumor-associated antigen or a part thereof, under conditions which favor production of cytolytic or cytokine-releasing T cells against said tumor-associated antigen or said part thereof, and
(iii) introducing the cytolytic or cytokine-releasing T cells into the patient in an amount suitable for lysing cells expressing the tumor-associated antigen or a part thereof,
said tumor-associated antigen having a sequence encoded by a nucleic acid which is selected from the group consisting of:
(a) a nucleic acid which comprises a nucleic acid sequence selected from the group consisting of SEQ ID NOs: 634, 1-540, 541, 545, 549, 553, 557, 560, 563, 566, 570, 574, 577, 580, 583, 587, 591, 595, 599, 602, 606, 610, 613, 617, 620, 624, 638, 642, 646, 649, 653, 656, 660, 664, 668, 671, 675, 679, 682, and 686, a part or derivative thereof,
(b) a nucleic acid which hybridizes with the nucleic acid of (a) under stringent conditions,
(c) a nucleic acid which is degenerate with respect to the nucleic acid of (a) or (b), and
(d) a nucleic acid which is complementary to the nucleic acid of (a), (b) or (c).

25. The method as claimed in claim 24, in which the host cell recombinantly expresses an MHC molecule binding to the tumor-associated antigen or to a part thereof or endogenously expresses an MHC molecule binding to the tumor-associated antigen or to a part thereof.

26. A method of inhibiting the development of cancer in a patient, which method comprises administering an effective amount of a pharmaceutical composition as claimed in any of claims 1-10.

27. An agent, which binds specifically to a protein or polypeptide or to a part thereof, said protein or polypeptide being encoded by a nucleic acid selected from the group consisting of:
(a) a nucleic acid which comprises a nucleic acid sequence selected from the group consisting of SEQ ID NOs: 634, 1-540, 541, 545, 549, 553, 557, 560, 563, 566, 570, 574, 577, 580, 583, 587, 591, 595, 599, 602, 606, 610, 613, 617, 620, 624, 638, 642, 646, 649, 653, 656, 660, 664, 668, 671, 675, 679, 682, and 686, a part or derivative thereof,
(b) a nucleic acid which hybridizes with the nucleic acid of (a) under stringent conditions,
(c) a nucleic acid which is degenerate with respect to the nucleic acid of (a) or (b), and
(d) a nucleic acid which is complementary to the nucleic acid of (a), (b) or (c).

28. The agent as claimed in claim 27, which is an antibody, preferably a monoclonal, chimeric, human or humanized antibody, or a fragment of an antibody.

29. An antibody, which binds selectively to a complex of:
(i) a protein or polypeptide or a part thereof and
(ii) an MHC molecule to which said protein or polypeptide or said part thereof binds,
with said antibody not binding to (i) or (ii) alone and said protein or polypeptide being encoded by a nucleic acid selected from the group consisting of:
(a) a nucleic acid which comprises a nucleic acid sequence selected from the group consisting of SEQ ID NOs: 634, 1-540, 541, 545, 549, 553, 557, 560,563,566,570,574,577,580,583,587,591,595,599,602,606,610, 613, 617, 620, 624, 638, 642, 646, 649, 653, 656, 660, 664, 668, 671, 675, 679, 682, and 686, a part or derivative thereof,
(b) a nucleic acid which hybridizes with the nucleic acid of (a) under stringent conditions,
(c) a nucleic acid which is degenerate with respect to the nucleic acid of (a) or (b), and
(d) a nucleic acid which is complementary to the nucleic acid of (a), (b) or (c).

30. The antibody as claimed in claim 29, which is a monoclonal, chimeric or humanized antibody, or is a fragment of an antibody.

31. A conjugate between an agent as claimed in claim 27 or 28 or an antibody as claimed in claim 29 or 30 and a therapeutic or diagnostic agent.

32. A kit for detecting cancer, which kit comprises agents for detecting or determining the quantity
(i) of a tumor-associated nucleic acid or of a part thereof, and/or
(ii) of a tumor-associated antigen or of a part thereof, and/or
(iii) of antibodies which bind to the tumor-associated antigen or to a part thereof, and/or
(iv) of T cells which are specific for a complex between the tumor-associated antigen or a part thereof and an MHC molecule,
said tumor-associated nucleic acid being selected from the group consisting of:
(a) a nucleic acid which comprises a nucleic acid sequence selected from the group consisting of SEQ ID NOs: 634, 1-540, 541, 545, 549, 553, 557, 560, 563, 566, 570, 574, 577, 580, 583, 587, 591, 595, 599, 602, 606, 610, 613, 617, 620, 624, 638, 642, 646, 649, 653, 656, 660, 664, 668, 671, 675, 679, 682, and 686, a part or derivative thereof,
(b) a nucleic acid which hybridizes with the nucleic acid of (a) under stringent conditions,
(c) a nucleic acid which is degenerate with respect to the nucleic acid of (a) or (b), and
(d) a nucleic acid which is complementary to the nucleic acid of (a), (b) or (c), and
said tumor-associated antigen having a sequence encoded by a nucleic acid which is selected from said group of nucleic acids.

33. The pharmaceutical composition as claimed in any of claims 1-10, the method as claimed in any of claims 11-26 or the kit as claimed in claim 32 in which the tumor-associated antigen comprises an amino acid sequence selected from the group consisting of SEQ ID NOs: 635, 542, 546, 550, 554, 567, 571, 584, 588, 592, 596, 603, 607, 614, 621, 625, 639, 643, 650, 657, 661, 665, 672, 676, and 683, a part or derivative thereof.

34. The agent as claimed in claim 27 or 28, the antibody as claimed in claim 29 or 30 or the conjugate as claimed in claim 31 in which the protein or polypeptide comprises an amino acid sequence selected from the group consisting of SEQ ID NOs: 635, 542, 546, 550, 554, 567, 571, 584, 588, 592, 596, 603, 607, 614, 621, 625, 639, 643, 650, 657, 661, 665, 672, 676, and 683, a part or derivative thereof.
